# EUROPEAN PATENT SPECIFICATION

(11) **EP 3 327 114 B1**
(45) Date of publication and mention of the grant of the patent: **06.11.2019**
(21) Application number: 18150288.1
(22) Date of filing: 22.04.2009
(51) Int. Cl.: C12N 5/0735, C12N 5/074

(54) **PLURIPOTENT CELLS**
PLURIPOTENTE ZELLEN
CELLULES PLURIPOTENTES

(30) Priority: 24.04.2008 US 108872
(43) Date of publication of application: 30.05.2018
(62) Divisional of application: 13163321.6
(73) Proprietor: Janssen Biotech, Inc., Horsham, PA 19044 (US)
(72) Inventor: REZANIA, Alireza, Hillsborough, NJ 08844 (US)
(74) Representative: Vossius & Partner Patentanwälte Rechtsanwälte mbB

(56) References cited:
- WO-A-00/29549
- WO-A2-2009/012428
- EZASHI T ET AL: "Low O2 tensions and the prevention of differentiation of hES cells", PROCEEDINGS OF THE NATIONAL ACADEMY OF SCIENCES OF USA, NATIONAL ACADEMY OF SCIENCE, WASHINGTON, DC, US, vol. 102, no. 13, 29 March 2005 (2005-03-29), pages 4783-4788, XP002466326, ISSN: 0027-8424
- KROON EVERT ET AL: "Pancreatic endoderm derived from human embryonic stem cells generates glucose-responsive insulin-secreting cells in vivo", NATURE BIOTECHNOLOGY APR 2008,, vol. 26, no. 4, 1 April 2008 (2008-04-01), pages 443-452, XP002561975,
- D'AMOUR K A ET AL: "Efficient differentiation of human embryonic stem cells to definitive endoderm", NATURE BIOTECHNOLOGY, NATURE PUBLISHING GROUP, NEW YORK, NY, US, vol. 23, no. 12, 28 October 2005 (2005-10-28), pages 1534-1541, XP002385437, ISSN: 1087-0156
- SHIRAKI N ET AL: "Guided differentiation of embryonic stem cells into Pdx1-expressing regional-specific definitive endoderm", STEM CELLS, ALPHAMED PRESS, DAYTON, OH, US, vol. 26, no. 4, 1 April 2008 (2008-04-01), pages 874-885, XP002547894, ISSN: 1066-5099 [retrieved on 2008-01-31]
- LUDWIG T E ET AL: "Derivation of human embryonic stem cells in defined conditions", NATURE BIOTECHNOLOGY 200602 US, vol. 24, no. 2, February 2006 (2006-02), pages 185-187, XP002564246,
- MORRISON SEAN J ET AL: "Culture in reduced levels of oxygen promotes clonogenic sympathoadrenal differentiation by isolated neural crest stem cells", JOURNAL OF NEUROSCIENCE, vol. 20, no. 19, 1 October 2000 (2000-10-01), pages 7370-7376, XP002552625, ISSN: 0270-6474
- KOLLER M R ET AL: "EFFECTS OF SYNERGISTIC CYTOKINE COMBINATIONS, LOW OXYGEN, AND IRRADIATED STROMA ON THE EXPANSION OF HUMAN CORD BLOOD PROGENITORS", BLOOD, AMERICAN SOCIETY OF HEMATOLOGY, US, vol. 80, no. 2, 15 July 1992 (1992-07-15), pages 403-411, XP000604784, ISSN: 0006-4971
- YASUDA EMIKO ET AL: "Development of cystic embryoid bodies with visceral yolk-sac-like structures from mouse embryonic stem cells using low-adherence 96-well plate.", JOURNAL OF BIOSCIENCE AND BIOENGINEERING APR 2009, vol. 107, no. 4, April 2009 (2009-04), pages 442-446, XP002564247, ISSN: 1347-4421

### SEQUENCE LISTING

<110> JANSSEN BIOTECH INC.
<120> PLURIPOTENT CELLS
<130> CEN5217USNP
<140> 12/108,872
   <141> 2008-04-24
<160> 3
<170> PatentIn version 3.5
<210> 1
   <211> 17
   <212> DNA
   <213> Artificial Sequence
<220>
   <223> SYNTHETIC CONSTRUCT
<400> 1
   tggcgcagca gatacca 17
<210> 2
   <211> 18
   <212> DNA
   <213> Artificial Sequence
<220>
   <223> SYNTHETIC CONSTRUCT
<400> 2
   agcgccttcc acgacttg 18
<210> 3
   <211> 23
   <212> DNA
   <213> Artificial Sequence
<220>
   <223> SYNTHETIC CONSTRUCT
<400> 3
   ccagcatctt gctcaactcg gcg 23

## Description

### FIELD OF THE INVENTION

The present invention is directed to pluripotent stem cells that can be readily expanded in culture on tissue culture polystyrene and do not require a feeder cell line. The present invention also provides methods to derive the pluripotent stem cell line from human embryonic stem cells.

### BACKGROUND

Advances in cell-replacement therapy for Type I diabetes mellitus and a shortage of transplantable islets of Langerhans have focused interest on developing sources of insulin-producing cells, or β cells, appropriate for engraftment. One approach is the generation of functional β cells from pluripotent stem cells, such as, for example, embryonic stem cells.

In vertebrate embryonic development, a pluripotent cell gives rise to a group of cells comprising three germ layers (ectoderm, mesoderm, and endoderm) in a process known as gastrulation. Tissues such as, for example, thyroid, thymus, pancreas, gut, and liver, will develop from the endoderm, via an intermediate stage. The intermediate stage in this process is the formation of definitive endoderm. Definitive endoderm cells express a number of markers, such as, HNF-3 beta, GATA-4, Mixl1, CXCR4 and SOX-17.

Formation of the pancreas arises from the differentiation of definitive endoderm into pancreatic endoderm. Cells of the pancreatic endoderm express the pancreatic-duodenal homeobox gene, PDX-1. In the absence of PDX-1, the pancreas fails to develop beyond the formation of ventral and dorsal buds. Thus, PDX-1 expression marks a critical step in pancreatic organogenesis. The mature pancreas contains, among other cell types, exocrine tissue and endocrine tissue. Exocrine and endocrine tissues arise from the differentiation of pancreatic endoderm.

Cells bearing the features of islet cells have reportedly been derived from embryonic cells of the mouse. For example, Lumelsky et al. (Science 292:1389, 2001) report differentiation of mouse embryonic stem cells to insulin-secreting structures similar to pancreatic islets. Soria et al. (Diabetes 49:157, 2000) report that insulin-secreting cells derived from mouse embryonic stem cells normalize glycemia in streptozotocin-induced diabetic mice.

In one example, Hori et al. (PNAS 99: 16105, 2002) disclose that treatment of mouse embryonic stem cells with inhibitors of phosphoinositide 3-kinase (LY294002) produced cells that resembled β cells.

In another example, Blyszczuk et al. (PNAS 100:998, 2003) reports the generation of insulin-producing cells from mouse embryonic stem cells constitutively expressing Pax4.

Micallef et al. reports that retinoic acid can regulate the commitment of embryonic stem cells to form PDX-1 positive pancreatic endoderm. Retinoic acid is most effective at inducing PDX-1 expression when added to cultures at day 4 of embryonic stem cell differentiation during a period corresponding to the end of gastrulation in the embryo (Diabetes 54:301, 2005).

Miyazaki et al. reports a mouse embryonic stem cell line over-expressing PDX-1. Their results show that exogenous PDX-1 expression clearly enhanced the expression of insulin, somatostatin, glucokinase, neurogenin3, P48, Pax6, and HNF6 genes in the resulting differentiated cells (Diabetes 53: 1030, 2004).

Skoudy et al. reports that activin-A (a member of the TGFβ superfamily) upregulates the expression of exocrine pancreatic genes (p48 and amylase) and endocrine genes (PDX-1, insulin, and glucagon) in mouse embryonic stem cells. The maximal effect was observed using InM activin-A. They also observed that the expression level of insulin and PDX-1 mRNA was not affected by retinoic acid; however, 3nM FGF-7 treatment resulted in an increased level of the transcript for PDX-1 (Biochem. J. 379: 749, 2004).

Shiraki et al. studied the effects of growth factors that specifically enhance differentiation of embryonic stem cells into PDX-1 positive cells. They observed that TGFβ2 reproducibly yielded a higher proportion of PDX-1 positive cells (Genes Cells. 2005 Jun; 10(6): 503-16.).

Gordon *et al*. demonstrated the induction of brachyury+/HNF-3 beta+ endoderm cells from mouse embryonic stem cells in the absence of serum and in the presence of activin along with an inhibitor of Wnt signaling (US2006/0003446A1).

Gordon et al. (PNAS, Vol 103, page 16806, 2006) states "Wnt and TGF-beta/nodal/ activin signaling simultaneously were required for the generation of the anterior primitive streak".

However, the mouse model of embryonic stem cell development may not exactly mimic the developmental program in higher mammals, such as, for example, humans.

Thomson et al. isolated embryonic stem cells from human blastocysts (Science 282:114, 1998). Concurrently, Gearhart and coworkers derived human embryonic germ (hEG) cell lines from fetal gonadal tissue (Shamblott et al., Proc. Natl. Acad. Sci. USA 95:13726, 1998). Unlike mouse embryonic stem cells, which can be prevented from differentiating simply by culturing with Leukemia Inhibitory Factor (LIF), human embryonic stem cells must be maintained under very special conditions (U.S. Pat. No. 6,200,806; WO 99/20741; WO 01/51616).

D'Amour *et al*. describes the production of enriched cultures of human embryonic stem cell-derived definitive endoderm in the presence of a high concentration of activin and low serum (D'Amour KA et al. 2005). Transplanting these cells under the kidney capsule of mice resulted in differentiation into more mature cells with characteristics of some endodermal organs. Human embryonic stem cell-derived definitive endoderm cells can be further differentiated into PDX-1 positive cells after addition of FGF-10 (US 2005/0266554A1).

D'Amour et al. (Nature Biotechnology - 24, 1392 - 1401 (2006)) states "We have developed a differentiation process that converts human embryonic stem (hES) cells to endocrine cells capable of synthesizing the pancreatic hormones insulin, glucagon, somatostatin, pancreatic polypeptide and ghrelin. This process mimics *in vivo* pancreatic organogenesis by directing cells through stages resembling definitive endoderm, gut-tube endoderm, pancreatic endoderm and endocrine precursor en route to cells that express endocrine hormones".

In another example, Fisk *et al*. reports a system for producing pancreatic islet cells from human embryonic stem cells (US2006/0040387A1). In this case, the differentiation pathway was divided into three stages. Human embryonic stem cells were first differentiated to endoderm using a combination of n-butyrate and activin-A. The cells were then cultured with TGFβ antagonists such as Noggin in combination with EGF or betacellulin to generate PDX-1 positive cells. The terminal differentiation was induced by nicotinamide.

In one example, Benvenistry et al. states: "We conclude that over-expression of PDX-1 enhanced expression of pancreatic enriched genes, induction of insulin expression may require additional signals that are only present in vivo*"* (Benvenistry et al, Stem Cells 2006; 24:1923-1930).

Current methods to culture human embryonic stem cells requires the use of either extracellular matrix proteins or a fibroblast feeder layer, or the addition of exogenous growth factors, such as, for example, bFGF.

In one example, Cheon et al (BioReprod DOI: 10.1095/biolreprod.105.046870, October 19, 2005) disclose a feeder-free, serum-free culture system in which embryonic stem cells are maintained in unconditioned serum replacement (SR) medium supplemented with different growth factors capable of triggering embryonic stem cell self-renewal.

In another example, Levenstein et al (Stem Cells 24: 568-574, 2006) disclose methods for the long-term culture of human embryonic stem cells in the absence of fibroblasts or conditioned medium, using media supplemented with bFGF.

In another example, US20050148070 discloses a method of culturing human embryonic stem cells in defined media without serum and without fibroblast feeder cells, the method comprising: culturing the stem cells in a culture medium containing albumin, amino acids, vitamins, minerals, at least one transferrin or transferrin substitute, at least one insulin or insulin substitute, the culture medium essentially free of mammalian fetal serum and containing at least about 100 ng/ml of a fibroblast growth factor capable of activating a fibroblast growth factor signaling receptor, wherein the growth factor is supplied from a source other than just a fibroblast feeder layer, the medium supported the proliferation of stem cells in an undifferentiated state without feeder cells or conditioned medium.

In another example, US20050233446 discloses a defined media useful in culturing stem cells, including undifferentiated primate primordial stem cells. In solution, the media is substantially isotonic as compared to the stem cells being cultured. In a given culture, the particular medium comprises a base medium and an amount of each of bFGF, insulin, and ascorbic acid necessary to support substantially undifferentiated growth of the primordial stem cells.

In another example, US6800480 states "In one embodiment, a cell culture medium for growing primate-derived primordial stem cells in a substantially undifferentiated state is provided which includes a low osmotic pressure, low endotoxin basic medium that is effective to support the growth of primate-derived primordial stem cells. The basic medium is combined with a nutrient serum effective to support the growth of primate-derived primordial stem cells and a substrate selected from the group consisting of feeder cells and an extracellular matrix component derived from feeder cells. The medium further includes nonessential amino acids, an anti-oxidant, and a first growth factor selected from the group consisting of nucleosides and a pyruvate salt."

In another example, US20050244962 states: "In one aspect the invention provides a method of culturing primate embryonic stem cells. One cultures the stem cells in a culture essentially free of mammalian fetal serum (preferably also essentially free of any animal serum) and in the presence of fibroblast growth factor that is supplied from a source other than just a fibroblast feeder layer. In a preferred form, the fibroblast feeder layer, previously required to sustain a stem cell culture, is rendered unnecessary by the addition of sufficient fibroblast growth factor."

In a further example, WO2005065354 discloses a defined, isotonic culture medium that is essentially feeder-free and serum-free, comprising: a. a basal medium; b. an amount of bFGF sufficient to support growth of substantially undifferentiated mammalian stem cells; c. an amount of insulin sufficient to support growth of substantially undifferentiated mammalian stem cells; and d. an amount of ascorbic acid sufficient to support growth of substantially undifferentiated mammalian stem cells.

In another example, WO2005086845 discloses a method for maintenance of an undifferentiated stem cell, said method comprising exposing a stem cell to a member of the transforming growth factor-beta (TGFβ) family of proteins, a member of the fibroblast growth factor (FGF) family of proteins, or nicotinamide (NIC) in an amount sufficient to maintain the cell in an undifferentiated state for a sufficient amount of time to achieve a desired result.

Ezashi et. al. (PNAS 102:13 p4783-4788, 2005) discloses that hypoxic culture would appear to be necessary to maintain full pluripotency of hES cells.

Additionally, formation of pancreatic endocrine cells, pancreatic hormone expressing cells, or pancreatic hormone secreting cells from human embryonic cells may require genetic manipulation of the human embryonic stem cells. Transfection of human embryonic stem cells using traditional techniques, such as, for example, lipofectamine or electroporation is inefficient.

WO2007027157 discloses a method comprising: (a) providing an embryonic stem (ES) cell; and (b) establishing a progenitor cell line from the embryonic stem cell; in which the progenitor cell line is selected based on its ability to self-renew. Preferably, the method selects against somatic cells based on their inability to self renew. Preferably, the progenitor cell line is derived or established in the absence of co-culture, preferably in the absence of feeder cells, which preferably selects against embryonic stem cells. Optionally, the method comprises (d) deriving a differentiated cell from the progenitor cell line.

Therefore, there still remains a significant need to develop conditions for establishing pluripotent stem cell lines that can be expanded to address the current clinical needs, while retaining the potential to differentiate into pancreatic endocrine cells, pancreatic hormone expressing cells, or pancreatic hormone secreting cells.

### SUMMARY OF THE INVENTION

The invention provides a method for deriving a population of cells comprising cells expressing pluripotency markers comprising the steps of:
a. culturing human embryonic stem cells, and
b. removing the cultured human embryonic stem cells, and subsequently culturing the removed cultured human embryonic stem cells under hypoxic conditions, on a tissue culture substrate that is not pre-treated with a protein or an extracellular matrix prior to culturing the cells, in culture medium contains serum, activin-A, a Wnt ligand, and optionally IGF-1
wherein the cells expressing pluripotency markers express at least one of the pluripotency markers selected from the group consisting of ABCG2, cripto, FoxD3, Connexin43, Connexin45, Oct4, SOX-2, Nanog, hTERT, UTF-I, ZFP42, SSEA-3, SSEA-4, Tra1-60, and Tra1-81,
and wherein the cells expressing pluripotency markers express markers characteristic of:
pre-primitive streak cells; or
primitive streak cells; or
mesoendoderm cells.

### SUMMARY OF THE DISCLOSURE

The present disclosure provides a cell population with characteristics of human embryonic stem cells, that can be readily expanded in culture, in low serum, that requires no feeder cell line or a coating of complex matrix proteins, can be passaged in single cell suspension, can be transfected with a very high efficiency, and cultured under hypoxic conditions. This combination of unique attributes separates the cells described in the present disclosure from the prior art.

In one embodiment, the present disclosure provides a method for deriving a population of cells comprising cells expressing pluripotency markers, comprising the steps of:
a. Obtaining cells, and
b. Culturing the cells under hypoxic conditions, on a tissue culture substrate that is not pre-treated with a protein or an extracellular matrix prior to culturing the cells.

The cells may be human embryonic stem cells, or they may be cells expressing markers characteristic of the definitive endoderm lineage. The human embryonic stem cells may be cultured in normoxic conditions prior to culturing the cells on a tissue culture substrate that is not pre-treated with a protein or an extracellular matrix. Alternatively, the human embryonic stem cells may be cultured in hypoxic conditions.

The human embryonic stem cells may be cultured in normoxic conditions prior to culturing the cells on a tissue culture substrate that is not pre-treated with a protein or an extracellular matrix, and treated with a Rho kinase inhibitor. Alternatively, the human embryonic stem cells may be cultured in hypoxic conditions, and treated with a Rho kinase inhibitor.

The cells expressing markers characteristic of the definitive endoderm lineage may be cultured in normoxic conditions prior to culturing the cells on a tissue culture substrate that is not pre-treated with a protein or an extracellular matrix. Alternatively, the cells expressing markers characteristic of the definitive endoderm lineage may be cultured in hypoxic conditions.

In one embodiment, the present disclosure provides a method for deriving a population of cells comprising cells expressing pluripotency markers, comprising the steps of:
a. Culturing human embryonic stem cells,
b. Differentiating the human embryonic stem cells into cells expressing markers characteristic of definitive endoderm cells, and
c. Removing the cells, and subsequently culturing them under hypoxic conditions, on a tissue culture substrate that is not pre-treated with a protein or an extracellular matrix prior to culturing the cells.

The cells may be cultured under hypoxic conditions, on a tissue culture substrate that is not pre-treated with a protein or an extracellular matrix, in medium containing serum, activin-A, and a Wnt ligand. Alternatively, the cells may be cultured under hypoxic conditions, on a tissue culture substrate that is not pretreated with a protein or an extracellular matrix, in medium containing serum, activin-A, a Wnt ligand, and IGF-1.

The cells may be cultured under hypoxic conditions, on a tissue culture substrate that is not pre-treated with a protein or an extracellular matrix, in medium containing serum, a Rho kinase inhibitor, activin-A, and a Wnt ligand. Alternatively, the cells may be cultured under hypoxic conditions, on a tissue culture substrate that is not pretreated with a protein or an extracellular matrix, in medium containing serum, a Rho kinase inhibitor, activin-A, a Wnt ligand, and IGF-1.

In one embodiment, the present disclosure provides a method for deriving a population of cells comprising cells expressing pluripotency markers, comprising the steps of:
a. Culturing human embryonic stem cells, and
b. Removing the cells, and subsequently culturing them under hypoxic conditions, on a tissue culture substrate that is not pre-treated with a protein or an extracellular matrix.

The cells may be cultured under hypoxic conditions, on a tissue culture substrate that is not pre-treated with a protein or an extracellular matrix, in medium containing serum, activin-A, and a Wnt ligand. Alternatively, the cells may be cultured under hypoxic conditions, on a tissue culture substrate that is not pretreated with a protein or an extracellular matrix, in medium containing serum, activin-A, a Wnt ligand, and IGF-1.

The cells may be cultured under hypoxic conditions, on a tissue culture substrate that is not pre-treated with a protein or an extracellular matrix, in medium containing serum, a Rho kinase inhibitor, activin-A, and a Wnt ligand. Alternatively, the cells may be cultured under hypoxic conditions, on a tissue culture substrate that is not pretreated with a protein or an extracellular matrix, in medium containing serum, a Rho kinase inhibitor, activin-A, a Wnt ligand, and IGF-1.

The cells expressing pluripotency markers derived by the methods of the present disclosure are capable of expansion in culture under hypoxic conditions, on tissue culture substrate that is not pre-treated with a protein or an extracellular matrix.

In one embodiment, the present disclosure provides a method to expand cells expressing markers characteristic of the definitive endoderm lineage, comprising the steps of culturing the cells under hypoxic conditions, on a tissue culture substrate that is not pre-treated with a protein or an extracellular matrix. In one embodiment, the cells expressing markers characteristic of the definitive endoderm lineage are derived from pluripotent cells formed by the methods of the present disclosure.

The cells may be cultured under hypoxic conditions, on a tissue culture substrate that is not pre-treated with a protein or an extracellular matrix, in medium containing serum, activin-A, a Wnt ligand, and a GSK-3B inhibitor.

### BRIEF DESCRIPTION OF THE FIGURES

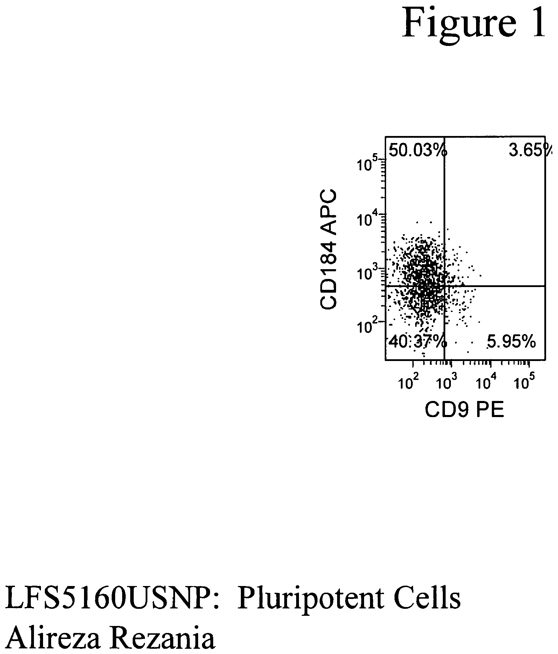

**Figure 1** shows the expression of CXCR4 (CD 184, Y-axis) and CD9 (X-axis) in cells from the human embryonic stem cell line H9 at passage 54 that have been differentiated into definitive endoderm following treatment with low serum +Activin-A + WNT-3A for 4 days.

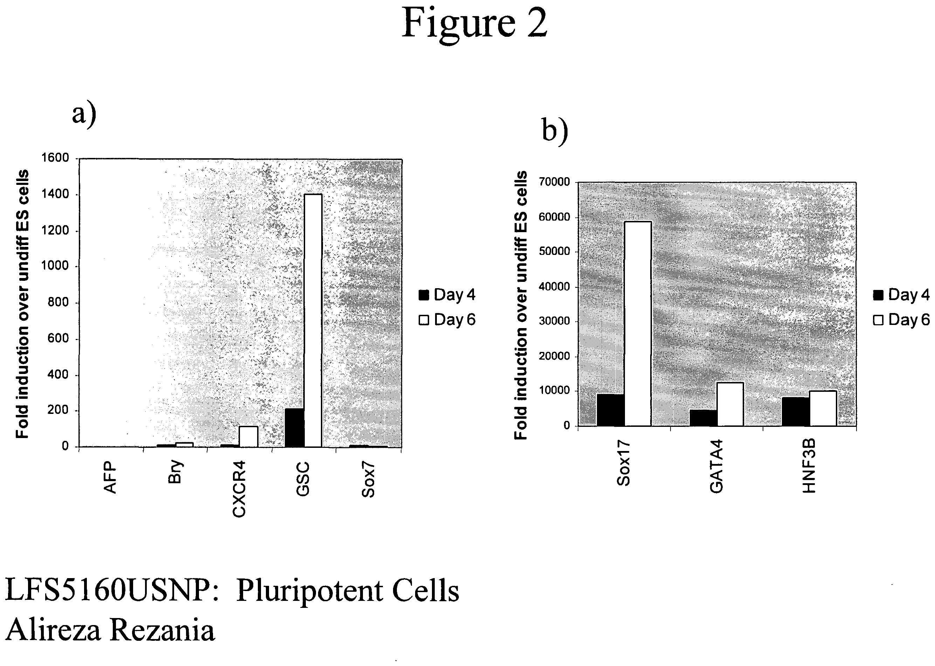

**Figure 2** shows the real-time PCR analysis of cells from the human embryonic stem cell line H9 at passage 54 at day 4 and 6 of the definitive endoderm differentiation protocol outlined in **Example 5.** Panel a) depicts expression of AFP, Bry, CXCR4, GSC, and SOX-7. Panel b) depicts the expression of SOX-17, GATA-4, and HNF-3 beta.

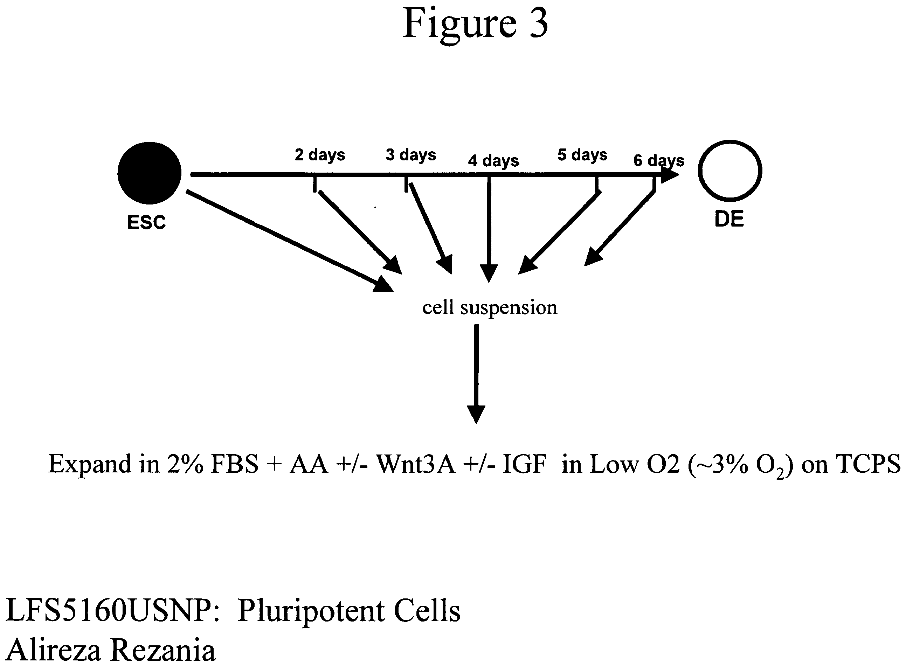

**Figure 3** shows the isolation protocol used to derive EXPRES cells from embryonic stem cells according to the methods of the present invention.

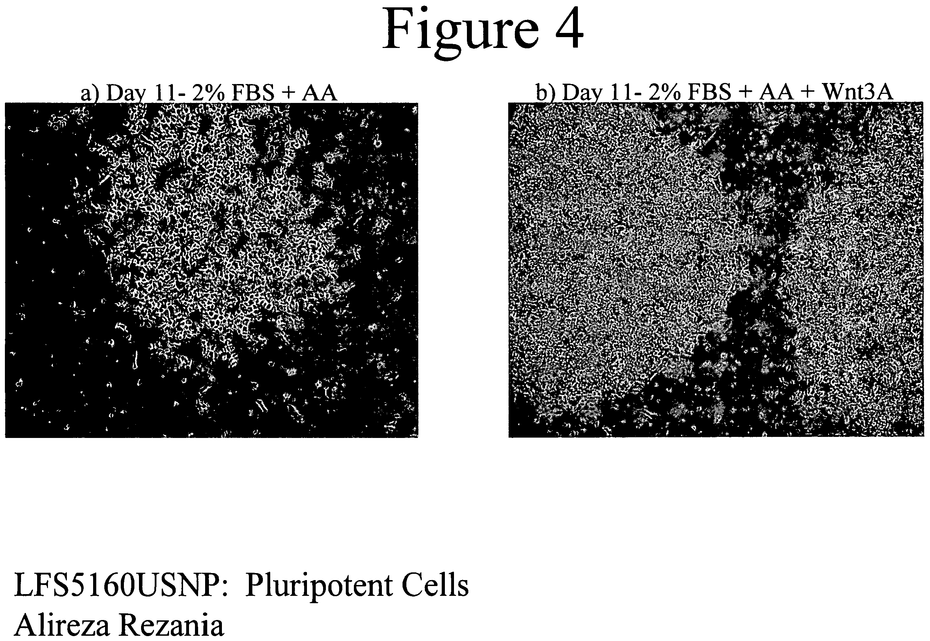

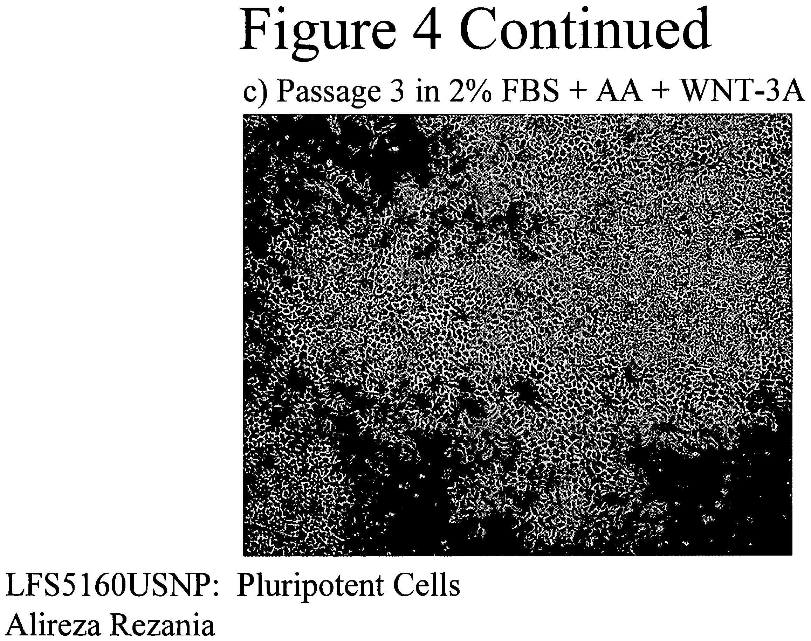

**Figure 4** shows the morphology of expanded EXPRES cells at P0, at day 11, that were cultured in 2% FBS + DMEM-F12 + 100 ng/ml of Activin-A (Panel a) or 2% FBS + DMEM-F12 + 100 ng/ml of Activin-A + 20 ng/ml of WNT-3A (Panel b). Panel c shows the morphology of the EXPRES cells at passage 3.

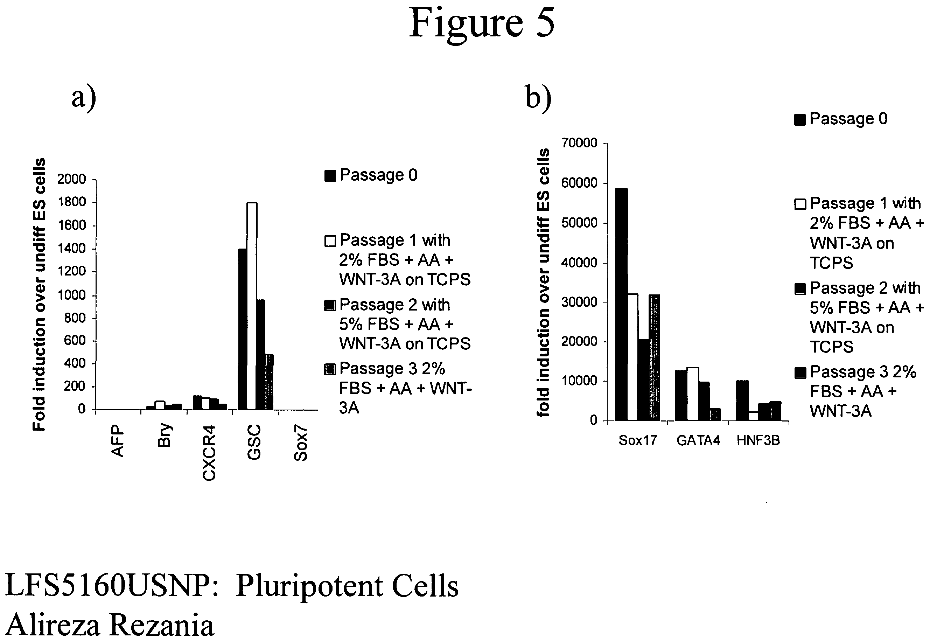

**Figure 5** shows the real-time PCR analysis of expanded EXPRES cells cultured in 2-5% FBS + DMEM-F12 + 100 ng/ml of Activin-A + 20 ng/ml of WNT-3A for three passages. Panel a) depicts expression of AFP, Bry, CXCR4, GSC, and SOX-7. Panel b) depicts the expression of SOX-17, GATA-4, and HNF-3 beta.

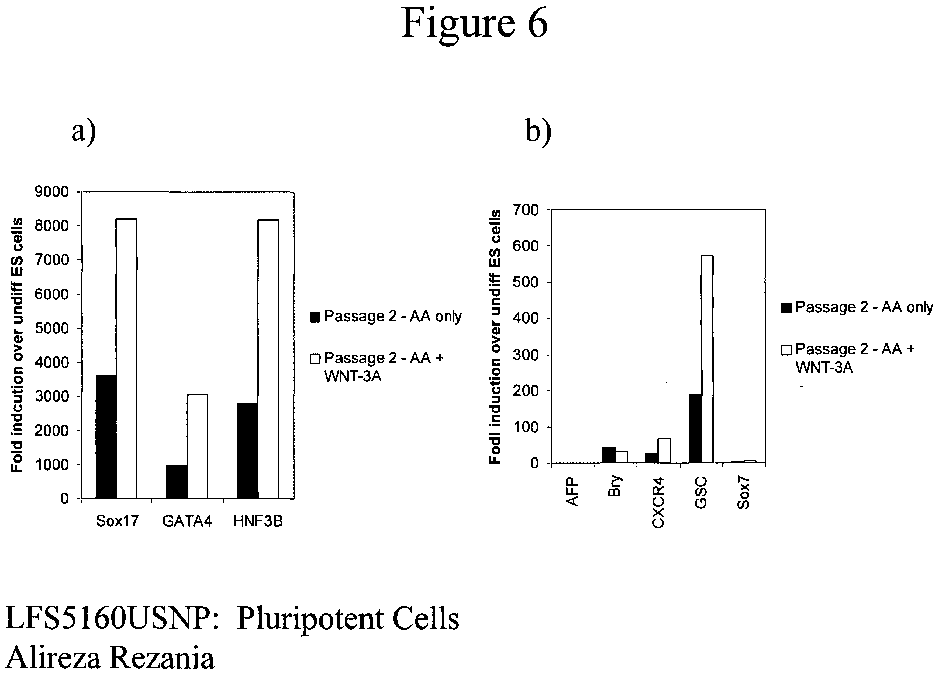

**Figure 6** shows the effect of the addition of Wnt-3A on gene expression in EXPRES cells. Panel a) depicts real-time PCR expression of SOX-17, GATA-4, and HNF-3 beta. Panel b) depicts real-time PCR expression of AFP, Bry, CXCR4, GSC, and SOX-7.

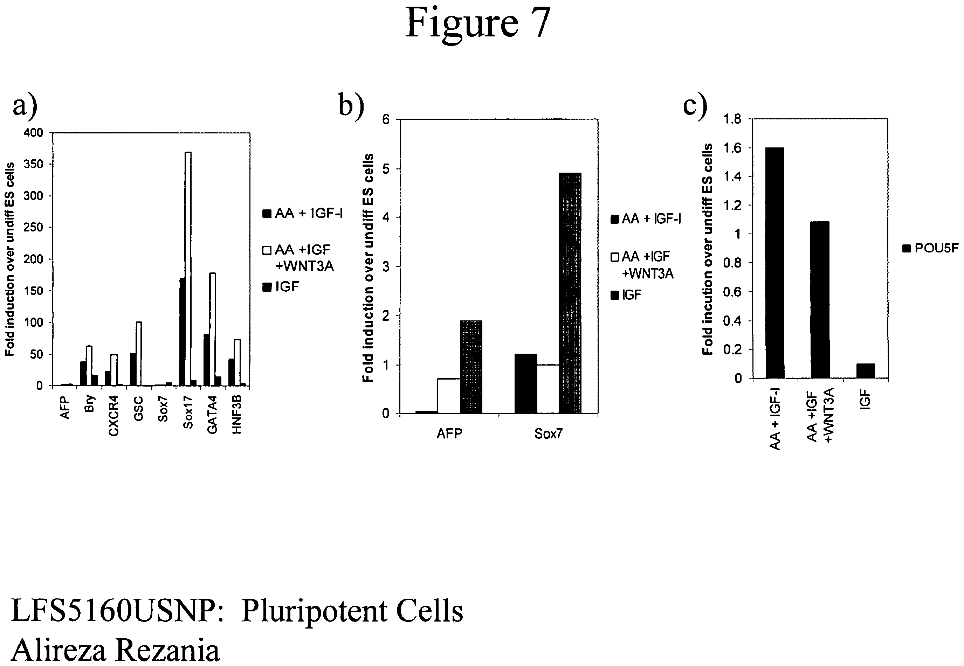

**Figure 7** shows the effect of IGF-1, Wnt-3A and activin-A on gene expression in EXPRES cells. Panel a) depicts real-time PCR expression of SOX-17, GATA-4, HNF-3 beta, Bry, CXCR4, and GSC. Panel b) depicts real-time PCR expression of and SOX-7 and AFP. Panel c) depicts real-time PCR expression of OCT-4.

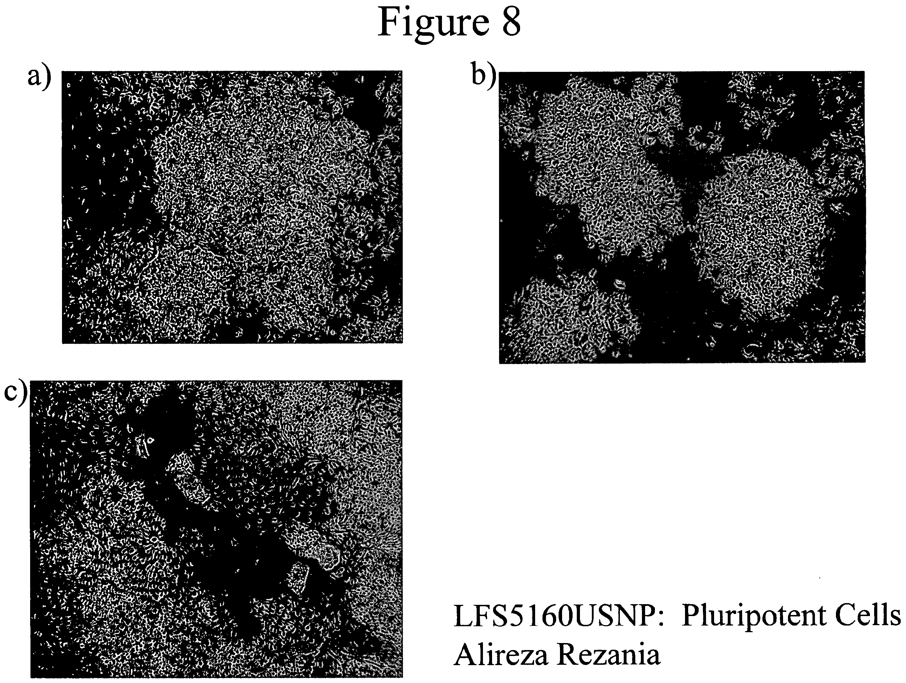

**Figure 8** shows the morphology of expanded EXPRES cells derived from the human embryonic stem cell line H9 at passage 54, cultured in a) 2% FBS +DMEM-F12 + 100 ng/ml of AA + 20 ng/ml of WNT-3A, b) 2% FBS + DMEMF12 + 100 ng/ml of AA, c) 2% FBS + DMEM-F12 + 50 ng/ml of IGF-I.

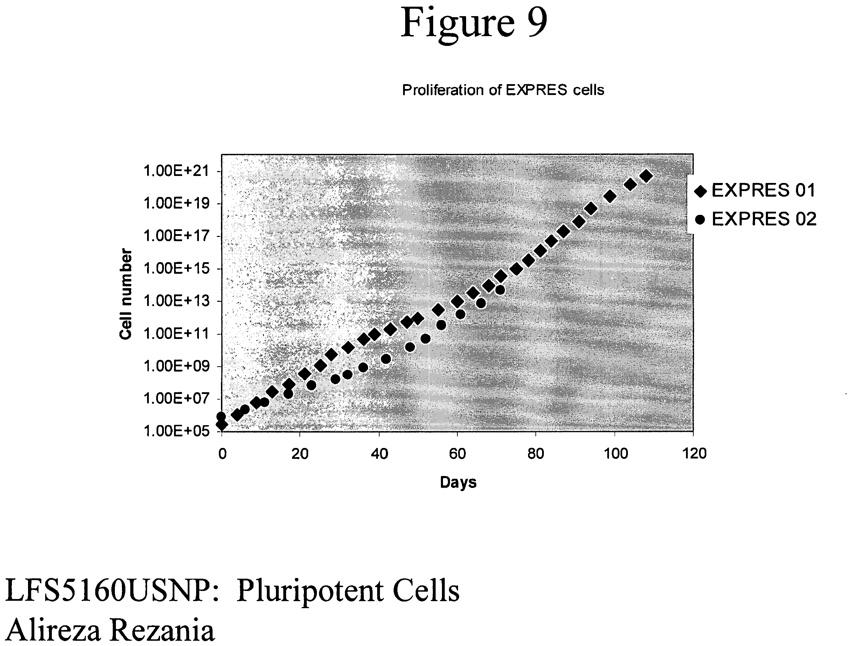

**Figure 9** shows the expansion potential of EXPRES 01 and 02 cells cultured on tissue culture polystyrene under hypoxic conditions. EXPRES 01 was cultured in 2% FBS + DM-F12 + 100 ng/ml of AA + 20 ng/ml of WNT-3A + 50 ng/ml of IGF-I and EXPRES 02 cells were cultured in 2% FBS + DM-F12 + 100 ng/ml of AA + 20 ng/ml of WNT-3A.

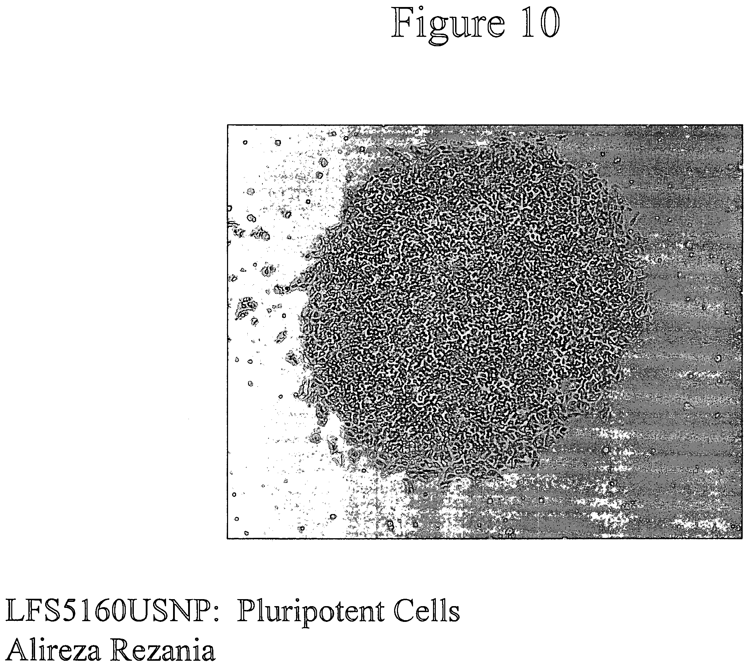

**Figure 10** shows the morphology of EXPRES cells derived from single cell suspension of undifferentiated ES cells on TCPS (tissue culture polystyrene) in DM-F12 + 2% FBS + 100 ng/ml AA + 20 ng/ml WNT3A + 50 ng/ml of IGF-I.

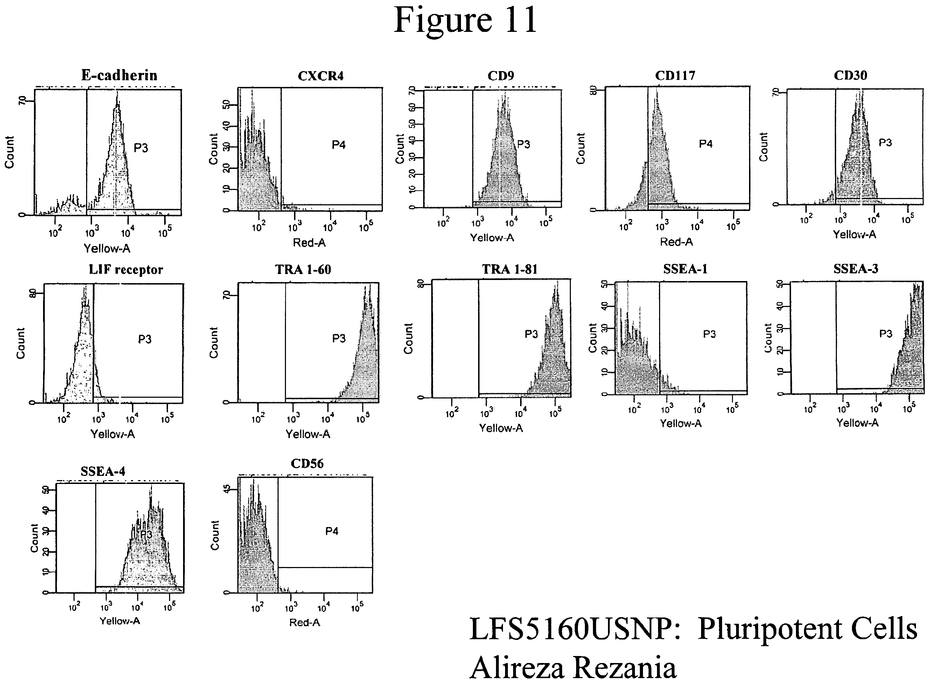

**Figure 11** shows the protein expression as determined by FACS in EXPRES 01 cells at passage 24 cells. Panel a) shows the expression levels of E-cadherin, panel b) shows the expression levels of CXCR4, panel c) shows the expression levels of CD9, panel d) shows the expression levels of CD117, panel e) shows the expression levels of CD30, panel f) shows the expression levels of LIF receptor, panel g) shows the expression levels of TRA 1-60, panel h) shows the expression levels of TRA 1-81, panel i) shows the expression levels of SSEA-1, panel j) shows the expression levels of SSEA-3, panel k) shows the expression levels of SSEA-4, and Panel 1) shows the expression levels of CD56.

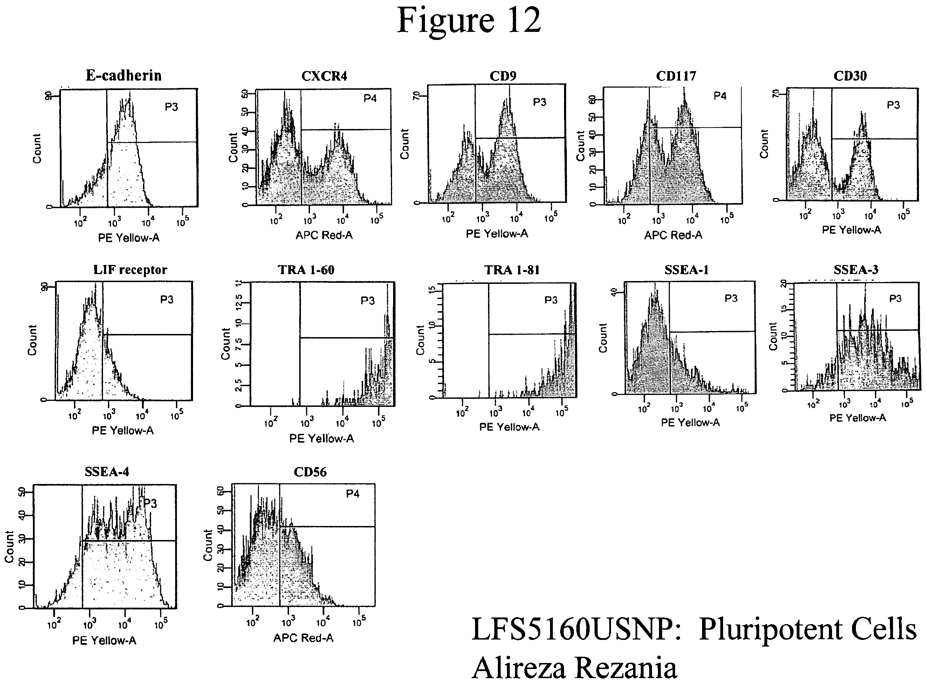

**Figure 12** shows the protein expression as determined by FACS in EXPRES 02 at passage 21 cells. Panel a) shows the expression levels of E-cadherin, panel b) shows the expression levels of CXCR4, panel c) shows the expression levels of CD9, panel d) shows the expression levels of CD117, panel e) shows the expression levels of CD30, panel f) shows the expression levels of LIF receptor, panel g) shows the expression levels of TRA 1-60, panel h) shows the expression levels of TRA 1-81, panel i) shows the expression levels of SSEA-1, panel j) shows the expression levels of SSEA-3, panel k) shows the expression levels of SSEA-4, and panel 1) shows the expression levels of CD56.

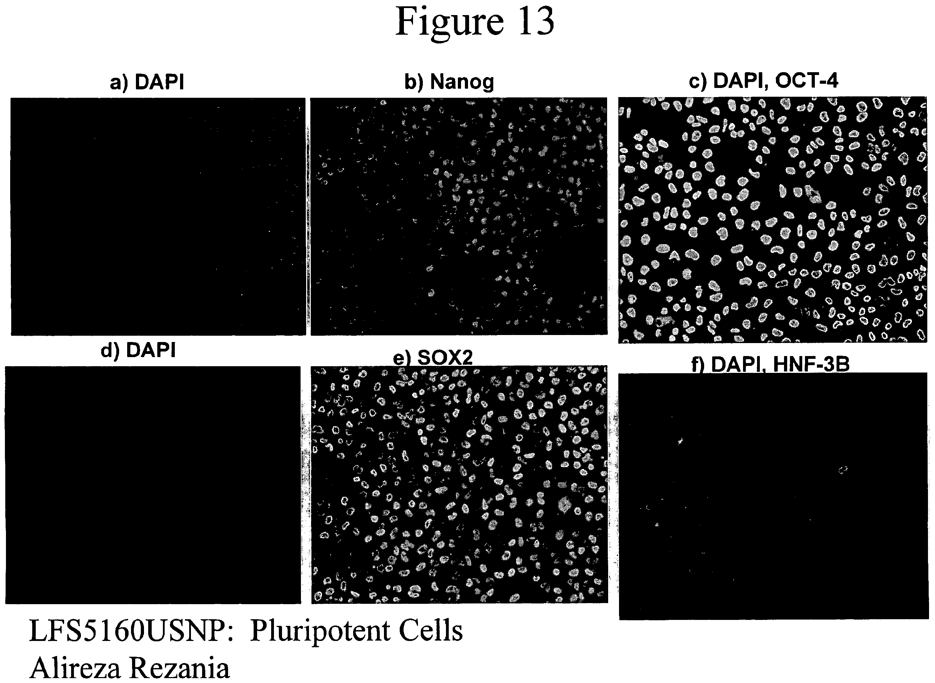

**Figure 13** shows immuno fluorescent images of EXPRES 01 at passage 10 cultured in 2% FBS + DMEM-F12 + 100 ng/ml of AA + 20 ng/ml of WNT-3A + 50 ng/ml of IGF-I. Panel a) DAPI image for panel b, panel b) Nanog, panel c) DAPI (blue) and Oct-4 (green) co-staining, panel d) DAPI image for panel e, panel e) SOX-2, and panel f) DAPI (blue) and HNF-3 beta (green) co-staining.

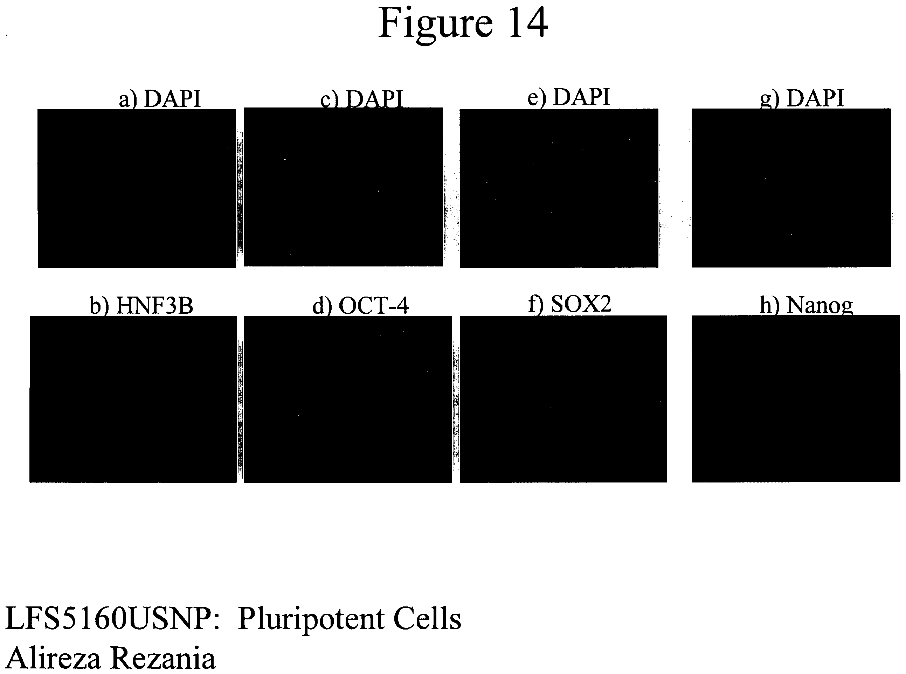

**Figure 14** shows immuno fluorescent images of EXPRES 02 at passage 9 cultured in 2% FBS + DMEM-F12 + 100 ng/ml of AA + 20 ng/ml of WNT-3A. Panel a) DAPI image for panel b, panel b) HNf3B, panel c) DAPI image for panel d, panel d) OCT-4, panel e) DAPI image for panel f, panel f) SOX-2, panel g) DAPI image for panel h, panel h) NANOG.

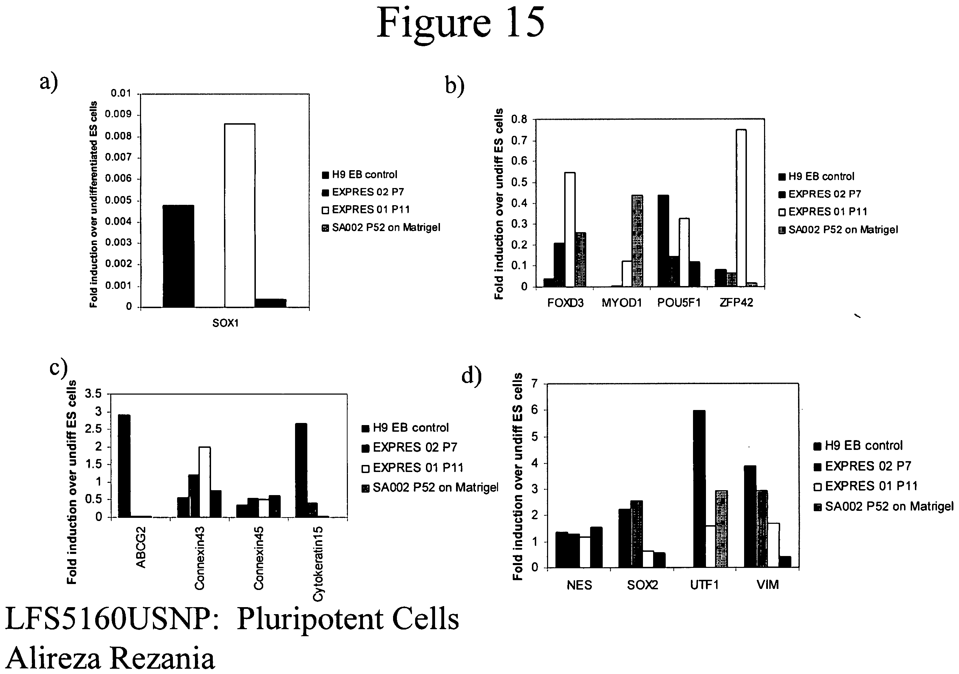

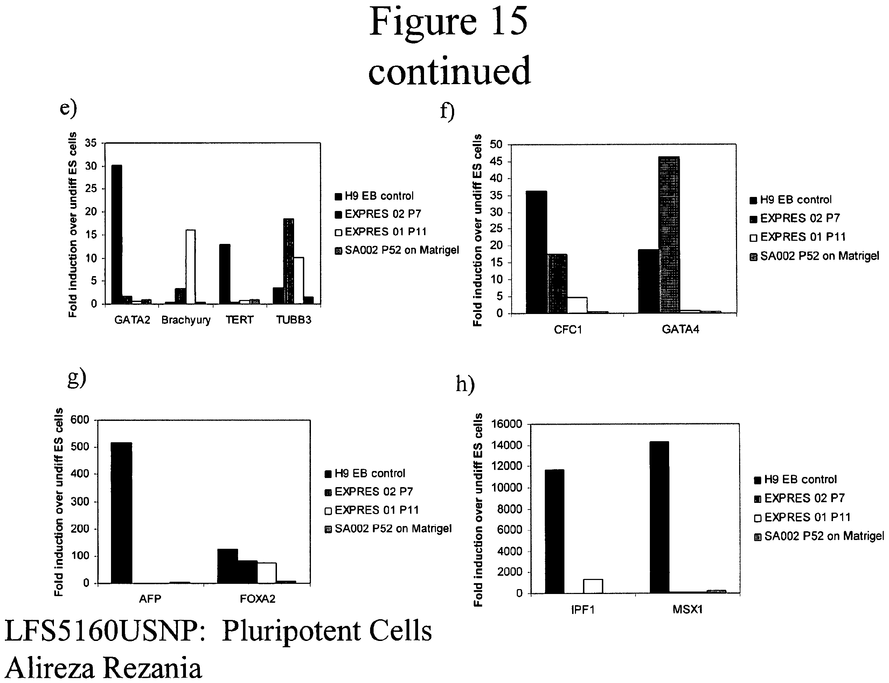

**Figure 15** shows gene expression as determined by real-time PCR for EXPRES 01 cells, EXPRES 02 cells, EB derived from H9 cells, SA002 cultured on MATRIGEL in MEF-CM, and undifferentiated H9 cells cultured on MATRIGEL in MEFCM. All the expression levels are normalized to undifferentiated H9 cells. Panel a) shows SOX-1 expression, panel b) shows FOXD3, MYOD1, POU5F1, and ZFP42 expression, panel c) shows ABCG2, Connexin 43, Connexin 45, and cytokeratin 15 expression, panel d) shows nestin, SOX-2, UTF1, and vimentin, panel e) shows GATA-2, Brachyury, TERT, and tubulin-beta III expression, panel f) shows CFC1, and GATA-4 expression, Panel g) shows AFP and FOXA2 expression, and Panel h) shows IPF1A and MSX1 expression.

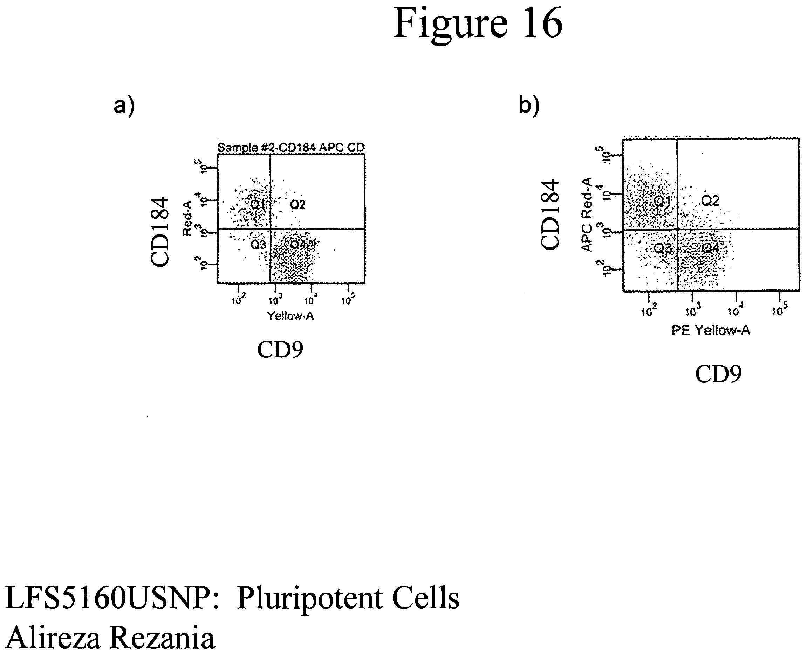

**Figure 16** shows the expression, as determined by FACS of CXCR4 (Y-axis) and CD9 (x-axis) in a) EXPRES 01 cells passage 5 cells cultured on tissue culture polystyrene in growth media and then switched to DMEM-F12 + 0.5% FBS + 100 ng/ml of activin-A and 20 ng/ml of WNT3A for 2 days followed by additional 2 days in DMEM-F12 + 2% FBS + 100 ng/ml of activin-A, b) EXPRES 02 cells passage 4 cells cultured on tissue culture polystyrene in growth media and then switched to DMEM-F12 + 0.5% FBS + 100 ng/ml of activin-A and 20 ng/ml of WNT3A for 2 days followed by additional 2 days in DMEM-F12 + 2% FBS + 100 ng/ml of activin-A.

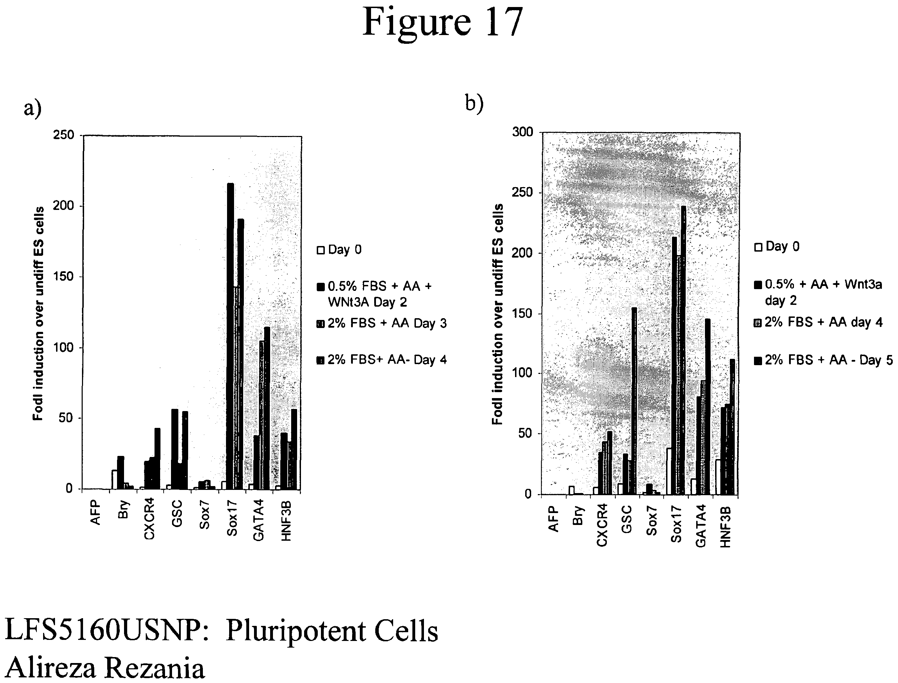

**Figure 17** shows gene expression as determined by real-time PCR in a) EXPRES 01 cells and b) EXPRES 02 cells treated with low serum plus AA + WNT3a.

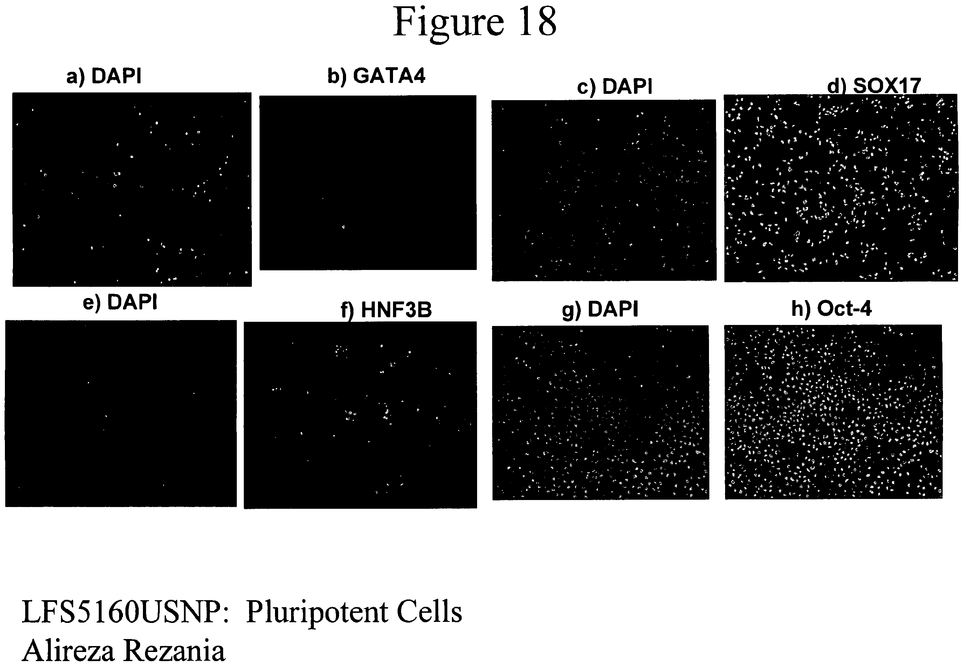

**Figure 18** shows immuno fluorescent images of EXPRES 01 cells at passage 5 cultured in 2% FBS + DMEM-F12 + 100 ng/ml of AA + 20 ng/ml of WNT-3A + 50 ng/ml of IGF-I and then switched to DMEM-F12 + 0.5% FBS + 100 ng/ml of activin-A and 20 ng/ml of WNT3A for 2 days, followed by additional 2 days in DMEM-F12 + 2% FBS + 100 ng/ml of activin-A. Panel a) DAPI image for panel b, panel b) GATA-4, panel c) DAPI image for panel d, panel d) SOX-17, panel e) DAPI image for panel f, panel f) HNF-3 beta, panel g) DAPI image for panel h, and panel h) OCT-4.

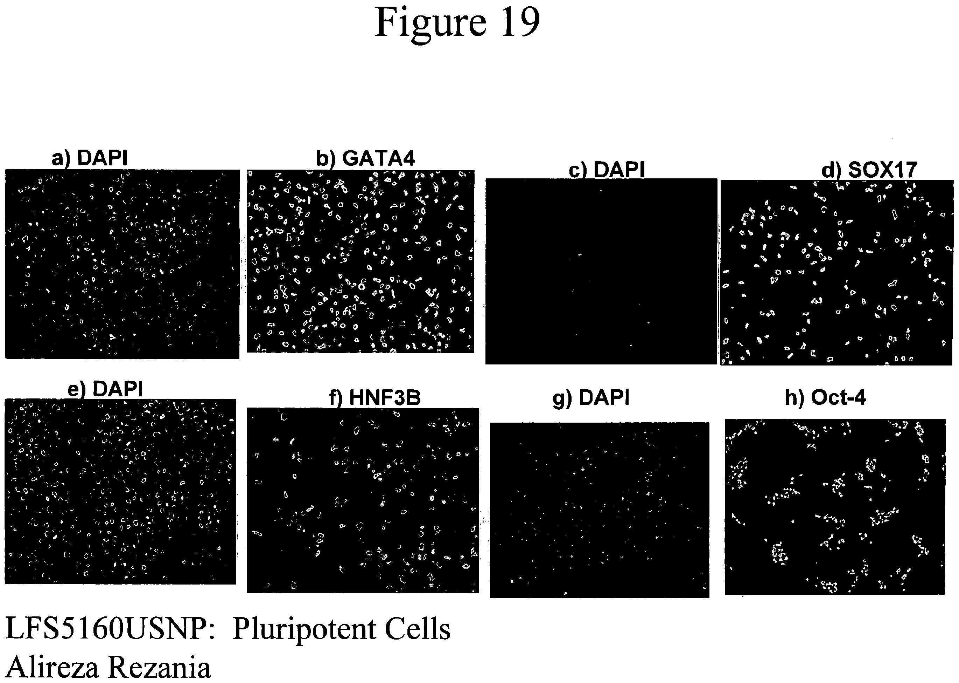

**Figure 19** shows immuno fluorescent images of EXPRES 02 cells at passage 4 cultured in 2% FBS + DMEM-F12 + 100 ng/ml of AA + 20 ng/ml of WNT-3A and then switched to DMEM-F12 + 0.5% FBS + 100 ng/ml of activin-A and 20 ng/ml of WNT3A for 2 days followed by additional 2 days in DMEM-F12 + 2% FBS + 100 ng/ml of activin-A. Panel a) DAPI image for panel b, panel b) GATA-4, panel c) DAPI image for panel d, panel d) SOX-17, panel e) DAPI image for panel f, panel f) HNF-3 beta, panel g) DAPI image for panel h, and panel h) OCT-4.

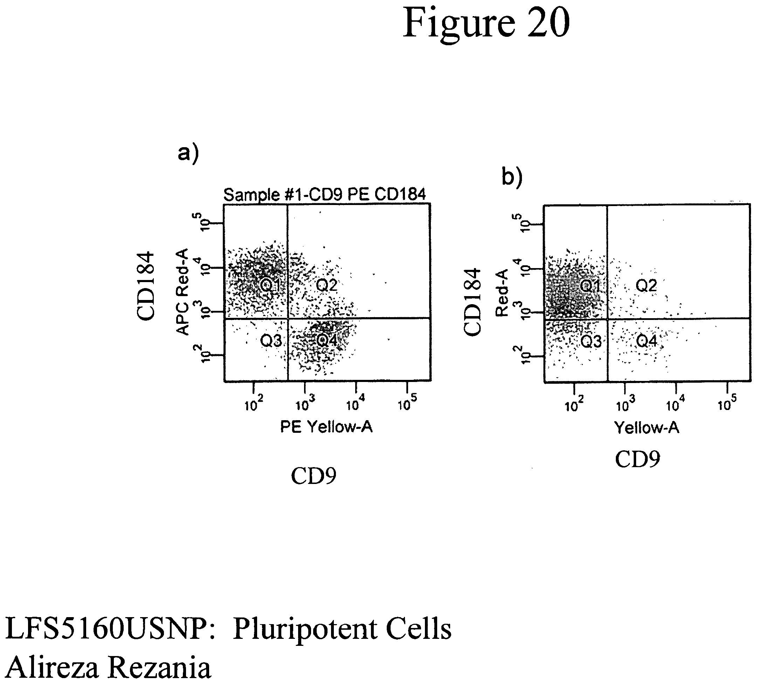

**Figure 20** shows protein expression as determined by FACS of CXCR4 (Y-axis) and CD9 (x-axis) for a) EXPRES 01 cells at passage 19 cells and b) EXPRES 02 cells at passage 14 cells cultured on tissue culture polystyrene in growth media and then switched to DMEM-F12 + 0.5% FBS + 100 ng/ml of activin-A + 100 nM GSK-3B inhibitor IX, and 20 ng/ml of WNT3A for 4days.

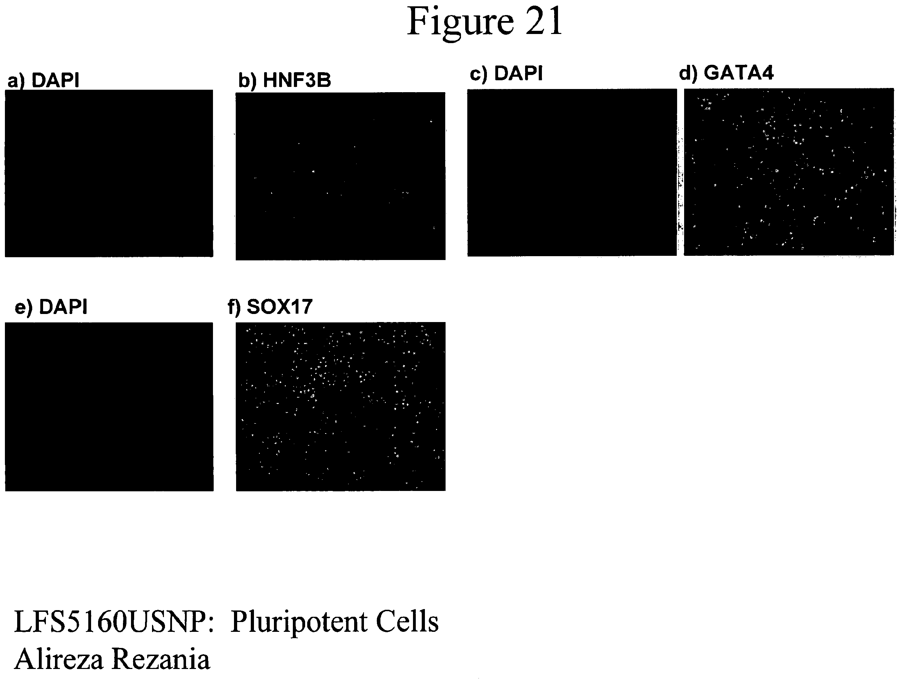

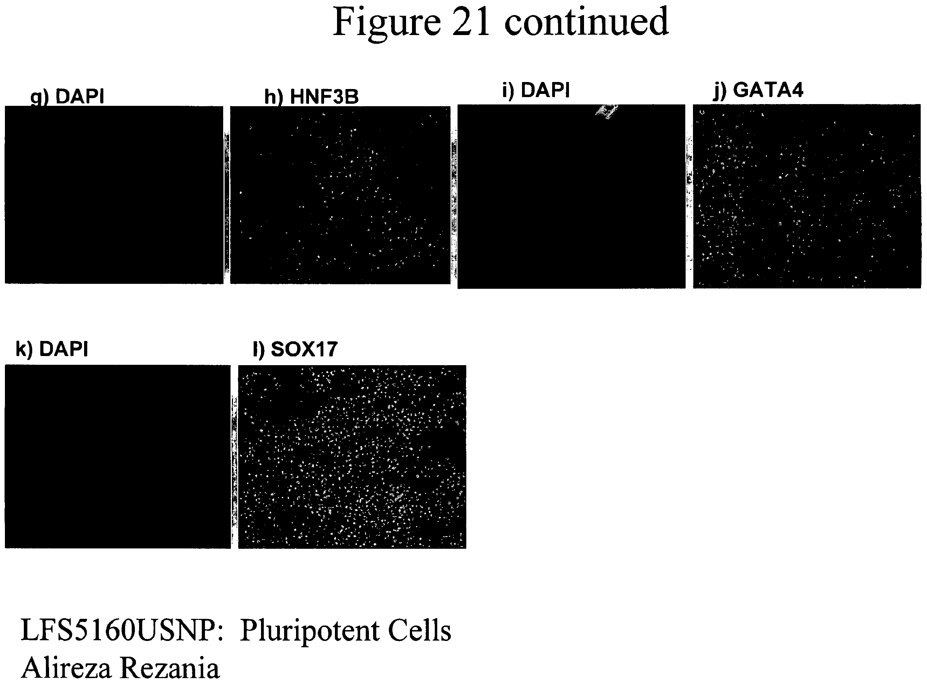

**Figure 21** shows immuno fluorescent images of EXPRES 01 cells at passage 19 cultured in 2% FBS + DMEM-F12 + 100 ng/ml of AA + 20 ng/ml of WNT-3A + 50 ng/ml of IGF-I and EXPRES 02 cells at passage 14 cultured in 2% FBS + DMEM-F12 + 100 ng/ml of AA + 20 ng/ml of WNT-3A and then switched to DMEM-F12 + 0.5% FBS + 100 ng/ml of activin-A + 100 nM GSK-3B inhibitor IX, and 20 ng/ml of WNT3A for 5days. Panel a) DAPI image for panel b, panel b) HNF-3 beta, panel c) DAPI image for panel d, panel d) GATA-4, panel e) DAPI image for panel f, panel f) SOX-17, panel g)DAPI image for panel h, panel h) HNF-3 beta, Panel i) DAPI image for panel j, panel j) GATA-4, panel k)DAPI image for panel 1, panel 1) SOX-17.

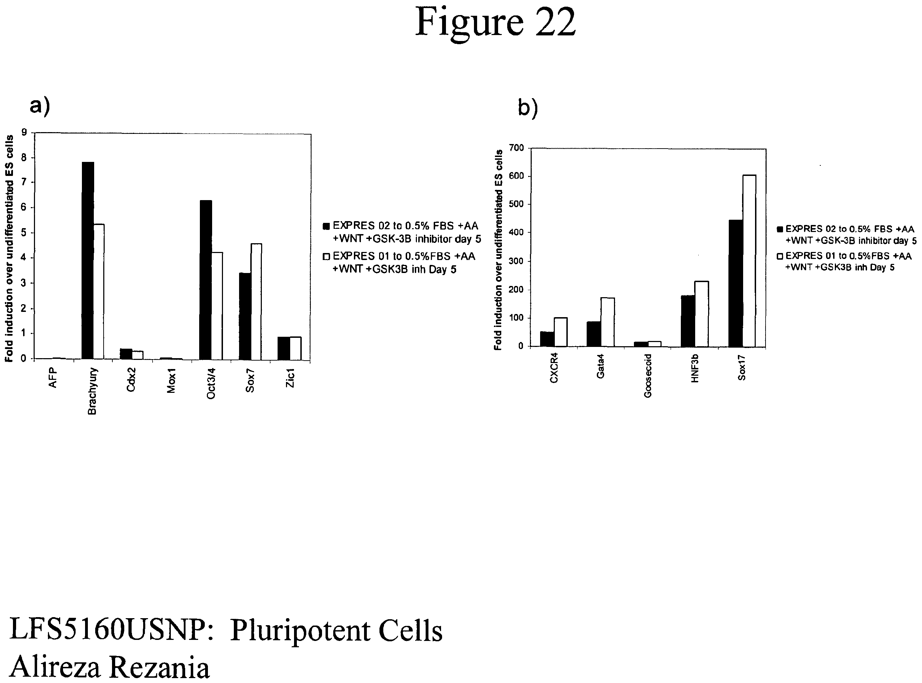

**Figure 22** shows gene expression as determined by real-time PCR data for a) EXPRES 01 and EXPRES 02 cells treated with low serum plus AA + WNT3a + GSK-3B IX inhibitor for 5 days. Panel a depicts expression of AFP, Brachyury, CDX2, Mox1, OCT3/4, SOX-7, and ZIC1 and panel b shows expression levels of CXCR4, GATA-4, Goosecoid, HNf3B, and SOX-17.

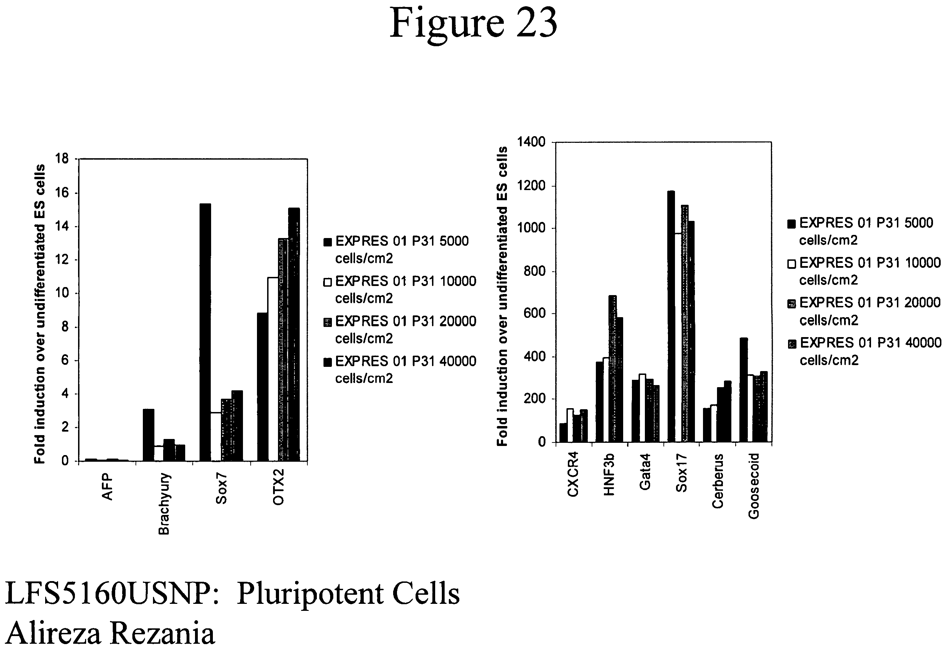

**Figure 23** shows gene expression as determined by real-time PCR for EXPRES 01 cells seeded at 5000-40000 cells/cm² on tissue culture polystyrene in growth media and then switched to DMEM-F12 + 0.5% FBS + 100 ng/ml AA + 20 ng/ml WNT3A + 100 nM GSK-3B inhibitor IX for four days under hypoxic conditions. Panel a depicts expression levels of AFP, Brachyury, SOX-7, and OTX2. Panel b depicts expression levels of CXCR4, HNF-3 beta, GATA-4, SOX-17, Cerb, and GSC.

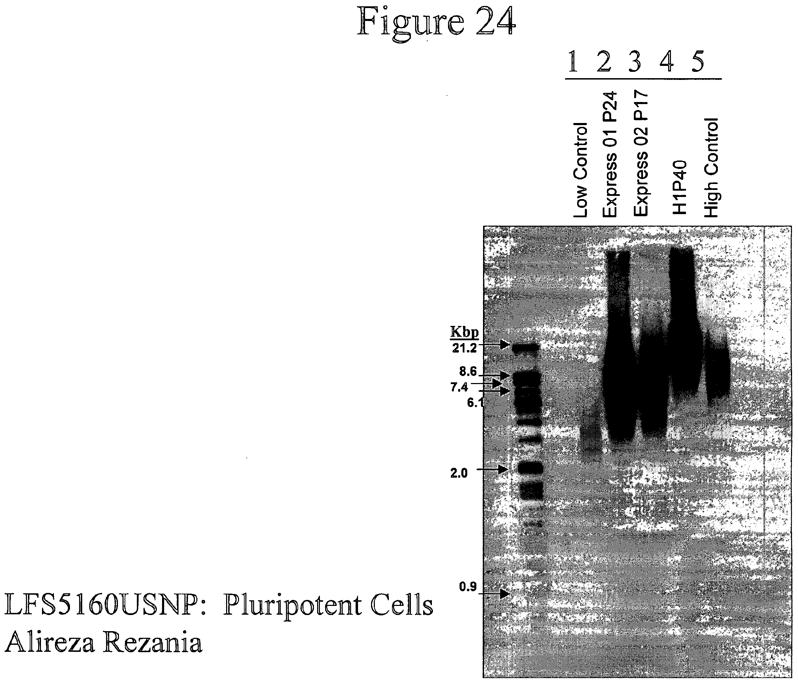

**Figure 24** shows the results of a telomere length assay in low telomere control cells (lane 1), EXPRES 01 cells at passage 24 (lane 2), EXPRES 02 cells at passage 17 (lane 3), undifferentiated cells from the human embryonic stem cell line H1 at passage 40 (lane 4), and high telomere length control cells (lane 5).

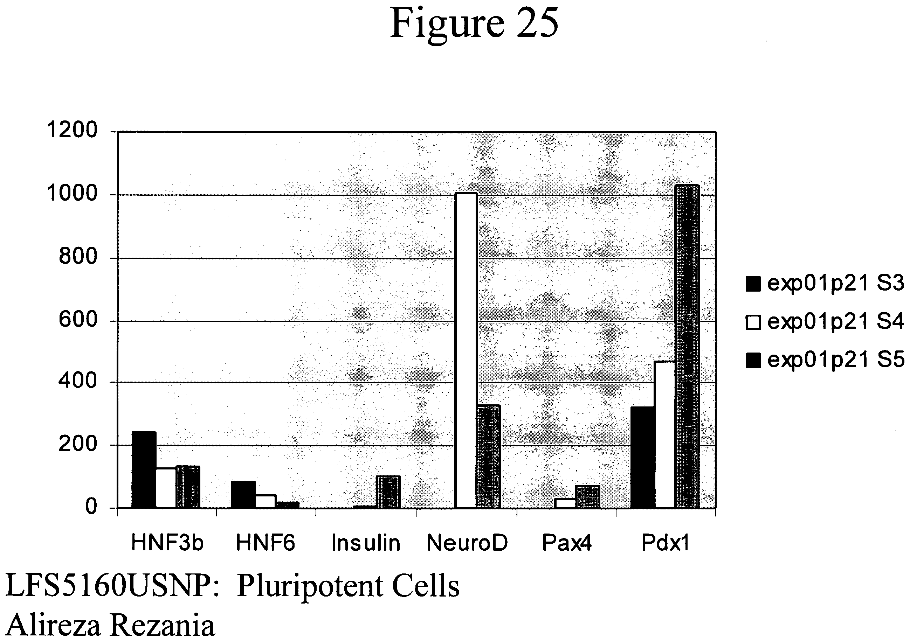

**Figure 25** shows gene expression as determined by real-time PCR data for EXPRES 01 cells at passage 21 that have been differentiated to foregut endoderm cells (S3), pancreatic endoderm cells (S4), and pancreatic endocrine cells (S5).

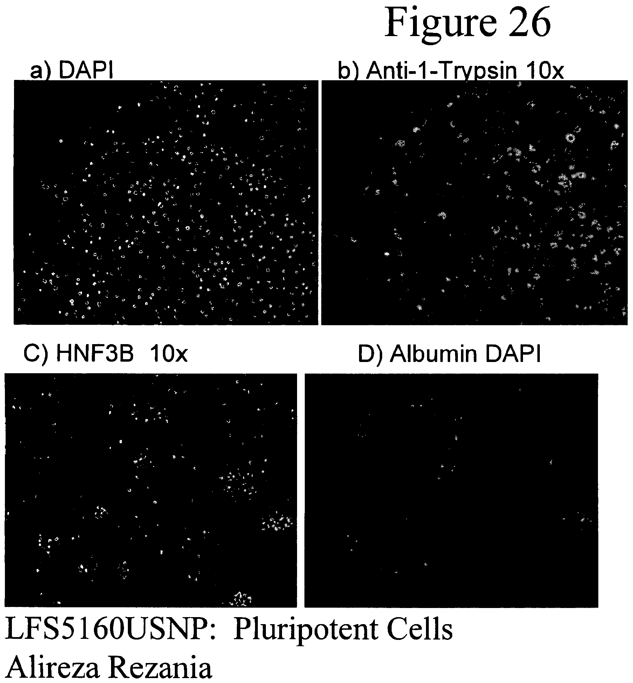

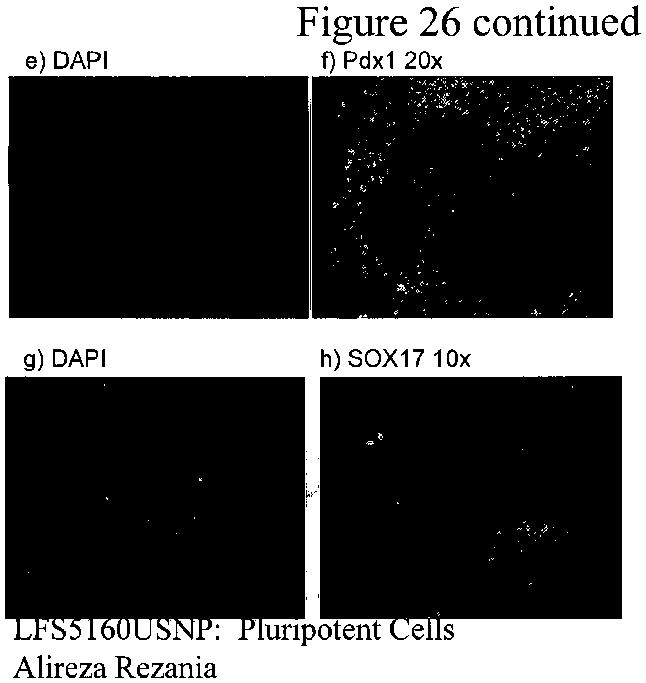

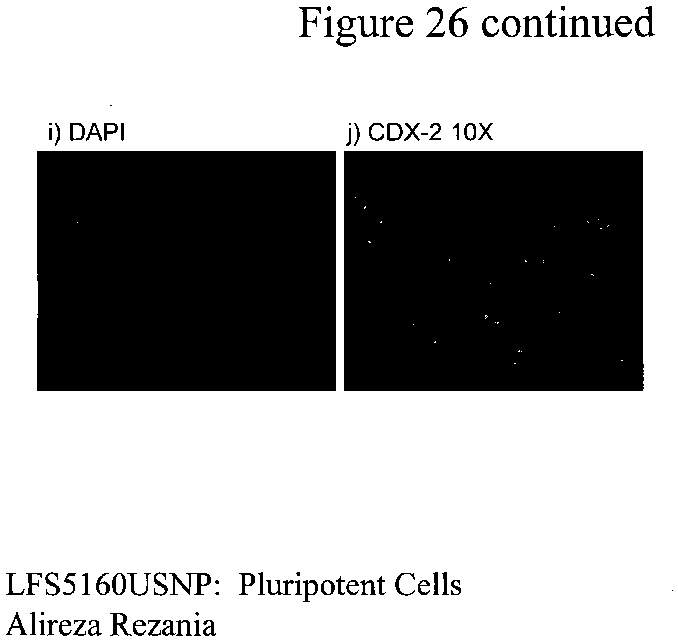

**Figure 26** shows immuno fluorescent images of EXPRES 01 at passage 35 cultured according to **Example 18.** Panel a) DAPI image for panel b, panel b) Anti-1 trypsin, panel c) HNF-3 beta in green Albumin in Red, panel d) Albumin in red and DAPI (blue), panel e) DAPI image for panel f, panel f) PDX-1, panel g) DAPI image for panel h, panel h) SOX-17, panel i) DAPI image for panel j, panel j) CDX-2.

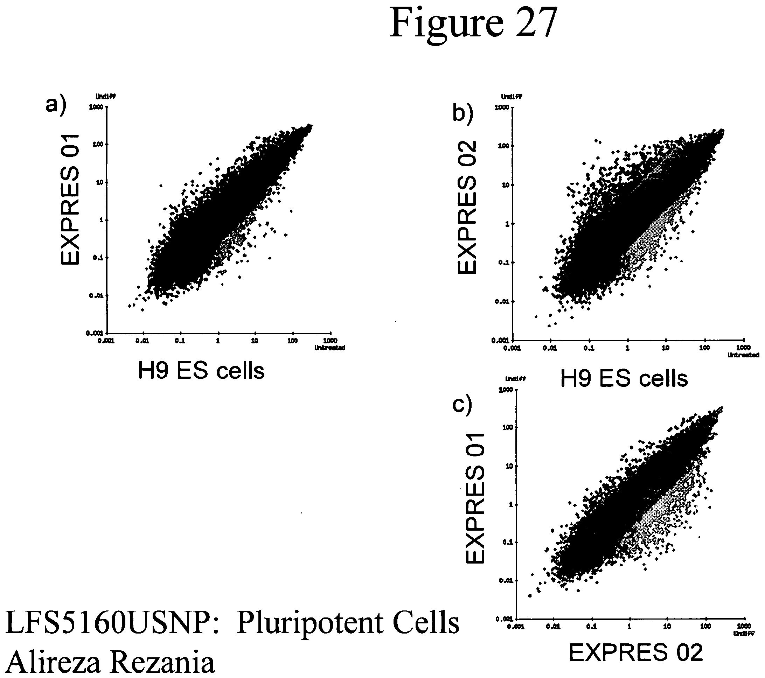

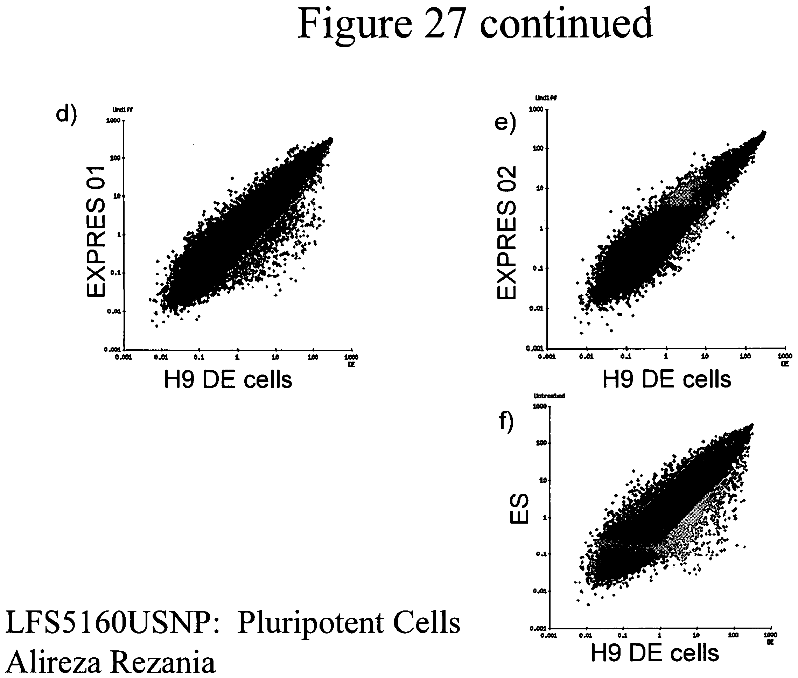

**Figure 27** shows scatter plots of microarray data comparing, panel a) EXPRES 01 cells (y-axis) to undifferentiated cells from the human embryonic stem cell line H9 (x-axis), panel b) EXPRES 02 cells (y-axis) to undifferentiated cells from the human embryonic stem cell line H9 (x-axis), panel c) EXPRES 01 cells (y-axis) to EXPRES 02 cells (x-axis), panel d) EXPRES 01 cells (y-axis) to cells from the human embryonic stem cell line H9 that have been differentiated into definitive endoderm (x-axis), panel e) EXPRES 02 cells (y-axis) to cells from the human embryonic stem cell line H9 that have been differentiated into definitive endoderm (x-axis), panel f) undifferentiated cells from the human embryonic stem cell line H9 (y-axis) to cells from the human embryonic stem cell line H9 that have been differentiated into definitive endoderm (x-axis).

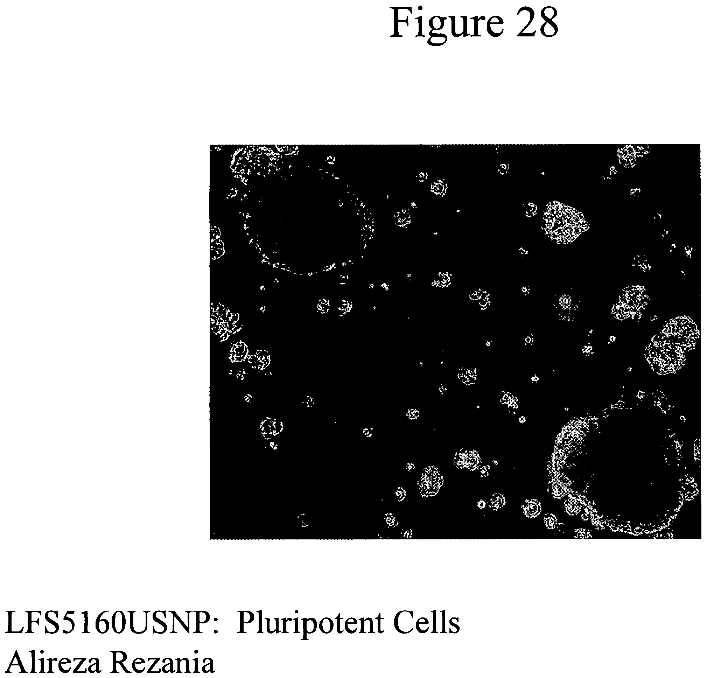

**Figure 28** shows the morphology of EB bodies formed by EXPRES 01 cells.

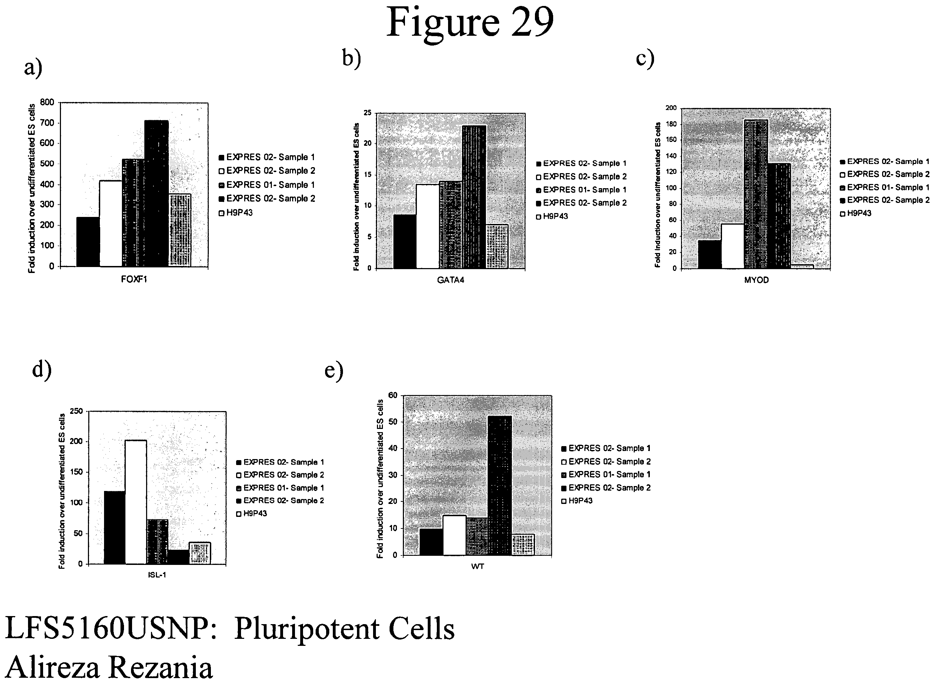

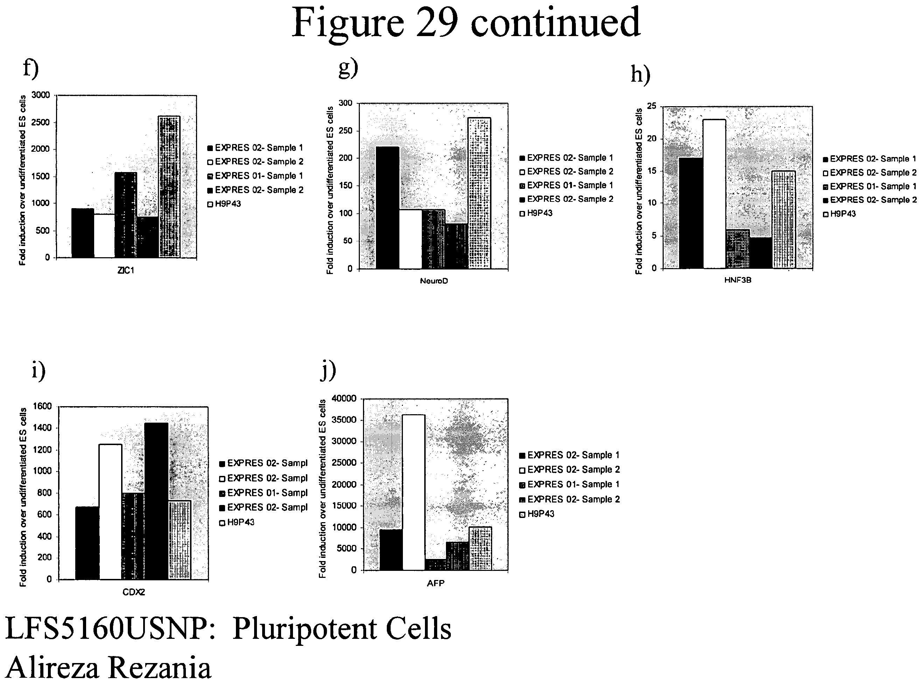

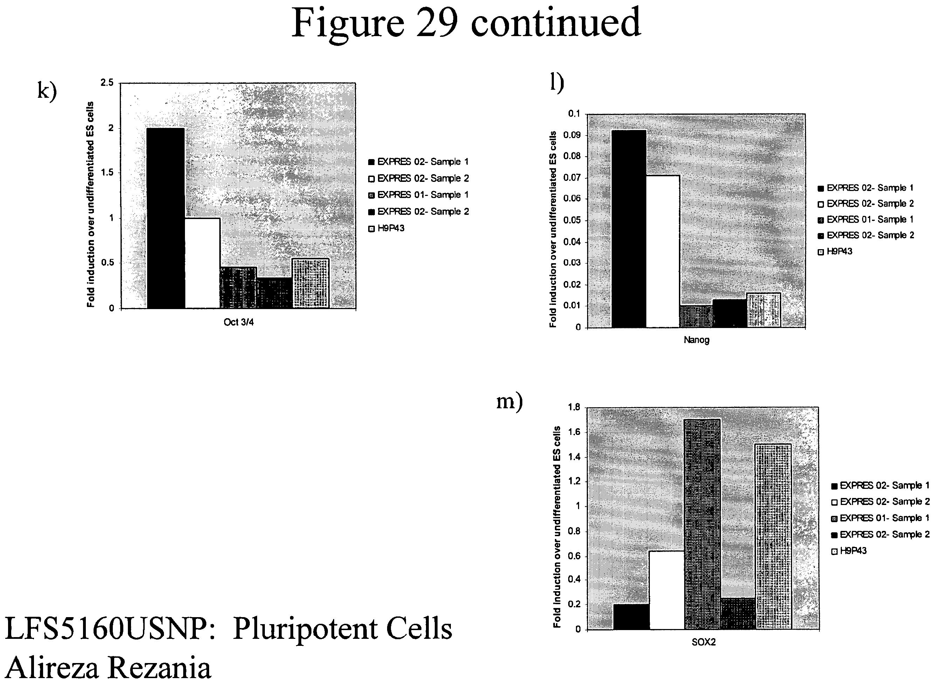

**Figure 29** shows gene expression as determined by real-time PCR for cells of the EXPRES 01 cell line, cells of the EXPRES 02 cell line, and cells from the human embryonic stem cell line H9 at passage 43, following five weeks of transplantation beneath the kidney capsule of NOD-SCID mice. Panels a-e, show mesoderm markers. Panels f & g show ectoderm markers. Panels h & i show endoderm markers. Panel j shows extra embryonic endoderm markers. Panels k-m show pluripotency markers.

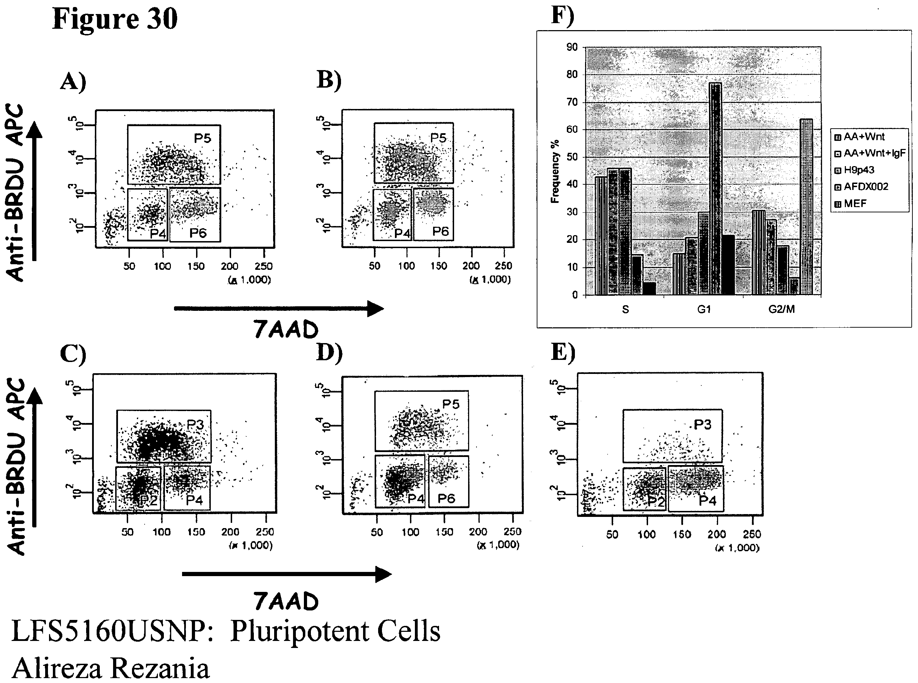

**Figure 30** shows the proliferation and cell cycle status of EXPRES 03 cells, as determined by BRDU incorporation. EXPRES 03 cells were cultured in 2% FBS/DMEM/F12, supplemented with, panel a) activin-A (100 ng/ml) and wnt3a (20ng/ml), panel b) Activin-A (100 ng/ml) and wnt3a (20ng/ml) and IGF (50nh/ml). Other cells shown include, panel c) hES cells (H9p43), panel d) Amniotic Fluid Cells (AFDX002) and panel e) mitomycin treated MEF cells. Panel f) shows the frequency of cells in S-phase, G1 and G2/M-phases of cell cycle for different cell populations studied.

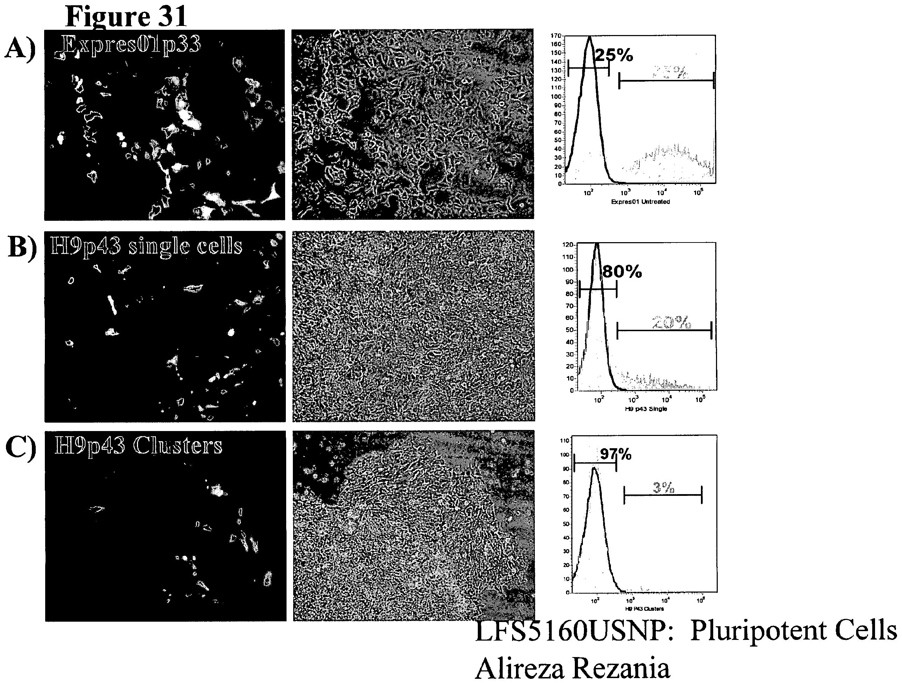

**Figure 31** shows the transfection efficiency and expression of EGFP in EXPRES 01 cells and human embryonic stem cells plated either as single cell dispersions or cell clusters. Cells were analyzed 24 hrs later by fluorescence microscopy and flow cytometry. Panel A) shows data obtained from EXPRES 01 cells. Panel B) shows data obtained from single cell dispersions of human embryonic stem cells and Panel C) shows data obtained from cell clusters of human embryonic stem cells.

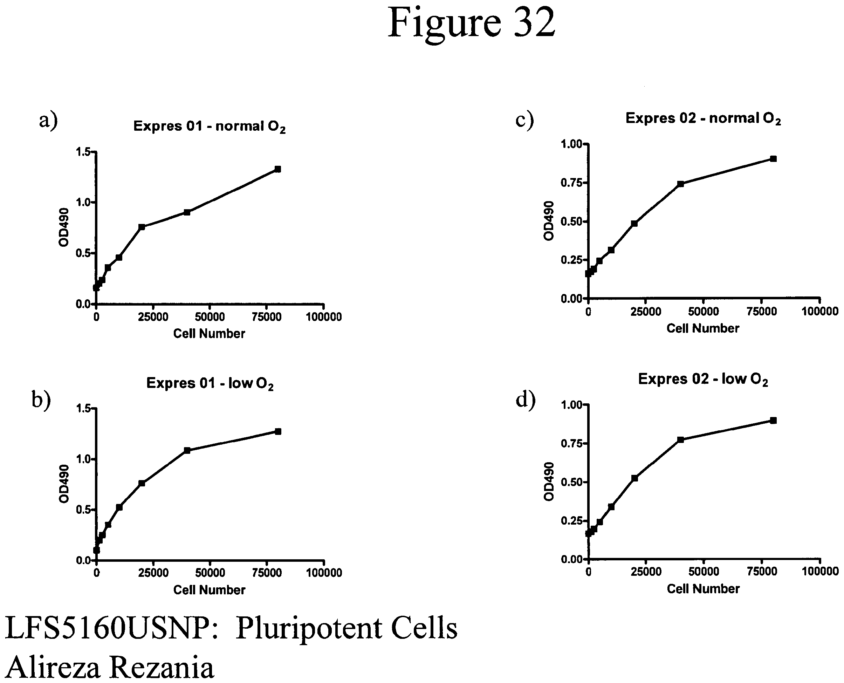

**Figure 32** shows average OD readings representing dehydrogenase enzyme activity versus cell number as measured by MTS assay for a) EXPRES 01 cells cultured in atmospheric oxygen (approximately 21%), b) EXPRES 01 cells cultured in 3% O2, c) EXPRES 02 cells cultured in atmospheric oxygen (approximately 21%), d) EXPRES 02 cells cultured in 3% O2 conditions.

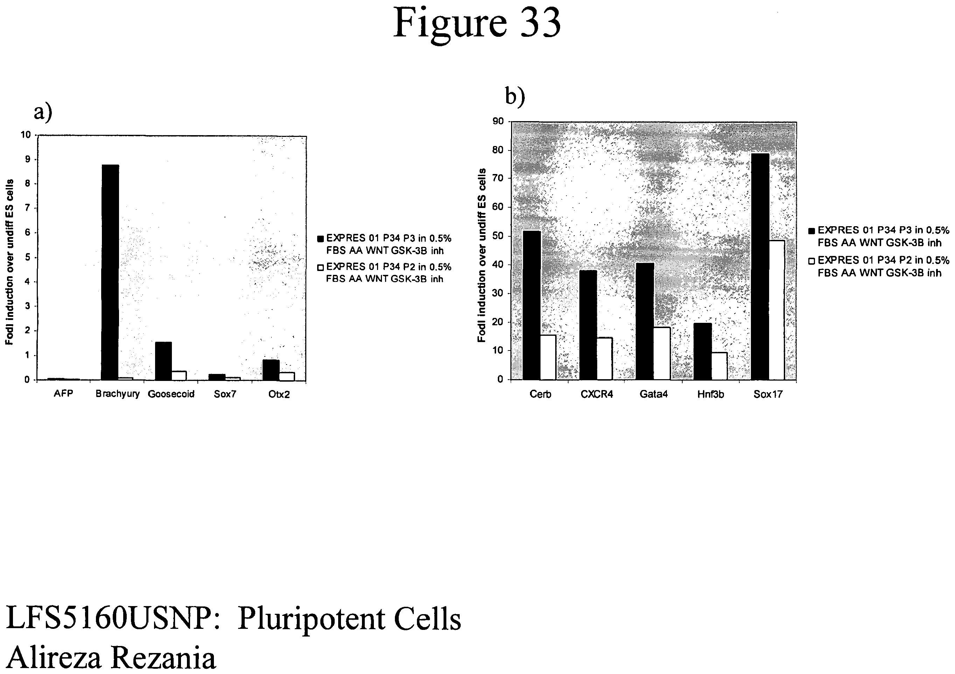

**Figure 33** shows gene expression as determined by real-time PCR for EXPRES 01 P27 cells seeded at 10000 cells/cm² on tissue culture polystyrene in DMEMF12 + 0.5% FBS + 100 ng/ml AA + 20 ng/ml WNT3A + 100 nM GSK-3B inhibitor IX.. Panel a depicts expression levels of AFP, Brachyury, SOX-7, and OTX2. Panel b depicts expression levels of CXCR4, HNF-3 beta, GATA-4, SOX-17, Cer1, and GSC.

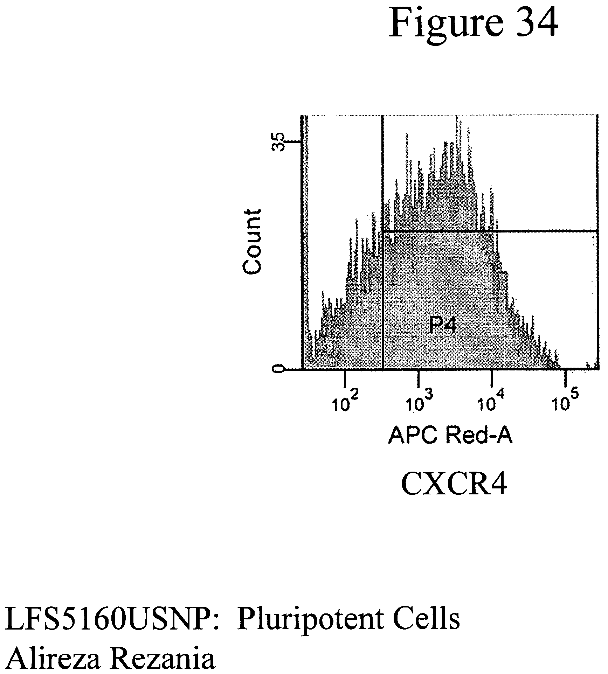

**Figure 34** shows protein expression of CXCR4 (Y-axis) and CD9 (x-axis), as determined by FACS for EXPRES 01 P27 cells cultured on tissue culture polystyrene in DMEM-F12 + 0.5% FBS + 100 ng/ml AA + 20 ng/ml WNT3A + 100 nM GSK-3B inhibitor IX for three passages.

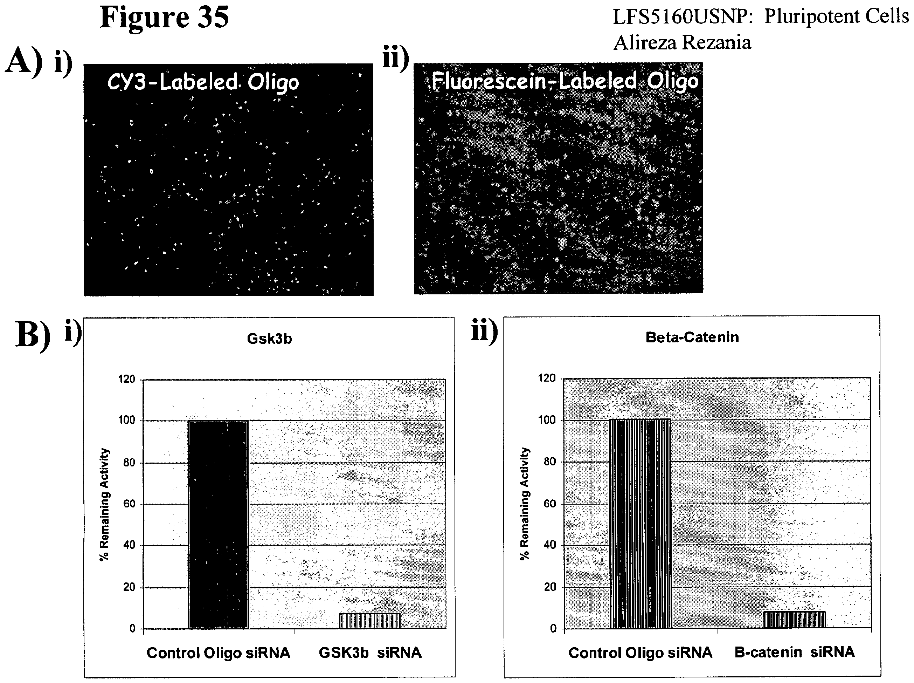

**Figure 35** shows the effects of siRNA transfection on the expression of GSK-3B and beta-catenin. EXPRES cells were analyzed by fluorescence microscopy and quantitative RT-PCR methods. A) Fluorescence microscopy of cells transfected with i) CY3 labeled siRNA and ii) Fluorescein labeled siRNA. B) Target gene knockdown expressed as % remaining activity in cells transfected with i) GSK3b and ii) Beta-catenin siRNA oligo sequences.

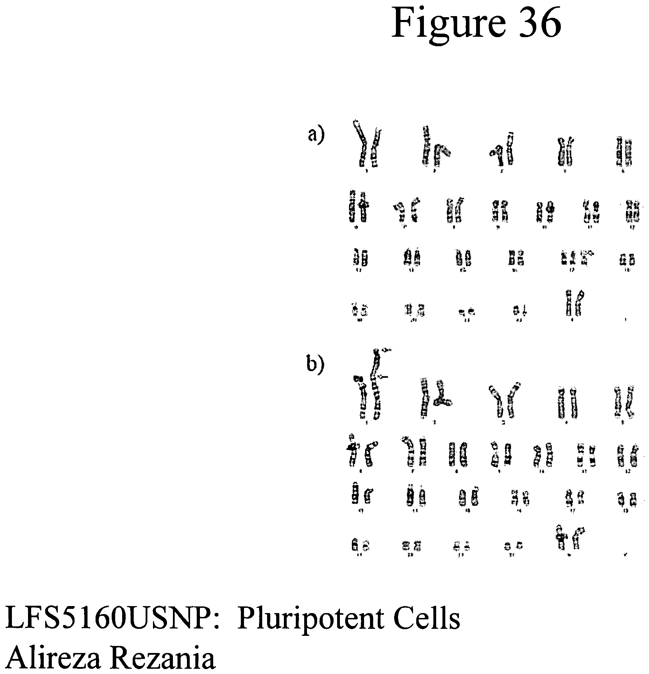

**Figure 36** shows the karyotype of two cell lines derived by methods of the present invention. Panel a: EXPRES 01 cell line. Panel b: EXPRES 02 cell line.

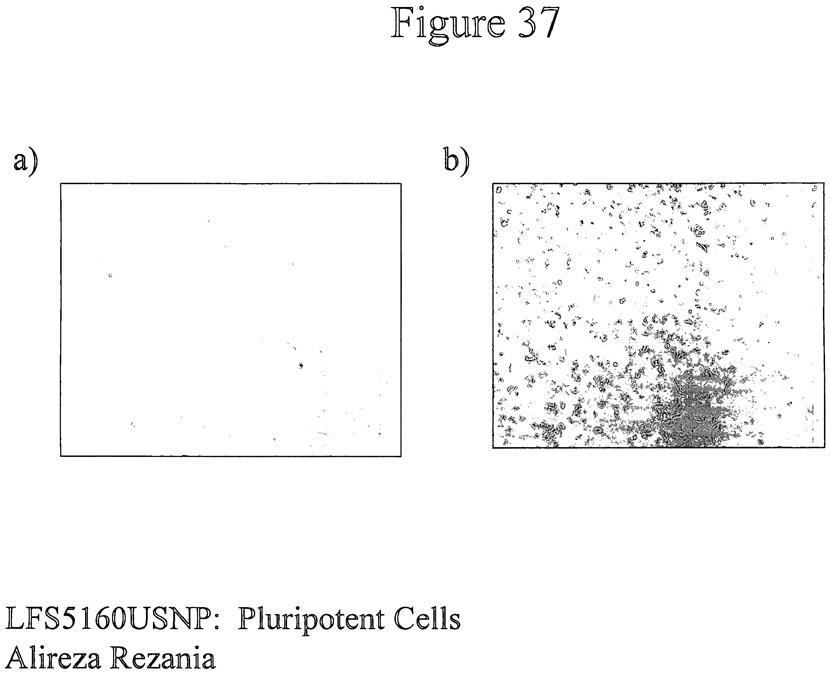

**Figure 37** shows the morphology of EXPRES 15 cells at passage 0 after 24h culture in a) 2% FBS + DM-F12 + 100 ng/ml of activin-A+ 20 ng/ml of WNT-3A + 50 ng/ml IGF or b) 2% FBS + DM-F12 + 100 ng/ml of Activin-A+ 20 ng/ml of WNT-3A + 50 ng/ml IGF + 10 µM of the Rho kinase inhibitor Y-27632.

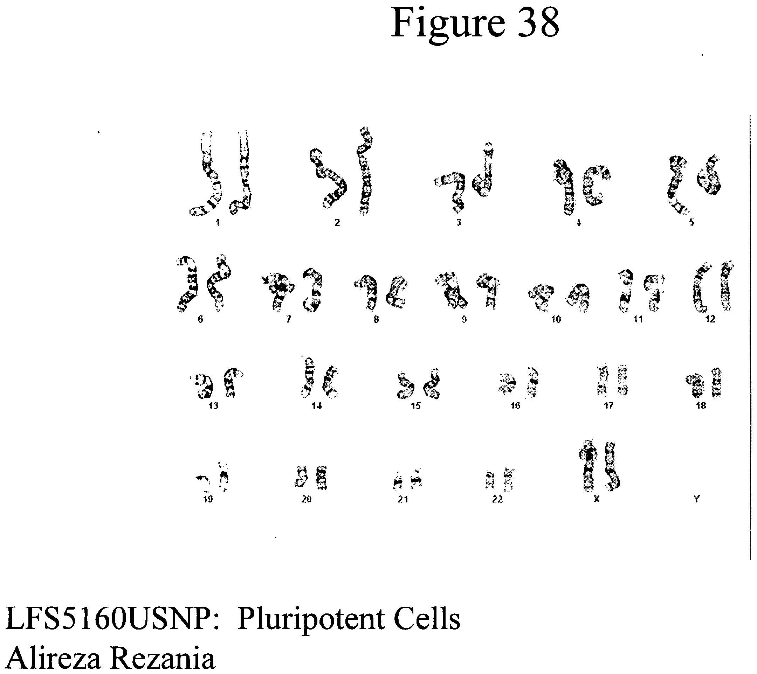

**Figure 38** shows the karyotype of EXPRES 15 cells cultured in 2% FBS + DM-F12 + 100 ng/ml of Activin-A+ 20 ng/ml of WNT-3A + 50 ng/ml IGF + 10 µM of the Rho kinase inhibitor Y-27632 for 12 passages.

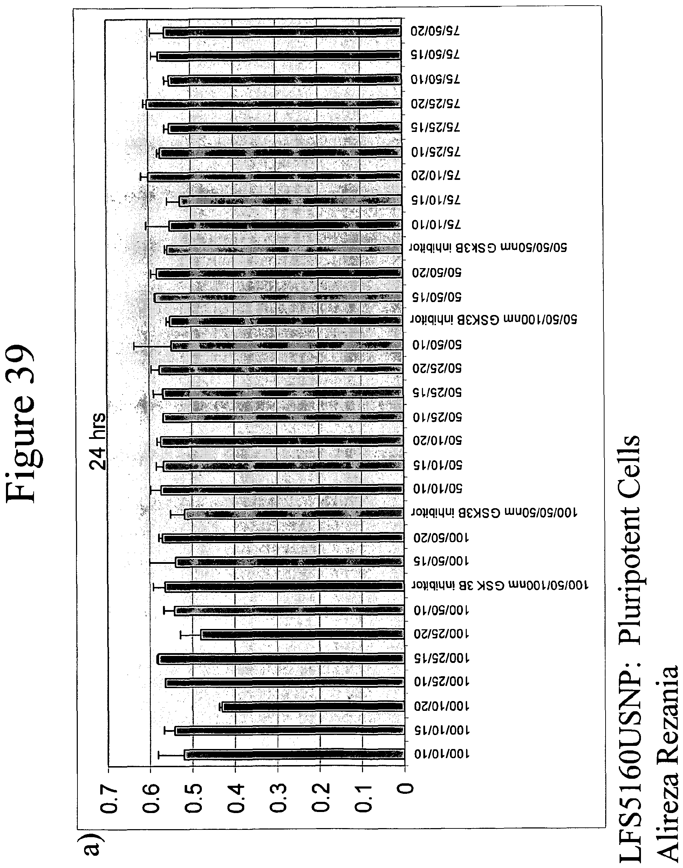

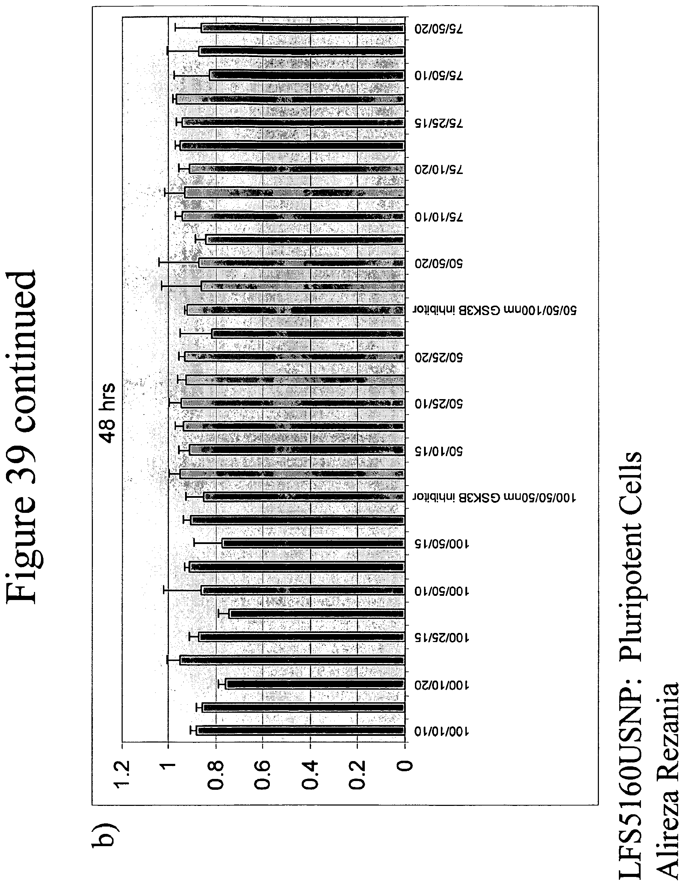

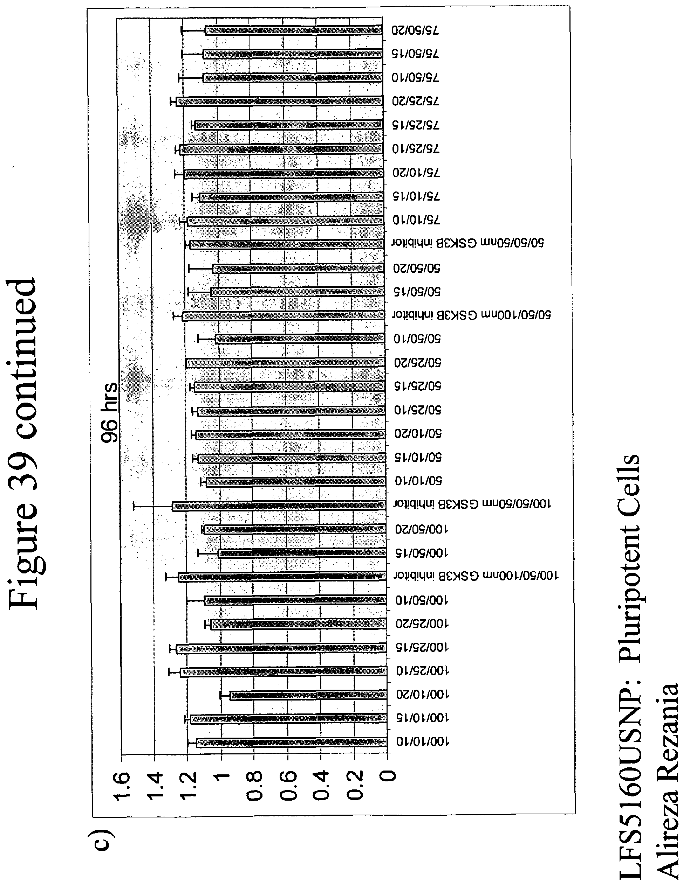

**Figure 39** shows the proliferation as determined by A₄₉₀ of EXPRES 11 cells cultured in basal media supplemented with IGF, activin-A, Wnt3A, and GSK inhibitor IX at the concentrations indicated at 24 h (panel a), 48 h (panel b), and 96 h (panel c).

### DETAILED DESCRIPTION

For clarity of disclosure, and not by way of limitation, the detailed description of the invention is divided into the following subsections that describe or illustrate certain features, embodiments, or applications of the present invention.

### Definitions

Stem cells are undifferentiated cells defined by their ability at the single cell level to both self-renew and differentiate to produce progeny cells, including self renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation and to contribute substantially to most, if not all, tissues following injection into blastocysts.

Stem cells are classified by their developmental potential as: (1) totipotent, meaning able to give rise to all embryonic and extraembryonic cell types; (2) pluripotent, meaning able to give rise to all embryonic cell types; (3) multipotent, meaning able to give rise to a subset of cell lineages, but all within a particular tissue, organ, or physiological system (for example, hematopoietic stem cells (HSC) can produce progeny that include HSC (self- renewal), blood cell restricted oligopotent progenitors and all cell types and elements (e.g., platelets) that are normal components of the blood); (4) oligopotent, meaning able to give rise to a more restricted subset of cell lineages than multipotent stem cells; and (5) unipotent, meaning able to give rise to a single cell lineage (e.g. , spermatogenic stem cells).

Differentiation is the process by which an unspecialized ("uncommitted") or less specialized cell acquires the features of a specialized cell such as, for example, a nerve cell or a muscle cell. A differentiated or differentiation-induced cell is one that has taken on a more specialized ("committed") position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. De-differentiation refers to the process by which a cell reverts to a less specialized (or committed) position within the lineage of a cell. As used herein, the lineage of a cell defines the heredity of the cell, i.e., which cells it came from and what cells it can give rise to. The lineage of a cell places the cell within a hereditary scheme of development and differentiation. A lineage-specific marker refers to a characteristic specifically associated with the phenotype of cells of a lineage of interest and can be used to assess the differentiation of an uncommitted cell to the lineage of interest.

"AFP" or "alpha-fetoprotein protein" as used herein, refers to an antigen produced at the onset of liver development. AFP may also be expressed in extraembryonic cells.

"Albumin" is a soluble monomeric protein that makes up about half of all serum proteins in adults.

"β-cell lineage" refer to cells with positive gene expression for the transcription factor PDX-1 and at least one of the following transcription factors: NGN-3, Nkx2.2, Nkx6.1, NeuroD, Isl-1, HNF-3 beta, MAFA, Pax4, and Pax6. Cells expressing markers characteristic of the β cell lineage include β cells.

"Brachyury", as used herein, is a T-box gene family member. It is the marker for primitive streak and mesoderm cells.

"Cells expressing markers characteristic of the definitive endoderm lineage" as used herein refer to cells expressing at least one of the following markers: SOX-17, GATA-4, HNF-3 beta, GSC, Cer1, Nodal, FGF8, Brachyury, Mix-like homeobox protein, FGF4 CD48, eomesodermin (EOMES), DKK4, FGF17, GATA-6, CXCR4, C-Kit, CD99, or OTX2. Cells expressing markers characteristic of the definitive endoderm lineage include primitive streak precursor cells, primitive streak cells, mesendoderm cells and definitive endoderm cells.

"c-Kit" and "CD117" both refer to a cell surface receptor tyrosine kinase having a sequence disclosed in Genbank Accession No. X06182, or a naturally occurring variant sequence thereof (e.g., allelic variant).

"CD99" as used herein refers to the protein encoded by the gene wit the accession number NM_002414.

"Cells expressing markers characteristic of the pancreatic endoderm lineage" as used herein refer to cells expressing at least one of the following markers: PDX-1, HNF-1beta, PTF-1 alpha, HNF-6, or HB9. Cells expressing markers characteristic of the pancreatic endoderm lineage include pancreatic endoderm cells.

"Cells expressing markers characteristic of the pancreatic endocrine lineage" as used herein refer to cells expressing at least one of the following markers: NGN-3, NeuroD, Islet-1, PDX-1, NKX6.1, Pax-4, Ngn-3, or PTF-1 alpha. Cells expressing markers characteristic of the pancreatic endocrine lineage include pancreatic endocrine cells, pancreatic hormone expressing cells, and pancreatic hormone secreting cells, and cells of the β-cell lineage.

"Cer1" or "Cerebrus" as used herein is a member of the cysteine knot superfamily of proteins.

"CXCR4" as used herein refers to the stromal cell-derived factor 1 (SDF-1) receptor, also known as "LESTR" or "fusin". In the gastrulating mouse embryo, CXCR4 is expressed in the definitive endoderm and mesoderm but not in extraembryonic endoderm.

"Definitive endoderm" as used herein refers to cells which bear the characteristics of cells arising from the epiblast during gastrulation and which form the gastrointestinal tract and its derivatives. Definitive endoderm cells express the following markers: HNF-3 beta, GATA-4, SOX-17, Cerberus, OTX2, goosecoid, C-Kit, CD99, and Mixl1.

"Extraembryonic endoderm" as used herein refers to a population of cells expressing at least one of the following markers: SOX-7, AFP, and SPARC.

"FGF-2", "FGF-4" "FGF-8" "FGF-10", and "FGF-17" as used herein, are members of the fibroblast growth factor family.

"GATA-4" and "GATA-6" are members of the GATA transcription factor family. This family of transcription factors is induced by TGF-β signaling, and contribute to the maintenance of early endoderm markers.

"GLUT-2", as used herein, refers to the glucose transporter molecule that is expressed in numerous fetal and adult tissues, including pancreas, liver, intestine, brain, and kidney.

"Goosecoid" or "GSC" as used herein, refers to a homeodomain transcription factor expressed in the dorsal lip of the blastopore.

"HB9" as used herein, refers to the homeobox gene 9.

"HNF-1 alpha", "HNF-1 beta", "HNF-3 beta", and "HNF-6" belong to the hepatic nuclear factor family of transcription factors, which is characterized by a highly conserved DNA binding domain and two short carboxy-terminal domains.

"Islet-1" or "Isl-1" as used herein is a member of the LIM/homeodomain family of transcription factors, and is expressed in the developing pancreas.

"MafA" as used herein is a transcription factor expressed in the pancreas, and controls the expression of genes involved in insulin biosynthesis and secretion.

"Markers" as used herein, are nucleic acid or polypeptide molecules that are differentially expressed in a cell of interest. In this context, differential expression means an increased level for a positive marker and a decreased level for a negative marker. The detectable level of the marker nucleic acid or polypeptide is sufficiently higher or lower in the cells of interest compared to other cells, such that the cell of interest can be identified and distinguished from other cells using any of a variety of methods known in the art.

"Mesendoderm cell" as used herein refers to a cell expressing at least one of the following markers: CD48, eomesodermin (EOMES), SOX-17, DKK4, HNF-3 beta, GSC, FGF17, GATA-6.

"Mixl1" as used herein refers to a homeobox gene, which is marker for the cells in the primitive streak, mesoderm, and endoderm.

"NeuroD" as used herein is basic helix-loop-helix (bHLH) transcription factor implicated in neurogenesis.

"NGN-3" as used herein, is a member of the neurogenin family of basic loophelix-loop transcription factors.

"Nkx-2.2" and "Nkx-6.1" as used herein are members of the Nkx transcription factor family.

"Nodal" as used herein, is a member of the TGF beta superfamily of proteins.

"Oct-4" is a member of the POU-domain transcription factor and is widely regarded as a hallmark of pluripotent stem cells. The relationship of Oct-4 to pluripotent stem cells is indicated by its tightly restricted expression to undifferentiated pluripotent stem cells. Upon differentiation to somatic lineages, the expression of Oct-4 disappears rapidly.

"Pancreatic endocrine cell", or "pancreatic hormone expressing cell" as used herein refers to a cell capable of expressing at least one of the following hormones: insulin, glucagon, somatostatin, and pancreatic polypeptide.

"Pancreatic hormone secreting cell" as used herein refers to a cell capable of secreting at least one of the following hormones: insulin, glucagon, somatostatin, and pancreatic polypeptide.

"Pax-4" and "Pax-6" as used herein are pancreatic β cell specific transcription factors that are implicated in islet development.

"PDX-1" as used herein refers to a homeodomain transcription factor implicated in pancreas development.

"Pre-primitive streak cell" as used herein refers to a cell expressing at least one of the following markers: Nodal, or FGF8

"Primitive streak cell" as used herein refers to a cell expressing at least one of the following markers: Brachyury, Mix-like homeobox protein, or FGF4.

"PTF-1 alpha" as used herein refers to a basic helix-loop-helix protein of 48 kD that is a sequence-specific DNA-binding subunit of the trimeric pancreas transcription factor-1 (PTF1).

"SOX-1", "SOX-2", "SOX-7", and "SOX-17"as used herein, are a members of the SOX transcription factor family, and are implicated in embryogenesis.

"SPARC" as used herein is also known as "secreted protein acidic and rich in cysteine".

"SSEA-1" (Stage Specific Embryonic Antigen-1) is a glycolipid surface antigen present on the surface of murine teratocarcinoma stem cells (EC), murine and human embryonic germ cells (EG), and murine embryonic stem cells (ES).

"SSEA-3" (Stage Specific Embryonic Antigen-3) is a glycolipid surface antigen present on the surface of human teratocarcinoma stem cells (EC), human embryonic germ cells (EG), and human embryonic stem cells (ES).

"SSEA-4" (Stage Specific Embryonic Antigen-4) is a glycolipid surface antigen present on the surface of human teratocarcinoma stem cells (EC), human embryonic germ cells (EG), and human embryonic stem cells (ES).

"TRA1-60" is a keratin sulfate related antigen that is expressed on the surface of human teratocarcinoma stem cells (EC), human embryonic germ cells (EG), and human embryonic stem cells (ES).

"TRA1-81" is a keratin sulfate related antigen that is expressed on the surface of human teratocarcinoma stem cells (EC), human embryonic germ cells (EG), and human embryonic stem cells (ES).

"TRA2-49" is an alkaline phosphatase isozyme expressed on the surface of human teratocarcinoma stem cells (EC) and human embryonic stem cells (ES).

"UTF-1" as used herein, refers a transcriptional co-activator expressed in pluripotent embryonic stem cells and extra-embryonic cells.

"Zic1" as used herein is a member of the Zic transcription factor family. Zic1 regulates the expression of neural and neural crest-specific genes and is expressed in the cells of the dorsal neural tube and the premigratory neural crest.

### Method to derive cells expressing pluripotency markers

The present disclosure provides a cell population with characteristics of human embryonic stem cells, that can be readily expanded in culture, in low serum, that requires no feeder cell line or a coating of complex matrix proteins, can be passaged in single cell suspension, can be transfected with a very high efficiency, and cultured under hypoxic conditions. This combination of unique attributes separates the cells described in the present disclosure from the prior art.

In one embodiment, the present disclosure provides a method for deriving a population of cells comprising cells expressing pluripotency markers, comprising the steps of:
a. Obtaining cells, and
b. Culturing the cells under hypoxic conditions, on a tissue culture substrate that is not pre-treated with a protein or an extracellular matrix prior to culturing the cells.

The cells may be human embryonic stem cells, or they may be cells expressing markers characteristic of the definitive endoderm lineage. The human embryonic stem cells may be cultured in normoxic conditions prior to culturing the cells on a tissue culture substrate that is not pre-treated with a protein or an extracellular matrix. Alternatively, the human embryonic stem cells may be cultured in hypoxic conditions.

The cells expressing markers characteristic of the definitive endoderm lineage may be cultured in normoxic conditions prior to culturing the cells on a tissue culture substrate that is not pre-treated with a protein or an extracellular matrix. Alternatively, the cells expressing markers characteristic of the definitive endoderm lineage may be cultured in hypoxic conditions.

In one embodiment, the present disclosure provides a method for deriving a population of cells comprising cells expressing pluripotency markers, comprising the steps of:
a. Culturing human embryonic stem cells,
b. Differentiating the human embryonic stem cells into cells expressing markers characteristic of definitive endoderm cells, and
c. Removing the cells, and subsequently culturing them under hypoxic conditions, on a tissue culture substrate that is not pre-treated with a protein or an extracellular matrix prior to culturing the cells.

In one embodiment, the present disclosure provides a method for deriving a population of cells comprising cells expressing pluripotency markers, comprising the steps of:
a. Culturing human embryonic stem cells, and
b. Removing the cells, and subsequently culturing them under hypoxic conditions, on a tissue culture substrate that is not pre-treated with a protein or an extracellular matrix.

### Cell culture under hypoxic conditions on a tissue culture substrate that is not pre-treated with a protein or an extracellular matrix

In one embodiment, the cells are cultured under hypoxic conditions, on a tissue culture substrate that is not coated with an extracellular matrix for about 1 to about 20 days. In an alternate embodiment, the cells are cultured under hypoxic conditions, on a tissue culture substrate that is not coated with an extracellular matrix for about 5 to about 20 days. In an alternate embodiment, the cells are cultured under hypoxic conditions, on a tissue culture substrate that is not coated with an extracellular matrix for about 15 days.

In one embodiment, the hypoxic condition is about 1% O₂ to about 20% O₂. In an alternate embodiment, the hypoxic condition is about 2% O₂ to about 10% O₂. In an alternate embodiment, the hypoxic condition is about 3% O₂.

The cells may be cultured, under hypoxic conditions on a tissue culture substrate that is not pre-treated with a protein or an extracellular matrix, in medium containing serum, activin-A, and a Wnt ligand. Alternatively, the medium may also contain IGF-1.

The culture medium may have a serum concentration in the range of about 2% to about 5%. In an alternate embodiment, the serum concentration may be about 2%.

Activin-A may be used at a concentration from about 1pg/ml to about 100µg/ml. In an alternate embodiment, the concentration may be about 1pg/ml to about 1µg/ml. In another alternate embodiment, the concentration may be about 1pg/ml to about 100ng/ml. In another alternate embodiment, the concentration may be about 50ng/ml to about 100ng/ml. In another alternate embodiment, the concentration may be about 100ng/ml.

The Wnt ligand may be selected from the group consisting of Wnt-1, Wnt-3a, Wnt-5a and Wnt-7a. In one embodiment, the Wnt ligand is Wnt-1. In an alternate embodiment, the Wnt ligand is Wnt-3a.

The Wnt ligand may be used at a concentration of about 1ng/ml to about 1000ng/ml. In an alternate embodiment, the Wnt ligand may be used at a concentration of about 10ng/ml to about 100ng/ml. In one embodiment, the concentration of the Wnt ligand is about 20ng/ml.

IGF-1 may be used at a concentration of about 1ng/ml to about 100ng/ml. In an alternate embodiment, the IGF-1 may be used at a concentration of about 10ng/ml to about 100ng/ml. In one embodiment, the concentration of IGF-1 is about 50ng/ml.

The cells expressing pluripotency markers derived by the methods of the present invention are capable of expansion in culture under hypoxic conditions, on tissue culture substrate that is not pre-treated with a protein or an extracellular matrix.

The cells expressing pluripotency markers derived by the methods of the present invention express at least one of the following pluripotency markers selected from the group consisting of: ABCG2, cripto, FoxD3, Connexin43, Connexin45, Oct4, SOX-2, Nanog, hTERT, UTF-1, ZFP42, SSEA-3, SSEA-4, Tra1-60, and Tra1-81.

In one embodiment, the cells expressing pluripotency markers derived by the methods of the present invention are capable of expressing markers characteristic of pre-primitive streak cells.

In one embodiment, the cells expressing pluripotency markers derived by the methods of the present invention are capable of expressing markers characteristic of primitive streak cells.

In one embodiment, the cells expressing pluripotency markers derived by the methods of the present invention are capable of expressing markers characteristic of mesendoderm cells.

In one embodiment, the cells expressing pluripotency markers derived by the methods of the present disclosure are capable of expressing markers characteristic of definitive endoderm cells.

### Further differentiation of cells expressing pluripotency markers derived by the methods of the present invention

Cells expressing pluripotency markers derived by the methods of the present invention may be differentiated into cells expressing markers characteristic of the definitive endoderm lineage by any method in the art.

For example, cells expressing pluripotency markers derived by the methods of the present invention may be differentiated into cells expressing markers characteristic of the definitive endoderm lineage according to the methods disclosed in D'Amour et al, Nature Biotechnology 23, 1534 - 1541 (2005).

For example, cells expressing pluripotency markers derived by the methods of the present invention may be differentiated into cells expressing markers characteristic of the definitive endoderm lineage according to the methods disclosed in Shinozaki et al, Development 131, 1651 - 1662 (2004).

For example, cells expressing pluripotency markers derived by the methods of the present invention may be differentiated into cells expressing markers characteristic of the definitive endoderm lineage according to the methods disclosed in McLean et al, Stem Cells 25, 29 - 38 (2007).

For example, cells expressing pluripotency markers derived by the methods of the present invention may be differentiated into cells expressing markers characteristic of the definitive endoderm lineage according to the methods disclosed in D'Amour et al, Nature Biotechnology 24, 1392 - 1401 (2006).

For example, cells expressing pluripotency markers derived by the methods of the present invention may be differentiated into cells expressing markers characteristic of the definitive endoderm lineage by culturing the pluripotent stem cells in medium containing activin-A in the absence of serum, then culturing the cells with activin-A and serum, and then culturing the cells with activin-A and serum of a different concentration. An example of this method is disclosed in D'Amour et al, Nature Biotechnology, 23, 1534-1541, 2005.

### Further differentiation of cells expressing markers characteristic of the definitive endoderm lineage

Cells expressing markers characteristic of the definitive endoderm lineage may be differentiated into cells expressing markers characteristic of the pancreatic endoderm lineage by any method in the art.

For example, cells expressing markers characteristic of the definitive endoderm lineage may be differentiated into cells expressing markers characteristic of the pancreatic endoderm lineage according to the methods disclosed in D'Amour et al, Nature Biotechnology 24, 1392 - 1401 (2006).

For example, cells expressing markers characteristic of the definitive endoderm lineage are further differentiated into cells expressing markers characteristic of the pancreatic endoderm lineage, by treating the cells expressing markers characteristic of the definitive endoderm lineage with a fibroblast growth factor and KAAD-cyclopamine, then removing the medium containing the fibroblast growth factor and KAAD-cyclopamine and subsequently culturing the cells in medium containing retinoic acid, a fibroblast growth factor and KAAD-cyclopamine. An example of this method is disclosed in D' Amour et al, Nature Biotechnology, 24: 1392-1401, (2006).

### Further differentiation of cells expressing markers characteristic of the pancreatic endoderm lineage

Cells expressing markers characteristic of the pancreatic endoderm lineage may be differentiated into cells expressing markers characteristic of the pancreatic endocrine lineage by any method in the art.

For example, cells expressing markers characteristic of the pancreatic endoderm lineage may be differentiated into cells expressing markers characteristic of the pancreatic endocrine lineage according to the methods disclosed in D'Amour et al, Nature Biotechnology 24, 1392 - 1401 (2006).

Without being subject to limitation, the following sections contain examples of methods to obtain cells that are suitable starting materials for forming cells expressing pluripotency markers and markers characteristic of the definitive endoderm lineage according to the methods of the present invention.

### Isolation, expansion and culture of human embryonic stem cells

*Characterization of human embryonic stem cells:* Human embryonic stem cells may express one or more of the stage-specific embryonic antigens (SSEA) 3 and 4, and markers detectable using antibodies designated Tra-1-60 and Tra-1-81 (Thomson et al., Science 282:1145, 1998). Differentiation of human embryonic stem cells *in vitro* results in the loss of SSEA-4, Tra- 1-60, and Tra-1-81 expression (if present) and increased expression of SSEA-1. Undifferentiated human embryonic stem cells typically have alkaline phosphatase activity, which can be detected by fixing the cells with 4% paraformaldehyde, and then developing with Vector Red as a substrate, as described by the manufacturer (Vector Laboratories, Burlingame Calif.). Undifferentiated pluripotent stem cells also typically express Oct-4 and TERT, as detected by RT-PCR.

Another desirable phenotype of propagated human embryonic stem cells is a potential to differentiate into cells of all three germinal layers: endoderm, mesoderm, and ectoderm tissues. Pluripotency of human embryonic stem cells can be confirmed, for example, by injecting cells into SCID mice, fixing the teratomas that form using 4% paraformaldehyde, and then examining them histologically for evidence of cell types from the three germ layers. Alternatively, pluripotency may be determined by the creation of embryoid bodies and assessing the embryoid bodies for the presence of markers associated with the three germinal layers.

Propagated human embryonic stem cell lines may be karyotyped using a standard G-banding technique and compared to published karyotypes of the corresponding primate species. It is desirable to obtain cells that have a "normal karyotype", which means that the cells are euploid, wherein all human chromosomes are present and not noticeably altered.

*Sources of human embryonic stem cells:* Types of human embryonic stem cells include established lines of human embryonic cells derived from tissue formed after gestation, including pre-embryonic tissue (such as, for example, a blastocyst), embryonic tissue, or fetal tissue taken any time during gestation, typically but not necessarily before approximately 10-12 weeks gestation. Non-limiting examples relevant to the disclosure are established lines of human embryonic stem cells or human embryonic germ cells, such as, for example the human embryonic stem cell lines HI, H7, and H9 (WiCell). Also disclosed is use of the compositions of this disclosure during the initial establishment or stabilization of such cells, in which case the source cells would be primary pluripotent cells taken directly from the source tissues. Also relevant to the disclosure are cells taken from a pluripotent stem cell population already cultured in the absence of feeder cells. Also relevant to the disclosure are mutant human embryonic stem cell lines, such as, for example, BG01v (BresaGen, Athens, GA).

In one embodiment of the disclosure, Human embryonic stem cells are prepared as described by Thomson et al. (U.S. Pat. No. 5,843,780; Science 282:1145, 1998; Curr. Top. Dev. Biol. 38:133 ff., 1998; Proc. Natl. Acad. Sci. U.S.A. 92:7844, 1995).

*Culture of human embryonic stem cells:* In one embodiment, human embryonic stem cells are cultured in a culture system that is essentially free of feeder cells, but nonetheless supports proliferation of human embryonic stem cells without undergoing substantial differentiation. The growth of human embryonic stem cells in feeder-free culture without differentiation is supported using a medium conditioned by culturing previously with another cell type. Alternatively, the growth of human embryonic stem cells in feeder-free culture without differentiation is supported using a chemically defined medium.

In an alternate embodiment, human embryonic stem cells are initially cultured layer of feeder cells that support the human embryonic stem cells in various ways. The human embryonic are then transferred to a culture system that is essentially free of feeder cells, but nonetheless supports proliferation of human embryonic stem cells without undergoing substantial differentiation.

Examples of conditioned media suitable for use in the present disclosure are disclosed in US20020072117, US6642048, WO2005014799, and Xu et al (Stem Cells 22: 972-980, 2004).

An example of a chemically defined medium suitable for use in the present disclosure may be found in US20070010011.

Suitable culture media may be made from the following components, such as, for example, Dulbecco's modified Eagle's medium (DMEM), Gibco # 11965-092; Knockout Dulbecco's modified Eagle's medium (KO DMEM), Gibco # 10829-018; Ham's F12/50% DMEM basal medium; 200 mM L-glutamine, Gibco #15039-027; non-essential amino acid solution, Gibco 11140-050; β- mercaptoethanol, Sigma # M7522; human recombinant basic fibroblast growth factor (bFGF), Gibco # 13256-029.

In one embodiment, the human embryonic stem cells are plated onto a suitable culture substrate that is treated prior to treatment according to the methods of the present disclosure. In one embodiment, the treatment is an extracellular matrix component, such as, for example, those derived from basement membrane or that may form part of adhesion molecule receptor-ligand couplings. In one embodiment, the suitable culture substrate is MATRIGEL (Becton Dickenson). MATRIGEL is a soluble preparation from Engelbreth-Holm-Swarm tumor cells that gels at room temperature to form a reconstituted basement membrane.

Other extracellular matrix components and component mixtures are suitable as an alternative. This may include laminin, fibronectin, proteoglycan, entactin, heparan sulfate, and the like, alone or in various combinations.

The human embryonic stem cells are plated onto the substrate in a suitable distribution and in the presence of a medium that promotes cell survival, propagation, and retention of the desirable characteristics. All these characteristics benefit from careful attention to the seeding distribution and can readily be determined by one of skill in the art.

The human embryonic stem cells are then removed from the treated tissue culture substrate and plated onto a non-treated tissue culture substrate, prior to treatment according to the methods of the present invention to form cells expressing pluripotency markers and markers characteristic of the definitive endoderm lineage.

### Differentiation of human embryonic stem cells into cells expressing markers characteristic of the definitive endoderm lineage

Human embryonic stem cells may be differentiated into cells expressing markers characteristic of the definitive endoderm lineage by any method in the art. The cells expressing markers characteristic of the definitive endoderm lineage are suitable for treating according to the methods of the present disclosure.

For example, human embryonic stem cells may be differentiated into cells expressing markers characteristic of the definitive endoderm lineage according to the methods disclosed in D'Amour et al, Nature Biotechnology 23, 1534 - 1541 (2005).

For example, human embryonic stem cells may be differentiated into cells expressing markers characteristic of the definitive endoderm lineage according to the methods disclosed in Shinozaki et al, Development 131, 1651 - 1662 (2004).

For example, human embryonic stem cells may be differentiated into cells expressing markers characteristic of the definitive endoderm lineage according to the methods disclosed in McLean et al, Stem Cells 25, 29 - 38 (2007).

### Differentiation of human embryonic stem cells cultured on an extracellular matrix into cells expressing markers characteristic of the definitive endoderm lineage

In one embodiment, the present disclosure provides a method for differentiating human embryonic stem cells expressing markers characteristic of the definitive endoderm lineage, comprising the steps of:
a. Plating the human embryonic stem cells on a tissue culture substrate coated with an extracellular matrix, and
b. Culturing the human embryonic stem cells with activin-A and a Wnt ligand.

The cells expressing markers characteristic of the definitive endoderm lineage are then subsequently treated by the methods of the present disclosure to form cells expressing pluripotency markers and markers characteristic of the definitive endoderm lineage.

Culturing the human embryonic stem cells with activin-A and a Wnt ligand may be performed in a single culture medium. Alternatively, culturing the human embryonic stem cells with activin-A and a Wnt ligand may be performed in more than one culture media. In one embodiment, culturing the human embryonic stem cells with activin-A and a Wnt ligand is performed in two culture media.

*Extracellular Matrix*: In one aspect of the present disclosure, the human embryonic stem cells are cultured and differentiated on a tissue culture substrate coated with an extracellular matrix. The extracellular matrix may be a solubilized basement membrane preparation extracted from mouse sarcoma cells (which is sold by BD Biosciences under the trade name MATRIGEL). Alternatively, the extracellular matrix may be growth factor-reduced MATRIGEL. Alternatively, the extracellular matrix may fibronectin. In an alternate embodiment, the human embryonic stem cells are cultured and differentiated on tissue culture substrate coated with human serum.

The extracellular matrix may be diluted prior to coating the tissue culture substrate. Examples of suitable methods for diluting the extracellular matrix and for coating the tissue culture substrate may be found in Kleinman, H.K., et al., Biochemistry 25:312 (1986), and Hadley, M.A., et al., J.Cell.Biol. 101:1511 (1985).

In one embodiment, the extracellular matrix is MATRIGEL. In one embodiment, the tissue culture substrate is coated with MATRIGEL at a 1:10 dilution. In an alternate embodiment, the tissue culture substrate is coated with MATRIGEL at a 1:15 dilution. In an alternate embodiment, the tissue culture substrate is coated with MATRIGEL at a 1:30 dilution. In an alternate embodiment, the tissue culture substrate is coated with MATRIGEL at a 1:60 dilution.

In one embodiment, the extracellular matrix is growth factor-reduced MATRIGEL. In one embodiment, the tissue culture substrate is coated with growth factor-reduced MATRIGEL at a 1:10 dilution. In an alternate embodiment, the tissue culture substrate is coated with growth factor-reduced MATRIGEL at a 1:15 dilution. In an alternate embodiment, the tissue culture substrate is coated with growth factor-reduced MATRIGEL at a 1:30 dilution. In an alternate embodiment, the tissue culture substrate is coated with growth factor reduced MATRIGEL at a 1:60 dilution.

Differentiation of human embryonic stem cells into cells expressing markers characteristic of the definitive endoderm lineage on an extracellular matrix, using a single culture medium: In one embodiment, the present disclosure provides a method for differentiating human embryonic stem cells expressing markers characteristic of the definitive endoderm lineage, comprising the steps of:
a. Plating the human embryonic stem cells on a tissue culture substrate coated with an extracellular matrix, and
b. Culturing the human embryonic stem cells with activin-A and a Wnt ligand.

The culture medium should contain sufficiently low concentrations of certain factors to allow the differentiation of human embryonic stem cells to definitive endoderm, such as, for example insulin and IGF (as disclosed in WO2006020919). This may be achieved by lowering the serum concentration, or alternatively, by using chemically defined media that lacks insulin and IGF. Examples of chemically defined media are disclosed in Wiles et al (Exp Cell Res. 1999 Feb 25; 247(1): 241-8.).

The culture medium may have a serum concentration in the range of about 0% to about 10%. In an alternate embodiment, the concentration may be in the range of about 0% to about 5%. In an alternate embodiment, the concentration may be in the range of about 0% to about 2%. In an alternate embodiment, the concentration may be about 2%.

The time of culturing with activin-A and a Wnt ligand may range from about 1 day to about 7 days. In an alternate embodiment, the time of culturing may range from about 1 day to about 3 days. In an alternate embodiment, the time of culturing may be about 3 days.

Activin-A may be used at any concentration suitable to cause differentiation of the human embryonic stem cells. The concentration maybe from about 1pg/ml to about 100µg/ml. In an alternate embodiment, the concentration may be about 1pg/ml to about 1µg/ml. In another alternate embodiment, the concentration may be about 1pg/ml to about 100ng/ml. In another alternate embodiment, the concentration may be about 50ng/ml to about 100ng/ml. In another alternate embodiment, the concentration may be about 100ng/ml.

The choice of the Wnt ligand may be optimized to improve the efficiency of the differentiation process. The Wnt ligand may be selected from the group consisting of Wnt-1, Wnt-3a, Wnt-5a and Wnt-7a. In one embodiment, the Wnt ligand is Wnt-1. In an alternate embodiment, the Wnt ligand is Wnt-3a.

The Wnt ligand may be at a concentration of about 1ng/ml to about 1000ng/ml. In an alternate embodiment, the concentration may be about 10ng/ml to about 100ng/ml.

The single culture medium may also contain a GSK-3B inhibitor. The GSK-3B inhibitor may be selected from the group consisting of GSK-3B inhibitor IX and GSK-3B inhibitor XI. In one embodiment, the GSK-3B inhibitor is GSK-3B inhibitor IX.

When culturing human embryonic stem cells with a GSK-3B inhibitor, the concentration of the GSK-3B inhibitor may be from about InM to about 1000nM. In an alternate embodiment, the human embryonic stem cells are cultured with the GSK-3B inhibitor at a concentration of about 10nM to about 100nM.

The single culture medium may also contain at least one other additional factor that may enhance the formation of cells expressing markers characteristic of the definitive endoderm lineage from human embryonic stem cells. Alternatively, the at least one other additional factor may enhance the proliferation of the cells expressing markers characteristic of the definitive endoderm lineage formed by the methods of the present invention. Further, the at least one other additional factor may enhance the ability of the cells expressing markers characteristic of the definitive endoderm lineage formed by the methods of the present invention to form other cell types, or improve the efficiency of any other additional differentiation steps.

The at least one additional factor may be, for example, nicotinamide, members of TGF-β family, including TGF-β1, 2, and 3, serum albumin, members of the fibroblast growth factor family, platelet-derived growth factor-AA, and -BB, platelet rich plasma, insulin growth factor (IGF-I, II), growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as, for example, triethylene pentamine, forskolin, Na-Butyrate, activin, betacellulin, ITS, noggin, neurite growth factor, nodal, valporic acid, trichostatin A, sodium butyrate, hepatocyte growth factor (HGF), sphingosine 1, VEGF, MG132 (EMD, CA), N2 and B27 supplements (Gibco, CA), steroid alkaloid such as, for example, cyclopamine (EMD, CA), keratinocyte growth factor (KGF), Dickkopf protein family, bovine pituitary extract, islet neogenesis-associated protein (INGAP), Indian hedgehog, sonic hedgehog, proteasome inhibitors, notch pathway inhibitors, sonic hedgehog inhibitors, or combinations thereof.

The at least one other additional factor may be supplied by conditioned media obtained from pancreatic cells lines such as, for example, PANC-1 (ATCC No: CRL-1469), CAPAN-1 (ATCC No: HTB-79), BxPC-3 (ATCC No: CRL-1687), HPAF-II (ATCC No: CRL-1997), hepatic cell lines such as, for example, HepG2 (ATCC No: HTB-8065), and intestinal cell lines such as, for example, FHs 74 (ATCC No: CCL-241).

Differentiation of human embryonic stem cells into cells expressing markers characteristic of the definitive endoderm lineage on an extracellular matrix, using two culture media: Differentiation of human embryonic stem cells into cells of a definitive endoderm lineage may be accomplished by culturing the human embryonic stem cells with activin-A and a Wnt ligand using two culture media. Thus, the differentiation of the human embryonic stem cells may be accomplished as follows:
a. Plating the human embryonic stem cells on a tissue culture substrate coated with an extracellular matrix,
b. Culturing the human embryonic stem cells with activin-A and a Wnt ligand in a first culture medium, and
c. Culturing the human embryonic stem cells with activin-A in a second culture medium.

The first culture medium may contain serum at a low concentration, and the second culture medium may contain serum at a higher concentration than the first culture medium.

The second culture medium may contain a Wnt ligand.

First Culture Medium: The first culture medium should contain sufficiently low concentrations of certain factors to allow the differentiation of human embryonic stem cells into cells expressing markers characteristic of the definitive endoderm lineage, such as, for example insulin and IGF (as disclosed in WO2006020919). This may be achieved by lowing the serum concentration, or alternatively, by using chemically defined media that lacks insulin and IGF. Examples of chemically defined media are disclosed in Wiles et al (Exp Cell Res. 1999 Feb 25; 247(1):241-8.).

In the first culture medium there may be a lower concentration of serum, relative to the second culture medium. Increasing the serum concentration in the second culture medium increases the survival of the cells, or, alternatively, may enhance the proliferation of the cells. The serum concentration of the first medium may be in the range of about 0% to about 10%. Alternatively, the serum concentration of the first medium may be in the range of about 0% to about 2%. Alternatively, the serum concentration of the first medium may be in the range of about 0% to about 1%. Alternatively, the serum concentration of the first medium may be about 0.5%.

When culturing the human embryonic stem cells with activin-A and a Wnt ligand using at least two culture media, the time of culturing in the first culture medium may range from about 1 day to about 3 days.

Activin-A may be used at any concentration suitable to cause differentiation of the human embryonic stem cells. The concentration maybe from about 1pg/ml to about 100µg/ml. In an alternate embodiment, the concentration may be about 1pg/ml to about 1µg/ml. In another alternate embodiment, the concentration may be about 1pg/ml to about 100ng/ml. In another alternate embodiment, the concentration may be about 50ng/ml to about 100ng/ml. In another alternate embodiment, the concentration may be about 100ng/ml.

The choice of the Wnt ligand may be optimized to improve the efficiency of the differentiation process. The Wnt ligand may be selected from the group consisting of Wnt-1, Wnt-3a, Wnt-5a and Wnt-7a. In one embodiment, the Wnt ligand is Wnt-1. In an alternate embodiment, the Wnt ligand is Wnt-3a.

The Wnt ligand may be at a concentration of about 1ng/ml to about 1000ng/ml. In an alternate embodiment, the concentration may be about 10ng/ml to about 100ng/ml.

The first culture medium may also contain a GSK-3B inhibitor. The GSK-3B inhibitor may be added to the first culture medium, to the second culture medium, or to both the first and second culture media.

The GSK-3B inhibitor may be selected from the group consisting of GSK-3B inhibitor IX and GSK-3B inhibitor XI. In one embodiment, the GSK-3B inhibitor is GSK-3B inhibitor IX.

When culturing human embryonic stem cells with a GSK-3B inhibitor, the concentration of the GSK-3B inhibitor may be from about InM to about 1000nM. In an alternate embodiment, the human embryonic stem cells are cultured with the GSK-3B inhibitor at a concentration of about 10nM to about 100nM.

The first culture medium may also contain at least one other additional factor that may enhance the formation of cells expressing markers characteristic of the definitive endoderm lineage from human embryonic stem cells. Alternatively, the at least one other additional factor may enhance the proliferation of the cells expressing markers characteristic of the definitive endoderm lineage formed by the methods of the present disclosure. Further, the at least one other additional factor may enhance the ability of the cells expressing markers characteristic of the definitive endoderm lineage formed by the methods of the present disclosure to form other cell types, or improve the efficiency of any other additional differentiation steps.

The at least one additional factor may be, for example, nicotinamide, members of the TGF-β family, including TGF-β1, 2, and 3, serum albumin, members of the fibroblast growth factor family, platelet-derived growth factor-AA, and -BB, platelet rich plasma, insulin growth factor (IGF-I, II), growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as, for example, triethylene pentamine, forskolin, Na-Butyrate, activin, betacellulin, ITS, noggin, neurite growth factor, nodal, valporic acid, trichostatin A, sodium butyrate, hepatocyte growth factor (HGF), sphingosine-1, VEGF, MG132 (EMD, CA), N2 and B27 supplements (Gibco, CA), steroid alkaloid such as, for example, cyclopamine (EMD, CA), keratinocyte growth factor (KGF), Dickkopf protein family, bovine pituitary extract, islet neogenesis-associated protein (INGAP), Indian hedgehog, sonic hedgehog, proteasome inhibitors, notch pathway inhibitors, sonic hedgehog inhibitors, or combinations thereof.

The at least one other additional factor may be supplied by conditioned media obtained from pancreatic cells lines such as, for example, PANC-1 (ATCC No: CRL-1469), CAPAN-1 (ATCC No: HTB-79), BxPC-3 (ATCC No: CRL-1687), HPAF-II (ATCC No: CRL-1997), hepatic cell lines such as, for example, HepG2 (ATCC No: HTB-8065), and intestinal cell lines such as, for example, FHs 74 (ATCC No: CCL-241).

Second Culture Medium: The second culture medium should contain certain factors, such as, for example, insulin and IGF (as disclosed in WO2006020919), at a sufficient concentration to promote the survival of the cultured cells. This may be achieved by increasing the serum concentration, or, alternatively, by using chemically defined media where the concentrations of insulin and IGF are increased relative to the first culture medium. Examples of chemically defined media are disclosed in Wiles et al (Exp Cell Res. 1999 Feb 25; 247(1): 241-8.).

In a second culture medium having higher concentrations of serum, the serum concentration of the second culture medium may be in the range about 0.5% to about 10%. Alternatively, the serum concentration of the second culture medium may be in the range of about 0.5% to about 5%. Alternatively, the serum concentration of the second culture medium may be in the range of about 0.5% to about 2%. Alternatively, the serum concentration of the second culture medium may be about 2%. When culturing human embryonic stem cells with the second culture medium, the time of culturing may range from about 1 day to about 4 days.

Similar to the first culture medium, Activin-A may be used at any concentration suitable to cause differentiation of the human embryonic stem cells. The concentration maybe from about 1pg/ml to about 100µg/ml. In an alternate embodiment, the concentration may be about 1pg/ml to about 1µg/ml. In another alternate embodiment, the concentration may be about 1pg/ml to about 100ng/ml. In another alternate embodiment, the concentration may be about 50ng/ml to about 100ng/ml. In another alternate embodiment, the concentration may be about 100ng/ml.

The Wnt ligand may be at a concentration of about 1ng/ml to about 1000ng/ml. In an alternate embodiment, the concentration may be about 10ng/ml to about 100ng/ml.

The Wnt ligand may be selected from the group consisting of Wnt-1, Wnt-3a, Wnt-5a and Wnt-7a. In one embodiment, the Wnt ligand is Wnt-1. In an alternate embodiment, the Wnt ligand is Wnt-3a.

The second culture medium may also contain a GSK-3B inhibitor. The GSK-3B inhibitor may be added to the first culture medium, to the second culture medium, or to both the first and second culture media.

The GSK-3B inhibitor may be selected from the group consisting of GSK-3B inhibitor IX and GSK-3B inhibitor XI. In one embodiment, the GSK-3B inhibitor is GSK-3B inhibitor IX.

When culturing human embryonic stem cells with a GSK-3B inhibitor, the concentration of the GSK-3B inhibitor may be from about InM to about 1000nM. In an alternate embodiment, the human embryonic stem cells are cultured with the GSK-3B inhibitor at a concentration of about 10nM to about 100nM.

Similar to the first culture medium, the second culture medium may also contain at least one other additional factor that may enhance the formation of cells expressing markers characteristic of the definitive endoderm lineage from human embryonic stem cells. Alternatively, the at least one other additional factor may enhance the proliferation of the cells expressing markers characteristic of the definitive endoderm lineage formed by the methods of the present invention. Further, the at least one other additional factor may enhance the ability of the cells expressing markers characteristic of the definitive endoderm lineage formed by the methods of the present invention to form other cell types, or improve the efficiency of any other additional differentiation steps.

The at least one additional factor may be, for example, nicotinamide, members of the TGF-β family, including TGF-β1, 2, and 3, serum albumin, members of the fibroblast growth factor family, platelet-derived growth factor-AA, and -BB, platelet rich plasma, insulin growth factor (IGF-I, II), growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as, for example, triethylene pentamine, forskolin, Na-Butyrate, activin, betacellulin, ITS, noggin, neurite growth factor, nodal, valporic acid, trichostatin A, sodium butyrate, hepatocyte growth factor (HGF), sphingosine-1, VEGF, MG132 (EMD, CA), N2 and B27 supplements (Gibco, CA), steroid alkaloid such as, for example, cyclopamine (EMD, CA), keratinocyte growth factor (KGF), Dickkopf protein family, bovine pituitary extract, islet neogenesis-associated protein (INGAP), Indian hedgehog, sonic hedgehog, proteasome inhibitors, notch pathway inhibitors, sonic hedgehog inhibitors, or combinations thereof.

The at least one other additional factor may be supplied by conditioned media obtained from pancreatic cells lines such as, for example, PANC-1 (ATCC No: CRL-1469), CAPAN-1 (ATCC No: HTB-79), BxPC-3 (ATCC No: CRL-1687), HPAF-II (ATCC No: CRL-1997), hepatic cell lines such as, for example, HepG2 (ATCC No: HTB-8065), and intestinal cell lines such as, for example, FHs 74 (ATCC No: CCL-241).

The present invention is further illustrated, but not limited by, the following examples.

### Reference Example 1

### Human Embryonic Stem Cell Culture

Stem cells are undifferentiated cells defined by their ability at the single cell level to both self-renew and differentiate to produce progeny cells, including self renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation and to contribute substantially to most, if not all, tissues following injection into blastocysts.

The human embryonic stem cell lines HI, H7 and H9 were obtained from WiCell Research Institute, Inc., (Madison, WI) and cultured according to instructions provided by the source institute. Briefly, cells were cultured on mouse embryonic fibroblast (MEF) feeder cells in ES cell medium consisting of DMEM/F12 (Invitrogen/GIBCO) supplemented with 20% knockout serum replacement, 100 nM MEM nonessential amino acids, 0.5 mM beta-mercaptoethanol, 2mM L-glutamine with 4ng/ml human basic fibroblast growth factor (bFGF) (all from Invitrogen/GIBCO). MEF cells, derived from E13 to 13.5 mouse embryos, were purchased from Charles River. MEF cells were expanded in DMEM medium supplemented with 10% FBS (Hyclone), 2mM glutamine, and 100 mM MEM nonessential amino acids. Sub-confluent MEF cell cultures were treated with 10µg/ml mitomycin C (Sigma, St. Louis, MO) for 3h to arrest cell division, then trypsinized and plated at 2x104/cm2 on 0.1% bovine gelatin-coated dishes. MEF cells from passage two through four were used as feeder layers. Human embryonic stem cells plated on MEF cell feeder layers were cultured at 37°C in an atmosphere of 5% CO₂ within a humidified tissue culture incubator. When confluent (approximately 5-7 days after plating), human embryonic stem cells were treated with 1mg/ml collagenase type IV (Invitrogen/GIBCO) for 5-10 min and then gently scraped off the surface using a 5-ml pipette. Cells were centrifuged at 900 rpm for 5 min, and the pellet was resuspended and re-plated at a 1:3 to 1:4 ratio of cells in fresh culture medium.

In parallel, HI, H7, and H9 human embryonic stem cells were also seeded on plates coated with a 1:30 dilution of growth factor reduced MATRIGEL (BD Biosciences) and cultured in MEF-conditioned media supplemented with 8 ng/ml bFGF. The cells cultured on MATRIGEL were routinely passaged with collagenase IV (Invitrogen/GIBCO), Dispase (BD Biosciences) or LIBERASE enzyme . Some of the human embryonic stem cell cultures were incubated under hypoxic conditions (approximately 3% O₂).

### Reference Example 2

### Fluorescence-Activated Cell Sorting (FACS) Analysis

Adhered human embryonic stem cells were removed from culture plates by a five-minute incubation with TrypLE™ Express solution (Invitrogen, CA). Released cells were resuspended in human embryonic stem cell culture medium and recovered by centrifugation, followed by washing and resuspending the cells in a staining buffer consisting of 2% BSA, 0.05% sodium azide in PBS (Sigma, MO). As appropriate, the cells were Fc-receptor blocked for 15 minutes using a 0.1% γ-globulin (Sigma) solution. Aliquots (approximately 1x10⁵ cells) were incubated with either phycoerythirin (PE) or allophycocyanin (APC) conjugated monoclonal antibodies (5 µl antibody per 1x10⁶ cells), as indicated in **Table IA**, or with an unconjugated primary antibody. Controls included appropriate isotype matched antibodies, unstained cells, and cells stained only with secondary conjugated antibody. All incubations with antibodies were performed for 30 mins at 4°C, after which the cells were washed with the staining buffer. Samples that were stained with unconjugated primary antibodies were incubated for an additional 30 mins at 4°C with secondary conjugated PE or - APC labeled antibodies. See **Table IB** for a list of secondary antibodies used. Washed cells were pelleted and resuspended in the staining buffer, and the cell surface molecules were identified using a FACS Array (BD Biosciences) instrument, collecting at least 10,000 events.

### Reference Example 3

### Immunocytochemistry

Adhered cells were fixed with 4% paraformaldheyde for 20 min at room temperature. Fixed cells were blocked for 1 h at room temperature with PBS/0.1%BSA/10% normal chick serum /0.5% Triton X-100 and then incubated overnight with primary antibodies in PBS/0.1%BSA/10% normal chick serum at 4°C. The list of primary antibodies and their working dilutions are shown in **Table IA.** After three washes in PBS/0.1% BSA, fluorescent secondary antibodies (**Table IB**) at a 1:100 dilution in PBS were incubated with cells for 1 h at room temperature to allow binding. Control samples included reactions where the primary antibody was omitted or where the primary antibody was replaced with corresponding matched negative control immunoglobulins at the same concentration as the primary antibodies. Stained samples were rinsed; a drop of PROLONG® (Invitrogen, CA) containing diamidino-2-phenylindole, dihydrochloride (DAPI) was added to each sample to counter-stain the nucleus and to function as an anti-fade reagent. Images were acquired using a Nikon Confocal Eclipse C-1 inverted microscope (Nikon, Japan) and a 10-60X objective.

### Example 4

### PCR Analysis of ES Derived-Cells

*RNA extraction, purification*, *and cDNA synthesis:* RNA samples were purified by binding to a silica-gel membrane (Rneasy Mini Kit, Qiagen, CA) in the presence of an ethanol-containing, high-salt buffer followed by washing to remove contaminants. The RNA was further purified using a TURBO DNA-free kit (Ambion, INC), and high-quality RNA was then eluted in water. Yield and purity were assessed by A260 and A280 readings on a spectrophotometer. CDNA copies were made from purified RNA using an ABI (ABI, CA) high capacity cDNA archive kit.

*Real*-*time PCR amplification and quantitative analysis:* Unless otherwise stated, all reagents were purchased from Applied Biosystems. Real-time PCR reactions were performed using the ABI PRISM® 7900 Sequence Detection System. TAQMAN® UNIVERSAL PCR MASTER MIX® (ABI, CA) was used with 20 ng of reverse transcribed RNA in a total reaction volume of 20 µl. Each cDNA sample was run in duplicate to correct for pipetting errors. Primers and FAM-labeled TAQMAN®probes were used at concentrations of 200 nM. The level of expression for each target gene was normalized using a human glyceraldehyde-3-phosphate dehydrogenase (GAPDH) endogenous control previously developed by Applied Biosystems. Primer and probe sets are listed in **Table II.** SOX-17 primers were designed using the PRIMERS program (ABI, CA) and were the following sequences: SOX-17: TGGCGCAGCAGATACCA, AGCGCCTTCCACGACTTG, and CCAGCATCTTGCTCAACTCGGCG. After an initial incubation at 50°C for 2 min followed by 95°C for 10 min, samples were cycled 40 times in two stages - a denaturation step at 95°C for 15 sec followed by an annealing/extension step at 60°C for 1 min. Data analysis was carried out using GENEAMP®7000 Sequence Detection System software. For each primer/probe set, a Ct value was determined as the cycle number at which the fluorescence intensity reached a specific value in the middle of the exponential region of amplification. Relative gene expression levels were calculated using the comparative Ct method. Briefly, for each cDNA sample, the endogenous control Ct value was subtracted from the gene of interest Ct to give the delta Ct value (ΔCt). The normalized amount of target was calculated as 2-ΔCt, assuming amplification to be 100% efficiency. Final data were expressed relative to a calibrator sample.

### Reference Example 5

### Differentiation of Human Embryonic Stem Cells Cultured on Tissue Culture Substrate Coated with MATRIGEL to Definitive Endoderm (DE)

Cells from the human embryonic stem cell line H9 at passage 54 were cultured under hypoxic conditions (approximately 3% O₂) and plated on MATRIGEL (1:30 dilution) coated dishes were exposed to DMEM/F12 medium supplemented with 0.5% FBS, 20 ng/ml WNT-3a (Catalog# 1324-WN-002, R&D Systems, MN), and 100 ng/ml Activin-A (R&D Systems, MN) for two days followed by treatment with DMEM/F12 media supplemented with 2% FBS and 100 ng/ml Activin-A (AA) for an additional 3-4 days. Figure 1 depicts the expression of CXCR4 by FACS at day 4. **Figure 2** displays the real-time PCR data for cultures of cells from the human embryonic stem cell line H9 treated with low serum + AA + WNT3A at days 4 and 6. This protocol resulted in significant upregulation of definitive endoderm markers. This procedure will be further referred to as the DE (Definite Endoderm) protocol.

### Reference Example 6

### Isolation and Expansion of Human Embryonic Stem Cell Derived Cells Differentiated to the Definitive Endoderm Stage

Cells from the human embryonic stem cell lines H1 and H9 various passages (Passage 30-54) were cultured under hypoxic conditions (approximately 3% O₂) for at least three passages. The cells were cultured in MEF-CM supplemented with 8 ng/ml of bFGF and plated on MATRIGEL coated plates according to **Example 1.** The cells were exposed to the DE protocol outlined in **Example 5.** At days 3-6, the cultures were exposed to TrypLE™ Express solution (Invitrogen, CA) for 5 mins. Released cells were resuspended in DMEM-F12 + 2% FBS medium, recovered by centrifugation, and counted using a hemocytometer. The released cells were seeded at 1000-10,000 cells/cm² on tissue culture polystyrene (TCPS) treated flasks and cultured in DMEM-F12 + 2% FBS + 100 ng/ml activin-A + 20 ng/ml WNT-3A under hypoxic conditions (approximately 3% O₂) at 37 °C in standard tissue culture incubator. The TCPS flaks were not coated with MATRIGEL or other extracellular matrix proteins. The media was changed daily. In some cultures, the media was further supplemented with 10-50 ng/ml of IGF-I (insulin growth factor-I from R&D Systems, MN) or 1X ITS (Insulin, transferrin, and selenium from Invitrogen, Ca). In some of the culture conditions the basal media (DM-F12 + 2% FBS) was further supplemented with 0.1 mM mercaptoethanol (Invitrogen, CA) and non-essential amino acids (IX, NEAA from Invitrogen, CA). The first passage cells are referred to as P1. In parallel, similar cultures were established under normoxic conditions (approximately 21% O₂). The outline of this isolation procedure is depicted in **Figure 3**.

Following 5-15 days of culturing, distinct cell colonies appeared surrounded by a large number of enlarged cells that appear to be in senescence (**Figure 4a****-b**). At approximately 50-60% confluency, the cultures were passaged by exposure to TrypLE™ Express solution for 5 mins at room temperature. The released cells were resuspended in DMEM-F12 + 2% FBS medium, recovered by centrifugation, and seeded at 10,000 cells/cm² on tissue culture polystyrene (TCPS) treated flasks in DMEM-F12 + 2%FBS + 100 ng/ml activin-A + 20 ng/ml WNT-3A +/- 50 ng/ml of IGF-I. This media will be further referred to as the "growth media". **Figure 4** **c** depicts the morphology of cells at passage 3 seeded at 10,000 cells/cm². At passage 3 (**panel c**) following the initial isolation, the cells appeared to have a uniform epithelial-like morphology with a large nucleus to cytoplasm ratio.

In some cultures the growth media was further supplemented with 1X NEAA plus 0.1 mM mercaptoethanol. Following three to four passages, the attached cells appeared to have a uniform morphology with a large nucleus to cytoplasm ratio. Parallel cultures established under normoxic conditions failed to show robust colony formation by attached cells. Following 2-3 passages, the cultures established under normoxic conditions were abandoned due to poor growth rate.

### Reference Example 7

### Role of activin-A, WNT3A, and IGF-I in Expansion and Maintenance of DE Markers Following Multiple Passages

Cultures derived from the parental human embryonic stem cell line H9 according to the methods described in **Example 6** were passaged every 4-7 days. **Figure 5** depicts real-time PCR results for the expanded cells cultured in 2% FBS + DMEM-F12 + 100 ng/ml of activin-A + 20 of WNT3A for three passages. This data is for a line isolated at day6 of the DE protocol outlined in **Example 5.** There is a clear decrease in DE markers, such as SOX-17 and HNF-3 beta following each passage. As shown in **Figure 6**, addition of WNT3A to the growth media containing activin-A led to a significant boost in the expression of DE markers. However, addition of 50 ng/ml of IGF-I and withdrawal of Activin-A and WNT-3A (**Figure 7 a-c**) led to a precipitous drop in the expression of DE markers along with OCT-4 and an increase in expression of visceral endoderm markers, such as SOX-7 and AFP. **Figure 8 a-c** depicts the morphology of expanded cells derived from H9p54 at passage 5 cultured in a) 2% FBS + DMF12 + 100 ng/ml of activin-A + 20 ng/ml of WNT-3A, b) 2% FBS + DM-F12 + 100 ng/ml of activin-A, c) 2% FBS + DM-F12 + 50 ng/ml of IGF-I. Morphology of cells cultured in the presence of activin-A or activin-A + WNT3A were very similar and distinct from morphology of cells cultured in 2% FBS + IGF-I.

### Reference Example 8

### Expansion Potential of Human Embryonic Stem Cell Derived Cells Differentiated to the Definitive Endoderm stage

Cultures established from the parental human embryonic stem cell line H9 according to the methods described in **Example 6** were passaged every 4-5 days when the growth media contained 50 ng/ml of IGF-I or ITS. However, cultures fed with growth media lacking the IGF or ITS supplements showed slower growth rate and were passaged every 5-7 days. The population doubling time of cells fed with growth media + 50 ng/ml of IGF-I was approximately 55 hrs where as the population doubling time of cultures fed only with the growth media was approximately 75 hrs. The cell population expanded according to **Example 6** will be referred to as EXPRES cells (EXpandable PRE-primitive Streak cells). **Table III** lists various EXPRES cells established according to methods outlined in **Example 6.** The expansion potential of two cell lines (EXPRES 01 and 02) is depicted in **Figure 9**.

### Reference Example 9

### Derivation of EXPRES Cells from Suspensions of Single Human Embryonic Stem Cells

Cells from the human embryonic stem cell lines H1 P33 and H9 P45 were cultured under hypoxic conditions (approximately 3% O₂) for at least three passages. The cells were cultured in MEF-CM supplemented with 8 ng/ml of bFGF and plated on MATRIGEL coated plates according to **Example 1.** At approximately 60% confluency, the cultures were exposed to TrypLE™ Express solution (Invitrogen, CA) for 5 mins. Released cells were resuspended in DMEM-F12 + 2% FBS medium, recovered by centrifugation, and counted using a hemocytometer. The released cells were seeded at 1000 to 10,000 cells/cm² on tissue culture polystyrene (TCPS) treated flasks and cultured in DM-F12 + 2% FBS + 100 ng/ml activin-A + 20 ng/ml WNT-3A + 50 ng/ml of IGF-I + 0.1 mM mercaptoethanol (Invitrogen, CA) and non-essential amino acids (IX, NEAA from Invitrogen, CA) under hypoxic conditions (approximately 3% O₂) at 37 °C in a standard tissue culture incubator. The TCPS flasks were not coated with MATRIGEL or other extarcellular matrix proteins. The media was changed daily. The first passage cells are referred to as P1. In parallel, similar cultures were established under normoxic conditions (approximately 21% O₂). Cultures established under atmospheric conditions did not result in colony formation and there was poor cell proliferation. However, cultures established under hypoxic conditions resulted in formation of many colonies (**Figure 10**) resembling embryonic stem cell colonies cultured on MATRIGEL or on MEF feeders. These cultures closely resemble the properties of EXPRES cells isolated during ES to DE differentiation.

### Reference Example 10

### Expression of Cell Surface Proteins by EXPRES Cells

The cell lines EXPRES 01 and EXPRES 02 were evaluated for expression of various cell-surface markers including markers associated with pluripotency. Cells from EXPRES 01 were evaluated at passages 9 to 24. Cells from EXPRES 02 were evaluated at passages 7 to 20. Both lines exhibited strong expression of pluripotency markers typically assigned to undifferentiated human embryonic stem cells. However, the EXPRES 02 line showed a higher percentage of expression of differentiation markers, such as CXCR4, LIF receptor, and NCAM as compared to the EXPRES 01 line. Representative FACS plots are depicted in **Figure 11** for EXPRES 01 P24 and in **Figure 12** for EXPRES 02 P21 line. **Table IV** lists the mean expression levels along with the ranges (in brackets) of the evaluated cell surface markers from three different experiments.

### Reference Example 11

### Expression of Pluripotency Associated Markers by EXPRES Cells- Immuno fluorescent (IF) Staining

EXPRES 01 and 02 cells maintained in their respective growth media were stained for pluripotency-associated markers using methods outlined in **Example 3.** **Figure 13** depicts IF images for OCT-4, Nanog, SOX-2, and HNF-3 beta for EXPRES 01 P10 cells cultured in 2% FBS + DM-F12 + 100 ng/ml activin-A + 20 ng/ml WNT3A + 50 ng/ml IGF-I. **Figure 14** depicts IF images for OCT-4, Nanog, SOX-2, and HNF-3 beta for EXPRES 02 passage 9 cells cultured in 2% FBS + DM-F12 + 100 ng/ml activin-A + 20 ng/ml WNT3A. EXPRES 01 cells strongly stain positive for OCT-4, Nanog, and SOX-2 and weakly for HNF-3 beta. However, EXPRES 02 cells show stronger expression for HNF-3 beta and weaker expression of OCT-4, NANOG, and SOX-2.

### Reference Example 12

### Expression of Definitive Endoderm and Undifferentiated Embryonic Stem Cell Markers by Real Time PCR

Real-time PCR analysis of embryonic markers (POU5F1, SOX-2, UTF1, ZFP42, Connexin43, Connexin45, FOXD3), extraembryonic markers (AFP, KRT15), ectoderm markers (SOX-1, TUBB3, NESTIN), endoderm markers (FOXA2, IPF1, KRT15, GATA-4), and mesoderm markers (GATA-4, GATA-2, MYOD, MSX1, CFC1, ABCG2) expressed by EXPRES 01 passage 11 and EXPRES 02passage 7 lines cultured in their respective growth media is depicted in **Figure 15 a-h****.** All of the data is normalized to fold change with respect to undifferentiated cells from the human embryonic stem cell line H9, cultured in MEF-CM on MATRIGEL coated plates. As a control, EB bodies were formed from H9 cells using standard methods of collagenase digestion and seeding on non-treated surfaces in DMEM-F12 + 20 % FBS for approximately 10 days. Gene expression of various germ layers was upregulated by EB bodies as compared to undifferentiated ES cells. Another reference RNA was collected from undifferentiated SA002 line (Cellartis, Sweden) cultured on MATRIGEL in MEF-CM. As expected gene expression of various germ layers was strongly upregulated by EB bodies as compared to EXPRES 01, EXPRES 02, SA002, and H9 lines. Both EXPRES 01 and 02 lines showed expression of FOXA2 as compared to undifferentiated SA002 and H9 lines. Neither of the EXPRES lines exhibited strong expression of extra embryonic, mesoderm or ectoderm markers. Furthermore, expression of embryonic markers by EXPRES cells was similar to the SA002 and H9 reference cell lines.

### Reference Example 13

### Various Growth Media Useful for Expansion of EXPRES Cells

EXPRES cells have been successfully cultured in the following media compositions for at least 2-30 passages:
1. DM-F12 + 2% FBS + 100 ng/ml AA + 20 ng/ml WNT-3A
2. DM-F12 + 2% FBS + 100 ng/ml AA + 20 ng/ml WNT-3A + 50 ng/ml IGF-I
3. DM-F12 + 2% FBS + 100 ng/ml AA + 20 ng/ml WNT-3A + 10 ng/ml IGF-I
4. DM-F12 + 2% FBS + 50 ng/ml AA + 20 ng/ml WNT-3A + 50 ng/ml IGF-I
5. DM-F12 + 2% FBS + 50 ng/ml AA + 10 ng/ml WNT-3A + 50 ng/ml IGF-I
6. DM-F12 + 2% FBS + 50 ng/ml AA + 20 ng/ml WNT-3A + 10 ng/ml IGF-I
7. DM-F12 + 2% FBS + 100 ng/ml AA + 10 ng/ml WNT-3A + 10 ng/ml IGF-I
8. HEScGRO defined media (Chemicon, CA)

The basal component of the above listed media may be replaced with similar media such as, RPMI, DMEM, CRML, Knockout ™DMEM, and F12.

### Reference Example 14

### Differentiation of EXPRES Cells Cultured on Tissue Culture Substrate to Definitive Endoderm (DE) cells

EXPRES 01 cells at passage 5 and EXPRES 02 cells at passage 4, cultured on TCPS in their respective media were exposed to DMEM/F12 medium supplemented with 0.5% FBS, 20 ng/ml WNT-3a, and 100 ng/ml activin-A (R&D Systems, MN) for two days followed by treatment with DMEM/F12 media supplemented with 2% FBS and 100 ng/ml activin-A (AA) for an additional 3-5 days. **Figure 16** depicts the expression of CXCR4 by FACS at days 4 for EXPRES 01 cells (**Figure 16** **a**, approximately 17% CXCR4 positive) and EXPRES 02 cells (**Figure 16b**, approximately 40% CXCR4 positive). **Figure 17** displays the real-time PCR data for EXPRES 01 cell (**Figure 17a**) and EXPRES 02 cell (**Figure 17b**) cultures treated with low serum + AA + WNT3A at days 2-5. **Figures 18** and **19** depict immunofluoresence images of EXPRES 01 and 02 cells, respectively, treated with the same treatment mentioned above for 4 days. Overall EXPRES 02 cells appear to show a stronger expression of DE markers as compared to EXPRES 01 cells. As evident by FACS, immuno staining, and PCR data, lowering of serum concentration and removal of IGF did enhance the expression of DE markers. However, the overall expression level of DE markers such as CXCR4 and HNF-3 beta was lower than what we have routinely observed in undifferentiated human ES cultures differentiated to the DE stage.

In order to enhance expression of DE markers, the DE differentiation protocol was changed to the following: EXPRES 01 cells at passage 19 and EXPRES 02 cells at passage 14 cultured on TCPS in their respective media were exposed to DMEM/F12 medium supplemented with 0.5% FBS, 20 ng/ml WNT-3a, 100 ng/ml activin-A, and 100 nM GSK-3B inhibitor IX (Catalog# 361550, Calbiochem, CA) for 4 to 5 days. **Figure 20** depicts the expression of CXCR4 by FACS at days 4 for EXPRES 01 (**Figure 20** **a**, approximately 57% CXCR4 positive) and EXPRES 02 (**Figure 20b**, approximately 86% CXCR4 positive). **Figure 21** depicts the corresponding immuno fluorescent images at day 5 for EXPRES 01 cells (**panels a-f**) and EXPRES 02 cells (**panels g-l**) cells differentiated to the DE stage. **Figure 22** displays the real-time PCR data for EXPRES 01 (**Figure 22a**) and EXPRES 02 (**Figure 22b**).

### Reference Example 15

### Effect of Seeding Density on the Differentiation of EXPRES 01 Cells to DE

EXPRES 01 P31 cells were seeded at 5000, 10000, 20000, or 40000 cells/cm² on TCPS plates in DM-F12 + 2% FBS + 0.1 mM mercaptoethanol + IX, NEAA + 100 ng/ml activin-A + 20 ng/ml WNT3A under hypoxic conditions (approximately 3% O₂) at 37 °C in standard tissue culture incubator. Following two days post seeding, the media was switched to DMEM-F12 + 0.5% FBS + 100 ng/ml activin-A + 20 ng/ml WNT3A + 100 nM GSK-3B inhibitor IX for four days under hypoxic conditions (approximately 3% O₂) at 37 °C in standard tissue culture incubator. **Figure 23** depicts the real-time PCR analysis of definitive endoderm markers at day 4 of differentiation. It appears that a seeding density of at least 10000-20000 cells/cm² is required for the robust formation of definitive endoderm.

### Reference Example 16

### Telomere Length of EXPRES Cells

The telomere length of two EXPRES lines isolated according to **Example 5** along with undifferentiated cells from the human embryonic stem cell line H1 was analyzed by using the *Telo TAGGG* Telomere Length Assay (Roche, IN) and following the manufacturer's instruction. **Figure 24** depicts the telomere length for EXPRES 01 cells at P24, EXPRES 02 cells at P17, H1 cells at P40, high and low telomere controls provided by the kit, along with the scale marker. Both lines appear to have shorter telomere length than undifferentiated ES cells.

### Reference Example 17

### Further Differentiation of EXPRES Cells Cultured on Tissue Culture Substrate to Pancreatic Endoderm

EXPRES 01 cells at passage 21 underwent a 5 day differentiation by treatment with 100ng/ml activin-A, 10ng/ml of Wnt3a, and 100 nM GSK3beta inhibitor IX in 0.5% FBS, DMEM:F12 media. Cells were analyzed by FACS and showed 80% of the cells were positive for CXCR4. The cells were then treated for 3 days in each of the following steps: 2%FBS DMEM:F12 containing 50ng/ml FGF-10, and 0.25µM KAAD-Cyclopamine (Calbiochem, CA); followed by, 1%B27 DMEM, low glucose containing 50 ng/ml FGF-10, 0.25µM KAAD-Cyclopamine and 1µM Retinoic Acid (Sigma, MO); followed by, 1%B27 DMEM, low glucose containing 1µM DAPT (Calbiochem, CA) plus 50 ng/ml Exendin4 (Sigma, MO); and lastly followed by, 1% B27 DMEM CMRL containing 50ng/ml each of IGF, HGF and Exendin4. Samples were taken at the end of each step, and RNA was extracted for the cells. Q-RT PCR was conducted for the markers shown. As depicted in **Figure 25****,** Insulin levels were increased 100 fold over untreated cells and PDX-1 levels were also increased over 1000 fold.

### Reference Example 18

### Further Differentiation of EXPRES Cells Cultured on Tissue Culture Substrate to Foregut Endoderm

EXPRES01 cells at passage 35 underwent a 5 day differentiation by treatment with 100ng/ml activin-A, 20ng/ml of Wnt3a, and 100 nM GSK3beta inhibitor IX in 0.5% FBS, DMEM:F12 media. Cells were analyzed by FACS and showed approximately 70% of the cells were positive for CXCR4. The cells were then treated for in each of the following steps: 2%FBS DMEM:F12 containing 50ng/ml FGF-10, and 0.25µM KAAD-Cyclopamine (Calbiochem, CA) for 3 days; Step3, 1%B27 DMEM, low glucose containing 50 ng/ml FGF-10, 0.25µM KAAD-Cyclopamine and 1µM Retinoic Acid (Sigma, MO) for 4 days; and Step 4, 1%B27 DMEM, low glucose containing 1µM DAPT (Calbiochem, CA) plus 50 ng/ml Exendin4 (Sigma, MO) for 4 days.. This protocol is based on a prior publication by D'Amour et al (Nature Biotech, 24, 1392, 2006). Cells were fixed at end of stage 4 and stained for PDX-1, HNF-3 beta, SOX-17, Albumin, anti-trypsin, and CDX-2. As depicted in **Figure 26**, EXPRES cells can be readily differentiated to foregut endoderm as measured by expression of PDX-1 (approximately 20% of culture stained positive), HNF-3 beta (approximately 90% positive), albumin (approximately 5% positive), anti-1- Trypsin (approximately 70% positive), SOX-17 (approximately 70%), and CDX-2 (approximately 5% positive).

### Reference Example 19

### Microarray Analysis of EXPRES Cells Versus Undifferentiated Human Embryonic Stem Cells

Total RNA was isolated from cultures of the human embryonic stem cell line H9,at passage 44, EXPRES 01 P11 and EXPRES 02 P7 using an RNeasy mini kit (Qiagen): All groups contained three biological replicates and each biological replicate was repeated on two separate gene chips. Sample preparation, hybridization, and image analysis were performed according to the Affymetrix Human Genome U133 Plus 2.0 Array. Following normalization and a log transformation, data analysis was performed using OmniViz® software (MA) and GENESIFTER (VizXLabs, WA). Significant differences in gene expression between the samples were evaluated using analysis of variance and an F-test with adjusted P-value (Benjamini and Hochberg correction) of less than or equal to 0.05. Only genes with a present call in at least one group were included in the analysis. **Table V** lists the mean normalized log-transformed signal intensity of genes showing at least 5-fold difference between groups (undifferentiated ES, EXPRES 01 and EXPRES 02 cells) along with the adjusted P-value for each gene. Genes representing primitive streak or definite endoderm are highlighted in bold. Only the top 200 genes that are upregulated or down regulated are shown in **Table V.**

### Reference Example 20

### Microarray Analysis of EXPRES Cells Differentiated to the DE Stage Versus Human Embryonic Stem Cells Differentiated to the DE Stage

Total RNA was isolated from the following cultures using an RNeasy mini kit (Qiagen): **A)** H9P33 cells cultured on MATRIGEL-coated plates (1:30 dilution) and exposed to DMEM/F12 medium supplemented with 0.5% FBS and 100 ng/ml Activin-A and 20 ng/ml of wnt3A for two days followed by treatment with DMEM/F12 medium supplemented with 2% FBS and 100 ng/ml Activin-A (AA) for an additional three days; **B)** EXPRES 01 P24 cells cultured on TCPS and exposed to DMEM/F12 medium supplemented with 0.5% FBS, 100 ng/ml Activin-A, 20 ng/ml of WNT3A, and 100 nm GSK-3B IX inhibitor (Catalog# 361550, Calbiochem, CA) for five days **C)** EXPRES02 P17 cells cultured on TCPS and exposed to DMEM/F12 medium supplemented with 0.5% FBS, 100 ng/ml Activin-A, 20 ng/ml of WNT3A, and 100 nm GSK-3B IX inhibitor (Catalog# 361550, Calbiochem, CA) for five days, **D)** H9P39 cells cultured on MATRIGEL-coated plates (1:30 dilution) and exposed to DMEM/F12 medium supplemented with 0.5% FBS and 100 ng/ml Activin-A and 20 ng/ml of wnt3A for two days followed by treatment with DMEM/F12 medium supplemented with 2% FBS and 100 ng/ml Activin-A (AA) for an additional two days RNA samples were collected from group **D** at 2hrs, 6hrs, 24 hrs, 30 hrs, 48 hrs, 72 hrs, and 96 hrs. EXPRES 01 and 02 cultured in their respective growth media were also included as controls. All groups contained three biological replicates and each biological replicate was repeated on two separate gene chips.

Sample preparation, hybridization, and image analysis were performed according to the Affymetrix Human Genome U133 Plus 2.0 Array. Following normalization and a log transformation, data analysis was performed using OmniViz® software (MA) and GENESIFTER (VizXLabs, WA). Significant differences in gene expression between the samples were evaluated using analysis of variance and an F-test with adjusted P-value (Benjamini and Hochberg correction) of less than or equal to 0.05. Only genes with a present call in at least one group were included in the analysis. **Table VI** lists the mean normalized log-transformed signal intensity of genes showing at least 5-fold between group A, group B, group C, and group D at various time points along with the adjusted P-value for each gene. Only the top 200 genes that are upregulated or down regulated are shown in **Table VI.** Genes representing primitive streak or definite endoderm are highlighted in bold. **Table VII** lists the correlation coefficient for each comparison group. Scatter plots corresponding to the correlation coefficients are depicted in **Figure 27**. The global expression profile of EXPRES 01 cells appear to resemble the expression profile of samples at 30 hrs or less of the DE stage, whereas the EXPRES 02 cells appear to resemble the expression profile of samples at greater than 48 hrs of the DE stage.

### Reference Example 21

### Formation of Embryoid Bodies and Differentiation to Various Lineages

EXPRES 01 passage 27 cultures were removed as single cells using TrypLE™ Express solution, spun at 200g for 4 mins, and resuspended in DMEM-F12 + 20% FBS. The cell suspension was seeded on low adhesion Petri dishes. 3-4 days post seeding, embryoid body (EB) like structures were formed (**Figure 28**). Treatment of the cultures with DMEM-F12 + 2% FBS + 1 micro molar retinoic acid induced expression of ectoderm markers, such as NeuroD.

### Reference Example 22

### Formation of Teratomas in the Kidney Capsules of NOD-SCID Mice

Undifferentiated cells from the human embryonic stem cell line H9 at passage 42, EXSPRES 01 Passage 30, and EXPRES 02 P22 cells were released from cultures using TRYPLE, washed in basal media, and then suspended in DMEM-F12 basal media. Approximately 1x10⁶ undifferentiated H9 P42, 1.5 million EXPRES 01 and 02 cells were injected into the kidney capsule of six week old NOD-SCID mice. Five weeks following transplantation, the animals were sacrificed; kidneys were excised and fixed in formalin or placed in lysis buffer to collect RNA for subsequent PCR analysis. **Figure 29** depicts expression of markers characteristic of mesoderm, ectoderm, endoderm, extraembryonic visceral endoderm, and pluripotency markers for collected samples. Similar to H9 line, both EXPRES lines show strong expression of all germ layers along with extra embryonic endoderm.

### Reference Example 23

### Proliferation And Cell Cycle Analysis of EXPRES 03 cells

Human embryonic stem cells have a unique cell cycle status that is distinguishable from other somatic cells, characterized by high proportion of cells in S-Phase, and shortened or truncated Gap phases (G1 and G2). The cell cycle control mechanisms in hES cells may be functionally linked to their self-renewal and pluripotency capacity of these cells, and may be different from control mechanism in their differentiated/committed counterparts. These experiments were designed to determine the cells cycle nature of EXPRES cells that have been shown to express many of the markers of pluripotency hES cells.

*Methods:* Cells were seeded at 5,000-10,000 cells /cm² in Cell Bind tissue culture flasks (Corning) and cultured for 2-4 days. EXPRES 03 cells were cultured either in growth medium containing 2% FBS inDMEM/F12 and Activin-A (100 ng/ml), wnt3a (10-20 ng/ml) and IGF (50 ng/ml). For comparative proliferation analysis, IGF was sometimes omitted from the growth factor cocktail. Cell proliferation was analyzed using the APC BRDU Flow kit according to the Manufacturer's recommendations (BD Biosciences, San Diego, CA). Briefly, cells were pulsed with BRDU for 1-2 hrs at the end of culture period, released using Tryple E Express and counted. Cells were fixed in BD Cytofix/Cytosperm Buffer, incubated for 30 min, followed by incubation with BD Cytoperm buffer for 10 minutes on ice. Cells were then treated with DNAse for 1 hr at 37° C to expose incorporated BRDU, followed by staining with APC conjugated anti-BRDU antibody. For cell cycle analysis, cells were stained with 7AAD, and analyzed on FACS Array.

*Results*: Similar to the highly hES cells, EXPRES 03 cells retained a highly proliferation rate, as shown by the high percentage of cells that in S-phase determined by their capacity to incorporate BRDU in culture. The frequency of cells in S-Phase was typically greater than 45% (range 40-60%), similar to hES cells (**Figure 30 a-c**). Although the number of EXPRES 03 cells grown in the presence of IGF were 2-3 times the number of cells grown in absence of IGF after 3-4 days, there was only marginal differences in frequency of cells in S-phase when cell were exposed to BRDU for 1 hour or more (**Figure 30** **f**). This high frequency of cell in S-phase and cell cycle structure is unlike that observed in somatic cells, as shown here for human amniotic fluid cells (AFDX002) (**Figure 30** **d**). Furthermore, mitomycin treated cells Mouse Embryo Fibroblast (MEF, **Figure 30** **e**) do not proceed into S-phase but show apparent accumulation in the G2/M phase (64%), which may be related to the inability of mitomycin treated cells to separate the DNA strands at mitosis.

### Reference Example 24

### Transfection Efficiency of ES cells vs EXPRES cells

A major limitation of genetic manipulation of hES is their relative resistance to conventional methods of transfection and viral transduction. In addition to their highly proliferative rates, EXPRES cells are easy to transfect *in vitro.* To compare transfection efficiency, in EXPRES and hES cells were transfected in culture with EGFP and analyzed by fluorescence microscopy and flow cytometric methods.

*Methods*: EXPRES 01 cells were seeded on human fibronectin coated (10µg/ml) 6 well tissue culture plates in growth medium comprising 2% FBS in DMEM/F12, Activin-A (100 ng/ml), wnt3a (10-20 ng/ml) and IGF (50 ng/ml). For cell clusters hES cell were passaged by routine methods using collagenase into MATRIGEL coated 6 well culture plates, and grown in MEF conditioned hES medium. For single cells, hES cells were passaged by exposing cells to TRYPLE for 3 minutes at 37° C, followed by plating onto MATRIGEL coated 6 well culture plates. When cells achieved desired confluency (70-80%), cells were transfected with Lipofectamine 2000 according to the manufacturer's recommendations (Invitrogen, Carlsbad, CA). Briefly, 4 µg of DNA was diluted into 250 µl Opti-MEM I Reduced serum medium. Five microliters of Lipofectamine 200 was mixed into total of 250 µl Opti-MEM I medium for 5 minutes and gently mixed with diluted DNA. The DNA/Lipofectamine complexes were allowed to form at room temperature for 20 minutes, and then added to the respective wells with gentle swirling motions of the plates for gentle mixing. Cells were incubated in presence of complexes for another 24 hrs followed by complete change of medium. Cells were analyzed by fluorescence microscopy and flow cytometry 48 hrs following transfection.

*Results*: The uptake and expression of eGFP was compared by transfection of EXPRES 01 cells and hES cells plated as single cell dispersion or cell clusters, and analyzed 48 hrs later. EXPRES 01 cell showed the highest level of eGFP protein expression, with 75% of cell expressing eGFP by flow cytometric analysis (**Figure 31**). In contrast, hES were more resistant to transfection, with only 3% of cell expressing eGFP protein by FACS when cell clusters were used. Preparing a single cell dispersion of hES enhanced the level of DNA uptake and eGFP expression to about 20 %, but which is still by about three fold less the expression in EXPRES 01 cells.

### Reference Example 25

### EXPRES Cells as a Versatile Tool for Screening

EXPRES cells were grown in DMEM:F12 medium containing 2% FBS, 100ng/ml recombinant human Activin-A (R&D Systems), and 20ng/ml recombinant mouse Wnt3a (R&D Systems). Growth medium for EXPRES 01 cells also contained 50ng/ml recombinant human IGF-1 (R&D Systems). Both cell lines were routinely grown at 37°C in an atmosphere of low oxygen (3%) and 5% CO₂. EXPRES 01 and EXPRES 02 cells were released from culture as a single cell suspension using TrypLE enzymatic digestion (Invitrogen, CA) then washed and counted to determine accurate cell numbers and viability (>95%). Aliquots ranging from 1,250 to 80,000 cells were distributed into human fibronectin-coated wells of a 96-well plate (Corning-Costar) in a final volume of 100 µl culture medium. Control wells were also fibronectin-coated and contained an equivalent volume of culture medium without cells. Plates were allowed to equilibrate overnight in a humidified chamber incubated under standard low oxygen, 5% CO₂ at 37°C. During this time, the cells attached as monolayer cultures with varying degrees of confluency dependent upon the initial seeding density. After overnight culture, 20µl of MTS reagent (CellTiter 96 Aqueous Assay; Promega) was added to each well. MTS is reduced to formazan and can be used as a measure of dehydrogenase enzyme activity directly proportional to the number of live cells. One plate was returned to low oxygen culture while an identical parallel plate was incubated in normal oxygen (20%). After 4 hours, absorbance was read at 490nm on a spectrophormetric plate reader (Molecular Devices). Statistical calculations for mean OD, standard deviation, and percent coefficient of variation (CV) were determined for replicate sample sets within each plate and then compared to similar wells between both plates.

Standard deviation and percent coefficient of variation values demonstrate that EXPRES cells can be evenly distributed between wells for high plating efficiency and good well-to-well reproducibility (**Table VIII a-f**). As seen with average OD₄₉₀ readings for equivalent numbers of cells, the EXPRES 01 line has a higher metabolic activity than the EXPRES 02 line. For each EXPRES line, percent CV values between the two plates suggest there are no differences in metabolic activity for parallel conditions regardless of atmospheric oxygen levels in this short-term assay. Optimal cell numbers per well within the linear range for this assay were determined by graphing average OD readings (**Figure 32**): less than 20,000 cells/well for EXPRES 01 and less than 40,000 cells/well for EXPRES 02. Again, atmospheric differences in oxygen levels did not affect optimal cell number results in this short-term assay. These results suggest that EXPRES cells are amenable to screening protocols that can measure toxicological effects of various agents on cell proliferation and/or metabolic rate.

### Reference Example 26

### Expansion of DE-like Cells Derived from EXPRES Cells

Previous examples establish that EXPRES cells can be derived from embryonic stem cells and can be readily expanded on TCPS in various growth media. Most of these media formulation contain IGF as a supplement or insulin/IGF in 2% FBS used in the growth media. These factors have been shown to inhibit DE related genes through the PI-3 kinase pathway (Stem cells, 25:29-38, 2007). An alternative media was formulated based on DM-F12 + 0.5% FBS + 100 ng/ml AA + 20 ng/ml WNT3A + 100 nM GSK-3B inhibitor IX (further referred to as "growth media for DE cells"). EXPRES 01 at passage 27 cells cultured in the above media were able to propagate at the same rate as cells cultured in DM-F12 + 2% FBS + 100 ng/ml AA + 20 ng/ml WNT3A + 50 ng/ml IGF-I. Furthermore, cells cultured in the growth media for DE cells expressed strong DE markers by real-time PCR (**Figure 33**) through three passages. Approximately 72% of cells expressed CXCR4 (**Figure 34**).

### Reference Example 27

### siRNA Knockdown of Target Genes in EXPRES cells

Efficient knockdown of target genes in human embryonic stem cells using siRNA is severely limited by the ability to achieve high levels of transfection in human embryonic stem cells grown as cluster colonies. EXPRES cells are easily transfected with siRNA using conventional methods, and thus offer a valuable system to screen siRNA oligo sequences, as well as evaluate the role played by targeted genes.

*Methods*: EXPRES 03 cells were seeded on fibronectin coated (10µg/ml) 6 well tissue culture plates in growth medium comprising 2% FBS in DMEM/F12, Activin-A (100 ng/ml), wnt3a (10-20 ng/ml) and IGF (50 ng/ml). For 6 well plates, 200,000 cells were seeded 24 hrs prior to transfection with the siRNA oligo sequences.

To evaluate target gene knockdown, cells at 70-80% confluency cells were transfected using Lipofectamine 2000 according to the manufacturer's recommendations (Ambion (Applied Biosystems), Foster City, CA). Briefly, the appropriate amount of siRNA was diluted into 250 µl Opti-MEM I Reduced serum medium to achieve a final concentration of 100 nmol. Five microlitres of Lipofectamine 2000 was diluted in 250 µl Opti-MEM I medium and incubated for 5 minutes. The complexes were incubated for 15-20 min at room temperature, and then added to the cells with gentle swirling motions of the plates for gentle mixing. Cells were incubated in presence of siRNA for another 24 hrs followed by complete change of medium. Cells were visualized with fluorescence microscopy 24-48 hrs following transfection, and RNA harvested for analysis of target gene knockdown by quantitative RT-PCR methods. The following pre-validated siRNA oligo sequences, purchased from Ambion were tested: Beta-catenin (Id. No. 42816) and GSK3b (Id. No. 42839).

*Results*: Fluorescence microscopy revealed a very high level of siRNA uptake by EXPRES cells (>80%) when using fluorescently labeled siRNA (**Figure 35**). RNA harvested from the cells was analyzed by PCR for target gene knockdown, and compared with control siRNA oligo transfected cells. Beta-catenin and GSK3b siRNA oligos achieved very high levels of gene knockdown in EXPRES cells, with greater than 93% gene knockdown. Other non-validated oligo sequences to other gene targets eg Hes-1, Oct-4 achieved lower and variable levels of target gene knockdown.. Specificity of oligo sequences was verified by analysis of other gene transcripts, which did not show any appreciable knockdown.

### Reference Example 28

### Cytokine Antibody Array analysis for EXPRES 01 and 02 lines

EXPRES 01 and 02 lines at passage 22 and 23, respectively, were grown to approximately 70% confluency in their respective media and then cell lystaes was collected using mammalian cell lysis kit (Sigma-Aldrich, MO). Cytokine array analysis was completed using Cytokine Array panels provided by RayBiotech, GA (http://www.raybiotech.com/). **Table IX a-c** lists cytokine, cytokine and growth factor receptor expression following normalization of the data and background subtraction. For each panel, positive and negative controls are also included. The panels were run for two different samples per cell type.

### Reference Example 29

### Karyotype Analysis

The karyotype of EXPRES 01 cells at passage 20 and EXPRES 02 cells at passage 15 was determined by G-band analysis. Cytogenetic analysis was performed on twenty-one G-banded cells from EXPRES 01 and on twenty G-banded cells from EXPRES 02. Half of the G-banded EXPRES 01 cells showed a normal 46XX karyotype while the rest showed abnormal karyotype such as trisomy 17. The EXPRES 02 line also showed a chromosome rearrangement with a duplication of almost all of the chromosome 1 short-arm.

### Reference Example 30

### Derivation of EXPRES Cells from a Suspension of Single Human Embryonic Stem Cells in the Presence of a Rho-Kinase (ROCK) Inhibitor

Cells from the human embryonic stem cell line H9 at passage 35 lines were cultured under hypoxic conditions (approximately 3% O₂) for at least three passages. The cells were cultured in MEF-CM supplemented with 8 ng/ml of bFGF and plated on MATRIGEL coated plates according to **Example 1**. At approximately 60% confluency, the cultures were exposed to TrypLE™ Express solution (Invitrogen, CA) for 5 mins. Released cells were resuspended in DM-F12 + 2% FBS medium, recovered by centrifugation, and counted using a hemocytometer. The released cells were seeded at 1000-10,000 cells/cm² on tissue culture polystyrene (TCPS) flasks and cultured in DM-F12 + 2% FBS + 100 ng/ml activin-A + 20 ng/ml WNT-3A + 50 ng/ml of IGF-I + 0.1 mM mercaptoethanol (Invitrogen, CA), non-essential amino acids (1X, NEAA from Invitrogen, CA) +/- 10 µm ROCK inhibitor (Y-27632, Calbiochem, CA) under hypoxic conditions (approximately 3% O₂) at 37 °C in a standard tissue culture incubator. The TCPS flaks were not coated with MATRIGEL or other extracellular matrix proteins. The media was changed daily. The first passage cells are referred to as P1. As shown in **Figure 37**, 24 hrs after seeding, addition of the ROCK inhibitor resulted in a significantly larger number of attached cells as compared to cultures derived in the absence of the ROCK inhibitor. EXPRES cells derived using the ROCK inhibitor, Y27632 were designated as EXPRES 15.

### Reference Example 31

### Karyotype Analysis

The karyotype of EXPRES 15 cells at passage 5 and 12 were determined by G-band analysis. Cytogenetic analysis was performed on twenty-one G-banded cells from EXPRES 15 all of the cells showed a normal 46XX karyotype (**Figure 38**). FISH analysis of chromosomes 12 and 17 also showed that all cells demonstrated a normal signal pattern for the ETV6 BAP (TEL) gene located on chromosome 12 and all cells demonstrated a normal signal pattern for the Her2/neu gene and 17 centromere on chromosome 17.

### Reference Example 32

### EXPRES Cells can be Maintained in Media Containing a Range of Concentrations of IGF, WNT3A, activin-A, and GSK-3B inhibitors

EXPRES 11 cells were grown in DMEM/F12 (Invitrogen) containing 2% FBS, 100ng/ml activin-A, 20ng/ml Wnt3a and 50ng/ml IGF. At 80% confluency, cells were passed using TrypLE Express (Invitrogen) into 96-well plates at a density of 4000 cells/well in DMEM/F12 containing 2% FBS. Cells were allowed to adhere to the substrate for 1 hour in a humidified incubator held at 37°C with 5% CO2, prior to the addition of activin-A ranging from 50 to 100ng/ml, Wnt3a ranging from 10 to 20ng/ml, IGF ranging from 10 to 50ng/ml, and 50 to 100 nM GSK-3B inhibitor (IX). At 24, 48 and 96 hours, cell viability was determined using the CellTiter® 96 AQueous One Solution Cell Proliferation Assay (Promega). Briefly, MTS reagent was added to the 96-well plates and allowed to incubate with the cells for 1-4 hours and then reading the absorbance at 490nm on a plate reader. The absorbance reading was directly proportional to the number of living cells. **Figure 39 a-c** shows the absorbance readings at a) 24 hrs, b) 48 hrs, and c) 96 hrs post seeding.

**Table IA: List of primary antibodies used for FACS and immuno staining analysis.**

| **Antibody** | **Supplier** | **Isotype** | **Catalog#** |
|---|---|---|---|
| SSEA-1 | Chemicon (CA) | Mouse IgM | MAB4301 |
| SSEA-3 | Chemicon (CA) | Mouse IgG3 | MAB4303 |
| SSEA-4 | Chemicon (CA) | Rat IgM | MAB1435 |
| TRA 1-60 | Chemicon (CA) | Mouse IgM | MAB4360 |
| TRA 1-81 | Chemicon (CA) | Mouse IgM | MAB4381 |
| TRA 1-85 | Chemicon (CA) | Mouse IgG1 | MAB4385 |
| AP | R&D Systems (MN) | Mouse IgG1 | FAB1448A |
| HNF3β | R&D Systems | Goat IgG | AF2400 |
| PDX1 | Santa Cruz Biotechnology, INC (CA) | Goat IgG | sc-14664 |
| GATA4 | R&D Systems | Goat IgG | AF2606 |
| Sox 17 | R&D Systems | Goat IgG | AF 1924 |
| CD 9 | BD Bioscience (CA) | Mouse IgG1 | 341647 |
| CXCR4 | R&D Systems | Mouse IgG2A | FAB170A |
| SOX2 | R&D Systems | Goat IgG | AF2018 |
| Nanog | R&D Systems | Goat IgG | AF1997 |
| OCT4 | R&D Systems | Goat IgG | AF1759 |
| Gata6 | R&D Systems | Goat IgG | AF 1700 |
| Ecad | R&D Systems | Mouse IgG | MAB1838 |
| Ncam | R&D Systems | Mouse IgG | MAB777 |
| HNF1b | R&D Systems | Goat IgG | AF3330 |
| b-catenin | R&D Systems | Mouse IgG | MAB13291 |
| HNF1a | BD Bioscience | Mouse IgG | 610902 |
| CD99 | Invitrogen (CA) | Mouse IgG | 18-0235 |
| Cerebus | Santa Cruz Biotechnology, INC | Goat IgG | SC15131 |
| Hex | Santa Cruz Biotechnology, INC | Goat IgG | SC15129 |
| AFP | R&D Systems | Mouse IgG | MAB1269 |
| Antitrypsin | DAKO (CA) | Rabbit | A0012 |
| islet 1 | R&D Systems | Goat IgG | AF1837 |

**Table IB: List of secondary conjugated antibodies used for FACS and immuno staining analysis.**

| **Secondary Conjugated Antibody** | **Supplier** | **Dilution** |
|---|---|---|
| Goat Anti-Mouse IgG APC conjugated | Jackson ImmunoResearch (PA) | 1:200 |
| Goat Anti-Mouse IgG PE conjugated | Jackson ImmunoResearch (PA) | 1:200 |
| Donkey anti-rabbit PE or - APC conjugated | Jackson ImmunoResearch (PA) | 1:200 |
| Donkey anti-goat PE or - APC conjugated | Jackson ImmunoResearch (PA) | 1:200 |
| Goat anti-mouse IgM PE | SouthernBiotech (AL) | 1:200 |
| Goat anti-Rat IgM PE | SouthernBiotech (AL) | 1:200 |
| Goat anti-mouse IgG3 PE | SouthernBiotech (AL) | 1:200 |

**Table II List of primers used for real-time PCR analysis using TAQMAN®probes**

| **Primer/Probe** | **Catelog Number** |
|---|---|
| 18s | 4310893E |
| ABCG2 | Hs00184979_m1 |
| AchE | Hs00241307_m1 |
| AFP | Hs00173490_m1 |
| ALB | Hs00609411_m1 |
| alpha-antitrypsin | Hs02384981_m1 |
| Amylase | Hs00420710_g1 |
| AP | Hs00240993_m1 |
| Atoh1 | Hs00944192_s1 |
| B₂MG | 4310886E |
| B3Tubulin | Hs00964962_g1 |
| B-Catenin | Hs99999168_m1 |
| Barx1 | Hs00222053_m1 |
| Brachyury (T) | Hs00610080_m1 |
| CCK | Hs00174937_m1 |
| Cdx1 | Hs00156451_m1 |
| Cdx2 | Hs00230919_m1 |
| CEBPa | Hs00269972_s1 |
| CEBPb | Hs00942496_s1 |
| Cerberus | Hs00193796_m1 |
| CFC1 (Cripto) | Hs00414425_m1 |
| CGA | Hs00174938_m1 |
| CK19 (KRT19) | Hs00761767_s1 |
| CLDN4 (claudin4) | Hs00533616_s1 |
| C-Myc Binding Protein | Hs00429315_g1 |
| Connexin32 (GJB-1) | Hs00939759_s1 |
| Connexin45 | Hs00271416_s1 |
| CTNNβ1 | Hs00170025_m1 |
| CXCR4 | Hs00237052_m1 |
| Cylcin-D1 | Hs00277039_m1 |
| Dapper1 (DACT-1) | Hs00420410_m1 |
| DKK1 | Hs00183740_m1 |
| DKK4 | Hs00205290_m1 |
| Endoglin | Hs00164438_m1 |
| Exo1 | Hs00243513_m1 |
| F3 | Hs00175225_m1 |
| FAH | Hs00164611_m1 |
| FGF4 | Hs00173564_m1 |
| FGF10 | Hs00610298_m1 |
| FGFR1 | Hs00241111_m1 |
| FGFR3 | Hs00179839_m1 |
| FGFR4 | Hs00242558_m1 |
| Flk1 | Hs00911705_g1 |
| FOXA1 | Hs00270129_m1 |
| FOXA3 | Hs00270130_m1 |
| FOXD3 | Hs00255287_s1 |
| FOXF1 | Hs00230962_m1 |
| GAPDH (human) | 4310884E |
| Gastrin | Hs00174945_m1 |
| GATA1 | Hs00231112_m1 |
| GATA4 | Hs00171403_m1 |
| GATA5 | Hs00388359_m1 |
| GATA6 | Hs00232018_m1 |
| GCK | Hs00175951_m1 |
| GFAP | Hs00157674_m1 |
| GIP | Hs00175030_m1 |
| Gli | Hs01110766_m1 |
| Glucagon | Hs00174967_m1 |
| Glut-2 | Hs00165775_m1 |
| Goosecoid | Hs00418279_m1 |
| GS | Hs00374213_m1 |
| Handy1 | Hs00231848_m1 |
| HB9 | Hs00232128_m1 |
| Hes1 | Hs00172878_m1 |
| Hex1 | Hs00172696_m1 |
| HEYL | Hs00232718_m1 |
| HHIP (hedgehog interacting protein) | Hs01011009_m1 |
| hHIF1α | Hs00153153_m1 |
| HNF1 | Hs00167041_m1 |
| HNF1α | Hs00167041_m1 |
| HNF1β | Hs00172123_m1 |
| HNP3β | Hs00232764_m1 |
| HNF4α | Hs00230853_m1 |
| HNF6 (onecut) | Hs00413554_m1 |
| HoxB1 | Hs00157973_m1 |
| IBSP | Hs00173720_m1 |
| Insulin II | Hs00355773_m1 |
| Islet-1 | Hs00158126_m1 |
| Jagged-1 (JAG1) | Hs00164982_m1 |
| KDR | Hs00176676_m1 |
| KRT15 | Hs00267035_m1 |
| MafA | Hs00999118_m1 |
| MAML1 | Hs00207373_m1 |
| Map2 | Hs00159041_m1 |
| MapK14 | Hs00176247_m1 |
| MapK8 | Hs00177083_m1 |
| MBP (myelin basic protein) | Hs00921945_m1 |
| Mixl1 | Hs00430824_g1 |
| MMP1 (matrix metalloprotease1) | Hs00233958_m1 |
| MOX1 | Hs00793059_m1 |
| MSX1 | Hs00427183_m1 |
| Mucin1 | Hs00904328_m1 |
| Mucin2 | Hs00159374_m1 |
| Myf5 | Hs00271574_m1 |
| MyoD1 | Hs00159528_m1 |
| N-Cadherin (CDH2) | Hs00169953_m1 |
| N-Cam1 | Hs00169851_m1 |
| NEFH | Hs00606024_m1 |
| NEFL | Hs00196245_m1 |
| Nestin | Hs00707120_s1 |
| NeuroD1 | Hs00159598_m1 |
| Ngn3 | Hs00360700_q1 |
| Nkx2.1 | Hs00163037_m1 |
| Nkx2.2 | Hs00159616_m1 |
| Nkx6.1 | Hs00232355_m1 |
| Notch1 | Hs01062014_m1 |
| NTS | Hs00175048_m1 |
| O₂ | Hs00377820_m1 |
| Oct3/4 | Hs00742896_s1 |
| OTX2 | Hs00222238_m1 |
| Pax3 | Hs00240950_m1 |
| Pax4 | Hs00173014_m1 |
| Pax6 | Hs00240871_m1 |
| Pax8 | Hs00247586_m1 |
| Pax9 | Hs00196354_m1 |
| PDX-1 (IPF1) | Hs00236830_m1 |
| Ptch (Patched) | Hs00970985_m1 |
| PTF1a | Hs00603586_q1 |
| PYY | Hs01062281_m1 |
| RA receptor alpha | Hs00230907_m1 |
| RALDH | Hs01125173_m1 |
| RBPSUH | Hs00794653_m1 |
| Rex1 (ZFP42) | Hs00399279_m1 |
| SCGB1 | Hs00171092_m1 |
| Secretin | Hs00360814_q1 |
| sFRP | Hs00610060_m1 |
| sFRP5 | Hs00169366_m1 |
| SHH | Hs00179843_m1 |
| SLC5A8 | Hs01068911_m1 |
| Snai1 | Hs00195591_m1 |
| Snai2 | Hs00161904_m1 |
| Somatostatin | Hs00174949_m1 |
| Sox1 | Hs00534426_s1 |
| Sox10 | Hs00366918_m1 |
| Sox17 | see "Sox17" tab |
| Sox2 | Hs00602736_s1 |
| Sox3 | Hs00271627_s1 |
| Sox7 | Hs00846731_s1 |
| Sox9 | Hs00165814_m1 |
| Sparc | Hs00234160_m1 |
| SP-C | Hs00161628_m1 |
| TBX6 | Hs00365539_m1 |
| Tert | Hs00162669_m1 |
| THBD | Hs00264920_s1 |
| Twist1 | Hs00361186_m1 |
| UtF1 | Hs00747497_q1 |
| Vim | Hs00185584_m1 |
| Wnt3a (human) | Hs01055707_m1 |
| Wnt3a (mouse) | Mm00437337_m1 |
| WT1 | Hs01103754_m1 |
| Zic1 | Hs00604749_m1 |
| Zic2 | Hs00600845_m1 |

**Table III List of EXPRES lines generated from H1 & H9 parent lines**

| ID# | ES Parent line, Passage number | Day of derivation during DE differentiation | Media used to propagate cells |
|---|---|---|---|
| EXPRES 01 | H9P54 | Day 4 | Growth media + 50 ng/ml IGF-I |
| EXPRES 02 | H9P54 | Day 6 | DM-F12-+ 2% FBS +100 ng/ml AA +20 ng/ml WNT-3A (growth media) |
| EXPRES 03 | H9P39 | Day 3 | Growth media + 50 ng/ml IGF-I |
| EXPRES 04 | H9P39 | Day 4 | Growth media + 50 ng/ml IGF-I |
| EXPRES 05 | H9P39 | Day 5 | Growth media + 50 ng/ml IGF-I |
| EXPRES 06 | H9P49 | Day 5 | Growth media |
| EXPRES 07 | H1P50 | Day 5 | Growth media |
| EXPRES 08 | H1P39 | Day 4 | Growth media + 50 ng/ml IGF-I + 0.1mM mercaptoethanol |
| EXPRES 09 | H1P39 | Day 6 | Growth media + 50 ng/ml IGF-I + 0.1mM mercaptoethanol |
| EXPRES 10 | H1P49 | Day 4 | Growth media |
| EXPRES 11 | H9P30 | Day 4 | Growth media + 50 ng/ml IGF-I + 0.1mM mercaptoethanol |
| EXPRES 12 | H9P30 | Day 6 | Growth media + 50 ng/ml IGF-I + 0.1mM mercaptoethanol |

**Table IV Expression of cell surface markers by EXPRES 01 and 02 lines**

| Marker | EXPRES 01- % Expression | EXPRES 02-% Expression |
|---|---|---|
| TRA 1-60 | 100 | 100 |
| TRA1-81 | 100 | 100 |
| SSEA-3 | 100 | 86 (85-88) |
| SSEA-4 | 100 | 87 (83-88) |
| CD56 (NCAM) | <1 | 38 (35-40) |
| CXCR4 | <1 | 20 (10-25) |
| SSEA-1 | <1 | 26 (9-32) |
| E-cadherin | 90 (85-95) | 75 (72-79) |
| CD9 | 99 | 66 (62-68) |
| CD30 | 97 (96-98) | 43 (40-46) |
| LIF-receptor | <1 | 20 (15-21) |
| CD117 | 92 (90-95) | 73 (67-75) |

**Table V DIFFERENTIAL EXPRESSION OF GENES BETWEEN UNDIFFERENTIATED EMBRYONIC STEM CELLS CULTURED ON MATRIGEL™ AND EXPRES 01 (A) AND 02 (B) CELLS CULTURED ON TCPS**

| **A) ES vs EXPRES 01-Intensity values are in Log2 format.** | | | | | | |
|---|---|---|---|---|---|---|
| ES | EXPRES 01 | Ratio | Direction | adj. p-value | Gene Identifier | Gene Name |
| -5.03662 | 3.037629 | 269.52 | Up | 8.34E-05 | BG099432 | ESTs |
| -2.82645 | 3.094183 | 60.57 | Up | 3.88E-03 | NM_013445 | Homo sapiens glutamate decarboxylase 1 (brain, 67kD) (GAD1), transcript variant GAD25, mRNA. /PROD=glutamate decarboxylase 1, isoform GAD25 /FL=gb:NM_013445.1 gb:AF178853.1 gb:BC002815.1 |
| -4.71714 | 0.961816 | 51.23 | Up | 6.59E-05 | AI796169 | GATA-binding protein 3 /FL=gb:NM_002051.1 gb:M69106.1 gb:BC003070.1 |
| -1.57973 | 4.092731 | 51 | Up | 6.13E-03 | AL513917 | solute carrier family 16 (monocarboxylic acid transporters), member 3 /FL=gb:U81800.1 gb:NM_004207.1 |
| -2.24566 | 3.409815 | 50.4 | Up | 1.19E-03 | NM_016588 | Homo sapiens neuritin (LOC51299), mRNA. /PROD=neuritin /FL=gb:NM_016588.1 gb:BC002683.1 gb:AF136631.1 |
| -0.66473 | 4.897427 | 47.25 | Up | 1.97E-04 | R06655 | ESTs, Moderately similar to AF078844 1 hqp0376 protein (H.sapiens) |
| -2.96204 | 2.589425 | 46.9 | Up | 1.79E-03 | NM_001311 | Homo sapiens cysteine-rich protein 1 (intestinal) (CRIP1), mRNA. /PROD=cysteine-rich protein 1 (intestinal) /FL=gb:U58630.1 gb:BC002738.1 gb:NM_001311.1 gb:U09770.1 |
| -0.08298 | 5.388254 | 44.36 | Up | 2.48E-04 | NM_004207 | Homo sapiens solute carrier family 16 (monocarboxylic acid transporters), member 3 (SLC16A3), mRNA. /PROD=solute carrier family 16 (monocarboxylic acidtransporters), member 3/FL=gb:U81800.1 gb:NM_004207.1 |
| -4.21525 | 1.240929 | 43.9 | Up | 4.36E-04 | NM_006119 | Homo sapiens fibroblast growth factor 8 (androgen-induced) (FGF8), mRNA. /PROD=fibroblast growth factor 8 (androgen-induced) /FL=gb:U36223.1 |
| | | | | | | gb:U46212.1 |
| | | | | | | gb:NM_006119.1 |
| -3.11273 | 2.306225 | 42.78 | Up | 5.07E-03 | AW444761 | ESTs |
| -4.05111 | 1.075159 | 34.93 | Up | 1.19E-03 | J03580 | Human, parathyroid-like protein (associated with humoral hypercalcemia of malignancy) mRNA, complete cds. /FL=gb:J03580.1 |
| -1.81534 | 3.025351 | 28.65 | Up | 7.00E-04 | AW590925 | ESTs |
| -2.36335 | 2.448218 | 28.08 | Up | 1.62E-02 | AI189359 | mevalonate (diphospho) decarboxylase /FL=gb:NM_002461.1 |
| | | | | | | gb:BC000011.1 |
| | | | | | | gb:U49260.1 |
| -3.00328 | 1.794842 | 27.82 | Up | 7.77E-03 | AI571798 | Rho GDP dissociation inhibitor (GDI) alpha |
| -4.17258 | 0.510307 | 25.69 | Up | 2.29E-04 | AI040887 | ESTs |
| -0.11332 | 4.420733 | 23.17 | Up | 8.78E-05 | NM_003670 | Homo sapiens basic helix-loop-helix domain containing, class B, 2 (BHLHB2), mRNA. /PROD=differentiated embryo chondrocyte expressed gene1 /FL=gb:AB004066.1 gb:NM_003670.1 |
| -1.94 | 2.590871 | 23.12 | Up | 3.90E-03 | NM_016569 | Homo sapiens TBX3-iso protein (TBX3-iso), mRNA. /PROD=TBX3-iso protein /FL=gb:NM_016569.1 gb:AF216750.1 |
| -2.18113 | 2.33099 | 22.82 | Up | 1.12E-02 | NM_004041 | Homo sapiens arrestin, beta 1 (ARRB1), transcript variant 1, mRNA. /PROD=arrestin beta 1, isoform A /FL=gb:BC003636.1 |
| | | | | | | gb:AF084040.1 |
| | | | | | | gb:NM_004041.2 |
| -2.85963 | 1.609384 | 22.15 | Up | 6.37E-03 | BC005961 | Homo sapiens, parathyroid hormone-like hormone, clone MGC:14611, mRNA, complete cds. /PROD=parathyroid hormone-like hormone /FL=gb:BC005961.1 |
| 0.044298 | 4.505436 | 22.03 | Up | 4.17E-05 | W57613 | ESTs |
| -3.65051 | 0.757197 | 21.23 | Up | 7.11E-04 | AF213459 | Homo sapiens ephrin receptor EPHA3 complete form (EPHA3) mRNA, complete cds. /PROD=ephrin receptor EPHA3 complete form /FL=gb:NM_005233.1 |
| | | | | | | gb:M83941.1 |
| | | | | | | gb:AF213459.1 |
| -1.25226 | 2.990178 | 18.93 | Up | 2.70E-04 | NM_013445 | Homo sapiens glutamate decarboxylase 1 (brain, 67kD) (GAD1), transcript variant GAD25, mRNA. /PROD=glutamate decarboxylase 1, isoform GAD25 /FL=gb:NM_013445.1 |
| | | | | | | gb:AF178853.1 |
| | | | | | | gb:BC002815.1 |
| -0.63516 | 3.41866 | 16.61 | Up | 1.05E-03 | AA149250 | ESTs, Weakly similar to WDNM RAT WDNM1 PROTEIN PRECURSOR (R.norvegicus) |
| -0.14916 | 3.880272 | 16.33 | Up | 8.45E-04 | NM_002521 | Homo sapiens natriuretic peptide precursor B (NPPB), mRNA. /PROD=natriuretic peptide precursor B /FL=gb:NM_002521.1 gb:M25296.1 |
| -2.88485 | 1.143234 | 16.31 | Up | 2.83E-03 | BF437711 | ESTs |
| -2.7136 | 1.194285 | 15.01 | Up | 2.14E-02 | NM_001898 | Homo sapiens cystatin SN (CST1), mRNA. /PROD=cystatin SN /FL=gb:J03870.1 gb:NM_001898.1 |
| -0.07353 | 3.816817 | 14.83 | Up | 1.62E-02 | AF116616 | Homo sapiens PRO0998 mRNA, complete cds. /PROD=PRO0998 /FL=gb:AF116616.1 |
| -1.44577 | 2.422113 | 14.6 | Up | 1.34E-02 | AL544576 | ESTs |
| 2.301255 | 6.091008 | 13.83 | Up | 6.50E-05 | BC020935 | Homo sapiens, Similar to otoconin 90, clone IMAGE:4277593, mRNA. |
| 3.006559 | 6.79047 | 13.77 | Up | 8.80E-05 | AI263909 | ras homolog gene family, member B /FL=gb:NM_004040.1 |
| -0.512 | 3.268087 | 13.74 | Up | 2.96E-03 | AF345910 | Homo sapiens NYD-SP14 mRNA, complete cds. /PROD=NYD-SP14 /FL=gb:AF345910.1 |
| 3.484047 | 7.24746 | 13.58 | Up | 4.12E-04 | NM_003564 | Homo sapiens transgelin 2 (TAGLN2), mRNA. /PROD=transgelin 2 /FL=gb:D21261.1 |
| | | | | | | gb:NM_003564.1 |
| **2.623145** | **6.382732** | **13.54** | **Up** | **7.32E-05** | **AI050866** | **nodal, mouse, homolog** |
| 2.91184 | 6.66195 | 13.46 | Up | 1.44E-04 | BC002616 | Homo sapiens, transgelin 2, clone MGC:2989, mRNA, complete cds. /PROD=transgelin 2 /FL=gb:BC002616.1 |
| -1.18027 | 2.546953 | 13.24 | Up | 1.42E-02 | AI123555 | ESTs |
| -0.92213 | 2.770179 | 12.93 | Up | 1.08E-04 | AK000345 | Homo sapiens cDNA FLJ20338 fis, clone HEP12179. |
| -1.85154 | 1.840613 | 12.93 | Up | 6.40E-03 | AI949760 | ESTs, Weakly similar to unnamed protein product (H.sapiens) |
| 0.899321 | 4.507212 | 12.19 | Up | 1.08E-04 | AW196940 | ESTs |
| 1.651788 | 5.252439 | 12.13 | Up | 5.58E-05 | NM_001553 | Homo sapiens insulin-like growth factor binding protein 7 (IGFBP7), mRNA. /PROD=insulin-like growth factor binding protein 7 /FL=gb:NM_001553.1 gb:L 19182.1 |
| -1.20347 | 2.387268 | 12.05 | Up | 2.76E-02 | BF063186 | ESTs |
| 3.669215 | 7.251816 | 11.98 | Up | 2.01E-05 | NM_000700 | Homo sapiens annexin A1 (ANXA1), mRNA. /PROD=annexin I /FL=gb:BC001275.1 gb:NM_000700.1 |
| -3.52039 | 0.041471 | 11.81 | Up | 1.58E-02 | AW162210 | Homo sapiens cDNA FLJ11490 fis, clone HEMBA1001918 |
| 3.006317 | 6.564471 | 11.78 | Up | 2.04E-04 | NM_006183 | Homo sapiens neurotensin (NTS), mRNA. /PROD=neurotensin precursor /FL=gb:NM_006183.2 gb:U91618.1 |
| 3.227575 | 6.777916 | 11.72 | Up | 2.01E-05 | U15174 | Homo sapiens BCL2adenovirus E1B 19kD-interacting protein 3 (BNIP3) mRNA, complete cds. /PROD=BCL2adenovirus E1B 19kD-interacting protein 3 /FL=gb:AF002697.1 gb:NM_004052.2 gb:U15174.1 |
| 1.869893 | 5.411751 | 11.65 | Up | 2.01E-05 | NM_001854 | Homo sapiens collagen, type XI, alpha 1 (COL11A1), mRNA. /PROD=collagen, type XI, alpha 1 /FL=gb:J04177.1 gb:NM_001854.1 |
| 2.851479 | 6.371229 | 11.47 | Up | 3.61E-05 | AI950472 | ESTs |
| -0.1081 | 3.393761 | 11.33 | Up | 1.39E-03 | BG028597 | ESTs |
| 0.41954 | 3.911282 | 11.25 | Up | 1.42E-04 | AI670948 | ESTs |
| -1.71451 | 1.771584 | 11.21 | Up | 7.77E-03 | BI254089 | Homo sapiens full length insert cDNA clone ZD50E03 |
| -1.66293 | 1.80547 | 11.07 | Up | 2.68E-02 | NM_000817 | Homo sapiens glutamate decarboxylase 1 (brain, 67kD) (GAD1), transcript variant GAD67, mRNA. /PROD=glutamate decarboxylase 1, isoform GAD67 /FL=gb:NM_000817.1 gb:M81883.1 gb:L16888.1 |
| 0.451086 | 3.904705 | 10.96 | Up | 5.45E-04 | NM_007038 | Homo sapiens a disintegrin-like and metalloprotease (reprolysin type) with thrombospondin type 1 motif, 5 (aggrecanase-2) (ADAMTS5), mRNA. /PROD=a disintegrin and metalloprotease withthrombospondin motifs-5 preproprotein /FL=gb:NM_007038.1 gb:AF14209 |
| -1.44798 | 1.988376 | 10.83 | Up | 1.07E-02 | AW376860 | ESTs |
| 1.19822 | 4.633038 | 10.81 | Up | 1.22E-04 | NM_016931 | Homo sapiens NADPH oxidase 4 (NOX4), mRNA. /PROD=NADPH oxidase 4 /FL=gb:AF261943.1 gb:NM_016931.1 gb:AF254621.1 gb:AB041035.1 |
| 1.552808 | 4.981033 | 10.76 | Up | 3.24E-04 | NM_000602 | Homo sapiens serine (or cysteine) proteinase inhibitor, clade E (nexin, plasminogen activator inhibitor type 1), member 1 (SERPINE1), mRNA. /PROD=serine (or cysteine) proteinase inhibitor, cladeE (nexin, plasminogen activator inhibitor type 1), membe |
| -0.62725 | 2.792215 | 10.7 | Up | 3.24E-04 | BC017942 | Homo sapiens, Similar to otoconin 90, clone IMAGE:4285317, mRNA. |
| -1.22834 | 2.18828 | 10.68 | Up | 2.50E-02 | BG402859 | ESTs |
| -0.85366 | 2.55067 | 10.59 | Up | 3.53E-04 | N36408 | hypothetical protein FLJ23306 /FL=gb:NM_024530.1 |
| 1.55625 | 4.952105 | 10.53 | Up | 1.52E-03 | BG164365 | microtubule-associated protein 1B /FL=gb:NM_005909.1 |
| 0.93094 | 4.285883 | 10.23 | Up | 4.17E-05 | AL038787 | 6-phosphofructo-2-kinasefructose-2,6-biphosphatase 4 |
| -3.33551 | 0.008356 | 10.15 | Up | 9.88E-03 | BF221850 | ESTs |
| -1.97569 | 1.35098 | 10.03 | Up | 2.12E-02 | NM_002149 | Homo sapiens hippocalcin-like 1 (HPCAL1), mRNA. /PROD=hippocalcin-like 1 /FL=gb:NM_002149.1 gb:D16227.1 |
| 0.624024 | 3.934756 | 9.92 | Up | 2.01 E-05 | AF154054 | Homo sapiens DRM (DRM) mRNA, complete cds. /PROD=DRM /FL=gb:NM_013372.1 gb:AF110137.2 gb:AF045800.1 gb:AF154054.1 |
| -2.37515 | 0.931587 | 9.9 | Up | 2.83E-02 | NM_025136 | Homo sapiens hypothetical protein FLJ22187 (FLJ22187), mRNA. /PROD=hypothetical protein FLJ22187 /FL=gb:BC005059.1 gb:NM_025136.1 |
| 0.906004 | 4.154799 | 9.51 | Up | 1.45E-04 | NM_013372 | Homo sapiens cysteine knot superfamily 1, BMP antagonist 1 (CKTSF1B1), mRNA. /PROD=cysteine knot superfamily 1, BMP antagonist 1 /FL=gb:NM_013372.1 gb:AF110137.2 gb:AF045800.1 gb:AF154054.1 |
| 1.423659 | 4.64236 | 9.31 | Up | 2.01 E-05 | AB019695 | Homo sapiens mRNA for thioredoxin reductase II beta, complete cds. /PROD=thioredoxin reductase II beta /FL=gb:AB019695.1 |
| 0.620478 | 3.822765 | 9.2 | Up | 6.53E-05 | AL567376 | G protein-coupled receptor 39 |
| 2.317589 | 5.509474 | 9.14 | Up | 1.02E-05 | J04177 | Cluster Incl. J04177:Human alpha-1 type XI collagen (COL11A1) mRNA, complete cds /cds=(161,5581) /gb=J04177/gi=179729 /ug=Hs.82772 /len=6158 |
| -0.01596 | 3.175621 | 9.14 | Up | 9.88E-03 | L37033 | Cluster Incl. L37033:Human FK-506 binding protein homologue (FKBP38) mRNA, complete cds /cds=(140,1207) /gb=L37033 /gi=965469 /ug=Hs.173464 /len=1613 |
| 3.119777 | 6.302535 | 9.08 | Up | 5.07E-03 | AI954041 | F-box only protein 9 /FL=gb:NM_012347.1 |
| 0.891337 | 4.058968 | 8.99 | Up | 1.66E-04 | NM_ 000325 | Homo sapiens paired-like homeodomain transcription factor 2 (PITX2), mRNA. /PROD=paired-like homeodomain transcription factor 2 /FL=gb:NM_000325.1 gb:U69961.1 gb:AF048720.1 |
| 2.106895 | 5.247359 | 8.82 | Up | 2.13E-04 | NM_001458 | Homo sapiens filamin C, gamma (actin-binding protein-280) (FLNC), mRNA. /PROD=gamma filamin /FL=gb:AF089841.1 gb:NM_001458.1 |
| -1.42687 | 1.693258 | 8.69 | Up | 2.43E-02 | AF243424 | Homo sapiens SG2NA beta isoform mRNA, partial cds. /PROD=SG2NA beta isoform |
| 0.657996 | 3.771559 | 8.66 | Up | 2.06E-02 | AU158380 | Homo sapiens cDNA FLJ13698 fis, clone PLACE2000176 |
| 0.23287 | 3.334996 | 8.59 | Up | 2.01E-05 | R40917 | phosphodiesterase 4D, cAMP-specific (dunce (Drosophila)-homolog phosphodiesterase E3) /FL=gb:L20969.1 gb:NM_006203.1 gb:U02882.1 |
| -0.06159 | 3.027095 | 8.51 | Up | 3.60E-04 | U16797 | Human LERK-5 (EPLG5) mRNA, complete cds. /PROD=LERK-5 /FL=gb:U16797.1 gb:NM_004093.1 gb:L38734.1 gb:U81262.1 |
| 4.07051 | 7.149479 | 8.45 | Up | 4.86E-05 | M10943 | Human metallothionein-If gene (hMT-If) |
| -0.35204 | 2.705807 | 8.33 | Up | 1.09E-03 | BC004490 | Homo sapiens, v-fos FBJ murine osteosarcoma viral oncogene homolog, clone MGC:11074, mRNA, complete cds. /PROD=v-fos FBJ murine osteosarcoma viral oncogenehomolog /FL=gb:NM_005252.2 gb:BC004490.1 |
| -2.0132 | 1.043139 | 8.32 | Up | 3.61E-02 | BE328496 | hypothetical protein PRO2032 /FL=gb:AF116683.1 gb:NM_018615.1 |
| -0.68954 | 2.36447 | 8.31 | Up | 8.94E-04 | NM_005181 | Homo sapiens carbonic anhydrase III, muscle specific (CA3), mRNA. /PROD=carbonic anhydrase III /FL=gb:BC004897.1 gb:NM_005181.2 |
| -0.00953 | 3.031935 | 8.23 | Up | 9.88E-03 | AA831438 | Mad4 homolog |
| 3.079271 | 6.1137 | 8.19 | Up | 4.17E-05 | NM_001553 | Homo sapiens insulin-like growth factor binding protein 7 (IGFBP7), mRNA. /PROD=insulin-like growth factor binding protein 7 /FL=gb:NM_001553.1 gb:L 19182.1 |
| 0.788995 | 3.806044 | 8.1 | Up | 6.41 E-05 | AV734646 | DNA segment on chromosome X (unique) 9928 expressed sequence |
| 0.9926 | 4.004698 | 8.07 | Up | 8.48E-05 | Al202327 | ESTs |
| 0.051719 | 3.059444 | 8.04 | Up | 6.98E-04 | NM_ 007350 | Homo sapiens pleckstrin homology-like domain, family A, member 1 (PHLDA1), mRNA. /PROD=pleckstrin homology-like domain, family A,member 1 /FL=gb:NM_007350.1 |
| -1.69103 | 1.312148 | 8.02 | Up | 1.82E-03 | U32500 | Human type 2 neuropeptide Y receptor mRNA, complete cds. /PROD=type 2 neuropeptide Y receptor /FL=gb:U42766.1 gb:U36269.1 gb:U32500.1 |
| -0.93931 | 2.060071 | 8 | Up | 6.51 E-03 | AA004300 | hypothetical protein DKFZp566I133 |
| 1.164666 | 4.161028 | 7.98 | Up | 2.51E-03 | BC005807 | Homo sapiens, clone MGC:10264, mRNA, complete cds. /PROD=Unknown (protein for MGC:10264) /FL=gb:BC005807.1 |
| 2.462987 | 5.441963 | 7.88 | Up | 1.45E-04 | NM_016651 | Homo sapiens heptacellular carcinoma novel gene-3 protein (LOC51339), mRNA. /PROD=heptacellular carcinoma novel gene-3 protein /FL=gb:NM_016651.2 gb:AF251079.2 |
| 0.581498 | 3.560033 | 7.88 | Up | 1.07E-04 | U38945 | Human hypothetical 18.1 kDa protein (CDKN2A) mRNA, complete cds. /FL=gb:U38945.1 gb:U26727.1 |
| 0.470814 | 3.417829 | 7.71 | Up | 8.26E-03 | AF176039 | Homo sapiens high mobility group protein-R mRNA, complete cds. /PROD=high mobility group protein-R /FL=gb:AF176039.1 |
| -1.50663 | 1.438639 | 7.7 | Up | 7.38E-04 | BF223214 | ESTs |
| -2.43082 | 0.495104 | 7.6 | Up | 1.71E-02 | BF508288 | ESTs |
| 1.084464 | 4.009912 | 7.6 | Up | 1.16E-03 | BC004865 | Homo sapiens, cleft lip and palate associated transmembrane protein 1, clone MGC:10593, mRNA, complete cds. /PROD=cleft lip and palate associated transmembraneprotein 1 /FL=gb:BC004865.1 |
| 4.232374 | 7.152167 | 7.57 | Up | 1.54E-03 | BF971587 | tubulin, beta polypeptide /FL=gb:BC001352.1 |
| **0.129315** | **3.042634** | **7.53** | **Up** | **2.17E-04** | **NM_005442** | **Homo sapiens eomesodermin (Xenopus laevis) homolog (EOMES), mRNA. /PROD=eomesodermin (Xenopus laevis) homolog /FL=gb:AB031038.1 gb:NM_005442.1** |
| -0.20075 | 2.710846 | 7.52 | Up | 2.58E-02 | NM_003377 | Homo sapiens vascular endothelial growth factor B (VEGFB), mRNA. /PROD=vascular endothelial growth factor B /FL=gb:NM_003377.1 gb:U43368.1 gb:U52819.1 |
| -0.45681 | 2.439084 | 7.44 | Up | 7.82E-03 | AI417362 | ESTs, Moderately similar to ALU1_HUMAN ALU SUBFAMILY J SEQUENCE CONTAMINATION WARNING ENTRY (H.sapiens) |
| 0.155309 | 3.037724 | 7.37 | Up | 8.78E-04 | NM_007061 | Homo sapiens serum constituent protein (MSE55), mRNA. /PROD=serum constituent protein /FL=gb:M88338.1 gb:NM_007061.1 |
| 1.506057 | 4.362244 | 7.24 | Up | 7.30E-05 | NM_001425 | Homo sapiens epithelial membrane protein 3 (EMP3), mRNA. /PROD=epithelial membrane protein 3 /FL=gb:U52101.1 gb:NM_001425.1 gb:U87947.1 |
| -1.75194 | 1.091177 | 7.18 Up | | 3.15E-02 | NM_021570 | Homo sapiens BarH-like homeobox 1 (BARX1), mRNA. /PROD=BarH-like homeobox 1 /FL=gb:NM_021570.2 gb:AF213356.1 |
| 2.455614 | 5.294065 | 7.15 | Up | 9.88E-03 | AF279899 | Homo sapiens PNAS-145 mRNA, complete cds. /PROD=PNAS-145 /FL=gb:U03105.1 gb:NM_006813.1 gb:AF279899.1 |
| 1.422626 | 4.257559 | 7.14 | Up | 2.01E-05 | NM_006365 | Homo sapiens transcriptional activator of the c-fos promoter (CROC4), mRNA. /PROD=transcriptional activator of the c-fos promoter /FL=gb:NM_006365.1 gb:U49857.1 |
| -2.40588 | 0.424388 | 7.11 | Up | 1.64E-02 | NM_002433 | Homo sapiens myelin oligodendrocyte glycoprotein (MOG), mRNA. /PROD=myelin oligodendrocyte glycoprotein /FL=gb:U18798.1 |
| | | | | | | gb:U64564.1 |
| | | | | | | gb:NM_002433.1 |
| -1.86904 | 0.959227 | 7.1 | Up | 4.35E-02 | AB033831 | Homo sapiens hSCDGF mRNA for spinal cord-derived growth factor, complete cds. /PROD=spinal cord-derived growth factor /FL=gb:NM_016205.1 |
| | | | | | | gb:AB033831.1 |
| | | | | | | gb:AF091434.1 |
| | | | | | | gb:AF244813.1 |
| 1.811736 | 4.639749 | 7.1 | Up | 1.09E-03 | M27830 | Human 28S ribosomal RNA gene, complete cds. |
| -0.54131 | 2.28595 | 7.1 | Up | 3.63E-03 | BE504838 | ESTs |
| 4.065536 | 6.887587 | 7.07 | Up | 4.08E-04 | NM_005796 | Homo sapiens nuclear transport factor 2 (placental protein 15) (PP15), mRNA. /PROD=nuclear transport factor 2 (placental protein 15) /FL=gb:BC002348.1 gb:NM_005796.1 gb:U43939.1 |
| -0.4348 | 2.379408 | 7.03 | Up | 4.24E-03 | BF686824 | death-associated protein kinase 3 /FL=gb:AB007144.1 |
| | | | | | | gb:AB022341.1 |
| | | | | | | gb:NM_001348.1 |
| 1.448288 | 4.255531 | 7 | Up | 2.01E-05 | AF096296 | Homo sapiens thymic stroma chemokine-1 precursor, mRNA, complete cds. /PROD=thymic stroma chemokine-1 precursor /FL=gb:AF142434.1 |
| | | | | | | gb:AF096296.1 |
| | | | | | | gb:AF124601.1 |
| | | | | | | gb:AB010447.1 |
| | | | | | | gb:NM_006072.1 |
| 3.504052 | 6.307554 | 6.98 | Up | 4.05E-05 | NM_006622 | Homo sapiens serum-inducible kinase (SNK), mRNA. /PROD=serum-inducible kinase /FL=gb:AF059617.1 |
| | | | | | | gb:NM_006622.1 |
| | | | | | | gb:AF223574.1 |
| 2.124541 | 4.927935 | 6.98 | Up | 8.80E-05 | J05008 | Homo sapiens endothelin-1 (EDN1) gene, complete cds /FL=gb:NM_001955.1 |
| 1.872394 | 4.674078 | 6.97 | Up | 1.02E-05 | AK022852 | Homo sapiens cDNA FLJ12790 fis, clone NT2RP2001985, weakly similar to Homo sapiens high-risk human papilloma viruses E6 oncoproteins targeted protein E6TP1 alpha mRNA. |
| 1.764338 | 4.563535 | 6.96 | Up | 1.43E-04 | AA909044 | tryptase, alpha |
| 1.401293 | 4.178686 | 6.86 | Up | 1.66E-04 | NM_003256 | Homo sapiens tissue inhibitor of metalloproteinase 4 (TIMP4), mRNA. /PROD=tissue inhibitor of metalloproteinase 4precursor /FL=gb:NM_003256.1 gb:U76456.1 |
| 0.767075 | 3.54446 | 6.86 | Up | 1.88E-03 | AY029208 | Homo sapiens type VI collagen alpha 2 chain precursor (COL6A2) mRNA, complete cds, alternatively spliced. /PROD=type VI collagen alpha 2 chain precursor /FL=gb:AY029208.1 |
| -1.94468 | 0.831091 | 6.85 | Up | 1.92E-02 | NM_153036 | Homo sapiens hypothetical protein FLJ32239 (FLJ32239), mRNA. /FL=gb:NM_153036.1 |
| 0.586923 | 3.359675 | 6.83 | Up | 4.17E-05 | NM_000047 | Homo sapiens arylsulfatase E (chondrodysplasia punctata 1) (ARSE), mRNA. /PROD=arylsulfatase E precursor /FL=gb:X83573.1 gb:NM_000047.1 |
| 1.064878 | 3.834572 | 6.82 | Up | 2.41E-04 | NM_002610 | Homo sapiens pyruvate dehydrogenase kinase, isoenzyme 1 (PDK1), nuclear gene encoding mitochondrial protein, mRNA. /PROD=pyruvate dehydrogenase kinase, isoenzyme 1 /FL=gb:NM_002610.2 gb:L42450.1 |
| -0.11882 | 2.64743 | 6.8 | Up | 6.88E-04 | AI702438 | ESTs |
| -0.34208 | 2.418198 | 6.78 | Up | 2.31 E-04 | AI860150 | ESTs, Weakly similar to A49134 Ig kappa chain V-I region (H.sapiens) |
| 4.156522 | 6.9125 | 6.76 | Up | 6.48E-05 | NM_003240 | Homo sapiens endometrial bleeding associated factor (left-right determination, factor A; transforming growth factor beta superfamily) (EBAF), mRNA. /PROD=transforming growth factor, beta 4 /FL=gb:U81523.1 |
| | | | | | | gb:NM_003240.1 |
| | | | | | | gb:AF081513.1 |
| -1.8911 | 0.860193 | 6.73 | Up | 2.83E-03 | NM_001191 | Homo sapiens BCL2-like 1 (BCL2L1), mRNA. /PROD=BCL2-like 1 /FL=gb:NM_001191.1 |
| -0.53771 | 2.208449 | 6.71 | Up | 1.41E-02 | AI806338 | T-box 3 (ulnar mammary syndrome) /FL=gb:AF170708.2 gb:NM_005996.1 |
| 3.824397 | 6.568635 | 6.7 | Up | 1.66E-04 | AL031602 | Human DNA sequence from clone RP5-1174N9 on chromosome 1p34.1-35.3. Contains the gene for a novel protein with IBR domain, a (pseudo?) gene for a novel protein similar to MT1E (metallothionein 1E (functional)), ESTs, STSs, GSSs and two putative Cp... |
| 0.001822 | 2.741586 | 6.68 | Up | 4.44E-05 | NM_004904 | Homo sapiens cAMP response element-binding protein CRE-BPa (H_GS165L15.1), mRNA. /PROD=cAMP response element-binding protein CRE-BPa /FL=gb:NM_004904.1 gb:L05911.1 |
| 0.301216 | 3.030236 | 6.63 | Up | 4.76E-04 | AA812232 | upregulated by 1,25-dihydroxyvitamin D-3 /FL=gb:NM_006472.1 gb:S73591.1 |
| 4.63387 | 7.360687 | 6.62 | Up | 4.17E-05 | BF217861 | metallothionein 1E (functional) |
| 0.413191 | 3.124723 | 6.55 | Up | 5.00E-02 | AL136925 | Homo sapiens mRNA; cDNA DKFZp586H1320 (from clone DKFZp586H1320); complete cds. /PROD=hypothetical protein /FL=gb:AL136925.1 |
| -0.28056 | 2.427947 | 6.54 | Up | 1.39E-03 | AW090187 | dihydropyrimidinase-like 4 /FL=gb:NM_006426.1 gb:AB006713.1 |
| 1.095892 | 3.804014 | 6.53 | Up | 2.43E-04 | NM_001146 | Homo sapiens angiopoietin 1 (ANGPT1), mRNA. /PROD=angiopoietin 1 /FL=gb:NM_001146.1 gb:D13628.1 gb:U83508.1 |
| -0.0845 | 2.62025 | 6.52 | Up | 2.59E-04 | AA625683 | ESTs |
| 2.13274 | 4.835771 | 6.51 | Up | 2.01E-04 | AL574210 | serine (or cysteine) proteinase inhibitor, clade E (nexin, plasminogen activator inhibitor type 1), member 1 /FL=gb:NM_000602.1 gb:M16006.1 |
| -0.64252 | 2.055951 | 6.49 | Up | 2.10E-04 | NM_001035 | Homo sapiens ryanodine receptor 2 (cardiac) (RYR2), mRNA. /PROD=ryanodine receptor 2 (cardiac) /FL=gb:NM_001035.1 |
| 4.450364 | 7.144308 | 6.47 | Up | 3.61E-05 | BF246115 | RNA helicase-related protein |
| 0.405368 | 3.094055 | 6.45 | Up | 6.25E-05 | AW188198 | tumor necrosis factor, alpha-induced protein 6 /FL=gb:NM_007115.1 |
| -0.34351 | 2.332751 | 6.39 | Up | 5.54E-05 | NM_000077 | Homo sapiens cyclin-dependent kinase inhibitor 2A (melanoma, p16, inhibits CDK4) (CDKN2A), mRNA. /PROD=cyclin-dependent kinase inhibitor 2A (melanoma,p16, inhibits CDK4) /FL=gb:NM_000077.1 gb:L27211.1 |
| -0.88122 | 1.790331 | 6.37 | Up | 1.37E-03 | AA775681 | hypothetical protein FLJ23091 |
| 3.508565 | 6.179917 | 6.37 | Up | 8.80E-05 | NM_000158 | Homo sapiens glucan (1,4-alpha-), branching enzyme 1 (glycogen branching enzyme, Andersen disease, glycogen storage disease type IV) (GBE1), mRNA. /PROD=glucan (1,4-alpha-), branching enzyme 1 (glycogen branching enzyme) /FL=gb:L07956.1 gb:NM_000158.1 |
| -1.84589 | 0.82486 | 6.37 | Up | 3.13E-03 | AV705309 | ESTs |
| -0.18263 | 2.487276 | 6.36 | Up | 1.49E-02 | AC006942 | Homo sapiens chromosome 19, cosmid R31181 |
| 3.759854 | 6.426778 | 6.35 | Up | 2.01E-05 | AF313413 | Homo sapiens putative small membrane protein NID67 mRNA, complete cds. /PROD=putative small membrane protein NID67 /FL=gb:AF313413.1 |
| 2.4094 | 5.066795 | 6.31 | Up | 3.61 E-05 | AI809870 | HSKM-B protein |
| -2.08089 | 0.570296 | 6.28 | Up | 7.70E-03 | BC016043 | Homo sapiens, hypothetical protein MGC2865, clone MGC:20246 IMAGE:4635389, mRNA, complete cds. /FL=gb:BC016043.1 |
| 1.37641 | 4.004952 | 6.18 | Up | 1.20E-04 | AU148611 | Human pTR7 mRNA for repetitive sequence |
| 1.384716 | 4.012773 | 6.18 | Up | 4.52E-04 | NM_006275 | Homo sapiens splicing factor, arginineserine-rich 6 (SFRS6), mRNA. /PROD=splicing factor, arginineserine-rich 6 /FL=gb:U30883.1 gb:NM_006275.1 |
| 2.979212 | 5.604053 | 6.17 | Up | 1.52E-04 | M27830 | Human 28S ribosomal RNA gene, complete cds. |
| 0.310893 | 2.921559 | 6.11 | Up | 3.42E-04 | NM_007279 | Homo sapiens U2 small nuclear ribonucleoprotein auxiliary factor (65kD) (U2AF65), mRNA. /PROD=U2 small nuclear ribonucleoprotein auxiliaryfactor (65kD) /FL=gb:NM_007279.1 |
| 0.65284 | 3.256353 | 6.08 | Up | 2.49E-04 | AI819043 | ESTs |
| 1.609773 | 4.213168 | 6.08 | Up | 1.35E-04 | M29277 | Human isolate JuSo MUC18 glycoprotein mRNA (3 variant), complete cds. /PROD=MUC18 glycoprotein /FL=gb:M29277.1 |
| -0.62167 | 1.968272 | 6.02 | Up | 4.11E-04 | AI963304 | ESTs |
| 4.459837 | 7.047717 | 6.01 | Up | 1.02E-04 | NM_004052 | Homo sapiens BCL2adenovirus E1B 19kD-interacting protein 3 (BNIP3), nuclear gene encoding mitochondrial protein, mRNA. /PROD=BCL2adenovirus E1B 19kD-interacting protein 3 /FL=gb:AF002697.1 |
| | | | | | | gb:NM_004052.2 |
| | | | | | | gb:U15174.1 |
| 3.178404 | 5.762898 | 6 | Up | 1.27E-04 | AB037810 | Homo sapiens mRNA for KIAA1389 protein, partial cds. /PROD=KIAA1389 protein |
| -1.08931 | 1.494664 | 6 | Up | 2.35E-03 | AB020683 | Homo sapiens mRNA for KIAA0876 protein, partial cds. /PROD=KIAA0876 protein |
| 1.9194 | 4.501184 | 5.99 | Up | 3.61E-05 | AK000162 | Homo sapiens cDNA FLJ20155 fis, clone COL08754, highly similar to ACSA_ECOLI ACETYL-COENZYME A SYNTHETASE. |
| 0.671205 | 3.243641 | 5.95 | Up | 2.48E-04 | AA463626 | ESTs |
| 0.091029 | 2.660133 | 5.93 | Up | 9.55E-04 | AF277174 | Homo sapiens PNAS-137 mRNA, complete cds. /PROD=PNAS-137 /FL=gb:AF277174.1 |
| 1.479222 | 4.042679 | 5.91 | Up | 6.34E-05 | NM_005454 | Homo sapiens cerberus 1 (Xenopus laevis) homolog (cysteine knot superfamily) (CER1), mRNA. /PROD=cerberus 1 /FL=gb:NM_005454.1 |
| -0.29455 | 2.259676 | 5.87 | Up | 2.79E-03 | AF312393 | Homo sapiens leucine-zipper protein FKSG13 (FKSG13) mRNA, complete cds. /PROD=leucine-zipper protein FKSG13 /FL=gb:AF312393.1 |
| -0.81321 | 1.740607 | 5.87 | Up | 3.72E-02 | NM_002160 | Homo sapiens hexabrachion (tenascin C, cytotactin) (HXB), mRNA. /PROD=hexabrachion (tenascin C, cytotactin) /FL=gb:M55618.1 gb:NM_002160.1 |
| 0.167058 | 2.720179 | 5.87 | Up | 1.02E-04 | NM_021223 | Homo sapiens myosin light chain 2a (LOC58498), mRNA. /PROD=myosin light chain 2a /FL=gb:NM_021223.1 |
| 0.901323 | 3.451092 | 5.86 | Up | 3.05E-05 | NM_005767 | Homo sapiens purinergic receptor (family A group 5) (P2Y5), mRNA. /PROD=purinergic receptor (family A group 5) /FL=gb:AF000546.1 gb:NM_005767.1 |
| 1.007578 | 3.543738 | 5.8 | Up | 2.13E-04 | NM_001955 | Homo sapiens endothelin 1 (EDN1), mRNA. /PROD=endothelin 1 /FL=gb:NM_001955.1 |
| 1.327779 | 3.862686 | 5.8 | Up | 1.51E-04 | NM_007115 | Homo sapiens tumor necrosis factor, alpha-induced protein 6 (TNFAIP6), mRNA. /PROD=tumor necrosis factor, alpha-induced protein 6 /FL=gb:NM_007115.1 |
| -0.2077 | 2.32716 | 5.8 | Up | 1.52E-04 | AA211909 | ESTs |
| 1.304257 | 3.835269 | 5.78 | Up | 3.02E-04 | NM_005451 | Homo sapiens enigma (LIM domain protein) (ENIGMA), mRNA. /PROD=enigma protein /FL=gb:BC001093.1 gb:NM_005451.2 gb:AF265209.1 |
| -0.30113 | 2.227329 | 5.77 | Up | 4.58E-03 | BE968750 | Homo sapiens ryanodine receptor 2 (cardiac) (RYR2), mRNA |
| 1.678635 | 4.204607 | 5.76 | Up | 9.75E-05 | AA086229 | enigma (LIM domain protein) |
| 1.411335 | 3.932627 | 5.74 | Up | 1.58E-02 | NM_003370 | Homo sapiens vasodilator-stimulated phosphoprotein (VASP), mRNA. /PROD=vasodilator-stimulated phosphoprotein /FL=gb:NM_003370.1 |
| -1.32153 | 1.183922 | 5.68 | Up | 2.72E-02 | AF217536 | Homo sapiens truncated mevalonate kinase mRNA, partial cds, alternatively spliced. /PROD=truncated mevalonate kinase |
| 1.058988 | 3.560886 | 5.66 | Up | 3.56E-03 | H05812 | insulin-like growth factor 1 receptor /FL=gb:NM_000875.2 |
| 4.435207 | 6.93459 | 5.65 | Up | 3.61E-05 | AF078844 | Homo sapiens hqp0376 protein mRNA, complete cds. /PROD=hqp0376 protein /FL=gb:AF078844.1 |
| 2.418835 | 4.908301 | 5.62 | Up | 3.47E-03 | NM_012268 | Homo sapiens similar to vaccinia virus HindIII K4L ORF (HU-K4), mRNA. /PROD=similar to vaccinia virus HindIII K4L ORF /FL=gb:U60644.1 gb:BC000553.1 gb:NM_012268.1 |
| -0.56112 | 1.927121 | 5.61 | Up | 6.37E-03 | AL041124 | hypothetical protein PP1665 |
| -1.63462 | 0.844727 | 5.58 | Up | 4.98E-03 | AI831874 | ESTs |
| -0.34446 | 2.130828 | 5.56 | Up | 3.75E-05 | NM_006379 | Homo sapiens sema domain, immunoglobulin domain (Ig), short basic domain, secreted, (semaphorin) 3C (SEMA3C), mRNA. /PROD=sema domain, immunoglobulin domain (Ig), shortbasic domain, secreted, (semaphorin) 3C /FL=gb:NM_006379.1 gb:AB000220.1 |
| 4.143258 | 6.618477 | 5.56 | Up | 1.18E-05 | AU146532 | pyruvate dehydrogenase kinase, isoenzyme 1 |
| -0.52233 | 1.950739 | 5.55 | Up | 1.54E-03 | AI091047 | solute carrier family 2 (facilitated glucose transporter), member 1 /FL=gb:K03195.1 gb:NM_006516.1 |
| 0.690529 | 3.155168 | 5.52 | Up | 9.91 E-04 | BC033088 | Homo sapiens, Similar to lamin AC, clone MGC:45654 IMAGE:3623265, mRNA, complete cds. /PROD=Similar to lamin AC /FL=gb:BC033088.1 |
| -0.00098 | 2.454314 | 5.48 | Up | 2.48E-04 | NM_013281 | Homo sapiens fibronectin leucine rich transmembrane protein 3 (FLRT3), mRNA. /PROD=fibronectin leucine rich transmembrane protein3 /FL=gb:AF169677 .1 gb:NM_013281.1 |
| -0.87622 | 1.577138 | 5.48 | Up | 1.84E-02 | M15329 | Human interleukin 1-alpha (IL1A) mRNA, complete cds. /PROD=interleukin 1-alpha /FL=gb:M15329.1 |
| 5.117294 | 7.564809 | 5.45 | Up | 6.25E-05 | M83248 | Human nephropontin mRNA, complete cds. /PROD=nephropontin /FL=gb:M83248.1 |
| 2.352834 | 4.796495 | 5.44 | Up | 4.17E-05 | AI653107 | ESTs |
| 3.27933 | 5.719116 | 5.43 | Up | 3.24E-04 | NM_016639 | Homo sapiens type I transmembrane protein Fn14 (FN14), mRNA. /PROD=type I transmembrane protein Fn14 /FL=gb:NM_016639.1 |
| | | | | | | gb:BC002718.1 |
| | | | | | | gb:AB035480.1 |
| | | | | | | gb:AF191148.1 |
| -2.06232 | 0.368788 | 5.39 | Up | 6.40E-03 | AA148534 | pregnancy-associated plasma protein A /FL=gb:NM_002581.1 gb:U28727.1 |
| 3.982825 | 6.413408 | 5.39 | Up | 7.48E-05 | AA678241 | stearoyl-CoA desaturase (delta-9-desaturase) /FL=gb:AB032261.1 gb:AF097514.1 gb:NM_005063.1 |
| 0.399566 | 2.809822 | 5.32 | Up | 7.81 E-04 | AF144103 | Homo sapiens NJAC protein (NJAC) mRNA, complete cds. /PROD=NJAC protein /FL=gb:AF144103.1 |
| | | | | | | gb:AF106911.1 |
| | | | | | | gb:AF073957.1 |
| | | | | | | gb:BC003513.1 |
| | | | | | | gb:NM_004887.1 |
| 4.15299 | 6.560926 | 5.31 | Up | 6.59E-05 | NM_000935 | Homo sapiens procollagen-lysine, 2-oxoglutarate 5-dioxygenase (lysine hydroxylase) 2 (PLOD2), mRNA./PROD= procollagen-lysine, 2-oxoglutarate 5-dioxygenase(lysine hydroxylase) 2 /FL=gb:NM_000935.1 gb:U84573.1 |
| 0.319769 | 2.724257 | 5.29 | Up | 8.94E-04 | AL552534 | Human mRNA for CD44 antigen, 5UTR (sequence from the 5cap to the start codon). |
| 3.347359 | 5.750515 | 5.29 | Up | 5.27E-03 | AF047002 | Homo sapiens transcriptional coactivator ALY mRNA, partial cds. /PROD=transcriptional coactivator ALY |
| 4.571448 | 6.973787 | 5.29 | Up | 3.53E-04 | AI631159 | solute carrier family 2 (facilitated glucose transporter), member 3 /FL=gb:NM_006931.1 gb:M20681.1 |
| 1.93843 | 4.333496 | 5.26 | Up | 1.21E-04 | NM_019886 | Homo sapiens carbohydrate (N-acetylglucosamine 6-O) sulfotransferase 7 (CHST7), mRNA. /PROD=carbohydrate (N-acetylglucosamine 6-O)sulfotransferase 7 /FL=gb:NM_019886.1 gb:AB037187.1 gb:AB040711.1 |
| -0.3185 | 2.064012 | 5.21 | Up | 1.55E-03 | U83508 | Human angiopoietin-1 mRNA, complete cds. /PROD=angiopoietin-1 /FL=gb:NM_001146.1 gb:D13628.1 gb:U83508.1 |
| 1.484424 | 3.866551 | 5.21 | Up | 3.49E-04 | BC000076 | Homo sapiens, cyclin D1 (PRAD1: parathyroid adenomatosis 1), clone MGC:2316, mRNA, complete cds. /PROD=cyclin D1 (PRAD1: parathyroid adenomatosis 1) /FL=gb:M73554.1 gb:BC000076.1 |
| 4.465458 | 6.844763 | 5.2 | Up | 1.52E-04 | AF003114 | Homo sapiens CYR61 mRNA, complete cds. /FL=gb:AF003114.1 |
| -1.33756 | 1.041706 | 5.2 | Up | 8.51E-03 | BF196010 | ESTs |
| 3.747557 | 6.125638 | 5.2 | Up | 4.52E-04 | AA554833 | neuroendocrine secretory protein 55 |
| -0.24196 | 2.129273 | 5.17 | Up | 2.80E-04 | AV734646 | DNA segment on chromosome X (unique) 9928 expressed sequence |
| 2.657439 | 5.023981 | 5.16 | Up | 1.66E-04 | NM_006472 | Homo sapiens upregulated by 1,25-dihydroxyvitamin D-3 (VDUP1), mRNA./PROD=upregulated by 1,25-dihydroxyvitamin D-3 /FL=gb:NM_006472.1 gb:S73591.1 |
| 1.275689 | 3.642133 | 5.16 | Up | 1.65E-04 | BF982289 | ESTs, Weakly similar to elastin like protein (D.melanogaster) |
| 2.11271 | 4.458591 | 5.08 | Up | 1.97E-04 | H15920 | ESTs, Weakly similar to RTA RAT PROBABLE G PROTEIN-COUPLED RECEPTOR RTA (R.norvegicus) |
| 2.624088 | 4.968498 | 5.08 | Up | 1.45E-04 | M28882 | Human MUC18 glycoprotein mRNA, complete cds. /PROD=MUC18 glycoprotein /FL=gb:M28882.1 |
| 4.046173 | 6.388326 | 5.07 | Up | 6.34E-05 | NM 015641 | Homo sapiens testin (DKFZP586B2022), mRNA. /PROD=testin /FL=gb:BC001451.1 gb:AF245357.1 gb:AF245356.1 gb:NM_015641.1 |
| 0.946455 | 3.284436 | 5.06 | Up | 5.46E-04 | BM976939 | Homo sapiens cDNA FLJ31611 fis, clone NT2RI2002923. |
| 1.389207 | 3.725552 | 5.05 | Up | 1.08E-04 | AL136805 | Homo sapiens mRNA; cDNA DKFZp434J1521 (from clone DKFZp434J1521); complete cds. /PROD=hypothetical protein /FL=gb:AL136805.1 |
| 0.710433 | 3.041407 | 5.03 | Up | 1.08E-03 | NM_ 004472 | Homo sapiens forkhead box D1 (FOXD1), mRNA. /PROD=forkhead box D1 /FL=gb:U59832.1 gb:NM_004472.1 |
| 2.800881 | 5.127991 | 5.02 | Up | 6.53E-05 | NM 017817 | Homo sapiens hypothetical protein FLJ20429 (FLJ20429), mRNA. /PROD=hypothetical protein FLJ20429 /FL=gb:NM_017817.1 |
| 0.432778 | 2.759117 | 5.02 | Up | 2.50E-03 | AI692523 | ESTs |
| -0.94056 | 1.385676 | 5.01 | Up | 4.17E-03 | AI927458 | Homo sapiens mRNA; cDNA DKFZp434A196 (from clone DKFZp434A196); complete cds |
| 0.224112 | 2.550074 | 5.01 | Up | 4.11 E-04 | BF060767 | ESTs |
| -0.47823 | 1.845598 | 5.01 | Up | 2.75E-03 | AA489100 | ESTs |
| 4.590104 | -3.36994 | 249.01 | Down | 2.26E-04 | AA167449 | nuclear receptor subfamily 1, group I, member 3 |
| 6.024741 | -1.32044 | 162.6 | Down | 1.79E-03 | AF017987 | Homo sapiens secreted apoptosis related protein 2 (SARP2) mRNA, complete cds. /PROD=secreted apoptosis related protein 2 /FL=gb:AF056087.1 gb:NM_003012.2 gb:AF017987.1 gb:AF001900.1 |
| 1.940449 | -5.04898 | 127.07 | Down | 3.05E-05 | BE644917 | nuclear receptor subfamily 1, group I, member 3 |
| 2.68759 | -3.61621 | 79 | Down | 1.25E-03 | AL569326 | Homo sapiens cAMP-dependent protein kinase inhibitor beta mRNA, complete cds |
| 5.005827 | -1.03397 | 65.79 | Down | 1.30E-04 | NM_003020 | Homo sapiens secretory granule, neuroendocrine protein 1 (7B2 protein) (SGNE1), mRNA. /PROD=secretory granule, neuroendocrine protein 1 (7B2protein) /FL=gb:BC005349.1 gb:NM_003020.1 |
| 0.357833 | -5.67924 | 65.67 | Down | 2.26E-03 | BG389789 | Human mRNA upregulated during camptothecin-induced apoptosis of U937 cells. |
| 6.500961 | 0.756526 | 53.61 | Down | 4.17E-05 | NM_003012 | Homo sapiens secreted frizzled-related protein 1 (SFRP1), mRNA. /PROD=secreted frizzled-related protein 1 /FL=gb:AF056087.1 gb:NM_003012.2 gb:AF017987.1 gb:AF001900.1 |
| 2.077151 | -3.59883 | 51.13 | Down | 3.75E-03 | BF223193 | nuclear receptor subfamily 1, group I, member 3 |
| 3.29779 | -2.3376 | 49.71 | Down | 3.05E-05 | U17496 | Human proteasome subunit LMP7 (allele LMP7B) mRNA, complete cds. /PROD=proteasome subunit LMP7 /FL=gb:U 17497.1 gb:U17496.1 |
| 4.803291 | -0.78667 | 48.17 | Down | 3.75E-05 | AA628440 | nuclear receptor subfamily 1, group I, member 3 |
| 4.124115 | -1.42293 | 46.75 | Down | 7.16E-04 | AW262311 | ESTs |
| 2.437935 | -3.10907 | 46.75 | Down | 2.41 E-04 | BE672557 | ESTs |
| 4.276201 | -1.19736 | 44.43 | Down | 9.47E-04 | AV699347 | nuclear receptor subfamily 1, group I, member 3 |
| 1.710253 | -3.7619 | 44.39 | Down | 8.66E-05 | NM_016179 | Homo sapiens transient receptor potential channel 4 (TRPC4), mRNA. /PROD=transient receptor potential 4 /FL=gb:NM_016179.1 gb:AF175406.1 |
| 2.313549 | -3.12747 | 43.44 | Down | 5.45E-04 | AV726956 | ESTs, Weakly similar to C35826 hypothetical 13K protein A (H.sapiens) |
| 2.835599 | -2.26295 | 34.26 | Down | 2.50E-03 | AI735586 | ESTs |
| 0.588183 | -4.50758 | 34.2 | Down | 2.89E-04 | AU118882 | endothelin receptor type A /FL=gb:NM_001957.1 gb:L06622.1 |
| 0.349102 | -4.73558 | 33.93 | Down | 2.50E-02 | BM682352 | Homo sapiens cDNA FLJ37204 fis, clone BRALZ2006976. |
| -0.10303 | -5.00884 | 29.98 | Down | 1.97E-04 | NM_022168 | Homo sapiens melanoma differentiation associated protein-5 (MDA5), mRNA. /PROD=melanoma differentiation associated protein-5 /FL=gb:AY017378.1 gb:NM_022168.1 gb:AF095844.1 |
| 2.325274 | -2.506 | 28.47 | Down | 1.35E-04 | AW193693 | DKFZP566K1924 protein |
| 0.252976 | -4.55112 | 27.94 | Down | 2.54E-02 | NM_018043 | Homo sapiens hypothetical protein FLJ10261 (FLJ10261), mRNA. /PROD=hypothetical protein FLJ10261 /FL=gb:NM_018043.1 |
| 2.719388 | -2.08299 | 27.9 | Down | 1.72E-02 | NM_002048 | Homo sapiens growth arrest-specific 1 (GAS1), mRNA. /PROD=growth arrest-specific 1 /FL=gb:NM_002048.1 gb:L 13698.1 |
| 3.257726 | -1.54343 | 27.88 | Down | 6.25E-05 | NM_004335 | Homo sapiens bone marrow stromal cell antigen 2 (BST2), mRNA. /PROD=bone marrow stromal cell antigen 2 /FL=gb:NM_004335.2 gb:D28137.1 |
| 4.353788 | -0.41404 | 27.24 | Down | 4.72E-04 | AI332407 | secreted frizzled-related protein 1 /FL=gb:AF056087.1 gb:NM_003012.2 gb:AF017987.1 gb:AF001900.1 |
| 4.387855 | -0.27888 | 25.4 | Down | 6.82E-05 | AF225513 | Homo sapiens cAMP-dependent protein kinase inhibitor beta mRNA, complete cds. /PROD=cAMP-dependent protein kinase inhibitor beta /FL=gb:AF225513.1 |
| 0.027478 | -4.62508 | 25.15 | Down | 9.89E-03 | NM_152647 | Homo sapiens hypothetical protein FLJ32800 (FLJ32800), mRNA. /FL=gb:NM_152647.1 |
| 1.767203 | -2.80617 | 23.81 | Down | 4.44E-04 | AI742043 | ESTs |
| 1.665643 | -2.69521 | 20.55 | Down | 1.08E-04 | AF282250 | Homo sapiens calneuron 1 (CALN1) mRNA, complete cds. /PROD=calneuron 1 /FL=gb:AF282250.1 |
| 0.414323 | -3.84422 | 19.14 | Down | 3.42E-04 | AF283777 | Homo sapiens clone TCBAP0702 mRNA sequence. |
| 3.779853 | -0.4664 | 18.98 | Down | 4.72E-04 | AV646597 | ESTs, Weakly similar to ALU7 HUMAN ALU SUBFAMILY SQ SEQUENCE CONTAMINATION WARNING ENTRY (H.sapiens) |
| 1.995324 | -2.233 | 18.74 | Down | 1.01E-03 | NM_ 014862 | Homo sapiens KIAA0307 gene product (KIAA0307), mRNA. /PROD=KIAA0307 gene product /FL=gb:AB002305.1 gb:NM_014862.1 |
| 1.946332 | -2.24096 | 18.22 | Down | 2.40E-03 | BG166705 | small inducible cytokine subfamily B (Cys-X-Cys), member 5 (epithelial-derived neutrophil-activating peptide 78) |
| 1.179963 | -2.9904 | 18.01 | Down | 2.13E-04 | U96136 | Homo sapiens delta-catenin mRNA, complete cds. /PROD=delta-catenin /FL=gb:NM_001332.1 gb:U72665.1 gb:AB013805.1 gb:U96136.1 gb:AF035302.1 |
| 2.060687 | -2.07873 | 17.62 | Down | 1.01E-03 | AI269290 | solute carrier family 18 (vesicular monoamine), member 2 /FL=gb:L 14269.1 gb:L23205.1 gb:L09118.1 gb:NM_003054.1 |
| 2.480221 | -1.64839 | 17.49 | Down | 1.22E-03 | NM_ 004900 | Homo sapiens phorbolin (similar to apolipoprotein B mRNA editing protein) (DJ742C19.2), mRNA. /PROD=phorbolin (similar to apolipoprotein B mRNAediting protein) /FL=gb:NM_004900.1 gb:U61083.1 |
| 0.971941 | -3.12304 | 17.09 | Down | 1.07E-02 | N M_018018 | Homo sapiens hypothetical protein FLJ10191 (FLJ10191), mRNA. /PROD=hypothetical protein FLJ10191 /FL=gb:NM_018018.1 |
| 2.263194 | -1.81469 | 16.89 | Down | 2.69E-03 | AF052108 | Homo sapiens clone 23687 mRNA sequence. |
| 2.115848 | -1.84267 | 15.55 | Down | 3.89E-02 | AF213678 | Homo sapiens HAI-2 related small protein (HAI-2) mRNA, complete cds. /PROD=HAI-2 related small protein /FL=gb:AB038317.1 gb:AF213678.1 |
| 2.348373 | -1.56046 | 15.02 | Down | 7.77E-03 | NM_016354 | Homo sapiens solute carrier family 21 (organic anion transporter), member 12 (SLC21A12), mRNA. /PROD=organic anion transporter OATP-E /FL=gb:AB031051.1 gb:NM_016354.1 gb:AF205072.1 gb:AF187817.1 |
| 1.910263 | -1.99418 | 14.97 | Down | 3.77E-04 | NM_006994 | Homo sapiens butyrophilin, subfamily 3, member A3 (BTN3A3), mRNA. /PROD=butyrophilin, subfamily 3, member A3 /FL=gb:U90548.1 gb:NM_006994.2 |
| 2.699795 | -1.16113 | 14.53 | Down | 5.07E-03 | NM 012281 | Homo sapiens potassium voltage-gated channel, Shal-related subfamily, member 2 (KCND2), mRNA. /PROD=potassium voltage-gated channel, Shal-relatedsubfamily, member 2 /FL=gb:NM_012281.1 gb:AB028967.1 gb:AF121104.1 |
| 2.172559 | -1.66608 | 14.31 | Down | 7.83E-03 | BE673445 | Homo sapiens chromosome 19, cosmid R28379 |
| 0.265765 | -3.53873 | 13.97 | Down | 2.21E-03 | AI675836 | Human DNA sequence from clone RP11-446H13 on chromosome 10. Contains the 3 end of the gene for a novel protein similar to KIAA1059 (ortholog of mouse VPS10 domain receptor protein SORCS), an RPL23A (60S ribosmal protein 23A) pseudogene, ESTs, STSs an |
| 5.440506 | 1.644236 | 13.89 | Down | 6.34E-05 | M34455 | Human interferon-gamma-inducible indoleamine 2,3-dioxygenase (IDO) mRNA, complete cds. /FL=gb:NM_002164.1 gb:M34455.1 |
| 0.330556 | -3.45471 | 13.79 | Down | 5.46E-04 | BE972639 | ESTs |
| 0.437404 | -3.31877 | 13.51 | Down | 4.52E-04 | H05254 | ESTs |
| 1.32524 | -2.42314 | 13.44 | Down | 1.57E-02 | H29627 | ESTs |
| 1.673028 | -2.06656 | 13.36 | Down | 5.30E-03 | AF141339 | Homo sapiens LYST-interacting protein LIP3 mRNA, partial cds. /PROD= LYST-interacting protein LIP3 |
| 1.319614 | -2.39234 | 13.1 | Down | 1.43E-02 | NM 004389 | Homo sapiens catenin (cadherin-associated protein), alpha 2 (CTNNA2), mRNA. /PROD=catenin (cadherin-associated protein), alpha 2/FL=gb:NM_004389.1 gb:M94151.2 |
| 1.708627 | -1.98647 | 12.95 | Down | 1.75E-02 | NM_017596 | Homo sapiens KIAA0449 protein (KIAA0449), mRNA. /PROD=hypothetical protein DKFZp434J212 /FL=gb:NM_017596.1 |
| 2.777148 | -0.8902 | 12.71 | Down | 4.36E-04 | NM_022034 | Homo sapiens estrogen regulated gene 1 (ERG-1), mRNA. /PROD=estrogen regulated gene 1 /FL=gb:AF305835.1 gb:NM_022034.1 |
| 1.982765 | -1.67669 | 12.64 | Down | 6.88E-04 | BE672659 | ESTs |
| 0.457009 | -3.18081 | 12.45 | Down | 3.30E-04 | AW449813 | KIAA0918 protein |
| 0.510314 | -3.12055 | 12.39 | Down | 1.71 E-02 | AW780006 | ESTs |
| 0.536704 | -3.05382 | 12.05 | Down | 1.92E-02 | AL049250 | Homo sapiens mRNA; cDNA DKFZp564D113 (from clone DKFZp564D113). |
| 2.294336 | -1.27392 | 11.86 | Down | 1.66E-02 | U11058 | Homo sapiens large conductance calcium- and voltage-dependent potassium channel alpha subunit (MaxiK) mRNA, complete cds. /PROD=large conductance calcium- and voltage-dependentpotassium channel alpha subunit /FL=gb:U23767.1 |
| | | | | | | gb:NM_002247.1 |
| | | | | | | gb:AF025999 |
| 2.177763 | -1.38062 | 11.78 | Down | 8.80E-05 | NM_002590 | Homo sapiens protocadherin 8 (PCDH8), mRNA. /PROD=protocadherin 8 /FL=gb:NM_002590.2 gb:AF061573.2 |
| -0.48821 | -4.01809 | 11.55 | Down | 2.29E-02 | AB040812 | Homo sapiens mRNA for protein kinase PAK5, complete cds. /PROD=protein kinase PAK5 /FL=gb:AB040812.1 |
| 1.785431 | -1.74236 | 11.53 | Down | 2.18E-03 | R15072 | ESTs |
| 1.459269 | -2.00069 | 11 | Down | 3.74E-04 | NM_ 000277 | Homo sapiens phenylalanine hydroxylase (PAH), mRNA. /PROD=phenylalanine hydroxylase /FL=gb:U49897.1 gb:NM_000277.1 |
| 1.995493 | -1.44407 | 10.85 | Down | 7.11E-03 | U91903 | Human Fritz mRNA, complete cds. /PROD=Fritz /FL=gb:U24163.1 gb:U68057.1 gb:NM_001463.1 gb:U91903.1 |
| 0.679665 | -2.7484 | 10.76 | Down | 5.57E-03 | BC000568 | Homo sapiens, clone MGC:3040, mRNA, complete cds. /PROD=Unknown (protein for MGC:3040) /FL=gb:BC000568.1 |
| 1.841888 | -1.57619 | 10.69 | Down | 1.16E-03 | AW072790 | contactin 1 |
| -0.42571 | -3.8118 | 10.45 | Down | 1.58E-02 | AK023699 | Homo sapiens cDNA FLJ13637 fis, clone PLACE1011165. |
| 1.923237 | -1.4606 | 10.44 | Down | 4.11E-04 | AW072102 | Homo sapiens mRNA; cDNA DKFZp434H205 (from clone DKFZp434H205) |
| 0.931661 | -2.45074 | 10.43 | Down | 1.07E-02 | AW149405 | neurexin 1 /FL=gb:AB035356.1 |
| 0.781816 | -2.56699 | 10.19 | Down | 2.50E-02 | NM_000698 | Homo sapiens arachidonate 5-lipoxygenase (ALOX5), mRNA. /PROD=arachidonate 5-lipoxygenase /FL=gb:NM_000698.1 gb:J03600.1 gb:J03571.1 |
| 1.939598 | -1.40726 | 10.17 | Down | 2.29E-03 | AB014737 | Homo sapiens mRNA for SMAP-2b, complete cds. /PROD=SMAP-2b /FL=gb:AB014737.1 |
| 0.874376 | -2.46854 | 10.15 | Down | 1.59E-02 | U82671 | Homo sapiens chromosome Xq28 melanoma antigen family A2a (MAGEA2A), melanoma antigen family A12 (MAGEA12), melanoma antigen family A2b (MAGEA2B), melanoma antigen family A3 (MAGEA3), caltractin (CALT), NAD(P)H dehydrogenase-like protein (NSDHL), a... |
| 3.315768 | 0.011663 | 9.88 | Down | 7.33E-04 | AK098525 | Homo sapiens cDNA FLJ25659 fis, clone TST00427, highly similar to Mus musculus hedgehog-interacting protein (Hip) mRNA. |
| 0.121165 | -3.1782 | 9.84 | Down | 4.65E-03 | AI985987 | ESTs, Moderately similar to ALU1_HUMAN ALU SUBFAMILY J SEQUENCE CONTAMINATION WARNING ENTRY (H.sapiens) |
| 0.686587 | -2.59339 | 9.71 | Down | 2.72E-02 | NM_000439 | Homo sapiens proprotein convertase subtilisinkexin type 1 (PCSK1), mRNA. /PROD=proprotein convertase subtilisinkexin type 1 /FL=gb:NM_000439.2 gb:M90753.1 |
| 3.113317 | -0.0881 | 9.2 | Down | 7.30E-05 | AL565745 | ESTs, Weakly similar to 2108402A carnitine palmitoyltransferase I (H.sapiens) |
| 2.890305 | -0.29832 | 9.12 | Down | 3.24E-04 | NM_ 015474 | Homo sapiens DKFZP564A032 protein (DKFZP564A032), mRNA. /PROD=DKFZP564A032 protein /FL=gb:AF228421.1 |
| | | | | | | gb:AL050267.1 |
| | | | | | | gb:AB013847.1 |
| | | | | | | gb:NM_015474.1 |
| 0.447048 | -2.73882 | 9.1 | Down | 4.19E-02 | BE645435 | ESTs |
| 3.142124 | -0.03564 | 9.05 | Down | 3.76E-03 | AL832535 | Homo sapiens mRNA; cDNA DKFZp547J1816 (from clone DKFZp547J1816). |
| 0.767101 | -2.38787 | 8.91 | Down | 1.92E-02 | BC003517 | Homo sapiens, clone IMAGE:3542589, mRNA, partial cds. /PROD=Unknown (protein for IMAGE:3542589) |
| 4.315048 | 1.168713 | 8.85 | Down | 7.92E-04 | NM_ 016139 | Homo sapiens 16.7Kd protein (LOC51142), mRNA. /PROD=16.7Kd protein /FL=gb:NM_016139.1 |
| | | | | | | gb:AF078845.1 |
| | | | | | | gb:BC003079.1 |
| -0.47937 | -3.61302 | 8.78 | Down | 2.49E-02 | BE968773 | Homo sapiens mRNA; cDNA DKFZp564O1262 (from clone DKFZp564O1262) |
| 3.26376 | 0.151239 | 8.65 | Down | 1.74E-04 | BE644809 | ESTs |
| 0.384182 | -2.72567 | 8.63 | Down | 3.02E-02 | NM_ 005825 | Homo sapiens RAS guanyl releasing protein 2 (calcium and DAG-regulated) (RASGRP2), mRNA. /PROD=RAS guanyl releasing protein 2 (calcium and DAG-regulated) /FL=gb:AF043723.1 gb:NM_005825.1 |
| 2.363587 | -0.72928 | 8.53 | Down | 7.38E-04 | NM_024645 | Homo sapiens hypothetical protein FLJ13842 (FLJ13842), mRNA. /PROD=hypothetical protein FLJ13842 /FL=gb:NM_024645.1 |
| 4.756657 | 1.668488 | 8.5 | Down | 1.30E-04 | AV715309 | ESTs, Weakly similar to ALU7 HUMAN ALU SUBFAMILY SQ SEQUENCE CONTAMINATION WARNING ENTRY (H.sapiens) |
| 0.995551 | -2.07915 | 8.43 | Down | 3.01 E-02 | NM_005386 | Homo sapiens neuronatin (NNAT), mRNA. /PROD=neuronatin /FL=gb:NM_005386.1 |
| | | | | | | gb:BC001768.1 |
| | | | | | | gb:AB002392.1 |
| | | | | | | gb:U25033.1 |
| 2.380226 | -0.68921 | 8.39 | Down | 8.34E-05 | NM_016546 | Homo sapiens complement C1 r-like proteinase precursor, (LOC51279), mRNA. /PROD=complement C1 r-like proteinase precursor, /FL=gb:AF178985.1 gb:NM_016546.1 |
| 2.598916 | -0.46577 | 8.37 | Down | 6.59E-05 | AW051591 | ESTs, Moderately similar to unnamed protein product (H.sapiens) |
| 1.045717 | -2.01579 | 8.35 | Down | 1.44E-02 | AI937060 | KIAA1151 protein |
| 3.009579 | -0.04543 | 8.31 | Down | 7.08E-04 | AB037730 | Homo sapiens mRNA for KIAA1309 protein, partial cds. /PROD=KIAA1309 protein |
| 1.440541 | -1.60998 | 8.29 | Down | 1.58E-02 | BE503640 | ESTs |
| 0.4785 | -2.56291 | 8.23 | Down | 2.46E-02 | BG532690 | integrin, alpha 4 (antigen CD49D, alpha 4 subunit of VLA-4 receptor) |
| 5.84374 | 2.812887 | 8.17 | Down | 6.34E-05 | BC000069 | Homo sapiens, retinoic acid receptor responder (tazarotene induced) 2, clone MGC:1544, mRNA, complete cds. /PROD=retinoic acid receptor responder (tazaroteneinduced) 2 /FL=gb:BC000069.1 |
| | | | | | | gb:NM_002889.2 |
| | | | | | | gb:AB015632.1 |
| | | | | | | gb:U77594.1 |
| 1.110683 | -1.89628 | 8.04 | Down | 8.48E-04 | AA557324 | ESTs, Weakly similar to fatty acid omega-hydroxylase (H.sapiens) |
| 1.386384 | -1.61585 | 8.01 | Down | 3.53E-04 | AF196571 | Homo sapiens Delta-like-1 protein (DLL1) mRNA, complete cds. /PROD=Delta-like-1 protein /FL=gb:NM_005618.2 gb:AF196571.1 |
| 5.363884 | 2.372618 | 7.95 | Down | 7.30E-05 | NM_007015 | Homo sapiens chondromodulin I precursor (CHM-I), mRNA. /PROD=chondromodulin I precursor /FL=gb:NM_007015.1 g b:AB006000.1 |
| 1.364581 | -1.62447 | 7.94 | Down | 2.16E-02 | NM_145280 | Homo sapiens similar to hepatocellular carcinoma-associated antigen HCA557b (LOC151194), mRNA. /FL=gb:BC009462.1 gb:NM_145280.1 |
| 2.628136 | -0.35867 | 7.93 | Down | 1.22E-04 | NM_001957 | Homo sapiens endothelin receptor type A (EDNRA), mRNA. /PROD=endothelin receptor type A /FL=gb:NM_001957.1 gb:L06622.1 |
| 1.712212 | -1.25121 | 7.8 | Down | 8.48E-04 | R62432 | Cluster Incl. R62432:yg52e11.s1 Homo sapiens cDNA, 3 end /clone=IMAGE-36023 /clone_end=3 /gb=R62432 /gi=834311 /ug=Hs.12321 /len=487 |
| 1.443731 | -1.51616 | 7.78 | Down | 4.37E-03 | AJ272267 | Homo sapiens partial mRNA for choline dehydrogenase (chdh gene). /PROD=choline dehydrogenase |
| 3.106596 | 0.148375 | 7.77 | Down | 1.23E-02 | NM_002305 | Homo sapiens lectin, galactoside-binding, soluble, 1 (galectin 1) (LGALS1), mRNA. /PROD=beta-galactosidase binding lectin precursor /FL=gb:NM_002305.2 gb:BC001693.1 gb:J04456.1 |
| 1.961633 | -0.98665 | 7.72 | Down | 8.93E-03 | NM_001463 | Homo sapiens frizzled-related protein (FRZB), mRNA. /PROD=frizzled-related protein /FL=gb:U24163.1 |
| | | | | | | gb:U68057.1 |
| | | | | | | gb:NM_001463.1 |
| | | | | | | gb:U91903.1 |
| 2.38092 | -0.54428 | 7.6 | Down | 6.34E-04 | AF053712 | Homo sapiens osteoprotegerin ligand mRNA, complete cds. /PROD=osteoprotegerin ligand /FL=gb:AF053712.1 gb:AF019047.1 |
| 0.685413 | -2.22507 | 7.52 | Down | 2.98E-02 | NM_018383 | Homo sapiens hypothetical protein FLJ11294 (FLJ11294), mRNA. /PROD=hypothetical protein FLJ11294 /FL=gb:NM_018383.1 |
| -0.24717 | -3.14972 | 7.48 | Down | 3.67E-04 | AF429305 | Homo sapiens C23up NCRMS mRNA, partial sequence; alternatively spliced. |
| 3.893148 | 0.997757 | 7.44 | Down | 1.29E-04 | AU144892 | Homo sapiens cDNA FLJ11569 fis, clone HEMBA1003304 |
| 0.82553 | -2.06523 | 7.42 | Down | 4.10E-02 | BC041933 | Homo sapiens, clone IMAGE:5300703, mRNA. |
| 0.728978 | -2.13318 | 7.27 | Down | 1.25E-03 | AL573058 | complement component 1, r subcomponent |
| 1.033324 | -1.8148 | 7.2 | Down | 5.93E-03 | N80918 | Novel human gene mapping to chomosome 13 |
| 2.858719 | 0.013383 | 7.19 | Down | 4.37E-03 | L10374 | Human (clone CTG-A4) mRNA sequence. |
| 2.260317 | -0.57539 | 7.14 | Down | 1.02E-03 | AI138603 | ESTs |
| 2.003902 | -0.82254 | 7.09 | Down | 8.00E-03 | BF109660 | ESTs |
| 3.85985 | 1.037668 | 7.07 | Down | 2.70E-04 | R40892 | ESTs |
| 3.921983 | 1.12675 | 6.94 | Down | 3.53E-04 | AA206141 | ESTs |
| -0.17018 | -2.96268 | 6.93 | Down | 2.08E-02 | AV741130 | ESTs, Moderately similar to ALU8_ HUMAN ALU SUBFAMILY SX SEQUENCE CONTAMINATION WARNING ENTRY (H.sapiens) |
| 1.610685 | -1.16078 | 6.83 | Down | 9.82E-04 | AA886335 | Homo sapiens serologically defined breast cancer antigen NY-BR-20 mRNA, partial cds |
| 2.859538 | 0.089114 | 6.82 | Down | 2.22E-03 | AW014734 | ESTs |
| 3.212409 | 0.467551 | 6.7 | Down | 2.69E-04 | BG434174 | Homo sapiens cDNA FLJ13555 fis, clone PLACE1007677 |
| 2.116702 | -0.60252 | 6.59 | Down | 3.35E-03 | AF107846 | Homo sapiens neuroendocrine-specific Golgi protein p55 (XLalphas) gene, exon XL2 and complete cds |
| 3.179908 | 0.462842 | 6.58 | Down | 3.24E-04 | U82811 | Human homeodomain-containing protein (HANF) mRNA, complete cds./PROD=HANF /FL=gb:U82811.1 gb:NM_003865.1 |
| 2.509452 | -0.20066 | 6.54 | Down | 1.04E-03 | NM_0016 09 | Homo sapiens acyl-Coenzyme A dehydrogenase, shortbranched chain (ACADSB), nuclear gene encoding mitochondrial protein, mRNA. /PROD=acyl-Coenzyme A dehydrogenase, shortbranchedchain precursor /FL=gb:U 12778.1 gb:NM_001609.1 |
| 2.596054 | -0.10733 | 6.51 | Down | 1.39E-03 | BC005107 | Homo sapiens, clone IMAGE:3840937, mRNA, partial cds. /PROD=Unknown (protein for IMAGE:3840937) |
| 0.559211 | -2.13754 | 6.48 | Down | 2.20E-02 | BF000203 | ESTs |
| 4.534853 | 1.872557 | 6.33 | Down | 9.75E-05 | R38389 | olfactomedin related ER localized protein |
| 2.068166 | -0.59258 | 6.32 | Down | 1.69E-03 | BF698797 | ESTs |
| 2.168796 | -0.49174 | 6.32 | Down | 2.44E-04 | NM_002522 | Homo sapiens neuronal pentraxin I (NPTX1), mRNA. /PROD=neuronal pentraxin I precursor /FL=gb:NM_002522.1 gb:U61849.1 |
| -0.10024 | -2.75761 | 6.31 | Down | 3.60E-02 | BC033812 | Homo sapiens, Similar to LOC155399, clone MGC:45477 IMAGE:5181460, mRNA, complete cds. /PROD=Similar to LOC155399 /FL=gb:BC033812.1 |
| 0.994947 | -1.65385 | 6.27 | Down | 7.39E-03 | NM_024034 | Homo sapiens hypothetical protein MGC3129 similar to ganglioside-induced differentiation-associated protein (MGC3129), mRNA. /PROD=hypothetical protein MGC3129 similar toganglioside-induced differentiation-associated protein /FL=gb:NM_024034.1 gb:BC0 |
| 3.758058 | 1.109291 | 6.27 | Down | 4.39E-04 | NM_005356 | Homo sapiens lymphocyte-specific protein tyrosine kinase (LCK), mRNA. /PROD= lymphocyte-specific protein tyrosine kinase /FL=gb:U07236.1 gb:NM_005356.1 gb:M36881.1 |
| 1.553207 | -1.09181 | 6.26 | Down | 2.54E-02 | AI653050 | ESTs, Weakly similar to HPPD_HUMAN 4-HYDROXYPHENYLPYRU VATE DIOXYGENASE (H.sapiens) |
| 3.702164 | 1.060081 | 6.24 | Down | 4.08E-04 | U26662 | Human neuronal pentraxin II (NPTX2) mRNA, partial cds. /PROD=neuronal pentraxin II |
| 4.260791 | 1.638346 | 6.16 | Down | 6.25E-05 | AA603344 | ESTs, Weakly similar to ALU7 HUMAN ALU SUBFAMILY SQ SEQUENCE CONTAMINATION WARNING ENTRY (H.sapiens) |
| 2.172331 | -0.44991 | 6.16 | Down | 2.44E-04 | NM_003839 | Homo sapiens tumor necrosis factor receptor superfamily, member 11a, activator of NFKB (TNFRSF11A), mRNA. /PROD=tumor necrosis factor receptor superfamily, member 11a, activator of NFKB /FL=gb:NM_003839.1 gb:AF018253.1 |
| 4.981307 | 2.359848 | 6.15 | Down | 4.17E-05 | NM_005103 | Homo sapiens fasciculation and elongation protein zeta 1 (zygin I) (FEZ1), transcript variant 1, mRNA. /PROD=zygin 1, isoform 1 /FL=gb:U60060.1 gb:U69139.1 gb:NM_005103.2 |
| 3.826815 | 1.207679 | 6.14 | Down | 6.48E-05 | BG401568 | Homo sapiens mRNA; cDNA DKFZp434H1235 (from clone DKFZp434H1235); partial cds |
| 1.027188 | -1.59055 | 6.14 | Down | 8.40E-03 | BG169832 | adenylate kinase 5 /FL=gb:NM_012093.1 gb:AF062595.1 |
| 2.054962 | -0.54704 | 6.07 | Down | 2.86E-03 | NM_002800 | Homo sapiens proteasome (prosome, macropain) subunit, beta type, 9 (large multifunctional protease 2) (PSMB9), mRNA. /PROD=proteasome (prosome, macropain) subunit, betatype, 9 (large multifunctional protease 2) /FL=gb:U01025.1 gb:NM_002800.1 |
| 5.372736 | 2.7744 | 6.06 | Down | 8.80E-05 | AA669799 | Cluster Incl. AA669799:ag36c04.s 1 Homo sapiens cDNA, 3 end /clone=IMAGE-1118886 /clone_end=3 /gb=AA669799 /gi=2631298 /ug=Hs.6315 /len=679 |
| 0.24636 | -2.3444 | 6.02 | Down | 1.31E-03 | AW057589 | ESTs |
| 1.145878 | -1.44369 | 6.02 | Down | 9.46E-03 | AI056877 | Human DNA sequence from clone RP4-530I15 on chromosome 20. Contains the 3 end of the PTPN1 gene for protein tyrosine phosphatase, non-receptor type 1 (EC 3.1.3.48), the gene for a novel protein similar to placental protein DIFF40, an RPL36 (60S Ribos |
| 0.976009 | -1.61054 | 6.01 | Down | 5.39E-03 | AW264204 | ESTs |
| 0.497515 | -2.08762 | 6 | Down | 2.83E-02 | AF052117 | Homo sapiens clone 23809 mRNA sequence. |
| 0.381933 | -2.20139 | 5.99 | Down | 8.66E-04 | AI913749 | ESTs |
| 0.528494 | -2.05438 | 5.99 | Down | 4.17E-02 | BG290908 | ESTs, Moderately similar to ALU8_ HUMAN ALU SUBFAMILY SX SEQUENCE CONTAMINATION WARNING ENTRY (H.sapiens) |
| 3.795925 | 1.232161 | 5.91 | Down | 2.20E-04 | NM_004688 | Homo sapiens N-myc (and STAT) interactor (NMI), mRNA. /PROD=N-myc and STAT interactor /FL=gb:BC001268.1 gb:NM_004688.1 gb:U32849.1 |
| 1.934415 | -0.62283 | 5.89 | Down | 4.93E-04 | NM_001351 | Homo sapiens deleted in azoospermia-like (DAZL), mRNA. /PROD=deleted in azoospermia-like /FL=gb:U66726.2 gb:NM_001351.1 gb:U65918.1 gb:U66078.1 |
| 1.805448 | -0.75007 | 5.88 | Down | 1.88E-03 | AI681917 | ESTs, Highly similar to IRX3 MOUSE IROQUOIS-CLASS HOMEODOMAIN PROTEIN IRX-3 (M.musculus) |
| 2.788534 | 0.237692 | 5.86 | Down | 7.40E-03 | AW450929 | heterogeneous nuclear ribonucleoprotein A1 |
| 2.99034 | 0.457208 | 5.79 | Down | 5.23E-04 | NM_002993 | Homo sapiens small inducible cytokine subfamily B (Cys-X-Cys), member 6 (granulocyte chemotactic protein 2) (SCYB6), mRNA. /PROD=small inducible cytokine subfamily B(Cys-X-Cys), member 6 (granulocyte chemotactic protein 2) /FL=gb:U81234.1 gb:NM_00299 |
| 0.362564 | -2.17012 | 5.79 | Down | 1.28E-02 | AB018580 | Homo sapiens mRNA for hluPGFS, complete cds. /PROD=hluPGFS /FL=gb:NM_003739.2 gb:AF149416.2 gb:AB018580.1 gb:D17793.1 |
| 1.673874 | -0.85315 | 5.76 | Down | 8.93E-03 | NM_005965 | Homo sapiens myosin, light polypeptide kinase (MYLK), mRNA. /PROD=myosin, light polypeptide kinase /FL=gb:AB037663.1 gb:AF069601.2 gb:NM_005965.1 |
| 1.858219 | -0.66565 | 5.75 | Down | 2.13E-04 | NM_005084 | Homo sapiens phospholipase A2, group VII (platelet-activating factor acetylhydrolase, plasma) (PLA2G7), mRNA. /PROD=phospholipase A2, group VII (platelet-activatingfactor acetylhydrolase, plasma) /FL=gb:U20157.1 |
| | | | | | | gb:U24577.1 |
| | | | | | | gb:NM_005084.1 |
| 3.016209 | 0.501482 | 5.71 | Down | 6.48E-05 | AW026379 | ESTs |
| 3.692792 | 1.179422 | 5.71 | Down | 1.52E-04 | N21096 | ESTs |
| 1.28245 | -1.23083 | 5.71 | Down | 3.20E-04 | AF063824 | Homo sapiens trp-related protein 4 truncated variant delta mRNA, complete cds. /PROD=trp-related protein 4 truncated variant delta /FL=gb:AF063824.1 |
| 3.772564 | 1.260638 | 5.7 | Down | 3.53E-04 | AA404269 | ESTs |
| 2.016986 | -0.49385 | 5.7 | Down | 6.37E-03 | AI741439 | ESTs |
| 2.453774 | -0.04433 | 5.65 | Down | 1.01E-03 | NM_007191 | Homo sapiens Wnt inhibitory factor-1 (WIF-1), mRNA. /PROD=Wnt inhibitory factor-1 /FL=gb:AF122922.1 gb:NM_007191.1 |
| 5.545158 | 3.061783 | 5.59 | Down | 1.71E-04 | BF057809 | ESTs |
| 5.219258 | 2.740374 | 5.57 | Down | 1.08E-04 | AA974416 | protein phosphatase 2 (formerly 2A), regulatory subunit B (PR 52), beta isoform |
| 2.913327 | 0.434686 | 5.57 | Down | 2.99E-04 | NM_012419 | Homo sapiens regulator of G-protein signalling 17 (RGS17), mRNA. /PROD=regulator of G-protein signalling 17 /FL=gb:AF202257.2 gb:NM_012419.2 |
| 1.651332 | -0.81962 | 5.54 | Down | 1.35E-02 | NM_007181 | Homo sapiens mitogen-activated protein kinase kinase kinase kinase 1 (MAP4K1), mRNA. /PROD= mitogen-activated protein kinase kinase kinasekinase 1 /FL=gb:U66464.1 gb:NM_007181.1 |
| 1.293775 | -1.16956 | 5.51 | Down | 1.15E-02 | BF593660 | ESTs |
| 1.417933 | -1.04305 | 5.51 | Down | 4.93E-04 | NM_002738 | Homo sapiens protein kinase C, beta 1 (PRKCB1), mRNA. /PROD=protein kinase C, beta 1 /FL=gb:NM_002738.1 |
| 3.66812 | 1.207732 | 5.5 | Down | 1.97E-04 | NM_133329 | Homo sapiens potassium voltage-gated channel, subfamily G, member 3 (KCNG3), transcript variant 1, mRNA. /PROD=potassium voltage-gated channel, subfamily G,member 3 isoform 1 /FL=gb:AF454548.1 gb:AF348982.1 gb:AB070604.1 gb:NM_133329.4 |
| 3.002731 | 0.549124 | 5.48 | Down | 4.89E-04 | R54042 | ESTs |
| 5.276628 | 2.824048 | 5.47 | Down | 6.48E-05 | BC005127 | Homo sapiens, adipose differe ntiation-related protein, clone MGC:10598, mRNA, complete cds. /PROD=adipose differentiation-related protein /FL=gb:BC005127.1 gb:NM_001122.1 |
| 3.380946 | 0.937118 | 5.44 | Down | 1.22E-04 | AU151342 | Homo sapiens cDNA FLJ12935 fis, clone NT2RP2004982 |
| 2.01577 | -0.42783 | 5.44 | Down | 1.92E-03 | BE220341 | casein kinase 2, alpha 1 polypeptide |
| 1.307387 | -1.12824 | 5.41 | Down | 1.08E-04 | AK021452 | Homo sapiens cDNA FLJ11390 fis, clone HEMBA1000561, weakly similar to ZINC FINGER PROTEIN 91. |
| 0.36507 | -2.02151 | 5.23 | Down | 4.98E-03 | BF062244 | ESTs |
| 0.756663 | -1.62346 | 5.21 | Down | 1.69E-03 | BG533580 | ESTs |
| 3.876819 | 1.518834 | 5.13 | Down | 4.76E-04 | AI651212 | ES Ts |
| 0.619594 | -1.73795 | 5.12 | Down | 1.18E-03 | BE467611 | ES Ts |
| 0.482843 | -1.87385 | 5.12 | Down | 1.04E-03 | BG284890 | ES Ts |
| 1.048359 | -1.30538 | 5.11 | Down | 8.93E-03 | AA464273 | ES Ts |
| 3.527039 | 1.198446 | 5.02 | Down | 2.69E-03 | BG283790 | ES Ts |
| 2.522705 | 0.194248 | 5.02 | Down | 3.09E-03 | U07236 | Human mutant lymphocyte-specific protein tyrosine kinase (LCK) mRNA, complete cds. /PROD=lymphocyte-specific protein tyrosine kinase /FL=gb:U07236.1 gb:NM_005356.1 gb:M36881.1 |
| 2.356401 | 0.028606 | 5.02 | Down | 6.23E-04 | NM_024893 | Homo sapiens hypothetical protein FLJ14220(FLJ14220), mRNA. /PROD=hypothetical protein FLJ14220 /FL=gb:NM_024893.1 |
| 4.587367 | 2.264046 | 5 | Down | 2.70E-04 | AL049589 | Human DNA sequence from clone 570L12 on chromosome Xq13.1-21.1. Contains the PGK1 gene for phosphoglycerate kinase 1, the gene for a novel protein similar to TAF2G (TATA box binding protein (TBP)-associated factor, RNA polymerase II, G, 32kD) (TAF... |

| **B) ES vs EXPRES 02-Intensity values are in Log2 format.** | | | | | | |
|---|---|---|---|---|---|---|
| ES | EXPRES 02 | Ratio | Direction | adj. p-value | Gene Identifier | Gene Name |
| -2.7136 | 7.077563 | 886 | Up | 9.62E-04 | NM_001898 | Homo sapiens cystatin SN (CST1), mRNA. /PROD=cystatin SN /FL=gb:J03870.1 gb:NM_ 001898.1 |
| -2.3713 | 6.959692 | 644.03 | Up | 6.55E-05 | R72286 | microfibrillar-associated protein 4 |
| -3.78394 | 5.172093 | 496.63 | Up | 1.06E-04 | AW157548 | insulin-like growth factor binding protein 5 /FL=gb:M65062.1 gb:M62782.1 gb:NM_000599.1 gb:AF055033.1 |
| -4.79257 | 3.432794 | 299.28 | Up | 1.81E-03 | AA352113 | ESTs |
| -5.16453 | 3.053666 | 297.8 | Up | 5.30E-03 | NM_002345 | Homo sapiens lumican (LUM), mRNA. /PROD=lumican /FL=gb:NM_002345.1 |
| | | | | | | gb:U18728.1 |
| | | | | | | gb:U21128.1 |
| -2.55753 | 5.625595 | 290.65 | Up | 1.79E-04 | D87811 | Homo sapiens mRNA for GATA-6, complete cds. /PROD=GATA-6 /FL=gb:U66075.1 |
| | | | | | | gb:NM_005257.1 |
| | | | | | | gb:D87811.1 |
| -5.03662 | 3.030028 | 268.1 | Up | 7.79E-05 | BG099432 | ESTs |
| -2.97296 | 4.901727 | 234.7 | Up | 5.95E-05 | AI821586 | ESTs, Moderately similar to JE0284 Mm-1 cell derived transplantability-associated protein 1b (H.sapiens) |
| -3.06613 | 4.772945 | 228.98 | Up | 2.57E-03 | AI422986 | ESTs |
| -4.37538 | 3.399327 | 218.99 | Up | 7.01E-05 | AA552969 | ESTs |
| -3.47045 | 4.200612 | 203.81 | Up | 4.26E-03 | NM_025140 | Homo sapiens hypothetical protein FLJ22471 (FLJ22471), mRNA. /PROD=hypothetical protein FLJ22471 /FL=gb:NM_025140.1 |
| -4.82833 | 2.813842 | 199.77 | Up | 1.40E-04 | BF064262 | ESTs |
| -5.1127 | 2.479359 | 192.95 | Up | 1.06E-04 | NM_002653 | Homo sapiens paired-like homeodomain transcription factor 1 (PITX1), mRNA. /PROD=paired-like homeodomain transcription factor 1 /FL=gb:NM_002653.1 |
| -3.4841 | 4.092799 | 190.93 | Up | 4.34E-04 | BM128432 | Homo sapiens full length insert cDNA clone YA81B05 |
| -4.64254 | 2.721466 | 164.74 | Up | 2.38E-05 | AY177407 | Homo sapiens homeobox protein goosecold mRNA, complete cds. /PROD=homeobox protein goosecold /FL=gb:AY177407.1 gb:NM_173849.1 |
| -3.40132 | 3.923976 | 160.37 | Up | 1.34E-02 | BF589787 | ESTs |
| -1.20324 | 6.071574 | 154.86 | Up | 2.23E-04 | NM_001643 | Homo sapiens apolipoprotein A-II (APOA2), mRNA. /PROD=apolipoprotein A-II precursor /FL=gb:M29882.1 |
| | | | | | | gb:NM_001643.1 |
| | | | | | | gb:BC005282.1 |
| -3.4292 | 3.808106 | 150.89 | Up | 4.62E-04 | AB028021 | Cluster Incl. Cluster Incl. AB028021:Homo sapiens HNF-3beta mRNA for hepatocyte nuclear factor-3 beta, complete cds /cds=(196,1569) /gb=AB028021 /gi=4958949 /ug=Hs.1556151 /len=1944 |
| -4.71714 | 2.374201 | 136.37 | Up | 6.98E-05 | AI796169 | GATA-binding protein 3 /FL=gb:NM_002051.1 gb:M69106.1 gb:BC003070.1 |
| -2.26798 | 4.781861 | 132.5 | Up | 9.04E-05 | NM_001322 | Homo sapiens cystatin SA (CST2), mRNA. /PROD=cystatin SA /FL=gb:NM_001322.1 |
| -0.81321 | 6.174771 | 126.94 | Up | 1.57E-03 | NM_002160 | Homo sapiens hexabrachion (tenascin C, cytotactin) (HXB), mRNA. /PROD=hexabrachion (tenascin C, cytotactin) /FL=gb:M55618.1 gb:NM_002160.1 |
| -2.38522 | 4.594021 | 126.17 | Up | 6.89E-05 | AA502609 | Homo sapiens ankyrin-like with transmembrane domains 1 (ANKTM1), mRNA |
| -3.75387 | 3.204091 | 124.32 | Up | 2.13E-04 | AI991459 | ESTs |
| -4.21525 | 2.671776 | 118.36 | Up | 1.99E-04 | NM_006119 | Homo sapiens fibroblast growth factor 8 (androgen-induced) (FGF8), mRNA. /PROD=fibroblast growth factor 8 (androgen-induced) /FC=gb:U36223.1 gb:U46212.1 gb:NM_006119.1 |
| -2.02474 | 4.768631 | 110.92 | Up | 7.31E-03 | NM_021827 | Homo sapiens hypothetical protein FLJ23514 (FLJ23514), mRNA. /PROD=hypothetical protein FLJ23514 /FL=gb:NM_021827.1 |
| -2.38432 | 4.381798 | 108.84 | Up | 1.01E-03 | AL534095 | hypothetical protein FLJ23091 |
| -2.25187 | 4.311095 | 94.55 | Up | 7.26E-03 | AL136944 | Homo sapiens mRNA; cDNA DKFZp586J0624 (from clone DKFZp586J0624); complete cds. /PROD=hypothetical protein /FL=gb:AF215636.1 |
| | | | | | | gb:NM_014585.1 |
| | | | | | | gb:AF231121.1 |
| | | | | | | gb:AF226614.1 |
| | | | | | | gb:AL 136944.1 |
| -1.92189 | 4.619888 | 93.17 Up | | 9.02E-04 | AV700724 | GATA-binding protein 4 /FL=gb:NM_002052.1 |
| | | | | | | gb;L34357.1 |
| | | | | | | gb:D78260.1 |
| -3.42725 | 2.997778 | 85.93 | Up | 2.95E-03 | NM_022469 | Homo sapiens hypothetical protein FLJ21195 similar to protein related to DAC and cerberus (FLJ21195), mRNA. /PROD=hypothetical protein FLJ21195 similar to proteinrelated to DAC and cerberus /FL=gb:NM_022469.1 |
| -3.14404 | 3.270152 | 85.28 | Up | 6.55E-05 | AB028021 | Homo sapiens HNF-3beta mRNA for hepatocyte nuclear factor-3 beta, complete cds. /PROD=hepatocyte nuclear factor-3 beta /FL=gb:AB028021.1 gb:NM_021784.1 |
| -0.88122 | 5.526622 | 84.91 | Up | 1.25E-04 | AA775681 | hypothetical protein FLJ23091 |
| -2.96204 | 3.439168 | 84.52 | Up | 1.08E-03 | NM_001311 | Homo sapiens cysteine-rich protein 1 (intestinal) (CRIP1), mRNA. /PROD=cysteine-rich protein 1 (intestinal) /FL=gb:U58630.1 |
| | | | | | | gb:BC002738.1 |
| | | | | | | gb:NM_001311.1 |
| | | | | | | gb:U09770.1 |
| -3.42819 | 2.876705 | 79.06 | Up | 2.31E-04 | AL121722 | Human DNA sequence from clone RP4-788L20 on chromosome 20 Contains the HNF3B (hepatocyte nuclear factor 3, beta) gene. a novel gene based on ESTs, ESTs, STSs, GSSs and CpG Islands |
| -1.01231 | 5.27686 | 78.2 | Up | 1.85E-04 | NM_001643 | Homo sapiens apolipoprotein A-II (APOA2), mRNA. /PROD=apolipoprotein A-II precursor /FL=gb:M29882.1 |
| | | | | | | gb:NM_001643.1 |
| | | | | | | gb:BC005282.1 |
| -2.13331 | 4.095704 | 75.01 | Up | 2.99E-04 | NM_003867 | Homo sapiens fibroblast growth factor 17(FGF17), mRNA. /PROD=fibroblast growth factor 17 /FL=gb:NM_003867.1 gb:AB009249.1 |
| -0.14916 | 6.052967 | 73.63 | Up | 2.30E-04 | NM_002521 | Homo sapiens natriuretic peptide precursor B (NPPB), mRNA. /PROD=natriuretic peptide precursor B /FL=gb:NM_002521.1 gb:M25296.1 |
| -2.9139 | 3.274181 | 72.91 | Up | 9.48E-04 | NM_005143 | Homo sapiens haptoglobin (HP), mRNA. /PROD=haptoglobin /FL=gb:K00422.1 gb:L29394.1 gb:NM_005143.1 |
| -2.33028 | 3.85605 | 72.82 | Up | 2.04E-04 | X02162 | Human mRNA for apolipoprotein AI (apo Al)=. /PROD=preproapolipo protein AI |
| -4.75249 | 1.427636 | 72.51 | Up | 8.97E-04 | AJ276395 | Homo sapiens mRNA for MSF-FN70 (FN gene). /PROD=migration stimulation factor FN70 |
| -1.50663 | 4.672617 | 72.47 | Up | 9.82E-05 | BF223214 | ESTs |
| -4.40099 | 1.778003 | 72.45 | Up | 8.87E-05 | AI733179 | ESTs |
| -2.56553 | 3.594638 | 71.51 | Up | 4.24E-04 | D31771 | Homo sapiens mRNA for MSX-2, complete cds. /PROD=MSX-2 /FL=gb:NM_002449.2 gb:D31771.1 |
| -3.67415 | 2.447201 | 69.62 | Up | 1.97E-02 | BC042378 | Homo sapiens, clone IMAGE:5277693, mRNA. |
| -1.33141 | 4.770833 | 68.7 | Up | 2.36E-04 | AI640307 | protocadherin 10 |
| -2.41293 | 3.673615 | 67.96 | Up | 8.00E-04 | NM_000846 | Homo sapiens glutathione S-transferase A2 (GSTA2), mRNA. /PROD=glutathione S-transferase A2 /FL=gb:BC002895.1 |
| | | | | | | gb:M25627.1 |
| | | | | | | gb:M16594.1 |
| | | | | | | gb:M14777.1 |
| | | | | | | gb:M15872.1 |
| | | | | | | gb:M21758.1 |
| | | | | | | gb:NM_000846.1 |
| -2.95346 | 3.079949 | 65.5 | Up | 7.93E-05 | NM_000420 | Homo sapiens Kell blood group (KEL), mRNA. /PROD=Kell blood group antigen /FL=gb:BC003135.1 gb:NM_000420.1 |
| -3.41755 | 2.612638 | 65.35 | Up | 1.03E-02 | AI808090 | ESTs |
| -4.08201 | 1.927438 | 64.42 | Up | 5.56E-04 | AK000680 | Homo sapiens cDNA FLJ20673 fis, clone KAIA4464. /FL=gb:AF240634.1 gb:NM_018440.1 |
| -0.1416 | 5.830942 | 62.79 | Up | 6.89E-05 | NM_022454 | Homo sapiens hypothetical protein FLJ22252 similar to SRY-box containing gene 17 (FLJ22252), mRNA. /PROD=hypothetical protein FLJ22252 similar to SRY-boxcontaining gene 17 /FL=gb:NM_022454.1 |
| -1.79329 | 4.15609 | 61.79 | Up | 7.58E-04 | AL575177 | noggin /FL=gb:NM_005450.1 |
| -0.88683 | 5.039046 | 60.79 | Up | 8.36E-05 | AI821669 | ESTs |
| -3.76697 | 2.141612 | 60.07 | Up | 2.25E-04 | NM_001147 | Homo sapiens angiopoietin 2 (ANGPT2), mRNA. /PROD=angiopoietin 2 /FL=gb:AB009865.1 gb:AF004327.1 gb:NM_001147.1 |
| 0.720535 | 6.617979 | 59.61 | Up | 5.95E-05 | AW007532 | Human insulin-like growth factor binding protein 5 (IGFBP5) mRNA |
| -1.03589 | 4.784337 | 56.5 | Up | 2.23E-04 | AI242583 | Homo sapiens cDNA FLJ11550 fis, clone HEMBA1002970 |
| -2.8856 | 3.531402 | 56.49 | Up | 4.54E-04 | AF211891 | Homo sapiens Mix-like homeobox protein 1 (MSLD1) mRNA, complete cds. /PROD=Mix-like homeobox protein 1 /FL=gb:AF211891.1 |
| -2.04403 | 3.759502 | 55.85 | Up | 2.17E-03 | NM_000039 | Homo sapiens apolipoprotein A-I (APOA1), mRNA. /PROD=apolipoprotein A-I precursor /FL=gb:M27875.1 |
| | | | | | | gb:M11791.1 |
| | | | | | | gb:NM_000039.1 |
| | | | | | | gb:BC005380.1 |
| 0.129315 | 5.920102 | 55.36 | Up | 6.89E-05 | NM_0054 | Homo sapiens eomesodermin (Xenopus laevis) homolog (EOMES), mRNA. /PROD=eomesodermi n (Xenopus laevis) homolog /FL=gb:ABG31038.1 gb:NM_005442.1 |
| -1.57973 | 4.207564 | 55.23 | Up | 5.25E-03 | AL513917 | solute carrier family 16 (monocarboxylic acid transporters), member 3/FL=gb:U81800.1 gb:NM_004207.1 |
| -4.11172 | 1.670966 | 55.05 | Up | 4.57E-04 | BG256677 | interferon, gamma-inducible protein 16 /FL=gb:AF208043.1 |
| -0.28789 | 5.482347 | 54.58 | Up | 6.89E-05 | AW772192 | Homo sapiens clone 23736 mRNA sequence |
| -1.44577 | 4.307421 | 53.94 | Up | 3.59E-03 | AL544576 | ESTs |
| -3.9513 | 1.76416 | 52.54 | Up | 7.33E-05 | NM_016341 | Homo sapiens pancreas-enriched phospholipase C (LOC51196), mRNA. /PROD=pancreas-enriched phospholipase C /FL=gb:AF117948.1 gb:NM_016341.1 gb:AF190642.2 |
| 0.05538 | 5.762108 | 52.23 | Up | 7.33E-05 | AI817041 | G protein-coupled receptor |
| -3.52376 | 2.14189 | 50.76 | Up | 8.23E-05 | AW300217 | ESTs, Moderately similar to C212 HUMAN 28.3 KD PROTEIN C21ORF2 (H.sapiens) |
| -3.18877 | 2.469472 | 50.5 | Up | 7.47E-03 | AF260333 | Homo sapiens AD036 mRNA, complete cds. /PROD=AD036 /FL=gb:AF260333.1 |
| -1.973 | 3.674867 | 50.14 | Up | 1.91E-04 | AA772920 | ESTs |
| -2.30094 | 3.341814 | 49.96 | Up | 7.23E-03 | AI806174 | transcription factor 8 (represses interleukin 2 expression) |
| -2.25531 | 3.356433 | 48.9 | Up | 6.63E-03 | AI380207 | ESTs |
| -2.71676 | 2.892681 | 48.82 | Up | 5.25E-04 | AU144167 | Homo sapiens cDNA FLJ11428 fis, clone HEMBA1001071, highly similar to PROCOLLAGEN ALPHA 1(III) CHAIN PRECURSOR |
| -0.16429 | 5.442379 | 48.73 | Up | 6.83E-04 | L01639 | Human (clone HSY3RR) neuropeptide Y receptor (NPYR) mRNA, complete cds. /PROD=neuropeptide Y receptor /FL=gb:L06797.1 |
| | | | | | | gb:NM_003467.1 |
| | | | | | | gb:AF025375.1 |
| | | | | | | gb:AF147204.1 |
| | | | | | | gb:M99293.1 |
| | | | | | | gb:L01639.1 |
| -4.03477 | 1.563749 | 48.45 | Up | 1.19E-02 | U12170 | Human zinc finger homeodomain protein mRNA, complete cds. /PROD=zinc finger homeodomain protein /FL=gb:U12170.1 |
| -0.08298 | 5.493663 | 47.72 | Up | 2.19E-04 | NM_004207 | Homo sapiens solute carrier family 16 (monocarboxylic acid transporters), member 3 (SLC16A3), mRNA. /PROD=solute carrier family 16 (monocarboxylic acidtransporters), member 3 /FL=gb:U81800.1 gb:NM_004207.1 |
| -2.99405 | 2.552238 | 46.73 | Up | 4.78E-04 | AI767388 | Human DNA sequence from clone RP5-1024N4 on chromosome 1p32.1-33. Contains the gene for a novel Sodium:solute symporter family member similar to SLC5A1 (SGLT1), a pseudogene similar to part of butyrophilin family members, a novel gene, ESTs, STSs, GS |
| -4.61432 | 0.925434 | 46.52 | Up | 2.53E-04 | NM_005221 | Homo sapiens distal-less homeo box 5 (DLX5), mRNA. /PROD=distal-less homeo box 5 /FL=gb:NM_005221.3 |
| -3.22309 | 2.302183 | 46.05 | Up | 4.60E-03 | AW207243 | ESTs |
| -2.82645 | 2.655384 | 44.69 | Up | 4.60E-03 | NM_013445 | Homo sapiens glutamate decarboxylase 1 (brain, 67kD) (GAD1), transcript variant GAD25, mRNA. /PROD=glutamate decarboxylase 1, isoform GAD25 /FL=gb:NM_013445.1 gb:AF178853.1 |
| | | | | | | gb:BC002815.1 |
| 0.533443 | 5.958927 | 42.98 | Up | 6.55E-05 | NM_001899 | Homo sapiens cystatin S (CST4), mRNA. /PROD=cystatin S /FL=gb:NM_001899.1 |
| 0.116207 | 5.518477 | 42.29 | Up | 1.27E-04 | NM_000599 | Homo sapiens insulin-like growth factor binding protein 5 (IGFBP5), mRNA. /PROD=insulin-like growth factor binding protein 5 /FL=gb:M65062.1 |
| | | | | | | gb:M62782.1 |
| | | | | | | gb:NM_000599.1 |
| | | | | | | gb:AF055033.1 |
| -2.1675 | 3.230235 | 42.16 | Up | 1.53E-04 | NM_021804 | Homo sapiens angiotensin I converting enzyme (peptidyl-dipeptidase A) 2 (ACE2), mRNA. /PROD=angiotensin I converting enzyme(peptidyl-dipeptidase A) 2 /FL=gb:NM_021804.1 |
| | | | | | | gb:AF241254.1 |
| | | | | | | gb:AB046569.1 |
| | | | | | | gb:AF291820.1 |
| -0.61985 | 4.752547 | 41.42 | Up | 3.54E-04 | NM_001963 | Homo sapiens epidermal growth factor (beta-urogastrone) (EGF), mRNA. /PROD=epidermal growth factor (beta-urogastrone) /FL=gb:NM_001963.2 |
| -1.73746 | 3.627971 | 41.22 | Up | 3.35E-04 | NM_014391 | Homo sapiens cardiac ankyrin repeat protein (CARP), mRNA. /PROD=cardiac ankyrin repeat protein /FL=gb:NM_014391.1 |
| -3.68848 | 1.657514 | 40.67 | Up | 2.86E-03 | NM_018557 | Homo sapiens low density lipoprotein-related protein 1B (deleted in tumors) (LRP1B), mRNA. /PROD=low density lipoprotein-related protein 1B(deleted in tumors) /FL=gb:AF176832.1 gb:NM_018557.1 |
| -3.11273 | 2.220281 | 40.31 | Up | 5.02E-03 | AW444761 | ESTs |
| -2.78886 | 2.517881 | 39.58 | Up | 7.89E-03 | NM 001115 | Homo sapiens adenylate cyclase 8 (brain) (ADCY8), mRNA. /PROD=adenylate cyclase 8 /FL=gb:NM_001115.1 |
| -1.93668 | 3.348112 | 38.98 | Up | 7.23E-03 | BE670361 | G protein-coupled receptor 72 /FL=gb:NM_016540.1 gb:AF236081.1 |
| -3.65051 | 1.595241 | 37.94 | Up | 3.80E-04 | AF213459 | Homo sapiens ephrin receptor EPHA3 complete form (EPHA3) mRNA, complete cds. /PROD=ephrin receptor EPHA3 complete form /FL=gb:NM_005233.1 gb:M83941.1 gb:AF213459.1 |
| 0.767075 | 5.998268 | 37.56 | Up | 2.55E-04 | AY029208 | Homo sapiens type VI collagen alpha 2 chain precursor (COL6A2) mRNA, complete cds, alternatively spliced. /PROD=type VI collagen alpha 2 chain precursor /FL=gb:AY029208.1 |
| -2.34732 | 2.874499 | 37.32 | Up | 6.51 E-03 | AI240545 | glycophorin B (includes Ss blood group) |
| -2.41622 | 2.773394 | 36.49 | Up | 6.29E-04 | NM_001977 | Homo sapiens glutamyl aminopeptidase (aminopeptidase A) (ENPEP), mRNA. /PROD=glutamyl aminopeptidase (aminopeptidase A) /FL=gb:L 12468.1 gb:NM_001977.1 gb:L14721.1 |
| -1.58875 | 3.588854 | 36.19 | Up | 2.45E-03 | NM_152506 | Homo sapiens hypothetical protein FLJ32835 (FLJ32835), mRNA. /FL=gb:NM_152506.1 |
| -0.93849 | 4.238505 | 36.18 | Up | 1.38E-02 | BE222344 | splicing factor, arginineserine-rich 5 |
| 1.479222 | 6.611842 | 35.08 | Up | 5.95E-05 | NM_005454 | Homo sapiens cerberus 1 (Xenopus laevis) homolog (cysteine knot superfamily) (CER1), mRNA. /PROD=cerberus 1 /FL=gb:NM_005454.1 |
| -0.46494 | 4.666106 | 35.04 | Up | 2.90E-04 | AI141603 | collagen, type VI, alpha 1 |
| -2.23021 | 2.897749 | 34.97 | Up | 4.92E-04 | NM_001785 | Homo sapiens cytidine deaminase (CDA), mRNA. /PROD=cytidine deaminase /FL=gb:L27943.1 gb:NM_001785.1 |
| -3.12836 | 1.991299 | 34.77 | Up | 8.63E-04 | NM_002770 | Homo sapiens protease, serine, 2 (trypsin 2) (PRSS2), mRNA. /PROD=protease, serine, 2 (trypsin 2) /FL=gb:M27602.1 gb:NM_002770.1 |
| -0.98659 | 4.115782 | 34.35 | Up | 1.19E-04 | M65062 | Human insulin-like growth factor binding protein 5 (IGFBP-5) mRNA, complete cds. /PROD=insulin-like growth factor binding protein 5 /FL=gb:M65062.1 |
| | | | | | | gb:M62782.1 |
| | | | | | | gb:NM_000599.1 |
| | | | | | | gb:AF055033.1 |
| -2.50194 | 2.569744 | 33.63 | Up | 4.84E-03 | AJ277914 | Homo sapiens partial LHX9 gene for LIM-homeobox 9, 3UTR. |
| -1.27526 | 3.792158 | 33.53 | Up | 1.12E-03 | AI688418 | plexin A2 |
| -3.34034 | 1.708137 | 33.09 | Up | 2.46E-03 | AA781795 | ESTs |
| 1.339655 | 6.36612 | 32.59 | Up | 7.93E05 | AJ224869 | Homo sapiens CXCR4 gene encoding receptor CXCR4 |
| -0.6944 | 4.326307 | 32.46 | Up | 7.33E-05 | R73554 | Human insulin-like growth factor binding protein 5 (IGFBP5) mRNA |
| -0.67187 | 4.337831 | 32.22 | Up | 7.54E-05 | AI692659 | heat shock 90kD protein 1, alpha |
| -0.46298 | 4.533319 | 31.92 | Up | 3.96E-04 | S69738 | MCP-1=monocyte chemotactic protein (human, aortic endothelial cells, mRNA, 661 nt). /PROD=MCP-1 |
| -2.56712 | 2.414446 | 31.59 | Up | 1.67E-03 | AI813758 | collagen, type III, alpha 1 (Ehlers-Danlos syndrome type IV, autosomal dominant) /FL=gb:NM_000090.1 |
| -0.31684 | 4.651259 | 31.3 | Up | 7.83E-04 | BF130943 | ESTs |
| -2.78124 | 2.184054 | 31.24 | Up | 8.36E-05 | NM_020995 | Homo sapiens haptoglobin-related protein (HPR), mRNA. /PROD=haptoglobin-related protein /FL=gb:NM_020995.1 |
| -1.24179 | 3.719223 | 31.15 | Up | 4.92E-04 | AL534095 | hypothetical protein FLJ23091 |
| -0.35003 | 4.606618 | 31.05 | Up | 6.88E-03 | NM_001778 | Homo sapiens CD48 antigen (B-cell membrane protein) (CD48), mRNA. /PROD=CD48 antigen (B-cell membrane protein) /FL=gb:NM_001778.1 gb:M37766.1 gb:M59904.1 |
| -1.81534 | 3.141104 | 31.05 | Up | 5.86E-04 | AW590925 | ESTs |
| -1.93655 | 3.000448 | 30.63 | Up | 5.42E-03 | NM_013369 | Homo sapiens DNA (cytosine-5-)-methyltransferase 3-like (DNMT3L), mRNA. /PROD=DNA (cytosine-5-)-methyltransferase 3-like /FL=gb:BC002560.1 |
| | | | | | | gb:NM_013369.1 |
| | | | | | | gb:AF194032.1 |
| 1.122063 | 6.03378 | 30.1 | Up | 7.33E-05 | X58851 | Human MLC1emb gene for embryonic myosin alkaline light chain, promoter and exon 1 |
| -0.4721 | 4.436335 | 30.03 | Up | 7.93E-05 | D89377 | Homo sapiens mRNA for MSX-2, complete cds. /PROD=MSX-2 /FL=gb:D89377.1 |
| 0.044298 | 4.952379 | 30.02 | Up | 6.89E-05 | W57613 | ESTs |
| -0.59928 | 4.305866 | 29.96 | Up | 1.03E-03 | AF020769 | Homo sapiens cardiac ventricular troponin C mRNA, complete cds. /PROD=cardiac ventricular troponin C /FL=gb:NM_003280.1 gb:AF020769.1 |
| -1.04571 | 3.857701 | 29.93 | Up | 4.57E-03 | NM_001362 | Homo sapiens deiodinase, iodothyronine, type III (DI03), mRNA. /PROD=thyroxine deiodinase type III /FL=gb:NM_001362.1 gb:S79854.1 |
| -3.24802 | 1.648226 | 29.78 | Up | 1.47E-03 | AI694325 | ESTs |
| -2.50665 | 2.356234 | 29.1 | Up | 8.64E-03 | BE222668 | ESTs |
| -2.27119 | 2.568908 | 28.64 | Up | 2.53E-03 | NM_002608 | Homo sapiens platelet-derived growth factor beta polypeptide (simian sarcoma viral (v-sis) oncogene homolog) (PDGFB), mRNA. /PROD=platelet-derived growth factor beta polypeptide(simian sarcoma viral (v-sis) oncogene homolog) /FL=gb:M12783.1 gb:NM_002 |
| -4.34018 | 0.485509 | 28.36 | Up | 7.34E-05 | AU144247 | Homo sapiens cDNA FLJ13443 fis, clone PLACE 1002853 |
| -1.02297 | 3.794799 | 28.2 | Up | 3.36E-03 | AF208043 | Homo sapiens IFI16b (IFI16b) mRNA, complete cds. /PROD=IFI16b /FL=gb:AF208043.1 |
| -2.79469 | 2.013598 | 28.02 | Up | 9.94E-05 | AI700341 | ESTs |
| -2.73255 | 2.070789 | 27.92 | Up | 1.35E-03 | X89271 | H.sapiens mRNA for HG11 orphan receptor. /PROD=HG11 orphan receptor /FL=gb:NM_005161.1 |
| -3.05797 | 1.724772 | 27.53 | Up | 3.48E-04 | AI801626 | ESTs, Moderately similar to ALU4_HUMAN ALU SUBFAMILY SB2 SEQUENCE CONTAMINATION WARNING ENTRY (H.sapiens) |
| -2.83953 | 1.922194 | 27.13 | Up | 5.16E-04 | BG290193 | hypothetical protein FLJ14299 /FL=gb:NM_025069.1 |
| -4.60716 | 0.149736 | 27.04 | Up | 1.22E-02 | NM_004696 | Homo sapiens solute carrier family 16 (monocarboxylic acid transporters), member 4 (SLC16A4), mRNA. /PROD=solute carrier family 16 (monocarboxylic acidtransporters), member 4 /FL=gb:U59185.1 gb:NM_004696.1 |
| -1.57919 | 3.174205 | 26.97 | Up | 5.94E-04 | AL524520 | G protein-coupled receptor 49 |
| -1.36626 | 3.37132 | 26.68 | Up | 2.16E-03 | AI824037 | ESTs, Weakly similar to FCE2 MOUSE LOW AFFINITY IMMUNOGLOBULIN EPSILON FC RECEPTOR (M.musculus) |
| 0.167058 | 4.895371 | 26.51 | Up | 6.90E-05 | NM_021223 | Homo sapiens myosin light chain 2a (LOC58498), mRNA. /PROD=myosin light chain 2a /FL=gb:NM_021223.1 |
| -1.4009 | 3.316905 | 26.31 | Up | 6.35E-04 | BC029835 | Homo sapiens, clone IMAGE:5169759, mRNA. |
| -2.16518 | 2.549789 | 26.26 | Up | 1.77E-03 | AI521166 | ESTs, Moderately similar to MTR5_HUMAN MYOTUBULARIN-RELATED PROTEIN 5 (H.sapiens) |
| -3.32235 | 1.375121 | 25.95 | Up | 2.00E-04 | BC039433 | Homo sapiens, clone IMAGE:5303550, mRNA. |
| -5.7338 | -1.05152 | 25.67 | Up | 8.80E-03 | NM_020130 | Homo sapiens chromosome 8 open reading frame 4 (C8ORF4), mRNA. /PROD=chromosome 8 open reading frame 4 /FL=gb:NM_020130.1 gb:AF268037.1 |
| 1.214325 | 5.878585 | 25.36 | Up | 7.93E-05 | M36172 | Human embryonic myosin alkali light chain (MLC1) mRNA, complete cds. /FL=gb:M36172.1 gb:M24121.1 gb:NM_002476.1 |
| -2.20104 | 2.461597 | 25.33 | Up | 4.67E-03 | NM_000939 | Homo sapiens proopiomelanocortin (adrenocorticotropin beta-lipotropin alpha-melanocyte stimulating hormone beta-melanocyte stimulating hormone beta-endorphin) (POMC), mRNA. /PROD=proopiomelan ocortin (ad renocorticotropin be ta-lipotropin alpha-melanocyt |
| -0.2674 | 4.336009 | 24.31 | Up | 9.18E-05 | N48299 | ESTs, Moderately similar to NFY-C (H.sapiens) |
| 1.367677 | 5.968973 | 24.27 | Up | 1.00E-04 | AF116676 | Homo sapiens PRO1957 mRNA, complete cds. /PROD=PRO1957 /FL=gb:AF116676.1 |
| -0.62697 | 3.974228 | 24.27 | Up | 4.69E-04 | NM_005531 | Homo sapiens interferon, gamma-inducible protein 16 (IFI16), mRNA. /PROD=interferon, gamma-inducible protein 16 /FL=gb:M63838.1 gb:NM_005531.1 |
| -3.90861 | 0.686447 | 24.17 | Up | 3.72E-03 | BC022531 | Homo sapiens, olfactomedin 3, clone MGC:26675 IMAGE:4812035, mRNA, complete cds. /PROD=olfactomedin 3 /FL=gb:AF397394.1 gb:BC022531.1 |
| -2.75089 | 1.840723 | 24.11 | Up | 2.60E-04 | AL365343 | Homo sapiens mRNA full length insert cDNA clone EUROIMAGE 32539. |
| -0.6097 | 3.975428 | 24 | Up | 1.05E-04 | L12468 | Homo sapiens aminopeptidase A mRNA, complete cds. /PROD=aminopeptidas e A/FL=gb:L12468.1 gb:NM_001977.1 gb:L14721.1 |
| -3.18285 | 1.397334 | 23.92 | Up | 3.32E-04 | BC041441 | Homo sapiens, clone IMAGE:5167131, mRNA. |
| -1.92796 | 2.645248 | 23.81 | Up | 1.20E-02 | BE551416 | ESTs, Weakly similar to KIAA1330 protein (H.sapiens) |
| -4.05111 | 0.51291 | 23.65 | Up | 1.05E-03 | J03580 | Human, parathyroid-like protein (associated with humoral hypercalcemia of malignancy) mRNA, complete cds. /FL=gb:J03580.1 |
| -2.79378 | 1.767388 | 23.61 | Up | 3.68E-02 | AW291402 | ESTs |
| -2.80173 | 1.752943 | 23.5 | Up | 7.26E-03 | AW088232 | ESTs |
| 0.422022 | 4.975085 | 23.48 | Up | 1.38E-04 | D49958 | Homo sapiens mRNA for membrane glycoprotein M6, complete cds. /PROD=membrane glycoprotein M6 /FL=gb:D49958.1 |
| -1.20314 | 3.343061 | 23.36 | Up | 3.63E-04 | M60721 | Human homeobox gene, complete cds. /FL=gb:M60721.1 gb:NM_021958.1 |
| 1.932439 | 6.477785 | 23.35 | Up | 7.33E-05 | H92988 | tyrosine 3-monooxygenasetrypto phan 5-monooxygenase activation protein, eta polypeptide |
| -3.00328 | 1.524799 | 23.07 | Up | 8.50E-03 | AI571798 | Rho GDP dissociation inhibitor (GDI) alpha |
| 0.526605 | 5.052252 | 23.03 | Up | 7.01 E-05 | N71923 | fibronectin leucine rich transmembrane protein 3 /FL=gb:AF169677.1 gb:NM_013281.1 |
| -1.26047 | 3.239318 | 22.62 | Up | 5.12E-03 | NM_007250 | Homo sapiens Kruppel-like factor 8 (KLF8), mRNA. /PROD=Kruppel-like factor 8 /FL=gb:U28282.1 gb:NM_007250.1 |
| -1.93118 | 2.556654 | 22.44 | Up | 2.34E-04 | L33477 | Human (clone 8B1) Br-cadherin mRNA, complete cds. /PROD=Br-cadherin /FL=gb:L34057.1 gb:L33477.1 gb:NM_004061.1 |
| 0.005876 | 4.456564 | 21.87 | Up | 6.89E-05 | AK023621 | Homo sapiens cDNA FLJ13559 fis, clone PLACE 1007852, highly similar to Homo sapiens mRNA for KIAA0878 protein. |
| -1.94 | 2.509492 | 21.85 | Up | 4.29E-03 | NM_016569 | Homo sapiens TBX3-iso protein (TBX3-iso), mRNA. /PROD=TBX3-iso protein /FL=gb:NM_016569.1 gb:AF216750.1 |
| -3.44347 | 1.001698 | 21.78 | Up | 1.61E-02 | AF187858 | Homo sapiens angiopoietin-2 isoform-1 mRNA, complete cds, alternatively spliced. /PROD=angiopoietin-2 isoform-1 /FL=gb:AF187858.1 |
| 0.586923 | 5.027737 | 21.72 | Up | 6.55E-05 | NM_000047 | Homo sapiens arylsulfatase E (chondrodysplasia punctata 1) (ARSE), mRNA. /PROD=arylsulfatase E precursor /FL=gb:X83573.1 gb:NM_000047.1 |
| 1.422626 | 5.860875 | 21.68 | Up | 5.95E-05 | NM_006365 | Homo sapiens transcriptional activator of the c-fos promoter (CROC4), mRNA. /PROD=transcriptional activator of the c-fos promoter /FL=gb:NM_006365.1 gb:U49857.1 |
| -4.18575 | 0.241573 | 21.52 | Up | 7.54E-03 | NM_001736 | Homo sapiens complement component 5 receptor 1 (C5a ligand) (C5R1), mRNA. /PROD=complement component 5 receptor 1 (C5a ligand) /FL=gb:M62505.1 gb:NM_001736.1 |
| 2.301255 | 6.712959 | 21.28 | Up | 7.68E-05 | BC020935 | Homo sapiens, Similar to otoconin 90, clone IMAGE:4277593, mRNA. |
| -3.61192 | 0.788317 | 21.12 | Up | 1.71E-03 | R37101 | ESTs |
| -0.62474 | 3.768383 | 21.01 | Up | 6.55E-05 | NM_000313 | Homo sapiens protein S (alpha) (PROS1), mRNA. /PROD=protein S (alpha) /FL=gb:M15036.1 gb:NM_000313.1 |
| -0.62725 | 3.760374 | 20.93 | Up | 1.52E-04 | BC017942 | Homo sapiens, Similar to otoconin 90, clone IMAGE:4285317, mRNA. |
| -1.52261 | 2.858274 | 20.83 | Up | 2.98E-03 | NM_002614 | Homo sapiens PDZ domain containing 1 (PDZK1), mRNA. /PROD=PDZ domain containing 1 /FL=gb:NM_002614.1 gb:AF012281.1 |
| -1.11347 | 3.25087 | 20.6 | Up | 4.26E-03 | N69091 | ESTs |
| -0.11332 | 4.231933 | 20.33 | Up | 1.05E-04 | NM_003670 | Homo sapiens basic helix-loop-helix domain containing, class B, 2 (BHLHB2), mRNA. /PROD=differentiated embryo chondrocyte expressed gene1 /FL=gb:AB004066.1 gb:NM_003670.1 |
| -1.69238 | 2.643704 | 20.2 | Up | 3.73E-03 | W05495 | ESTs |
| -2.18113 | 2.138504 | 19.97 | Up | 1.23E-02 | NM_004041 | Homo sapiens arrestin, beta 1 (ARRB1), transcript variant 1, mRNA. /PROD=arrestin beta 1, isoform A /FL=gb:BC003636.1 gb:AF084040.1 gb:NM_004041.2 |
| 1.306329 | 5.625069 | 19.96 | Up | 6.90E-05 | NM_001200 | Homo sapiens bone morphogenetic protein 2 (BMP2), mRNA. /PROD=bone morphogenetic protein 2 precursor /FL=gb:NM_001200.1 |
| -1.23777 | 3.066156 | 19.75 | Up | 6.27E-04 | AI917371 | ESTs |
| -0.00098 | 4.266076 | 19.25 | Up | 9.82E-05 | NM_013281 | Homo sapiens fibronectin leucine rich transmembrane protein 3 (FLRT3), mRNA. /PROD=fibronectin leucine rich transmembrane protein3 /FL=gb:AF169677.1 gb:NM_013281.1 |
| -2.0132 | 2.250432 | 19.21 | Up | 1.37E-02 | BE328496 | hypothetical protein PRO2032 /FL=gb:AF116683.1 gb:NM_018615.1 |
| 1.758844 | 6.008618 | 19.02 | Up | 6.55E-05 | N21138 | KIAA0878 protein /FL=gb:NM_014899.1 gb:AB020685.1 |
| 0.095309 | 4.341668 | 18.98 | Up | 8.55E-04 | AA524250 | deleted in liver cancer 1 |
| -0.96212 | 3.265791 | 18.74 | Up | 2.04E-04 | AY113699 | Homo sapiens presenilin stabilization factor b (PSF) mRNA, complete cds; alternatively spliced. /PROD=presenilin stabilization factor b /FL=gb:AY113699.1 |
| 0.502339 | 4.722511 | 18.64 | Up | 1.04E-04 | AI676059 | ESTs |
| -1.74369 | 2.474001 | 18.61 | Up | 7.33E-05 | NM_001882 | Homo sapiens corticotropin releasing hormone-binding protein (CRHBP), mRNA. /PROD=corticotropin releasing hormone-binding protein /FL=gb:NM_001882.2 |
| 0.70975 | 4.924051 | 18.56 | Up | 1.64E-04 | BF939489 | glycoprotein M6A /FL=gb:D49958.1 |
| -2.40752 | 1.7892 | 18.34 | Up | 4.46E-03 | AI738662 | homeo box HB9 /FL=gb:NM_005515.1 |
| -1.34609 | 2.844235 | 18.26 | Up | 7.33E-05 | NM_018388 | Homo sapiens hypothetical protein FLJ11316 (FLJ11316), mRNA. /PROD=hypothetical protein FLJ11316 /FL=gb:NM_018388.1 |
| -0.74044 | 3.448679 | 18.24 | Up | 7.33E-05 | N63706 | ESTs |
| -1.55229 | 2.611591 | 17.92 | Up | 4.34E-04 | AU158444 | Homo sapiens cDNA FLJ14216 fis, clone NT2RP3003672, weakly similar to T-CELL SURFACE GLYCOPROTEIN E2 PRECURSOR |
| 0.082922 | 4.230149 | 17.72 | Up | 1.63E-04 | AI632223 | hypothetical protein DKFZp434F2322 |
| -1.66293 | 2.482886 | 17.7 Up | | 1.56E-02 | NM_000817 | Homo sapiens glutamate decarboxylase 1 (brain, 67kD) (GAD1), transcript variant GAD67, mRNA. /PROD=glutamate decarboxylase 1, isoform GAD67 /FL=gb:NM_000817.1 gb:M81883.1 gb:L 16888.1 |
| -1.82505 | 2.319685 | 17.69 | Up | 7.89E-03 | T16257 | G protein-coupled receptor 37 (endothelin receptor type B-like) /FL=gb:NM_005302.1 gb:AF017262.1 |
| -3.11387 | 1.027176 | 17.64 | Up | 9.68E-03 | U70370 | Human hindlimb expressed homeobox protein backfoot (Bft) mRNA, complete cds. /PROD=hindlimb expressed homeobox protein backfoot /FL=gb:U70370.1 gb:BC003685.1 |
| -0.98938 | 3.143558 | 17.54 | Up | 6.04E-03 | AF190725 | Homo sapiens beta-site APP cleaving enzyme (BACE) mRNA, complete cds. /PROD=beta-site APP cleaving enzyme /FL=gb:AF200343.1 |
| | | | | | | gb:AF204943.1 |
| | | | | | | gb:AF190725.1 |
| | | | | | | gb:AF201468.1 |
| | | | | | | gb:NM_012104.1 |
| -0.74839 | 3.363245 | 17.29 | Up | 5.33E-03 | M17955 | Human MHC class II HLA-DQ-beta mRNA, complete cds. /FL=gb:M33907.1 |
| | | | | | | gb:M17955.1 |
| | | | | | | gb:M16996.1 |
| | | | | | | gb:M17563.1 |
| | | | | | | gb:M20432.1 |
| | | | | | | gb:M26042.1 |
| 2.851479 | 6.958929 | 17.24 | Up | 5.95E-05 | AI950472 | ESTs |
| -2.85963 | 1.242896 | 17.18 | Up | 7.55E-03 | BC005961 | Homo sapiens, parathyroid hormone-like hormone, clone MGC:14611, mRNA, complete cds. /PROD=parathyroid hormone-like hormone /FL=gb:BC005961.1 |
| -1.18027 | 2.905057 | 16.97 | Up | 1.03E-02 | AI123555 | ESTs |
| -1.53522 | 2.547506 | 16.94 | Up | 2.58E-03 | NM_006456 | Homo sapiens sialyltransferase (STHM), mRNA. /PROD=sialyltransfera se /FL=gb:U14550.1 gb:NM_006456.1 |
| 1.620279 | 5.700292 | 16.91 | Up | 1.08E-04 | AI583173 | major histocompatibility complex, class II, DQ beta 1 |
| 1.446234 | 5.516116 | 16.79 | Up | 6.55E-05 | AF348491 | Homo sapiens chemokine receptor CXCR4 mRNA, complete cds. /PROD=chemokine receptor CXCR4 /FL=gb:AF348491.1 |
| 0.880085 | 4.948285 | 16.77 | Up | 6.90E-05 | NM_006096 | Homo sapiens N-myc downstream regulated (NDRG1), mRNA. /PROD=N-myc downstream regulated /FL=gb:AF004162.1 |
| | | | | | | gb:BC003175.1 |
| | | | | | | gb:D87953.1 |
| | | | | | | gb:NM_006096.1 |
| -1.11823 | 2.945099 | 16.72 | Up | 1.28E-02 | NM 002100 | Homo sapiens glycophorin B (includes Ss blood group) (GYPB), mRNA. /PROD=glycophorin B precursor /FL=gb:J02982.1 gb:NM_002100.2 |
| -1.68075 | 2.377232 | 16.66 | Up | 4.35E-03 | NM_012431 | Homo sapiens sema domain, immunoglobulin domain (Ig), short basic domain, secreted, (semaphorin) 3E (SEMA3E), mRNA. /PROD=sema domain, immunoglobulin domain (Ig), shortbasic domain, secreted, (semaphorin) 3E /FL=gb:NM_012431.1 gb:AB002329.1 |
| 0.70158 | 4.759011 | 16.65 | Up | 1.07E-04 | U46768 | Human stanniocalcin mRNA, complete cds. /PROD=stanniocalcin /FL=gb:U25997.1 gb:NM_003155.1 gb:U46768.1 |
| 2.429693 | 6.487025 | 16.65 | Up | 6.89E-05 | AA284532 | tyrosine 3-monooxygenasetrypto phan 5-monooxygenase activation protein, eta polypeptide |
| 1.964573 | 6.018949 | 16.61 | Up | 1.18E-04 | NM_030781 | Homo sapiens scavenger receptor with C-type lectin (SRCL), mRNA. /PROD=scavenger receptor with C-type lectin /FL=gb:NM_030781.1 |
| 0.399566 | 4.451136 | 16.58 | Up | 1.57E-04 | AF144103 | Homo sapiens NJAC protein (NJAC) mRNA, complete cds. /PROD=NJAC protein /FL=gb:AF144103.1 |
| | | | | | | gb:AF106911.1 |
| | | | | | | gb:AF073957.1 |
| | | | | | | gb:BC003513.1 |
| | | | | | | gb:NM_004887.1 |
| 4.590104 | -3.21322 | 223.38 | Down | 7.77E-05 | AA167449 | nuclear receptor subfamily 1, group I, member 3 |
| 1.940449 | -5.370717 | 158.81 | Down | 8.36E-05 | BE644917 | nuclear receptor subfamily 1, group I, member 3 |
| 3.29779 | -3.911445 | 147.98 | Down | 2.38E-05 | U17496 | Human proteasome subunit LMP7 (allele LMP7B) mRNA, complete cds. /PROD=proteasome subunit LMP7 /FL=gb:U17497.1 gb:U17496.1 |
| 4.124115 | -2.85548 | 126.2 | Down | 2.99E-03 | AW262311 | ESTs |
| 3.702164 | -2.970402 | 102.01 | Down | 2.53E-03 | U26662 | Human neuronal pentraxin II (NPTX2) mRNA, partial cds. /PROD=neuronal pentraxin II |
| 1.785431 | -4.509786 | 78.53 | Down | 1.45E-03 | R15072 | ESTs |
| 5.545158 | -0.710745 | 76.42 | Down | 7.01E-05 | BF057809 | ESTs |
| 1.946332 | -3.865792 | 56.19 | Down | 7.43E-04 | BG166705 | small inducible cytokine subfamily B (Cys-X-Cys), member 5 (epithelial-derived neutrophil-activating peptide 78) |
| 1.717184 | -3.902593 | 49.17 | Down | 1.30E-04 | AF237813 | Homo sapiens NPD009 mRNA, complete cds. /PROD=NPD009 /FL=gb:NM_020686.1 gb:AF237813.1 |
| 3.179908 | -2.381796 | 47.23 | Down | 9.96E-04 | U82811 | Human homeodomain-containing protein (HANF) mRNA, complete cds. /PROD=HANF /FL=gb:U82811.1 gb:NM_003865.1 |
| 2.359289 | -3.177031 | 46.41 | Down | 1.72E-04 | NM_006334 | Homo sapiens neuroblastoma (nerve tissue) protein (AMY), mRNA. /PROD=neuroblastom a (nerve tissue) protein /FL=gb:NM_006334.1 gb:D82343.1 |
| 4.276201 | -1.247561 | 46.01 | Down | 7.33E-05 | AV699347 | nuclear receptor subfamily 1, group I, member 3 |
| 2.435058 | -2.963261 | 42.18 | Down | 1.36E-04 | BF056204 | Homo sapiens, clone IMAGE:3603836, mRNA, partial cds |
| 2.348373 | -2.98029 | 40.19 | Down | 7.34E-05 | NM_016354 | Homo sapiens solute carrier family 21 (organic anion transporter), member 12 (SLC21A12), mRNA. /PROD=organic anion transporter OATP-E /FL=gb:AB031051.1 |
| | | | | | | gb:NM_016354.1 |
| | | | | | | gb:AF205072.1 |
| | | | | | | gb:AF187817.1 |
| 4.315048 | -0.819678 | 35.13 | Down | 3.33E-03 | NM_016139 | Homo sapiens 16.7Kd protein (LOC51142), mRNA. /PROD=16.7Kd protein /FL=gb:NM_016139.1 gb:AF078845.1 gb:BC003079.1 |
| 2.348717 | -2.72835 | 33.76 | Down | 9.42E-04 | NM_001877 | Homo sapiens complement component (3dEpstein Barr virus) receptor 2 (CR2), mRNA. /PROD=complement component (3dEpstein Barr virus)receptor 2 /FL=gb:NM_001877.1 gb:M26004.1 |
| 3.342331 | -1.710595 | 33.2 | Down | 6.55E-05 | AA876372 | Homo sapiens mRNA; cDNA DKFZp667D095 (from clone DKFZp667D095) |
| 1.995324 | -3.045119 | 32.91 | Down | 4.60E-03 | NM_014862 | Homo sapiens KIAA0307 gene product (KIAA0307), mRNA. /PROD=KIAA0307 gene product /FL=gb:AB002305.1 gb:NM_014862.1 |
| 2.890305 | -2.133881 | 32.54 | Down | 1.71E-03 | NM_015474 | Homo sapiens DKFZP564A032 protein (DKFZP564A032), mRNA. /PROD=DKFZP564A0 32 protein /FL=gb:AF228421.1 |
| | | | | | | gb:AL050267.1 |
| | | | | | | gb:AB013847.1 |
| | | | | | | gb:NM_015474.1 |
| 2.443445 | -2.518495 | 31.17 | Down | 7.43E-03 | NM_152332 | Homo sapiens chromosome 14 open reading frame 47 (C14orf47), mRNA. /FL=gb:NM_152332.1 |
| 3.692792 | -1.250743 | 30.77 | Down | 8.80E-04 | N21096 | ESTs |
| 2.266312 | -2.653405 | 30.27 | Down | 7.22E-03 | NM 003026 | Homo sapiens SH3-domain GRB2-like 2 (SH3GL2), mRNA. /PROD=SH3-domain GRB2-like 2 /FL=gb:AF036268.1 gb:NM_003026.1 |
| 2.453774 | -2.402069 | 28.96 | Down | 7.93E-05 | NM 007191 | Homo sapiens Wnt inhibitory factor-1 (WIF-1), mRNA. /PROD=Wnt inhibitory factor-1 /FL=gb:AF122922.1 |
| | | | | | | gb:NM_007191.1 |
| 2.325274 | -2.512615 | 28.6 | Down | 3.19E-05 | AW193693 | DKFZP566K1924 protein |
| 1.982765 | -2.836916 | 28.24 | Down | 1.21E-04 | BE672659 | ESTs |
| 0.997895 | -3.793735 | 27.7 | Down | 3.97E-03 | AW025928 | ESTs, Weakly similar to I38588 reverse transcriptase homolog (H.sapiens) |
| 4.756657 | 0.005012 | 26.94 | Down | 6.55E-05 | AV715309 | ESTs, Weakly similar to ALU7_ HUMAN ALU SUBFAMILY SQ SEQUENCE CONTAMINATION WARNING ENTRY (H.sapiens) |
| 4.803291 | 0.085342 | 26.32 | Down | 7.34E-05 | AA628440 | nuclear receptor subfamily 1, group I, member 3 |
| 1.124153 | -3.582082 | 26.1 | Down | 5.42E-03 | W52934 | ESTs, Weakly similar to similar to serinethreonine kinase (C.elegans) |
| 0.874376 | -3.810554 | 25.72 | Down | 2.24E-02 | U82671 | Homo sapiens chromosome Xq28 melanoma antigen family A2a (MAGEA2A), melanoma antigen family A12 (MAGEA12), melanoma antigen family A2b (MAGEA2B), melanoma antigen family A3 (MAGEA3), caltractin (CALT), NAD(P)H dehyd rogenase-like protein (NSDHL), a... |
| 0.621075 | -4.029928 | 25.12 | Down | 5.18E-03 | BC028721 | Homo sapiens, Similar to solute carrier family 1 (high affinity aspa rtateg lutamate transporter), member 6, clone MGC:33092 IMAGE:5269300, mRNA, complete cds. /PROD=Similar to solute carrier family 1 (highaffinity aspa rtateg lutamate transporter), memb |
| 1.49883 | -3.130556 | 24.75 | Down | 7.87E-04 | BF057784 | ESTs |
| 2.074855 | -2.552806 | 24.72 | Down | 1.87E-03 | AI420156 | ESTs |
| 3.2597 | -1.357805 | 24.55 | Down | 1.05E-03 | NM_017671 | Homo sapiens hypothetical protein FLJ20116 (FLJ20116), mRNA. /PROD=hypothetical protein FLJ20116 /FL=gb:NM_017671.1 |
| -0.10303 | -4.717227 | 24.49 | Down | 7.33E-05 | NM_022168 | Homo sapiens melanoma differentiation associated protein-5 (MDA5), mRNA. /PROD=melanoma differentiation associated protein-5 /FL=gb:A Y017378.1 gb:NM_022168.1 gb:AF095844.1 |
| 2.688175 | -1.908993 | 24.2 | Down | 2.73E-04 | NM_014178 | Homo sapiens HSPC156 protein (HSPC156), mRNA. /PROD=HSPC156 protein /FL=gb:NM_014178.1 gb:AF161505.1 |
| 1.24677 | -3.309288 | 23.52 | Down | 2.54E-02 | BC028359 | Homo sapiens, clone IMAGE:4828836, mRNA. |
| 0.686587 | -3.862863 | 23.42 | Down | 1.28E-03 | NM_000439 | Homo sapiens proprotein convertase subtilisinkexin type 1 (PCSK1), mRNA. /PROD=proprotein convertase subtilisinkexin type 1 /FL=gb:NM_000439.2 gb:M90753.1 |
| 5.363884 | 0.82261 | 23.28 | Down | 7.68E-05 | NM_007015 | Homo sapiens chondromodulin I precursor (CHM-I), mRNA. /PROD=chondromodul in I precursor /FL=gb:NM_007015.1 gb:AB006000.1 |
| 3.962328 | -0.577927 | 23.27 | Down | 6.90E-05 | AL533416 | axonal transport of synaptic vesicles |
| 2.086969 | -2.410461 | 22.59 | Down | 9.65E-03 | AL359055 | Homo sapiens mRNA full length insert cDNA clone EUROIMAGE 2344436. |
| 0.536704 | -3.939442 | 22.26 | Down | 4.78E-03 | AL049250 | Homo sapiens mRNA; cDNA DKFZp564D113 (from clone DKFZp564D113). |
| 4.260791 | -0.214844 | 22.25 | Down | 6.89E-05 | AA603344 | ESTs, Weakly similar to ALU7_HUMAN ALU SUBFAMILY SQ SEQUENCE CONTAMINATION WARNING ENTRY (H.sapiens) |
| 0.121165 | -4.34837 | 22.15 | Down | 1.36E-04 | AI985987 | ESTs, Moderately similar to ALU1_HUMAN ALU SUBFAMILY J SEQUENCE CONTAMINATION WARNING ENTRY (H.sapiens) |
| 0.437404 | -4.019211 | 21.96 | Down | 2.48E-02 | H05254 | ESTs |
| 2.263194 | -2.16575 | 21.54 | Down | 7.34E-04 | AF052108 | Homo sapiens clone 23687 mRNA sequence. |
| 4.055226 | -0.3701 | 21.49 | Down | 1.16E-04 | NM_0013 07 | Homo sapiens claudin 7 (CLDN7), mRNA. /PROD=claudin 7 /FL=gb:BC001055.1 gb:NM_001307.1 |
| 5.440506 | 1.023282 | 21.37 | Down | 1.02E-04 | M34455 | Human interferon-gamma-inducible indoleamine 2,3-dioxygenase (IDO) mRNA, complete cds. /FL=gb:NM_002164.1 gb:M34455.1 |
| 2.169025 | -2.207268 | 20.77 | Down | 3.41E-03 | NM_022467 | Homo sapiens N-acetylgalactosamine-4-O-sulfotransferase (GALNAC-4-ST1), mRNA. /PROD=N-acetylgalactosamine-4-O-sulfotransferase /FL=gb:NM_022467.1 gb:AF300612.1 |
| 0.252976 | -4.116368 | 20.67 | Down | 3.36E-03 | NM_018043 | Homo sapiens hypothetical protein FLJ10261 (FLJ10261), mRNA. /PROD=hypothetical protein FLJ10261 /FL=gb:NM_018043.1 |
| 1.353776 | -3.010836 | 20.6 | Down | 2.85E-02 | AF147427 | Homo sapiens full length insert cDNA clone YP80A10. |
| 2.437935 | -1.918781 | 20.49 | Down | 1.70E-03 | BE672557 | ESTs |
| 2.46341 | -1.870689 | 20.17 | Down | 4.16E-04 | NM_003182 | Homo sapiens tachykinin, precursor 1 (substance K, substance P, neurokinin 1, neurokinin 2, neuromedin L, neurokinin alpha, neuropeptide K, neuropeptide gamma) (TAC1), transcript variant beta, mRNA. /PROD=tachykinin 2 precursor, isoform beta /FL=gb:U3 |
| 2.294336 | -2.028037 | 20.01 | Down | 7.10E-03 | U11058 | Homo sapiens large conductance calcium- and voltage-dependent potassium channel alpha subunit (MaxiK) mRNA, complete cds. /PROD=large conductance calcium- and voltage-dependentpotassium channel alpha subunit /FL=gb:U23767.1 gb:NM_002247.1 gb:AF025999 |
| 4.534853 | 0.221559 | 19.88 | Down | 7.68E-05 | R38389 | olfactomedin related ER localized protein |
| 3.142124 | -1.136699 | 19.41 | Down | 1.73E-02 | AL832535 | Homo sapiens mRNA; cDNA DKFZp547J1816 (from clone DKFZp547J1816). |
| 3.85985 | -0.410223 | 19.29 | Down | 7.34E-05 | R40892 | ESTs |
| 4.557488 | 0.304559 | 19.07 | Down | 1.43E-04 | AK026546 | Homo sapiens cDNA: FLJ22893 fis, clone KAT04792. |
| 5.966394 | 1.719122 | 18.99 | Down | 1.99E-04 | AL117612 | Homo sapiens mRNA; cDNA DKFZp564B 1264 (from clone DKFZp564B1264). |
| 1.212373 | -3.013161 | 18.71 | Down | 5.22E-04 | AI564075 | ESTs |
| 2.27629 | -1.932316 | 18.49 | Down | 1.09E-04 | BC044830 | Homo sapiens, Similar to RIKEN cDNA 1700011F14 gene, clone MGC:35062 IMAGE:5166167, mRNA, complete cds. /PROD=Similar to RIKEN cDNA 1700011F14 gene /FL=gb:BC044830.1 |
| 3.18092 | -1.007603 | 18.23 | Down | 1.29E-03 | AJ011712 | Homo sapiens TNNT1 gene, exons 1-11 (and joined CDS) |
| 2.598916 | -1.553059 | 17.78 | Down | 2.46E-03 | AW051591 | ESTs, Moderately similar to unnamed protein product (H.sapiens) |
| 2.003902 | -2.139208 | 17.67 | Down | 4.71E-03 | BF109660 | ESTs |
| 1.905278 | -2.229324 | 17.56 | Down | 5.05E-04 | NM_018964 | Homo sapiens solute carrier family 37 (glycerol-3-phosphate transporter), member 1 (SLC37A1), mRNA. /PROD=solute carrier family 37 (glycerol-3-phosphatetransporter), member 1 /FL=gb:NM_018964.1 |
| 0.330556 | -3.792993 | 17.43 | Down | 3.26E-04 | BE972639 | ESTs |
| 1.044277 | -3.067425 | 17.29 | Down | 1.23E-02 | AL529104 | ESTs |
| 0.733534 | -3.349221 | 16.94 | Down | 4.39E-03 | BF061389 | ESTs, Weakly similar to JC1405 6-pyruvoyltetrahydropteri n synthase (H.sapiens) |
| 2.684281 | -1.394654 | 16.9 | Down | 2.91 E-04 | BF449063 | collagen, type XIV, alpha 1 (undulin) |
| 4.572354 | 0.512428 | 16.68 | Down | 5.89E-04 | BF437747 | ESTs, Weakly similar to ALU7_HUMAN ALU SUBFAMILY SQ SEQUENCE CONTAMINATION WARNING ENTRY (H.sapiens) |
| 2.313549 | -1.734907 | 16.55 | Down | 6.84E-03 | AV726956 | ESTs, Weakly similar to C35826 hypothetical 13K protein A (H.sapiens) |
| 0.679665 | -3.355919 | 16.4 | Down | 1.43E-03 | BC000568 | Homo sapiens, clone MGC:3040, mRNA, complete cds. /PROD=Unknown (protein for MGC:3040) /FL=gb:BC000568.1 |
| 4.099873 | 0.092088 | 16.09 | Down | 6.90E-05 | AI057619 | ESTs, Highly similar to T42654 hypothetical protein DKFZp434G1115.1 (H.sapiens) |
| 1.459269 | -2.519 | 15.76 | Down | 6.26E-03 | NM_000277 | Homo sapiens phenylalanine hydroxylase (PAH), mRNA. /PROD=phenylalanine hydroxylase /FL=gb:U49897.1 gb:NM_000277.1 |
| 2.220936 | -1.733714 | 15.5 | Down | 1.34E-04 | AF237813 | Homo sapiens NPD009 mRNA, complete cds. /PROD=NPD009 /FL=gb:NM_020686.1 gb:AF237813.1 |
| 2.81177 | -1.140906 | 15.48 | Down | 6.89E-05 | NM_002593 | Homo sapiens procollagen C-endopeptidase enhancer (PCOLCE), mRNA. /PROD=procollagen C-endopeptidase enhancer /FL=gb:BC000574.1 gb:NM_002593.2 gb:AB008549.1 gb:L33799.1 |
| 1.529971 | -2.399749 | 15.24 | Down | 2.29E-02 | NM_024789 | Homo sapiens hypothetical protein FLJ22529 (FLJ22529), mRNA. /PROD=hypothetical protein FLJ22529 /FL=gb:NM_024789.1 |
| 0.255558 | -3.655262 | 15.04 | Down | 4.26E-03 | BC039509 | Homo sapiens, clone IMAGE:5578073, mRNA. |
| 0.970967 | -2.921559 | 14.85 | Down | 4.46E-03 | AI144156 | ESTs |
| 2.38092 | -1.504443 | 14.78 | Down | 4.36E-04 | AF053712 | Homo sapiens osteoprotegerin ligand mRNA, complete cds. /PROD=osteoprotegeri n ligand /FL=gb:AF053712.1 gb:AF019047.1 |
| 3.779853 | -0.095194 | 14.67 | Down | 3.22E-04 | AV646597 | ESTs, Weakly similar to ALU7 HUMAN ALU SUBFAMILY SQ SEQUENCE CONTAMINATION WARNING ENTRY (H.sapiens) |
| 1.651332 | -2.189895 | 14.33 | Down | 3.45E-03 | NM_007181 | Homo sapiens mitogen-activated protein kinase kinase kinase kinase 1 (MAP4K1), mRNA. /PROD=mitogen-activated protein kinase kinase kinasekinase 1 /FL=gb:U66464.1 gb:NM_007181.1 |
| 3.786136 | -0.030985 | 14.1 | Down | 1.49E-04 | BF057731 | ESTs |
| 3.257726 | -0.553697 | 14.04 | Down | 4.48E-04 | NM_004335 | Homo sapiens bone marrow stromal cell antigen 2 (BST2), mRNA. /PROD=bone marrow stromal cell antigen 2 /FL=gb:NM_004335.2 gb:D28137.1 |
| -0.33527 | -4.146199 | 14.03 | Down | 4.21E-03 | BE968806 | ESTs, Weakly similar to ALU4_HUMAN ALU SUBFAMILY SB2 SEQUENCE CONTAMINATION WARNING ENTRY (H.sapiens) |
| 0.349102 | -3.450633 | 13.93 | Down | 1.30E-02 | BM682352 | Homo sapiens cDNA FLJ37204 fis, clone BRALZ2006976. |
| 2.172559 | -1.624126 | 13.9 | Down | 2.32E-03 | BE673445 | Homo sapiens chromosome 19, cosmid R28379 |
| 0.381933 | -3.390833 | 13.67 | Down | 1.84E-02 | AI913749 | ESTs |
| 1.120467 | -2.648498 | 13.63 | Down | 1.85E-03 | BE672607 | ESTs |
| 1.924097 | -1.801676 | 13.23 | Down | 8.92E-03 | AW242920 | ESTs |
| 1.491527 | -2.224633 | 13.14 | Down | 7.01E-05 | BG258131 | ESTs |
| 2.060687 | -1.652733 | 13.12 | Down | 5.63E-03 | AI269290 | solute carrier family 18 (vesicular monoamine), member 2 /FL=gb:L14269.1 gb:L23205.1 gb:L09118.1 gb:NM_003054.1 |
| 2.886142 | -0.811762 | 12.98 | Down | 4.93E-04 | U83115 | Human non-lens beta gamma-crystallin like protein (AIM1) mRNA, partial cds. /PROD=non-lens beta gamma-crystallin like protein |
| 0.626797 | -3.063928 | 12.91 | Down | 4.51 E-02 | AF393369 | Homo sapiens sigma 1C adaptin (AP1S3) mRNA, complete cds. /PROD=sigma 1C adaptin /FL=gb:AF393369.1 |
| 1.575227 | -2.112834 | 12.89 | Down | 1.85E-03 | BE856544 | hypothetical protein FLJ22573 |
| 0.4785 | -3.197824 | 12.78 | Down | 1.72E-04 | BG532690 | integrin, alpha 4 (antigen CD49D, alpha 4 subunit of VLA-4 receptor) |
| 1.707178 | -1.969084 | 12.78 | Down | 1.84E-02 | AF043179 | Homo sapiens T cell receptor beta chain (TCRBV13S1-TCRBJ2S1) mRNA, complete cds. /PROD=T cell receptor beta chain /FL=gb:AF043179.1 |
| 0.47298 | -3.177323 | 12.56 | Down | 8.00E-03 | AF311324 | Homo sapiens ubiquitin-like fusion protein mRNA, complete cds. /PROD=ubiquitin-like fusion protein /FL=gb:AF311324.1 |
| 2.641731 | -0.985721 | 12.36 | Down | 1.49E-04 | AL133653 | Homo sapiens mRNA; cDNA DKFZp434M2415 (from clone DKFZp434M2415). |
| 2.913327 | -0.707745 | 12.3 | Down | 3.70E-03 | NM_012419 | Homo sapiens regulator of G-protein signalling 17 (RGS17), mRNA. /PROD=regulator of G-protein signalling 17 /FL=gb:AF202257.2 gb:NM_012419.2 |
| 2.127724 | -1.49095 | 12.28 | Down | 1.49E-03 | NM_001275 | Homo sapiens chromogranin A (parathyroid secretory protein 1) (CHGA), mRNA. /PROD=chromogranin A/FL=gb:BC001059.1 |
| | | | | | | gb:NM_001275.2 |
| | | | | | | gb:J03915.1 |
| | | | | | | gb:J03483.1 |
| 0.80034 | -2.81487 | 12.25 | Down | 4.62E-03 | AW003107 | ESTs |
| 2.11511 | -1.494168 | 12.2 | Down | 1.27E-03 | NM_000593 | Homo sapiens ATP-binding cassette, subfamily B (MDRTAP), member 2 (ABCB2), mRNA. /PROD=ATP-binding cassette, subfamily B, member 2 /FL=gb:NM_000593.2 |
| | | | | | | gb:L21205.1 |
| | | | | | | gb:L21206.1 |
| | | | | | | gb:L21207.1 |
| | | | | | | gb:L21204.1 |
| | | | | | | gb:L21208.1 |
| 3.690675 | 0.086414 | 12.16 | Down | 1.85E-03 | AW274018 | ESTs |
| 1.283746 | -2.317044 | 12.13 | Down | 4.10E-03 | NM_014031 | Homo sapiens VLCS-H1 protein (VLCS-H1), mRNA. /PROD=very long-chain acyl-CoA synthetase homolog 1 /FL=gb:AF064254.1 gb:NM_014031.1 |
| 0.467003 | -3.132847 | 12.12 | Down | 4.39E-02 | AL117530 | Homo sapiens mRNA; cDNA DKFZp434B172 (from clone DKFZp434B172); partial cds. /PROD=hypothetical protein |
| 2.6402 | -0.955567 | 12.09 | Down | 3.80E-02 | NM 013267 | Homo sapiens breast cell glutaminase (GA), mRNA. /PROD=breast cell glutaminase /FL=gb:AF110331.1 |
| | | | | | | gb:AF110330.1 |
| | | | | | | gb:AF223944.1 |
| | | | | | | gb:NM_013267.1 |
| 1.858597 | -1.724557 | 11.98 | Down | 5.91 E-04 | AA757457 | ESTs |
| 0.945333 | -2.635993 | 11.97 | Down | 2.41 E-03 | BC040959 | Homo sapiens, clone IMAGE:5242616, mRNA. |
| 3.795925 | 0.223304 | 11.9 | Down | 1.71E-03 | NM_004688 | Homo sapiens N-myc (and STAT) interactor (NMI), mRNA. /PROD=N-myc and STAT interactor /FL=gb:BC001268.1 |
| | | | | | | gb:NM_004688.1 |
| | | | | | | gb:U32849.1 |
| 2.074972 | -1.490898 | 11.84 | Down | 2.94E-03 | NM_017933 | Homo sapiens hypothetical protein FLJ20701 (FLJ20701), mRNA. /PROD=hypothetical protein FLJ20701 /FL=gb:NM_017933.1 |
| -0.42571 | -3.975429 | 11.71 | Down | 1.54E-03 | AK023699 | Homo sapiens cDNA FLJ13637 fis, clone PLACE1011165. |
| 3.742964 | 0.195658 | 11.69 | Down | 1.19E-04 | T62571 | microtubule-associated protein 7 /FL=gb:NM_003980.1 |
| 1.621518 | -1.924672 | 11.68 | Down | 2.63E-02 | NM_003725 | Homo sapiens oxidative 3 alpha hydroxysteroid dehydrogenase; retinol dehydrogenase; 3-hydroxysteroid epimerase (RODH), mRNA. /PROD=oxidative 3 alpha hydroxysteroid dehydrogenase;retinol dehydrogenase; 3-hydroxysteroid epimerase /FL=gb:AF016509.1 gb:A |
| 6.076777 | 2.566425 | 11.4 | Down | 6.90E-05 | AF229180 | Homo sapiens alpha-aminoadipate semialdehyde synthase mRNA, complete cds. /PROD=alpha-aminoadipate semialdehyde synthase /FL=gb:AF229180.1 |
| 3.758058 | 0.25238 | 11.36 | Down | 1.89E-04 | NM 005356 | Homo sapiens lymphocyte-specific protein tyrosine kinase (LCK), mRNA. /PROD=lymphocyte-specific protein tyrosine kinase /FL=gb:U07236.1 gb:NM_005356.1 gb:M36881.1 |
| 3.797019 | 0.297284 | 11.31 | Down | 2.69E-04 | AI871745 | ESTs |
| 2.469508 | -1.020673 | 11.24 | Down | 9.14E-04 | W63754 | Homo sapiens mRNA for gycosyltransferase (ORF1) |
| 1.048359 | -2.438018 | 11.21 | Down | 1.39E-02 | AA464273 | ESTs |
| 3.146674 | -0.339455 | 11.21 | Down | 1.26E-04 | BF435773 | cortactin SH3 domain-binding protein |
| 1.627801 | -1.848139 | 11.13 | Down | 3.70E-03 | L39833 | Homo sapiens (clone hKvBeta3) K+ channel beta subunit mRNA, complete cds. /PROD=K+ channel beta-subunit /FL=gb:U16953.1 gb:L39833.1 |
| 1.594689 | -1.879508 | 11.11 | Down | 4.03E-02 | AJ236915 | Homo sapiens mRNA for pak5 protein. /PROD=pak5 protein /FL=gb:NM_020168.1 gb:AF276893.1 |
| 2.806504 | -0.6571 | 11.03 | Down | 1.21E-04 | NM_003645 | Homo sapiens fatty-acid-Coenzyme A ligase, very long-chain 1 (FACVL1), mRNA. /PROD=very long-chain fatty-acid-Coenzyme A ligase 1 /FL=gb:AF096290.1 gb:D88308.1 gb:NM_003645.1 |
| 3.826815 | 0.364324 | 11.02 | Down | 6.55E-05 | BG401568 | Homo sapiens mRNA; cDNA DKFZp434H1235 (from clone DKFZp434H1235); partial cds |
| 2.99034 | -0.471936 | 11.02 | Down | 7.16E-04 | NM_002993 | Homo sapiens small inducible cytokine subfamily B (Cys-X-Cys), member 6 (granulocyte chemotactic protein 2) (SCYB6), mRNA./PROD=small inducible cytokine subfamily B(Cys-X-Cys), member 6 (granulocyte chemotactic protein 2) /FL=gb:U81234.1 |
| | | | | | | gb:NM_00299 |
| 3.788599 | 0.329467 | 11 | Down | 8.58E-05 | NM_002252 | Homo sapiens potassium voltage-gated channel, delayed-rectifier, subfamily S, member 3 (KCNS3), mRNA. /PROD=potassium voltage-gated channel,delayed-rectifier, subfamily S, member 3 /FL=gb:AF043472.1 |
| | | | | | | gb:NM_002252.1 |
| | | | | | | gb:BC004987.1 |
| | | | | | | gb:BC004148.1 |
| 4.397278 | 0.939176 | 10.99 | Down | 1.22E-04 | NM_021614 | Homo sapiens potassium intermediatesmall conductance calcium-activated channel, subfamily N, member 2 (KCNN2), mRNA. /PROD=potassium intermediatesmall conductancecalcium-activated channel, subfamily N, member 2 /FL=gb:NM_021614.1 gb:AF239613.1 |
| 1.027188 | -2.423197 | 10.93 | Down | 1.22E-03 | BG169832 | adenylate kinase 5 /FL=gb:NM_012093.1 gb:AF062595.1 |
| 1.022495 | -2.421211 | 10.88 | Down | 2.47E-02 | BC002832 | Homo sapiens, Similar to butyrophilin, subfamily 3, member A2, clone MGC:3790, mRNA, complete cds. /PROD=Similar to butyrophilin, subfamily 3, member A2 /FL=gb:U90144.1 |
| | | | | | | gb:NM_007047.1 |
| | | | | | | gb:U90546.1 |
| | | | | | | gb:BC002832.1 |
| 0.311203 | -3.122447 | 10.81 | Down | 1.70E-03 | AI964053 | ESTs |
| 1.673874 | -1.728682 | 10.57 | Down | 2.25E-03 | NM_005965 | Homo sapiens myosin, light polypeptide kinase (MYLK), mRNA. /PROD=myosin, light |
| | | | | | | polypeptide kinase /FL=gb:AB037663.1 |
| | | | | | | gb:AF069601.2 |
| | | | | | | gb:NM_005965.1 |
| 1.443731 | -1.955774 | 10.55 | Down | 1.43E-03 | AJ272267 | Homo sapiens partial mRNA for choline dehydrogenase (chdh gene). /PROD=choline dehydrogenase |
| 0.383563 | -3.004508 | 10.47 | Down | 2.48E-02 | AI377688 | ESTs |
| 0.995551 | -2.39165 | 10.46 | Down | 5.14E-03 | NM 005386 | Homo sapiens neuronatin (NNAT), mRNA. /PROD=neuronatin /FL=gb:NM_005386.1 |
| | | | | | | gb:BC001768.1 |
| | | | | | | gb:AB002392.1 |
| | | | | | | gb:U25033.1 |
| 3.45531 | 0.070626 | 10.44 | Down | 1.94E-04 | AV733308 | integrin, alpha 6 |
| 3.527039 | 0.143408 | 10.44 | Down | 5.95E-05 | BG283790 | ESTs |
| 2.950823 | -0.418278 | 10.33 | Down | 1.38E-03 | NM_003645 | Homo sapiens fatty-acid-Coenzyme A ligase, very long-chain 1 (FACVL1), mRNA. /PROD=very long-chain fatty-acid-Coenzyme A ligase 1 /FL=gb:AF096290.1 gb:D88308.1 gb:NM_003645.1 |
| 1.55078 | -1.80799 | 10.26 | Down | 3.70E-03 | H28597 | thymopoietin |
| 5.005827 | 1.649812 | 10.24 | Down | 1.90E-04 | NM 003020 | Homo sapiens secretory granule, neuroendocrine protein 1 (7B2 protein) (SGNE1), mRNA. /PROD=secretory granule, neuroendocrine protein 1 (7B2protein) /FL=gb:BC005349.1 gb:NM_003020.1 |
| 0.760646 | -2.590248 | 10.2 | Down | 2.29E-02 | AW589793 | ESTs |
| 2.209313 | -1.126222 | 10.09 | Down | 5.95E-05 | AA747379 | calcitonin-related polypeptide, beta /FL=gb:NM_000728.1 |
| -0.10418 | -3.43217 | 10.04 | Down | 4.66E-03 | NM 017655 | Homo sapiens hypothetical protein FLJ20075 (FLJ20075), mRNA. /PROD=hypothetical protein FLJ20075 /FL=gb:NM_017655.1 |
| 2.598488 | -0.722855 | 10 | Down | 2.03E-03 | L08599 | Human uvomorulin (E-cadherin) (UVO) mRNA, complete cds. /PROD=uvomorulin/FL=gb:NM_004360.1 |
| | | | | | | gb:L08599.1 |
| 1.858219 | -1.457169 | 9.95 | Down | 1.81E-03 | NM_005084 | Homo sapiens phospholipase A2, group VII (platelet-activating factor acetylhydrolase, plasma) (PLA2G7), mRNA. /PROD=phospholipase A2, group VII (platelet-activatingfactor acetylhydrolase, plasma) /FL=gb:U20157.1 gb:U24577.1 gb:NM_005084.1 |
| 1.910263 | -1.3945 | 9.88 | Down | 1.27E-03 | NM_006994 | Homo sapiens butyrophilin, subfamily 3, member A3 (BTN3A3), mRNA. /PROD=butyrophilin, subfamily 3, member A3 /FL=gb:U90548.1 gb:NM_006994.2 |
| 2.001097 | -1.297629 | 9.84 | Down | 1.19E-03 | BC028721 | Homo sapiens, Similar to solute carrier family 1 (high affinity aspartateglutamate transporter), member 6, clone MGC:33092 IMAGE:5269300, mRNA, complete cds. /PROD=Similar to solute carrier family 1 (highaffinity aspartateglutamate transporter), memb |
| 5.020283 | 1.723791 | 9.83 | Down | 1.63E-04 | AK025084 | Homo sapiens cDNA: FLJ21431 fis, clone COL04214, highly similar to HSU80736 Homo sapiens CAGF9 mRNA. |
| 2.474373 | -0.811562 | 9.75 | Down | 5.94E-03 | NM_013272 | Homo sapiens solute carrier family 21 (organic anion transporter), member 11 (SLC21A11), mRNA. /PROD=solute carrier family 21 (organic aniontransporter), member 11 /FL=gb:NM_013272.2 |
| | | | | | | gb:AF205074.1 |
| | | | | | | gb:AB031050.2 |
| | | | | | | gb:AF187816.1 |
| 2.116702 | -1.16308 | 9.71 | Down | 4.40E-02 | AF107846 | Homo sapiens |
| | | | | | | neuroendocrine-specific Golgi protein p55 (XLalphas) gene, exon XL2 and complete cds |
| 2.472358 | -0.803312 | 9.68 | Down | 2.33E-03 | NM 000363 | Homo sapiens troponin I, cardiac (TNNI3), mRNA. /PROD=troponin I, cardiac /FL=gb:M64247.1 |
| | | | | | | gb:NM_000363.1 |
| 0.384182 | -2.880367 | 9.61 | Down | 4.26E-03 | NM_005825 | Homo sapiens RAS guanyl releasing protein 2 (calcium and DAG-regulated) (RASGRP2), mRNA. /PROD=RAS guanyl releasing protein 2 (calcium andDAG-regulated) /FL=gb:AF043723.1 |
| | | | | | | gb:NM_005825.1 |
| 2.394742 | -0.866876 | 9.59 | Down | 1.57E-04 | AI733027 | ESTs, Weakly similar to RET2_HUMAN RETINOL-BINDING PROTEIN II, CELLULAR (H.sapiens) |
| 1.319792 | -1.930717 | 9.52 | Down | 5.95E-05 | M18767 | Human complement subcomponent C1s, alpha- and beta-chains, complete cds. /FL=gb:J04080.1 |
| | | | | | | gb:M18767.1 |
| | | | | | | gb:NM_001734.1 |
| 3.859451 | 0.610002 | 9.51 | Down | 5.73E-04 | AJ242502 | Homo sapiens mRNA for E-MAP-115105 (MAP gene). /PROD=epithelial microtubule-associated protein |
| 3.009579 | -0.239729 | 9.51 | Down | 5.22E-04 | AB037730 | Homo sapiens mRNA for KIAA1309 protein, partial cds. /PROD=KIAA1309 protein |
| 2.600702 | -0.646217 | 9.49 | Down | 1.43E-02 | AA740186 | biliverdin reductase A /FL=gb:NM_000712.1 gb:U34877.1 |
| 0.971941 | -2.274152 | 9.49 | Down | 7.72E-04 | NM_018018 | Homo sapiens hypothetical protein FLJ10191 (FLJ10191), mRNA. /PROD=hypothetical protein FLJ10191 /FL=gb:NM_018018.1 |
| 0.414323 | -2.829854 | 9.48 | Down | 6.30E-03 | AF283777 | Homo sapiens clone TCBAP0702 mRNA sequence. |
| 6.139688 | 2.900659 | 9.44 | Down | 4.48E-04 | BC036488 | Homo sapiens, clone MGC:43145 IMAGE:5261574, mRNA, complete cds. /PROD=Unknown (protein for MGC:43145) /FL=gb:BC036488.1 |
| 1.569503 | -1.668679 | 9.44 | Down | 1.24E-03 | BF195118 | ESTs, Weakly similar to ALU7 HUMAN ALU SUBFAMILY SQ SEQUENCE CONTAMINATION WARNING ENTRY (H.sapiens) |
| 1.665643 | -1.567308 | 9.4 | Down | 8.63E-04 | AF282250 | Homo sapiens calneuron 1 (CALN1) mRNA, complete cds. /PROD=calneuron 1 /FL=gb:AF282250.1 |
| 4.319584 | 1.104655 | 9.29 | Down | 5.53E-04 | AA618420 | Homo sapiens cDNA: FLJ23597 fis, clone LNG15281 |
| 4.436354 | 1.227541 | 9.25 | Down | 2.94E-04 | AI912275 | B-cell CLLlymphoma 11A (zinc finger protein) /FL=gb:NM_022893.1 gb:NM_018014.1 |
| 1.923237 | -1.283751 | 9.23 | Down | 3.70E-03 | AW072102 | Homo sapiens mRNA; cDNA DKFZp434H205 (from clone DKFZp434H205) |
| 2.59299 | -0.613677 | 9.23 | Down | 1.94E-04 | NM_003887 | Homo sapiens development and differentiation enhancing factor 2 (DDEF2), mRNA. /PROD=ADP-ribosylation factor(arf)-directed GTPaseactivating protein /FL=gb:AB007860.1 gb:NM_003887.1 |
| 3.20248 | 0.002668 | 9.19 | Down | 5.80E-04 | AF220153 | Homo sapiens four and a half LIM domains 1 protein isoform C (FHL1) mRNA, complete cds, alternatively spliced. /PROD=four and a half LIM domains 1 protein isoform C /FL=gb:AF220153.1 |
| 1.689335 | -1.505873 | 9.16 | Down | 2.46E-03 | NM_031272 | Homo sapiens testis expressed sequence 14 (TEX14), mRNA. /PROD=testis expressed sequence 14 /FL=gb:NM_031272.1 |
| 3.026209 | -0.167981 | 9.15 | Down | 6.90E-05 | BC032302 | Homo sapiens, similar to UDP-glucose ceramide glucosyltransferase-like 2, clone MGC:40268 IMAGE:5169731, mRNA, complete cds. /PROD=similar to UDP-glucose ceramideglucosyltransf erase-like 2 /FL=gb:BC032302.1 |
| 3.116245 | -0.070826 | 9.11 | Down | 7.93E-05 | NM_017947 | Homo sapiens hypothetical protein FLJ20733 (FLJ20733), mRNA. /PROD=hypothetical protein FLJ20733 /FL=gb:NM_017947.1 |
| 2.91664 | -0.26326 | 9.06 | Down | 1.68E-03 | AW003367 | Homo sapiens clone 25237 mRNA sequence |
| 4.453662 | 1.27718 | 9.04 | Down | 7.33E-05 | AV681807 | v-erb-b2 avian erythroblastic leukemia viral oncogene homolog 3 |
| 3.454075 | 0.278604 | 9.03 | Down | 1.86E-04 | AV691491 | Homo sapiens mRNA; cDNA DKFZp564D 1462 (from clone DKFZp564D1462) |
| 1.883578 | -1.291525 | 9.03 | Down | 2.67E-02 | BF970287 | Human DNA sequence from clone RP1-310O13 on chromosome 20q11.2. Contains (part of) four novel genes, ESTs, STSs, GSSs and four putative CpG islands |
| 2.326737 | -0.838174 | 8.97 | Down | 2.61E-03 | NM_015982 | Homo sapiens germ cell specific Y-box binding protein (LOC51087), mRNA. /PROD=germ cell specific Y-box binding protein /FL=gb:NM 015982.1 gb:AF096834.1 |
| 0.392456 | -2.771428 | 8.96 | Down | 2.73E-02 | NM_003007 | Homo sapiens semenogelin I (SEMG1), mRNA. /PROD=semenogelin I /FL=gb:J04440.1 gb:NM_003007.1 |
| 3.116953 | -0.041715 | 8.93 | Down | 3.90E-04 | AA469071 | Cluster Incl.AA469071:ne17f11.s1 Homo sapiens cDNA, 3 end /clone=IMAGE-881517 /clone_end=3 /gb=AA469071 /gi=2195605 /ug=Hs.180479 /len=758 |
| 3.113317 | -0.040507 | 8.9 | Down | 2.36E-03 | AL565745 | ESTs, Weakly similar to 2108402A carnitine palmitoyltransferase I (H.sapiens) |
| 5.010828 | 1.86984 | 8.82 | Down | 3.28E-04 | AK027006 | Homo sapiens cDNA: FLJ23353 fis, clone HEP14321, highly similar to HSU80736 Homo sapiens CAGF9 mRNA. |
| 3.249727 | 0.113464 | 8.79 | Down | 2.14E-04 | BC001745 | Homo sapiens, clone MGC:3328, mRNA, complete cds. /PROD=Unknown (protein for MGC:3328) /FL=gb:BC001745.1 gb:NM_014392.1 |
| 1.532208 | -1.601117 | 8.77 | Down | 3.19E-03 | AI796813 | advillin |
| 5.063502 | 1.934512 | 8.75 | Down | 1.01E-04 | AB018289 | Homo sapiens mRNA for KIAA0746 protein, partial cds. /PROD=KIAA0746 protein |
| 1.934415 | -1.192238 | 8.73 | Down | 3.13E-03 | NM_001351 | Homo sapiens deleted in azoospermia-like (DAZL), mRNA. /PROD=deleted in azoospermia-like /FL=gb:U66726.2 |
| | | | | | | gb:NM_001351.1 |
| | | | | | | gb:U65918.1 |
| | | | | | | gb:U66078.1 |
| 2.522705 | -0.592752 | 8.67 | Down | 9.13E-03 | U07236 | Human mutant lymphocyte-specific protein tyrosine kinase (LCK) mRNA, complete cds. /PROD=lymphocyte-specific protein tyrosine kinase /FL=gb:U07236.1 |
| | | | | | | gb:NM_005356.1 |
| | | | | | | gb:M36881.1 |
| 3.995648 | 0.881994 | 8.66 | Down | 6.55E-05 | AI961231 | KIAA0808 gene product /FL=gb:AB018351.1 gb:NM_014729.1 |
| 2.077151 | -1.020617 | 8.56 | Down | 7.33E-05 | BF223193 | nuclear receptor subfamily 1, group I, member 3 |
| 6.650173 | 3.554864 | 8.55 | Down | 8.55E-04 | NM_004360 | Homo sapiens cadherin 1, type 1, E-cadherin (epithelial) (CDH1), mRNA. /PROD=cadherin 1, type 1, E-cadherin (epithelial) /FL=gb:NM_004360.1 gb:L08599.1 |
| 1.440541 | -1.650241 | 8.52 | Down | 1.11E-03 | BE503640 | ESTs |
| 1.344522 | -1.721548 | 8.37 | Down | 1.97E-02 | AW071705 | dipeptidylpeptidase VI |
| 4.319671 | 1.253603 | 8.37 | Down | 1.05E-04 | AF063002 | Homo sapiens LIM protein SLIMMER mRNA, complete cds. /PROD=LIM protein SLIMMER /FL=gb:AF063002.1 gb:AF098518.1 |
| 2.575606 | -0.489298 | 8.37 | Down | 1.24E-03 | BC016785 | Homo sapiens, Similar to hypothetical protein PRO1722, clone IMAGE:4096414, mRNA. |
| 1.070412 | -1.992167 | 8.35 | Down | 7.29E-04 | AF070642 | Homo sapiens clone 24488 mRNA sequence. |
| 3.083054 | 0.021788 | 8.35 | Down | 2.33E-03 | BC026969 | Homo sapiens, clone IMAGE:5116073, mRNA, partial cds. |
| 6.075972 | 3.015223 | 8.34 | Down | 1.35E-04 | NM_001449 | Homo sapiens four and a half LIM domains 1 (FHL1), mRNA. /PROD=four and a half LIM domains 1 /FL=gb:U60115.1 |
| | | | | | | gb:NM_001449.1 |
| | | | | | | gb:U29538.1 |
| 0.779095 | -2.27952 | 8.33 | Down | 1.58E-02 | AF497717 | Homo sapiens tissue-type lung unknown mRNA. |
| 4.227241 | 1.173625 | 8.3 | Down | 3.47E-04 | AI193252 | ESTs, Weakly similar to AF133270 1 SLIT2 (H.sapiens) |
| 1.139113 | -1.908335 | 8.27 | Down | 1.40E-02 | AI741469 | ESTs |
| 3.120689 | 0.079671 | 8.23 | Down | 6.27E-04 | U01874 | Human me20m mRNA, complete cds. /PROD=me20m /FL=gb:NM_006928.1 |
| | | | | | | gb:BC001414.1 |
| | | | | | | gb:U01874.1 |
| 3.499377 | 0.460367 | 8.22 | Down | 2.16E-03 | NM_024572 | Homo sapiens hypothetical protein FLJ12691 (FLJ12691), mRNA. /PROD=hypothetical protein FLJ12691 /FL=gb:NM_024572.1 |
| 2.568736 | -0.463022 | 8.18 | Down | 4.78E-03 | AA531287 | ESTs |

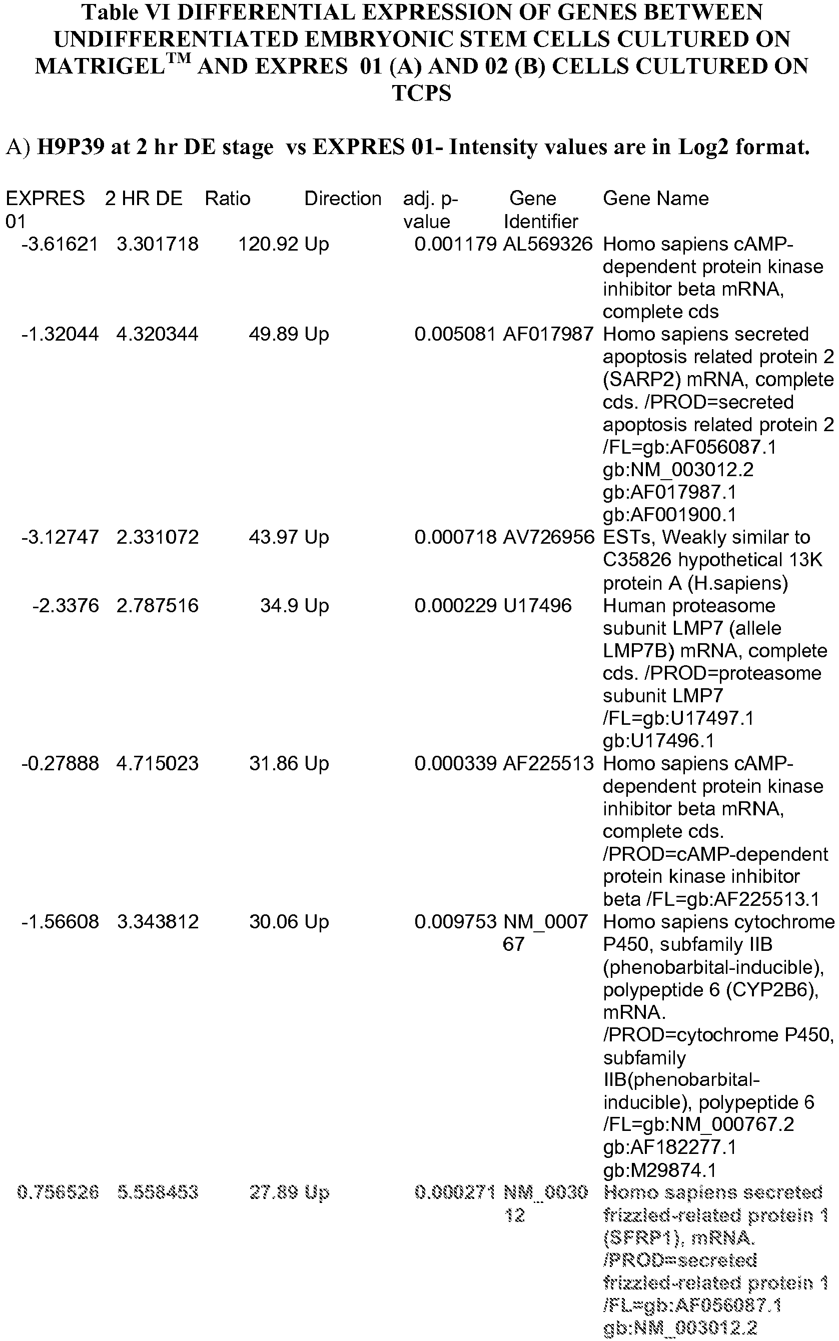

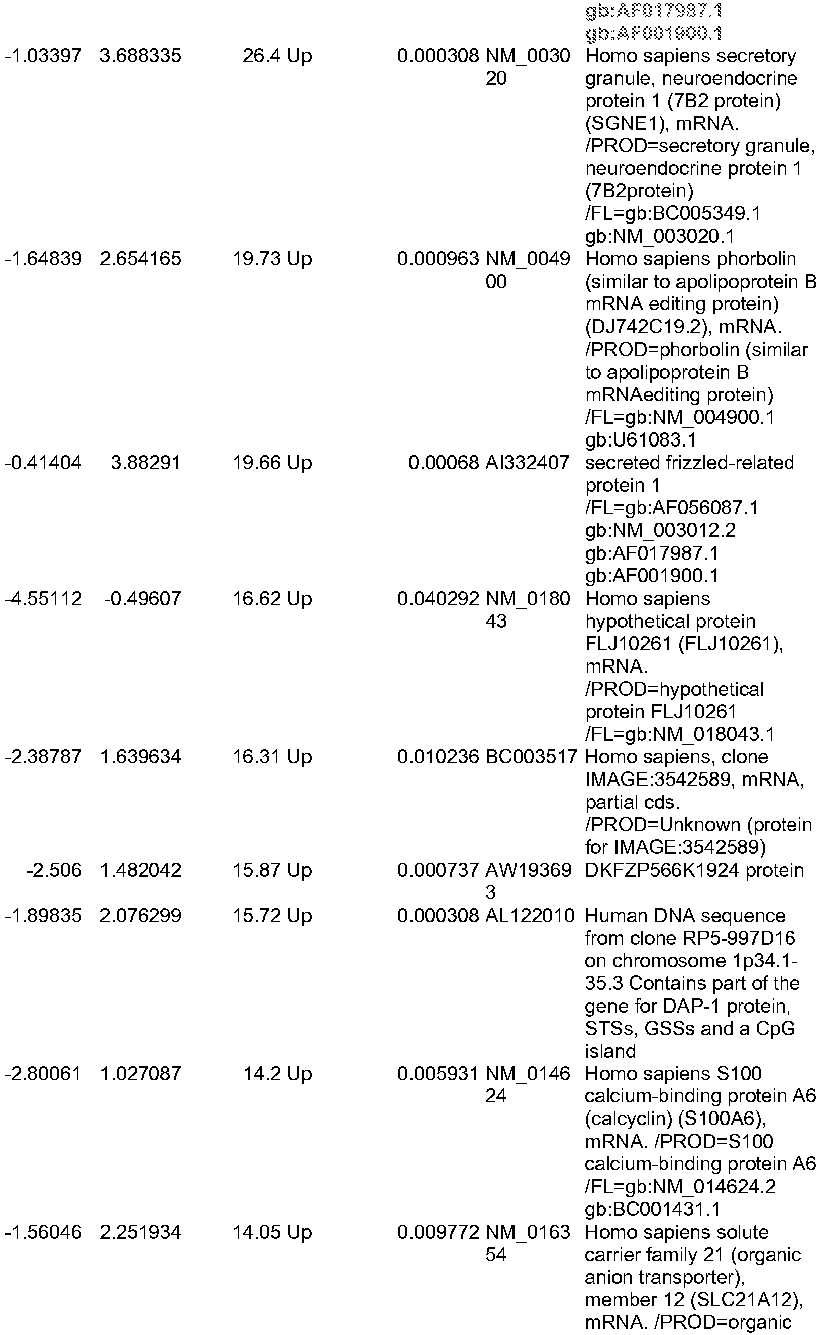

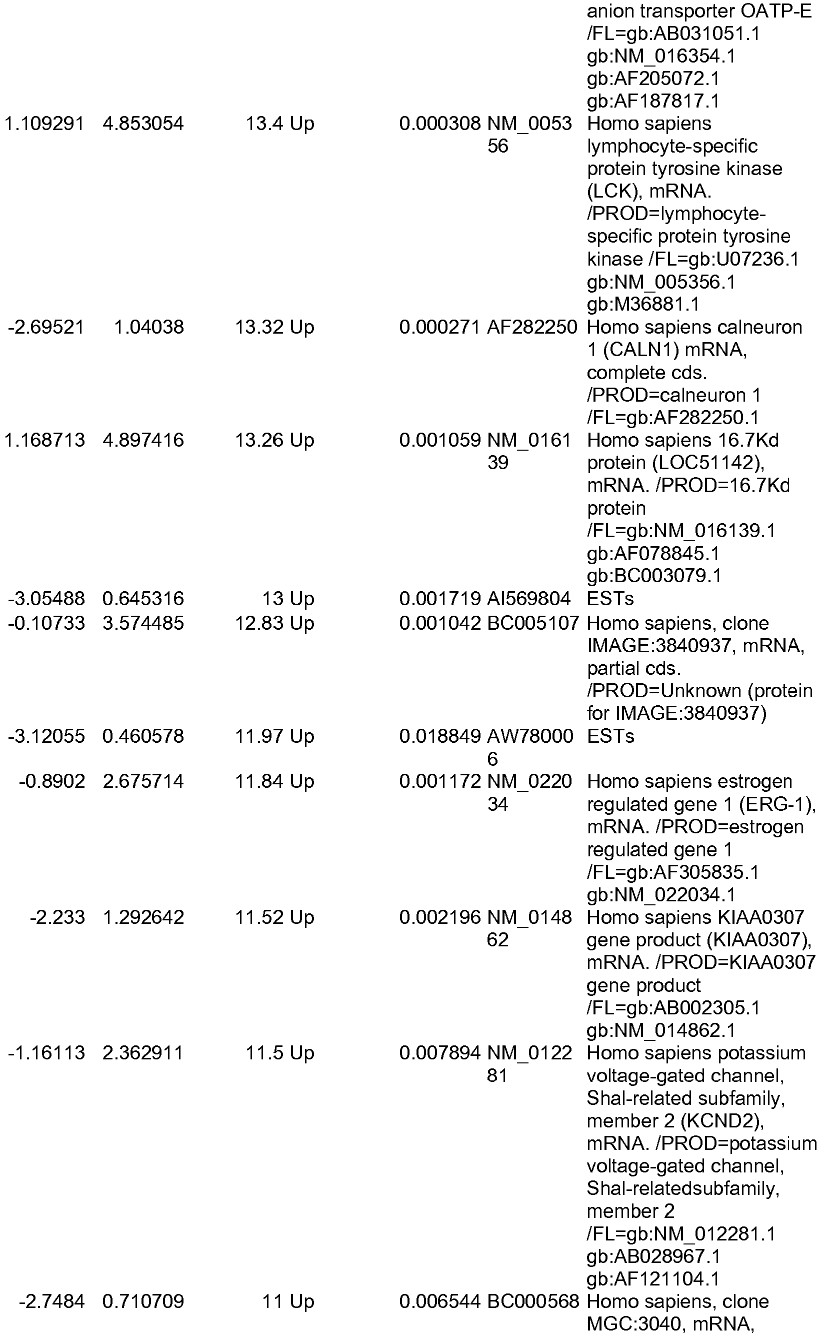

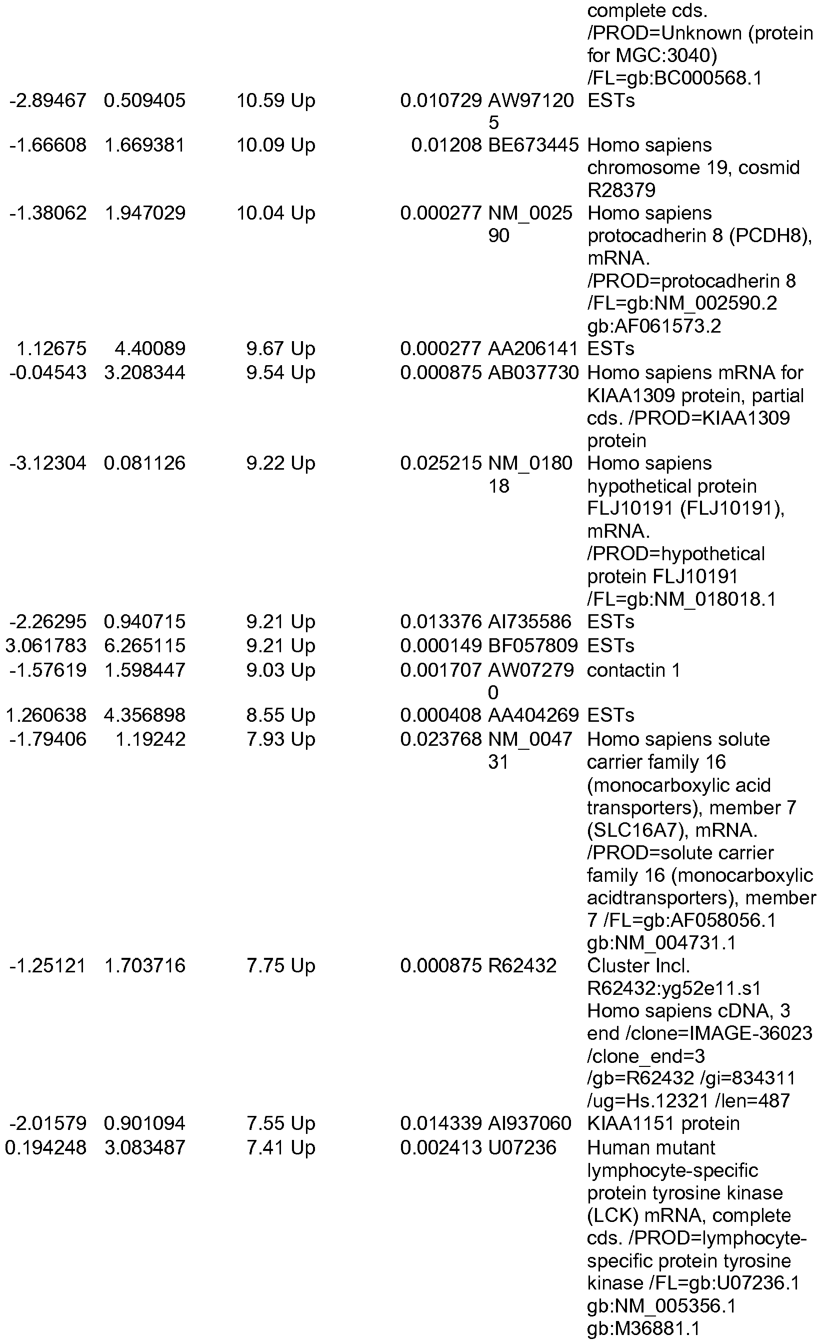

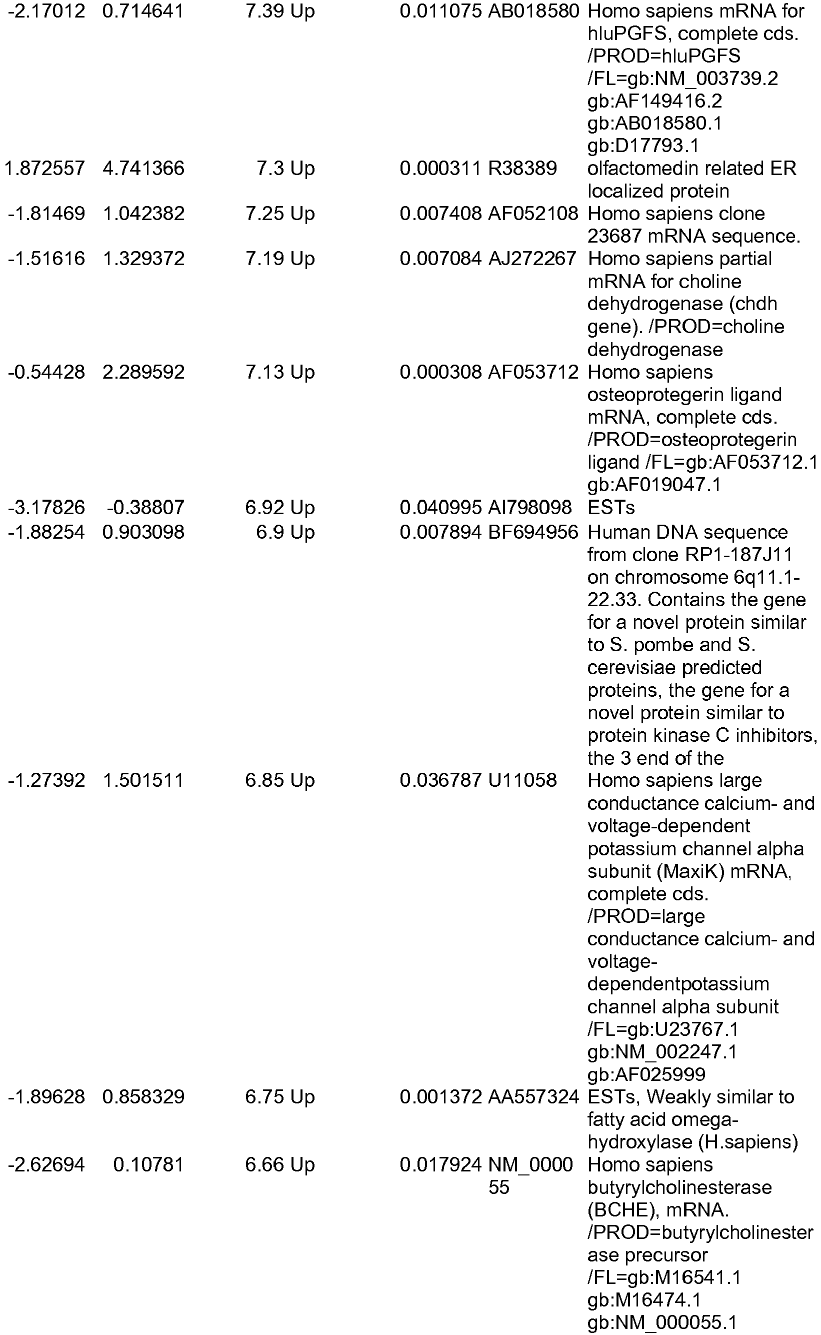

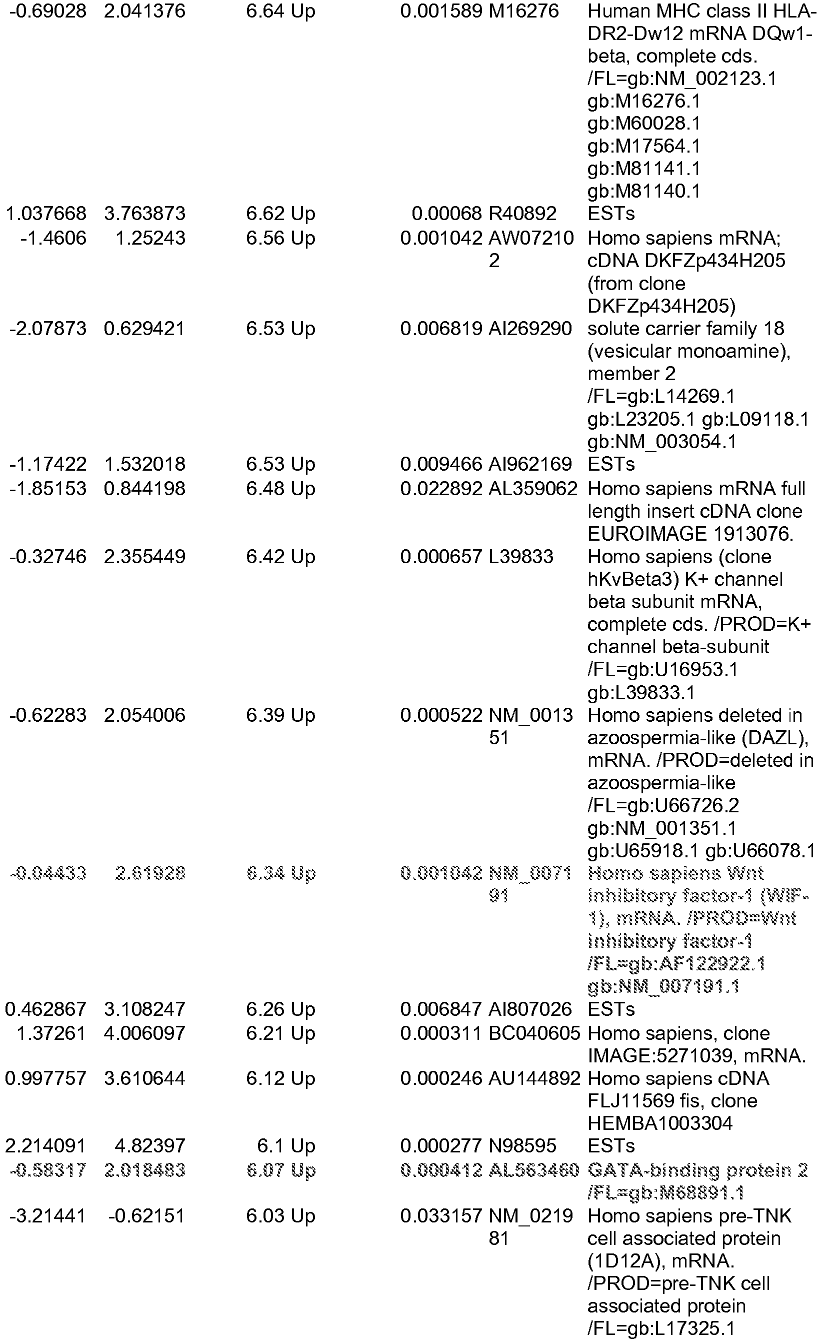

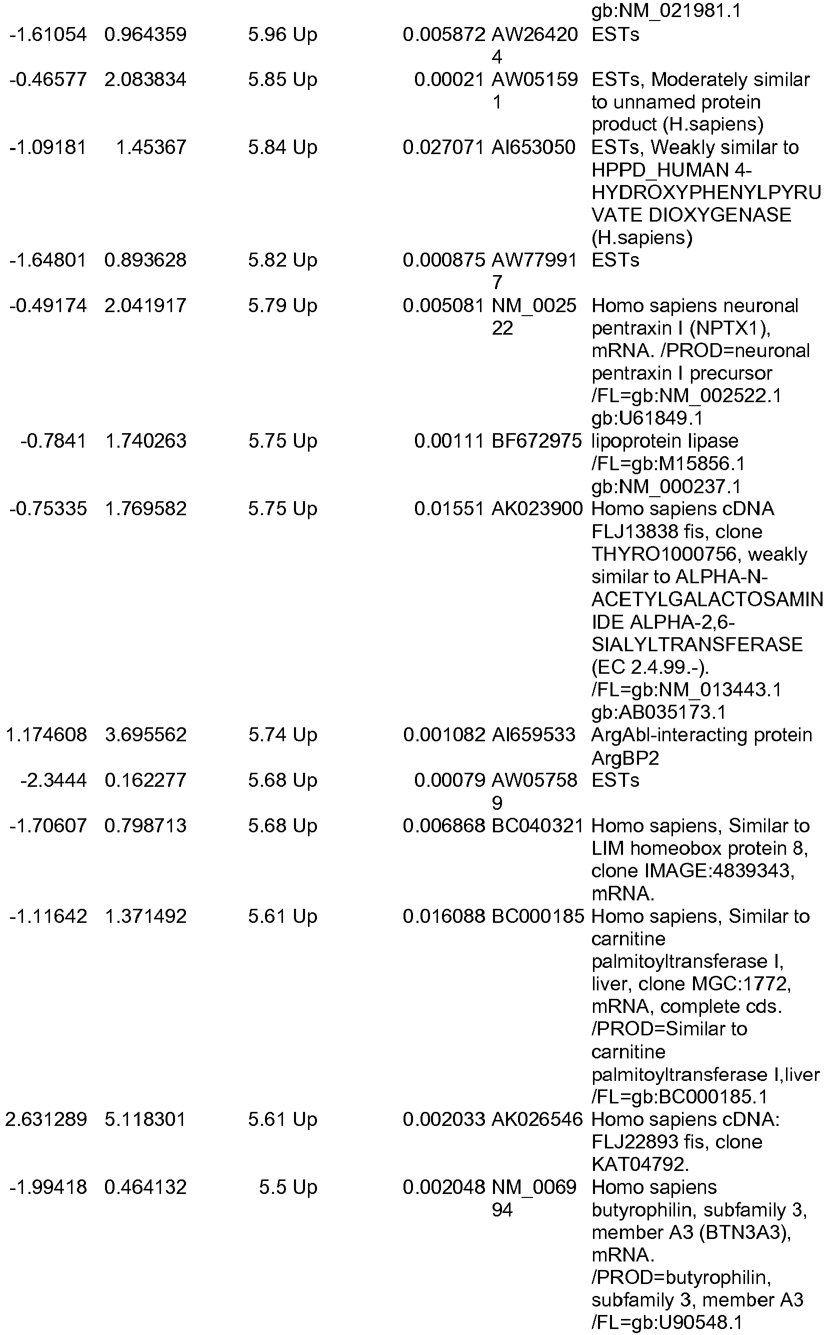

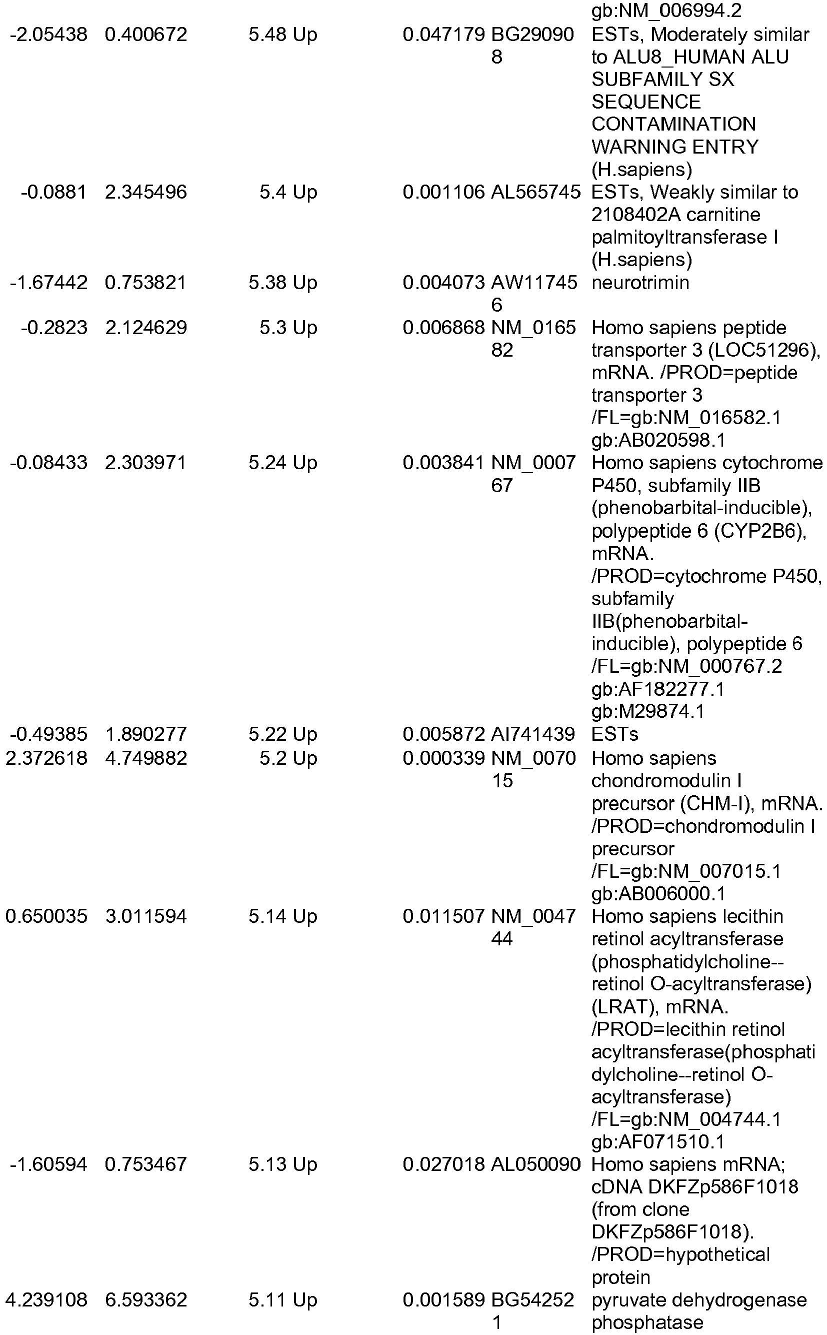

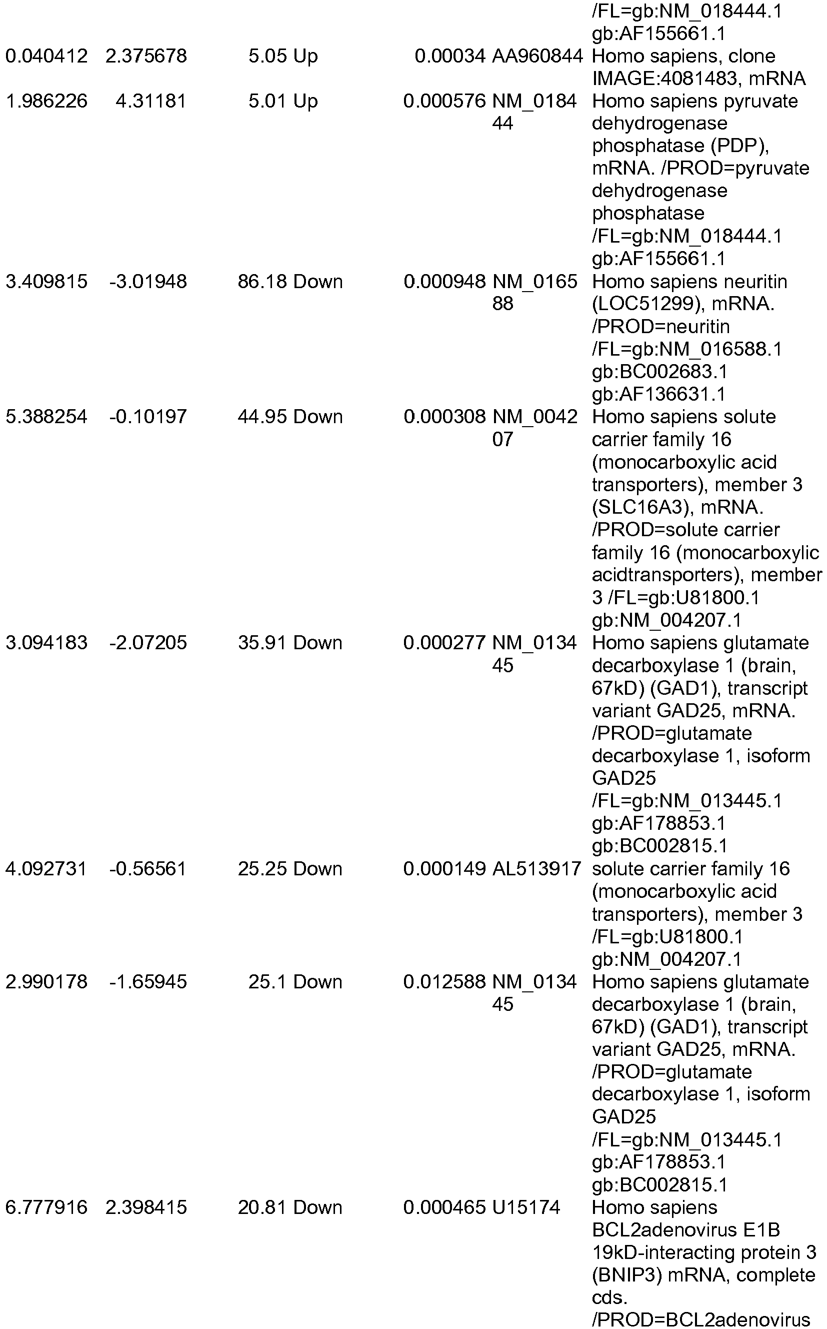

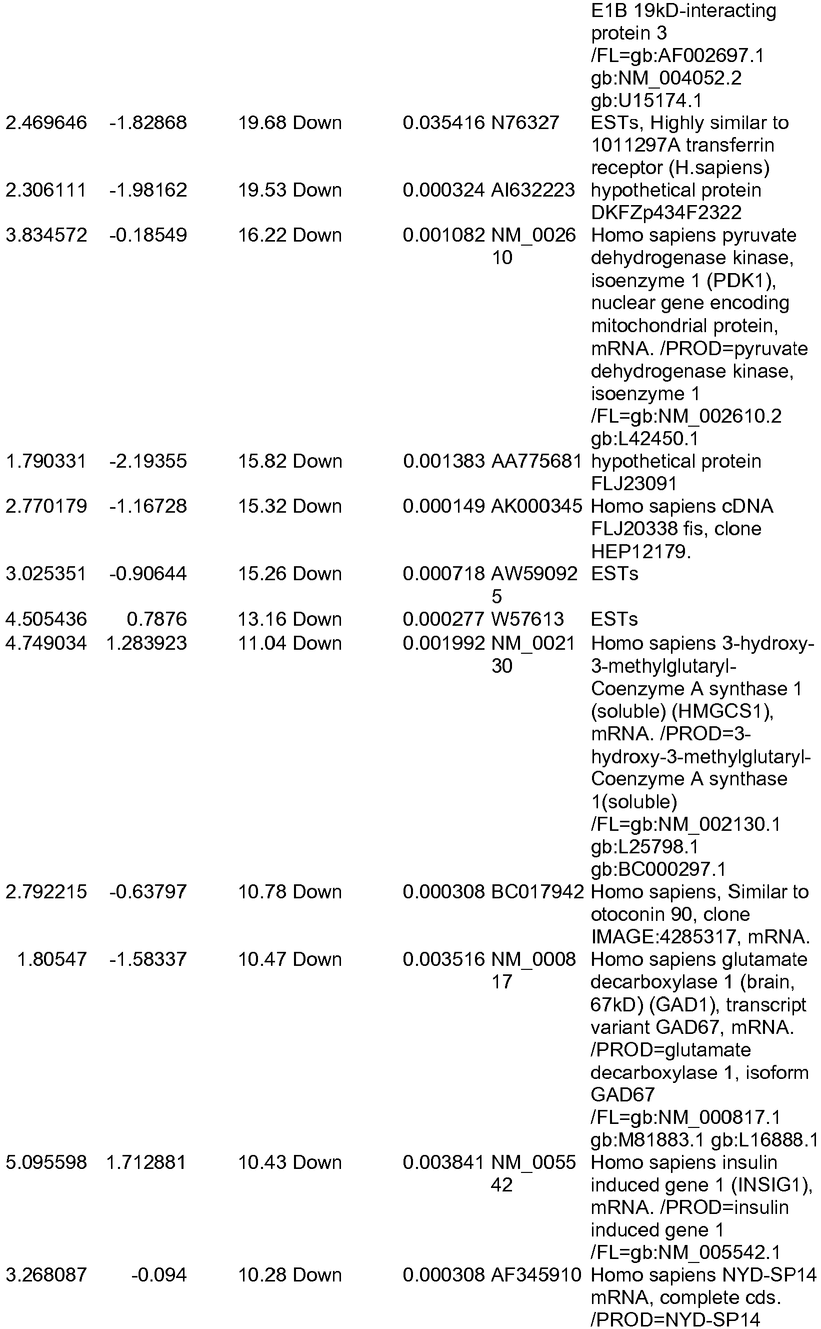

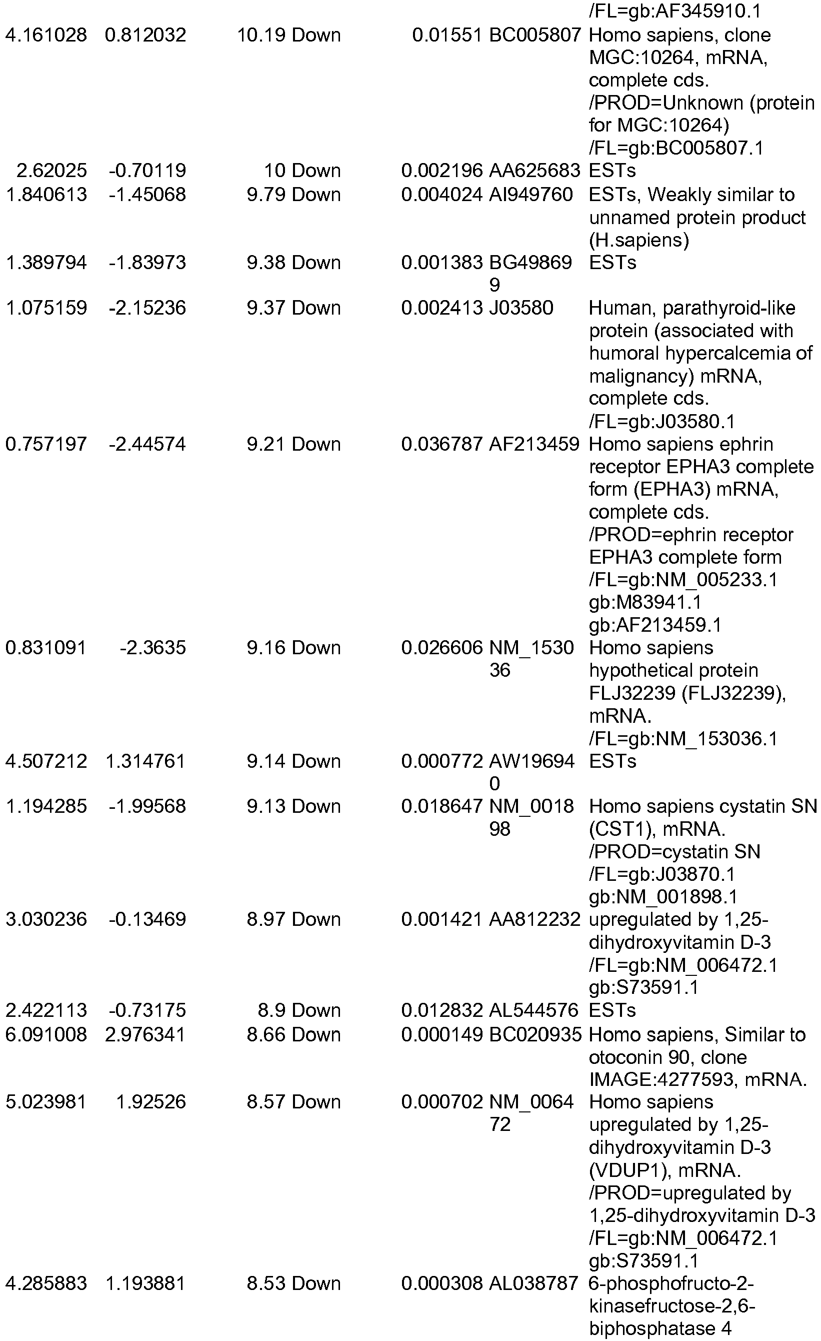

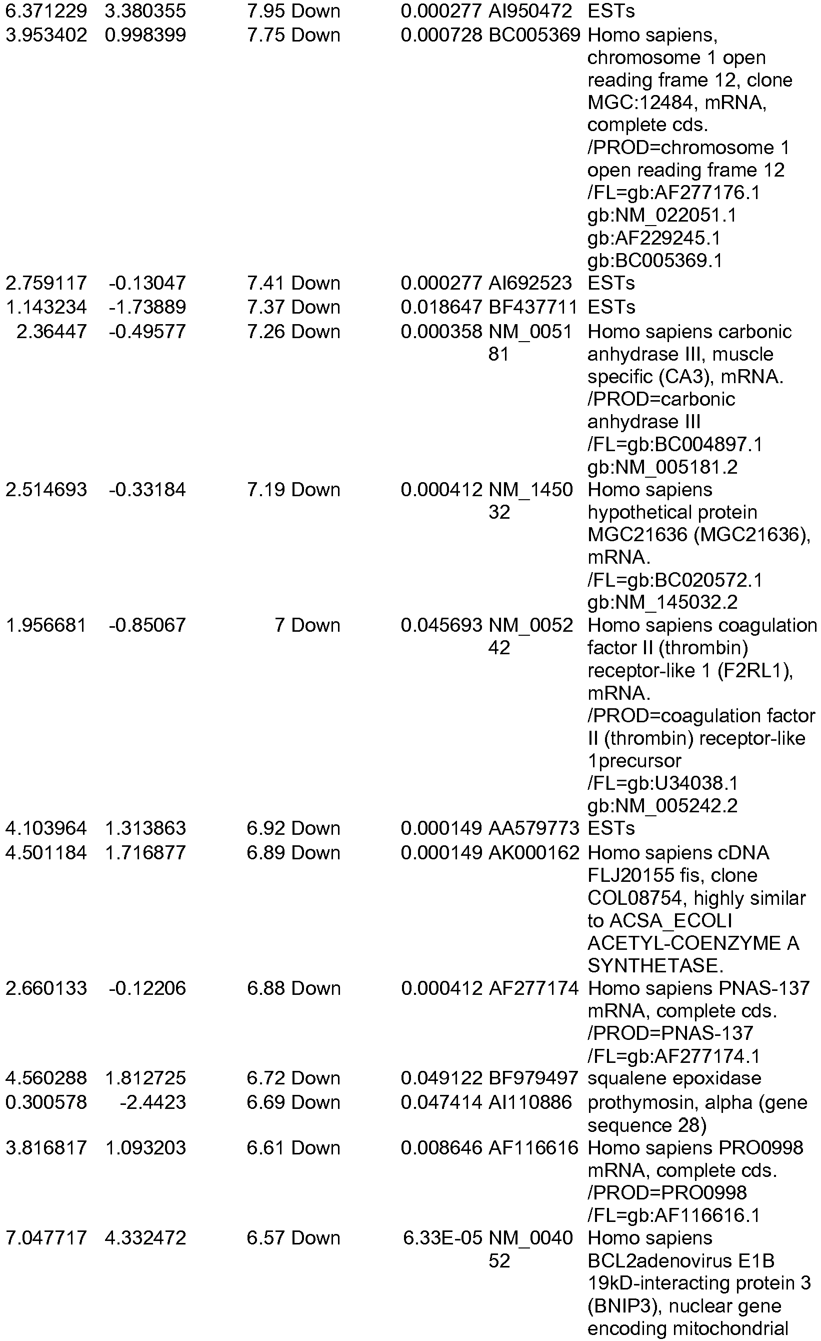

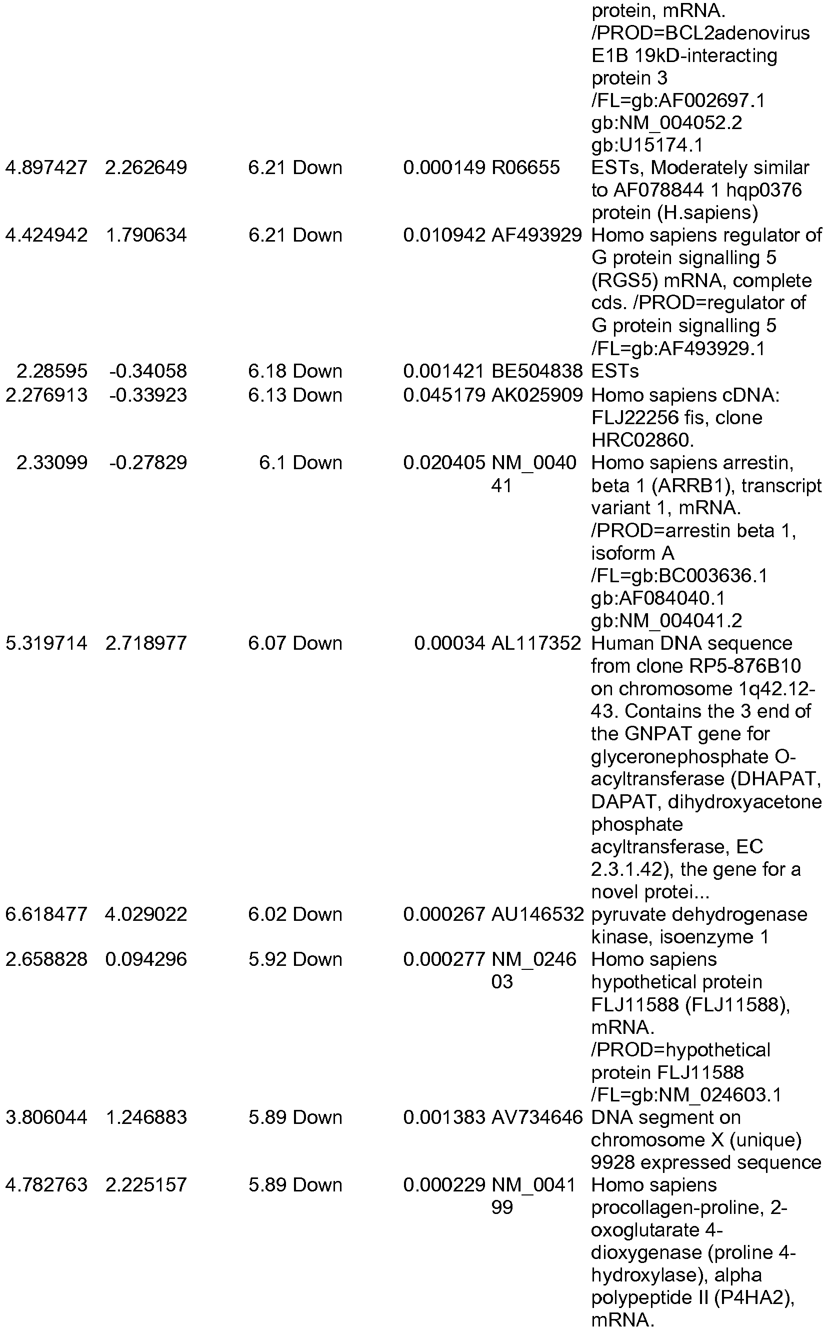

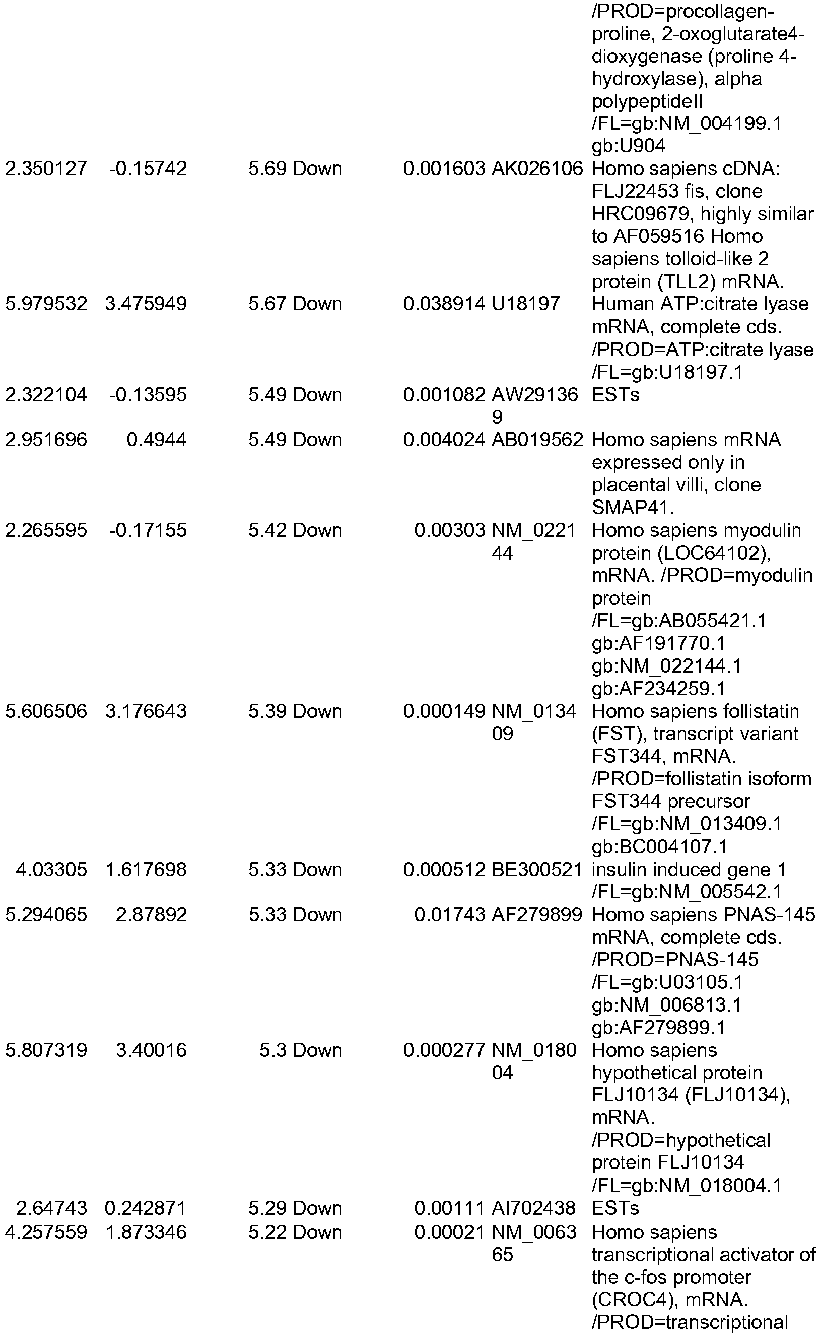

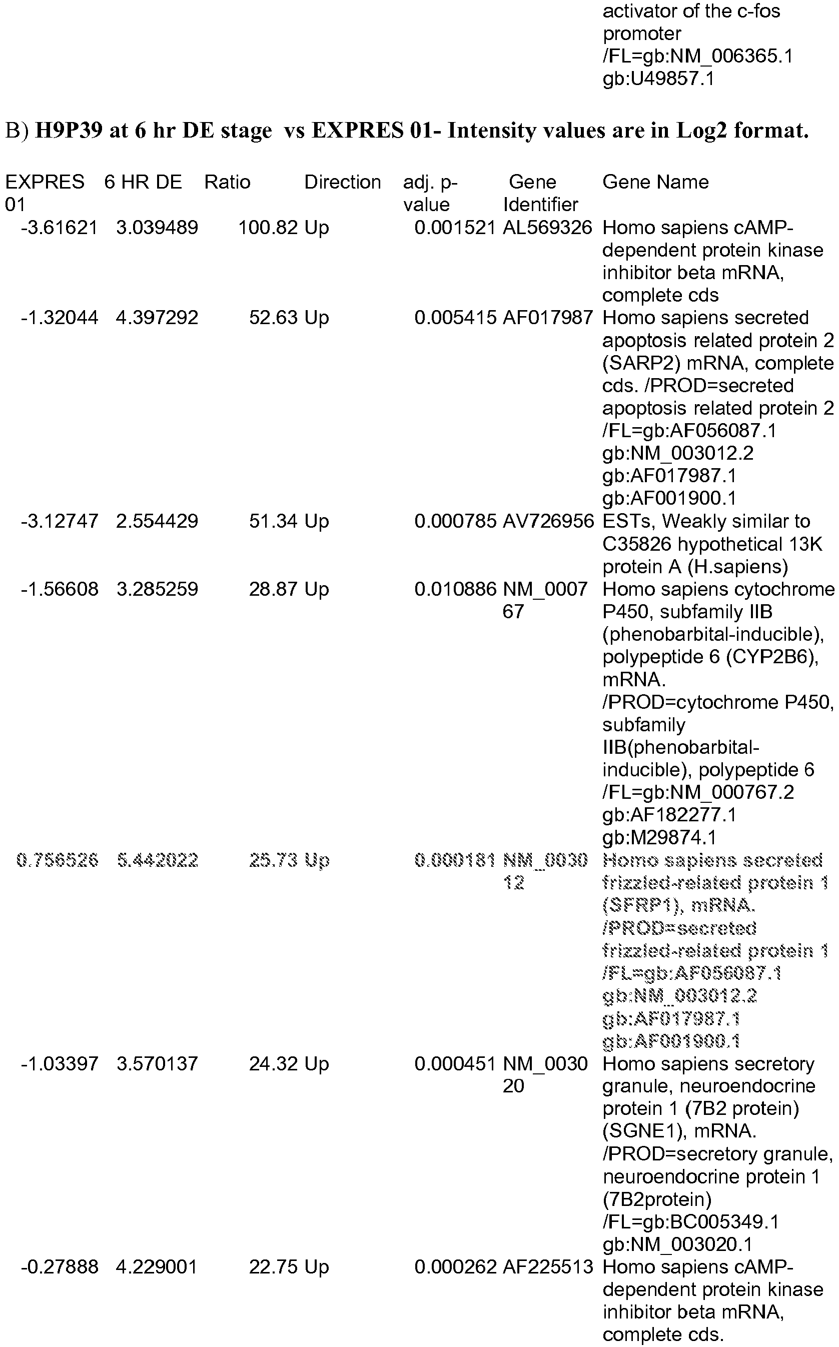

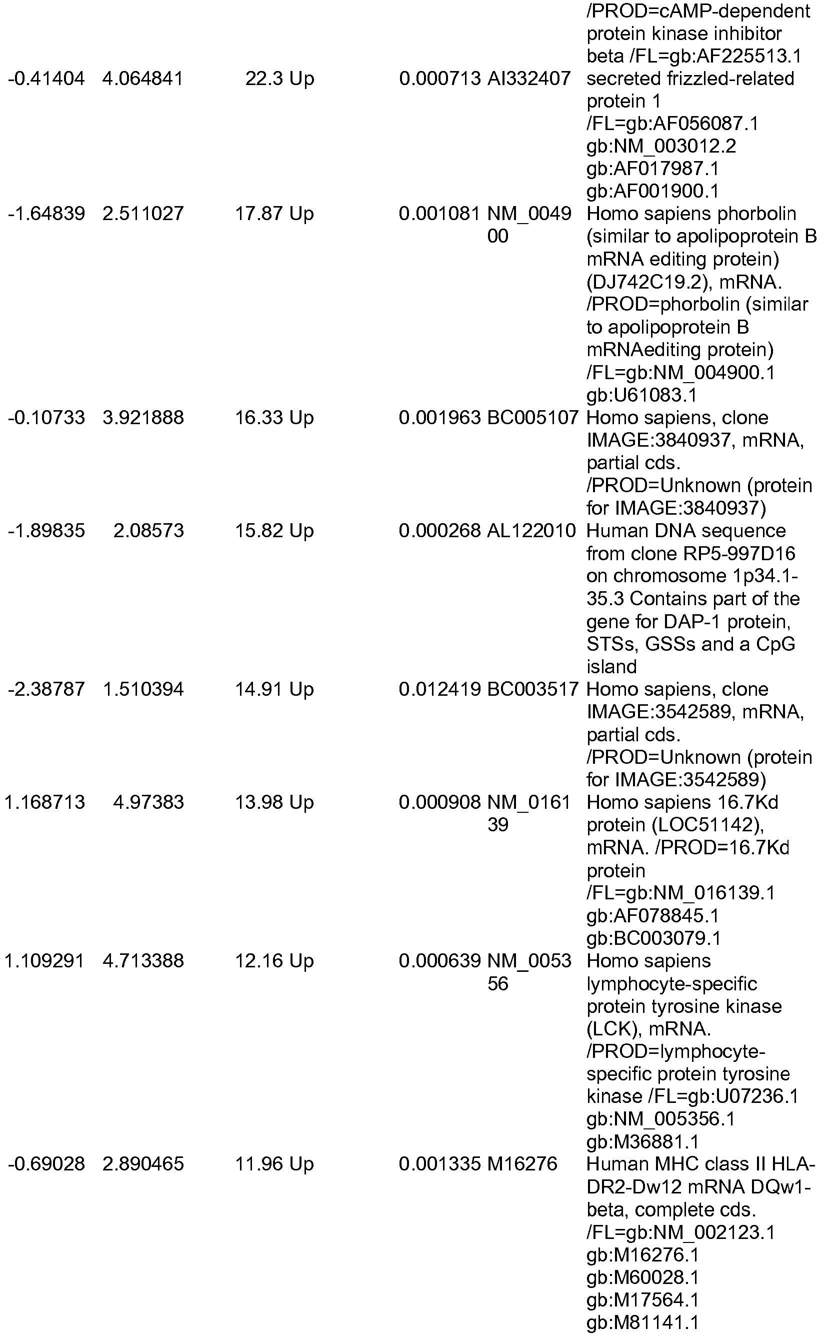

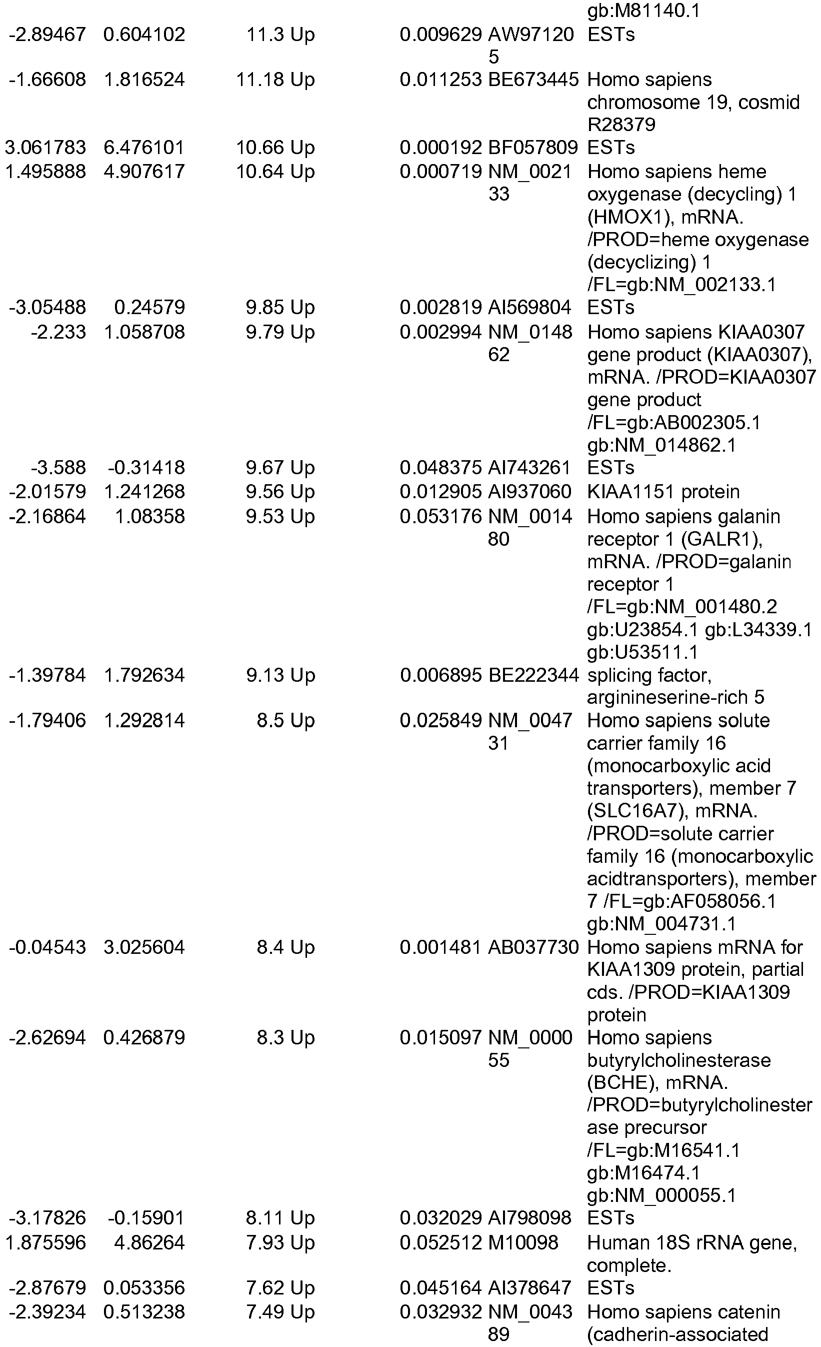

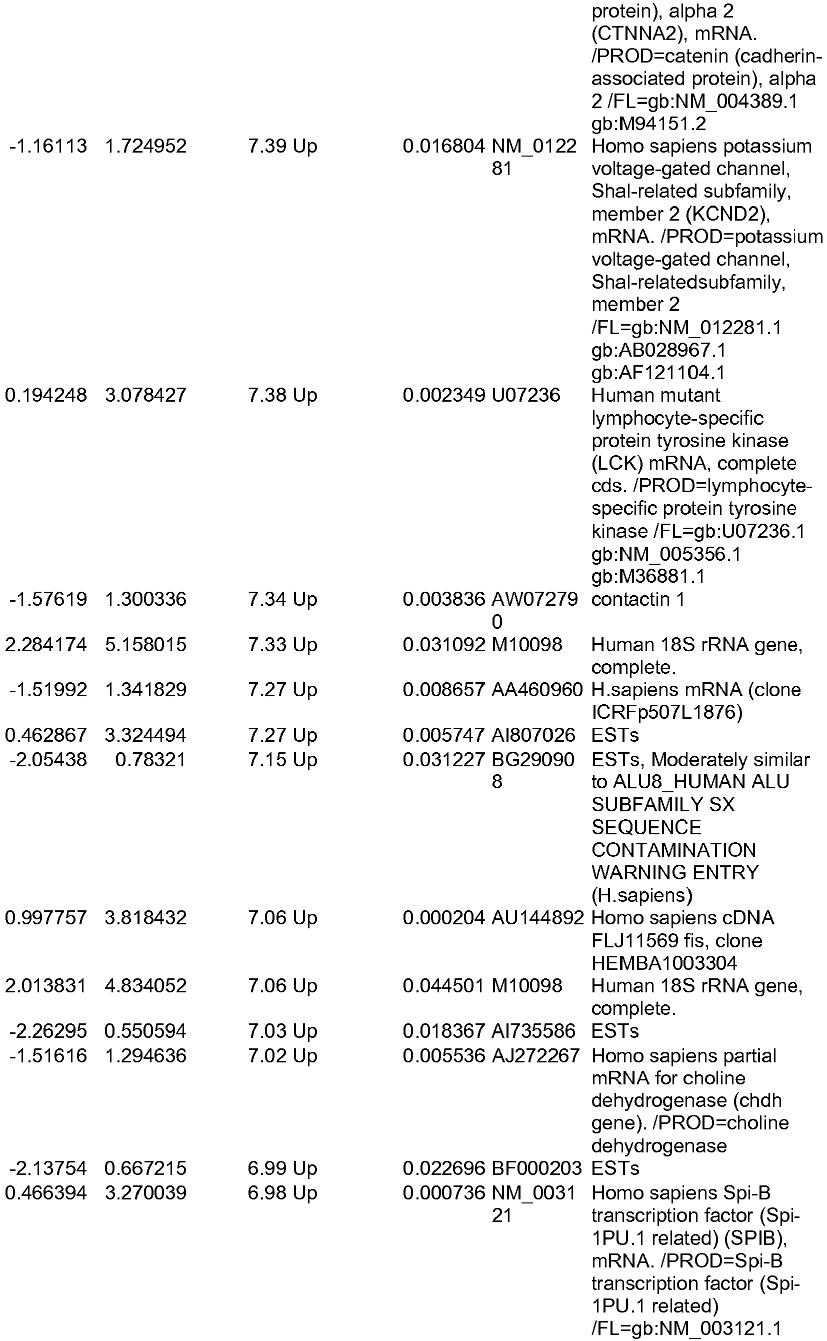

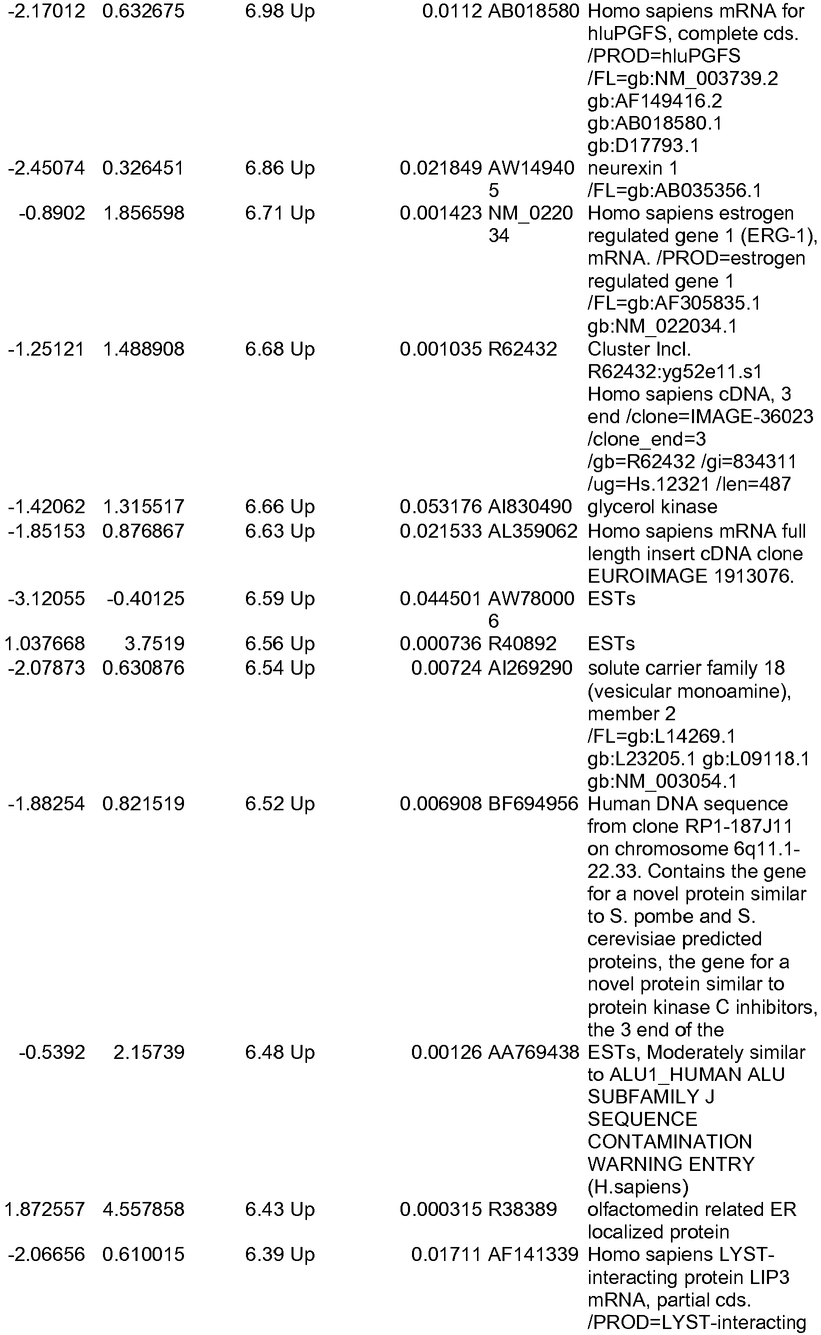

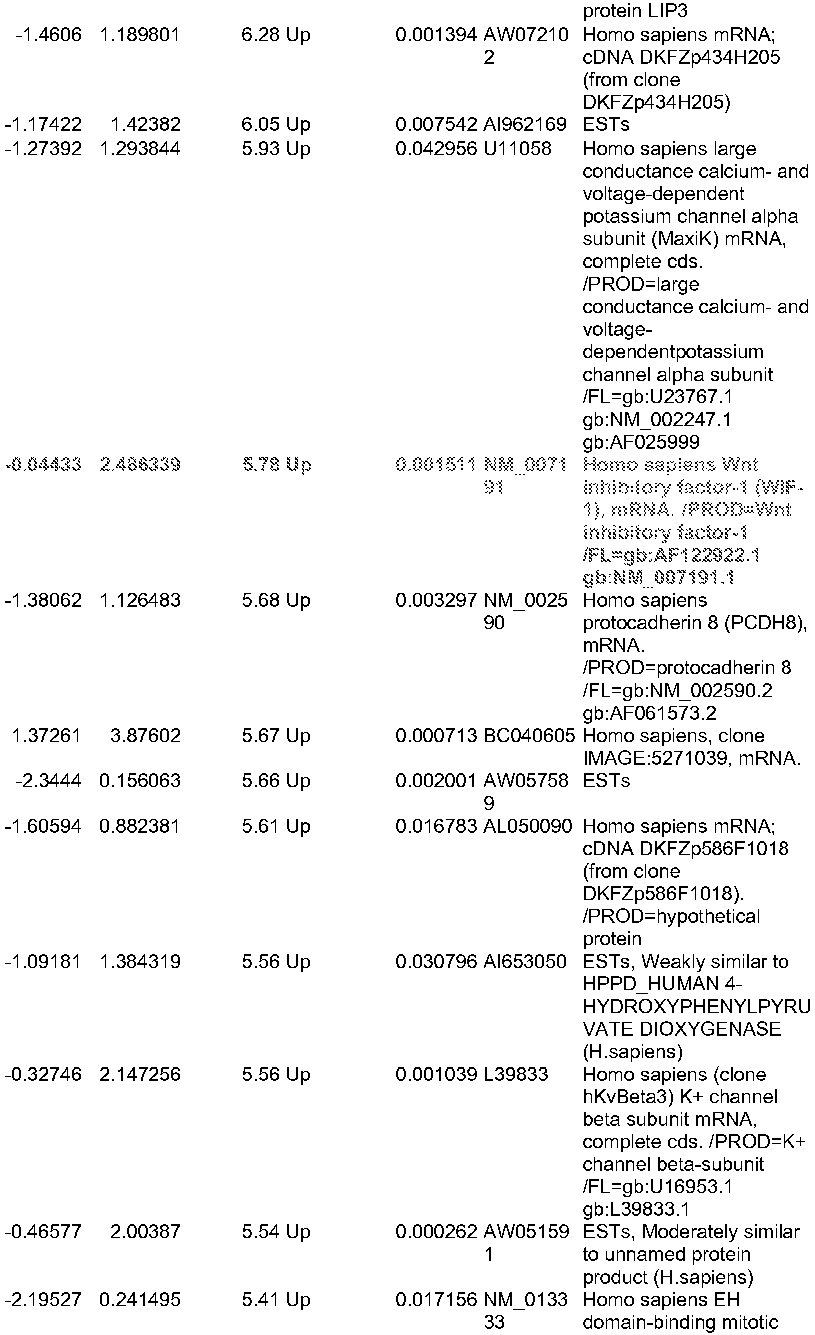

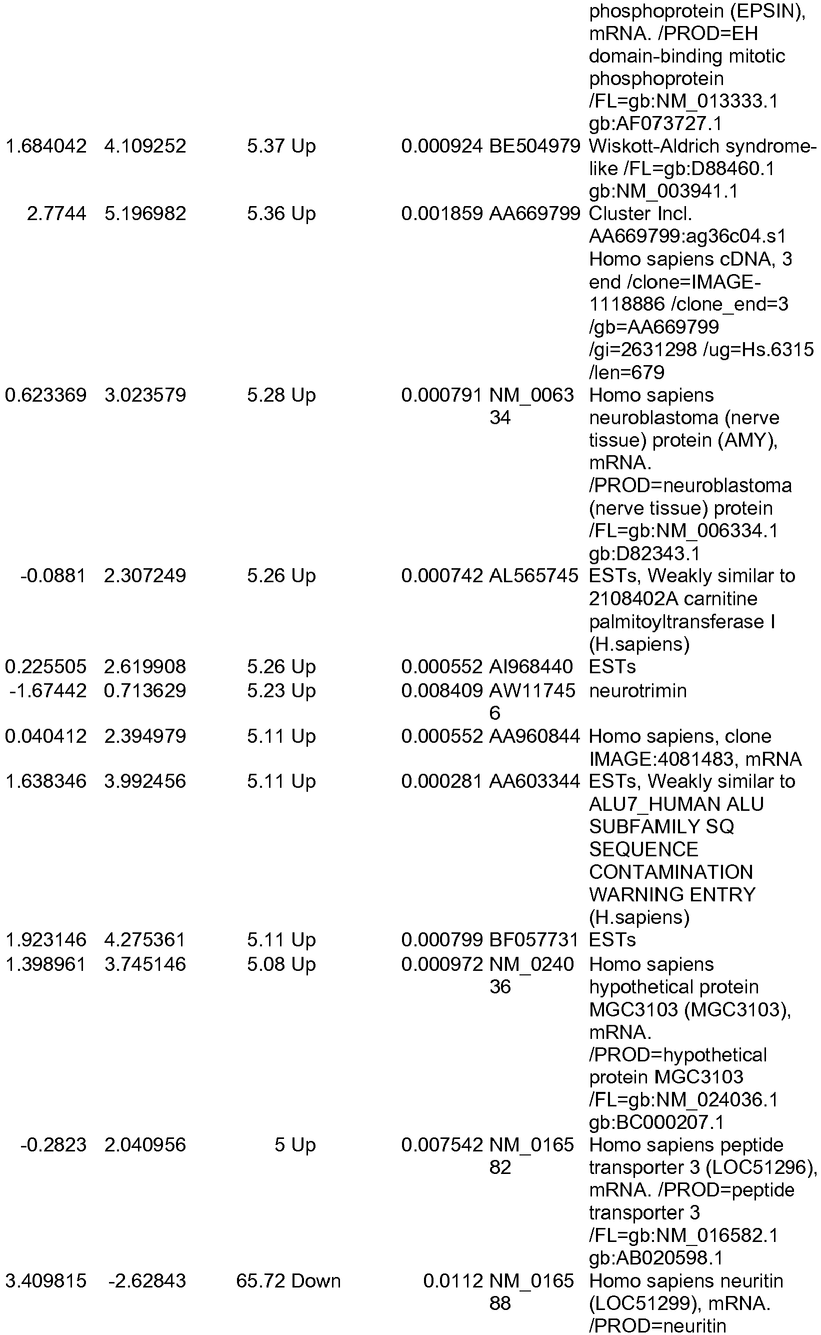

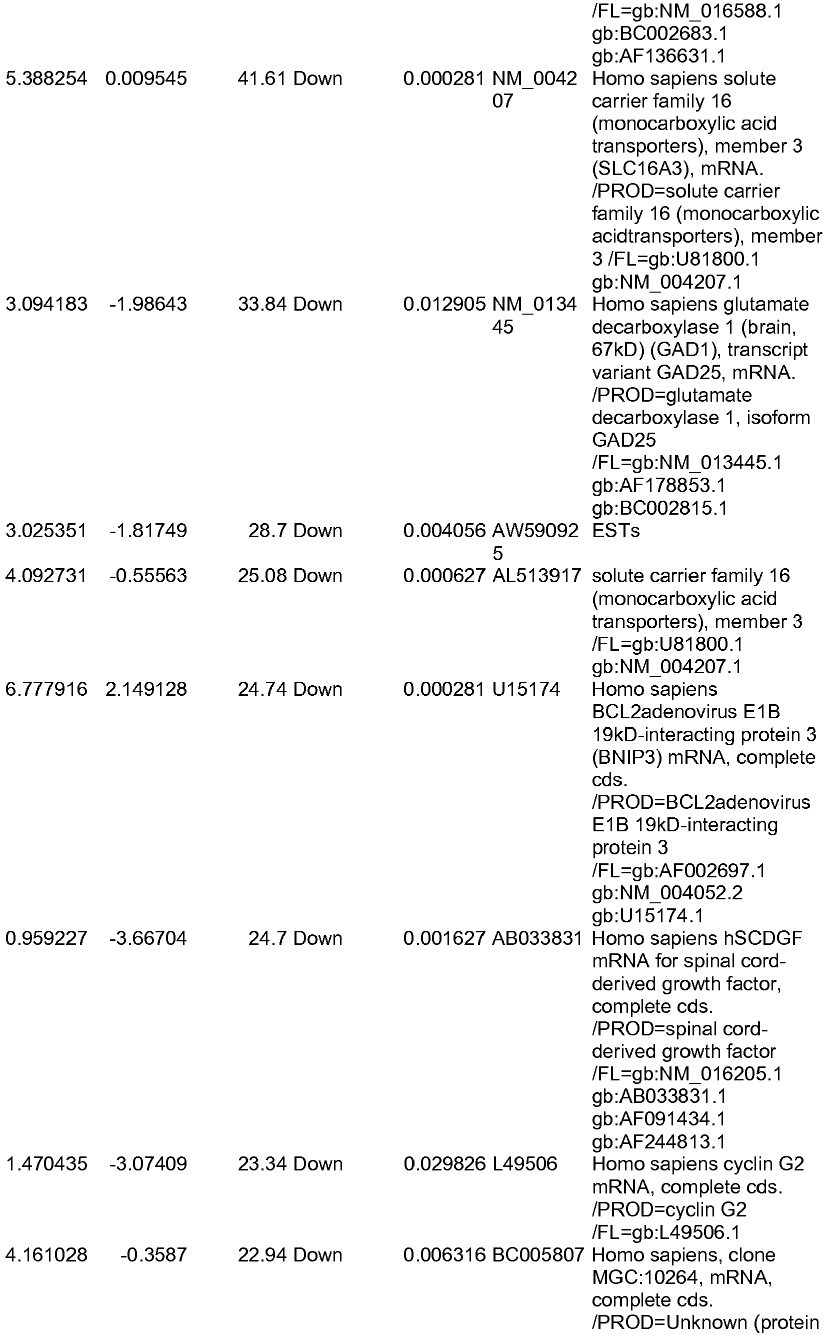

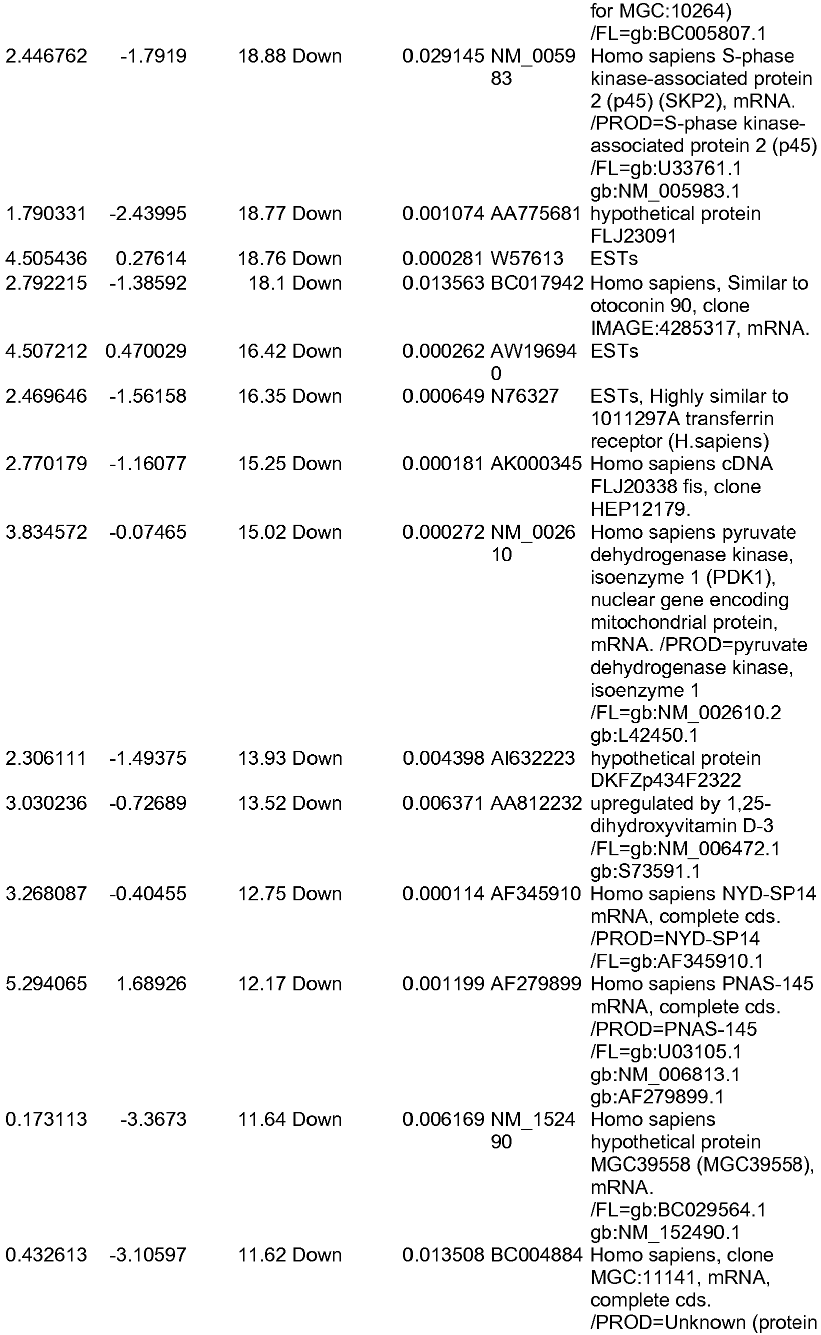

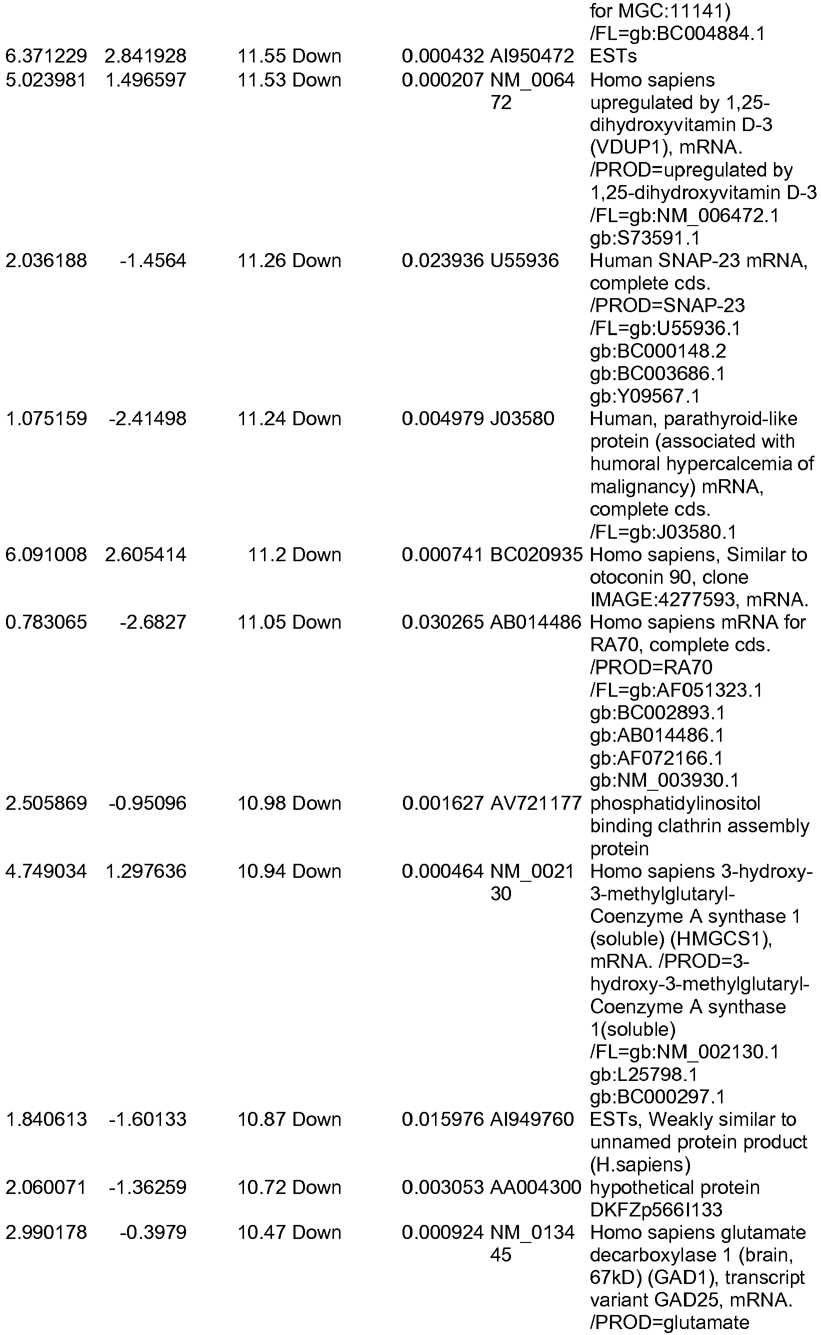

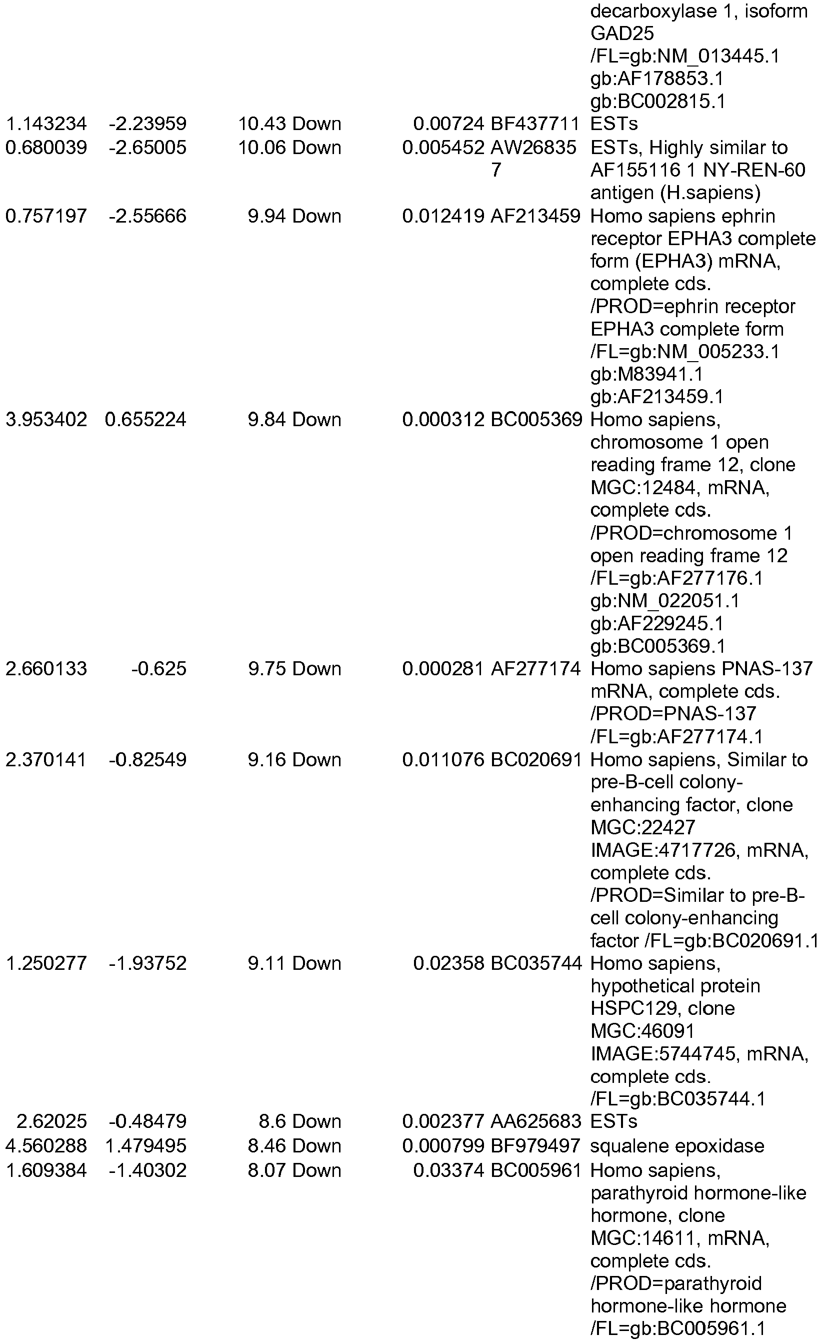

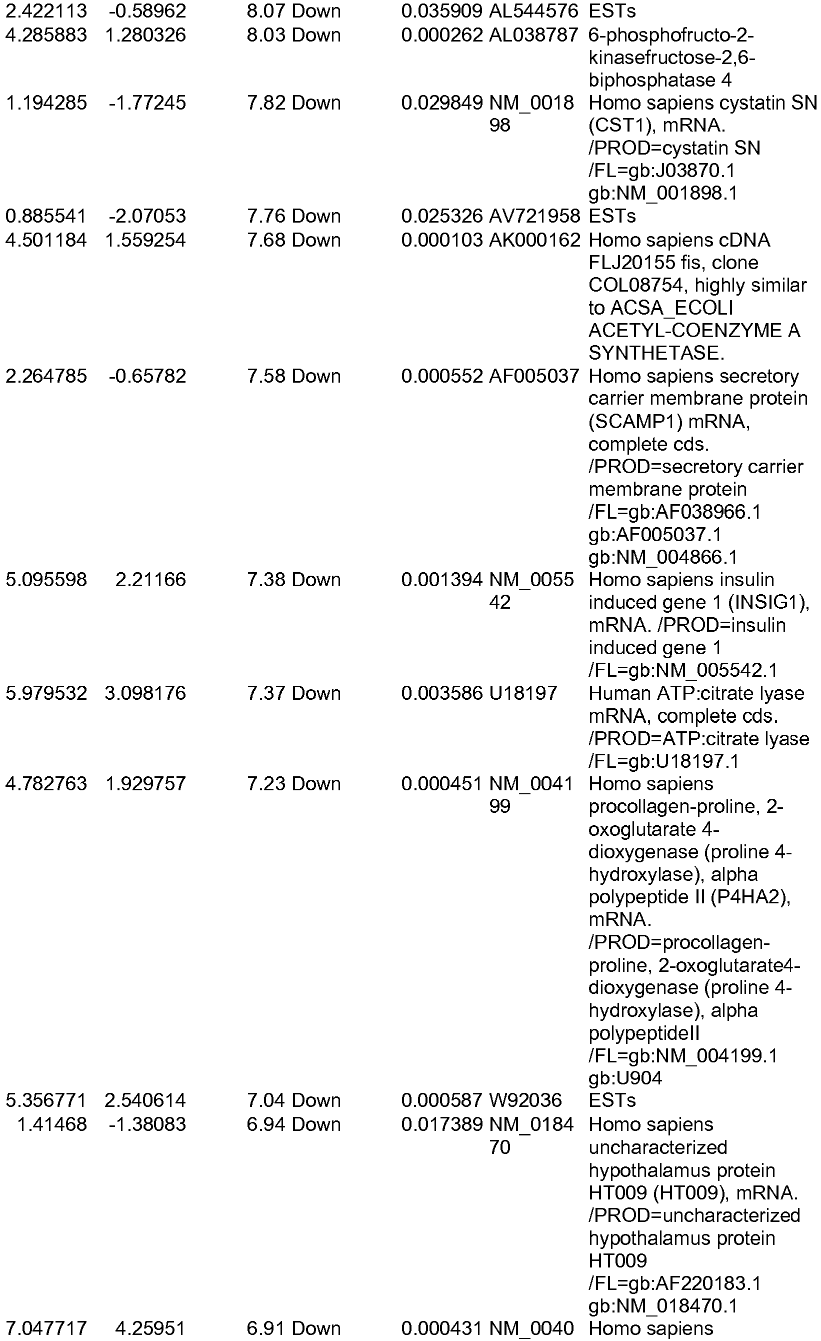

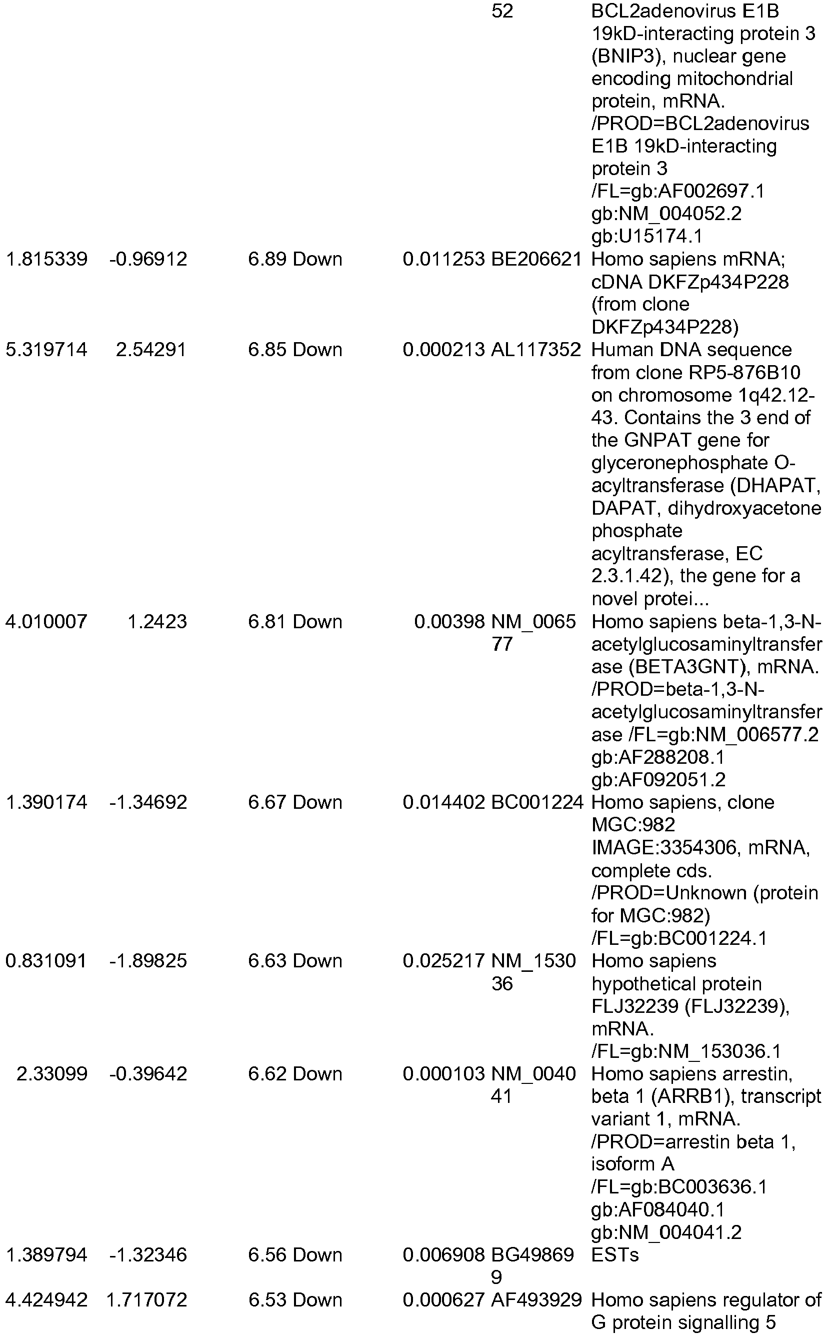

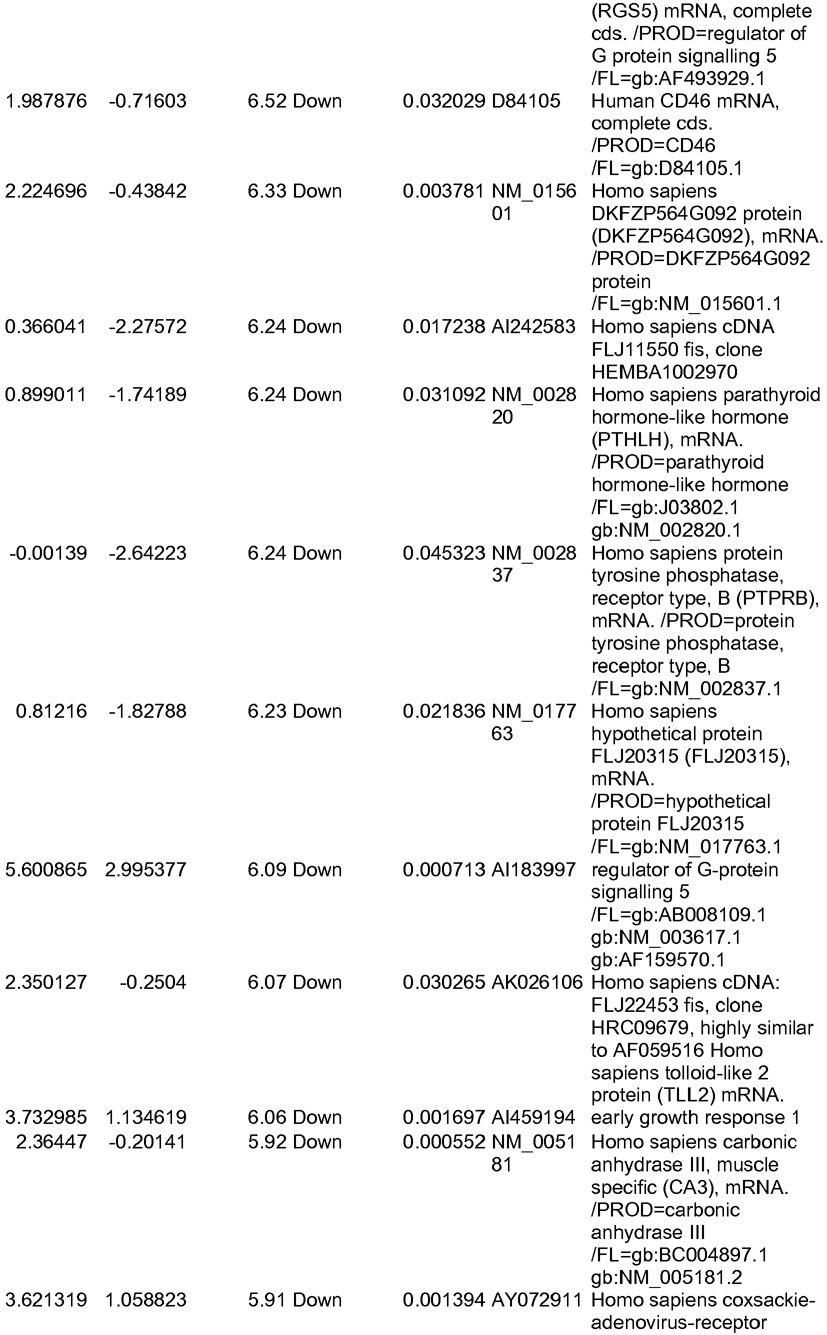

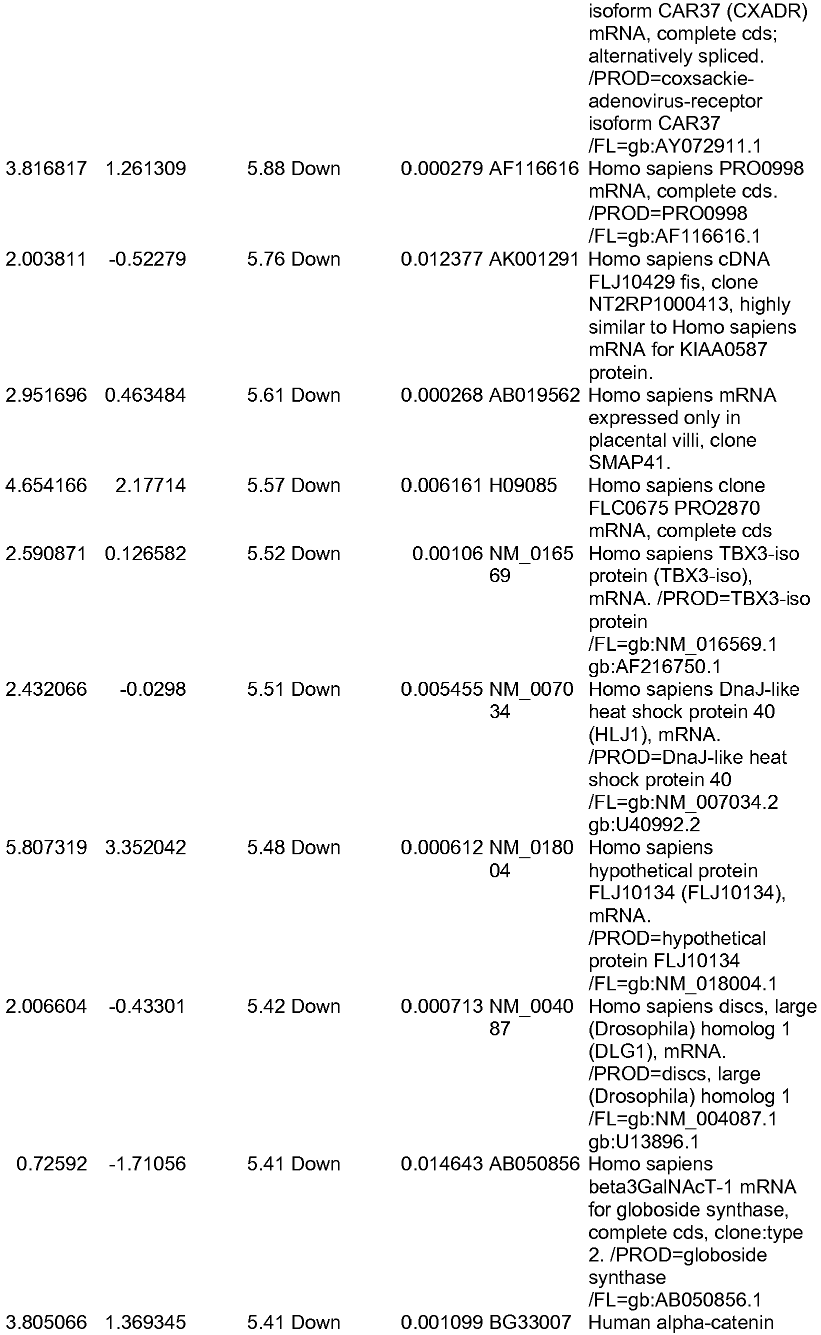

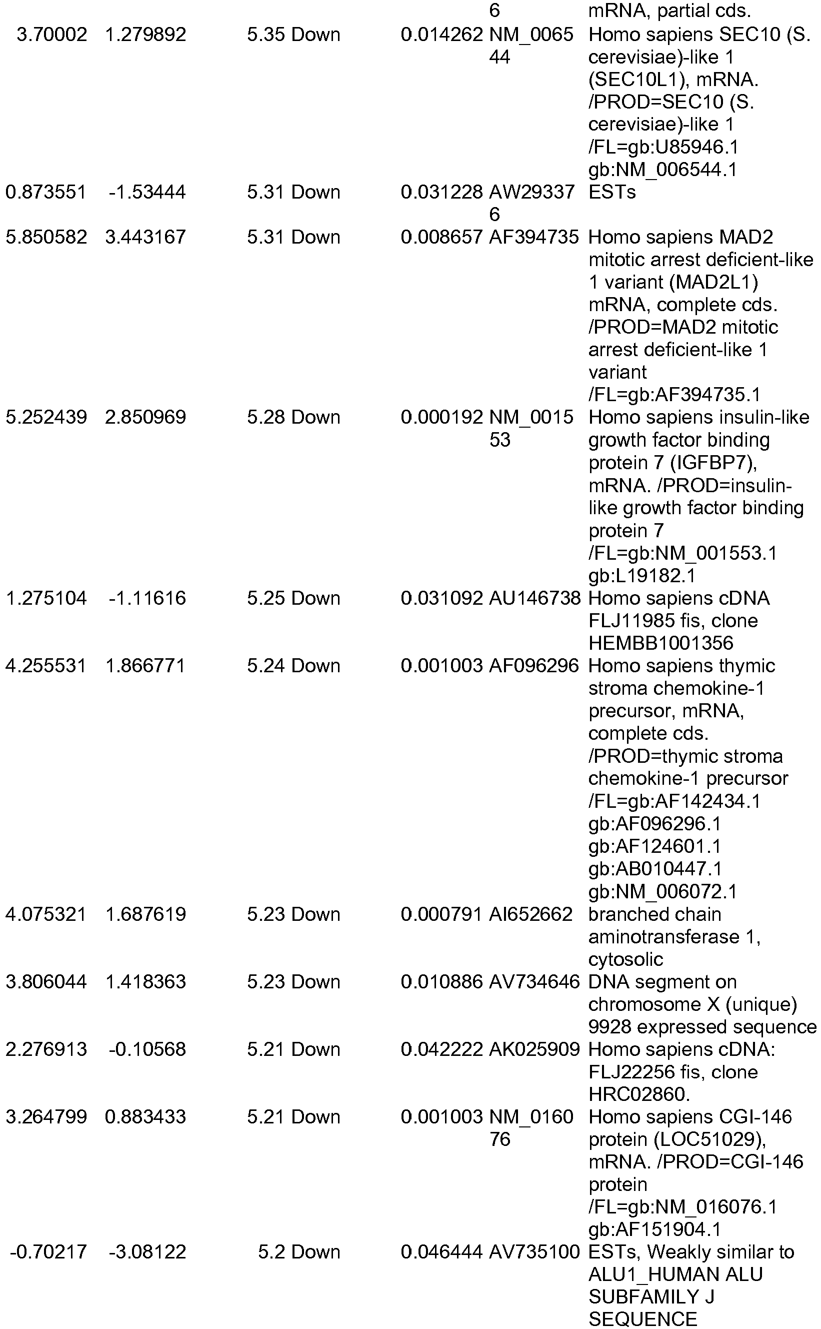

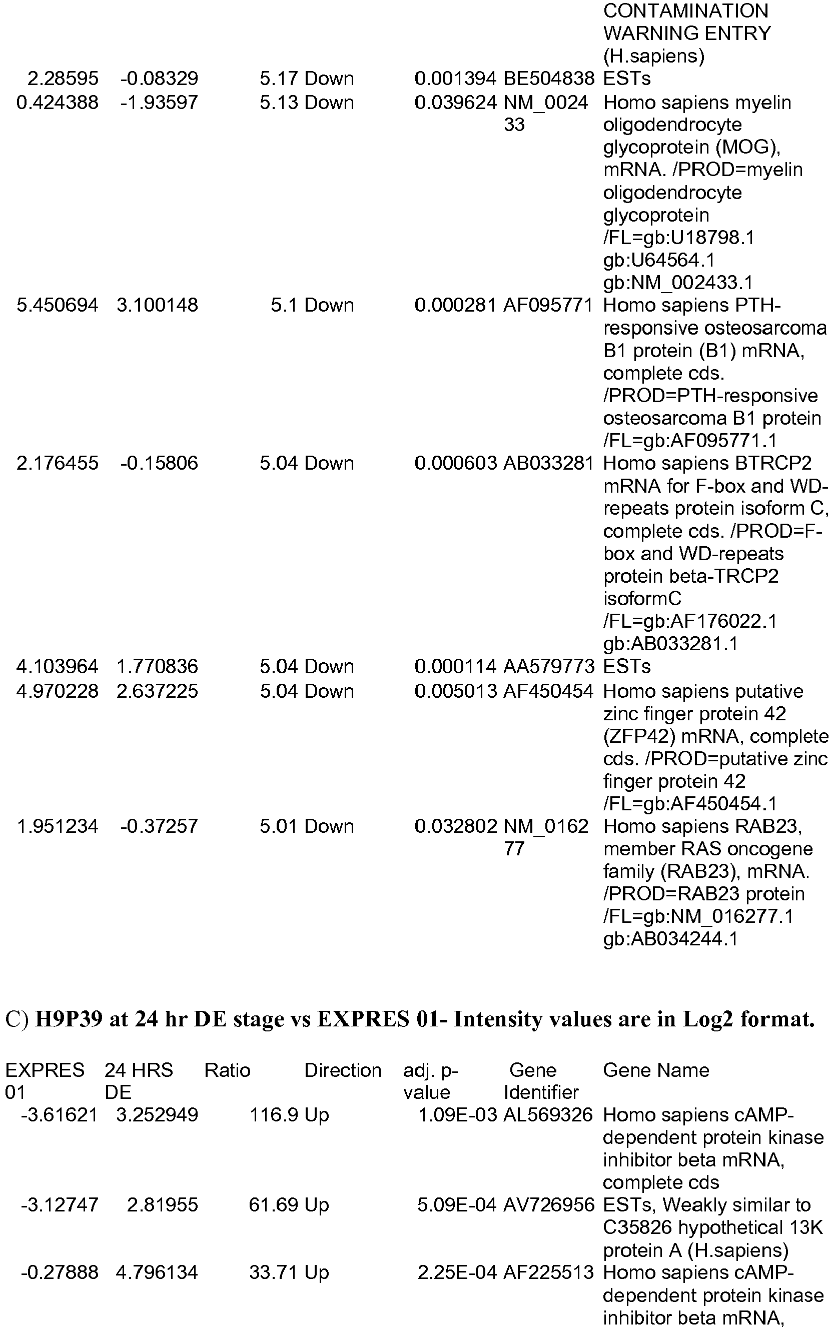

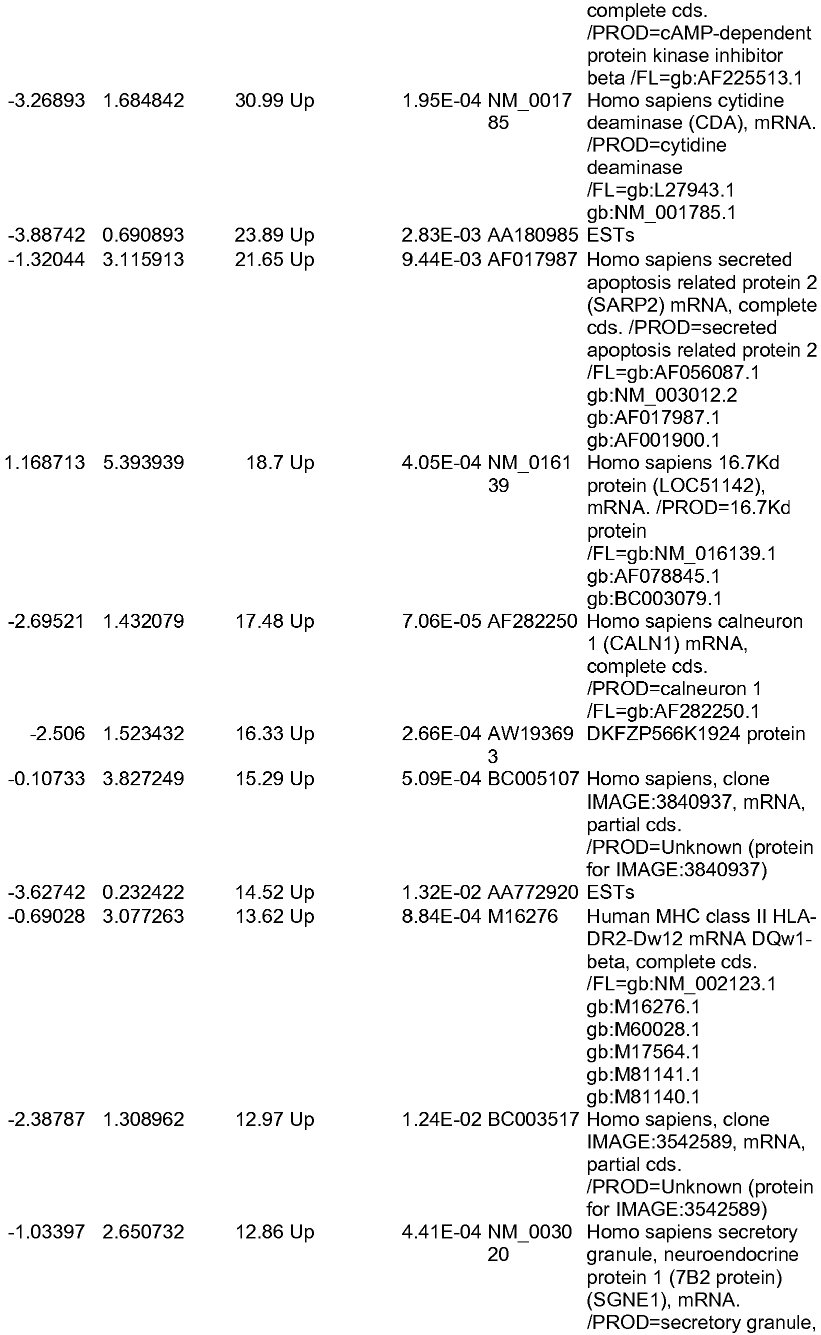

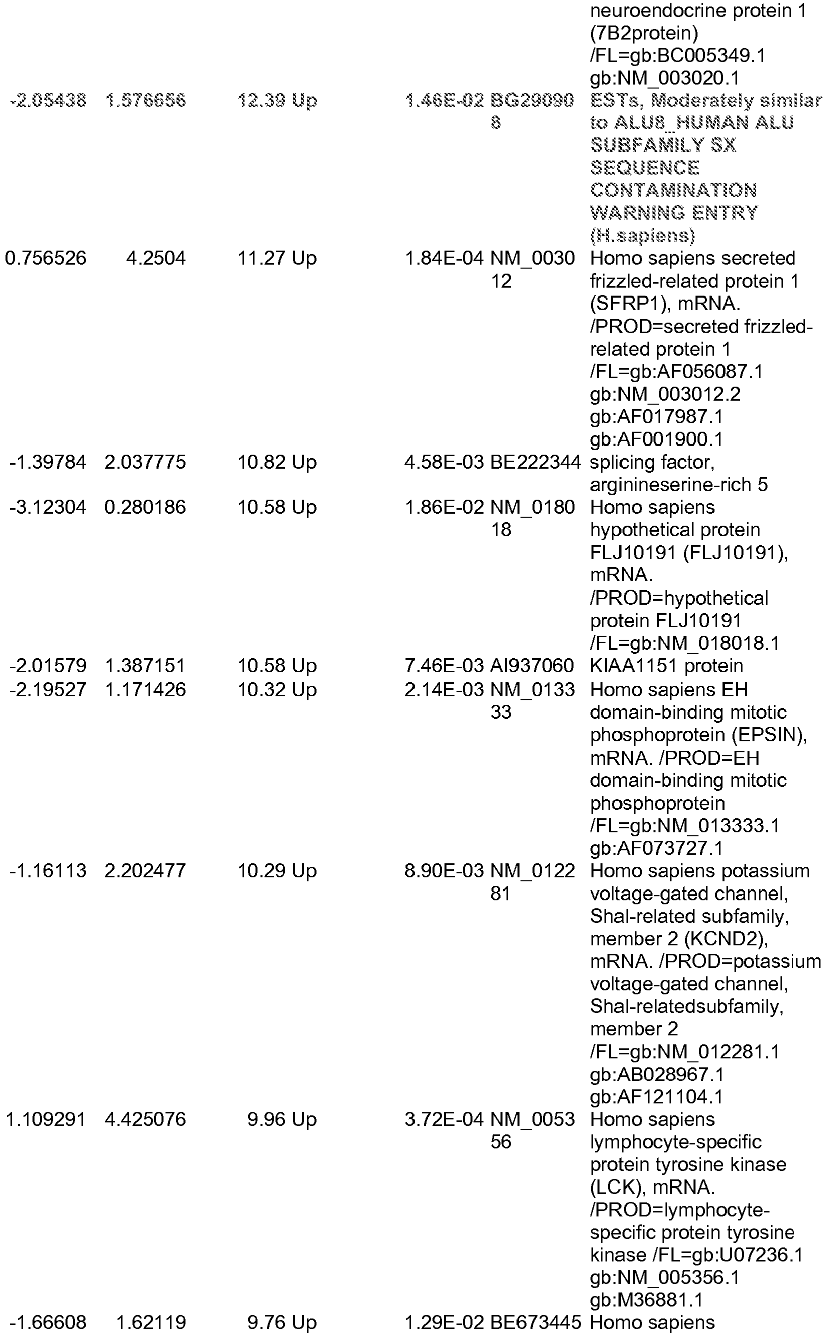

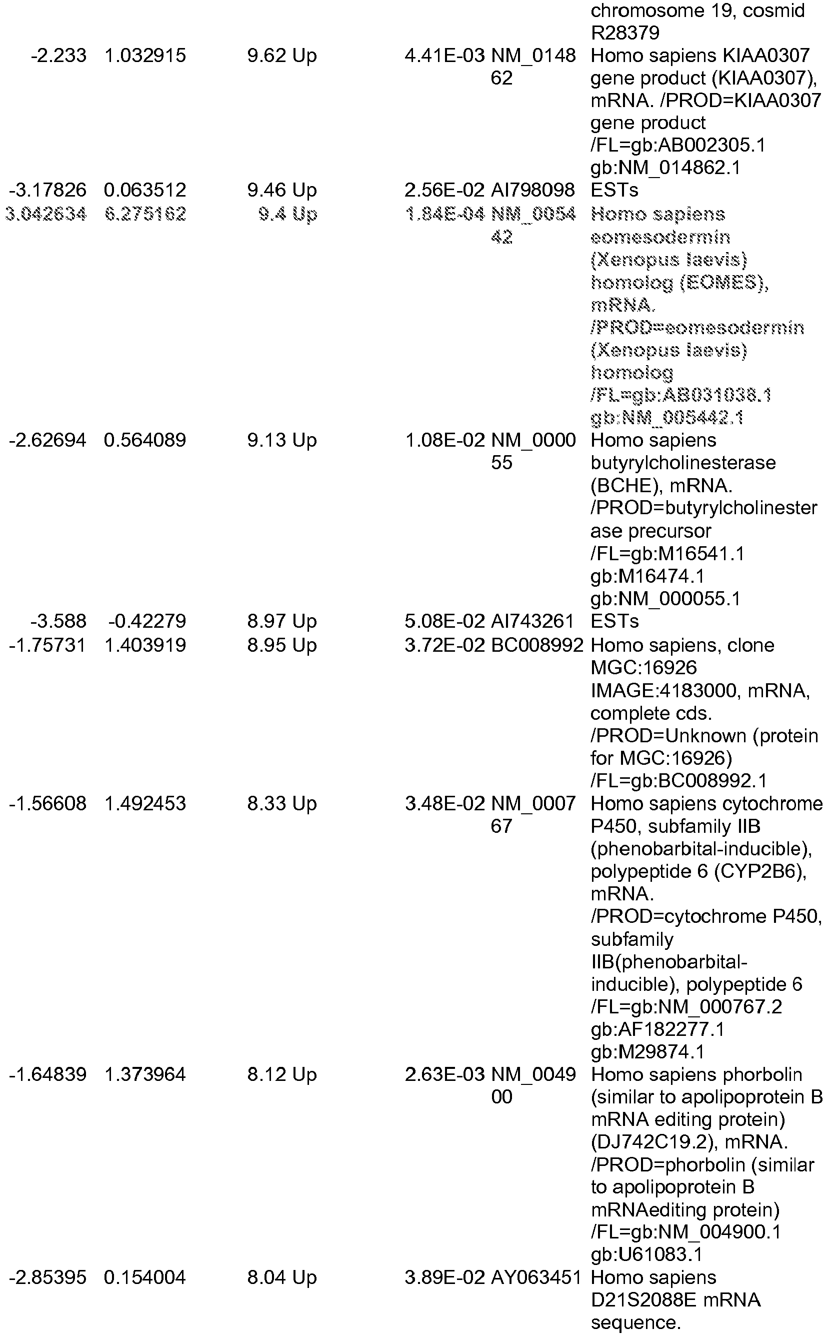

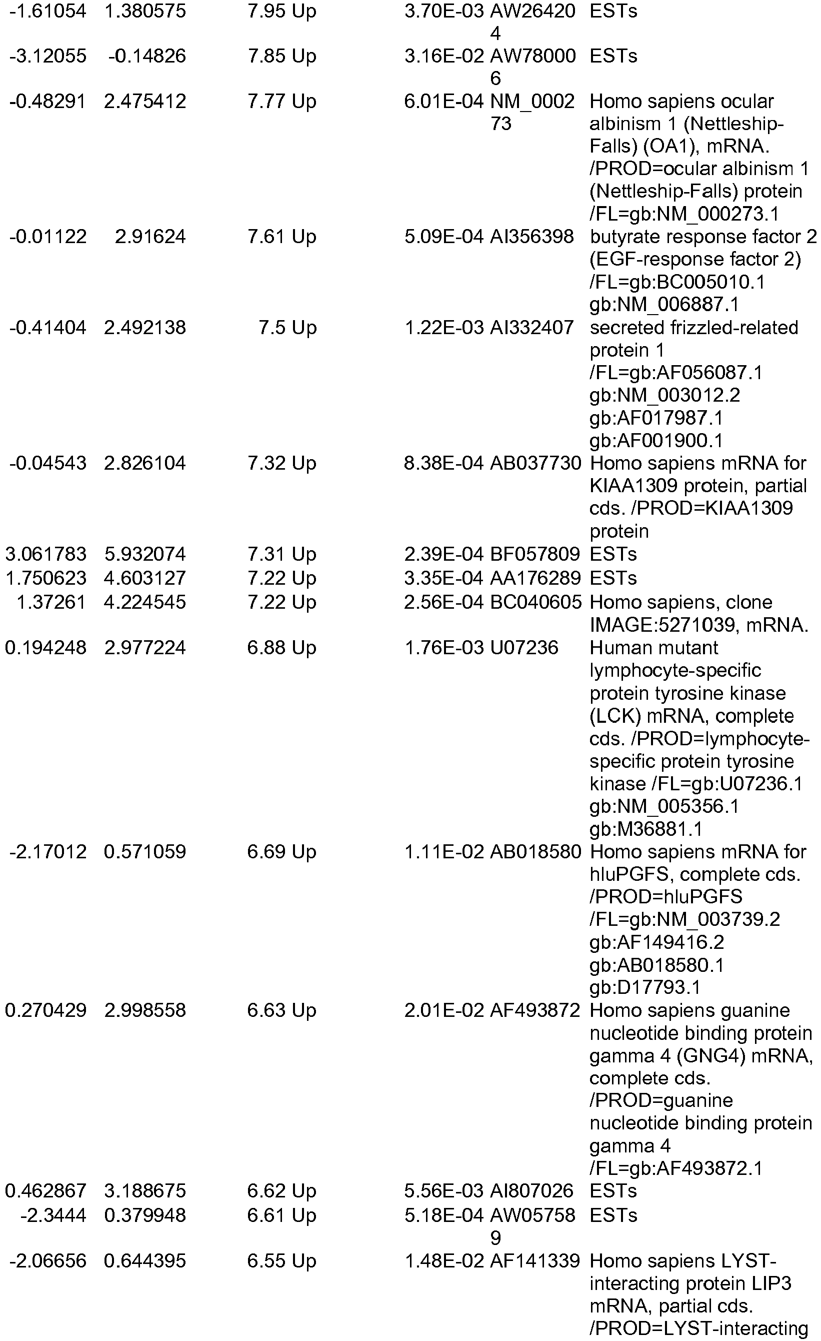

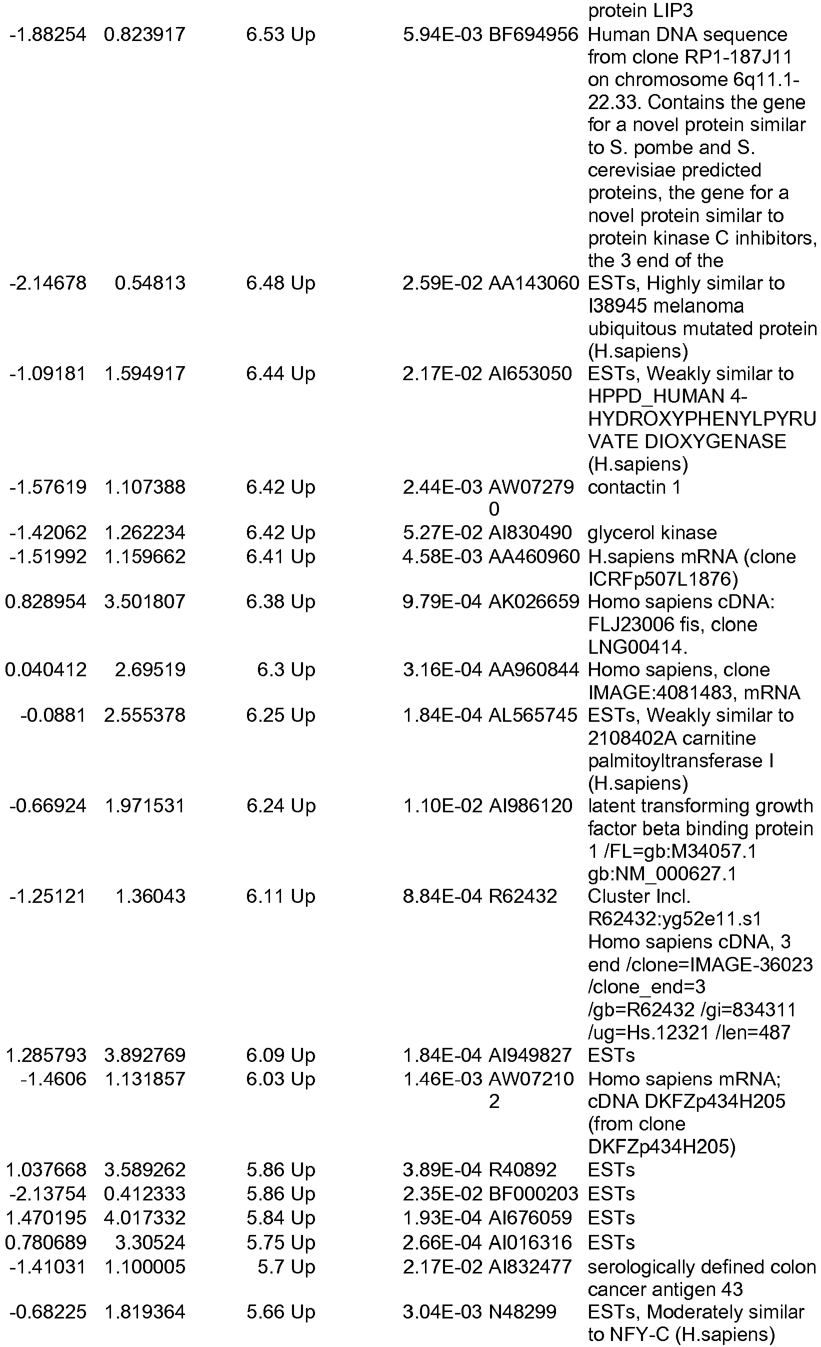

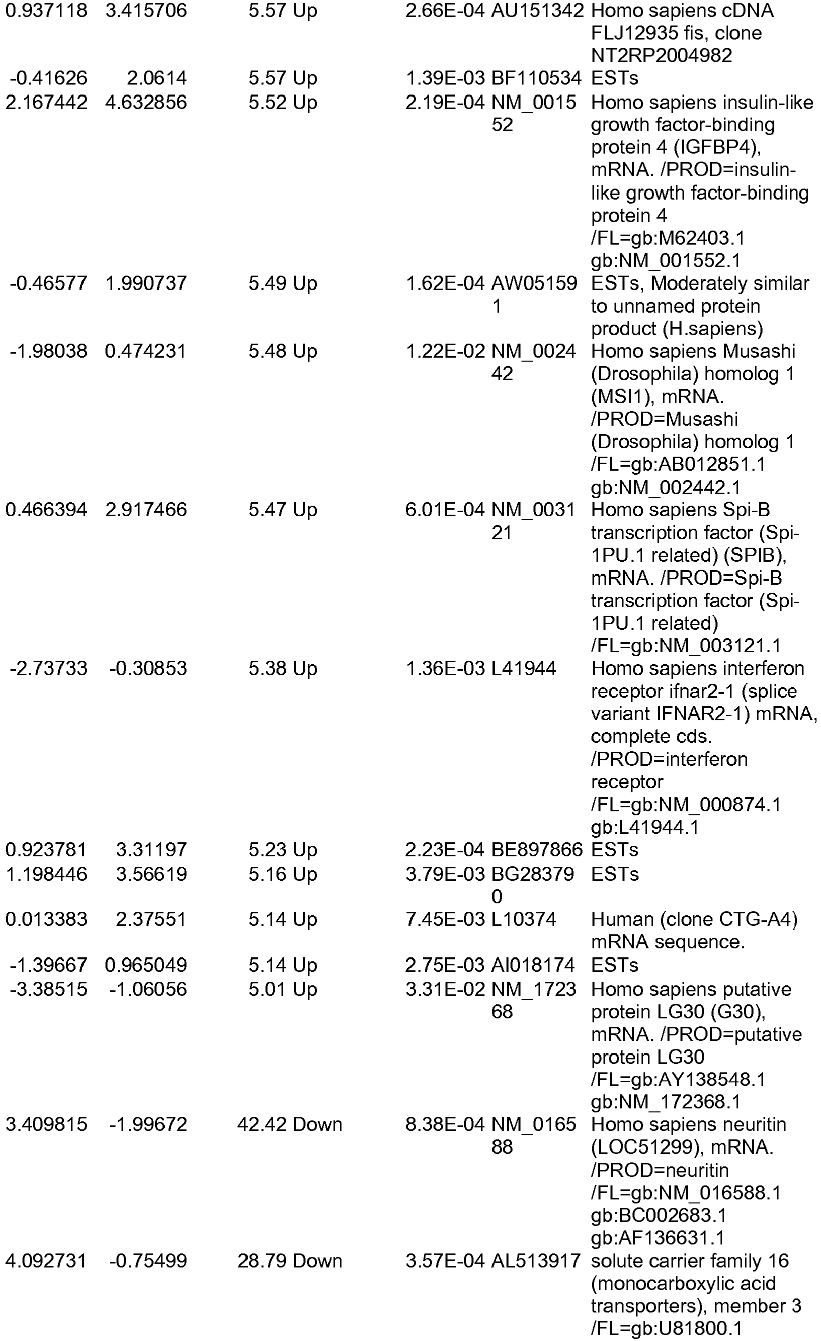

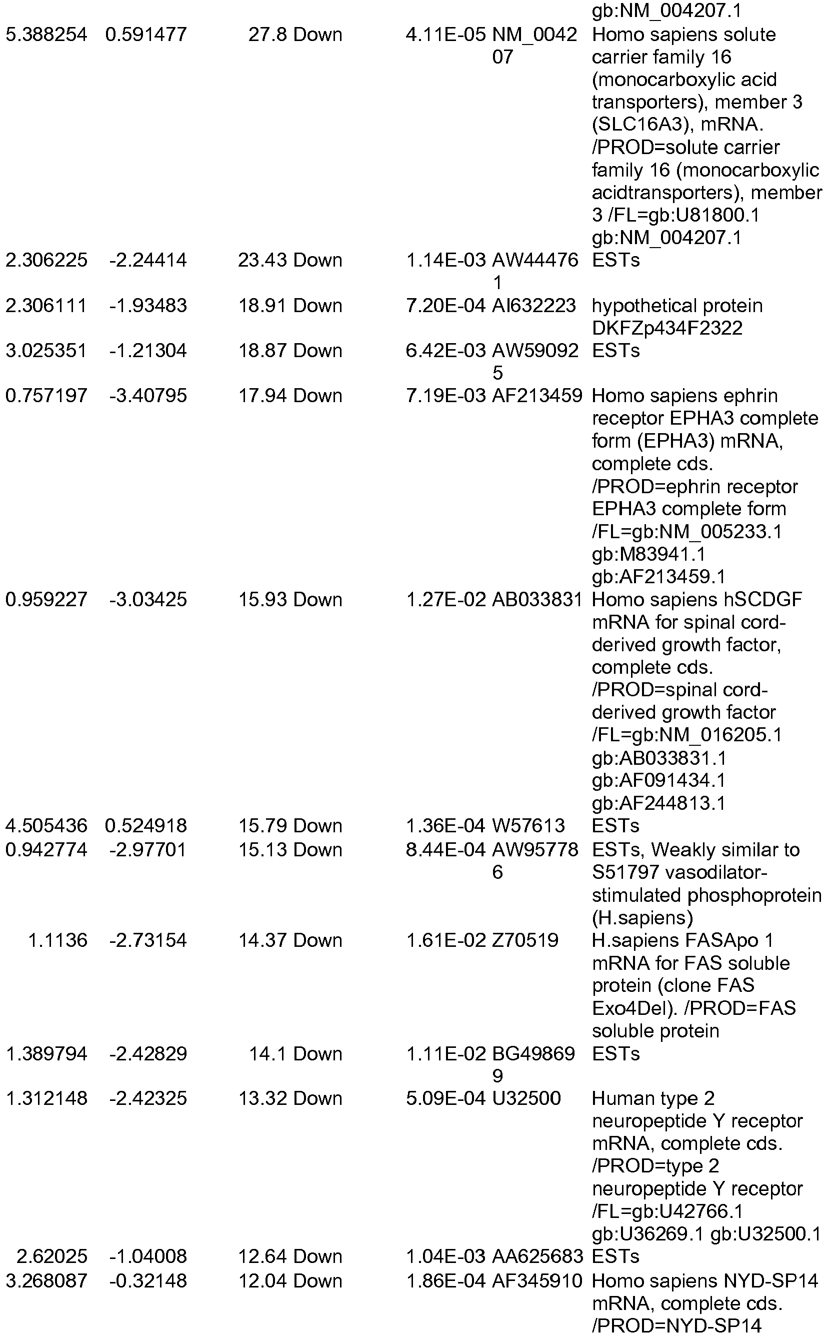

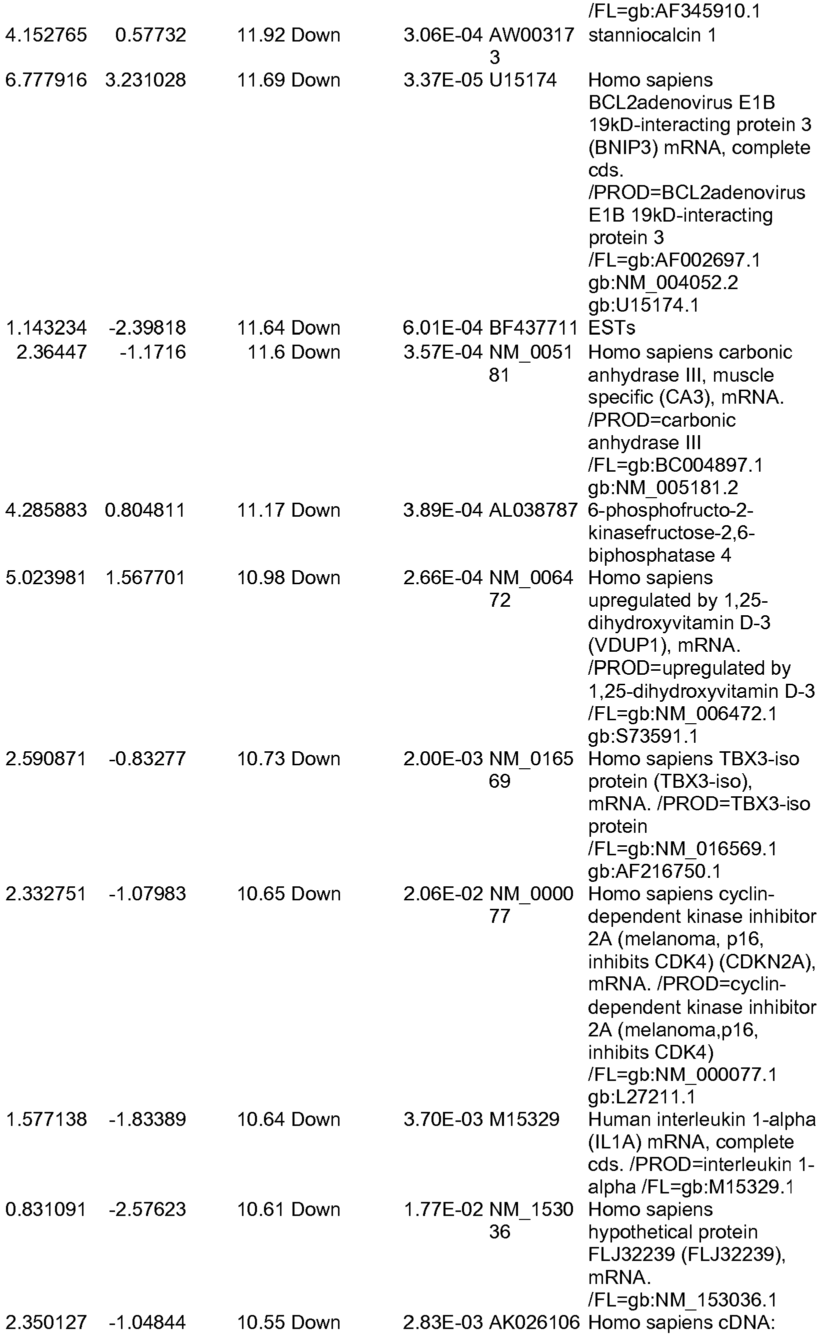

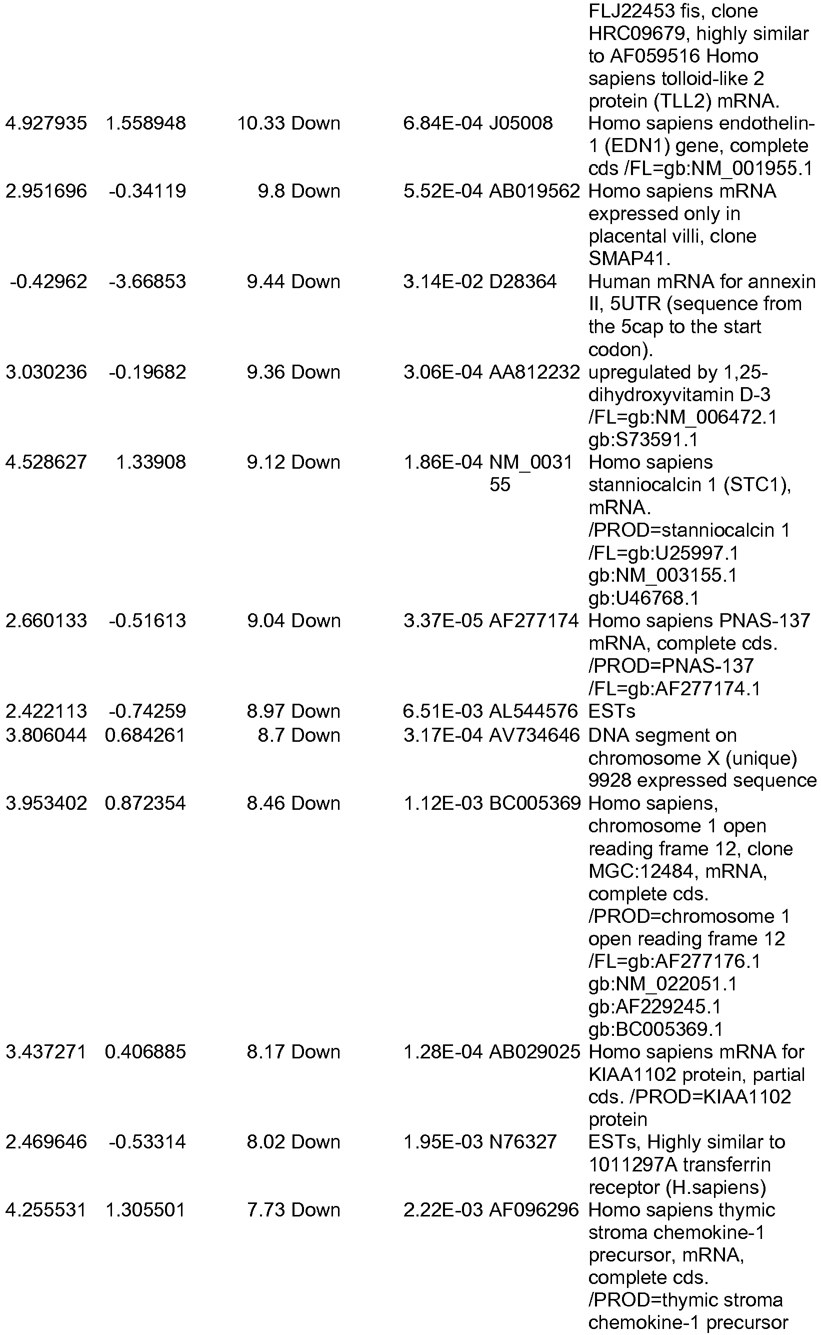

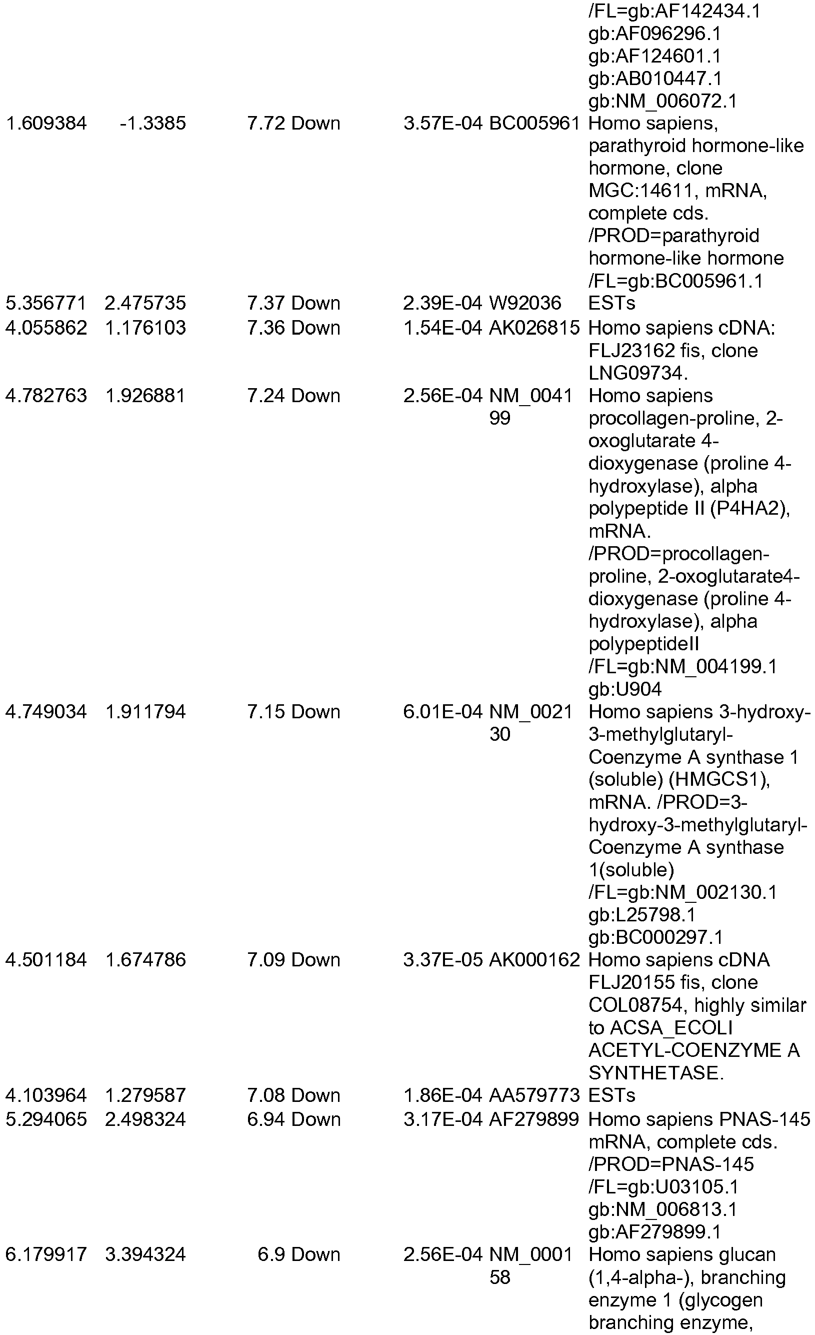

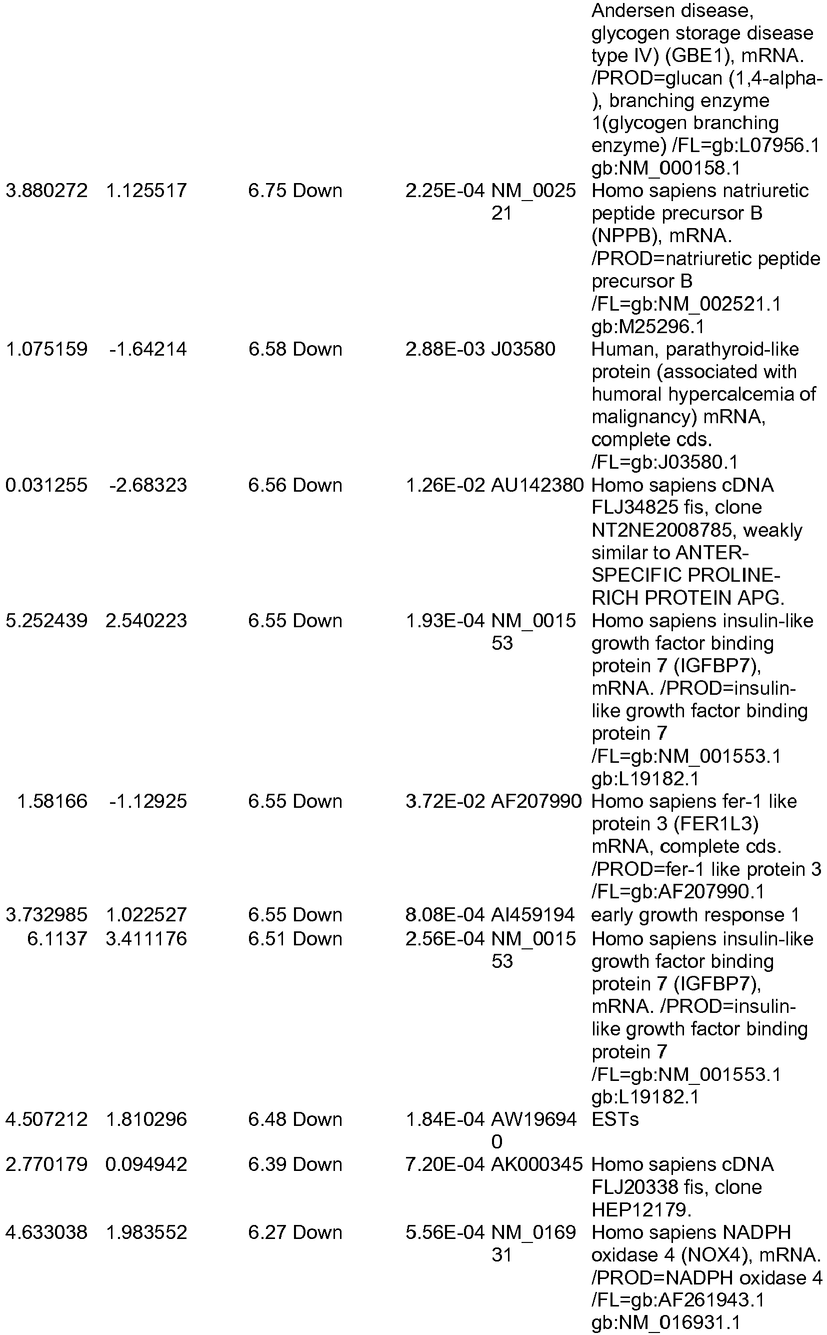

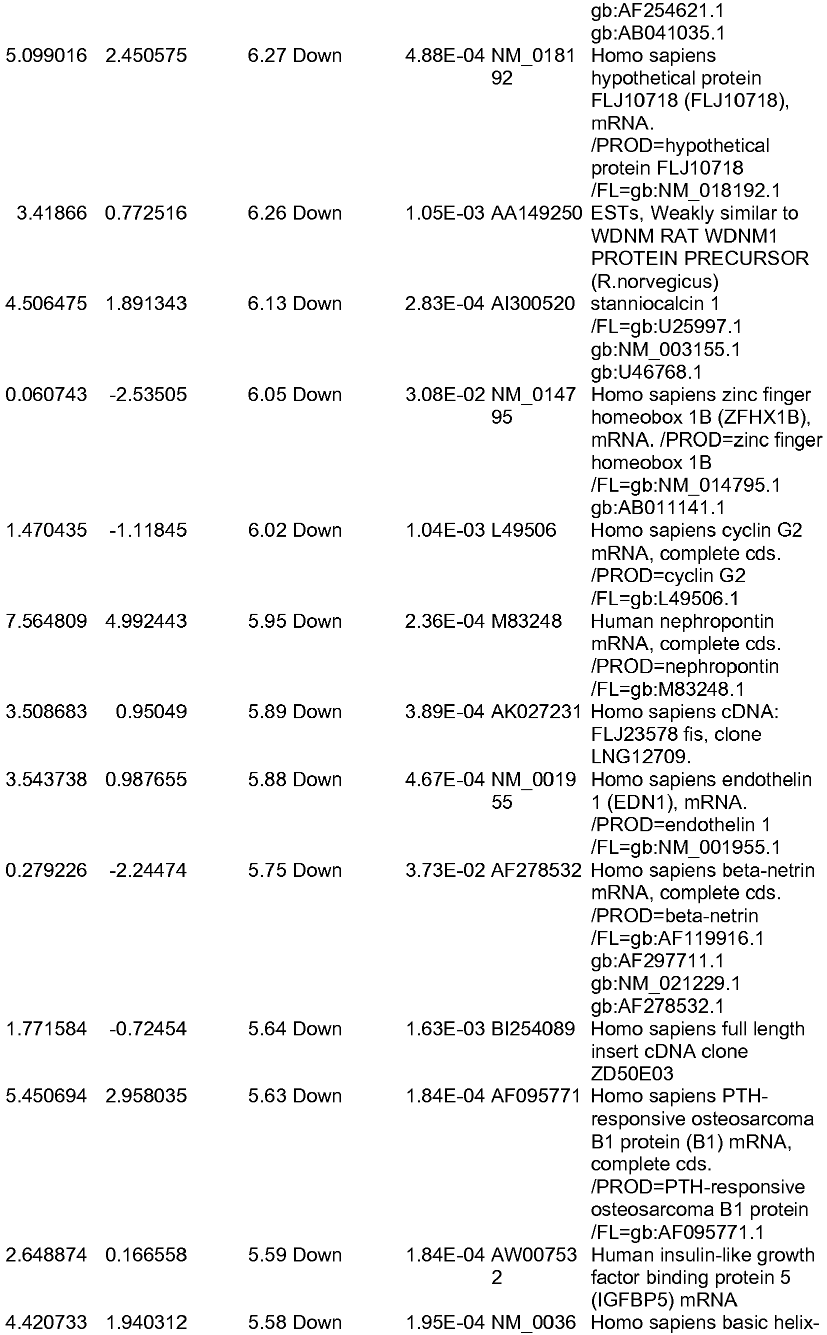

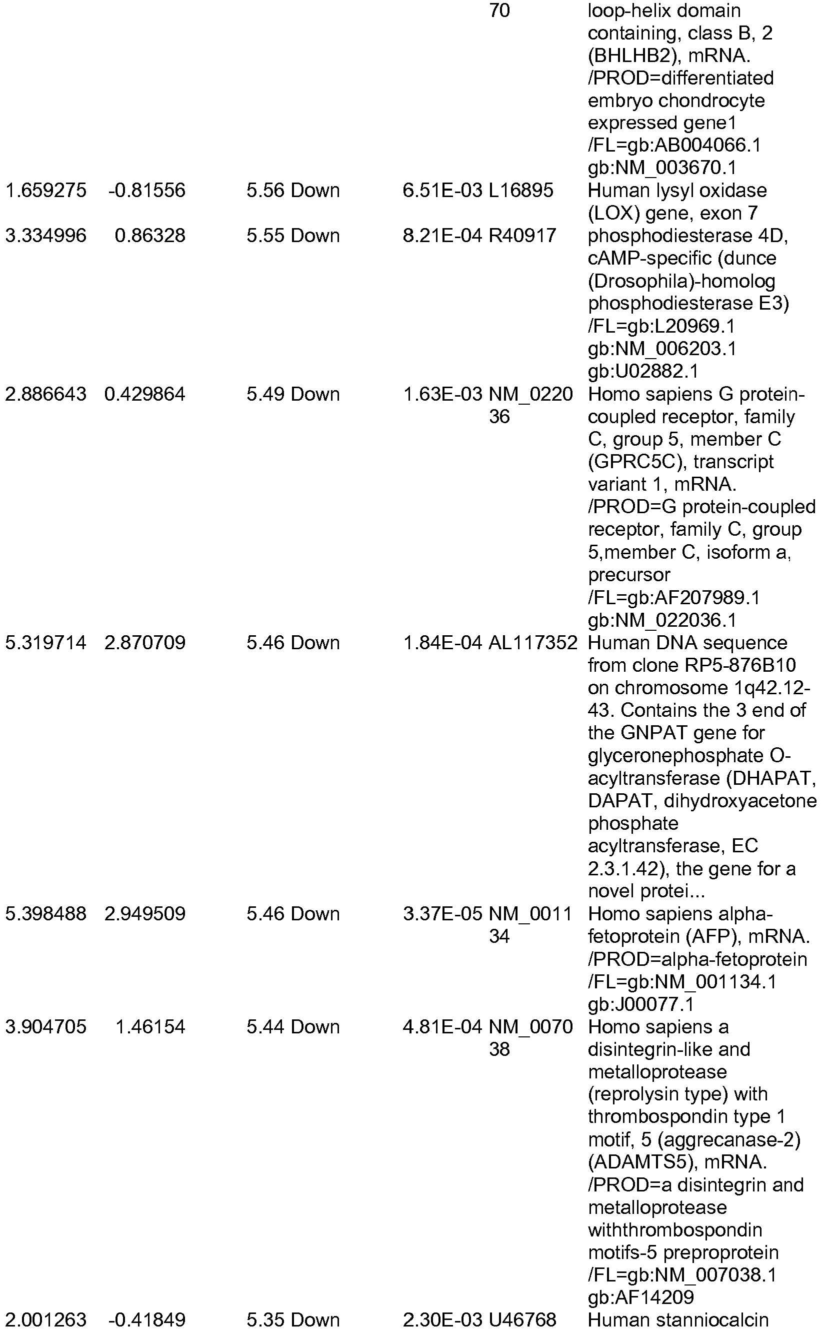

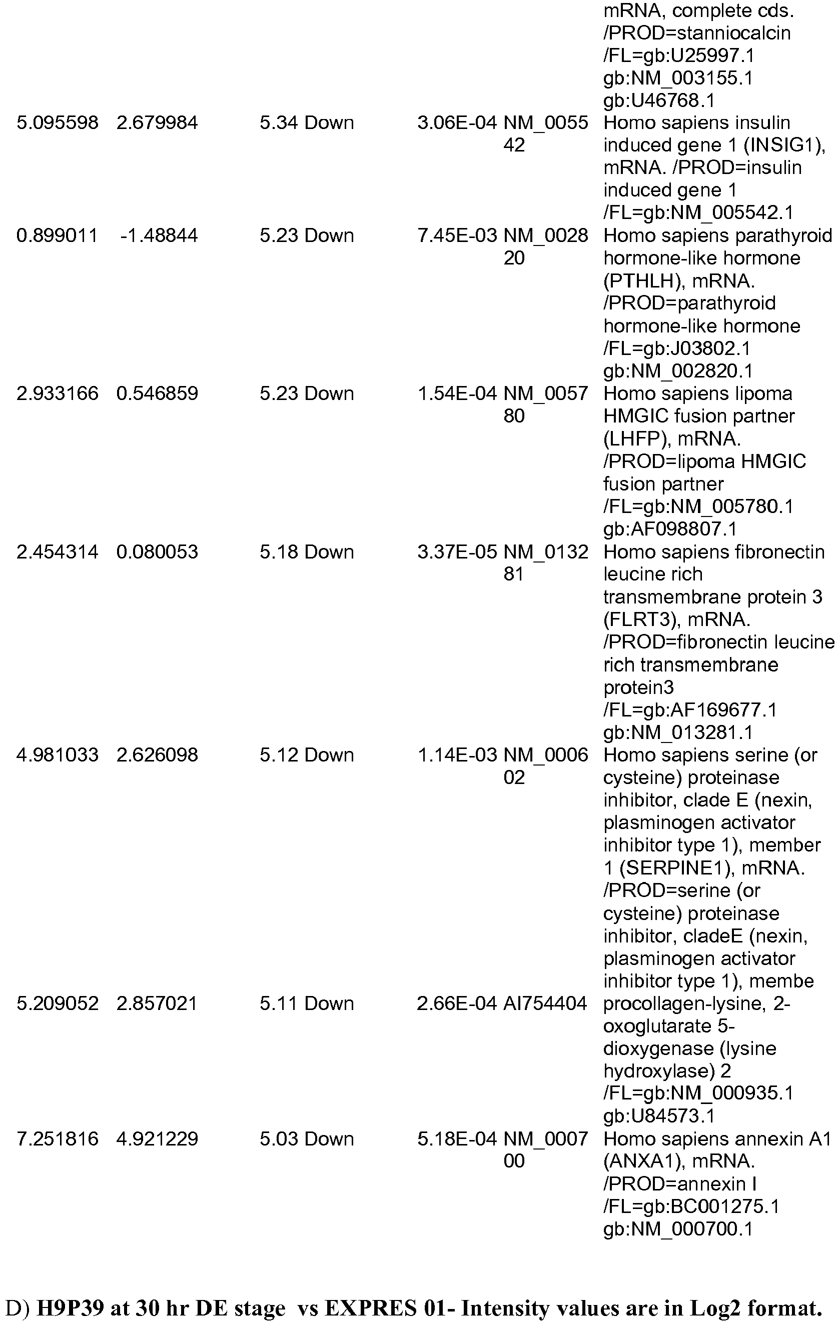

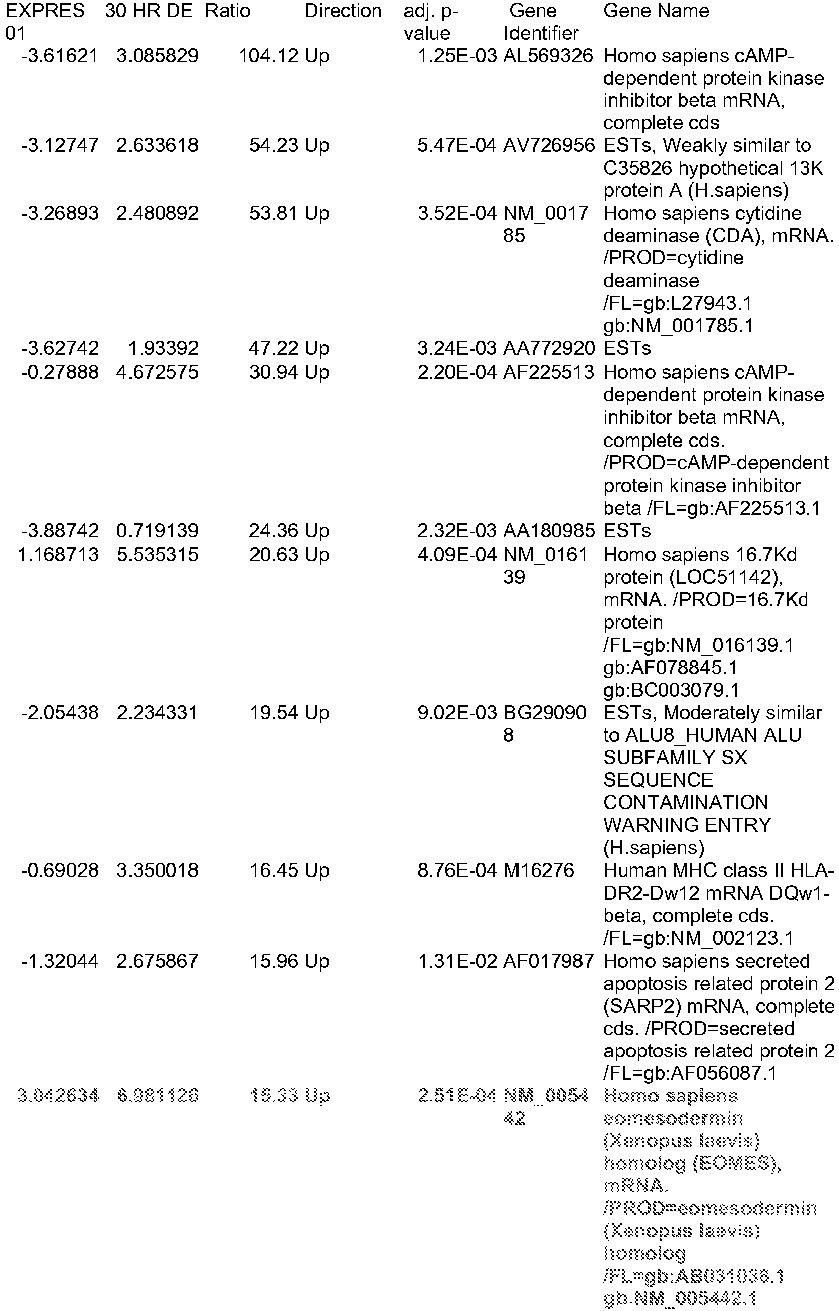

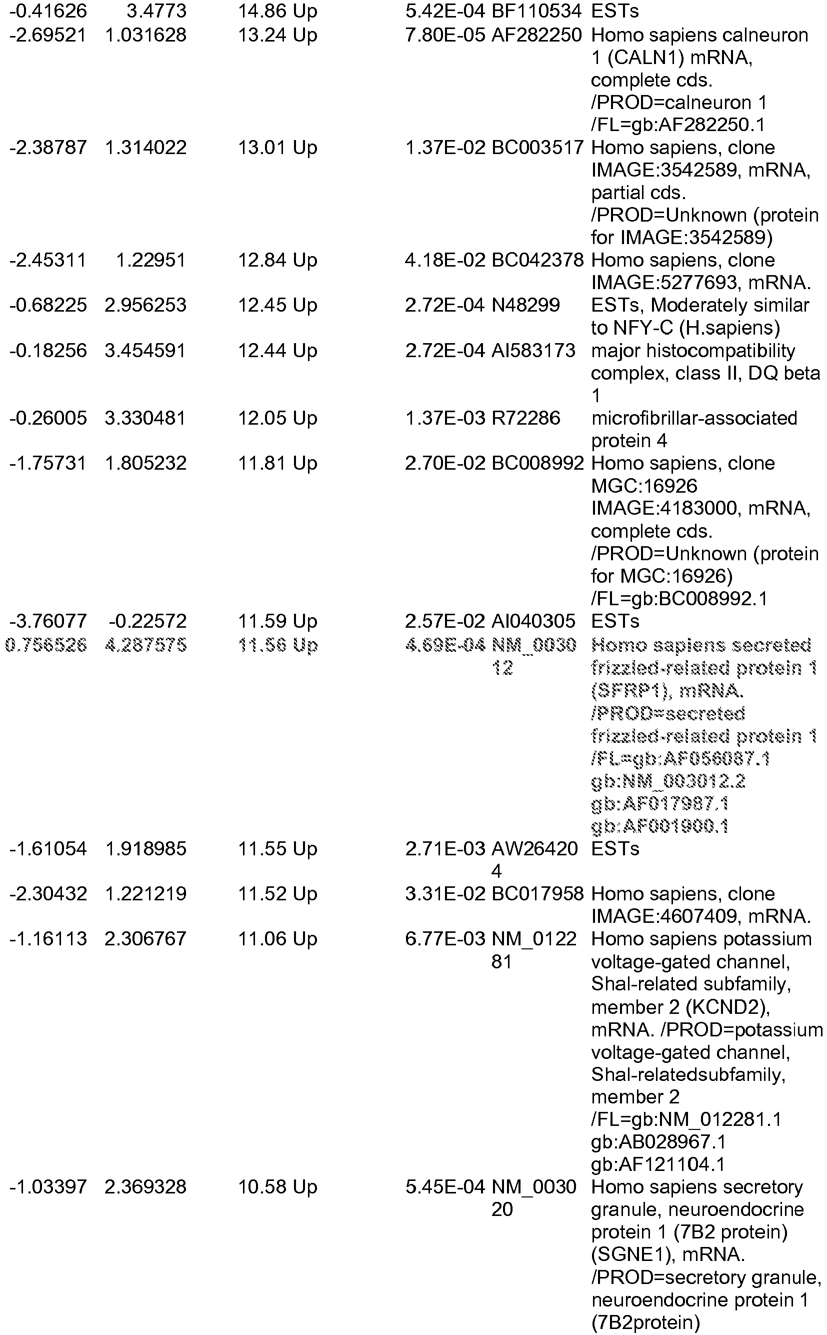

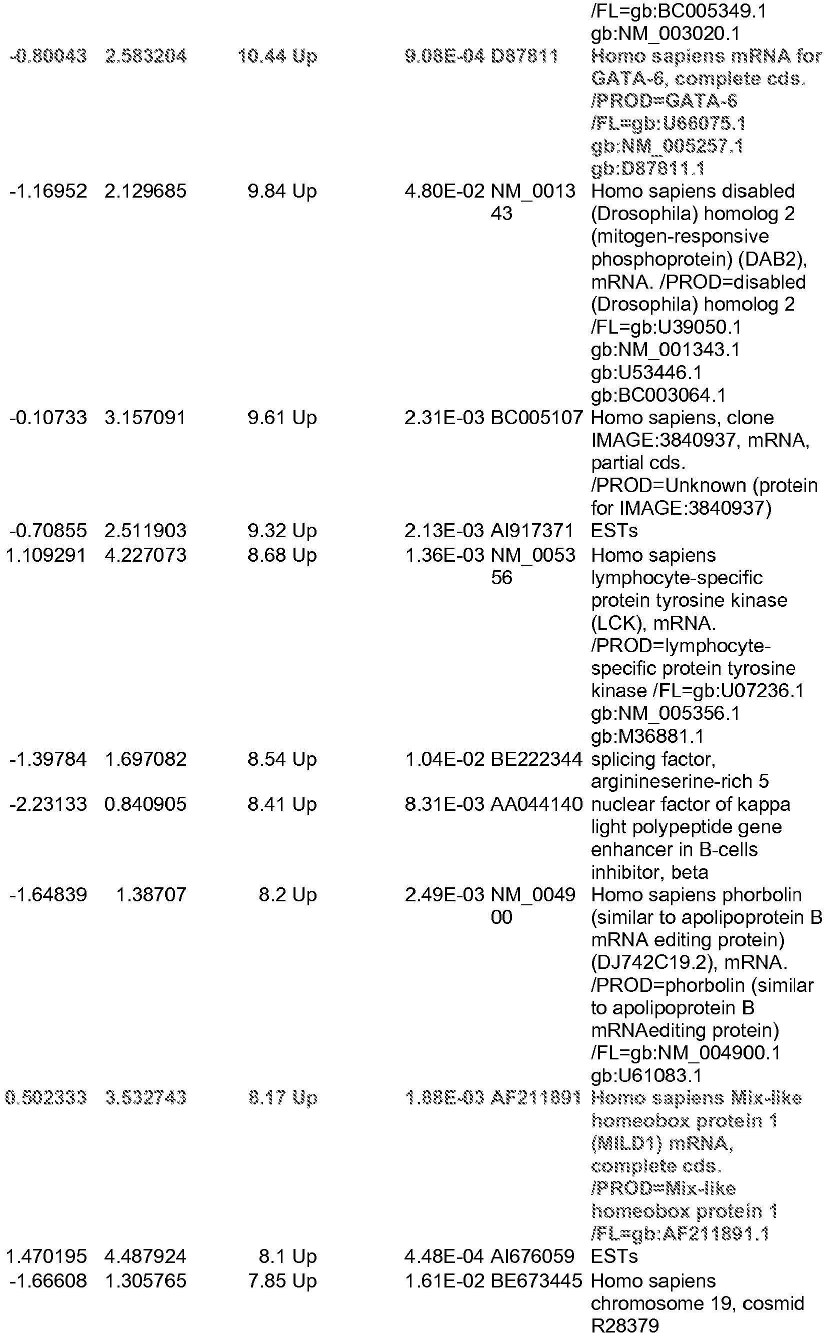

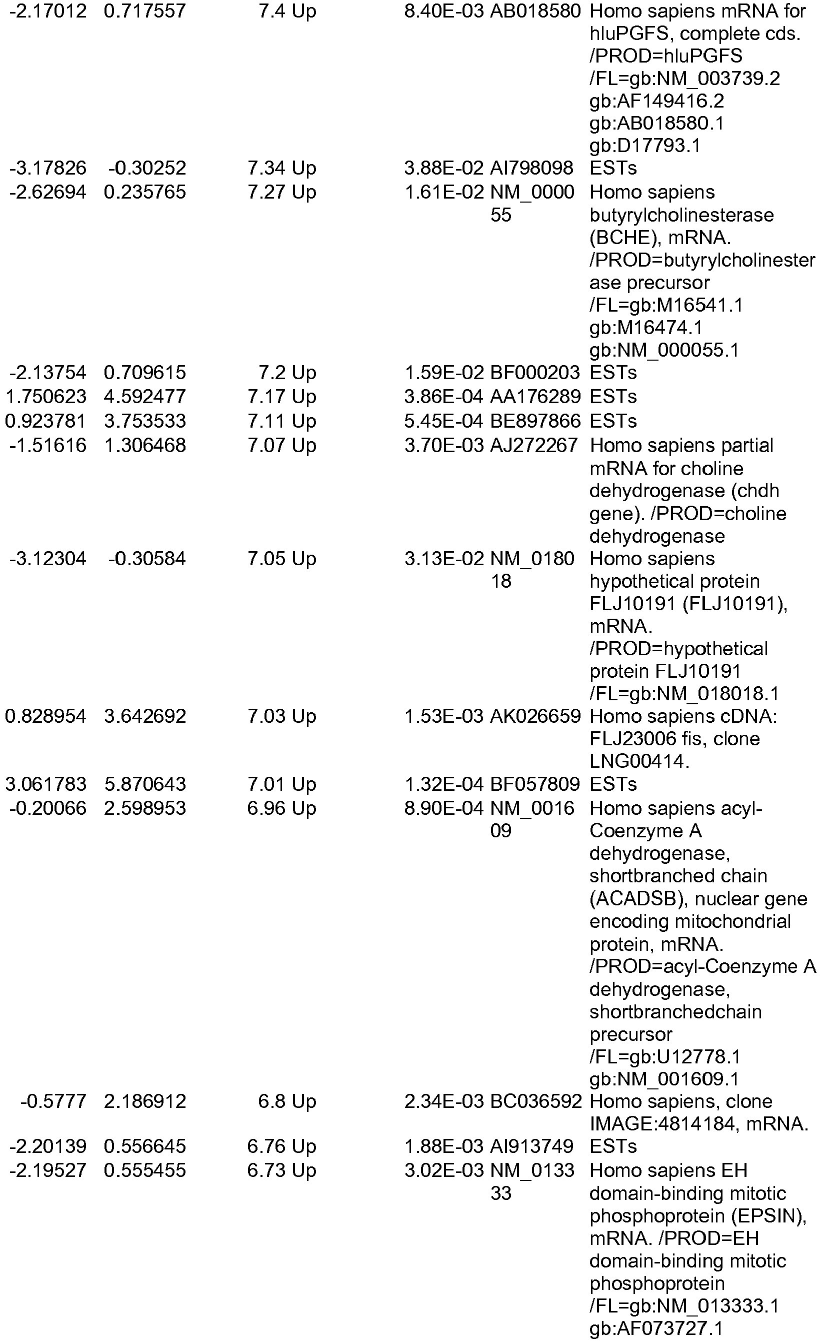

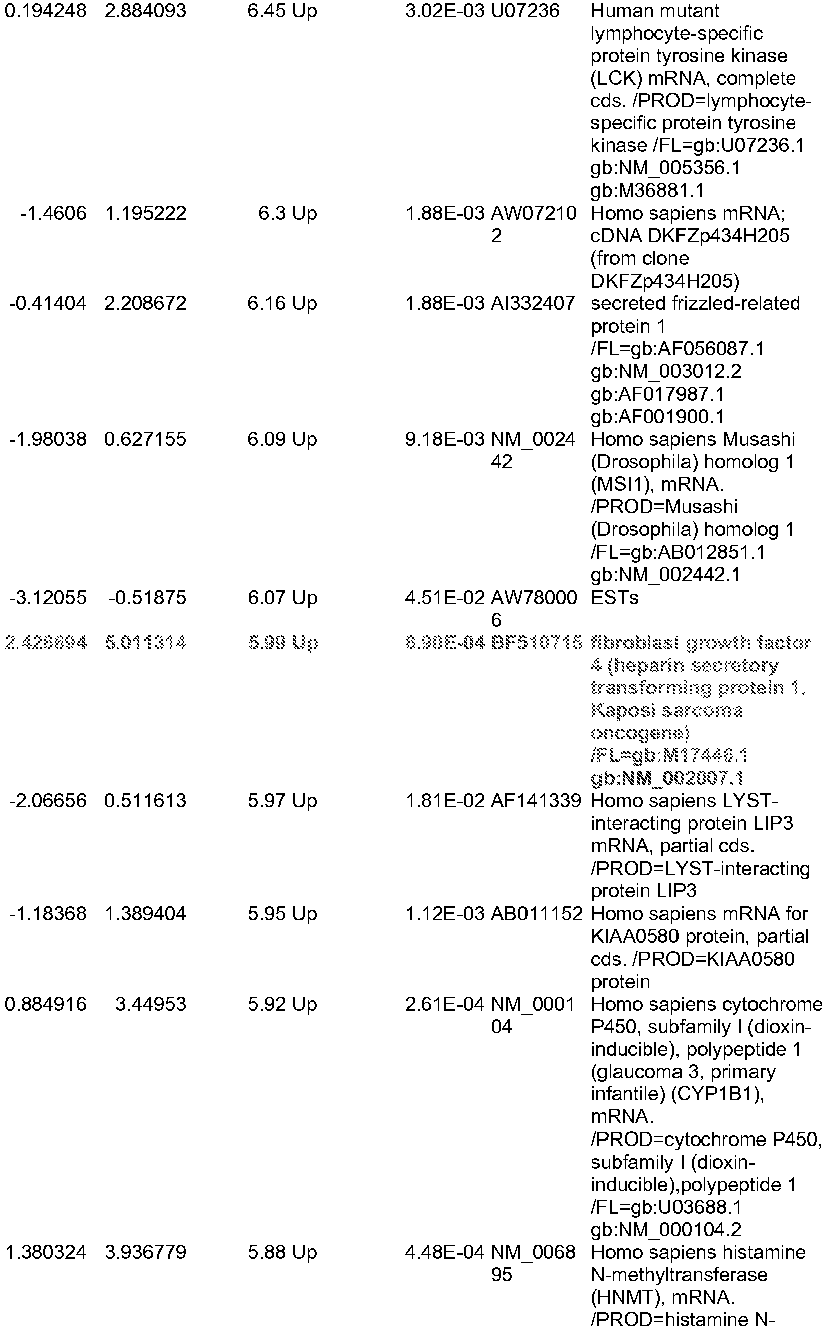

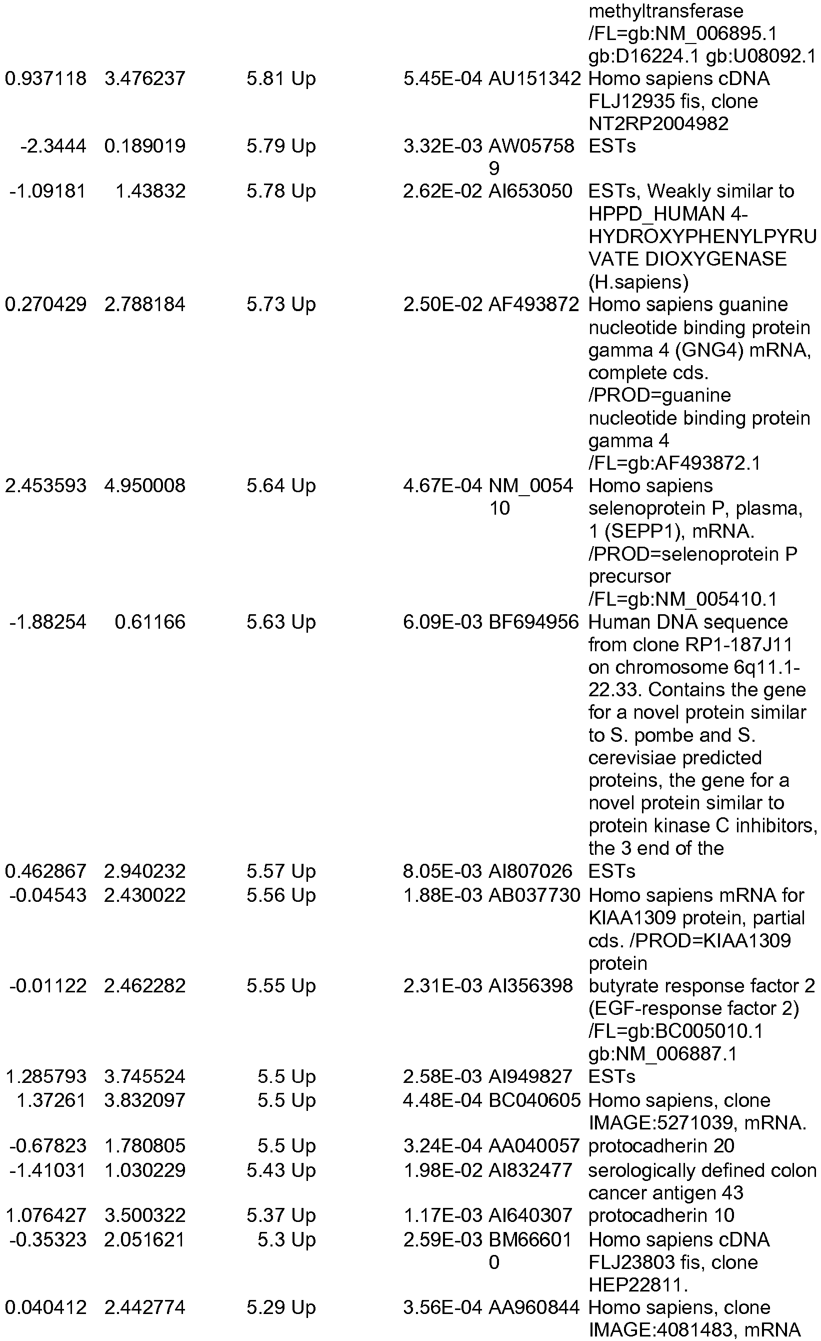

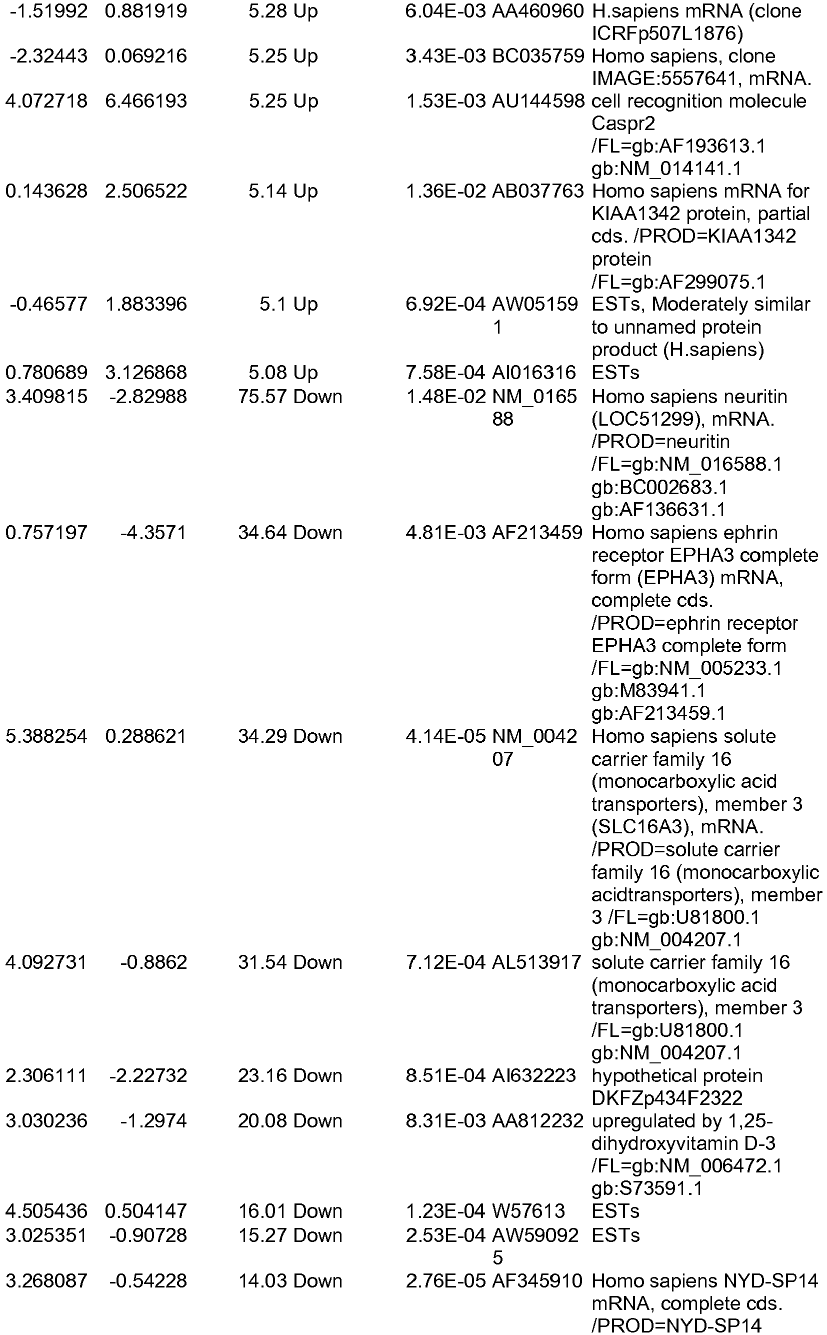

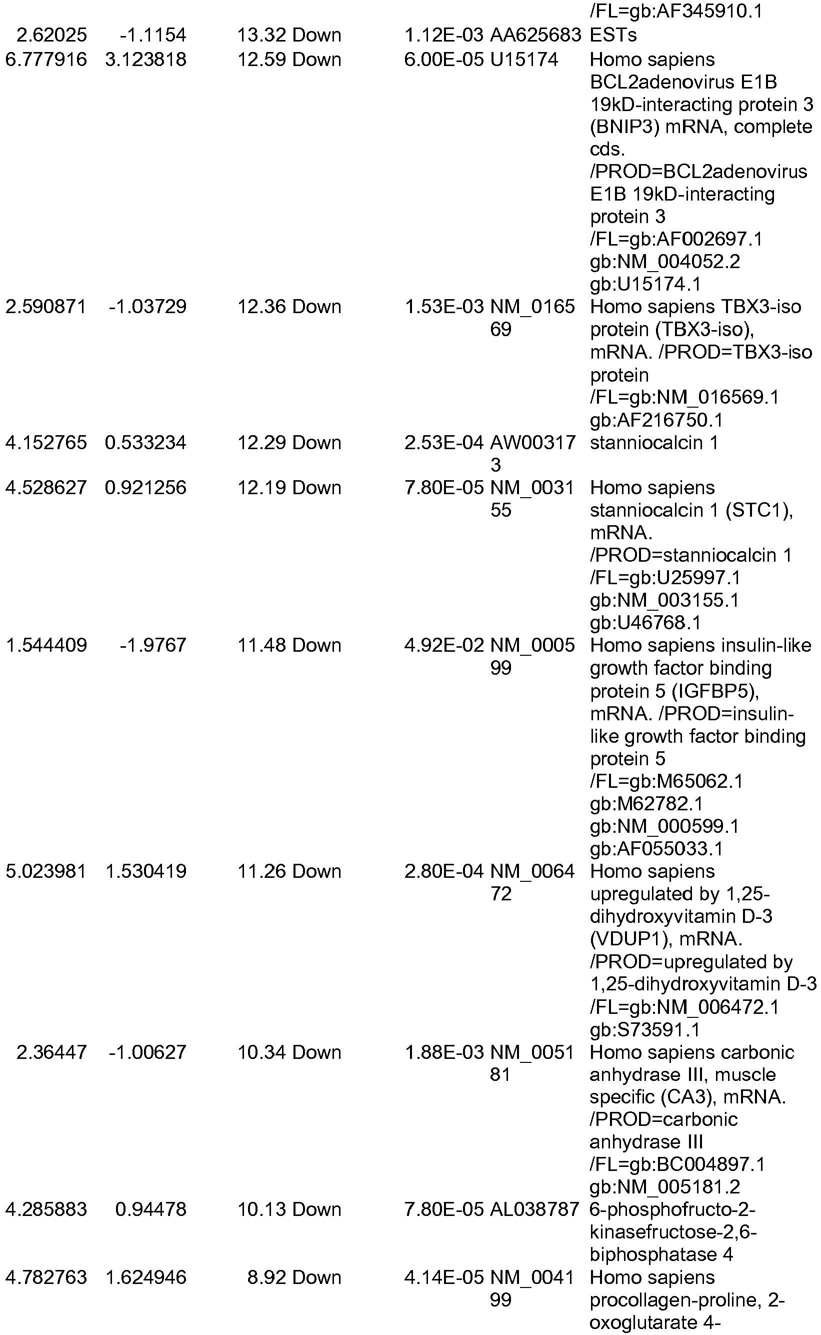

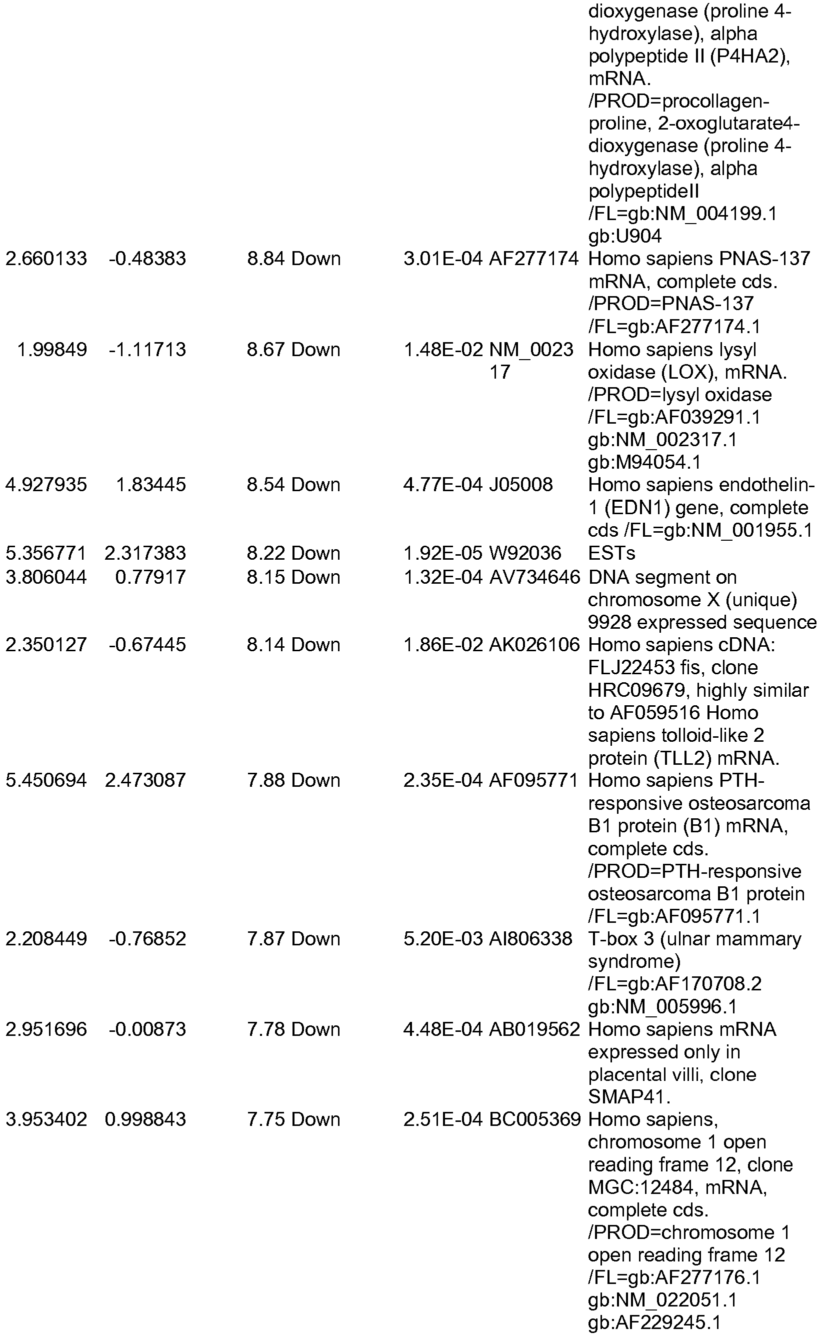

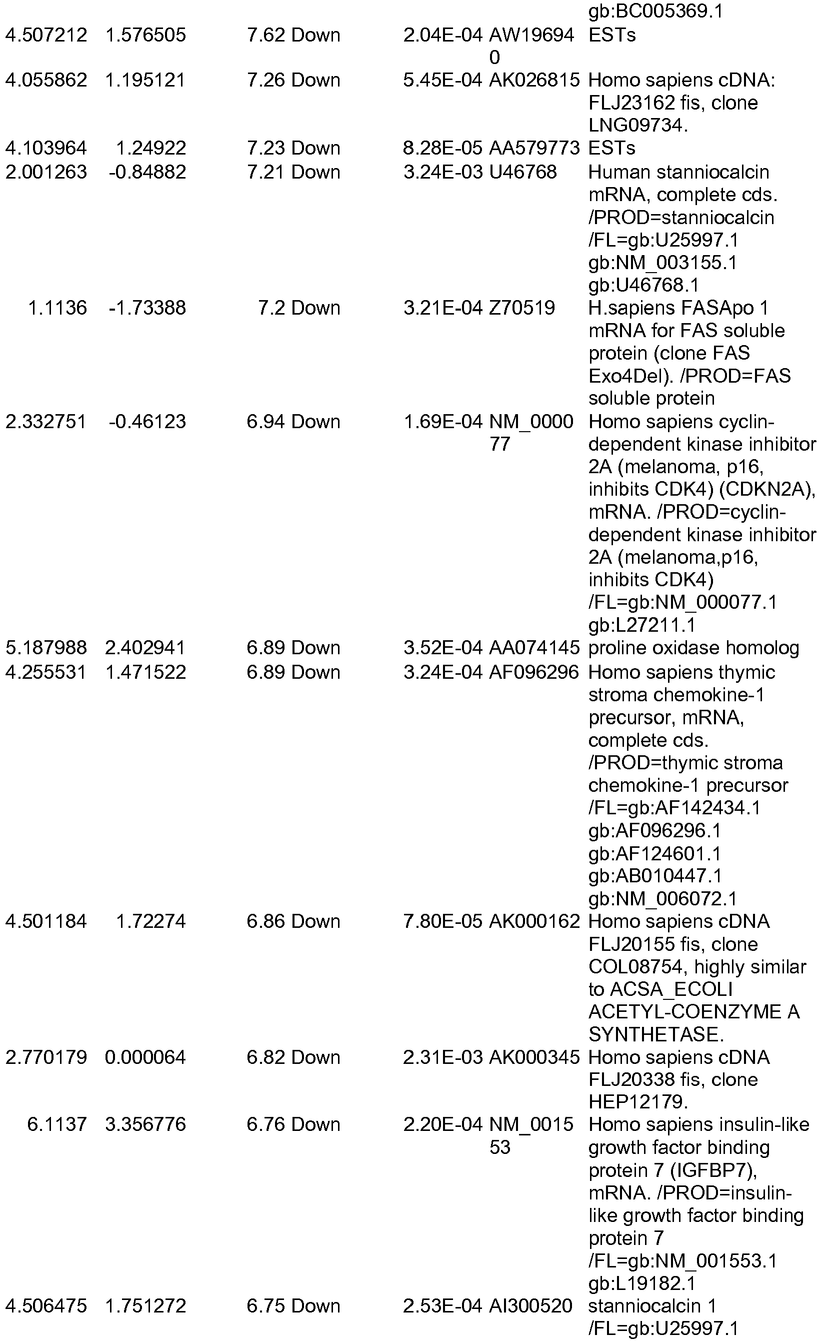

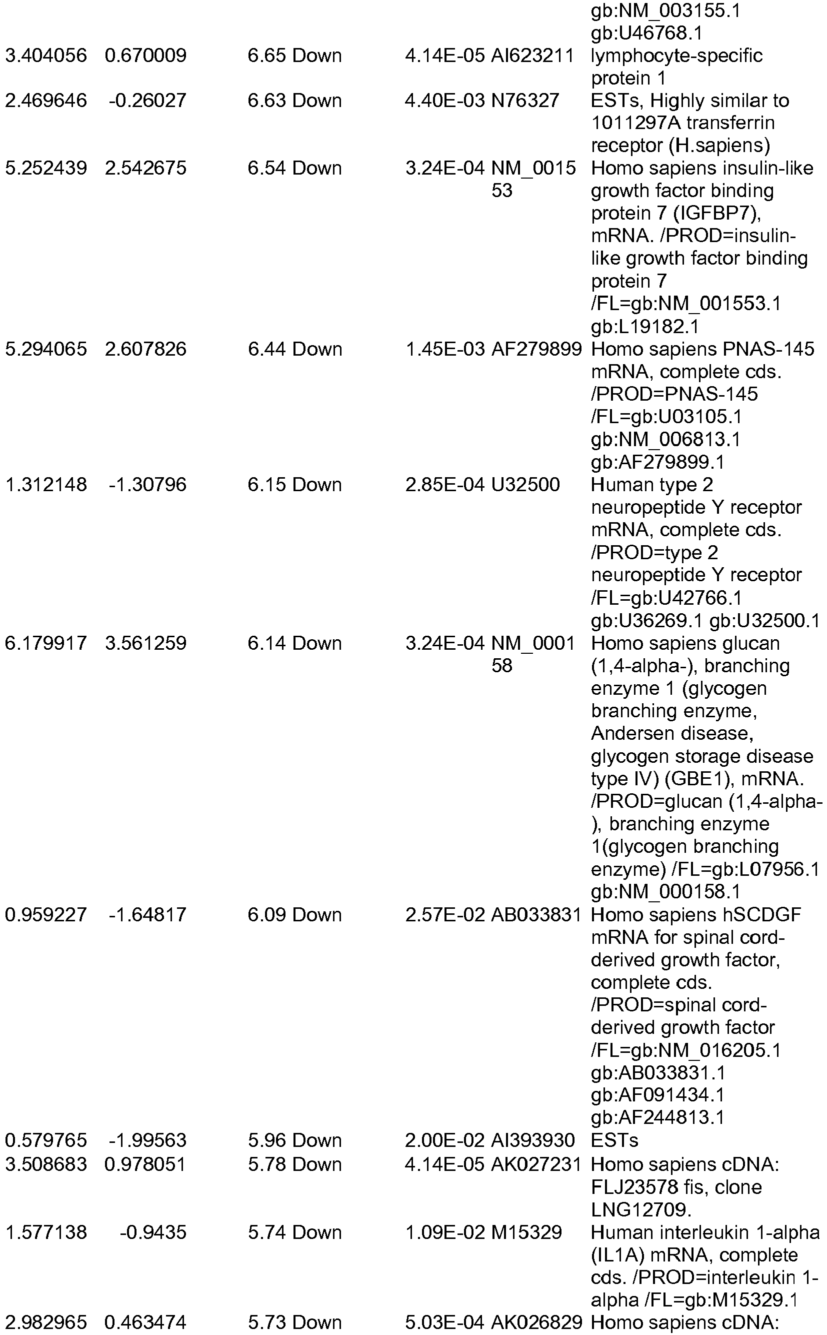

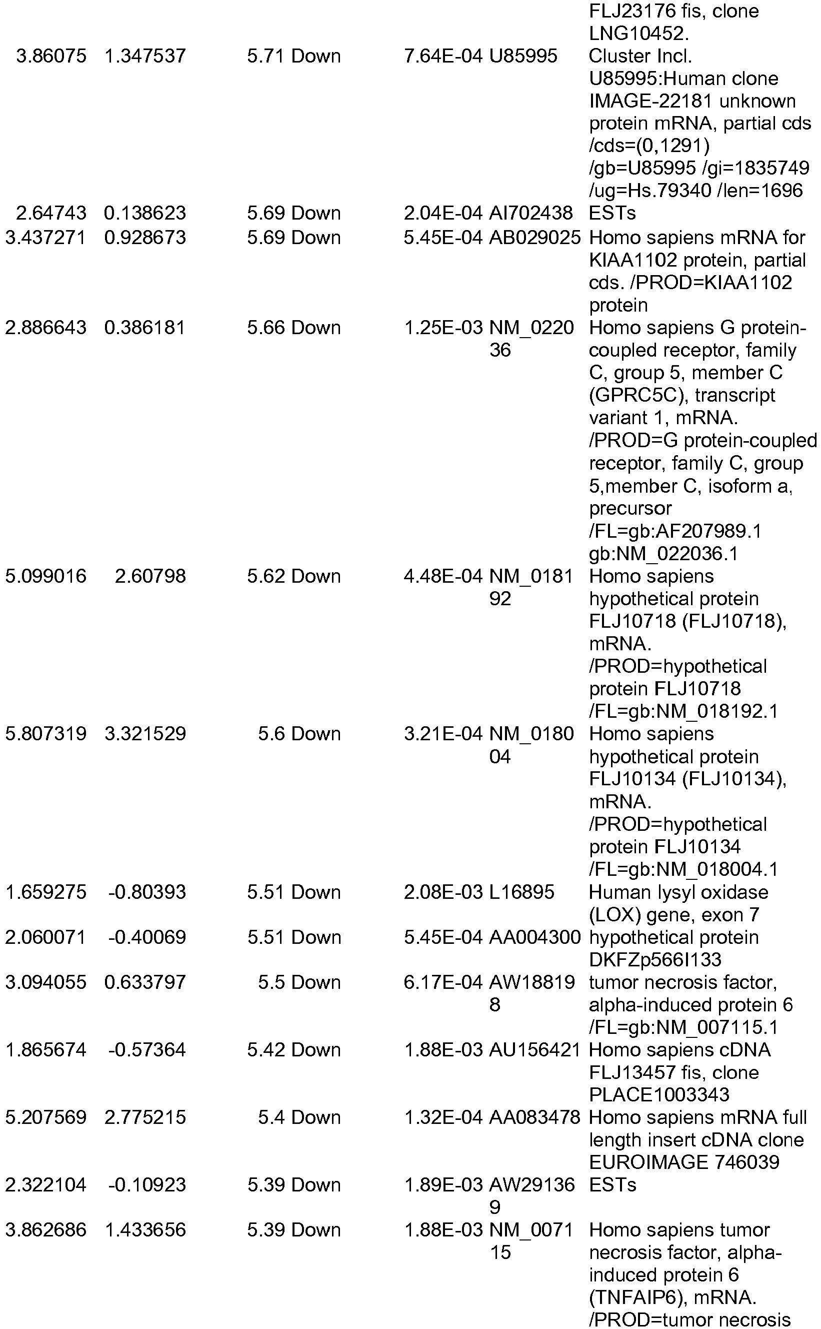

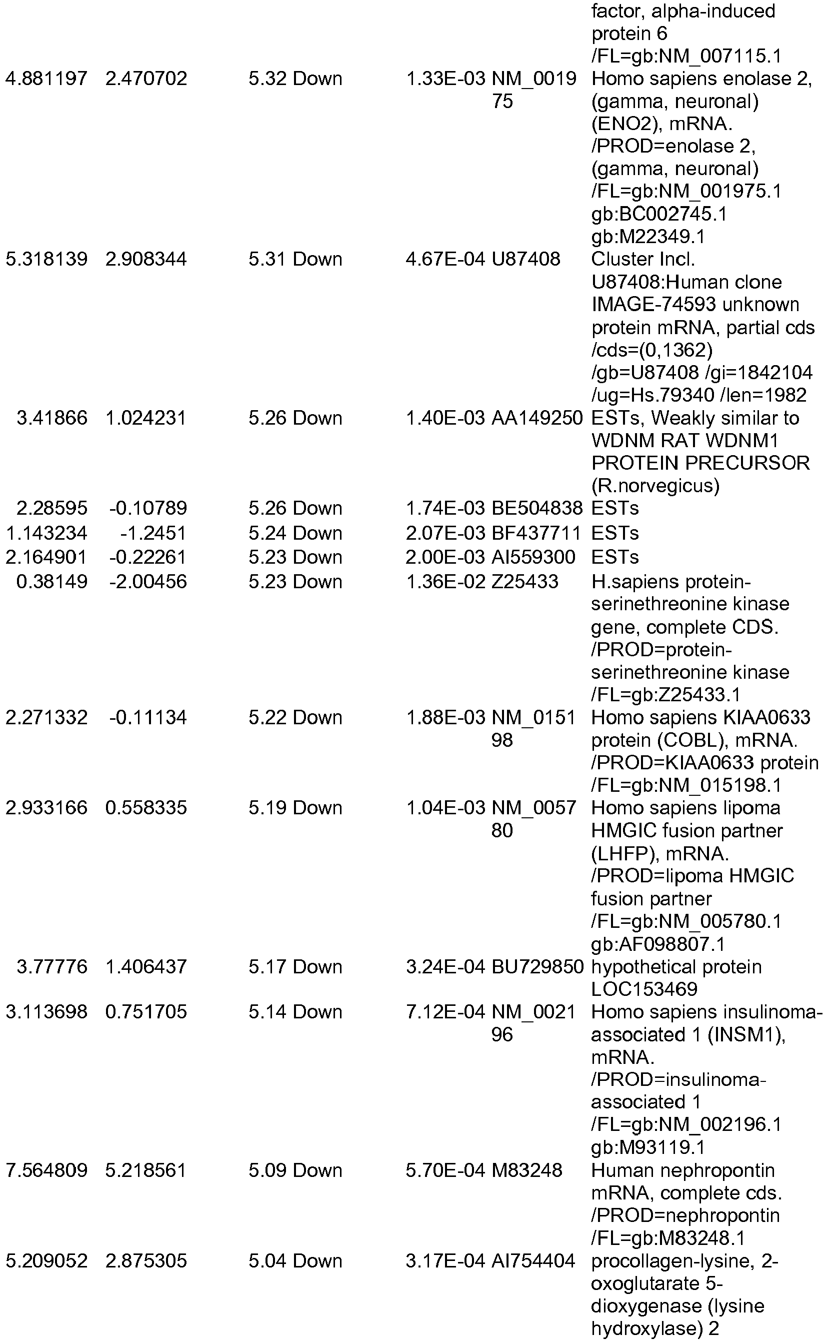

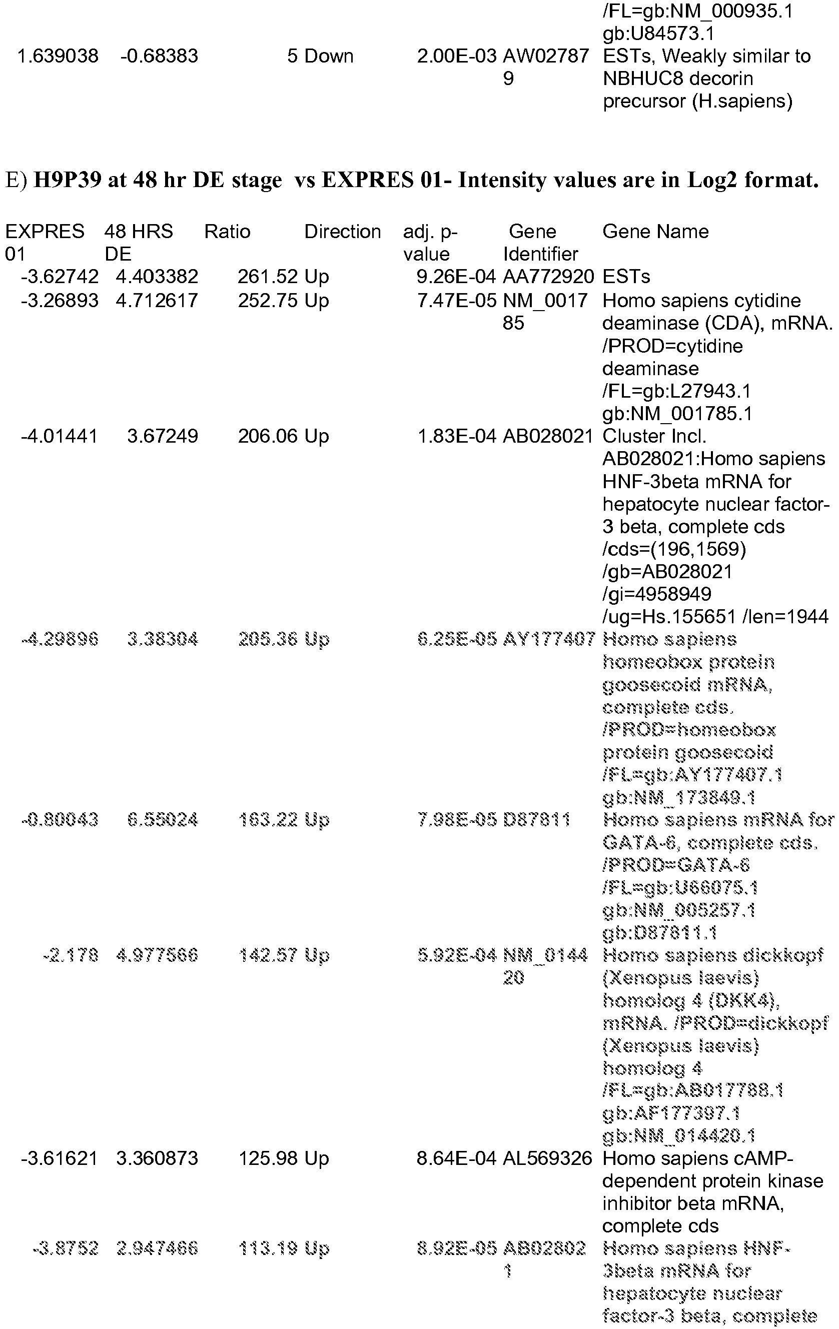

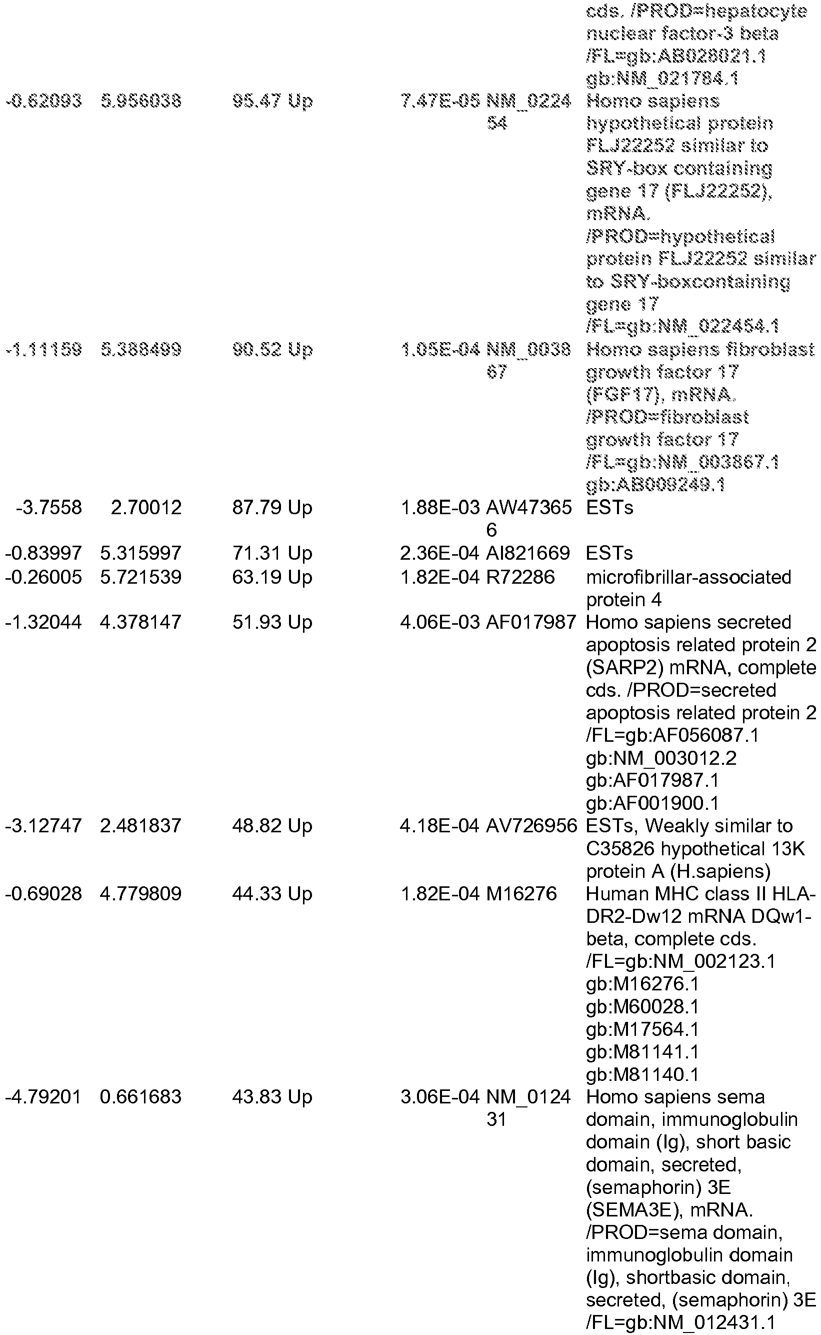

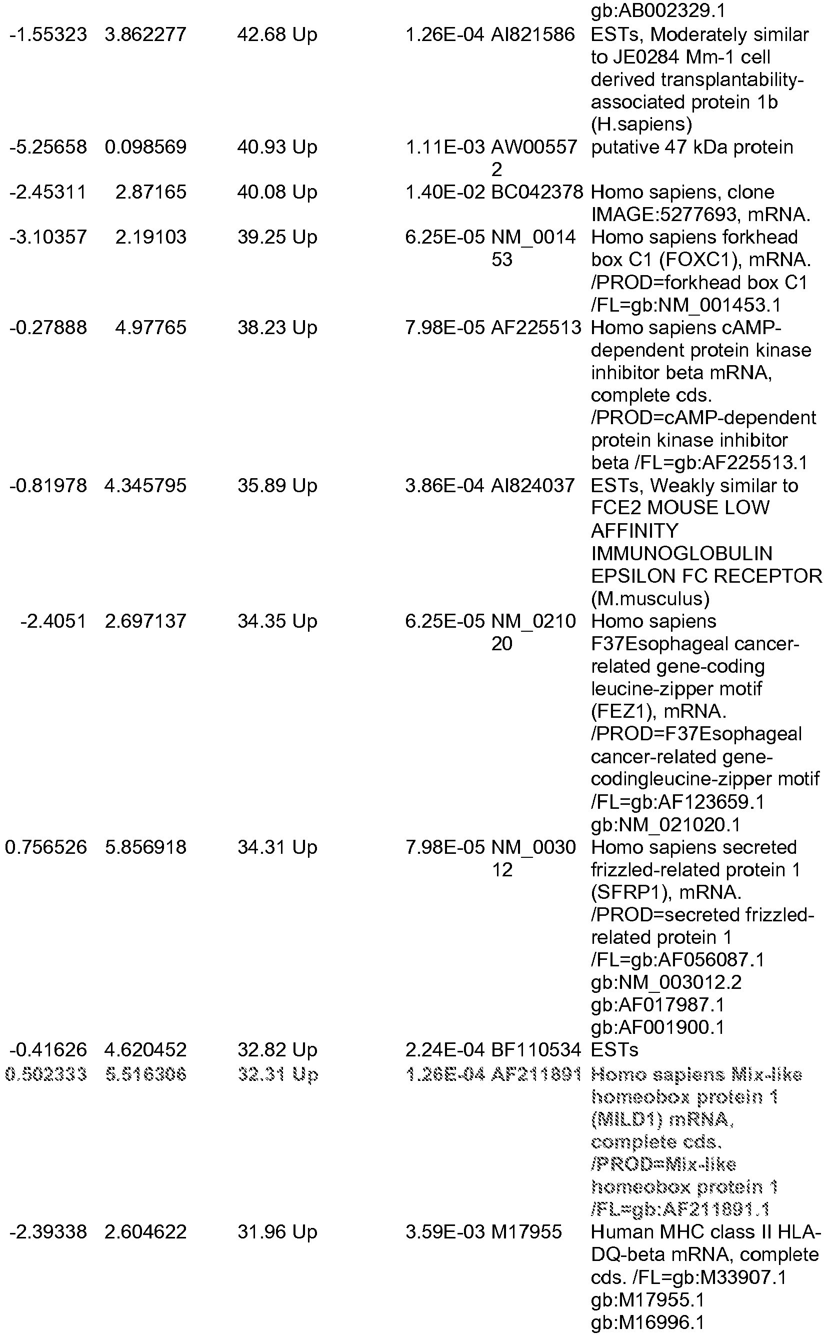

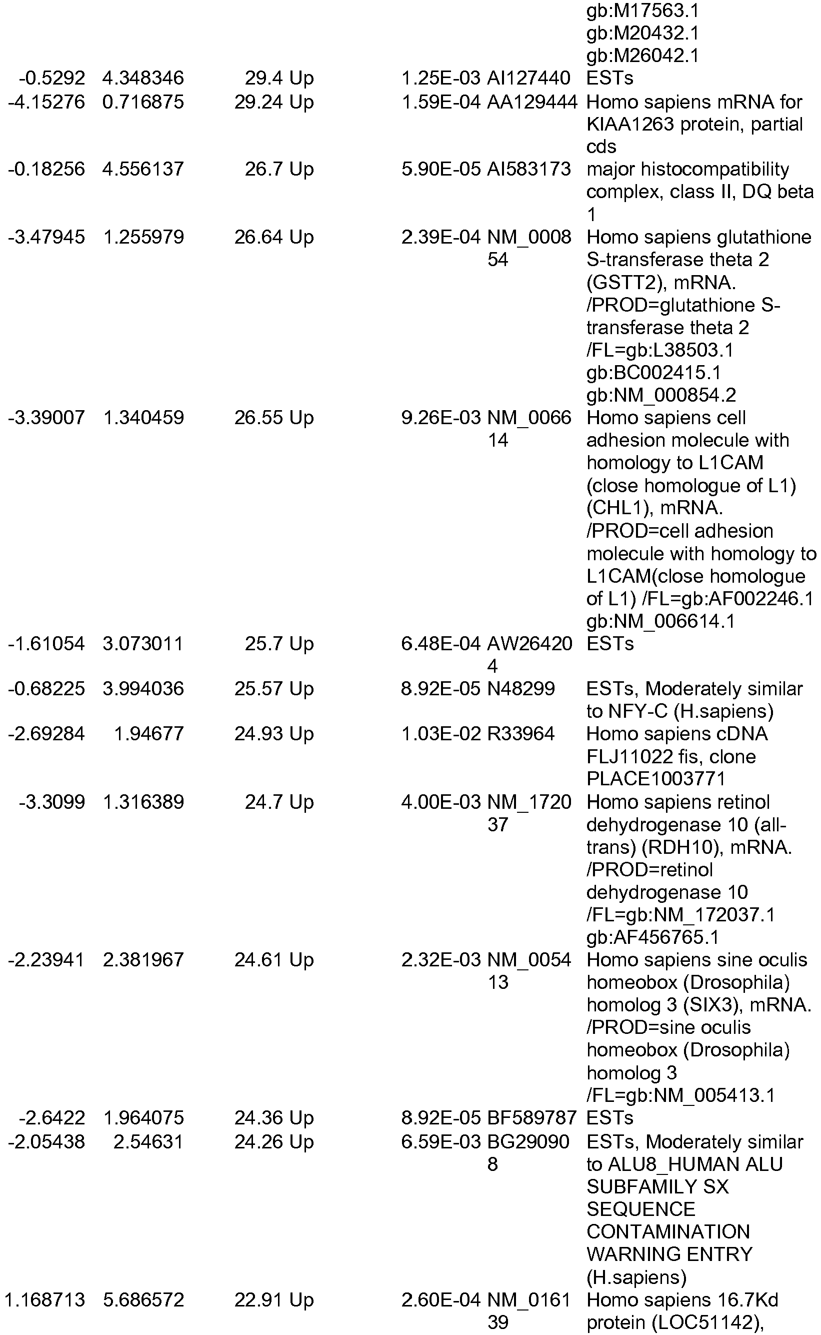

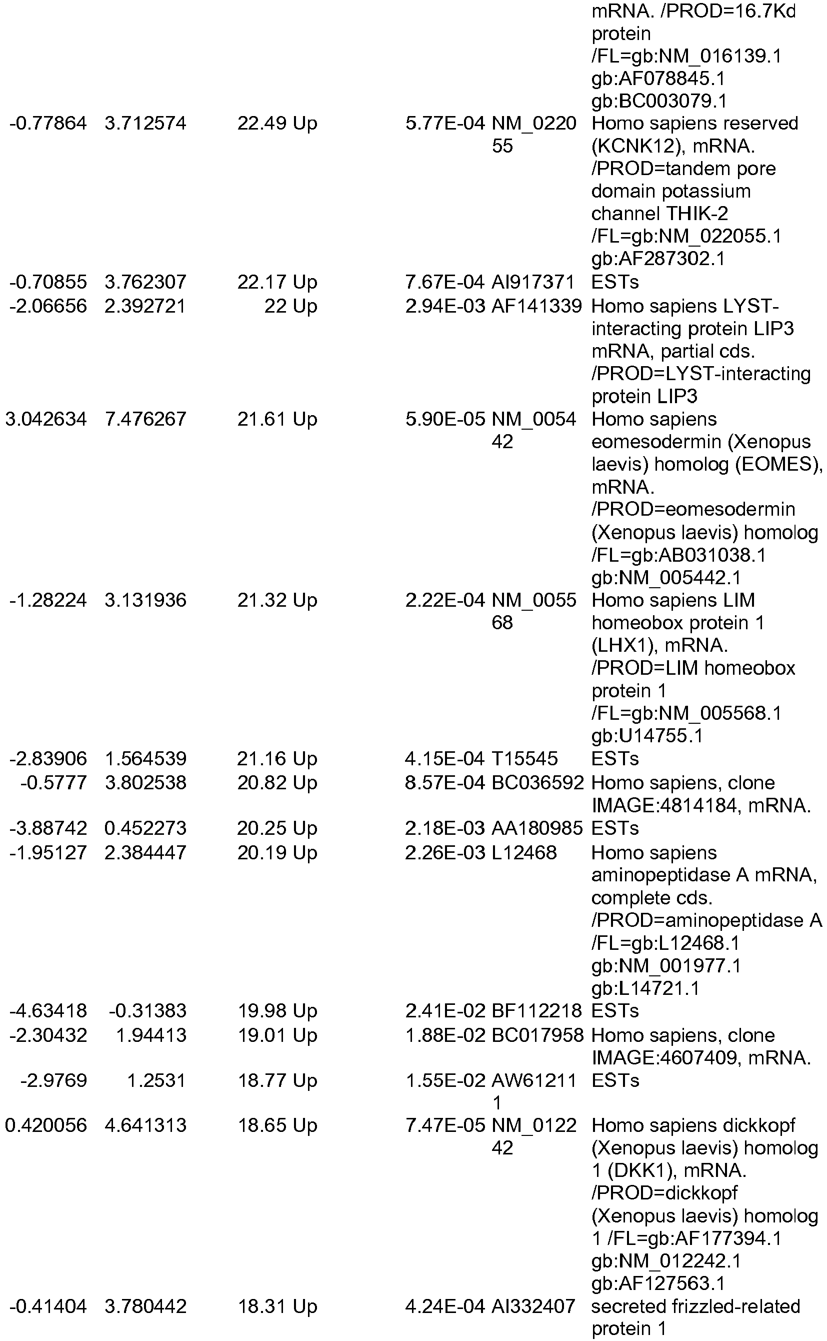

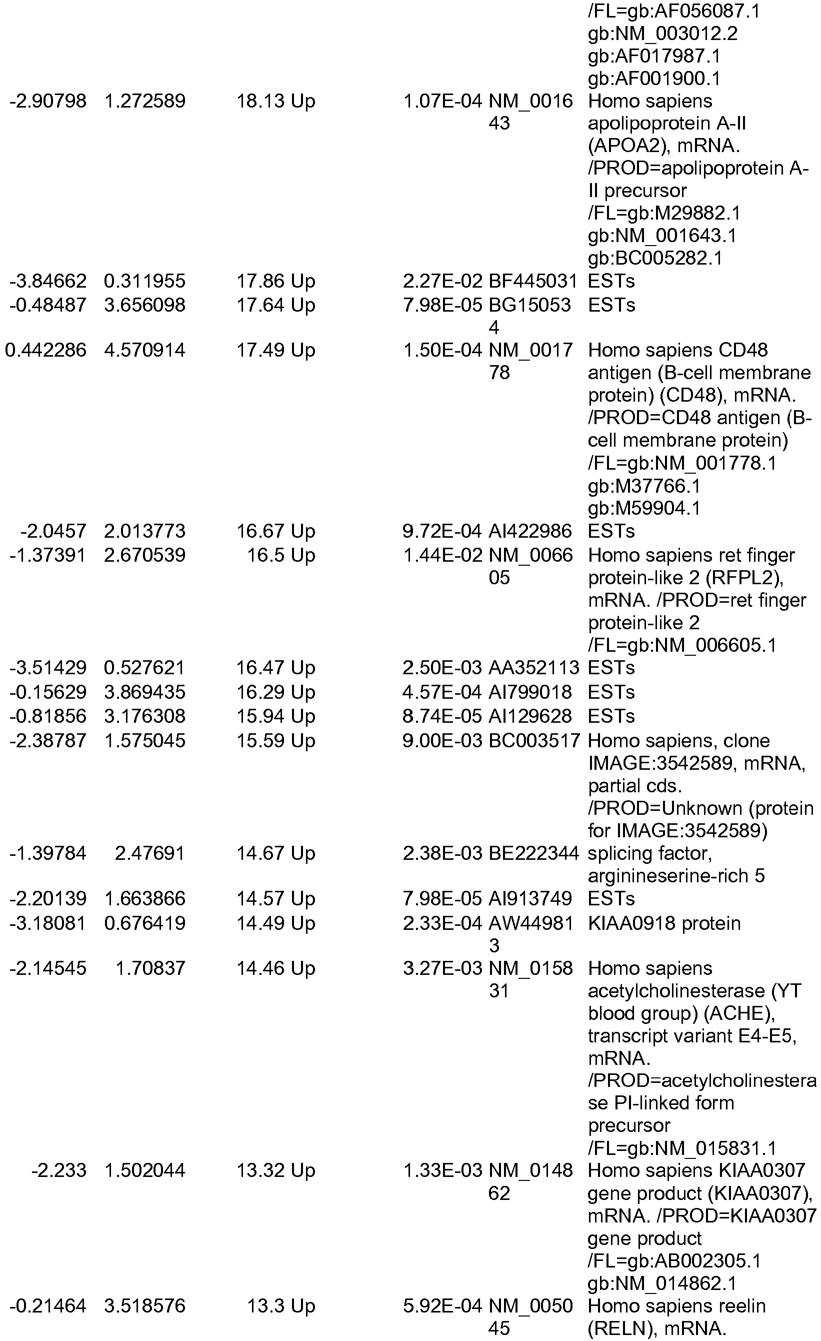

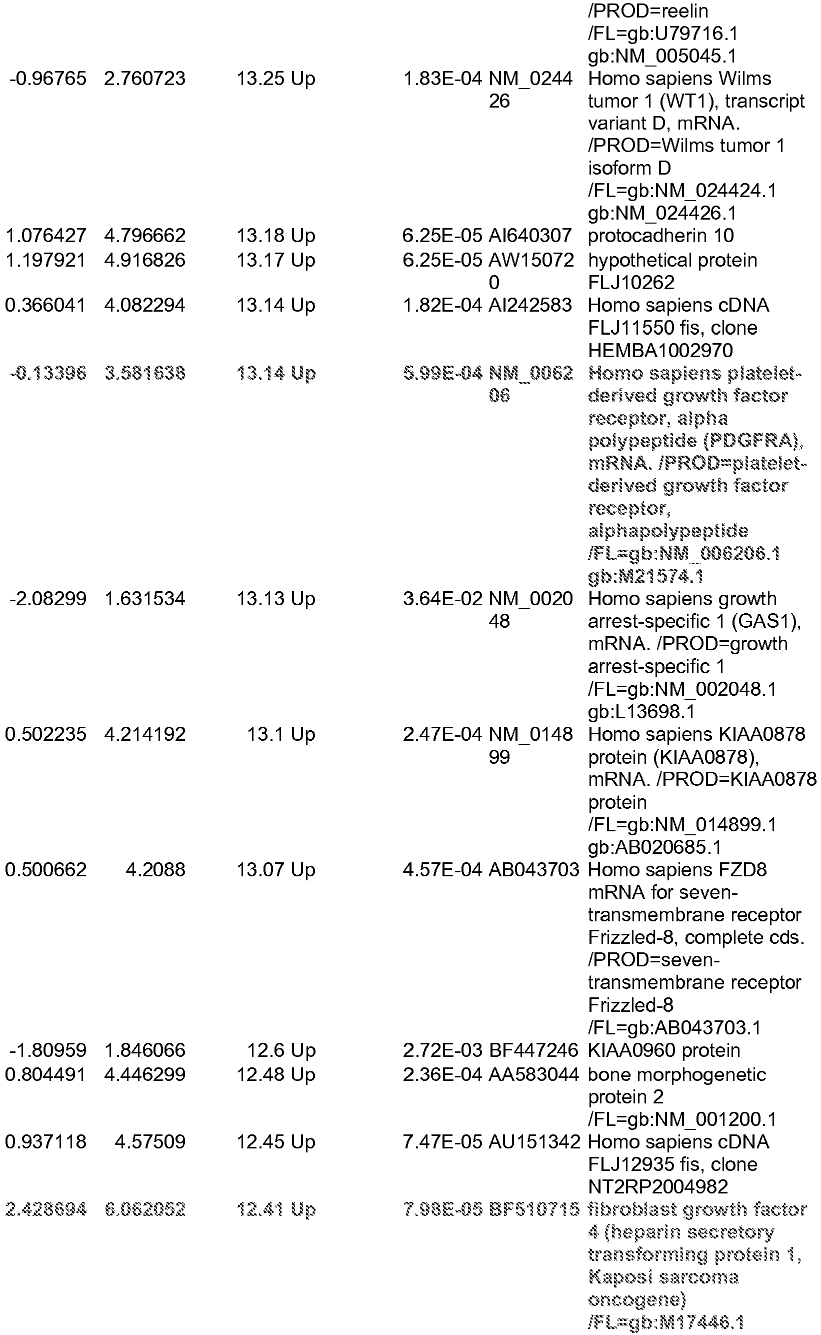

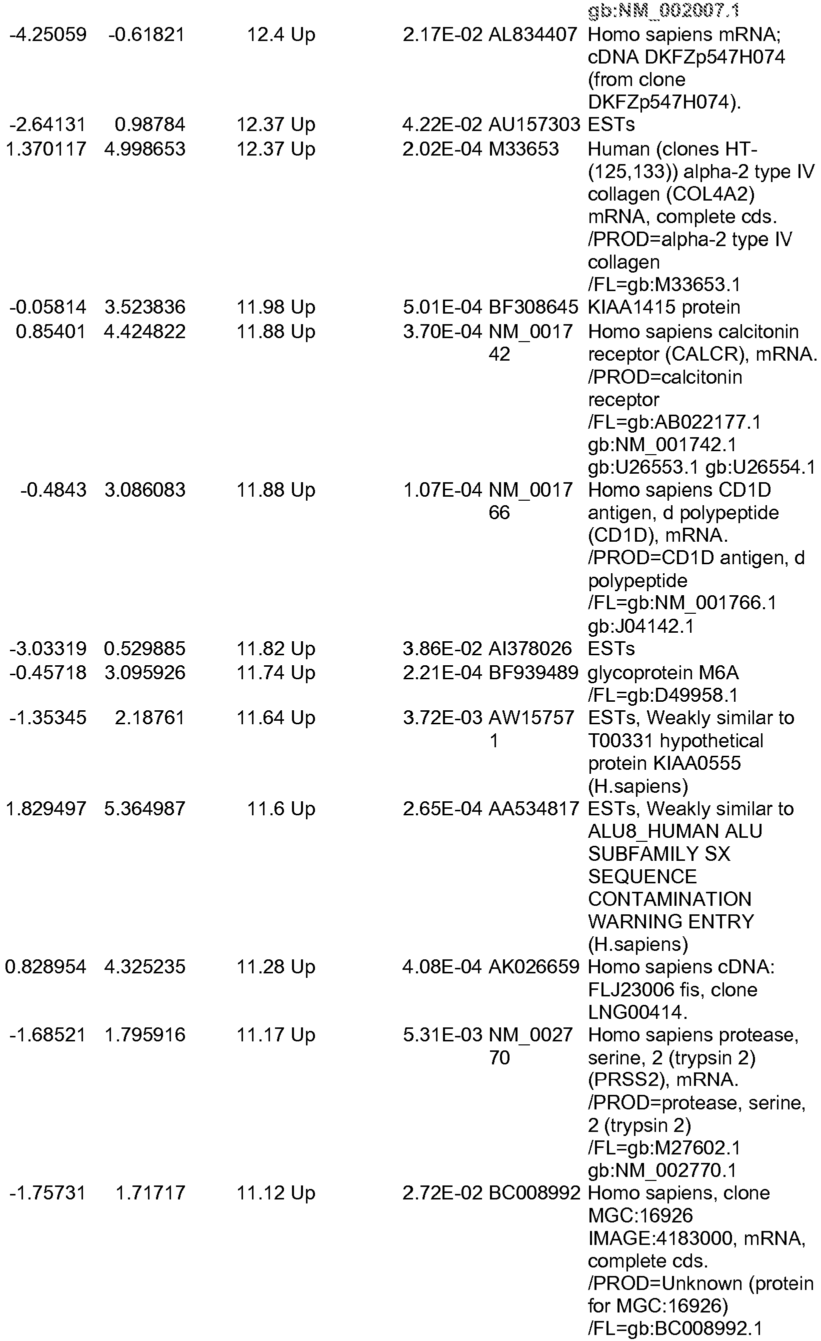

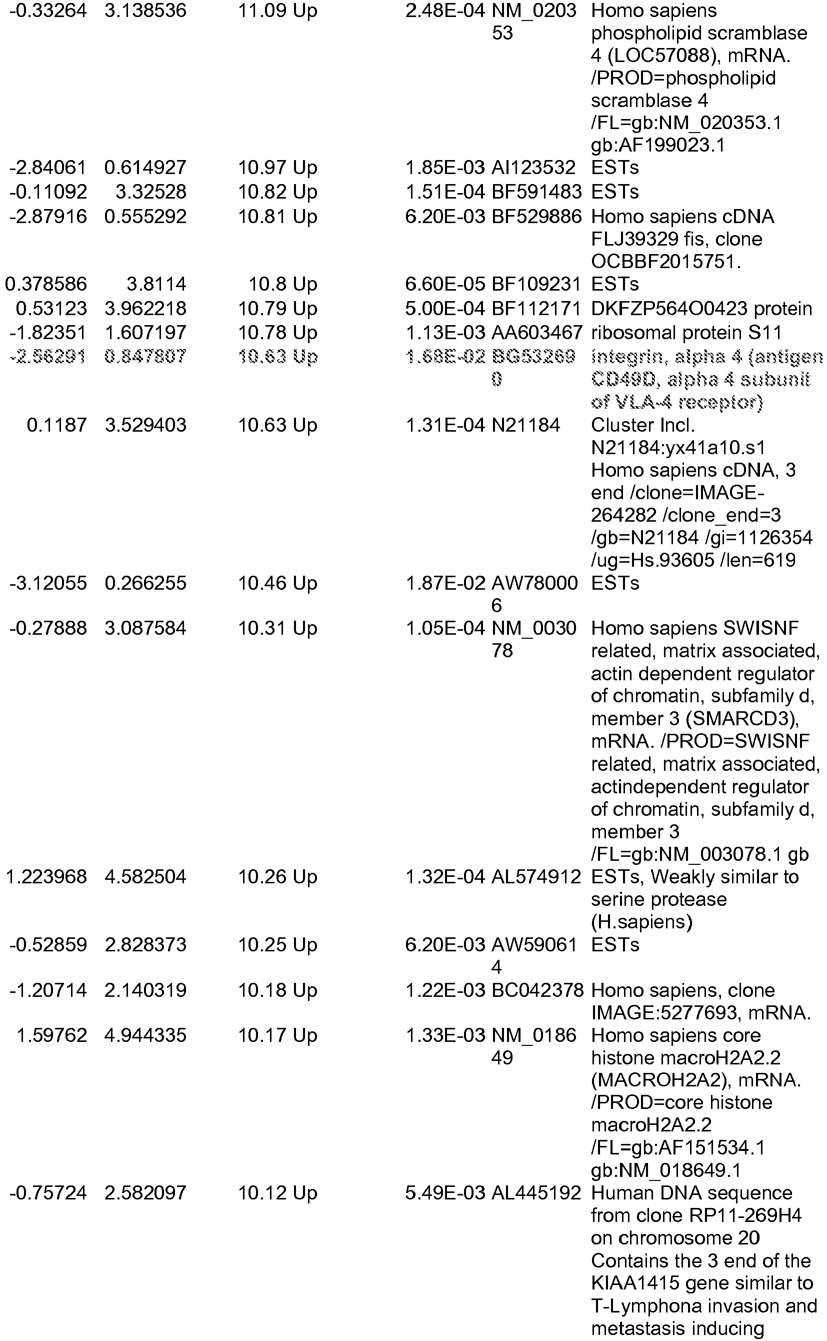

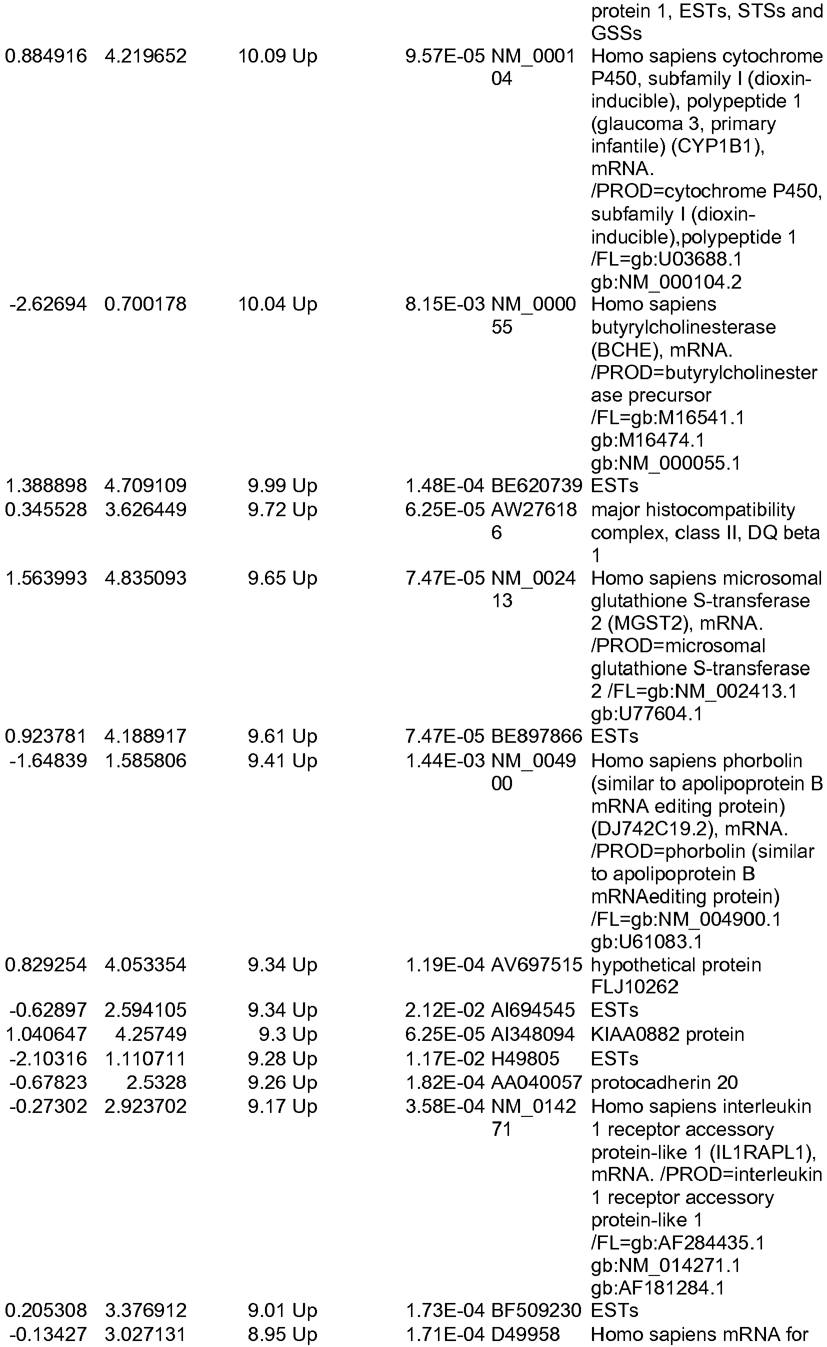

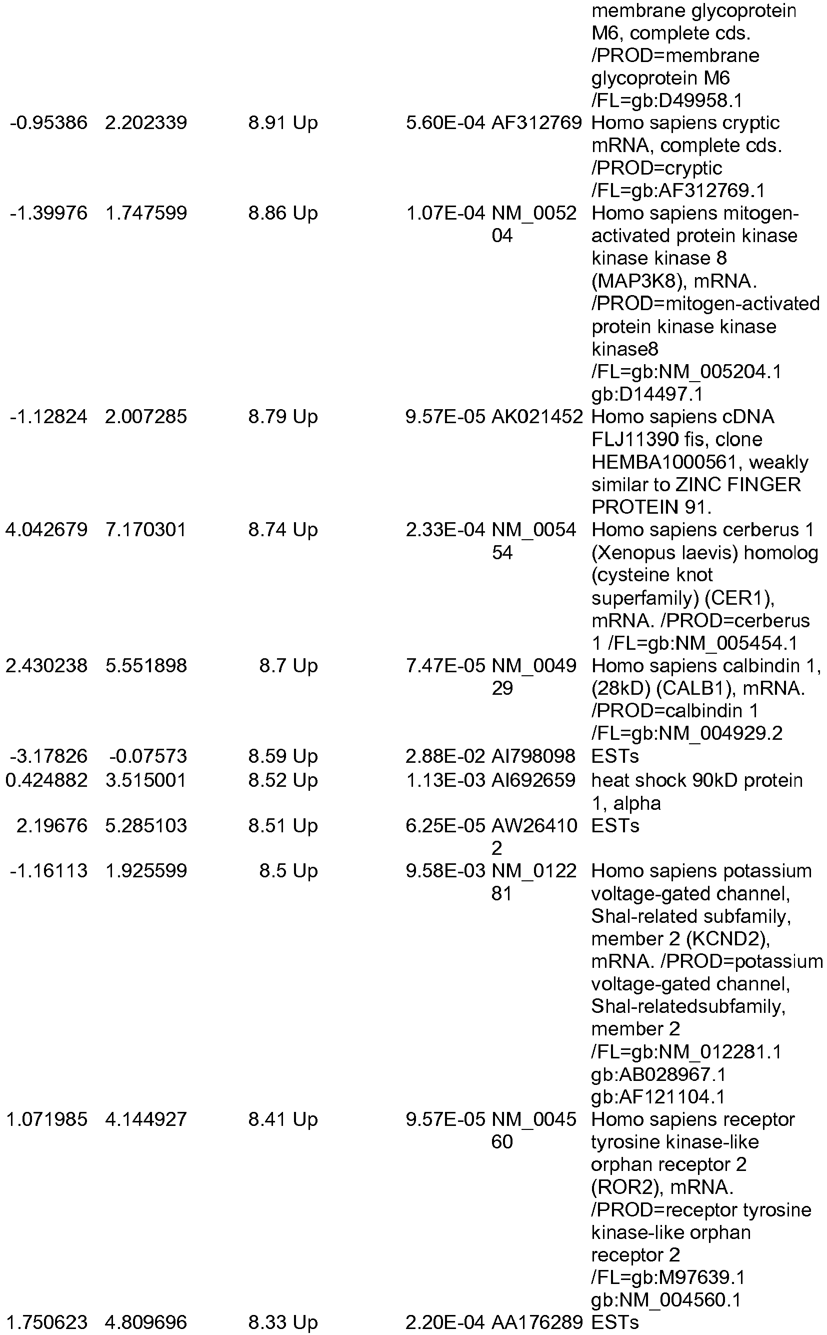

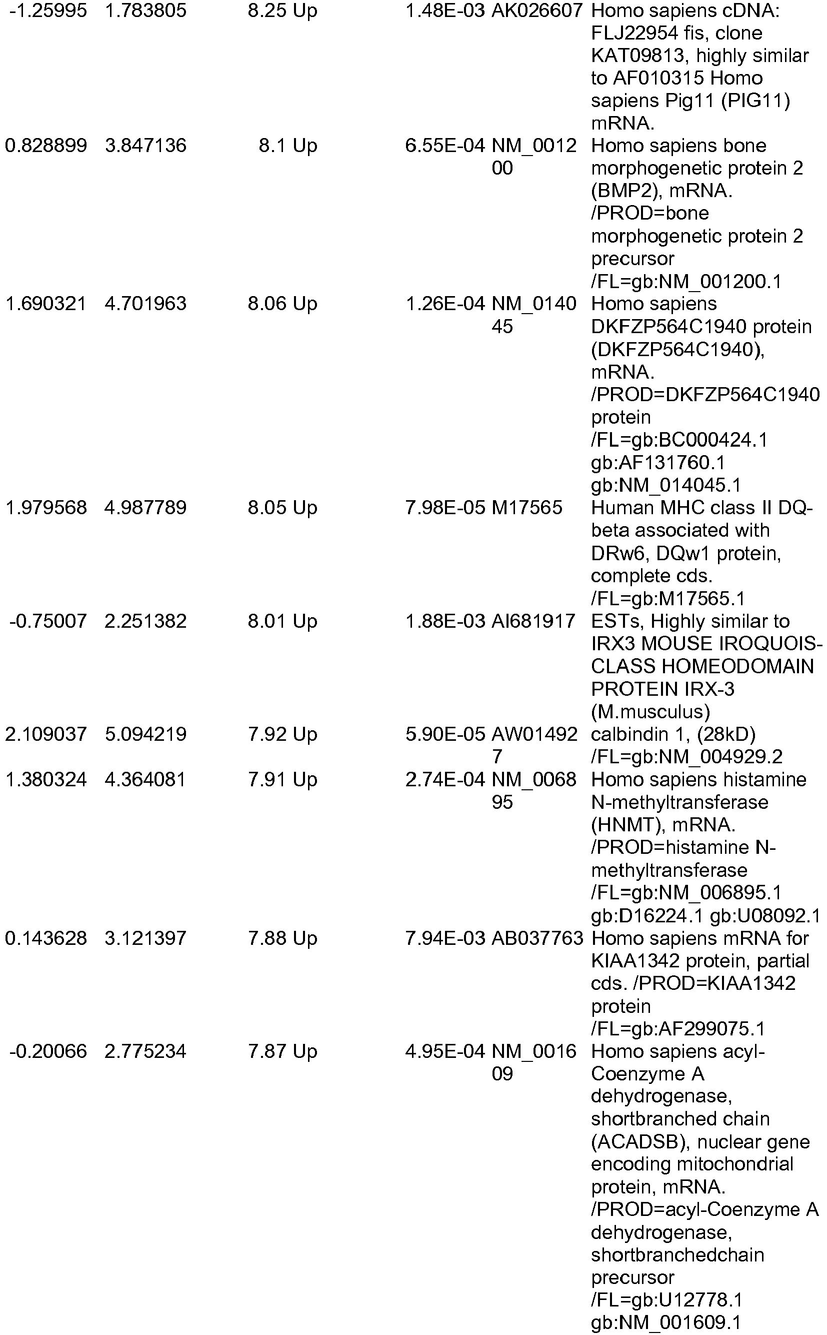

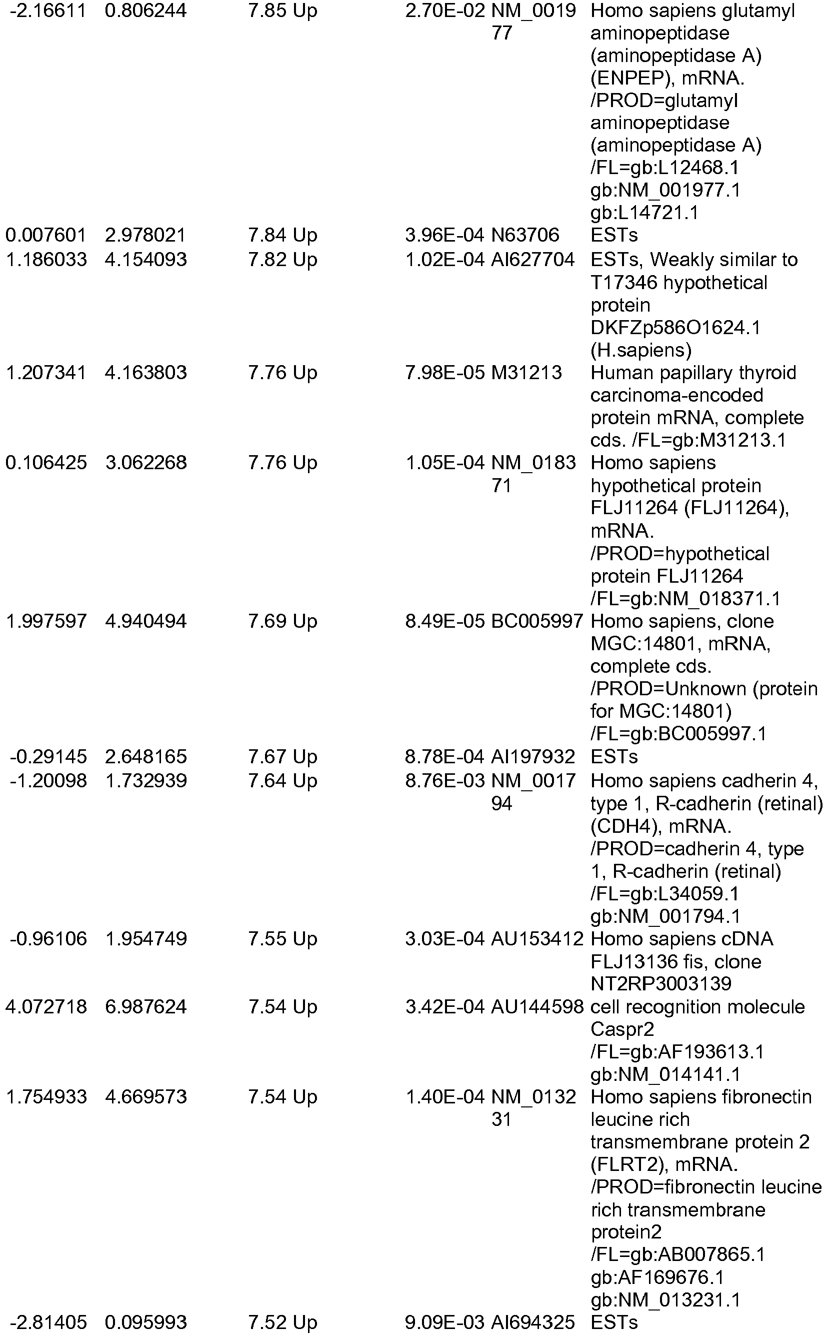

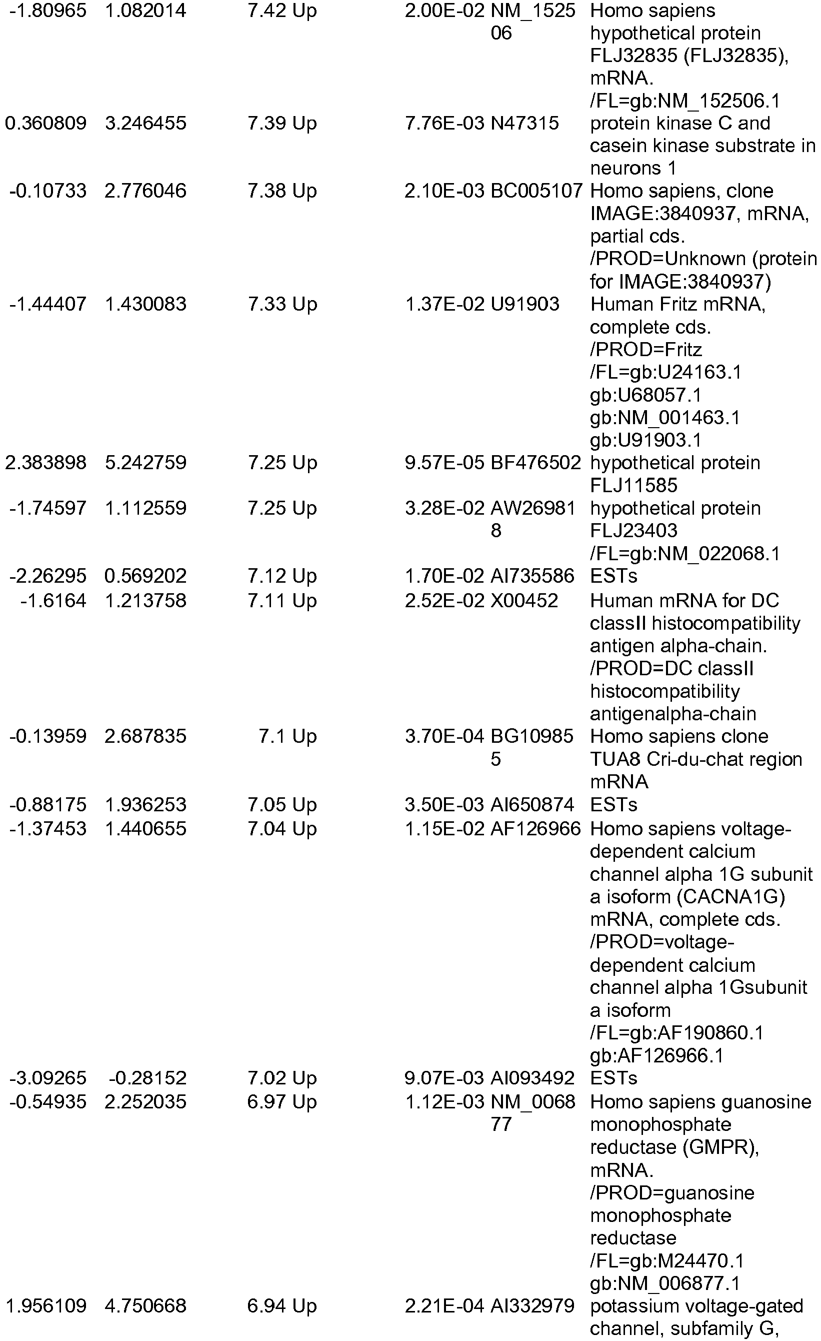

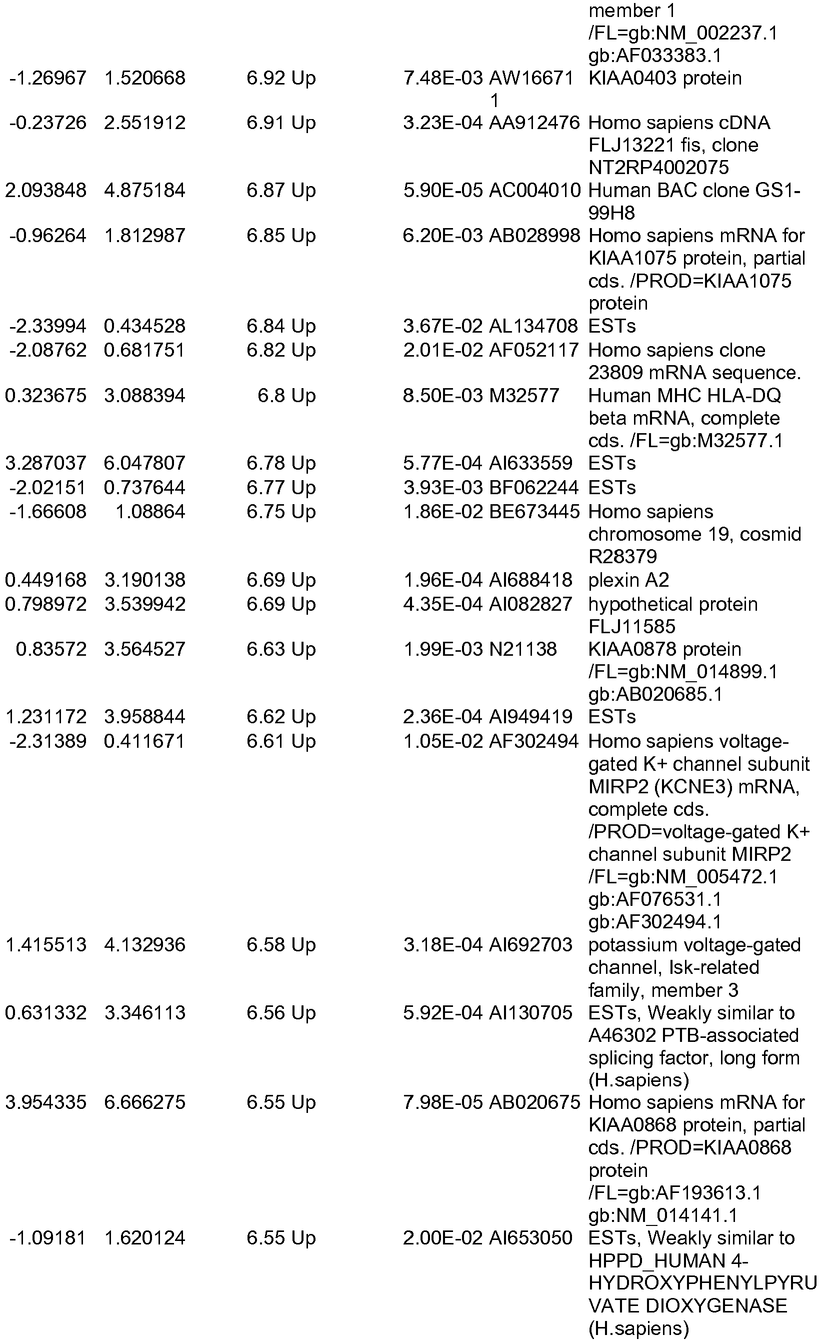

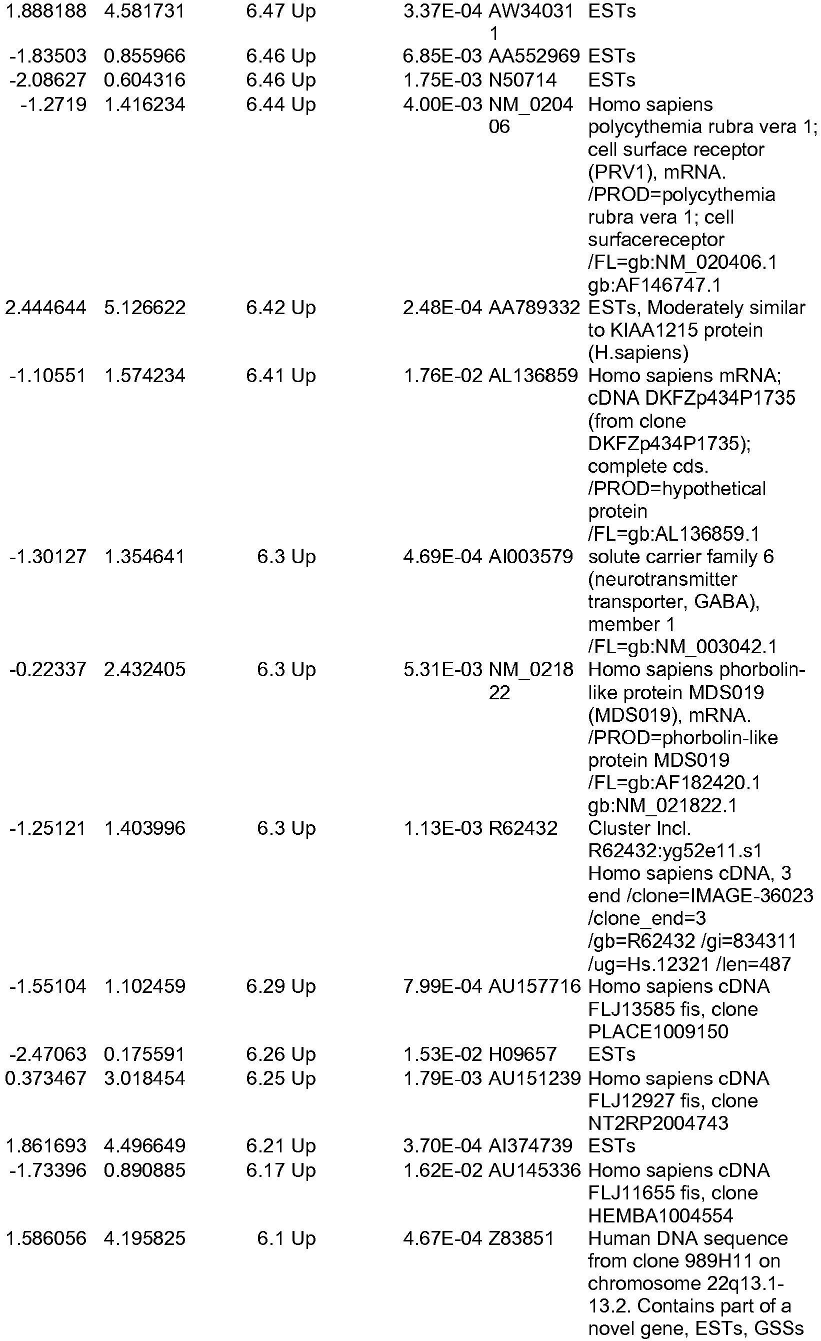

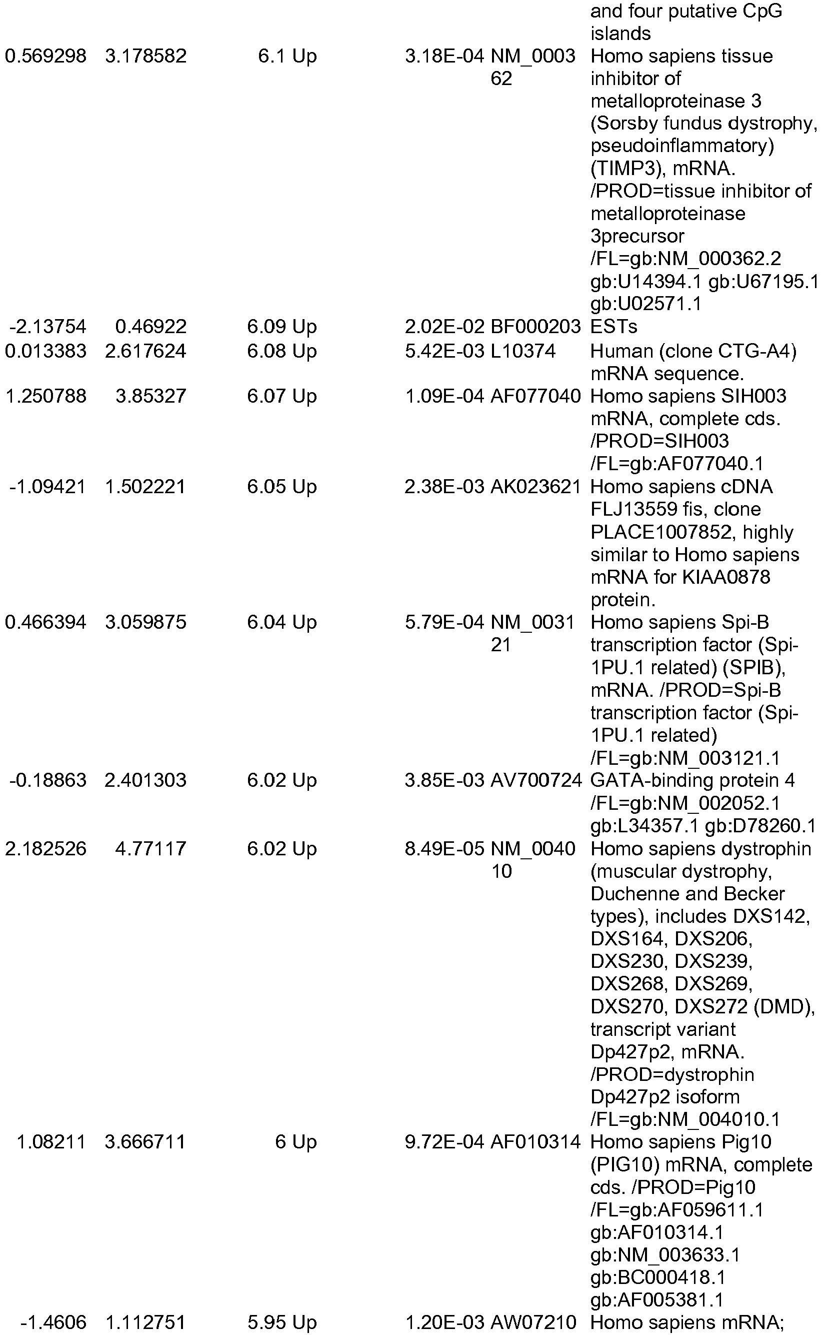

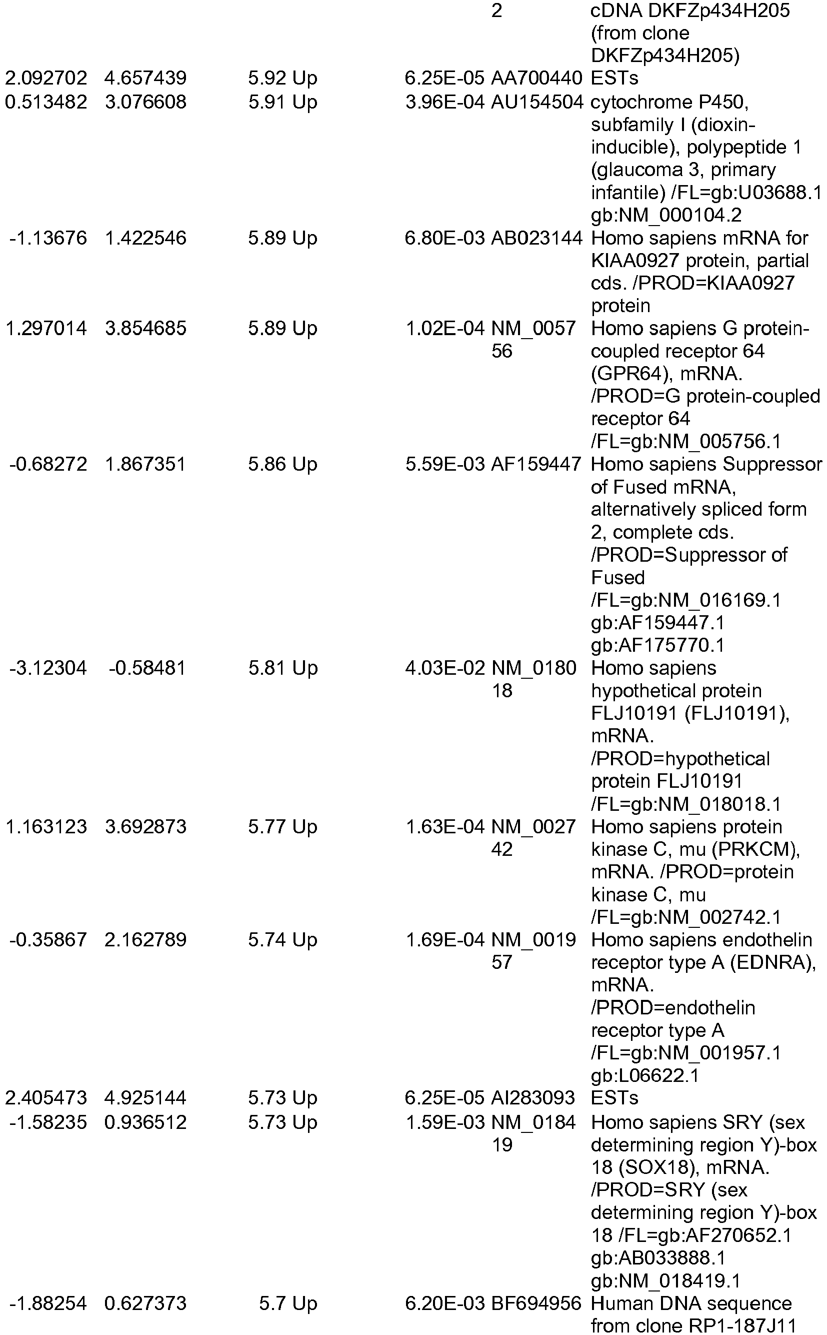

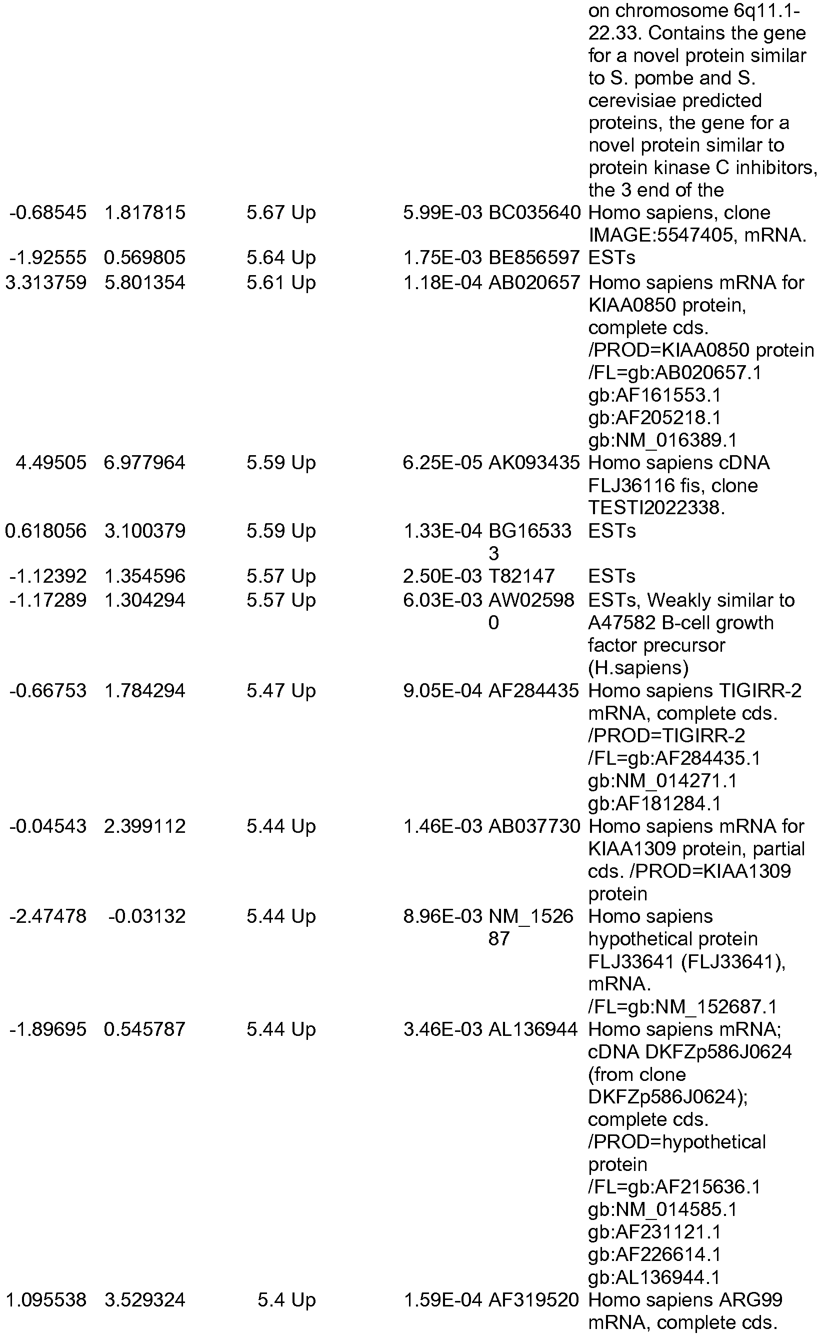

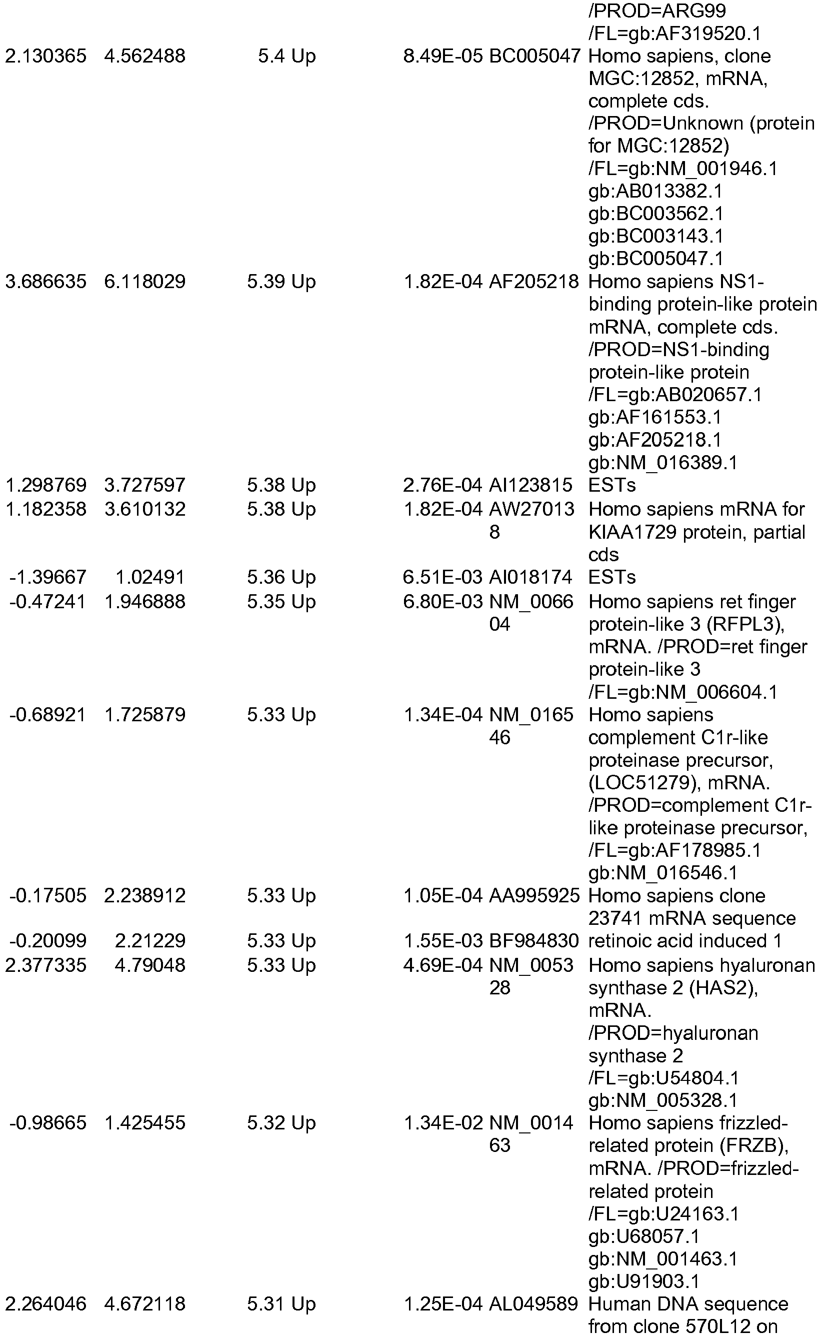

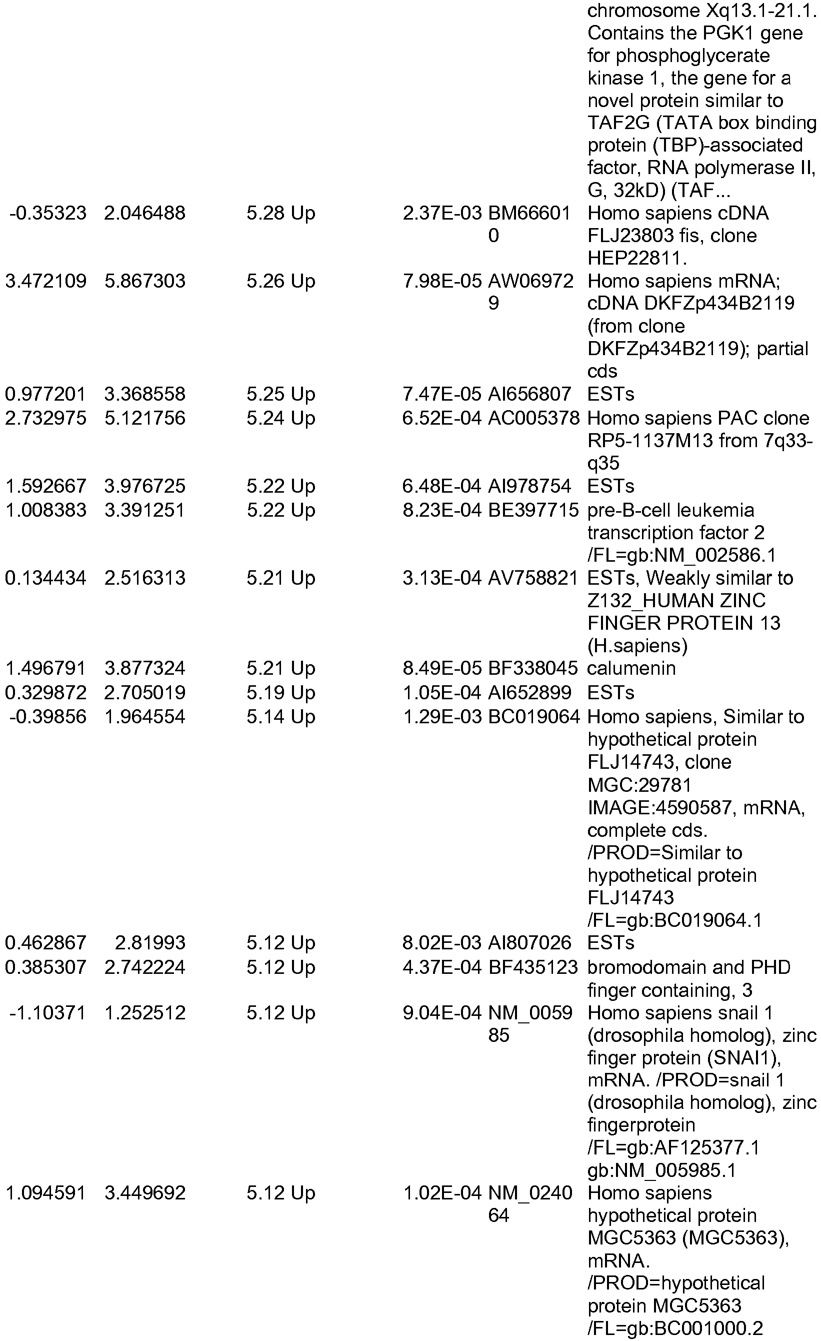

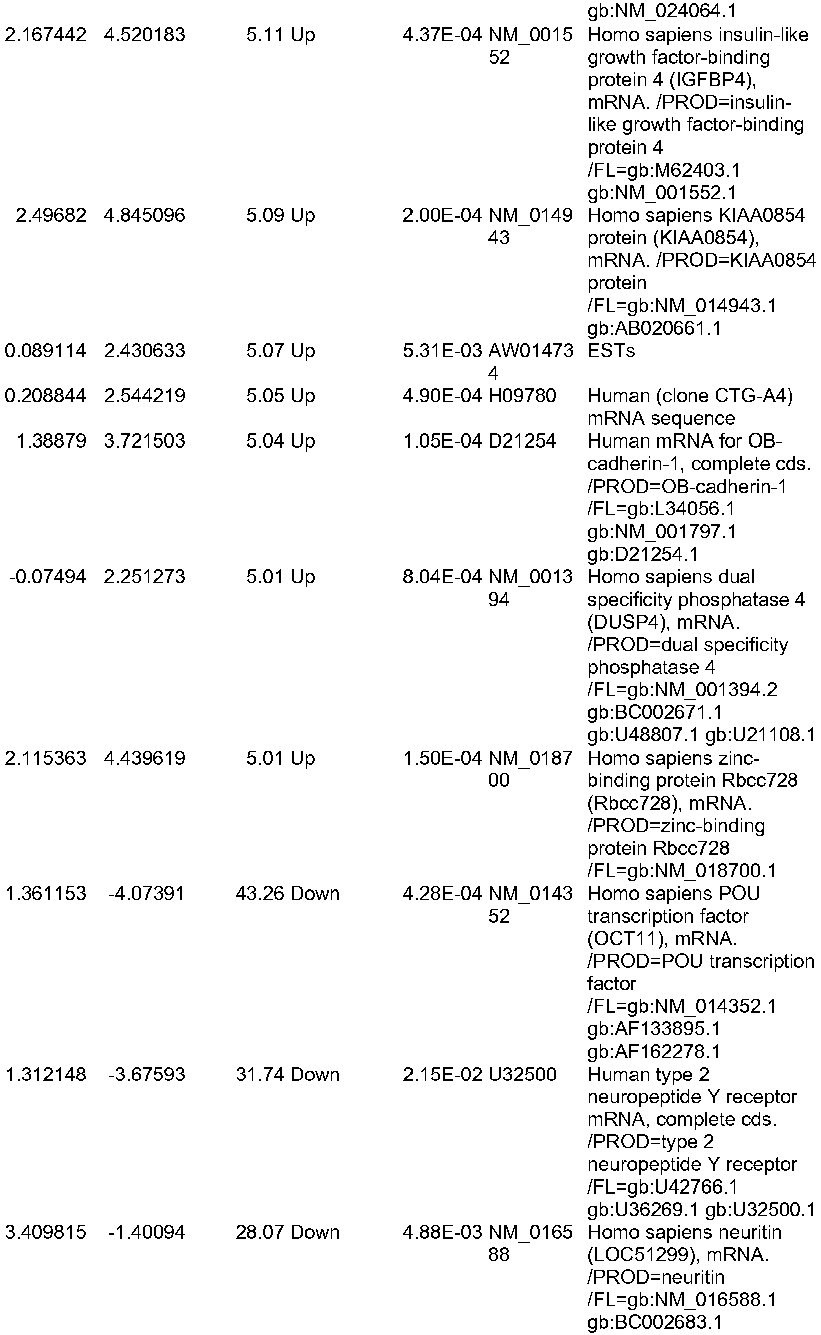

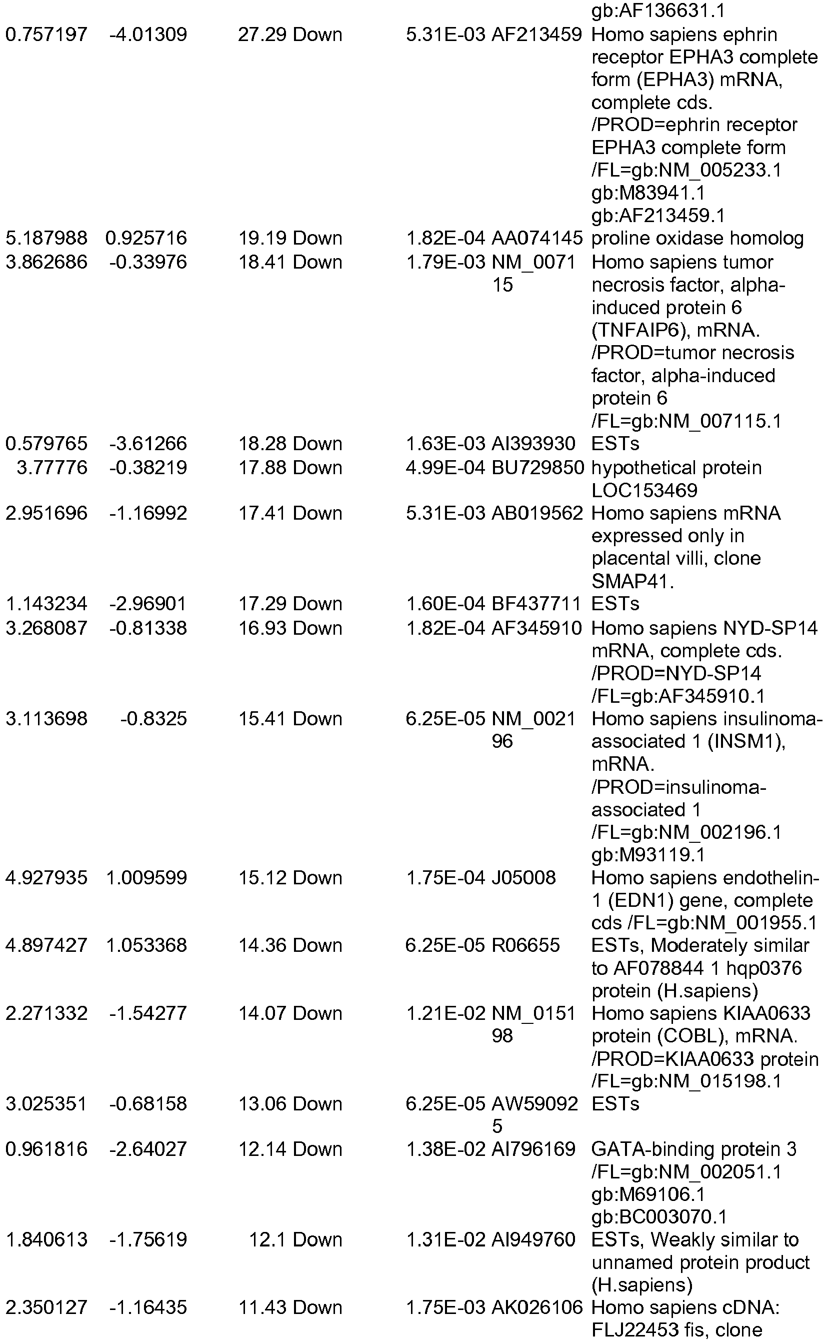

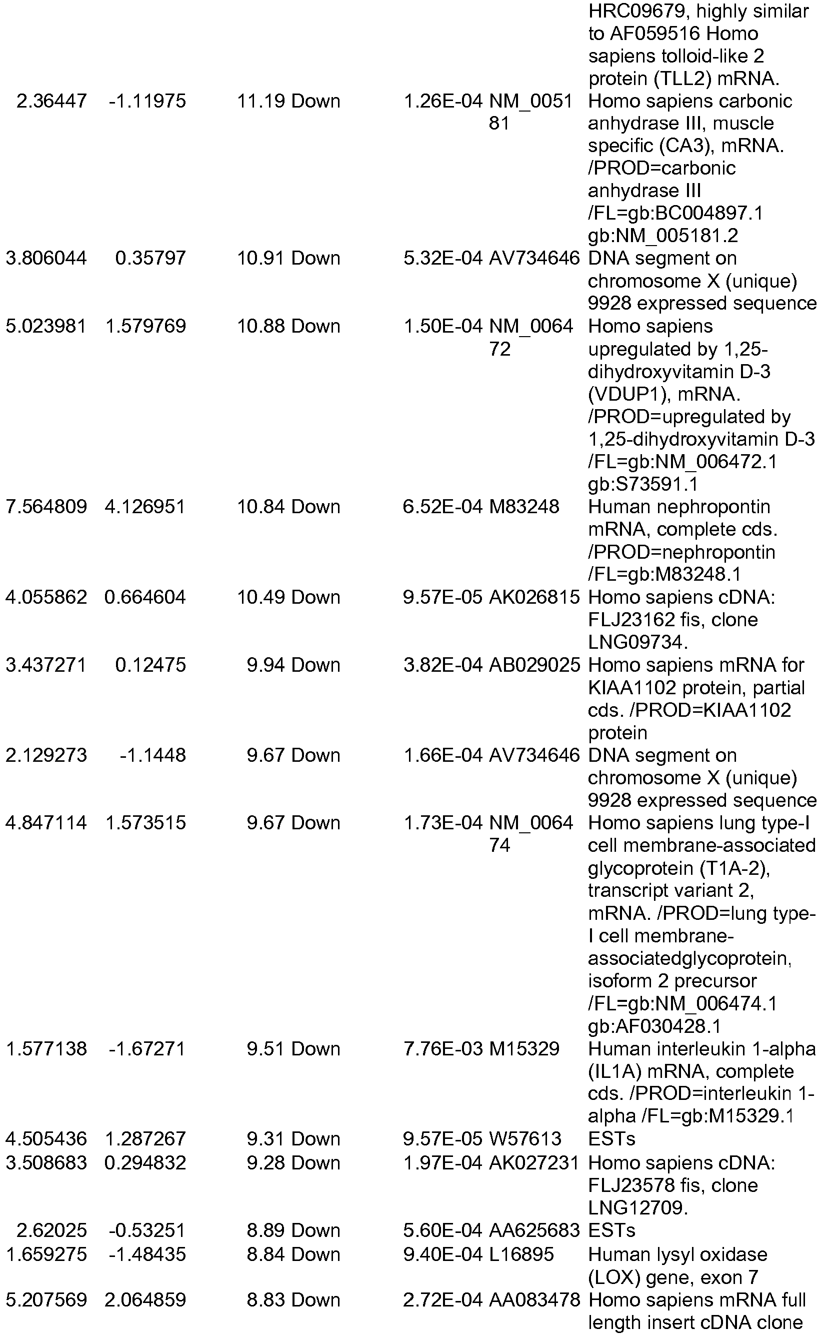

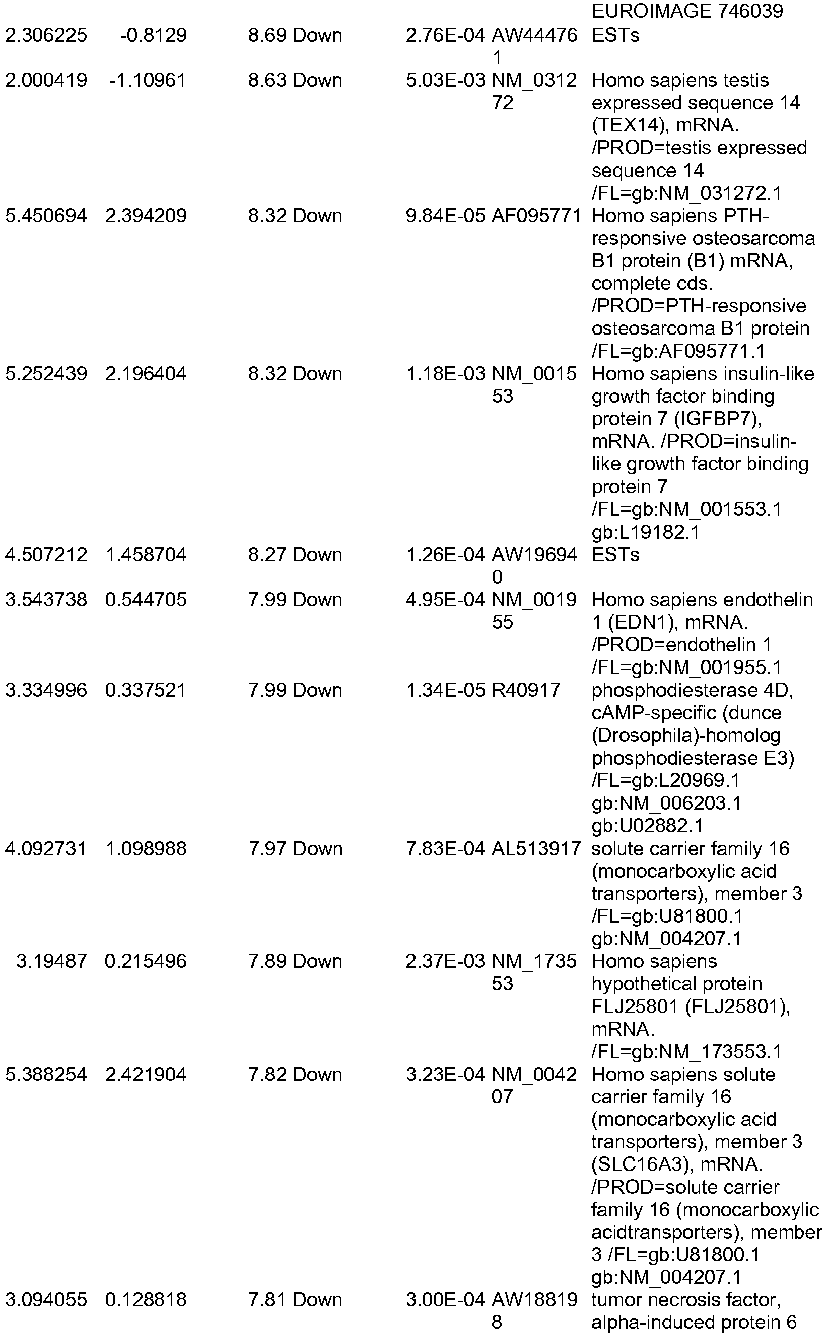

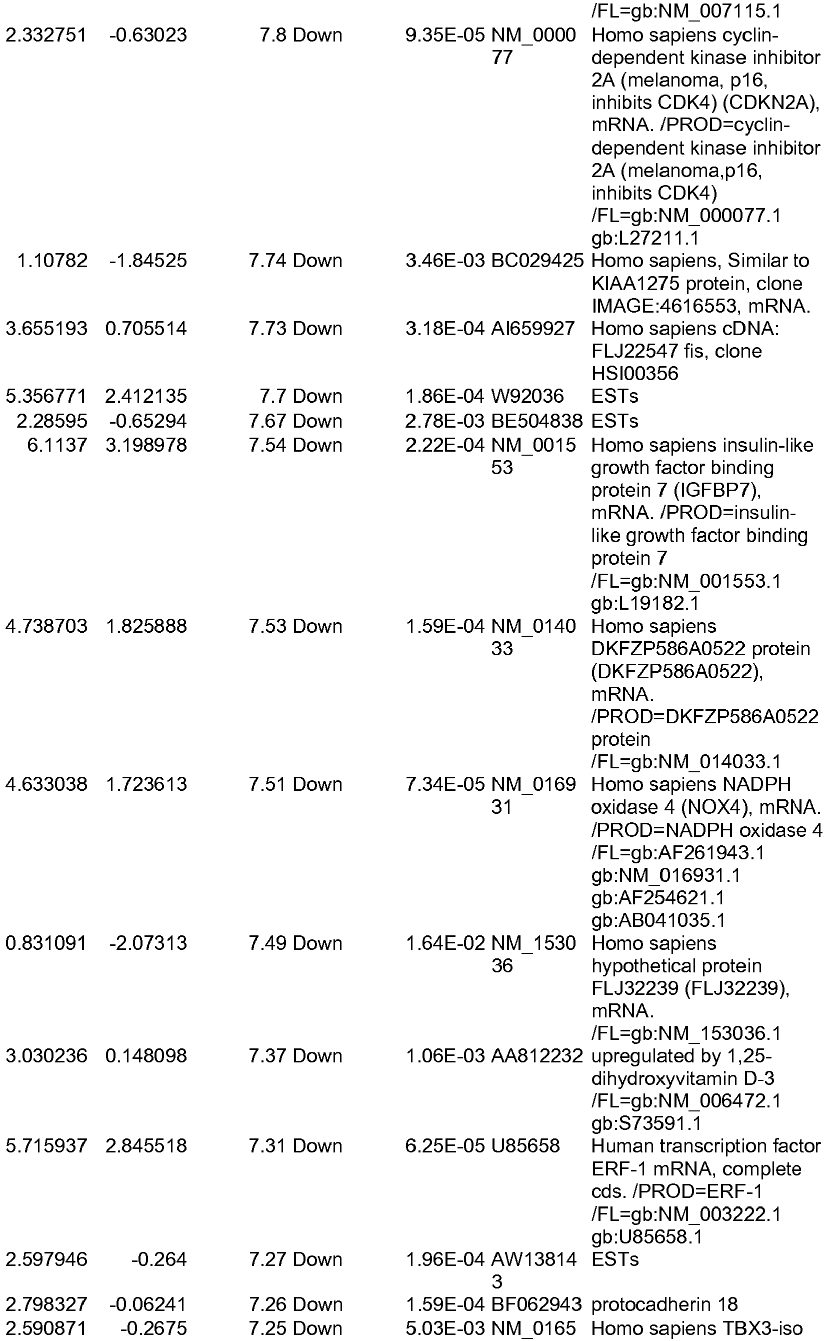

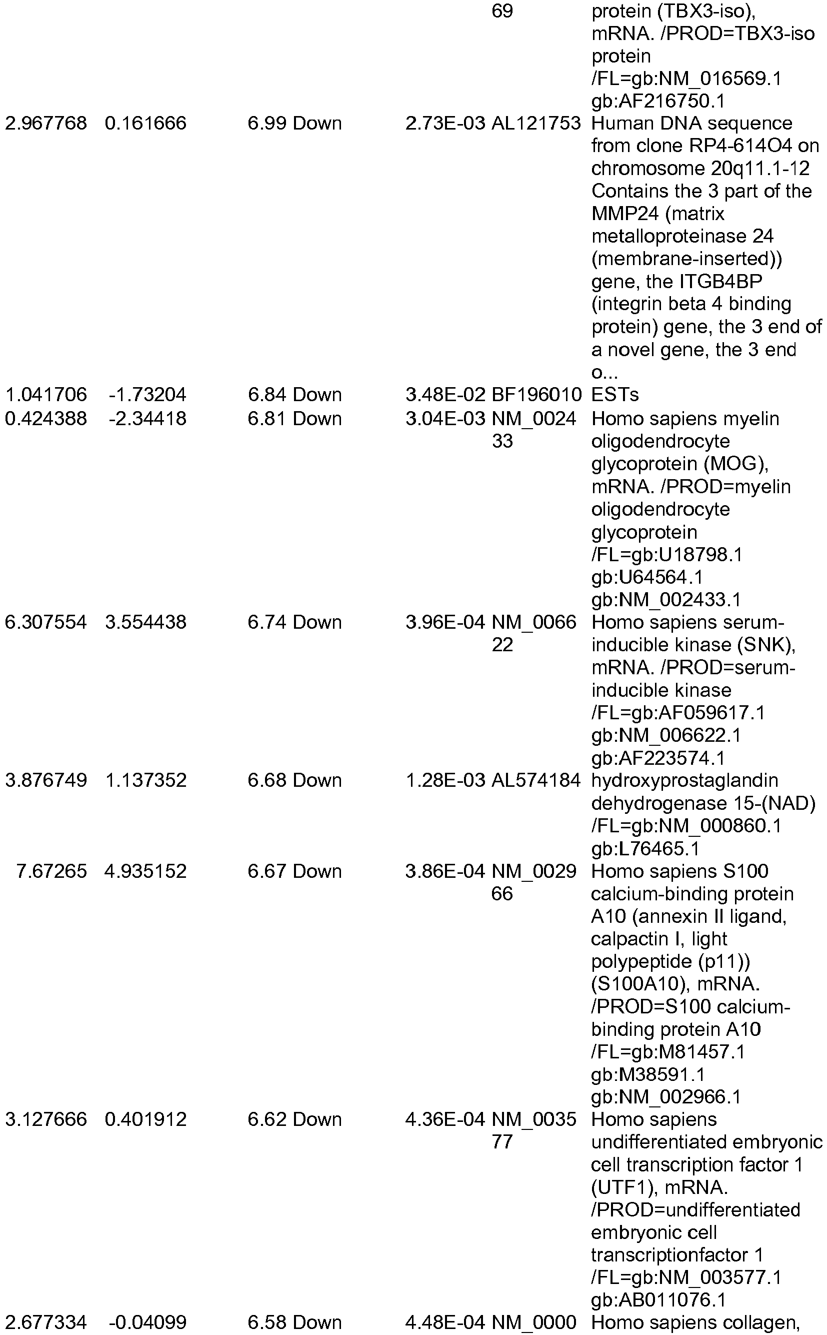

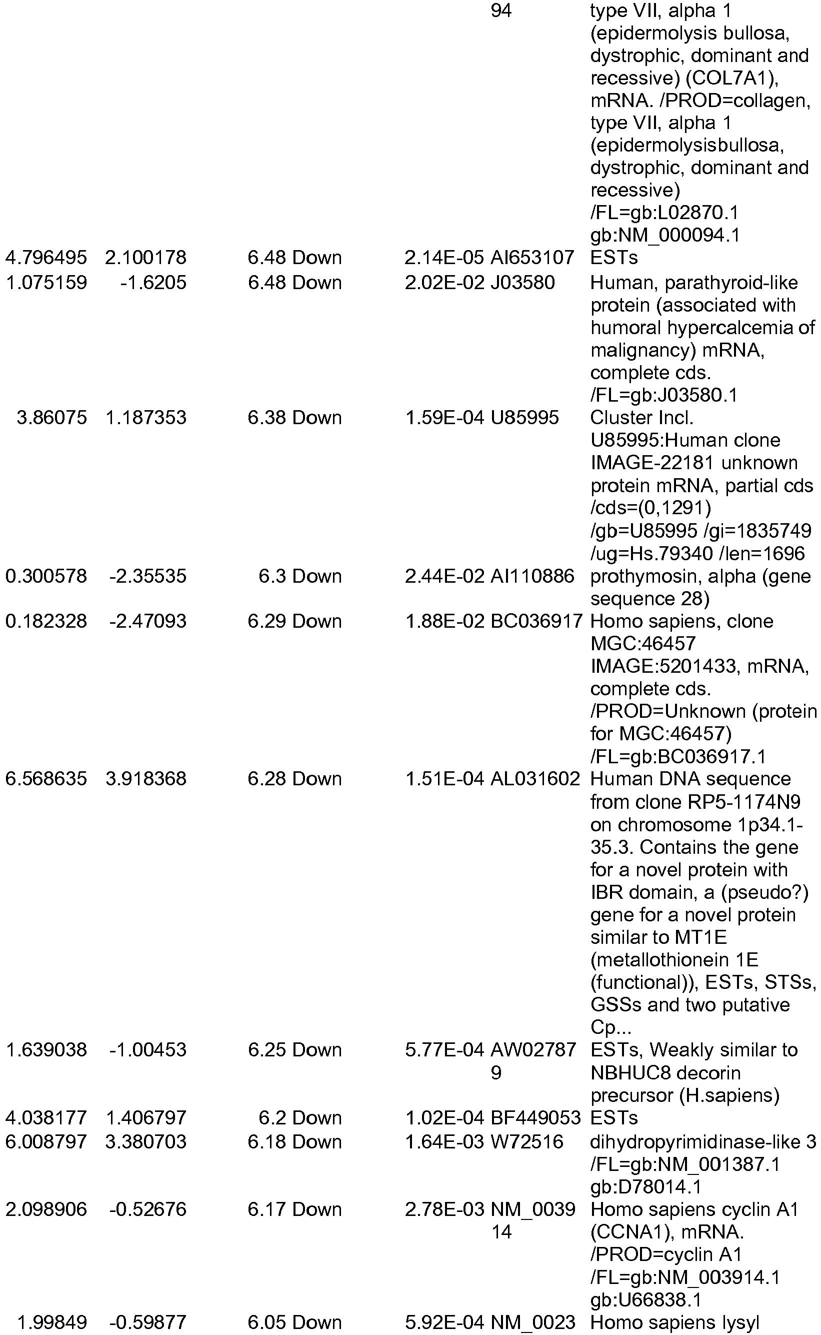

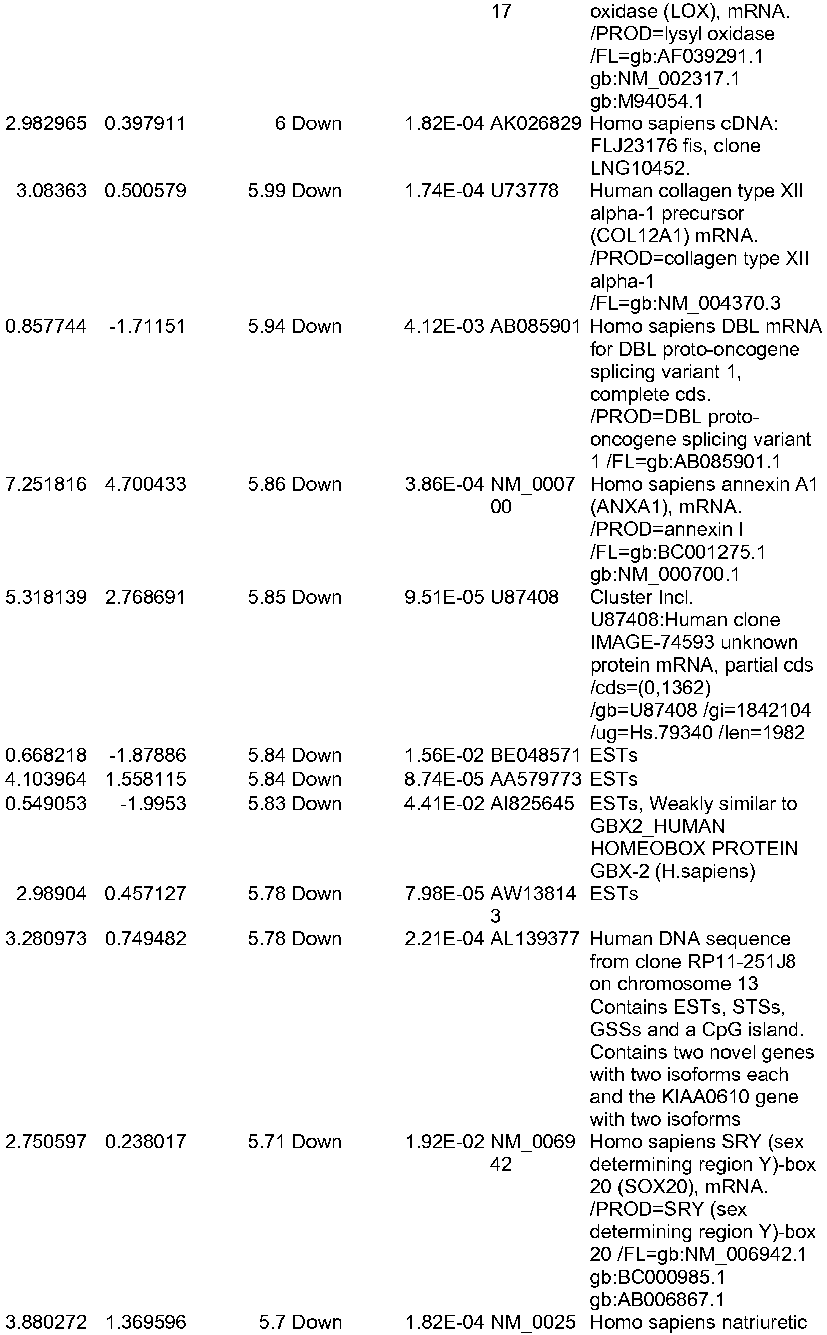

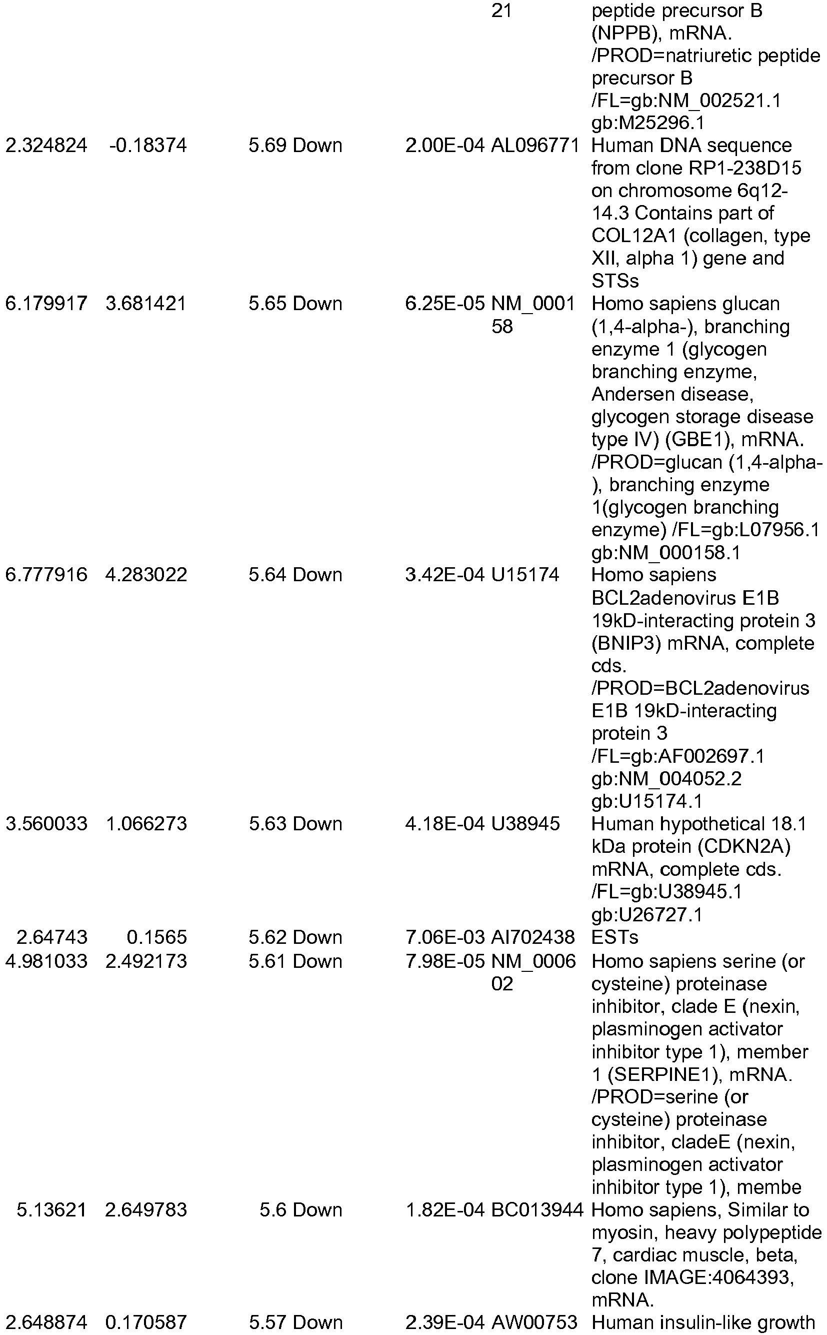

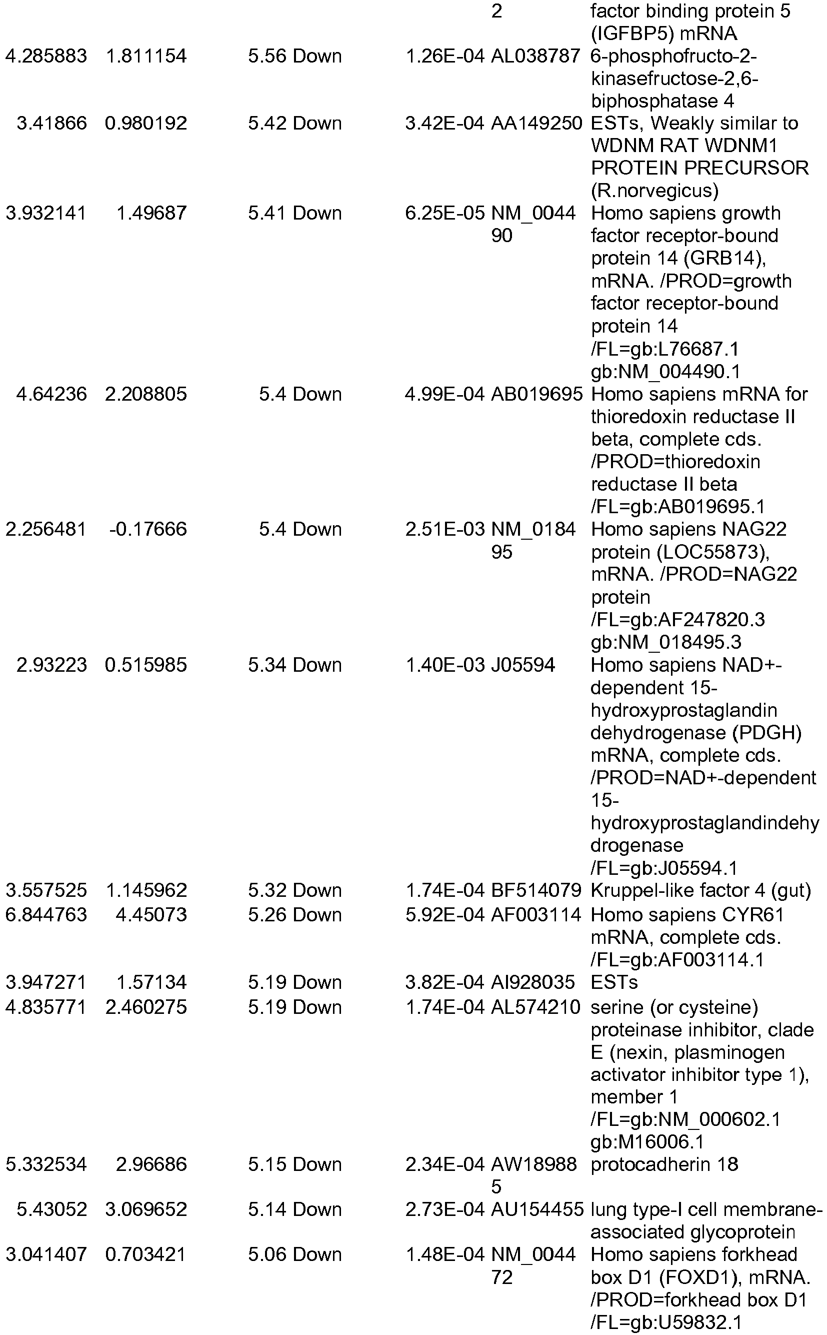

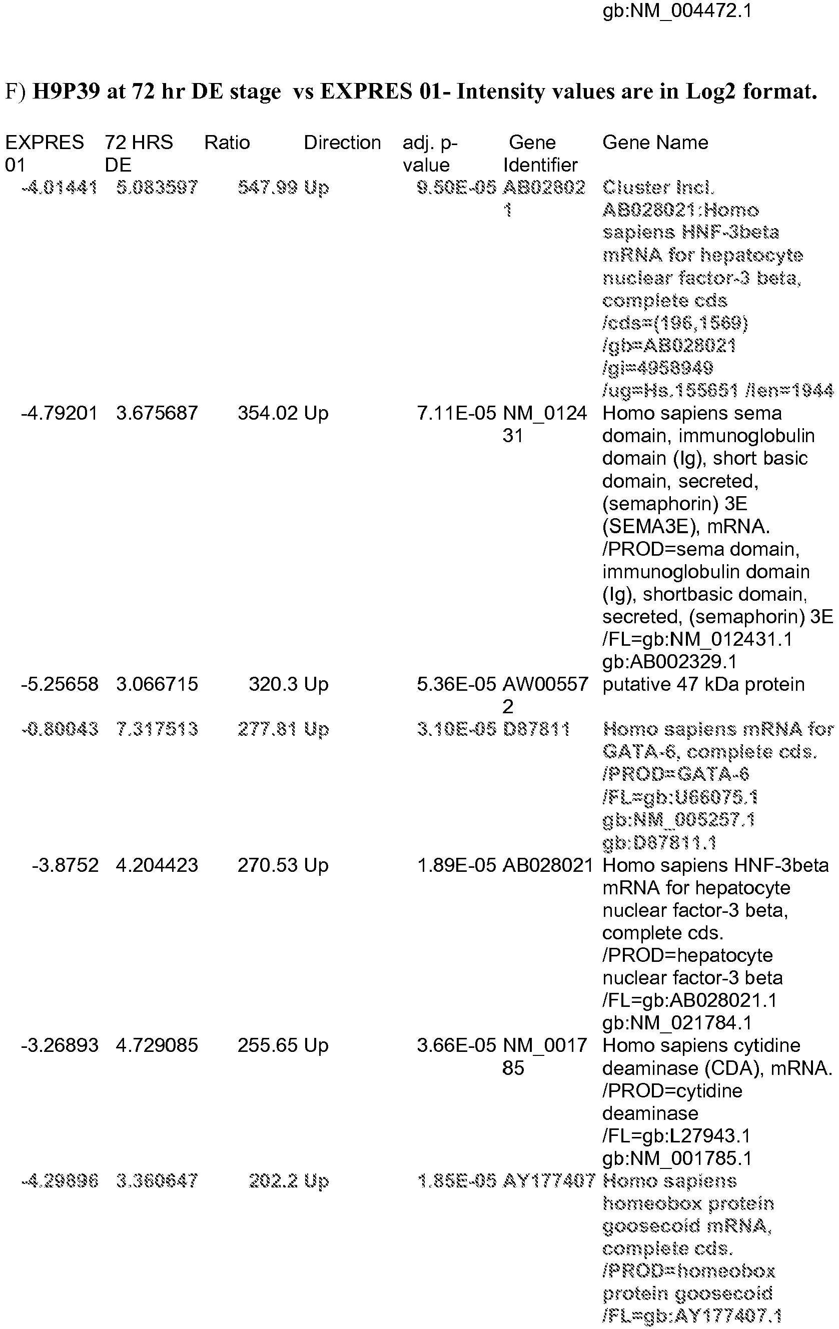

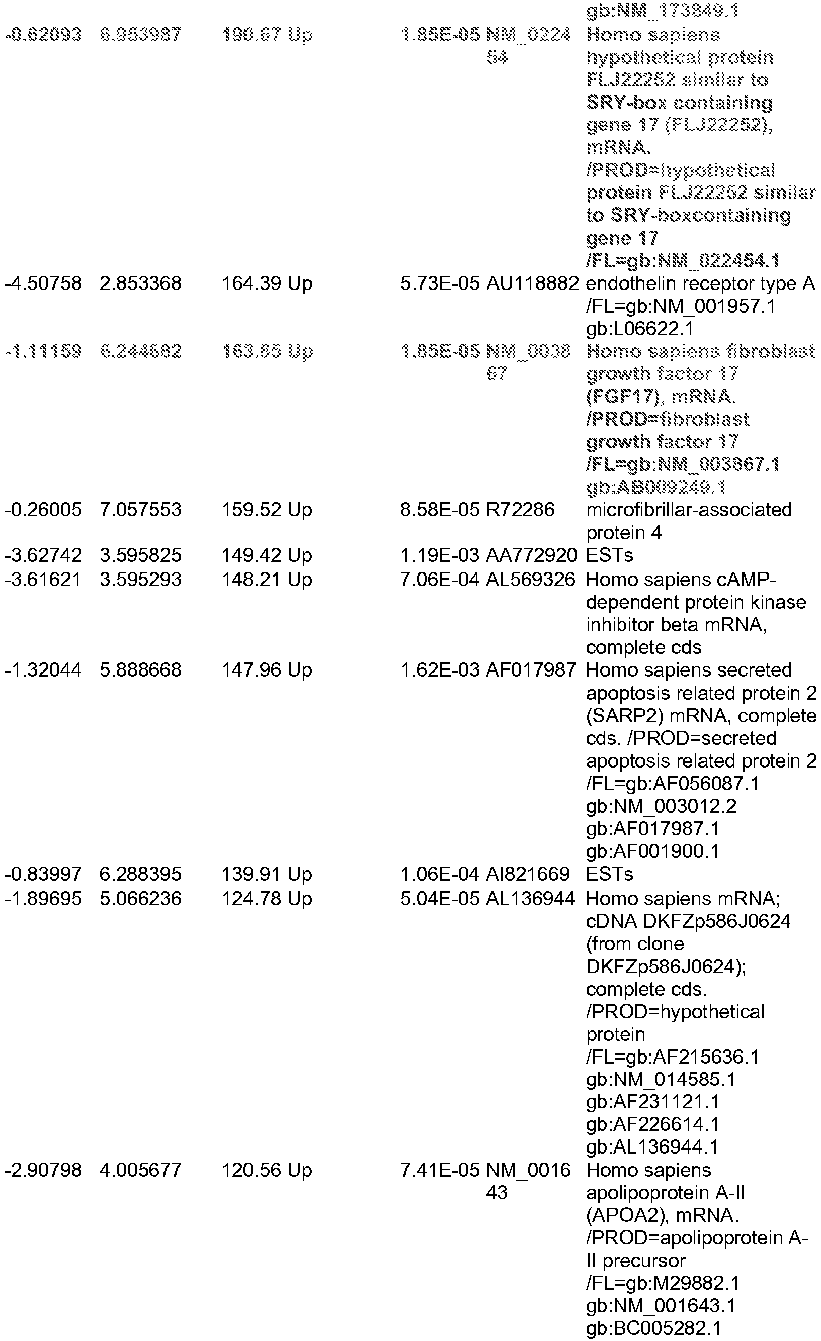

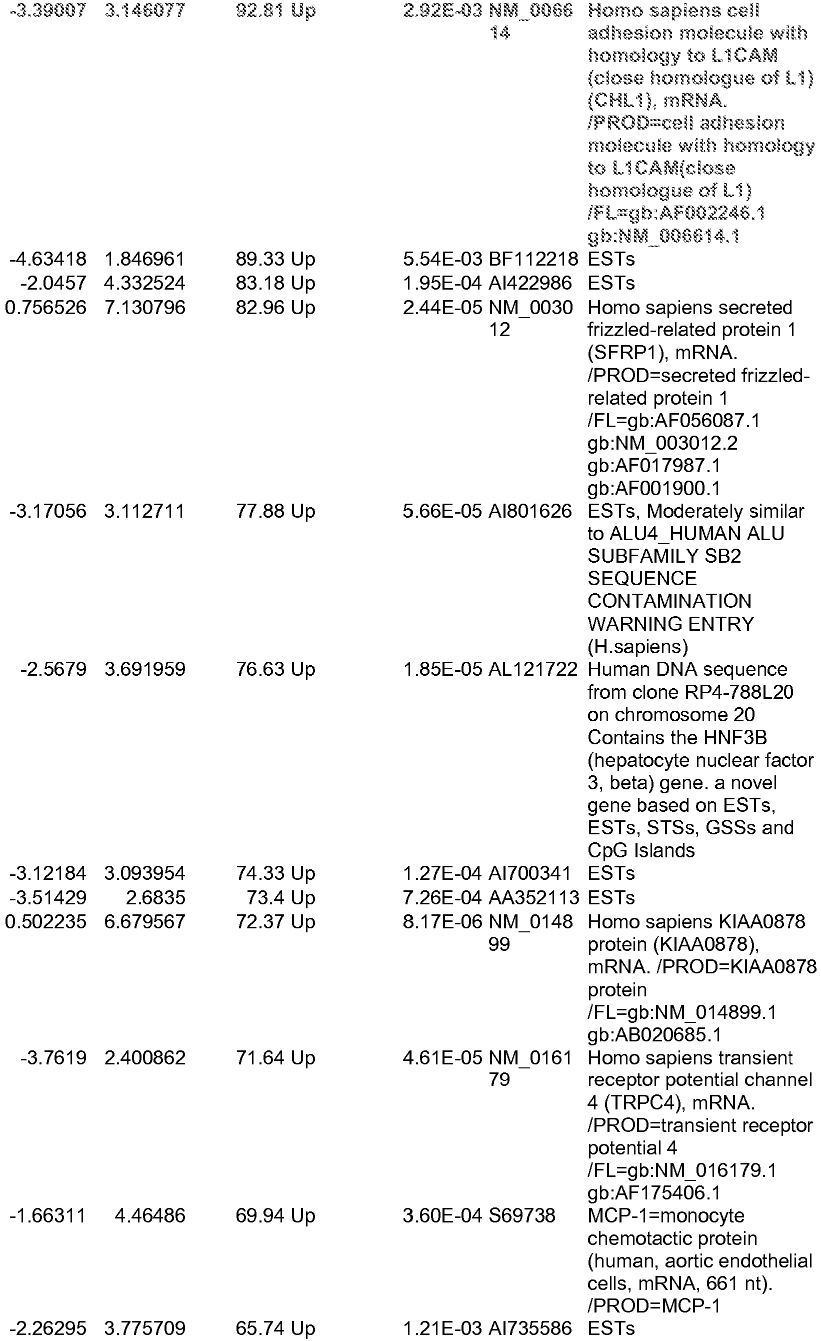

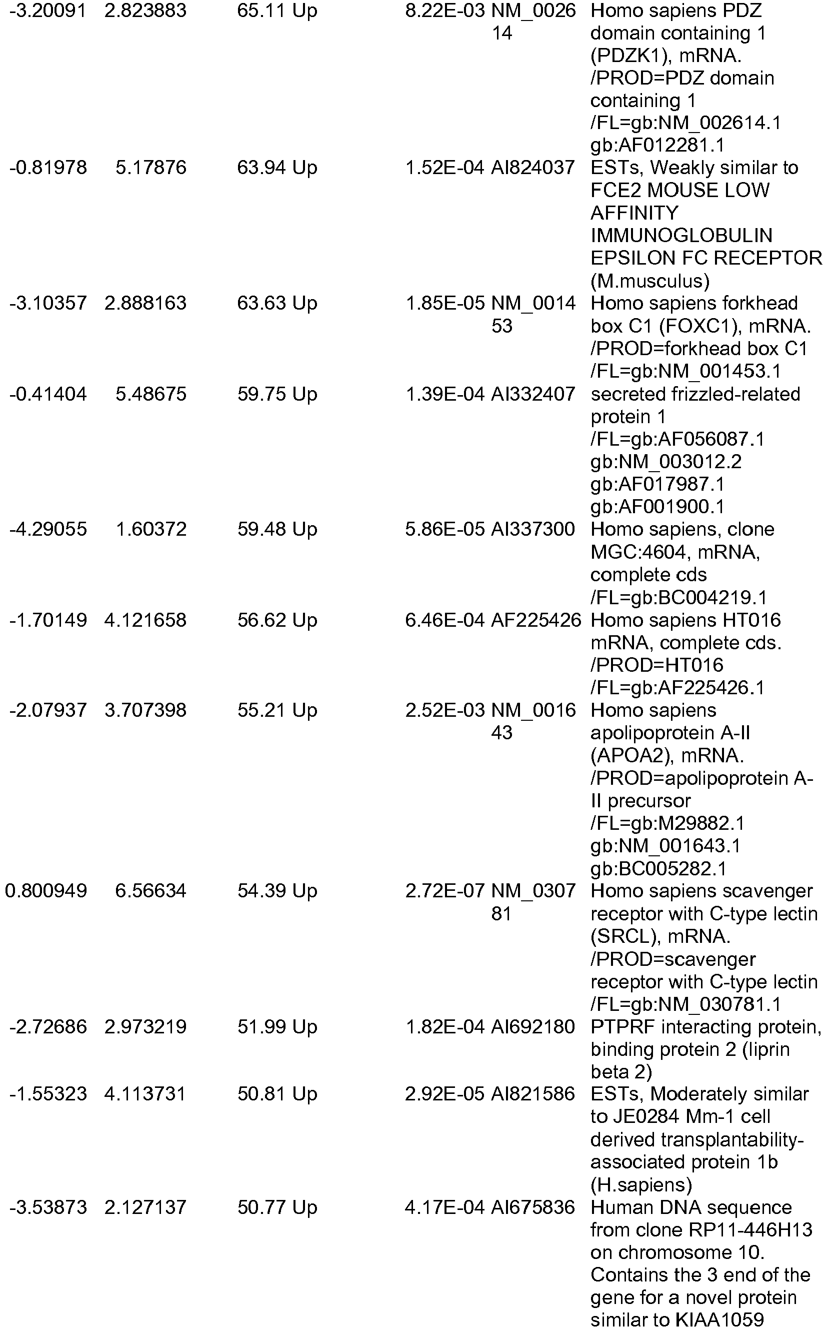

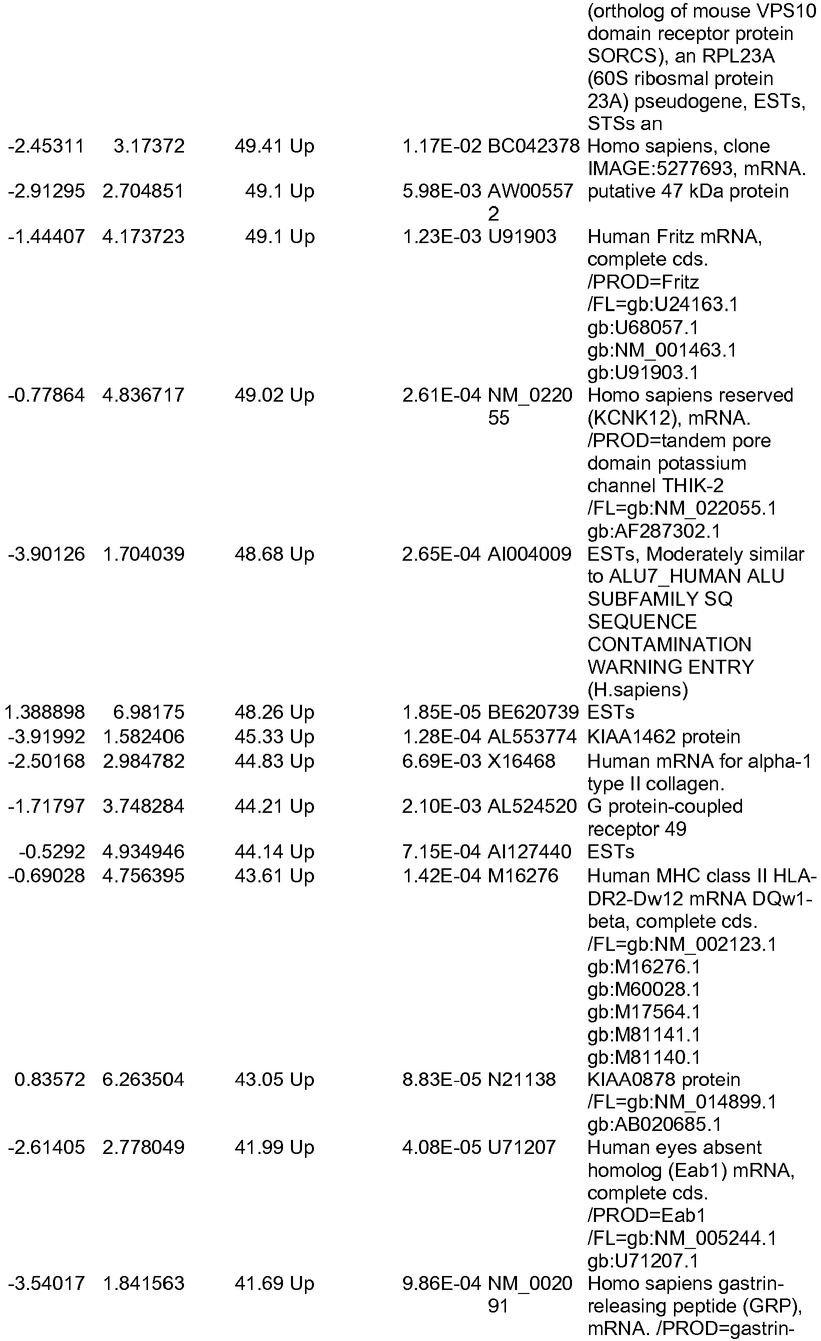

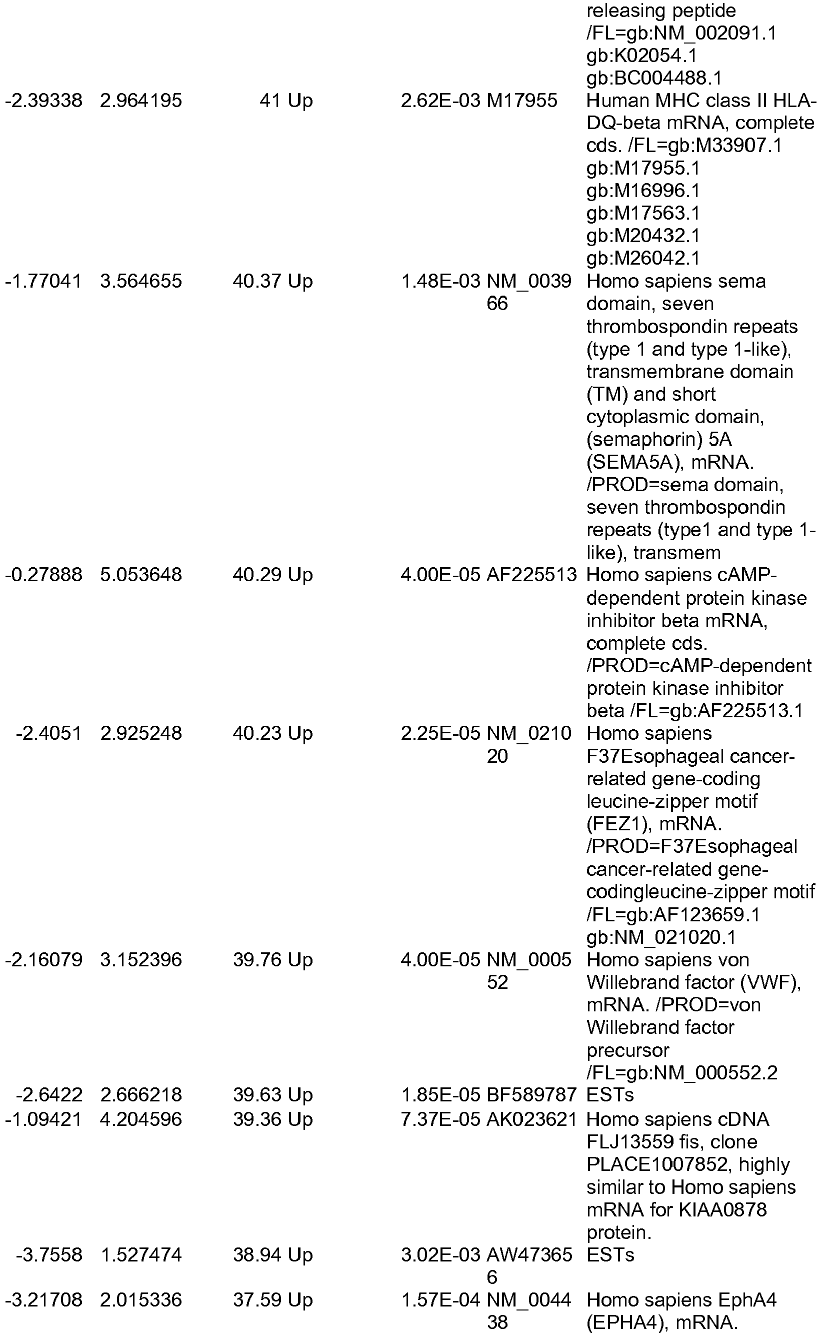

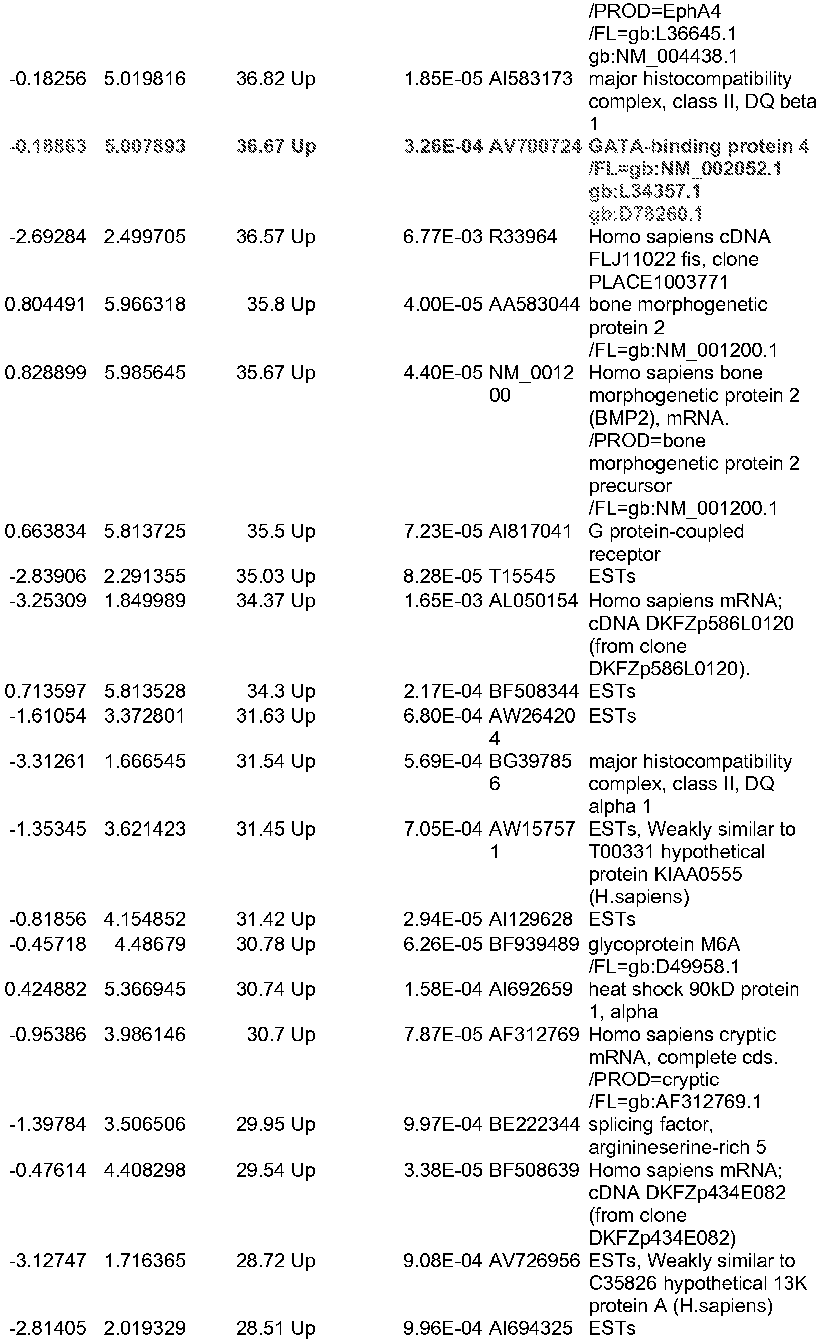

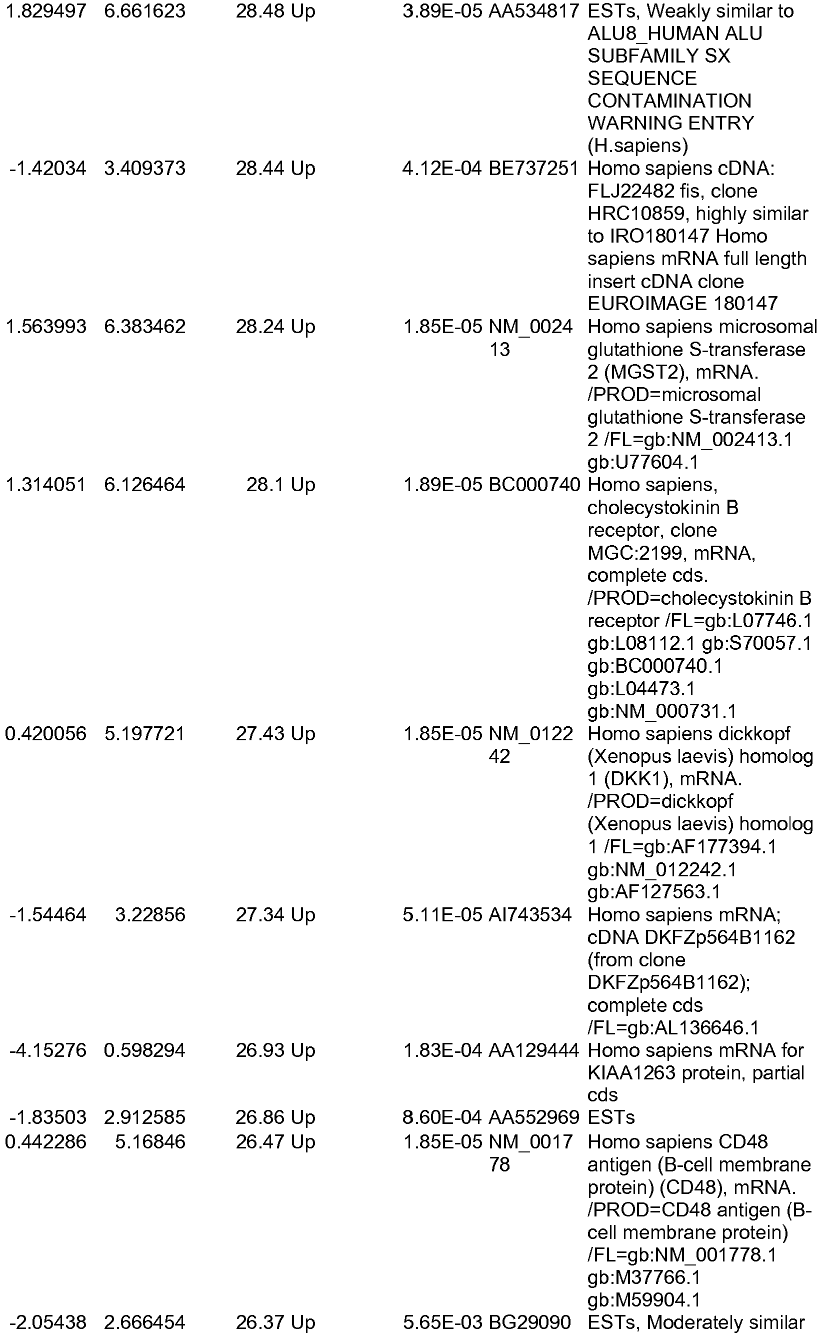

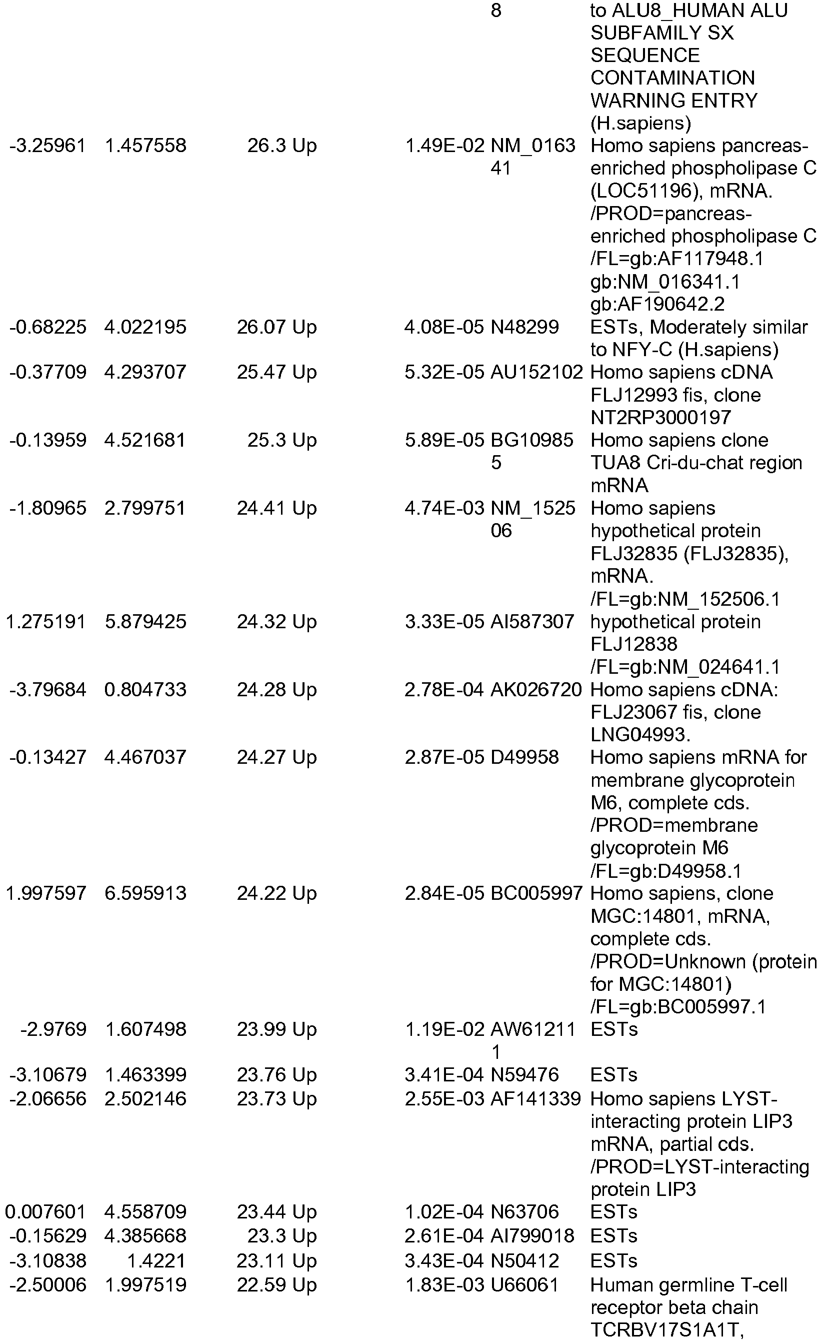

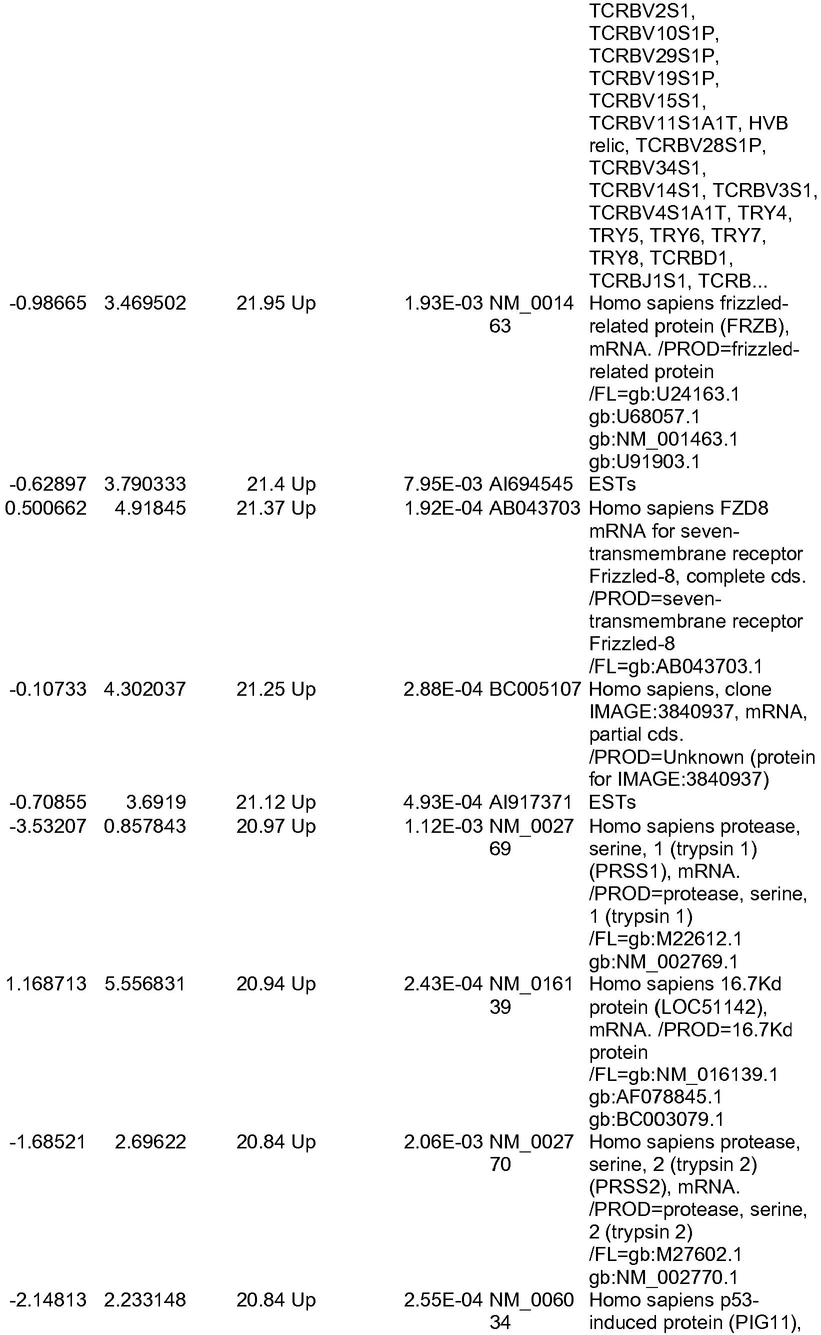

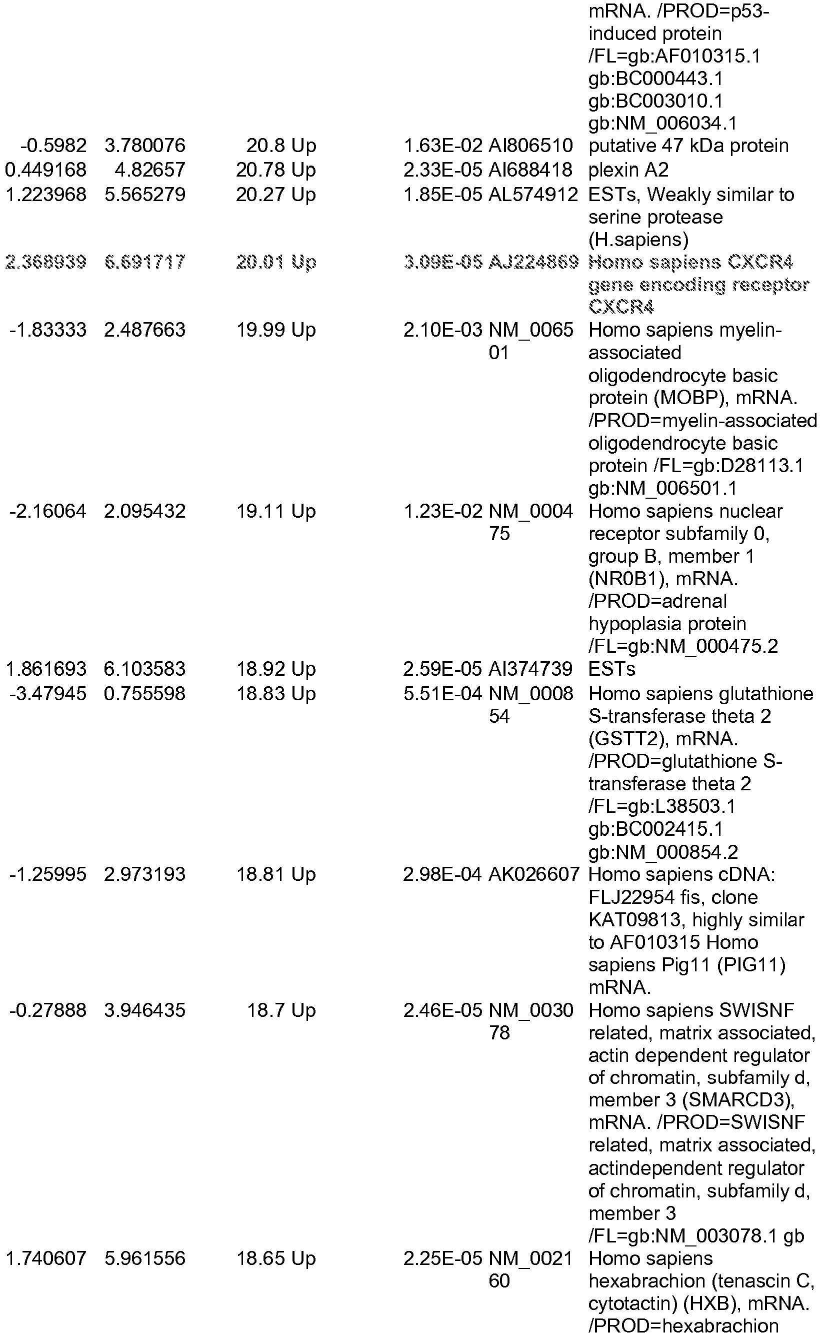

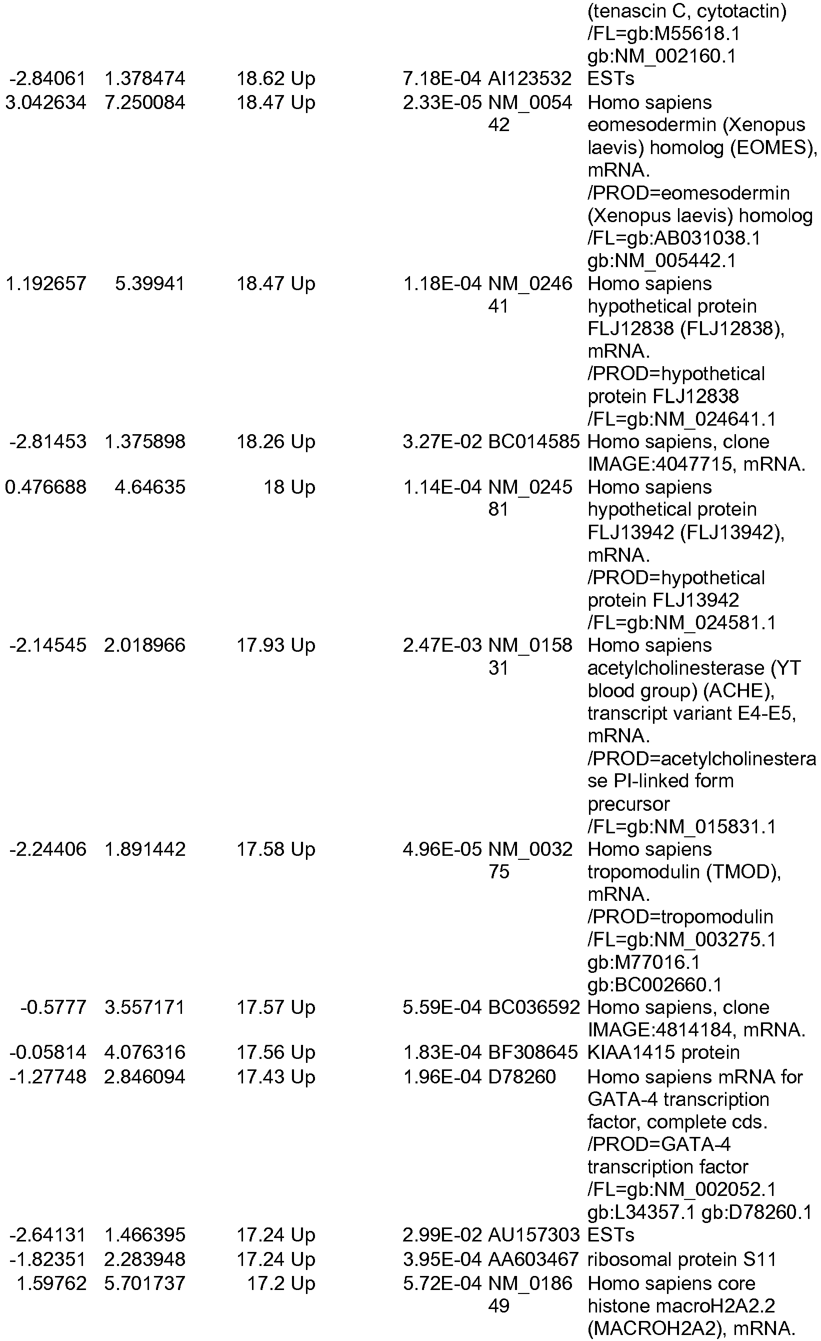

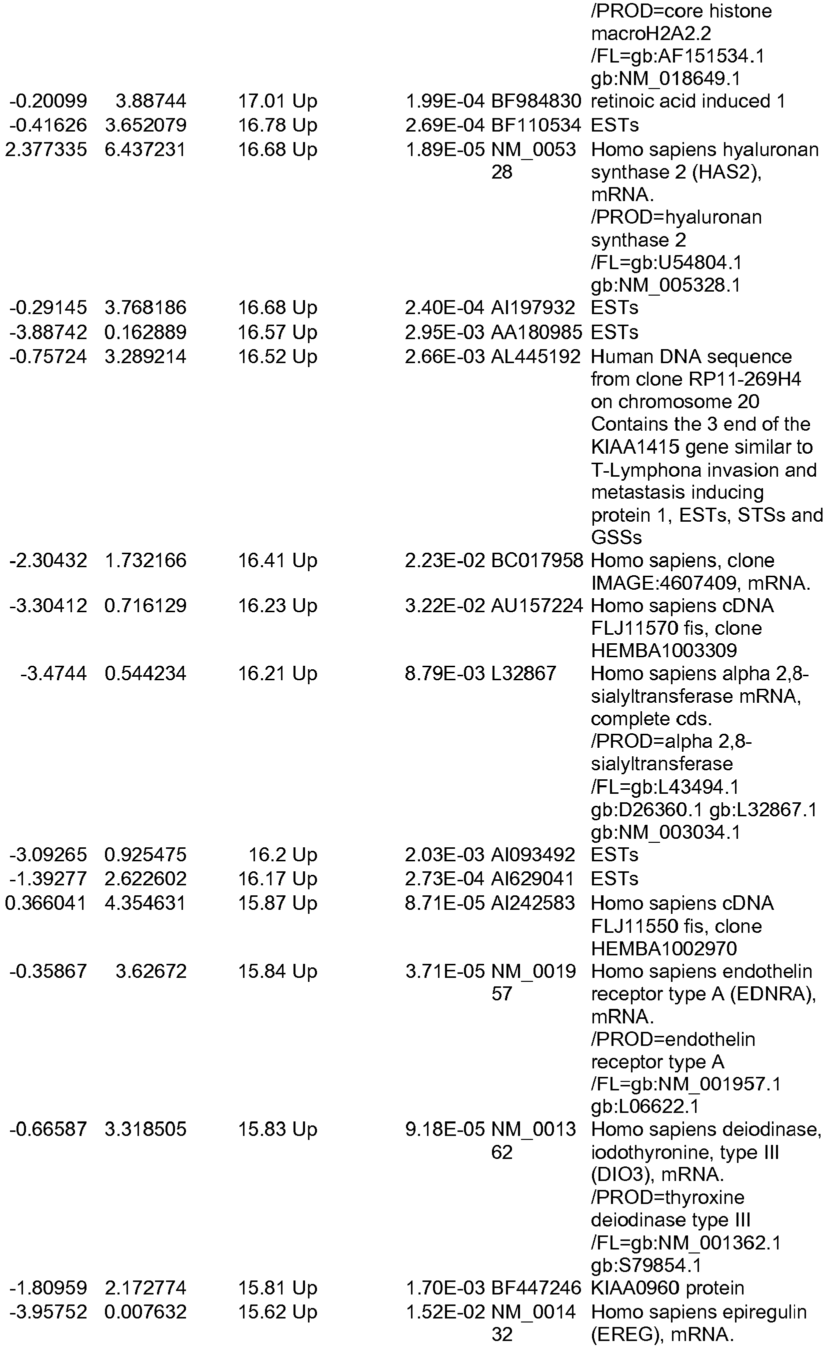

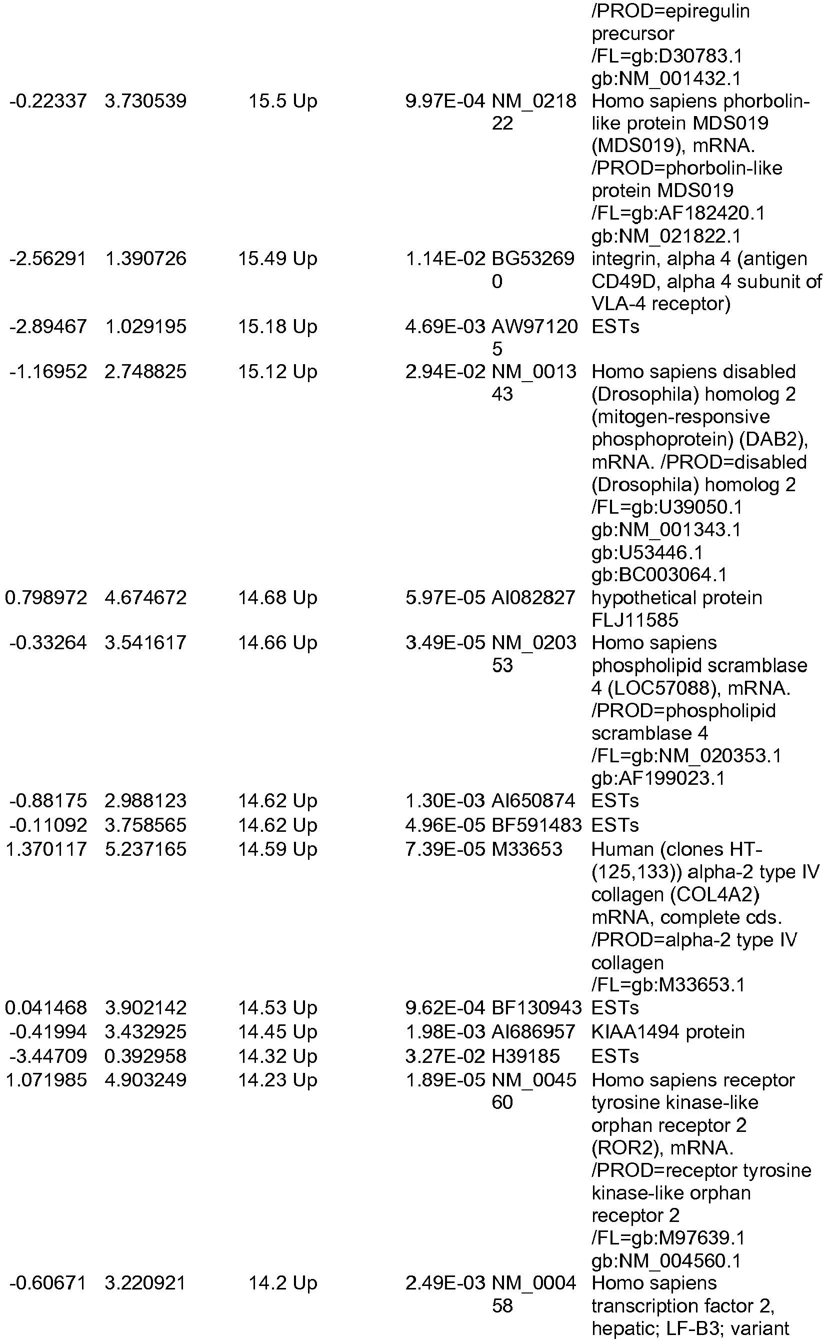

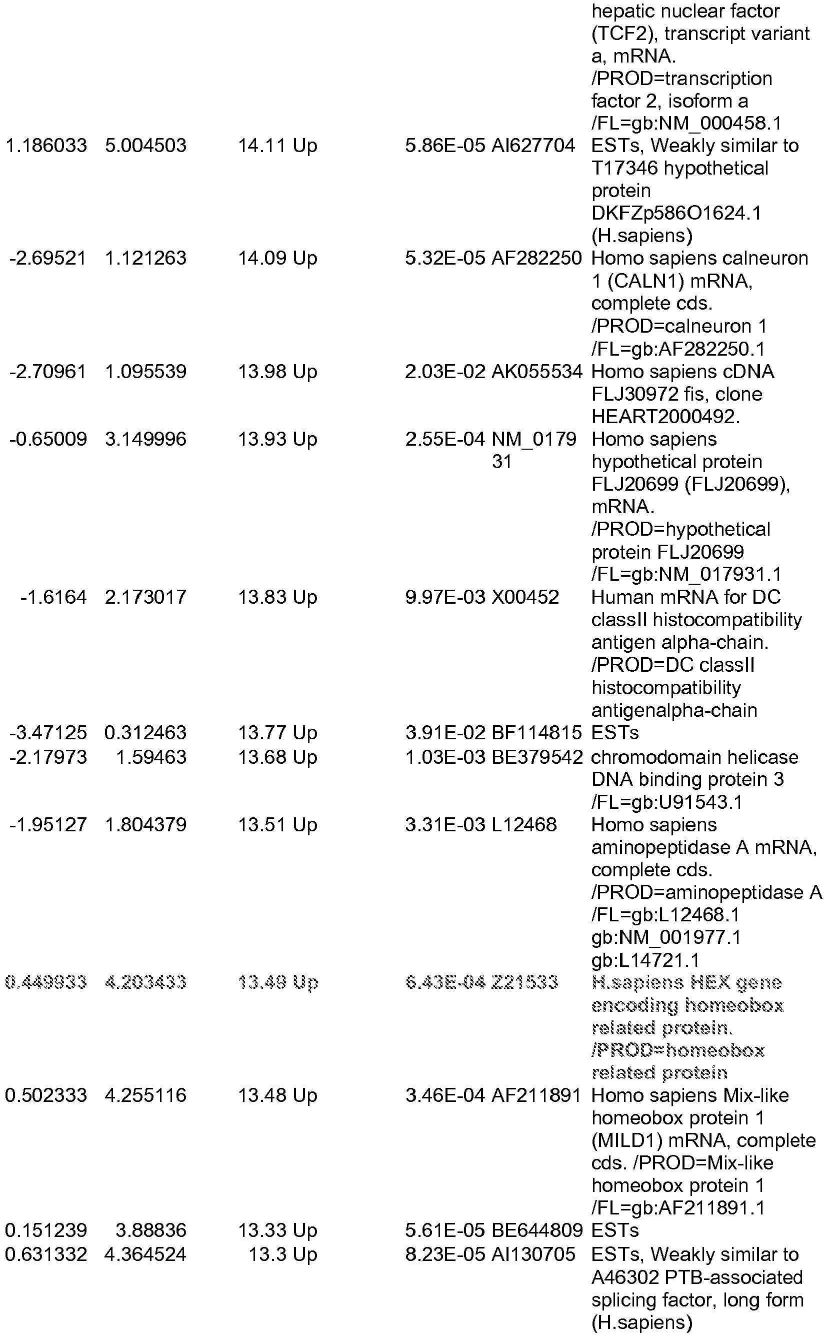

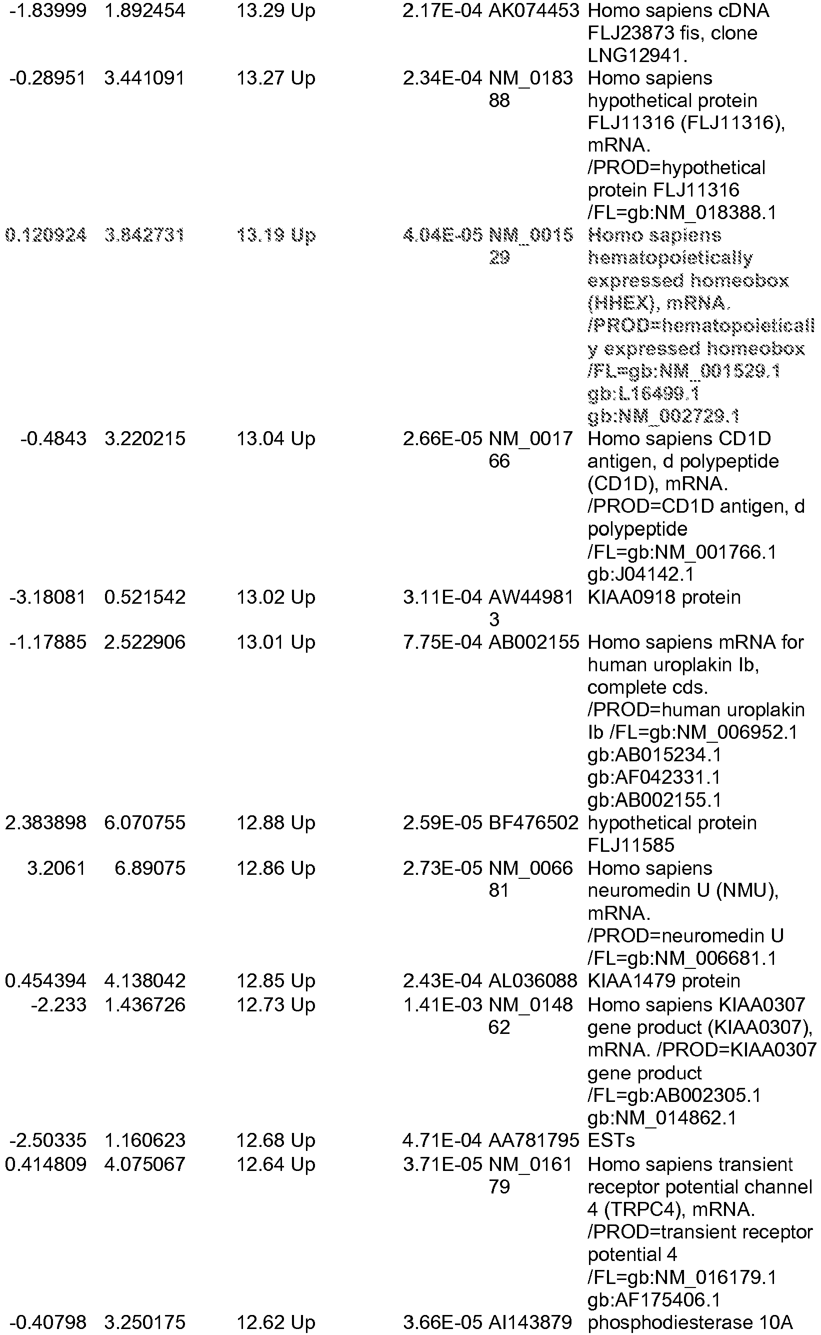

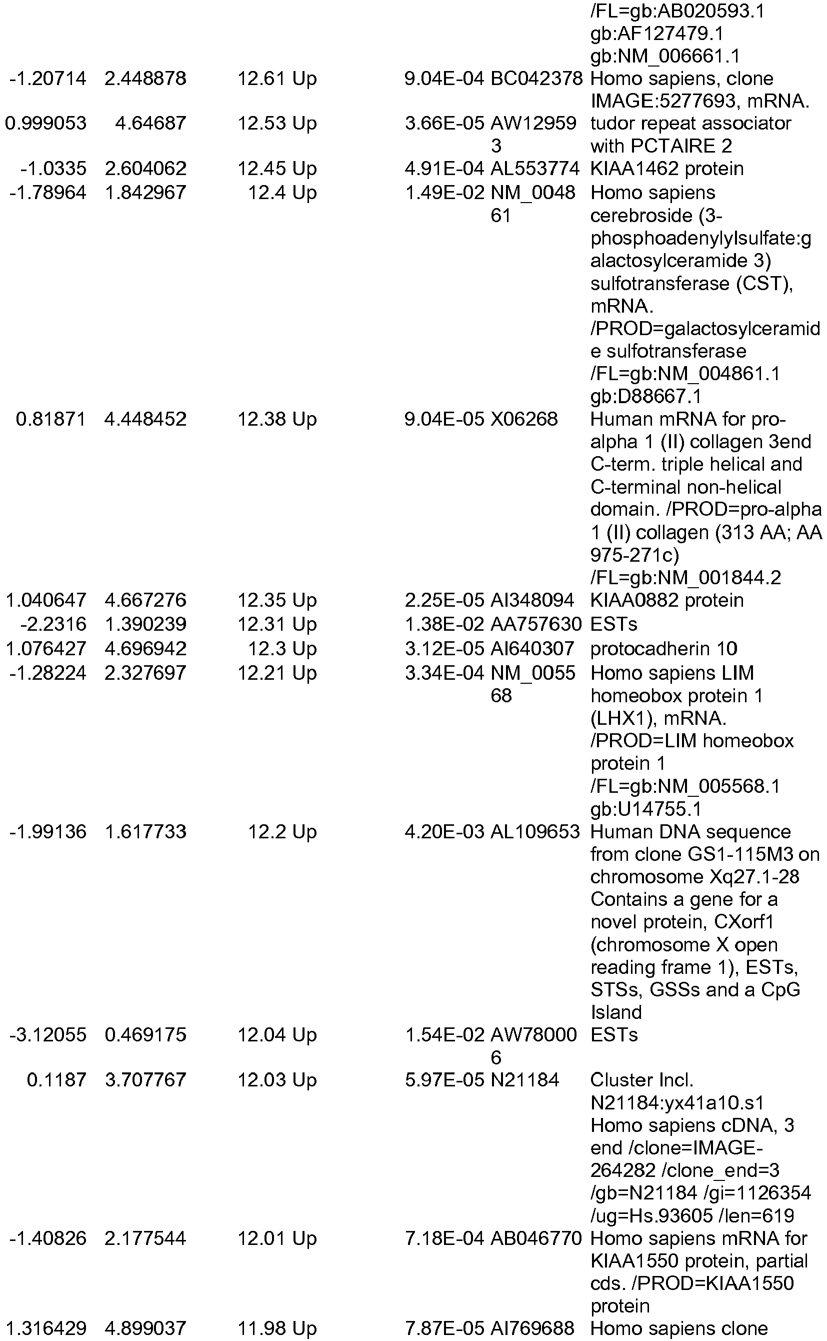

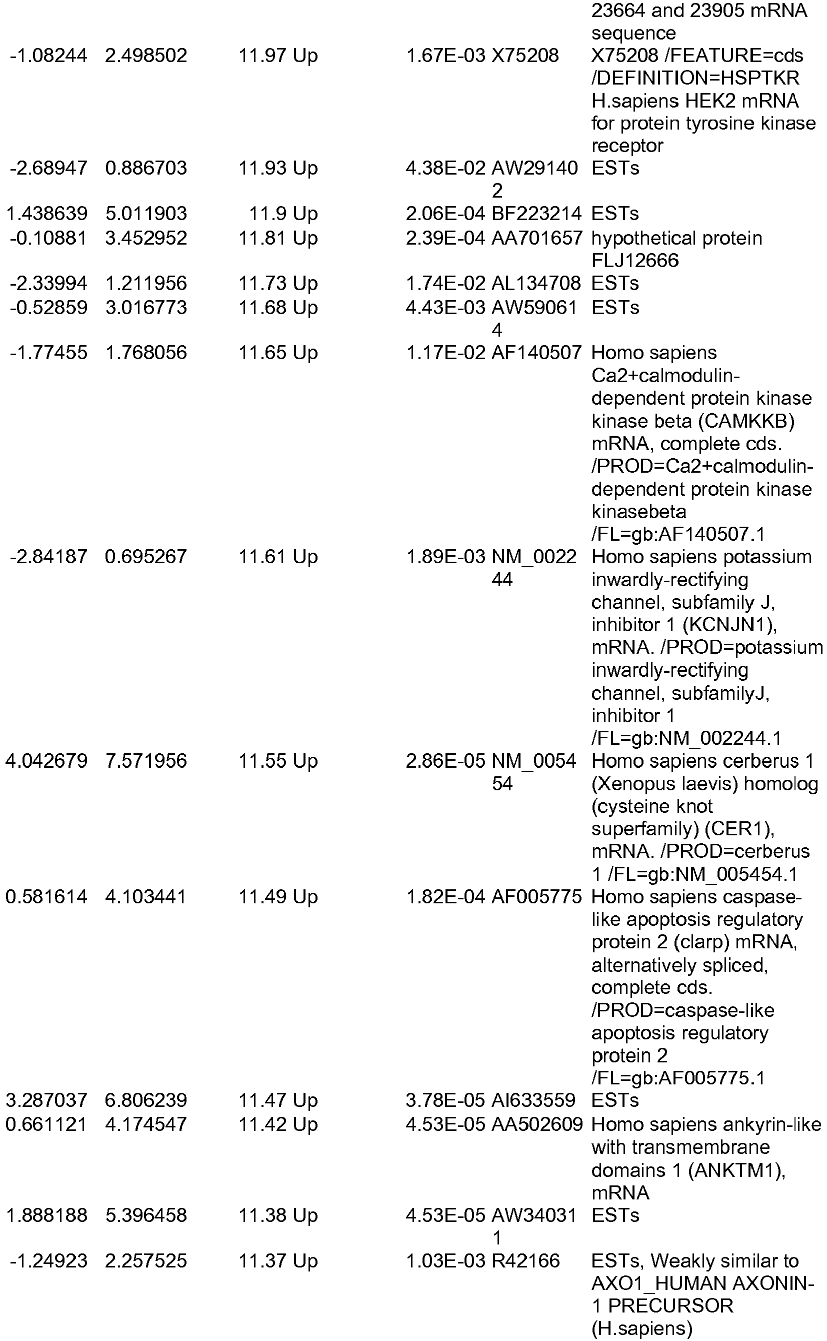

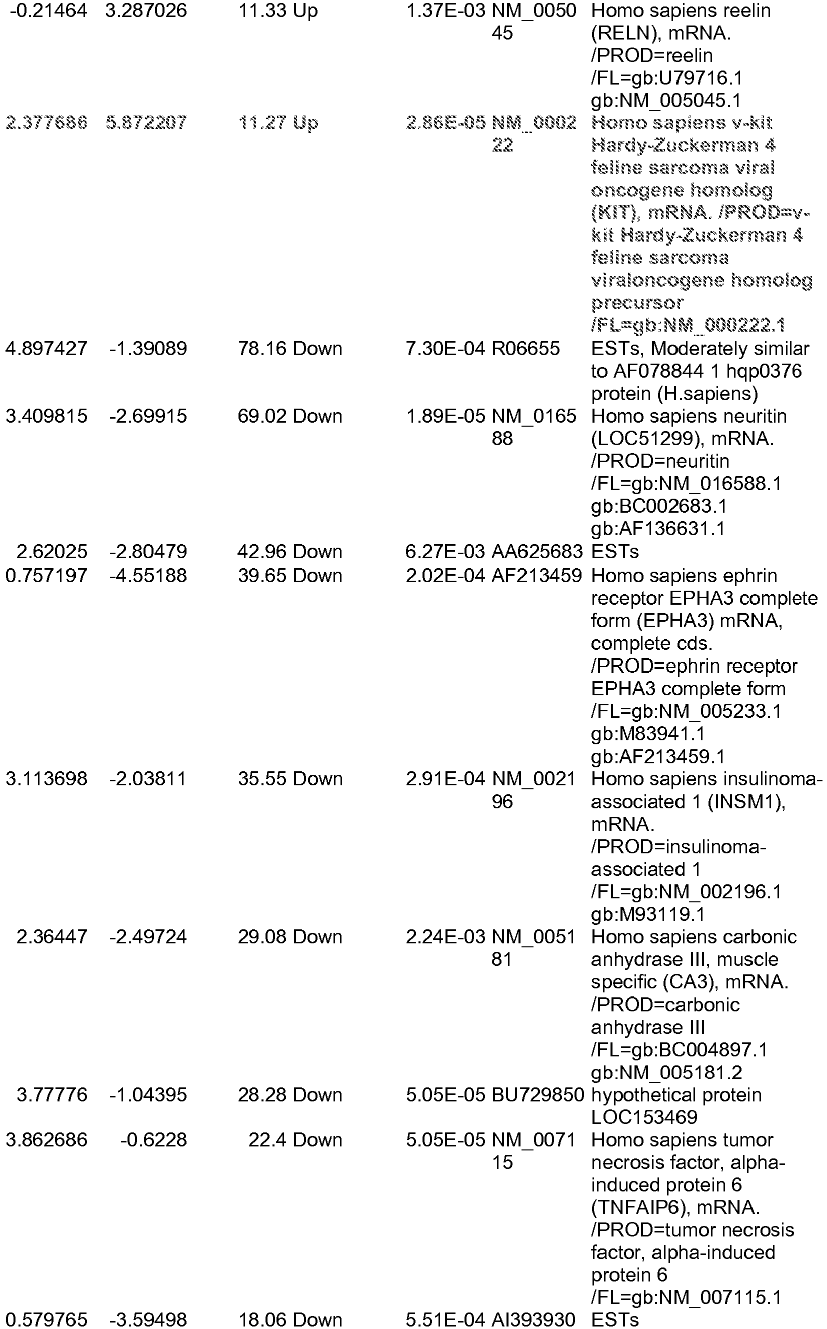

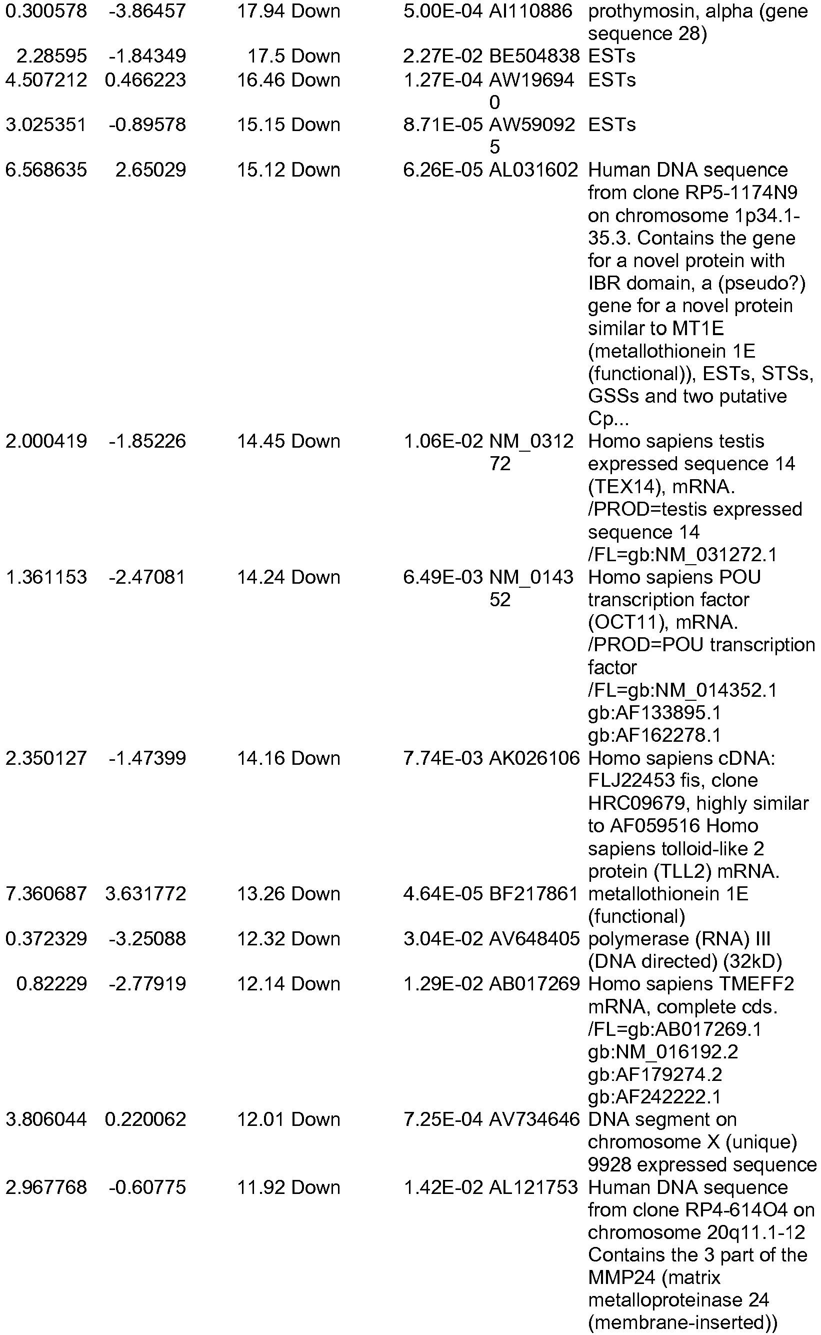

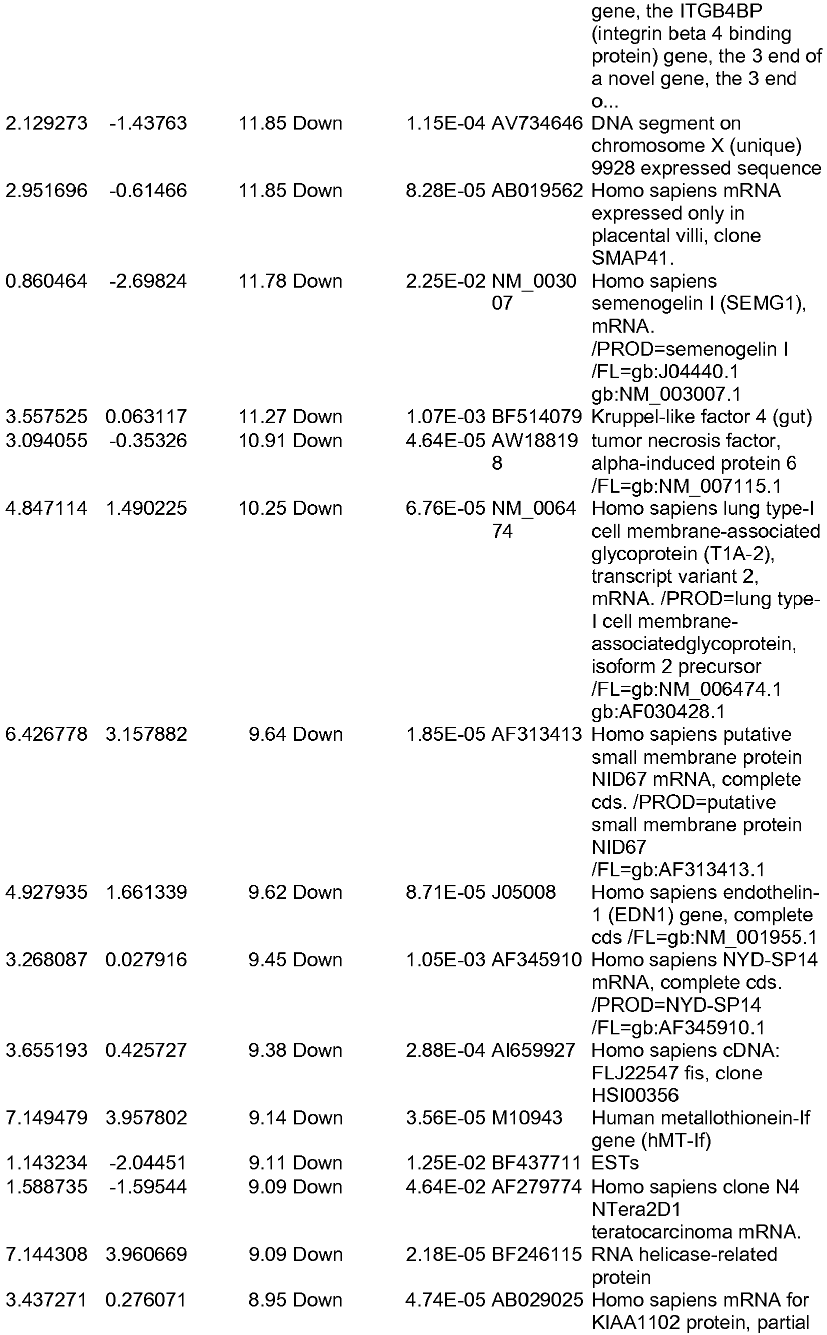

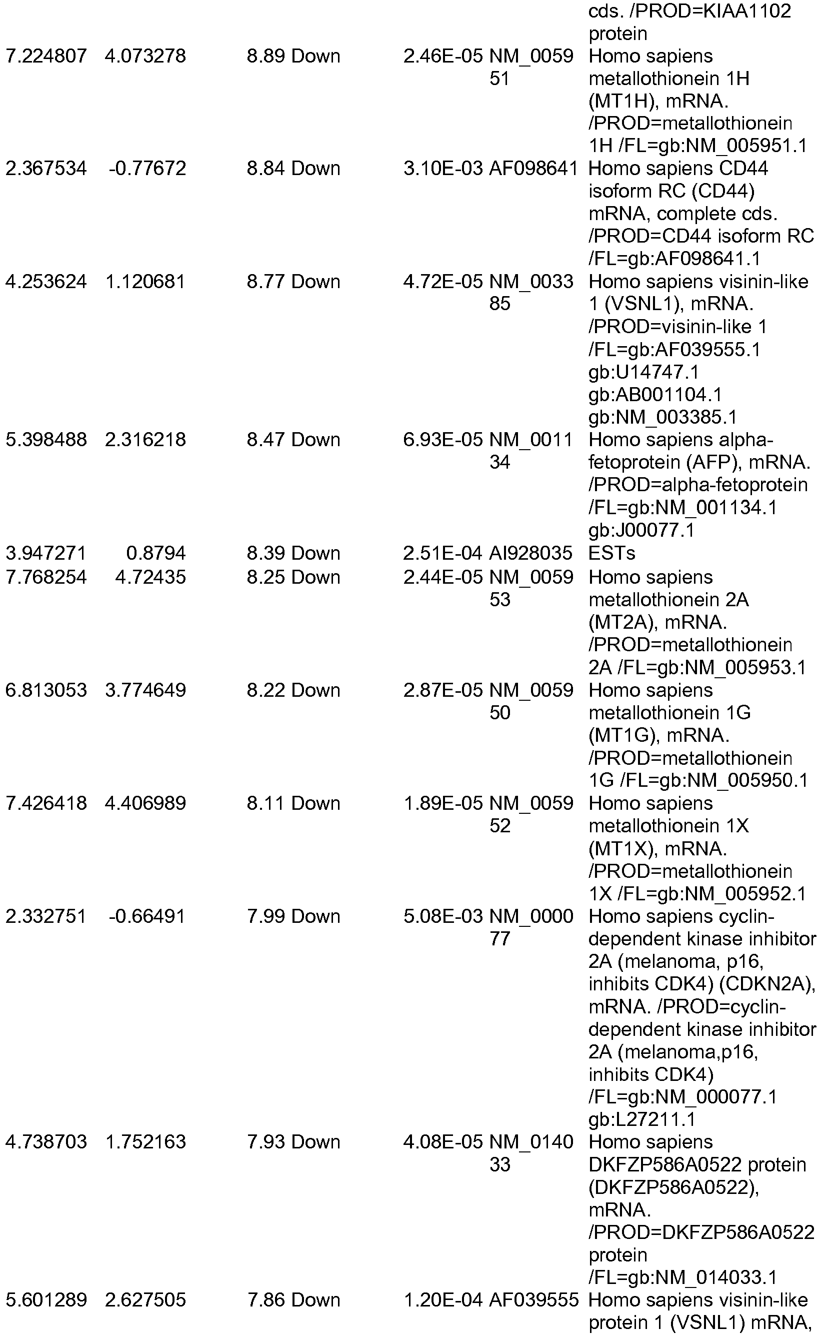

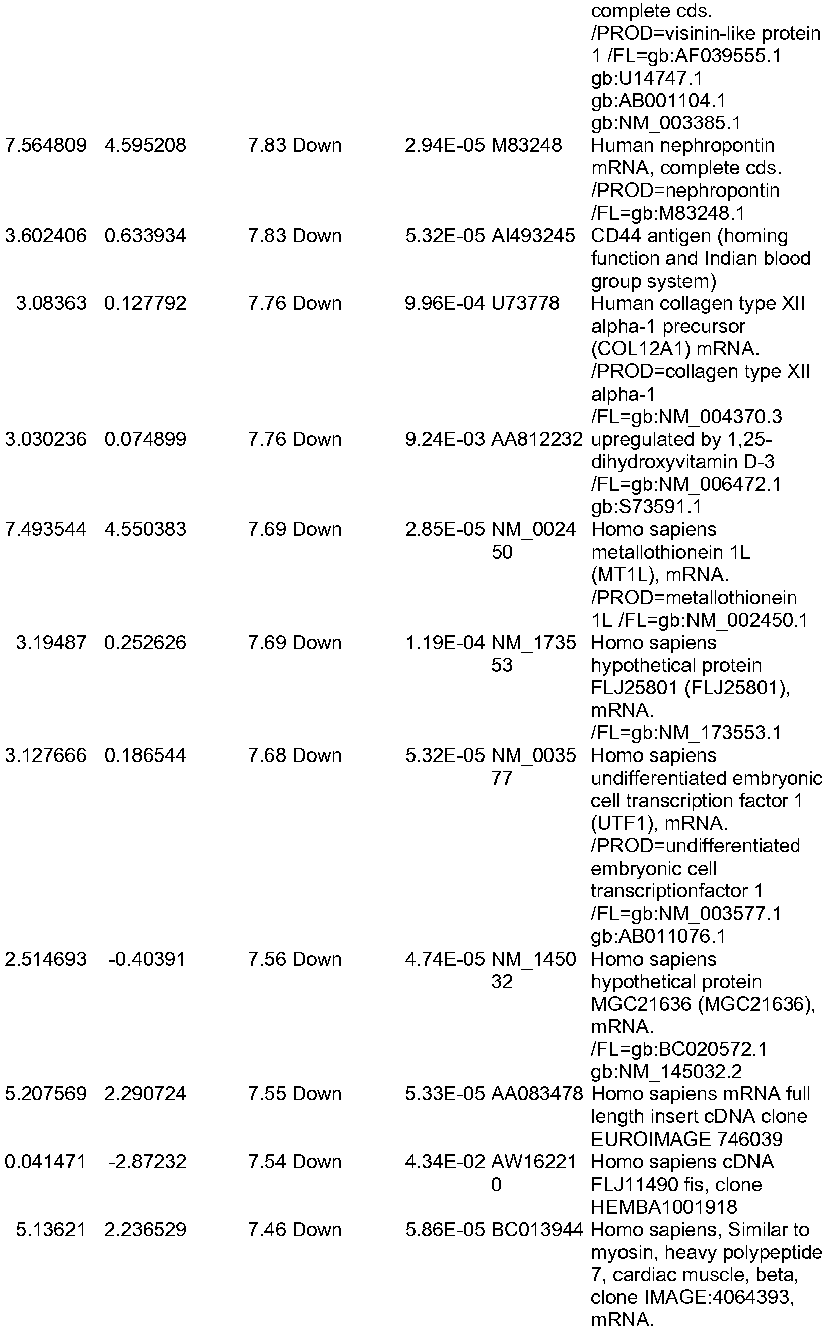

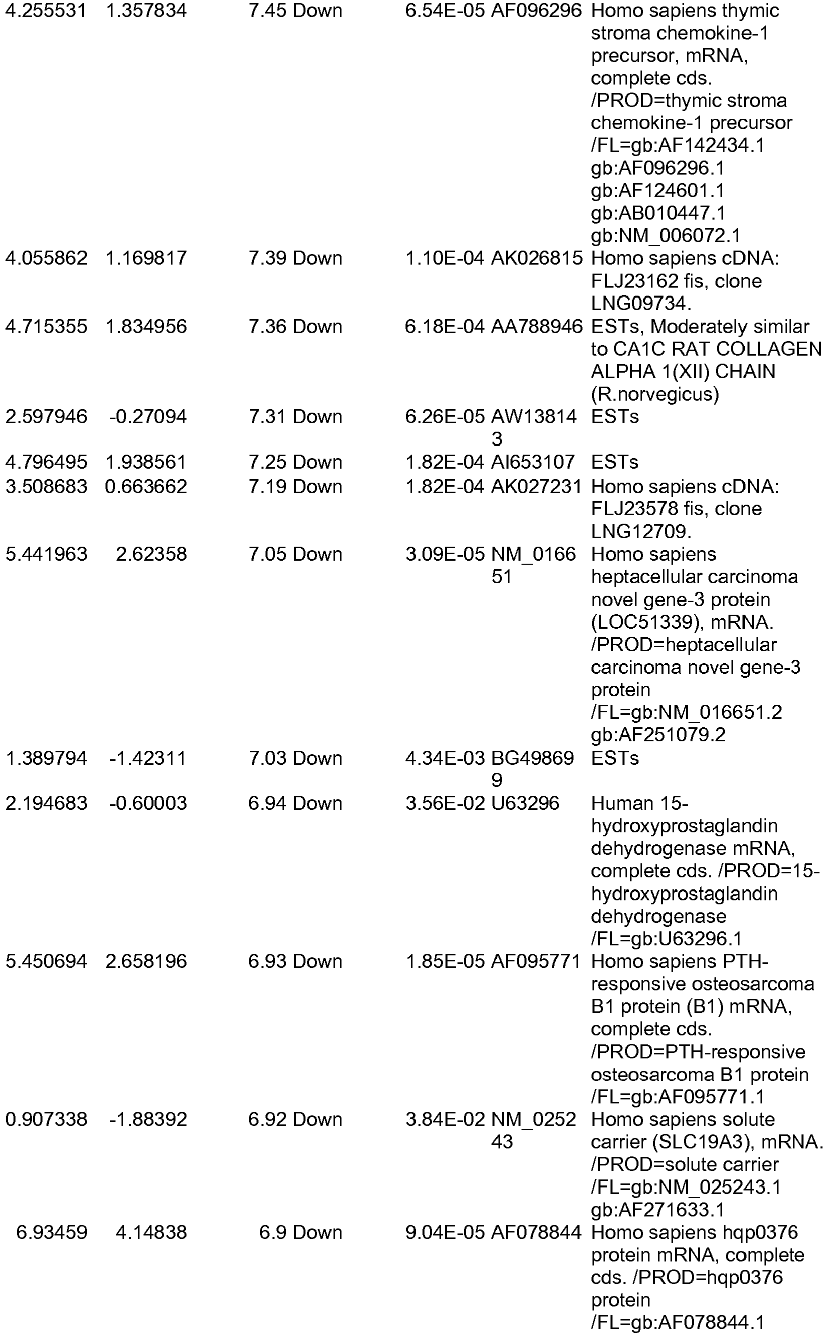

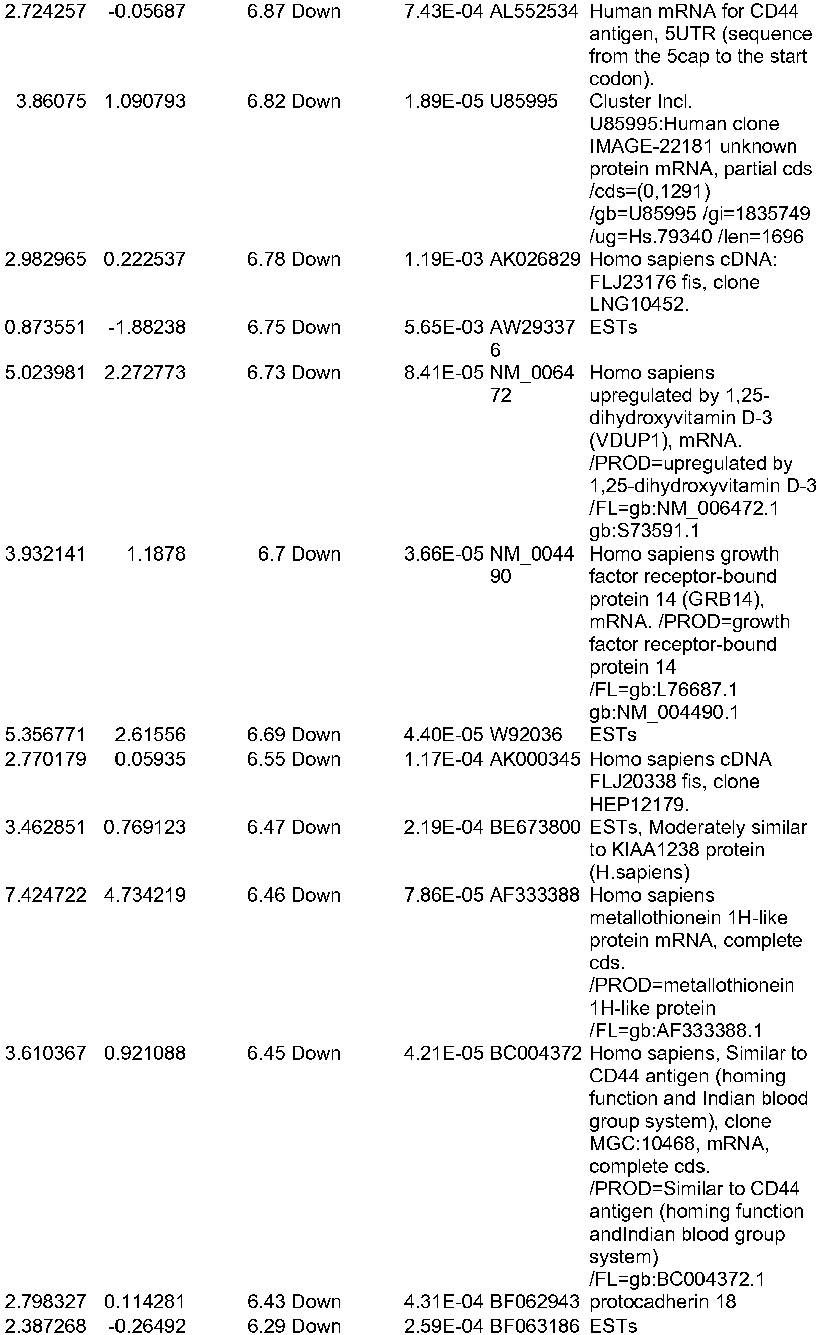

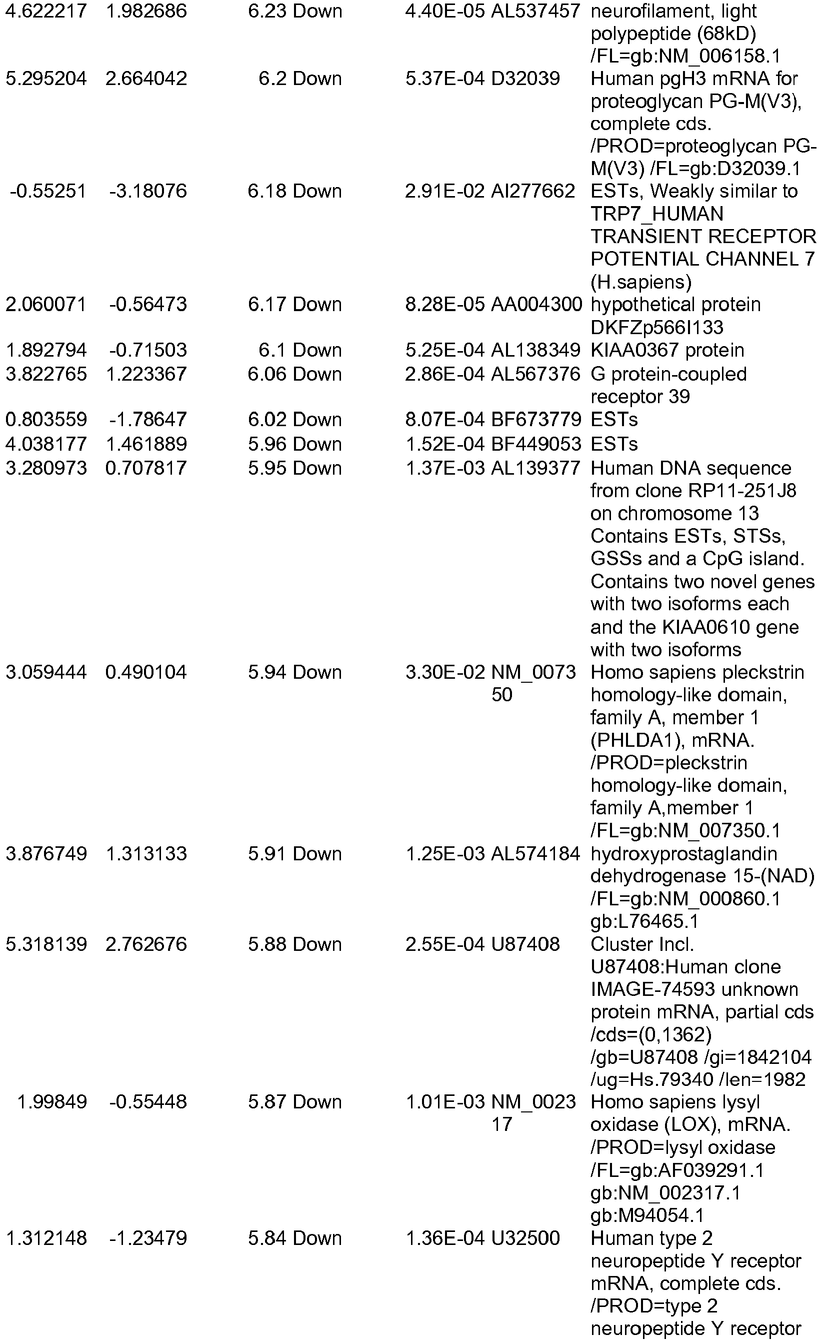

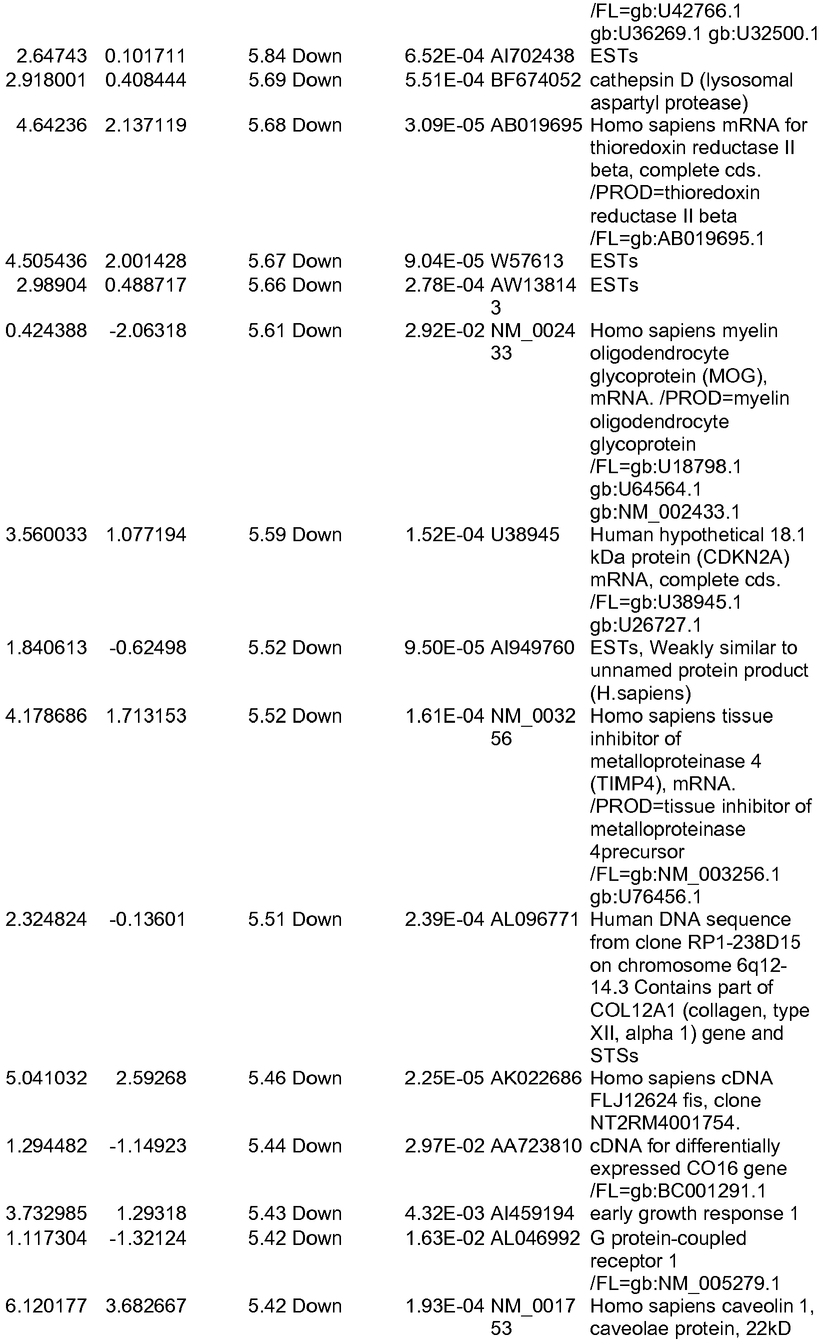

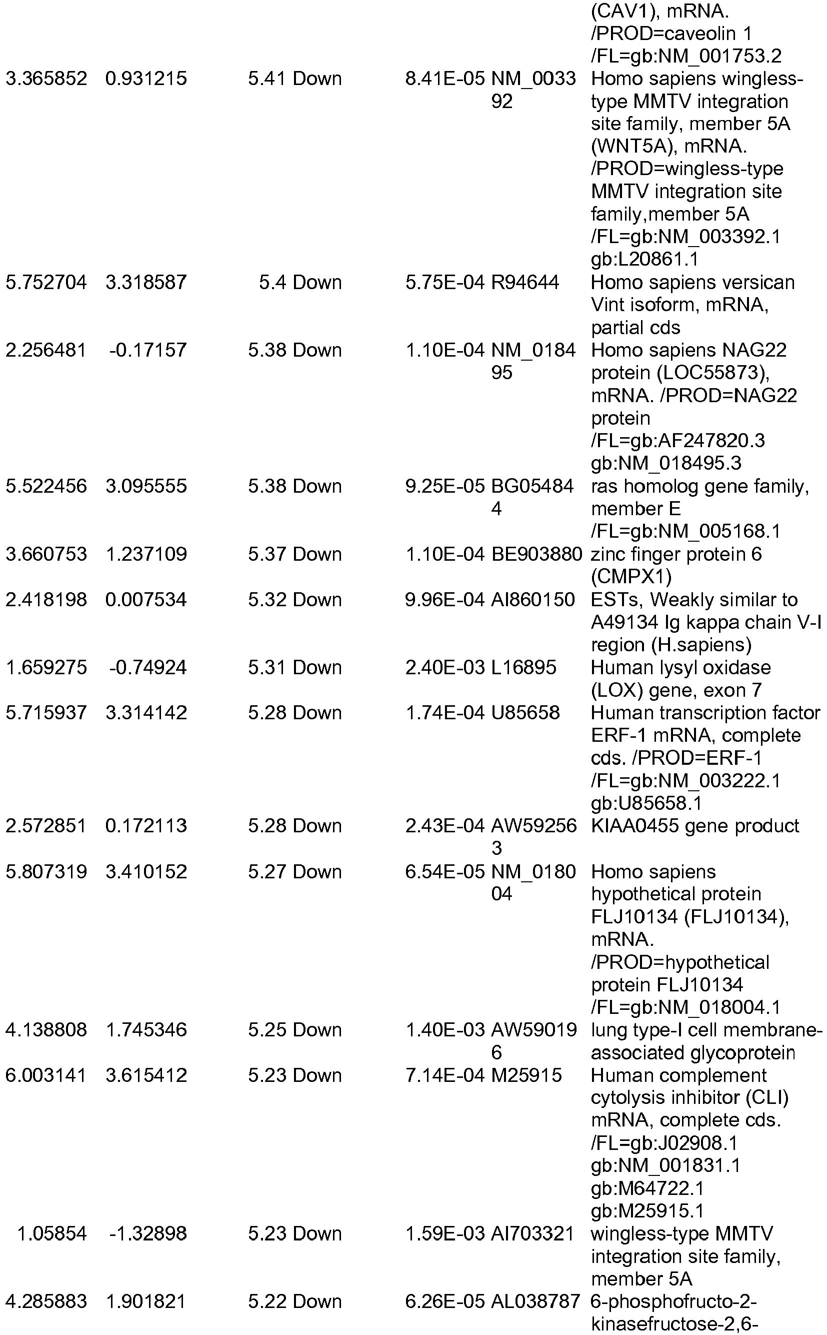

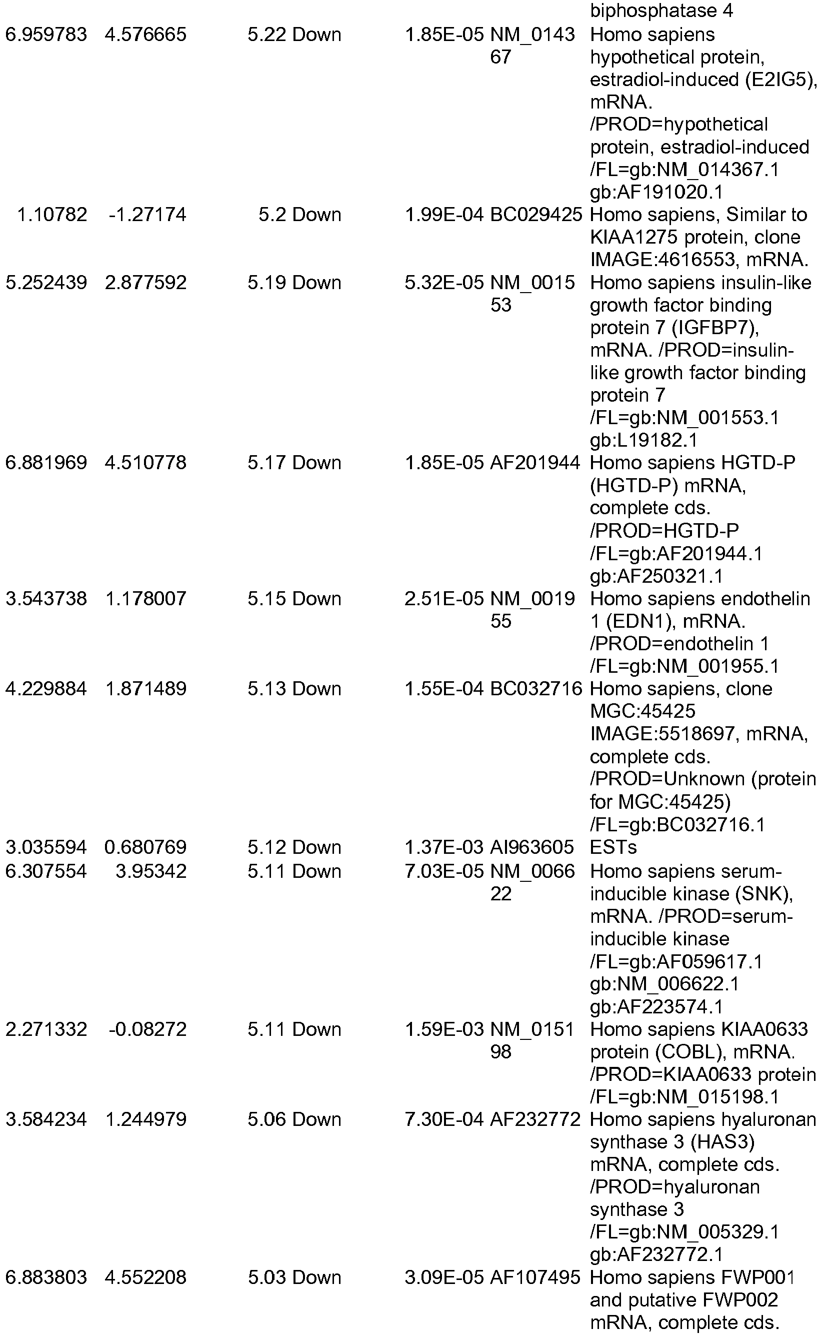

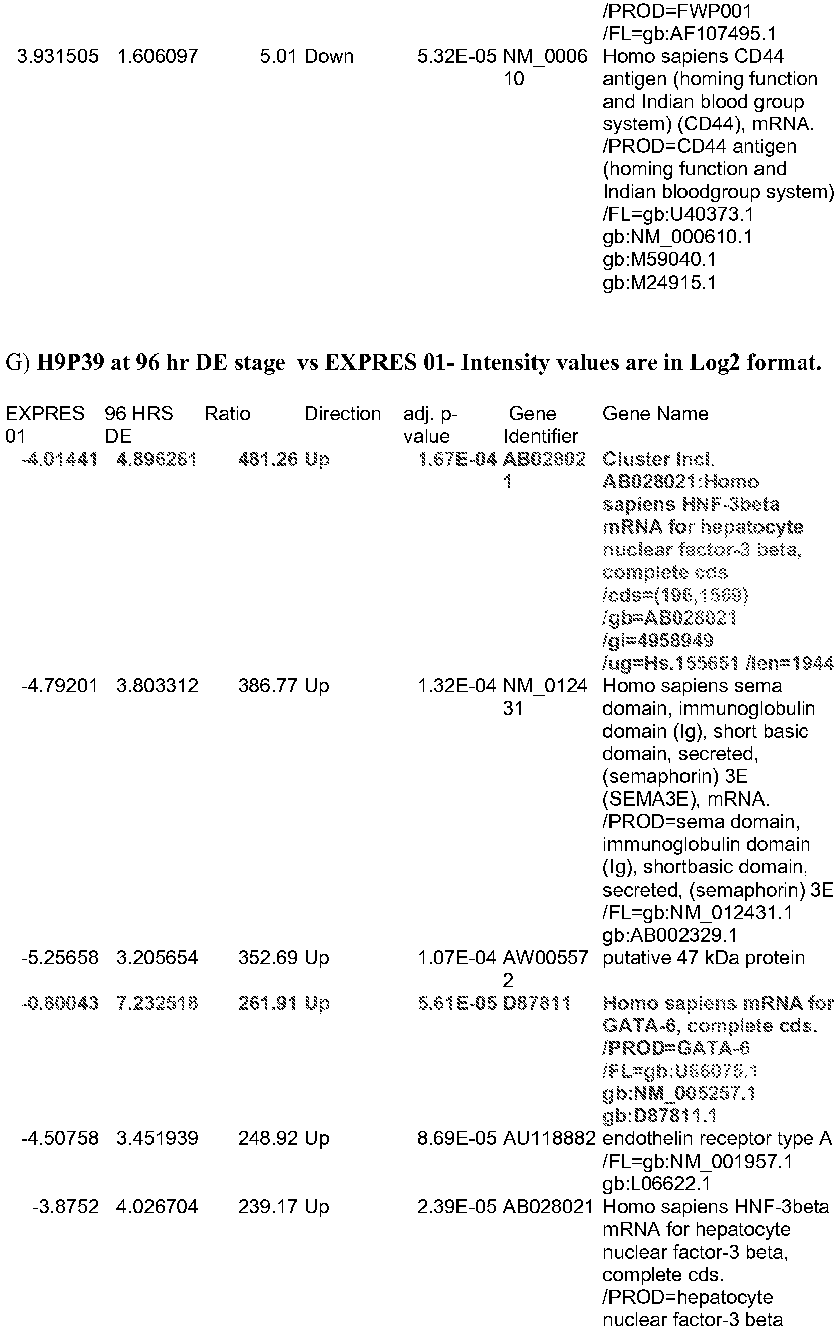

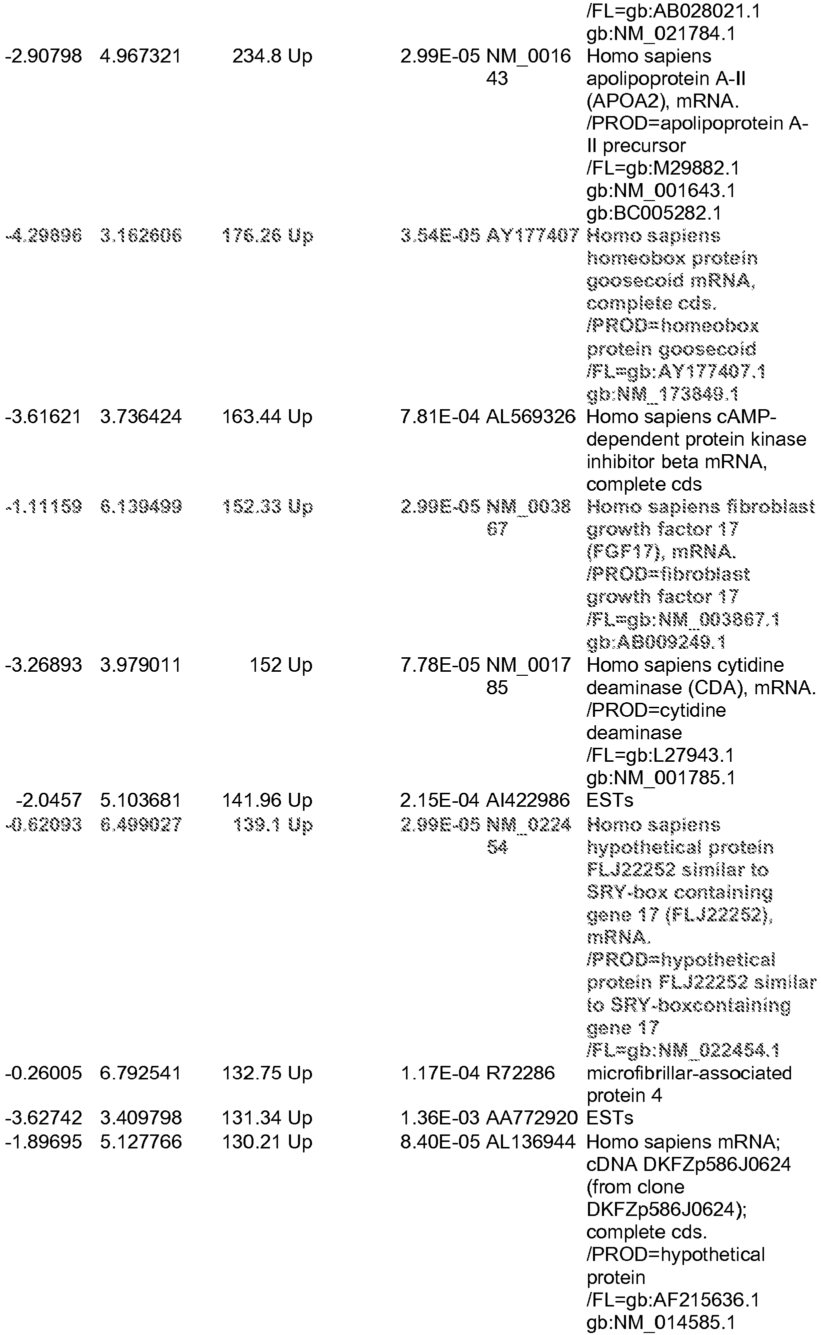

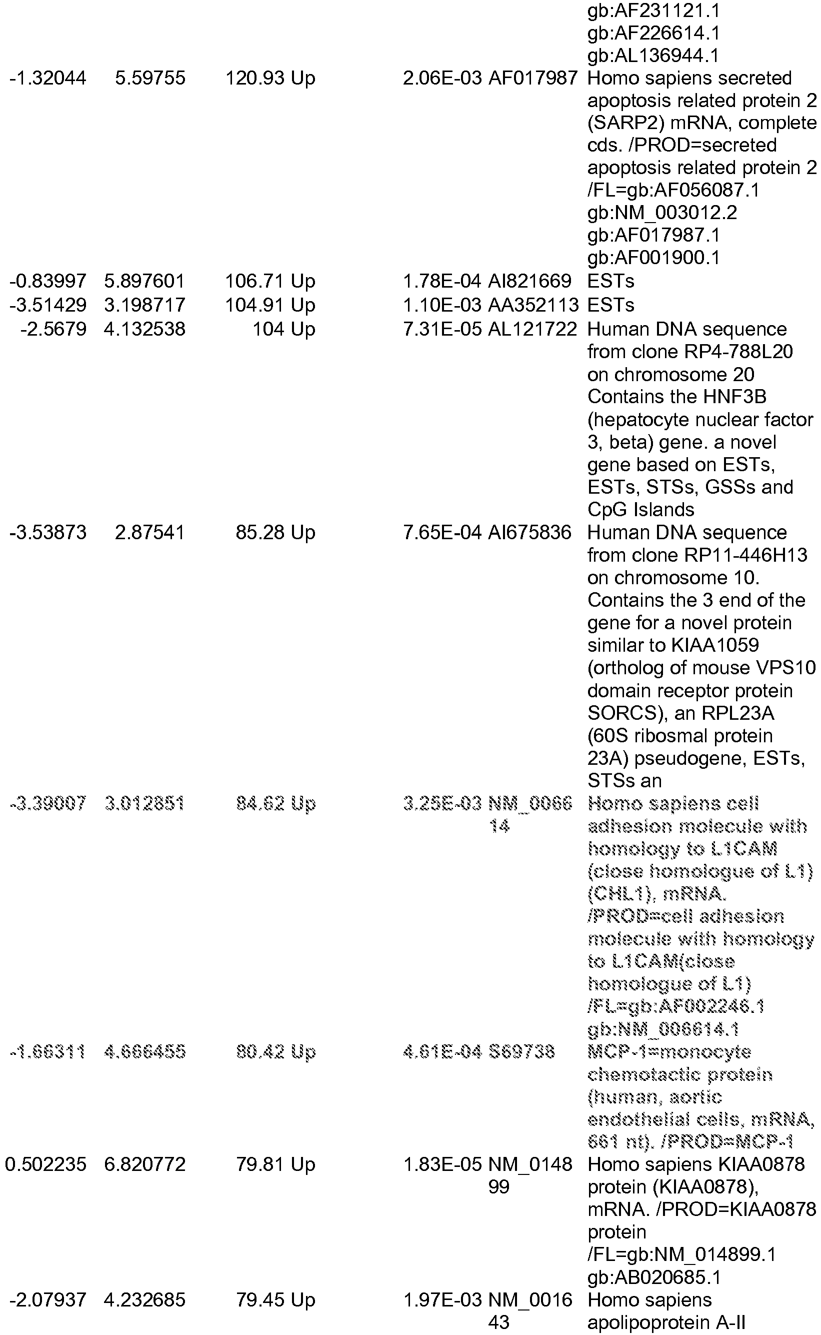

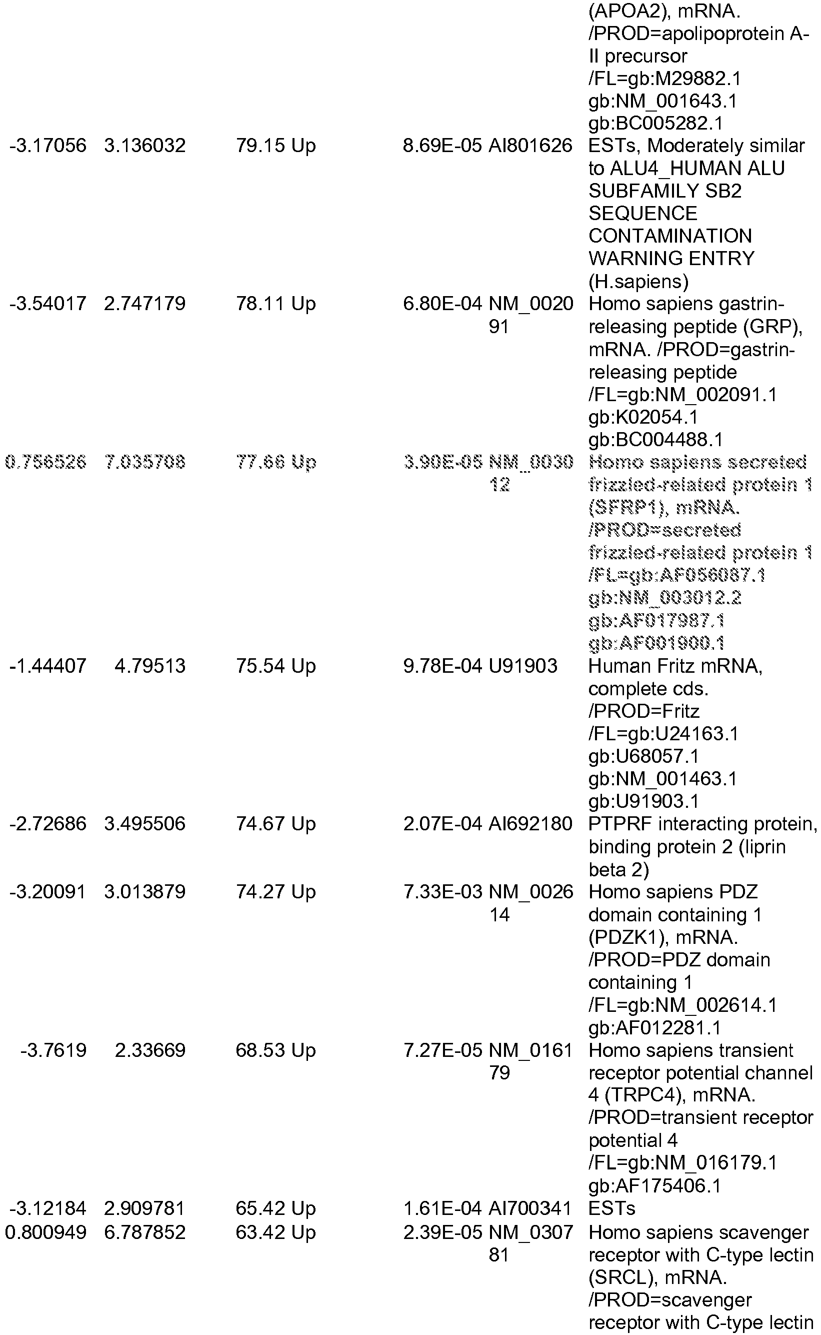

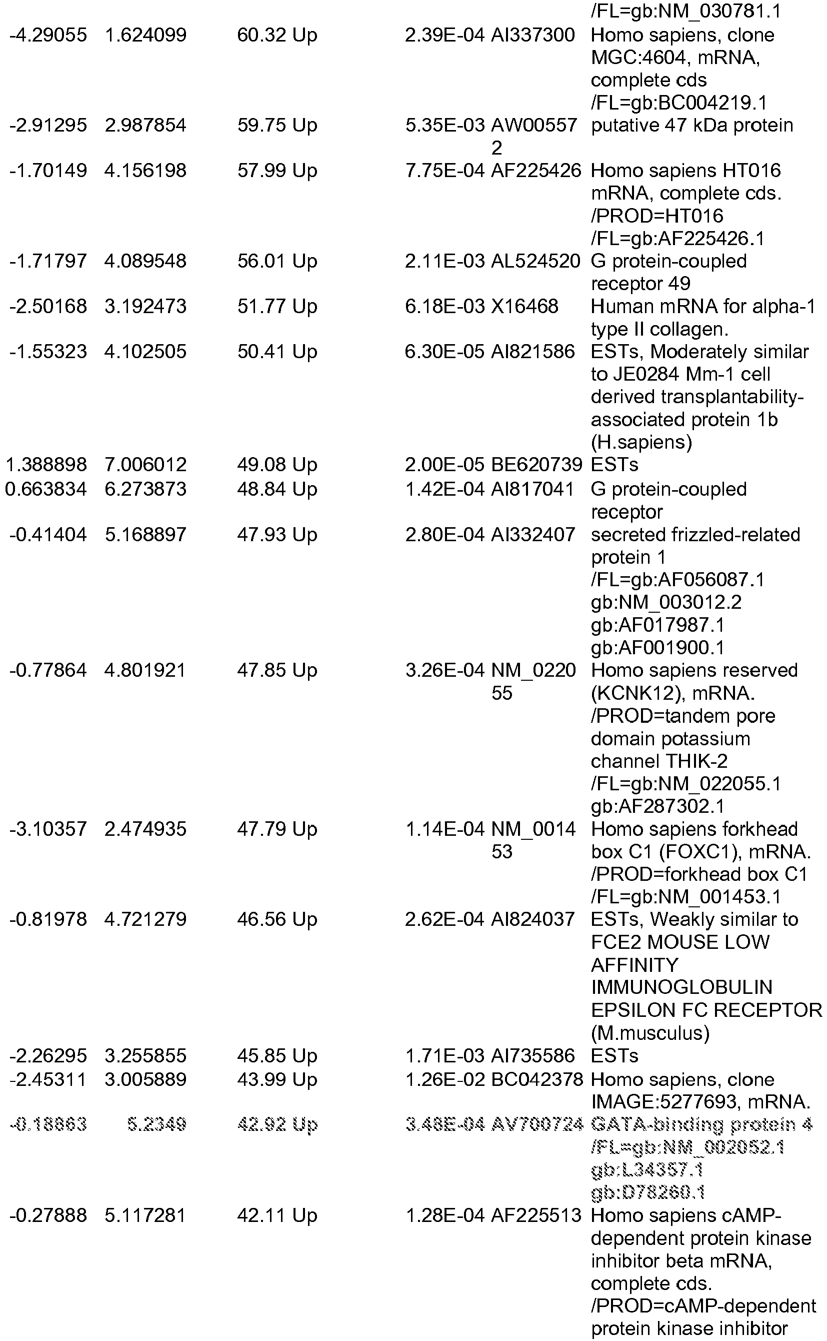

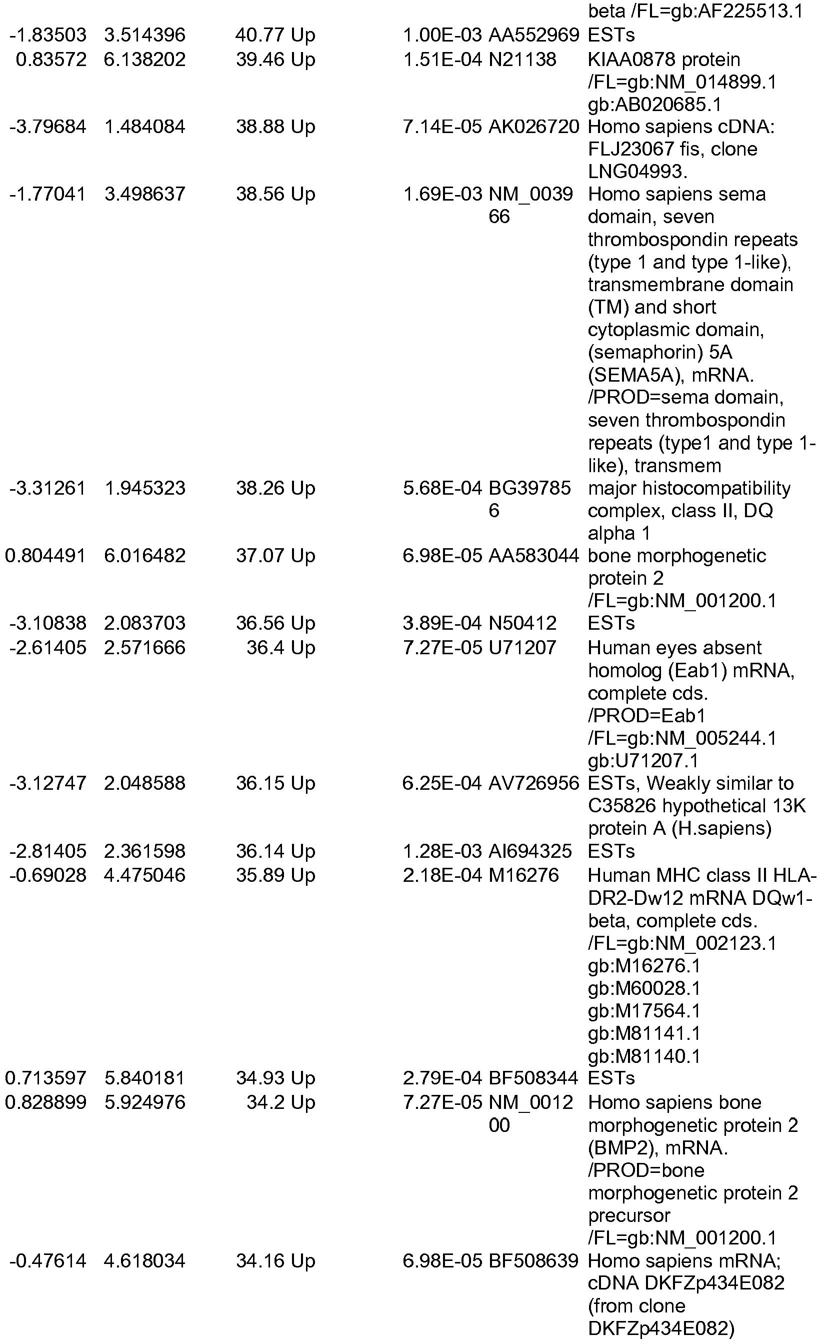

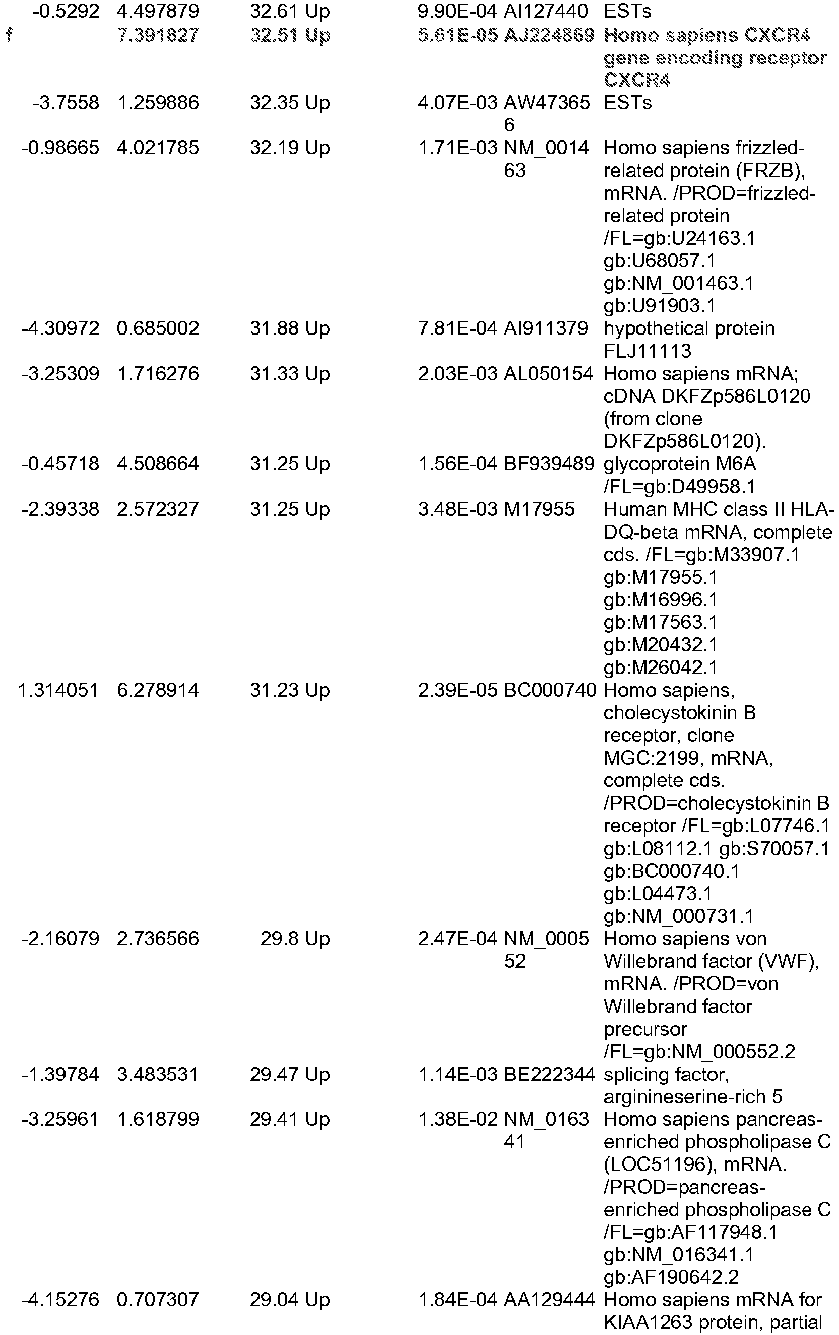

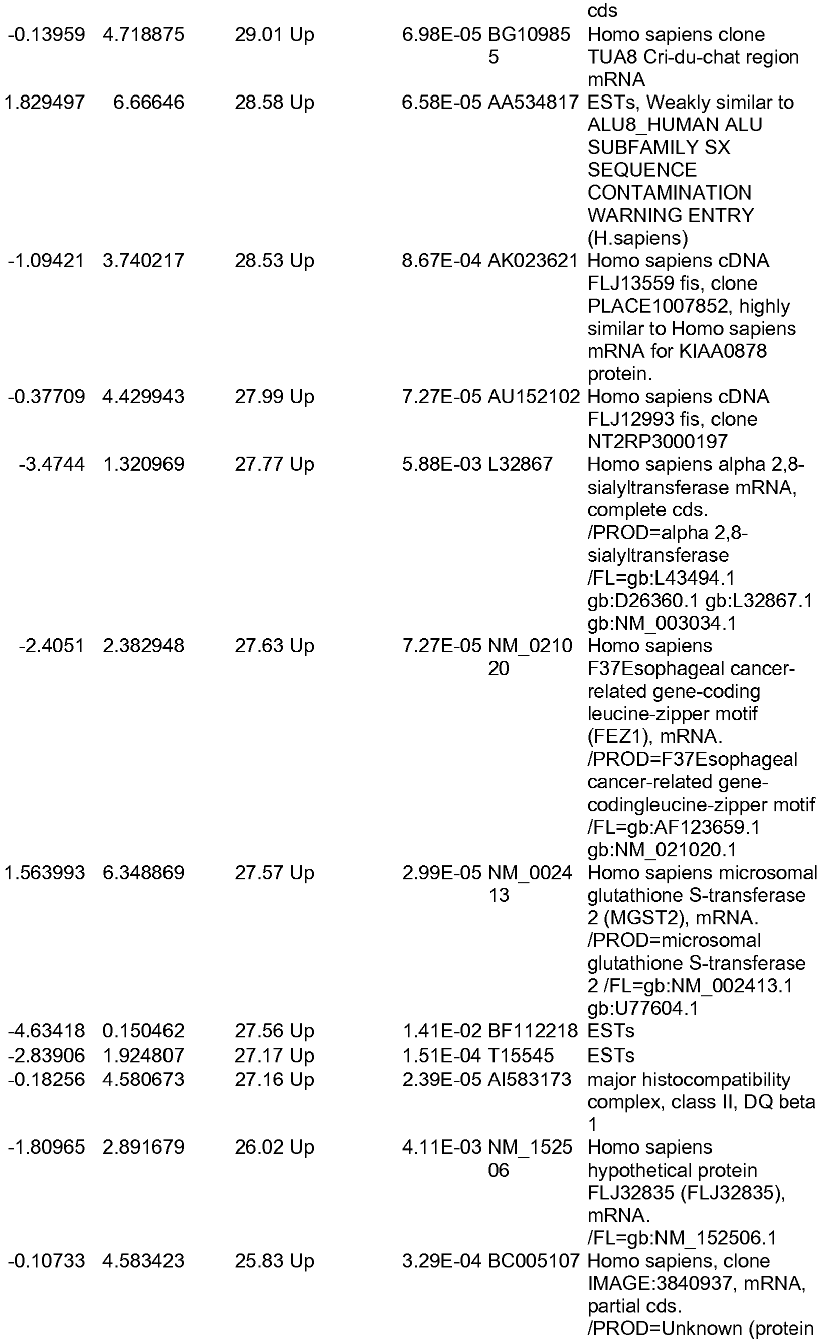

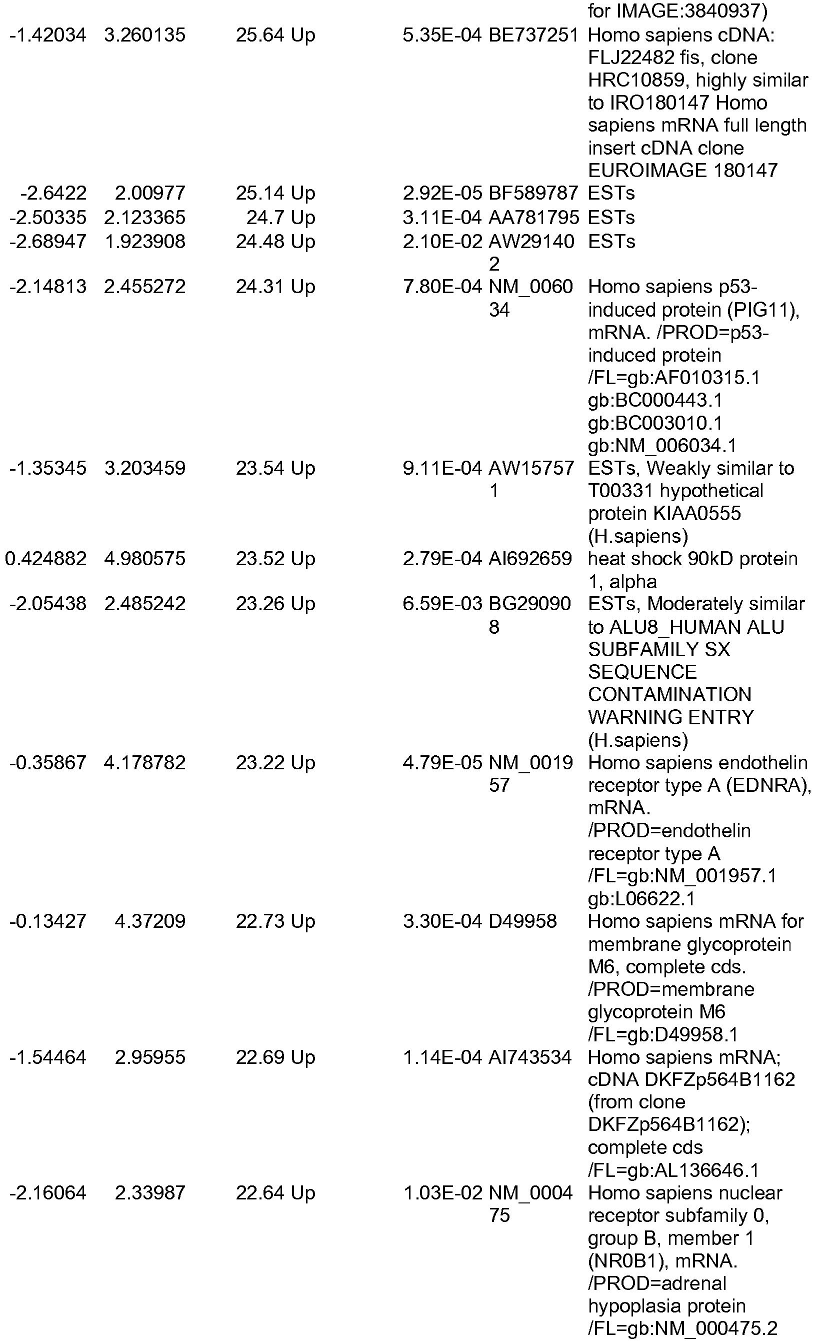

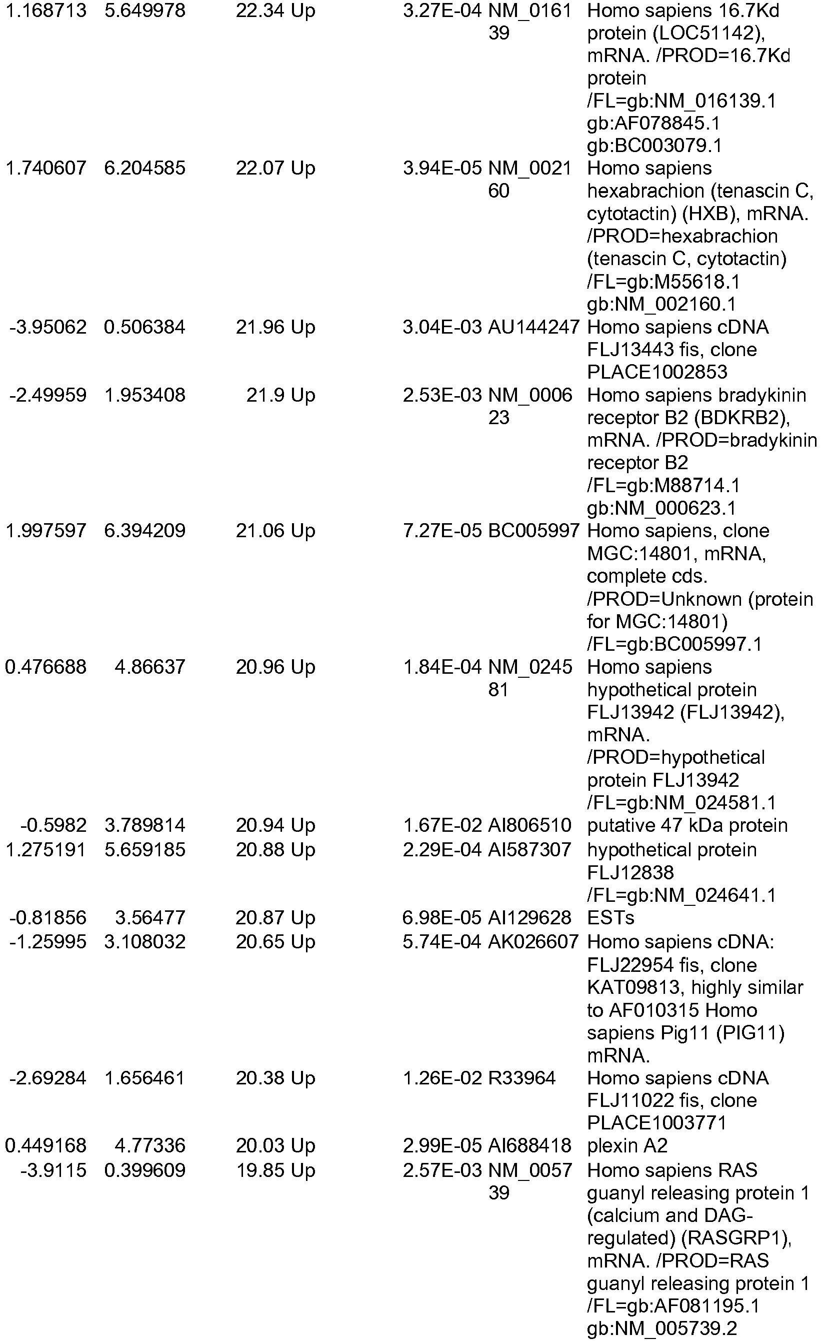

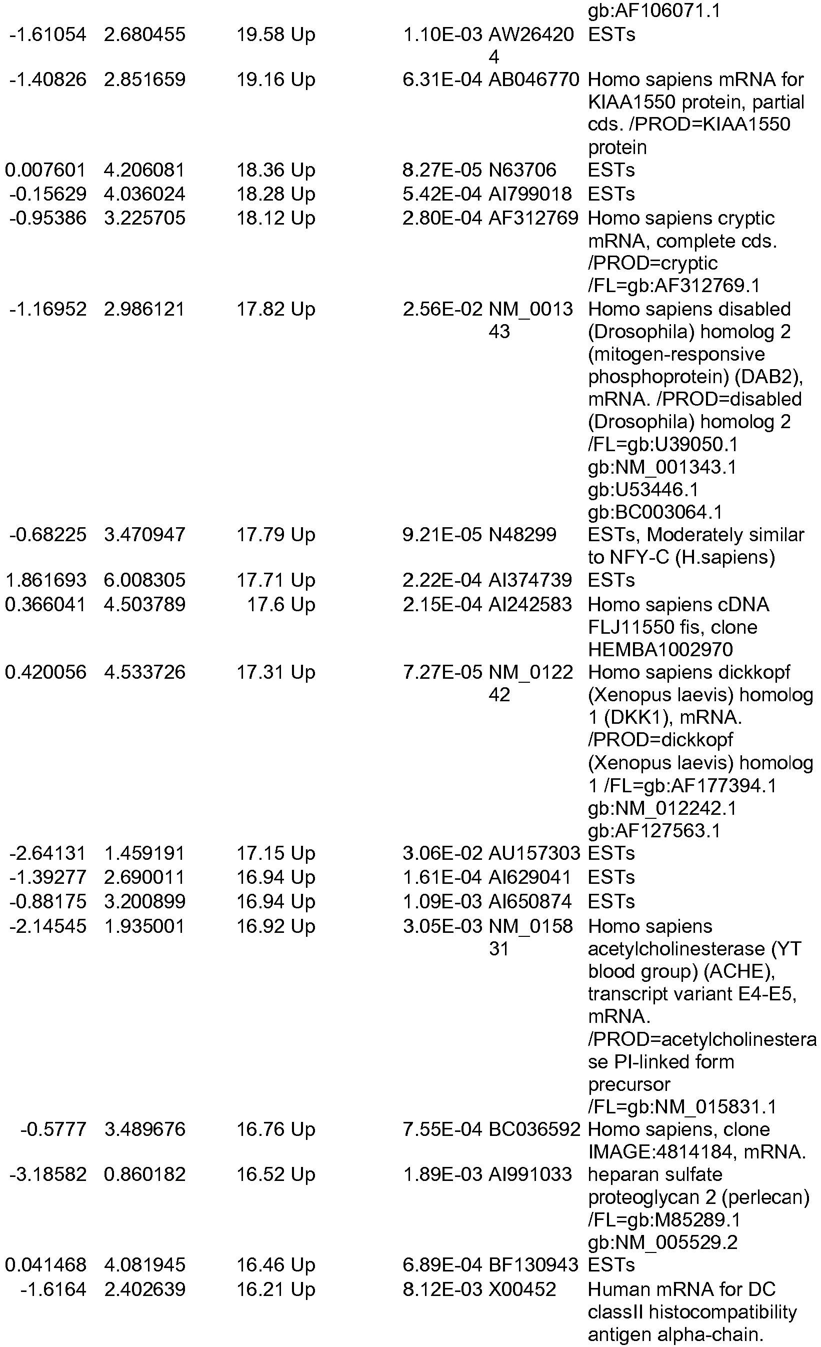

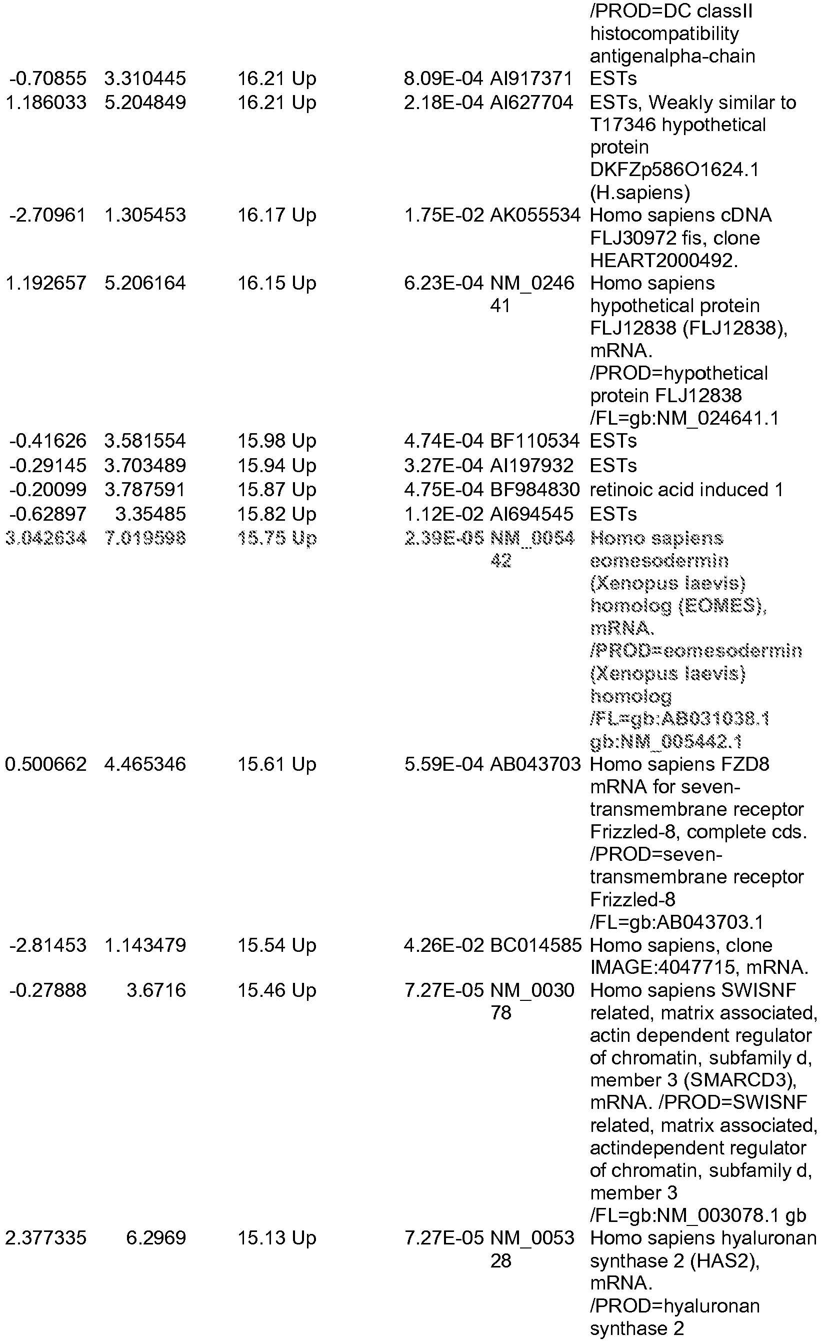

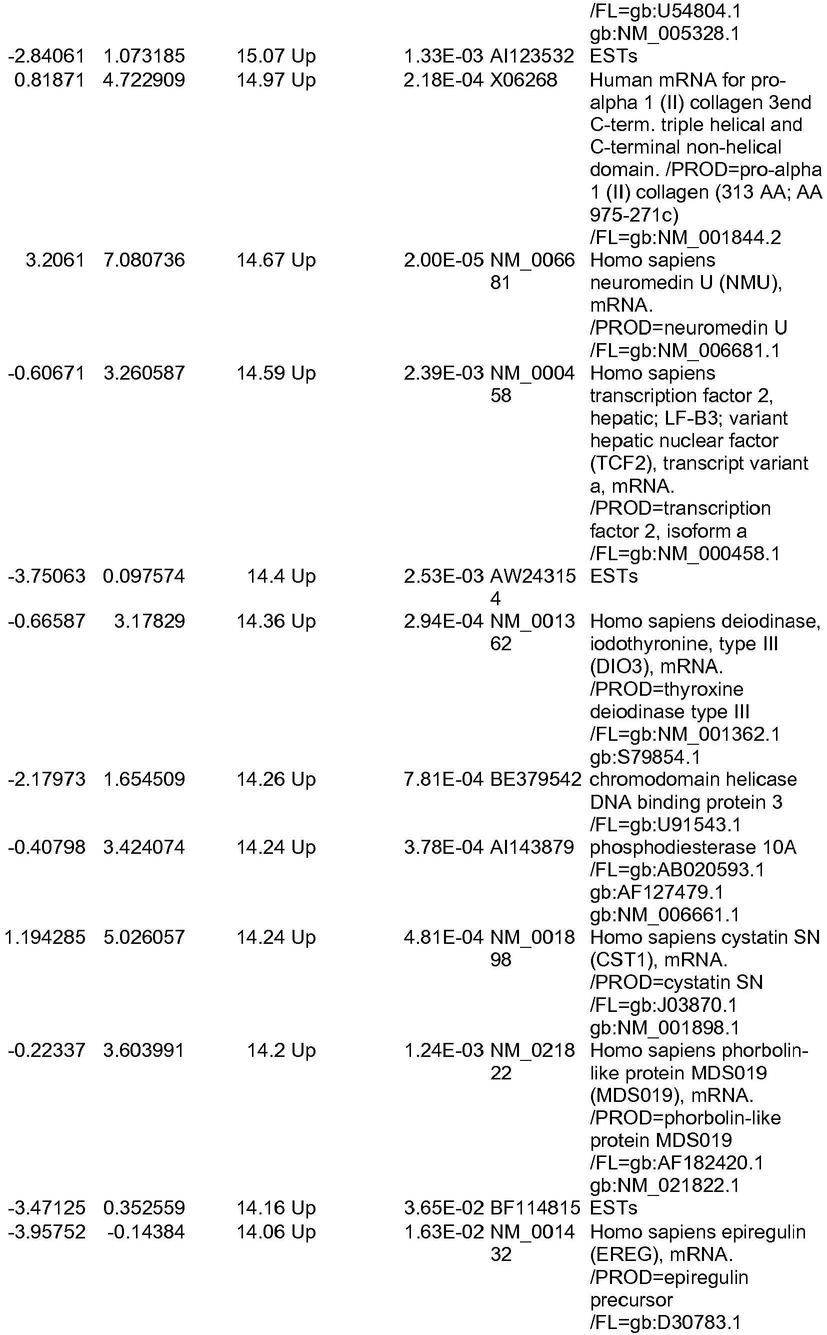

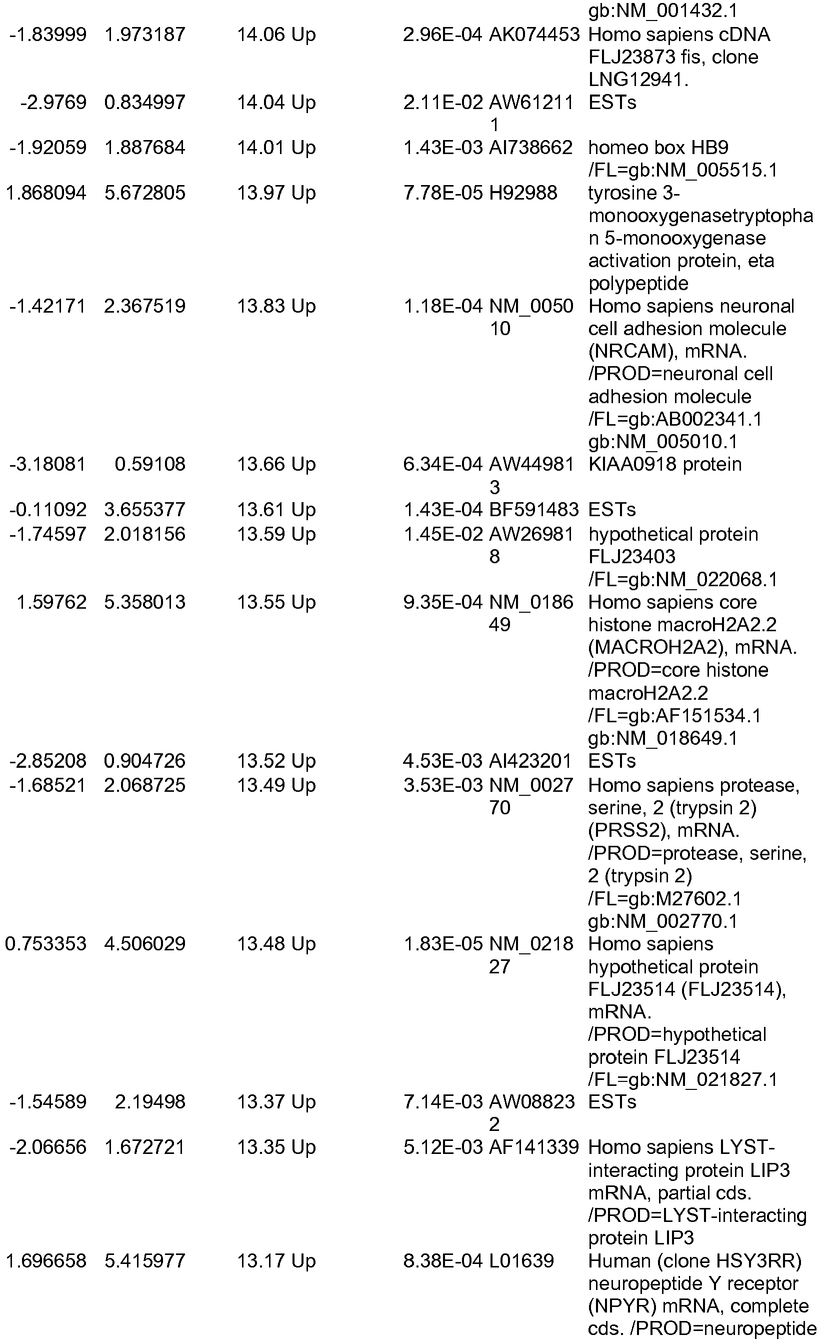

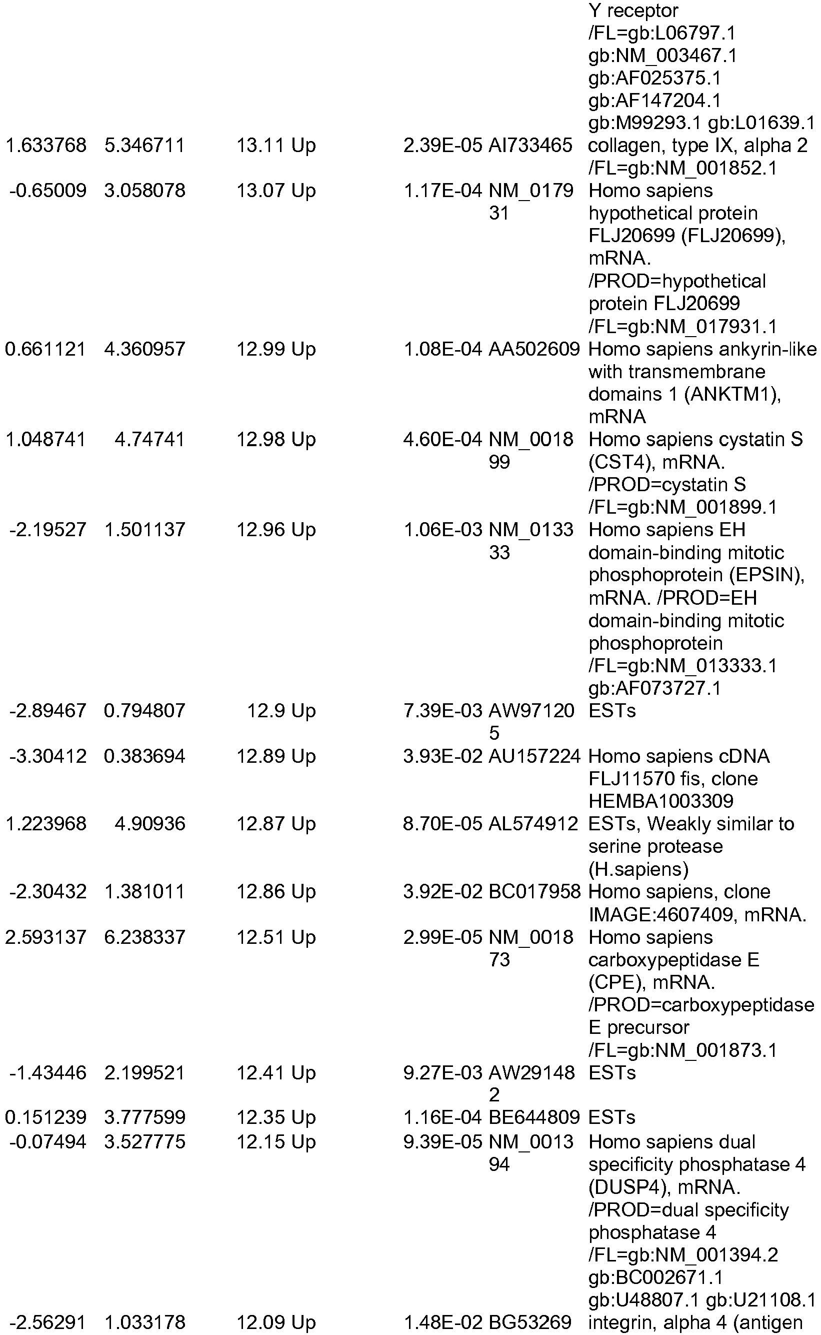

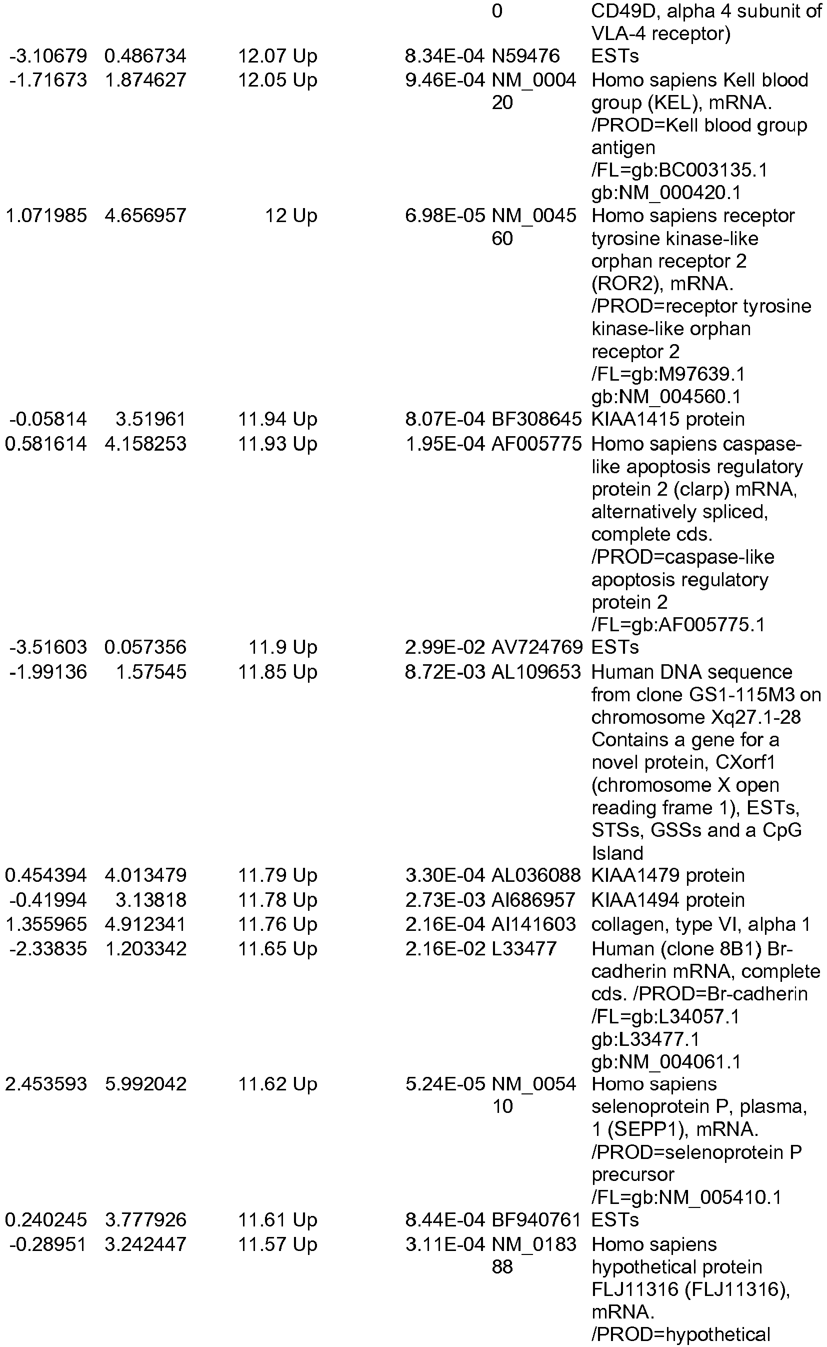

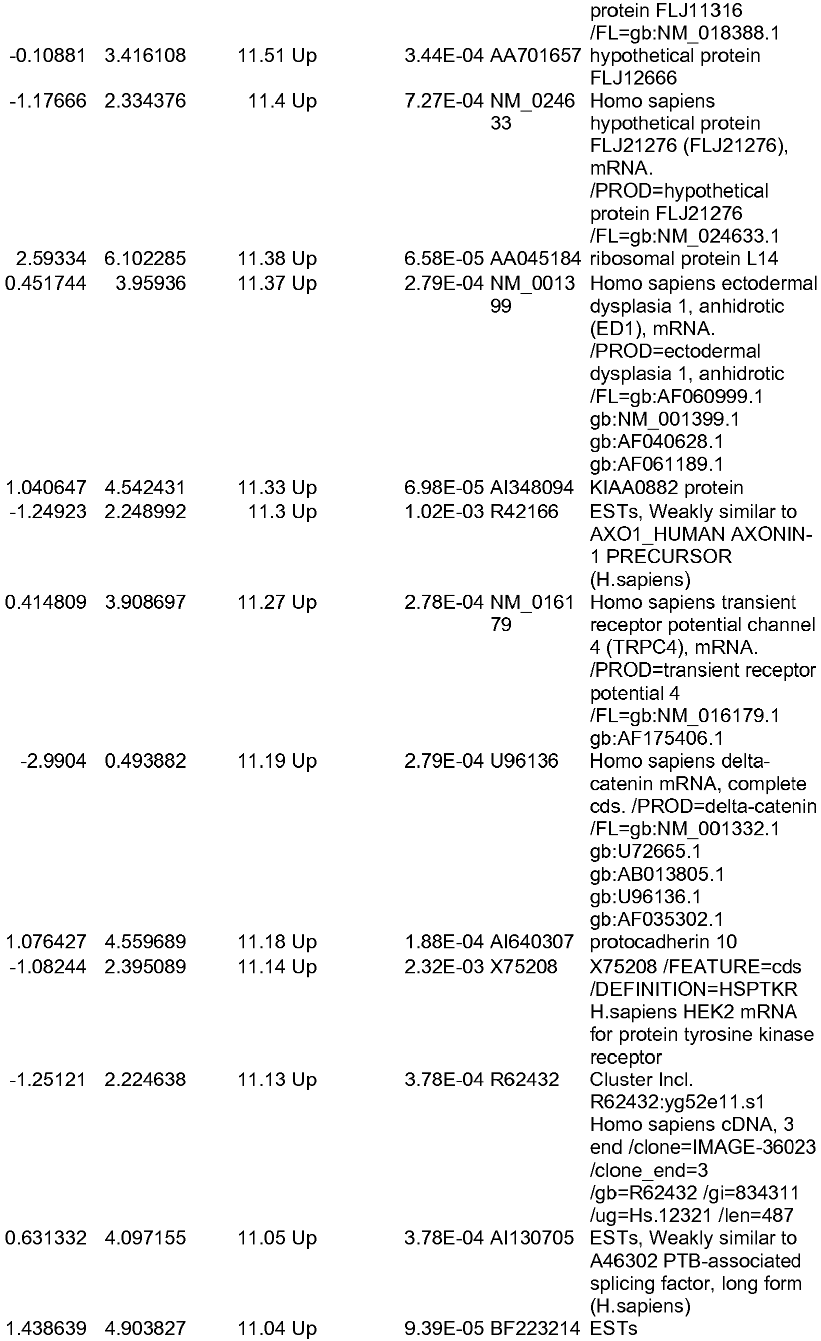

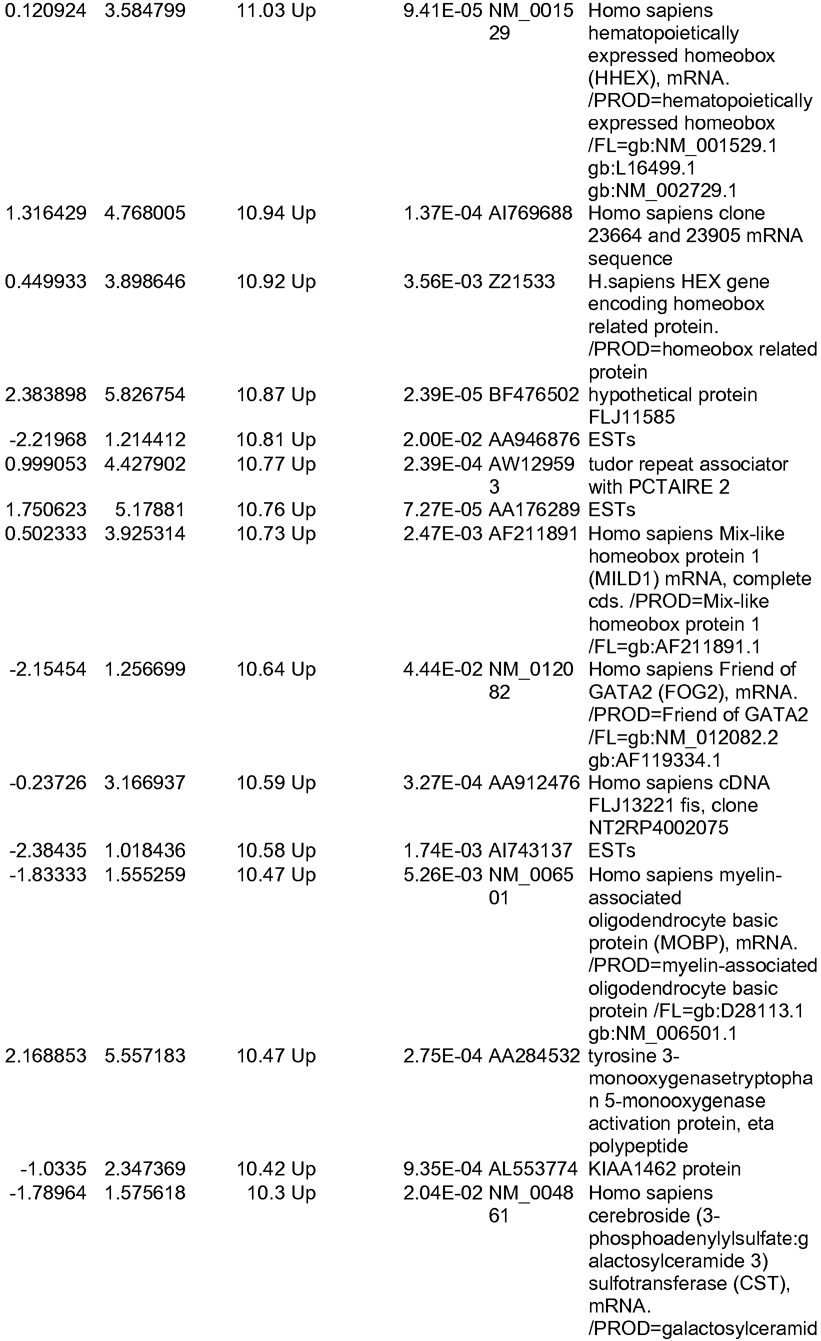

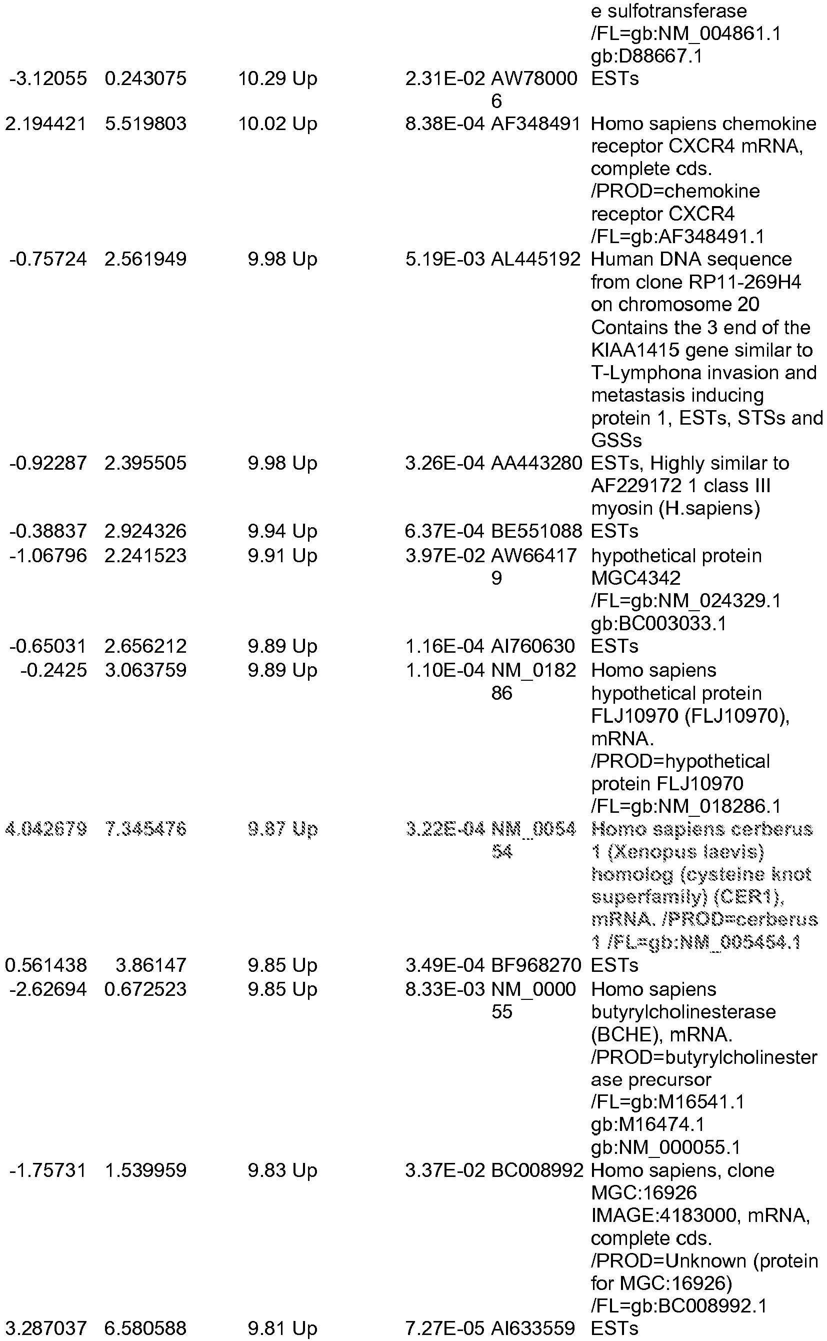

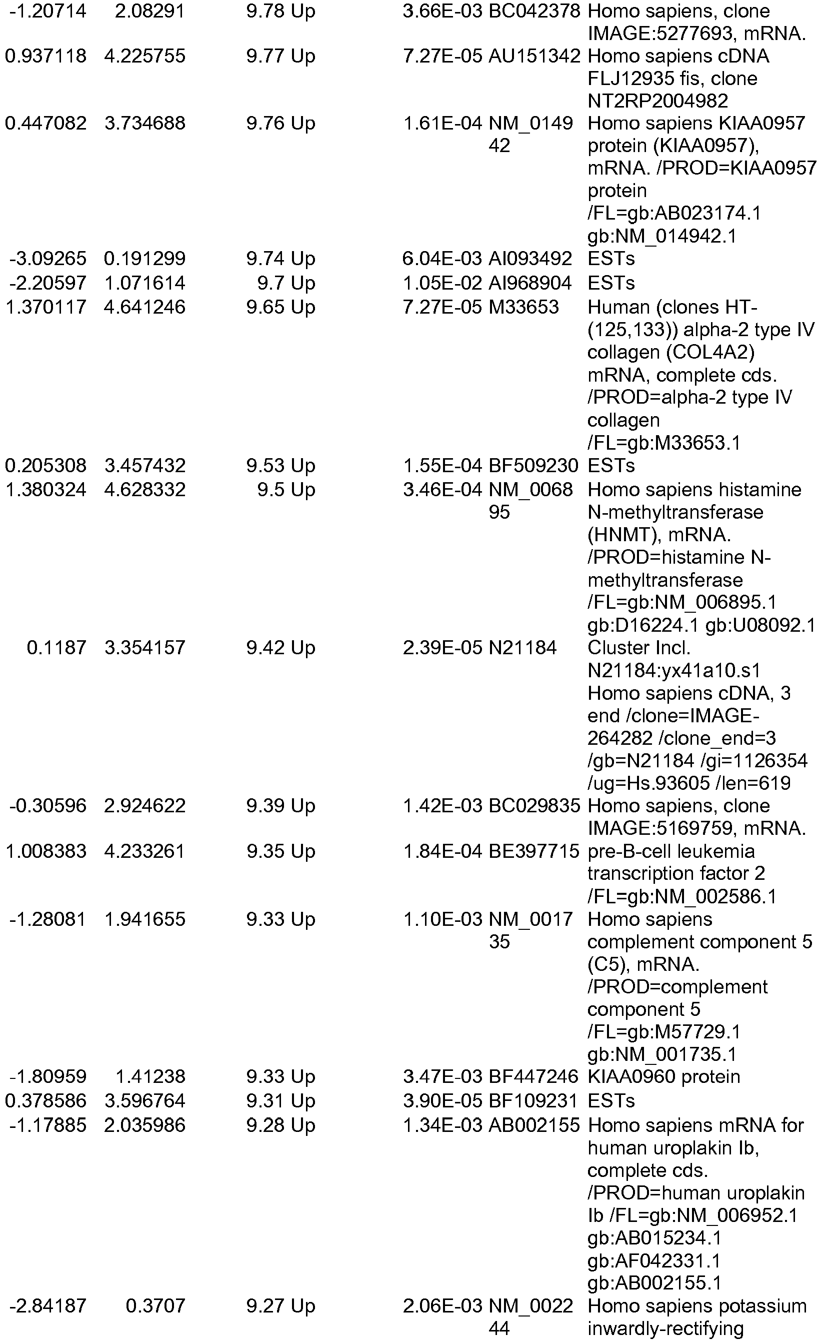

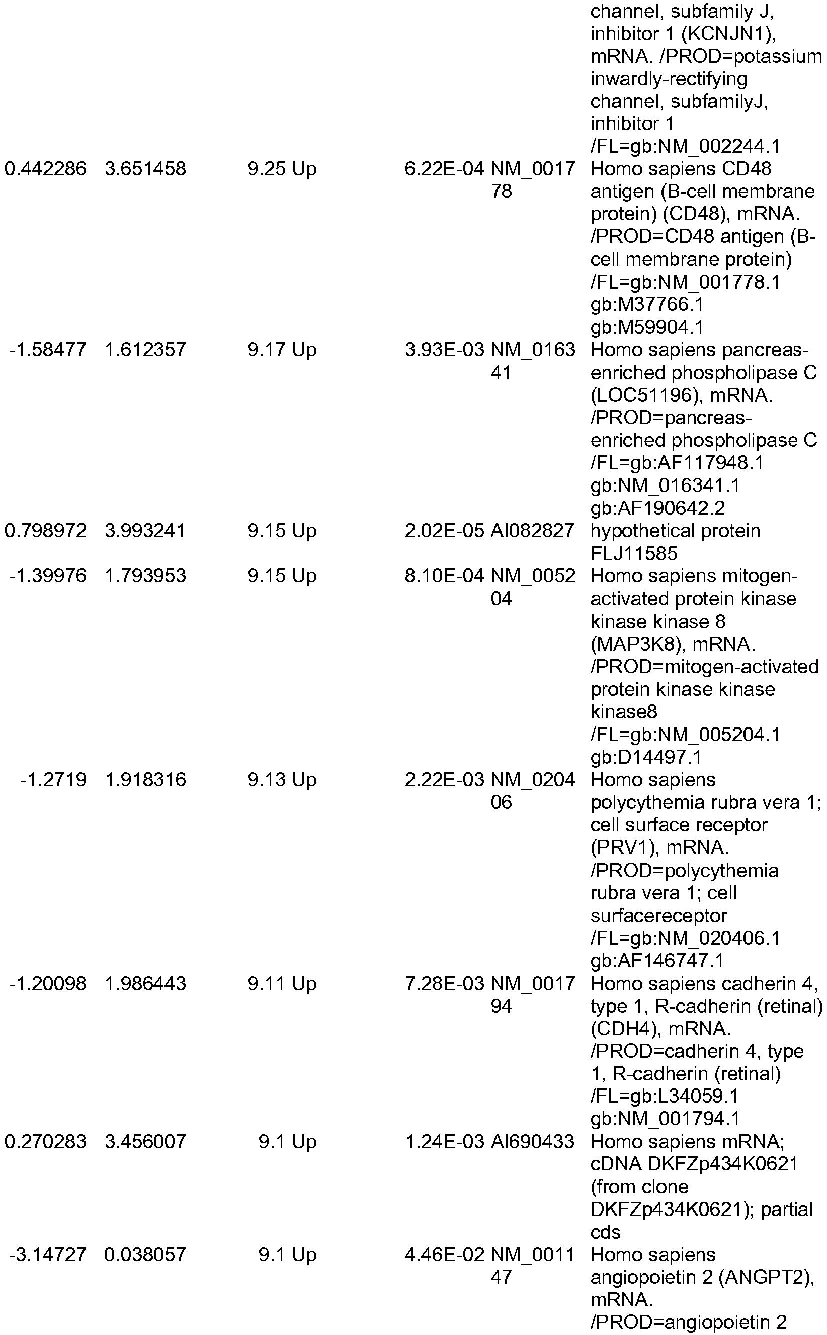

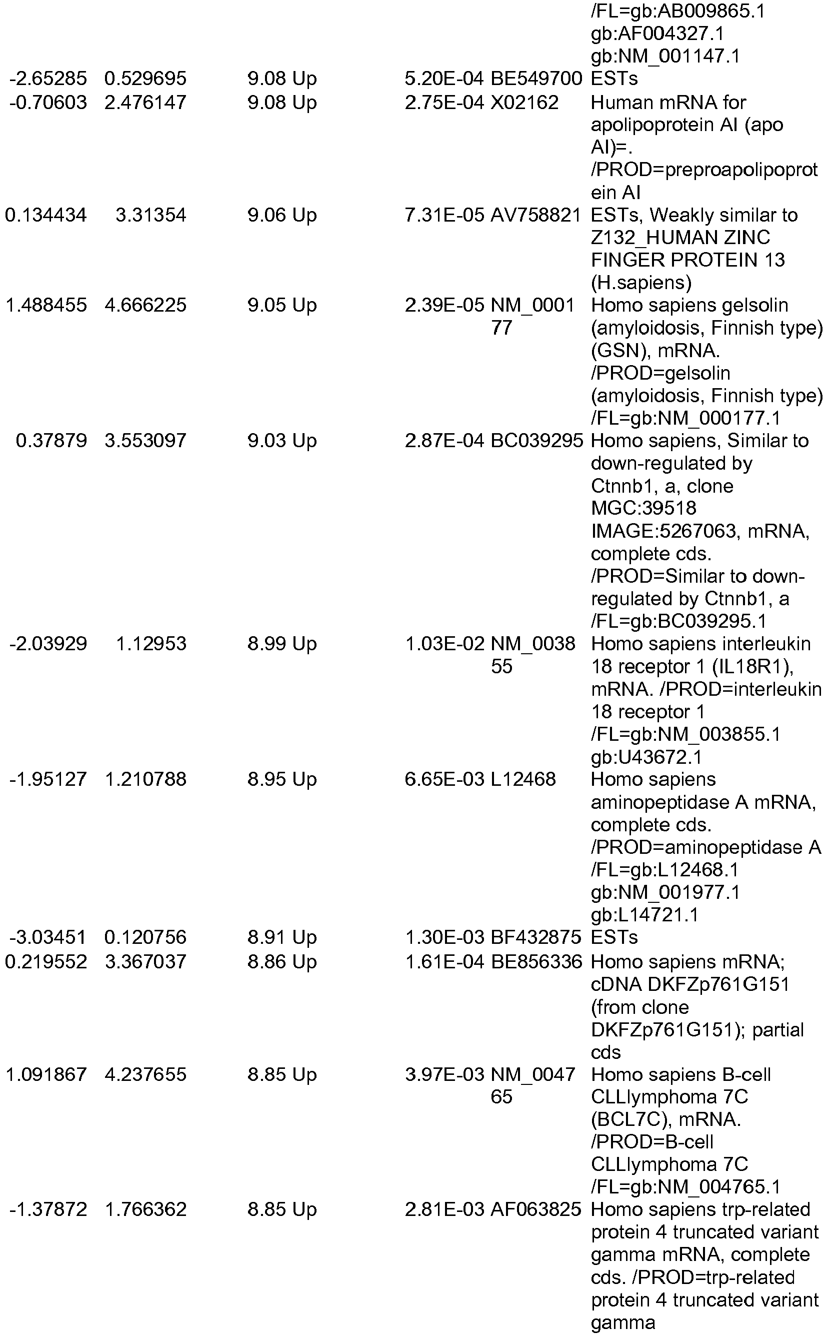

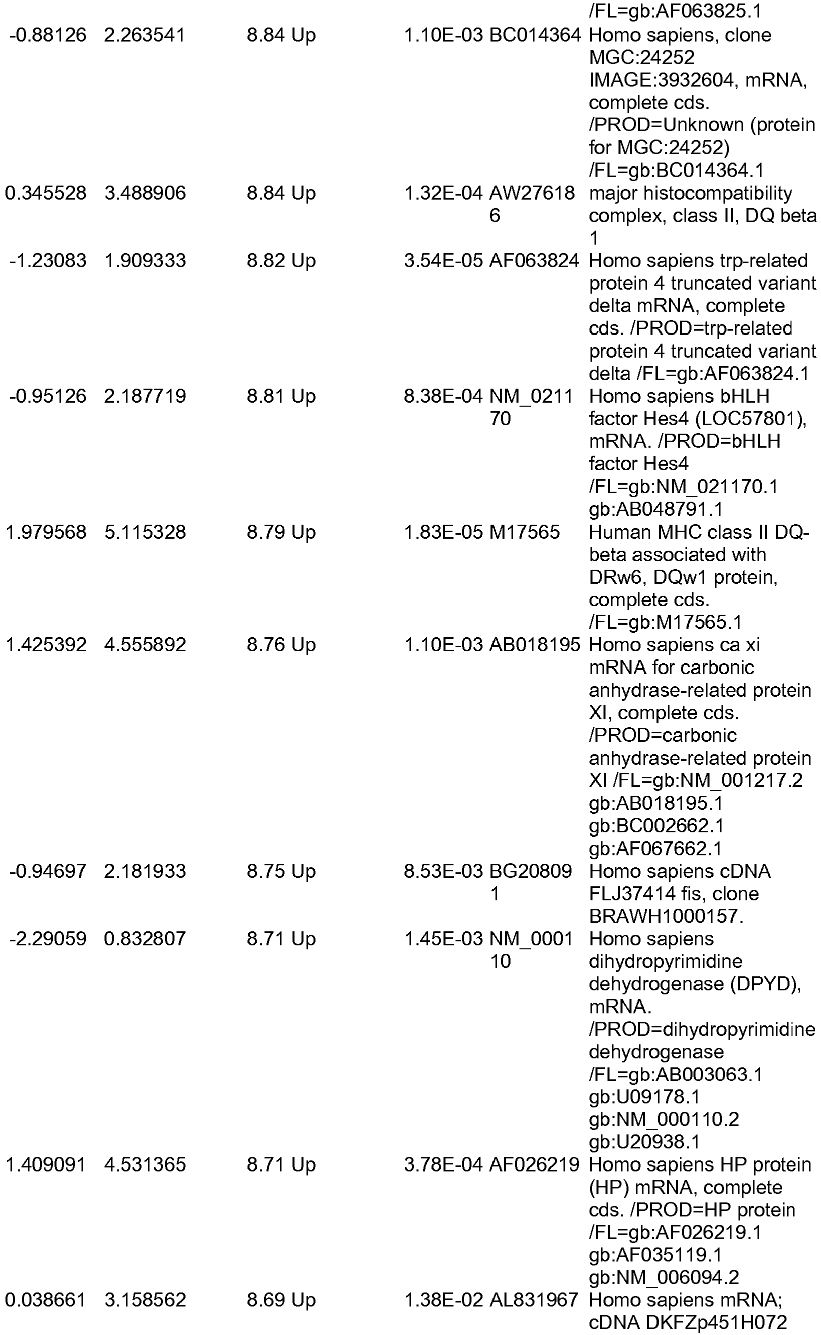

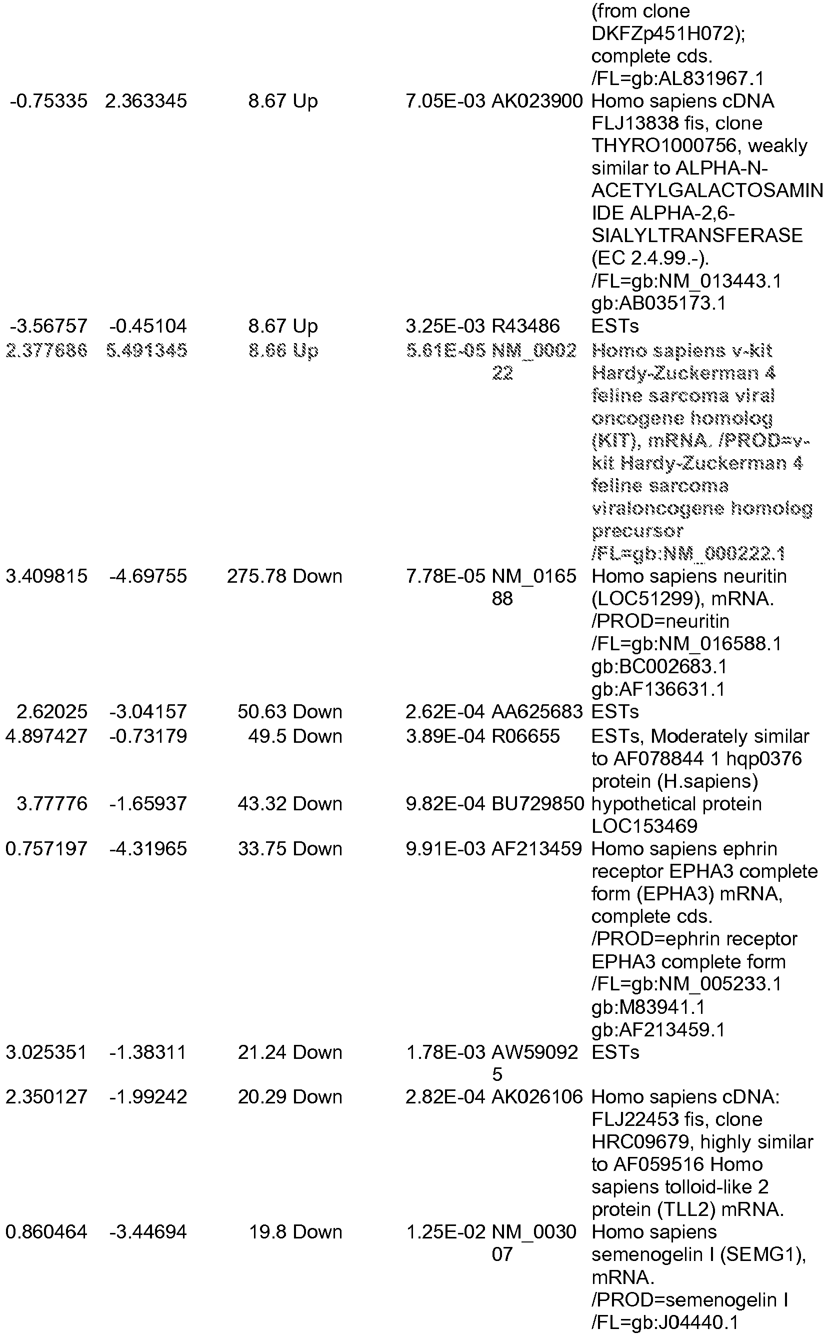

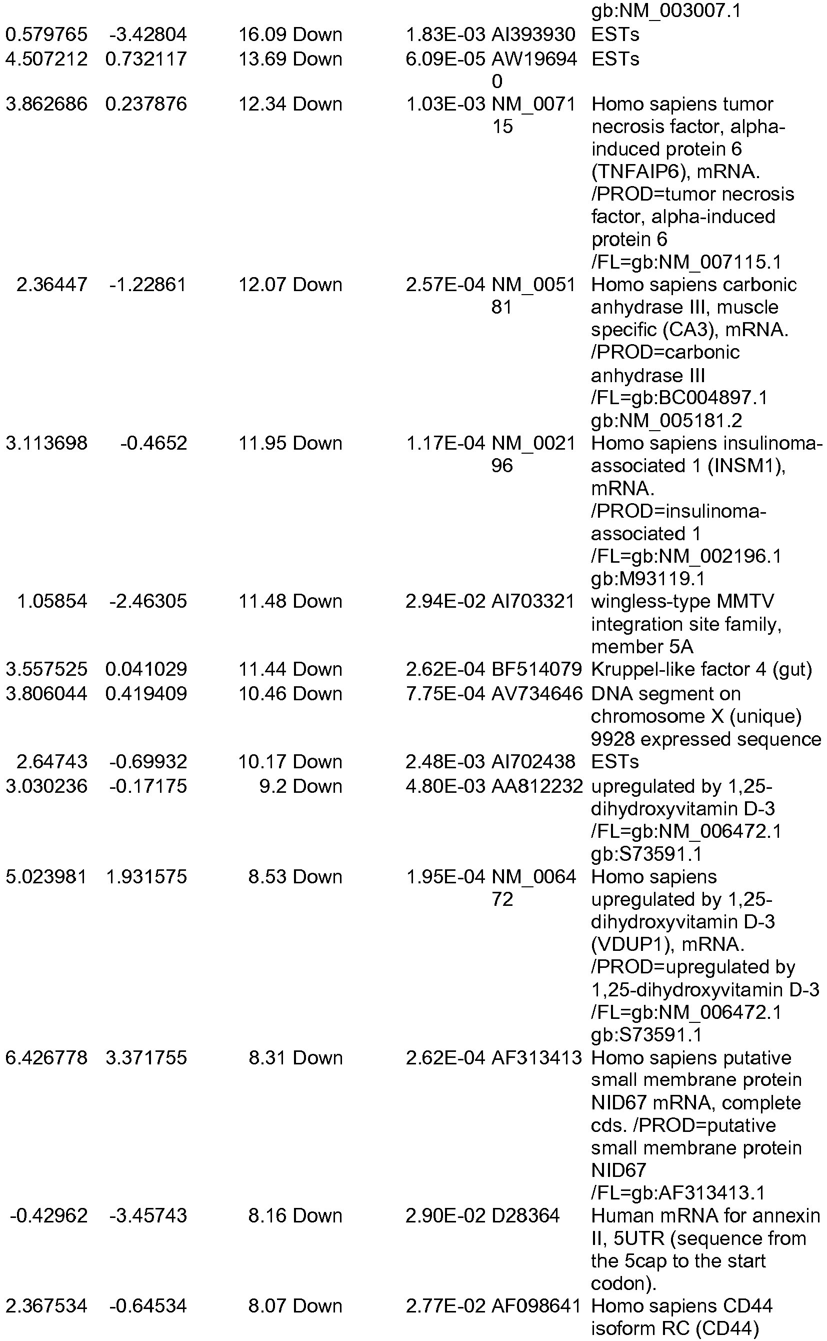

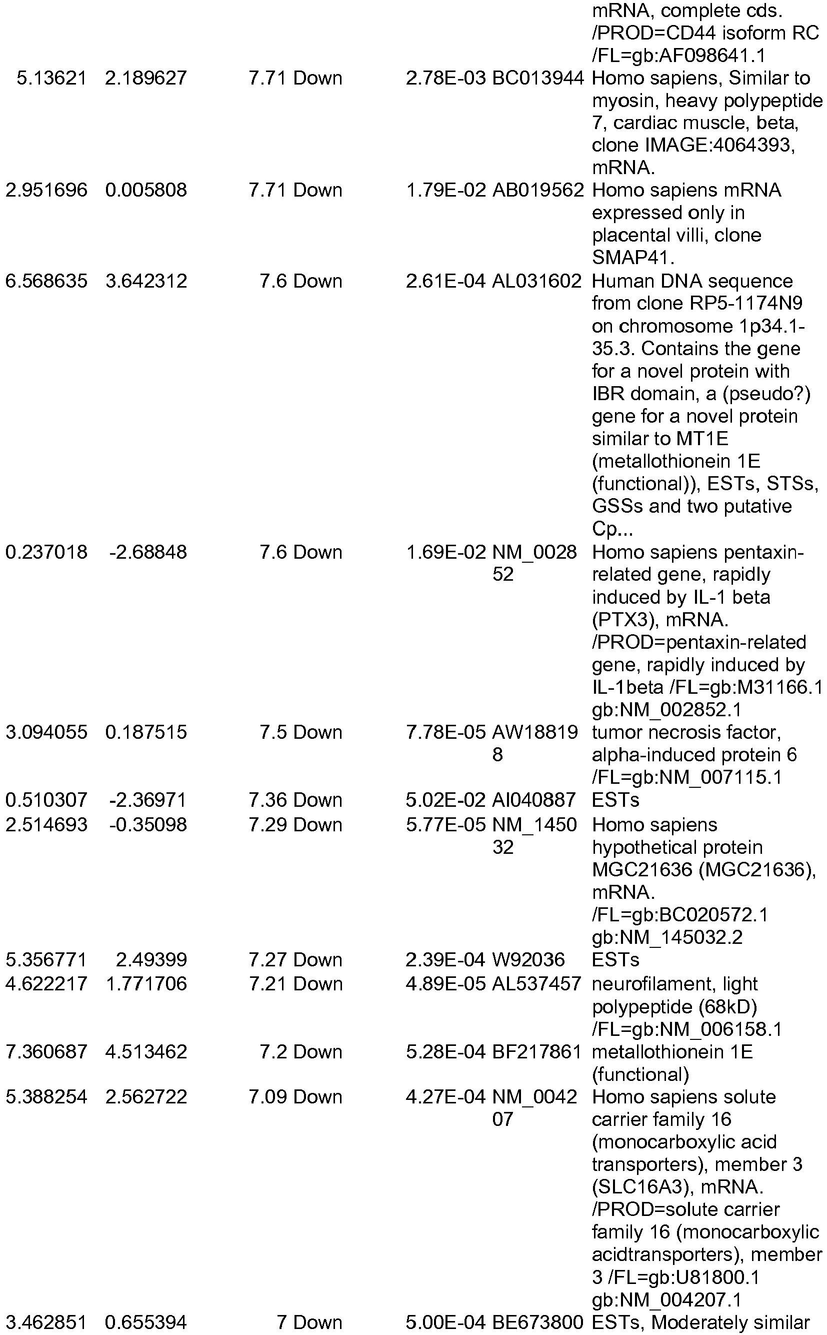

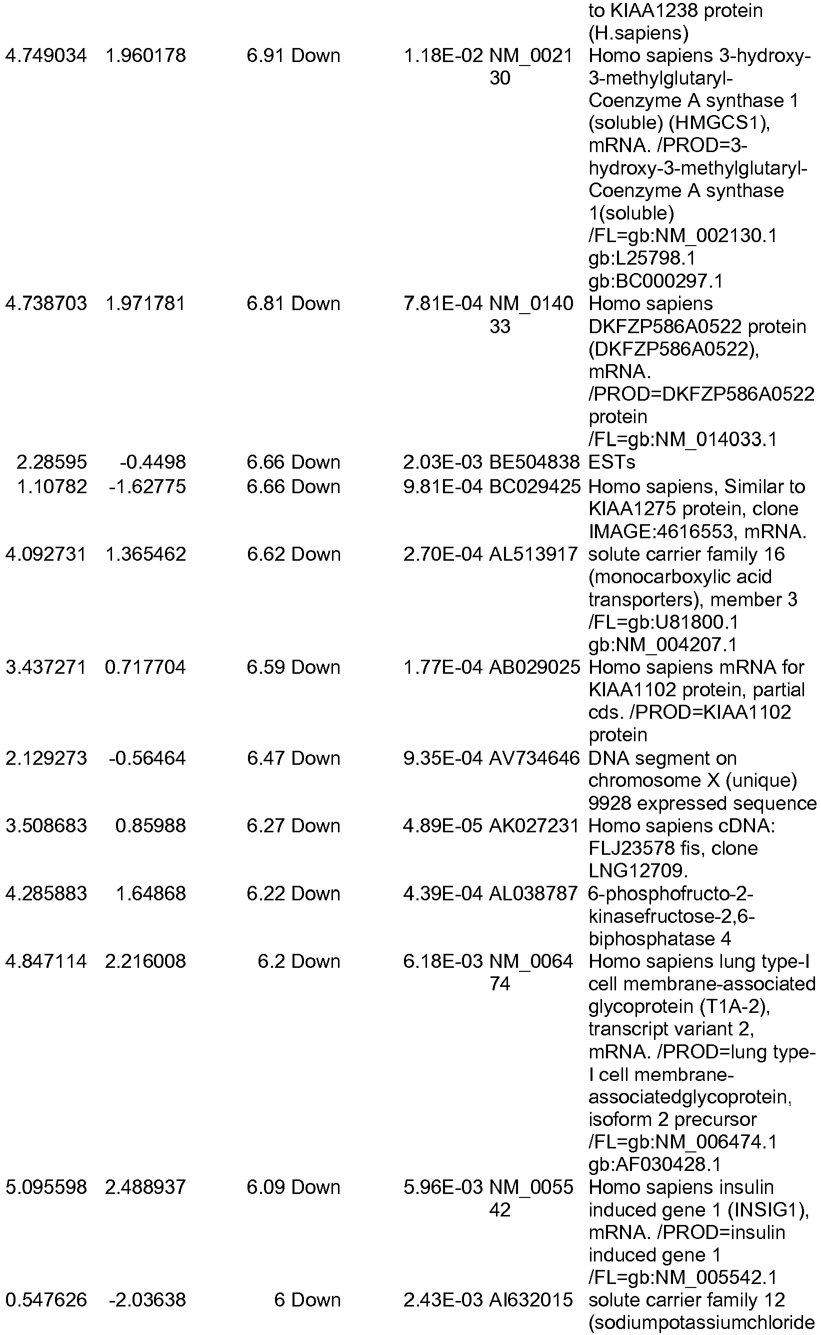

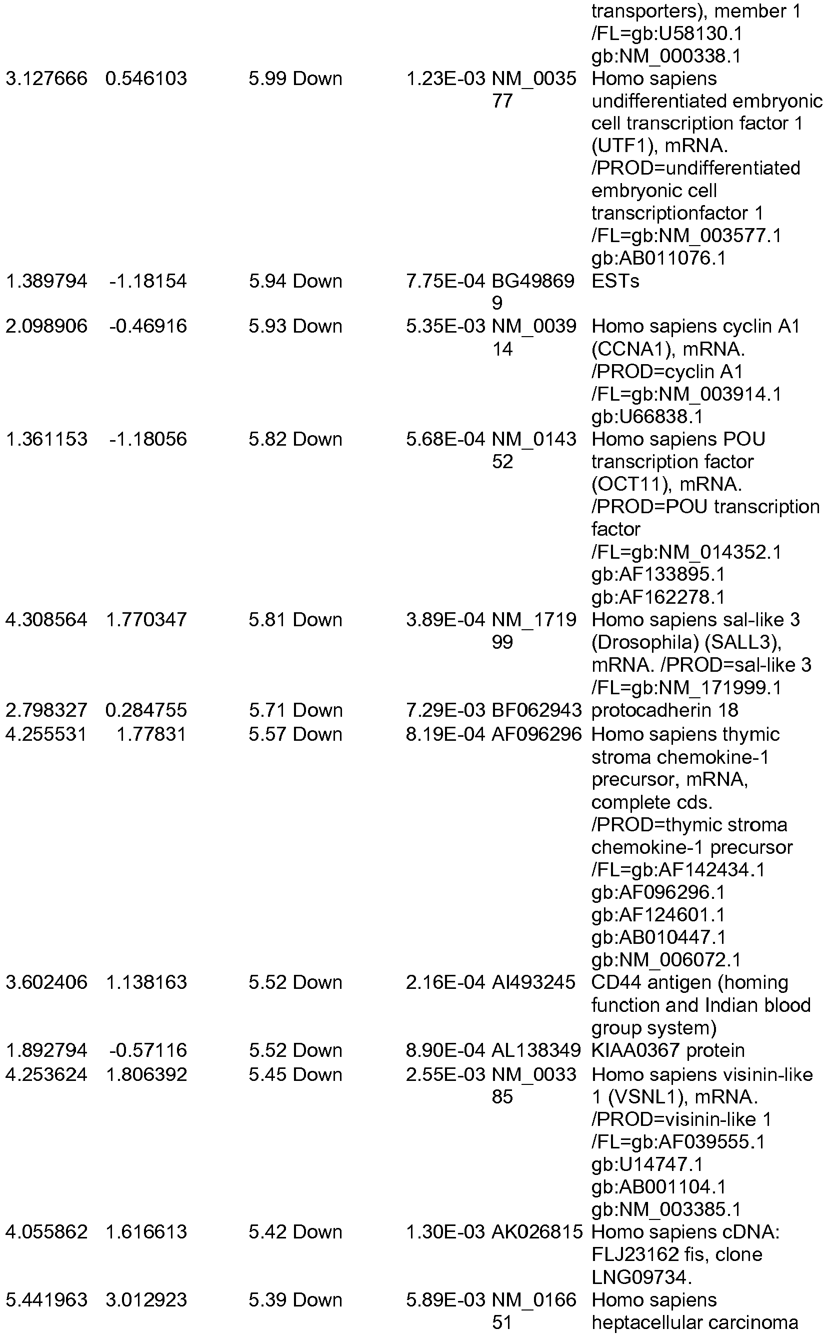

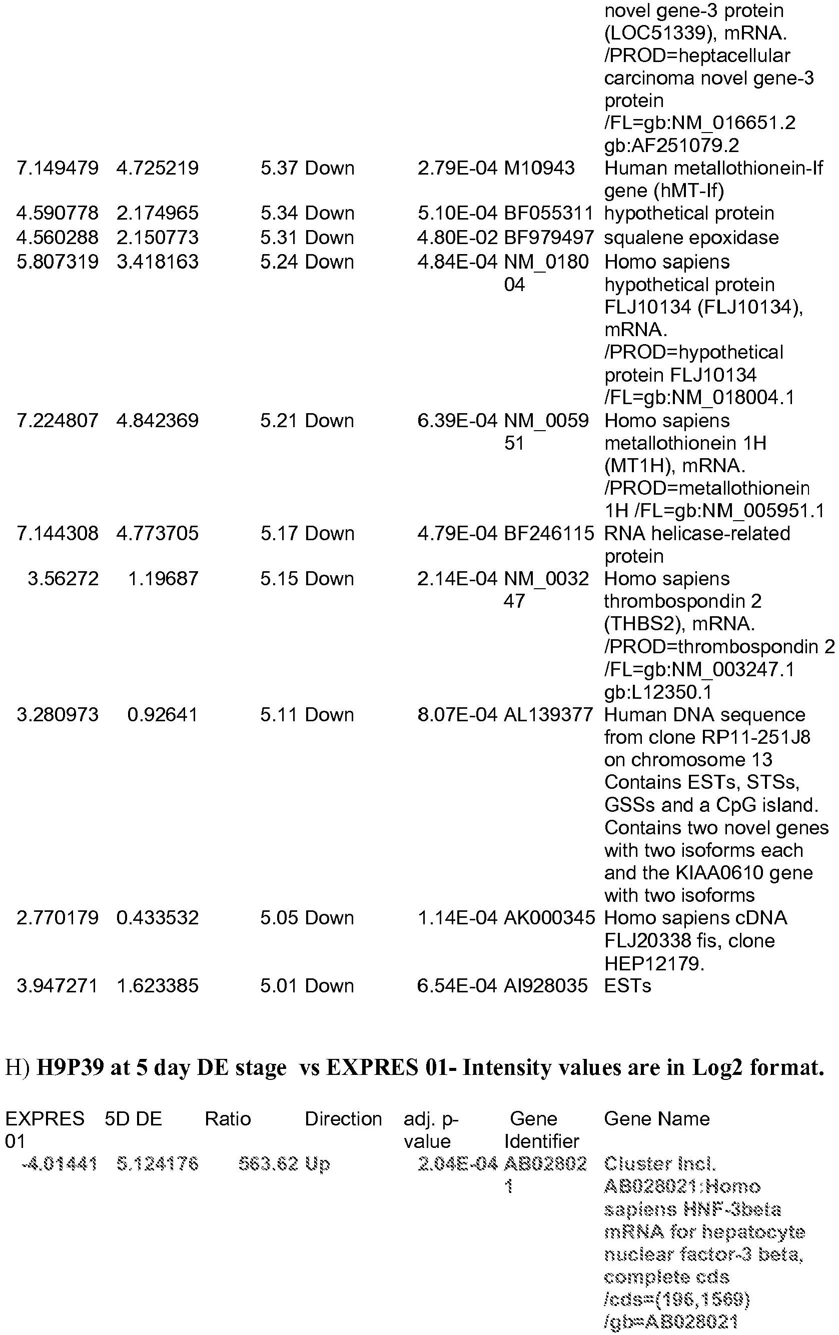

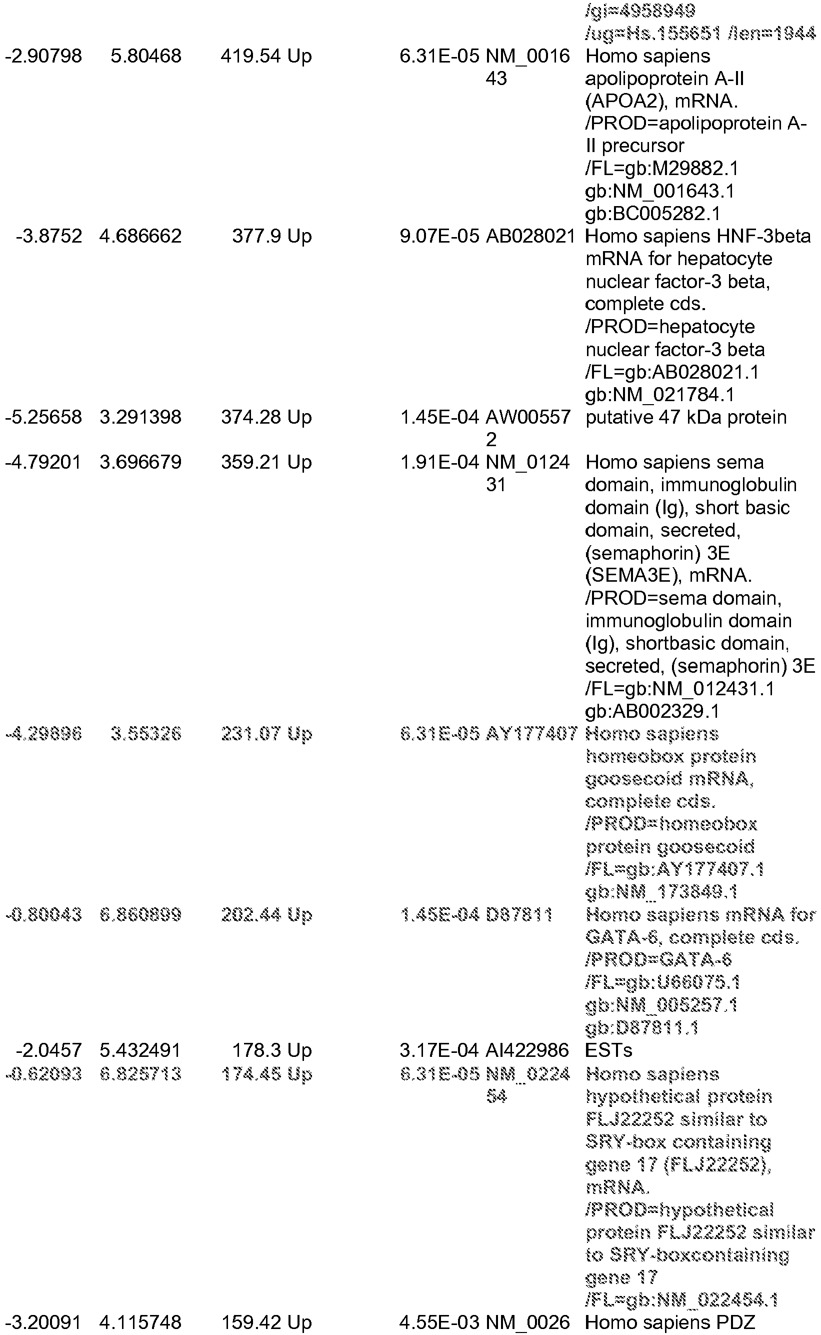

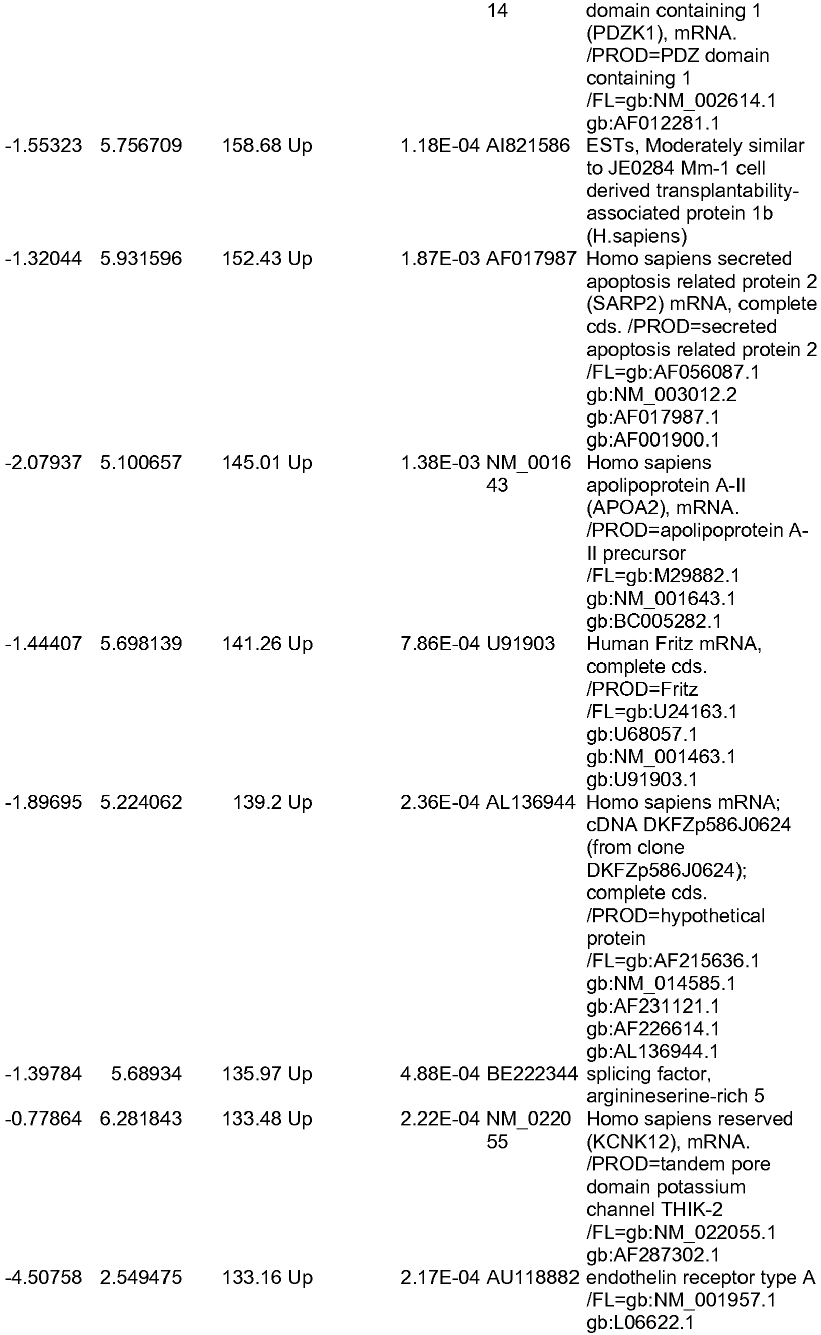

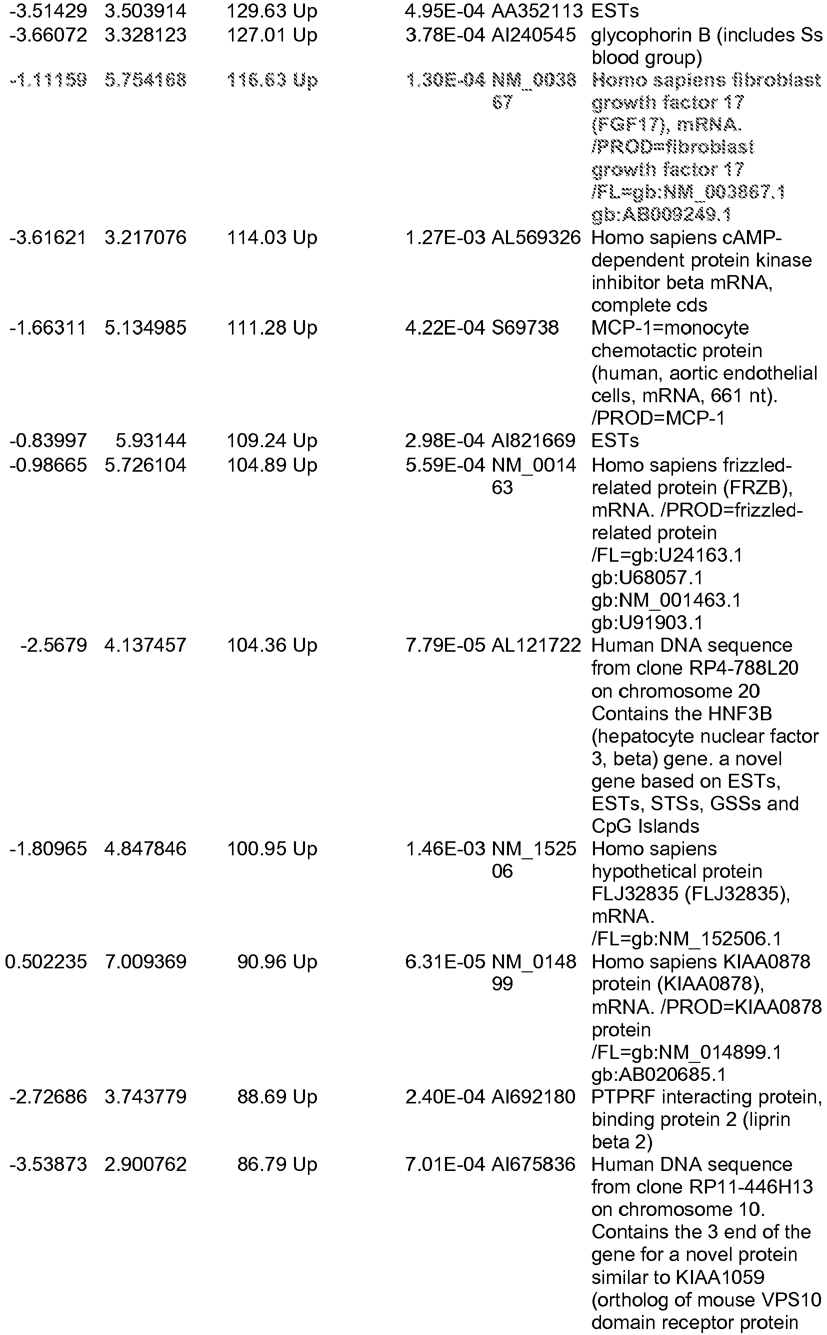

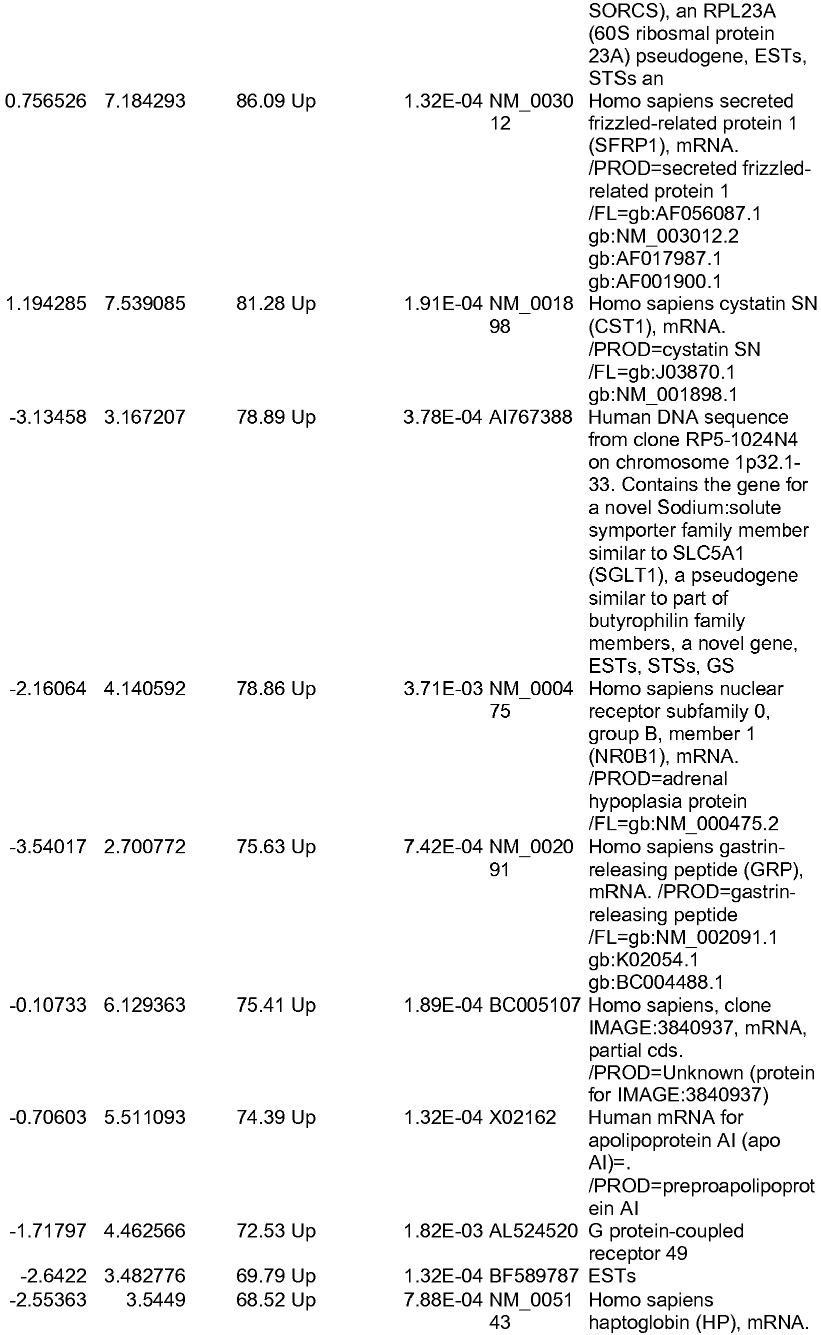

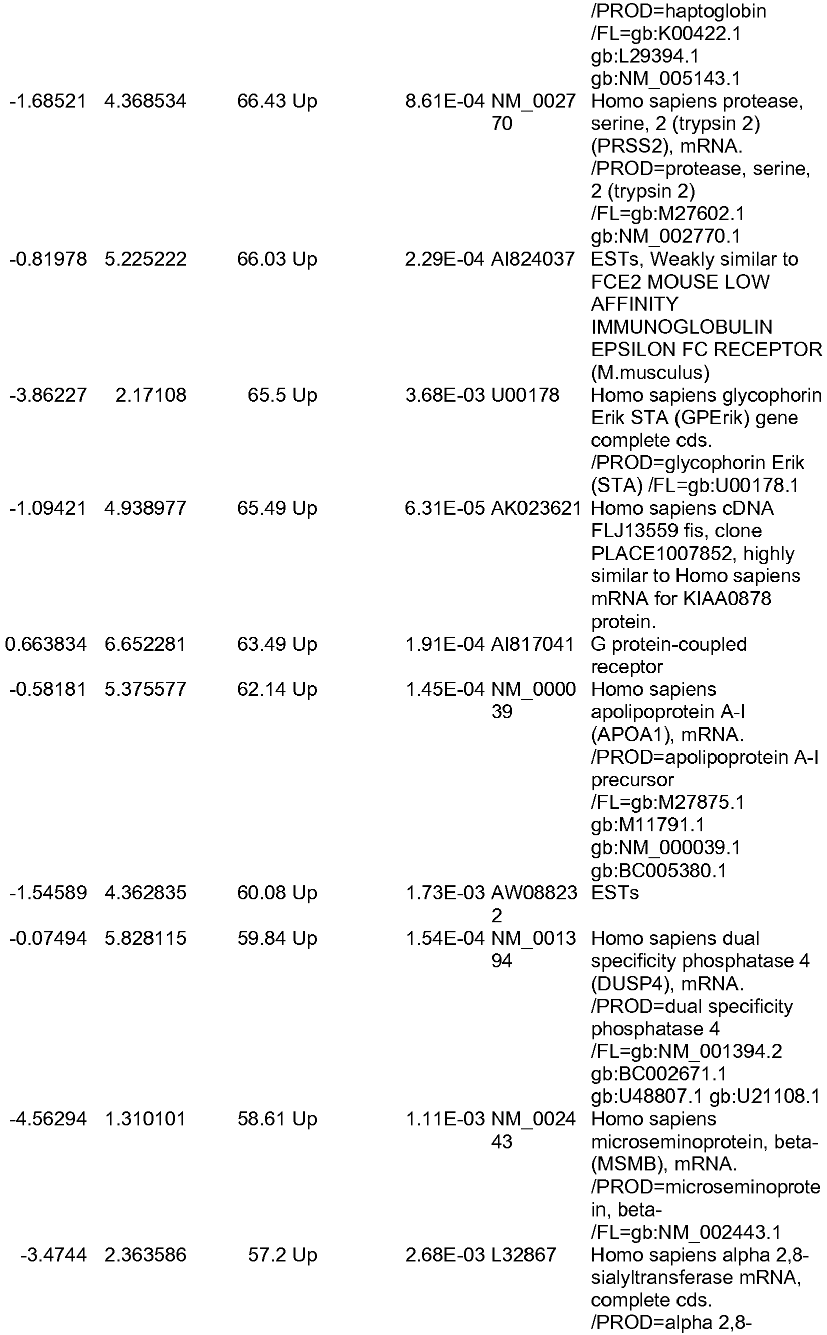

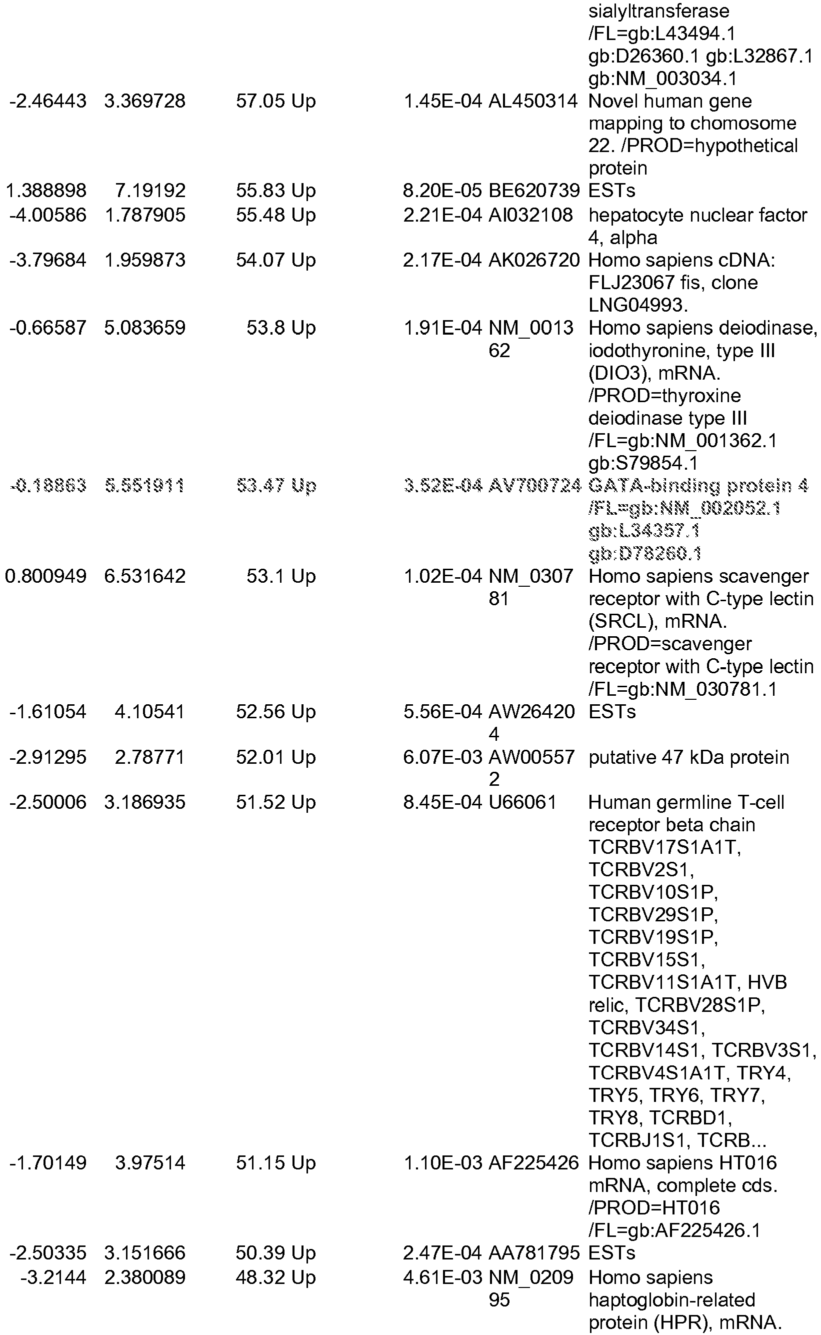

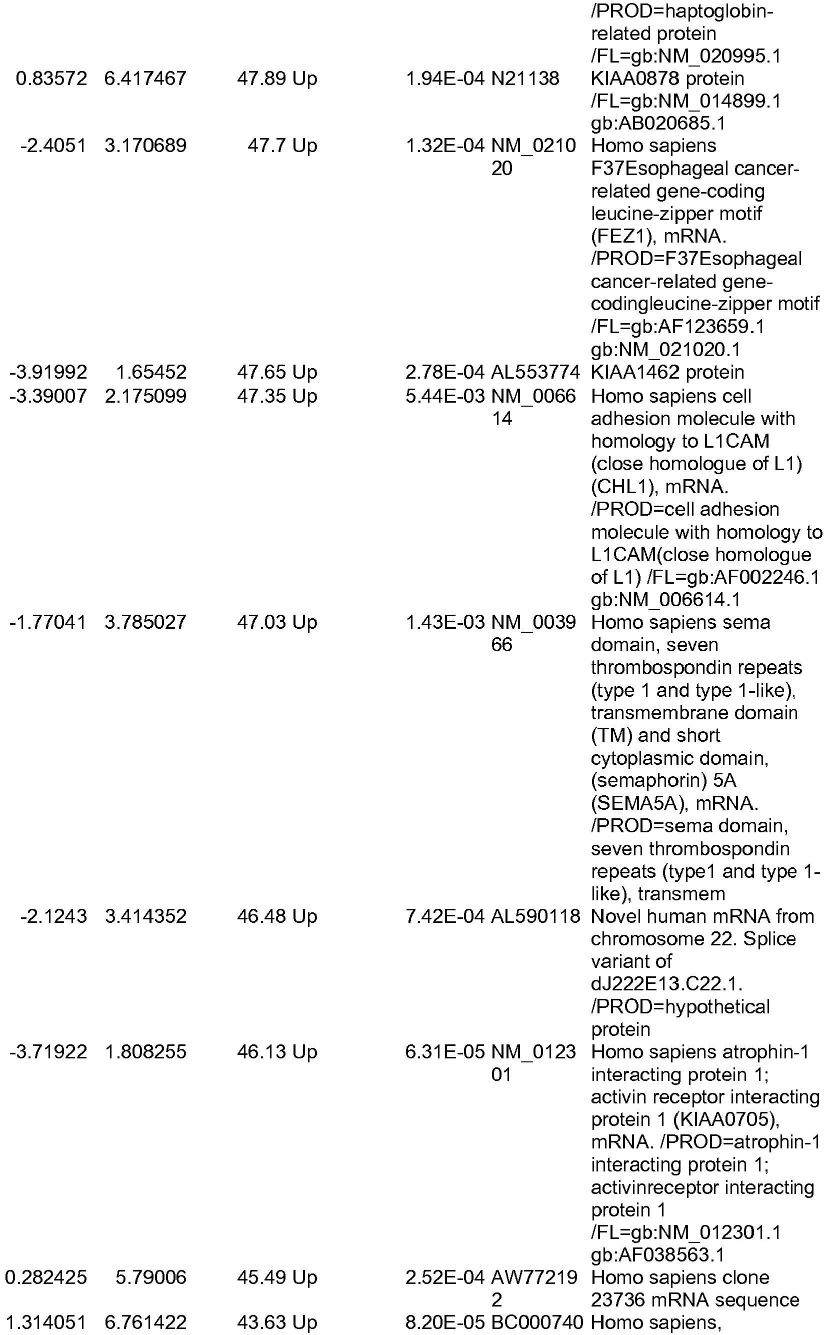

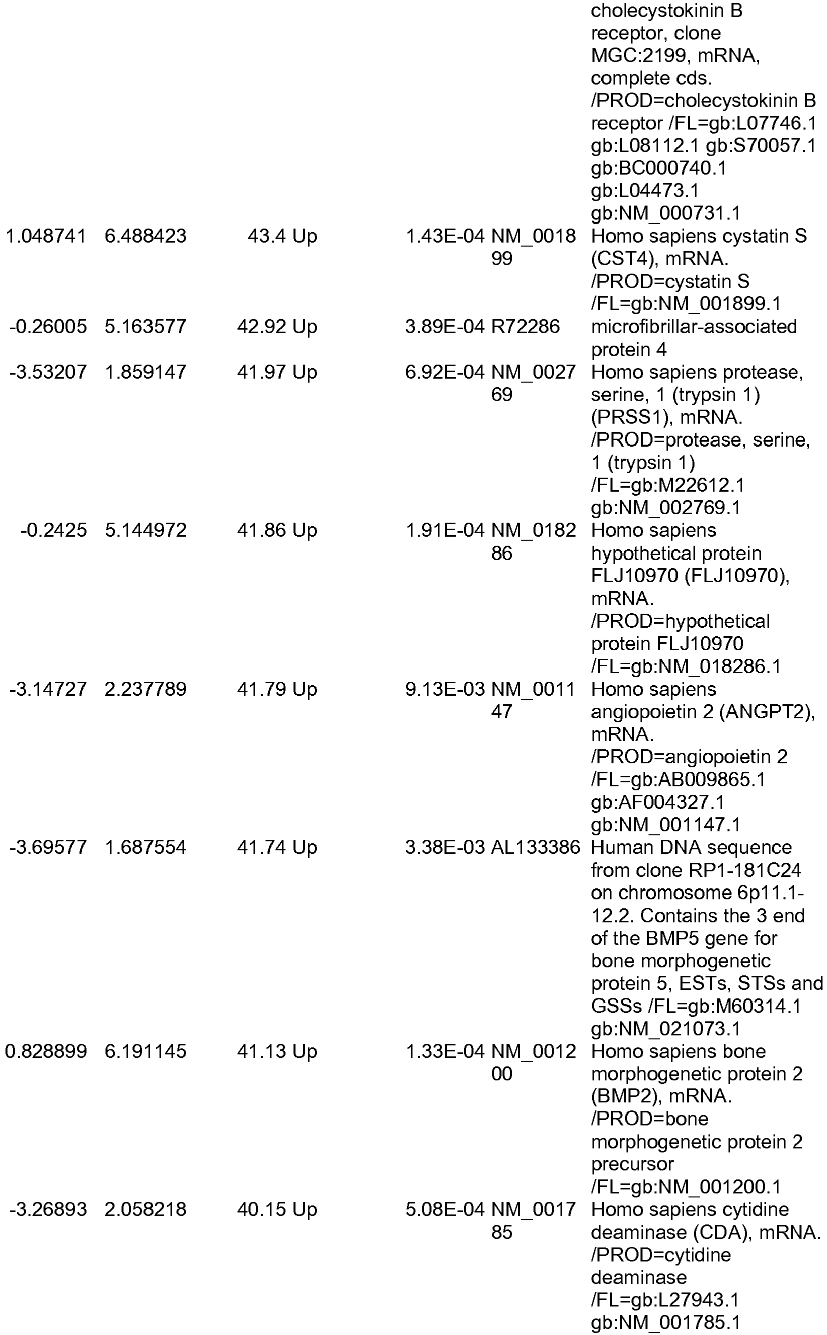

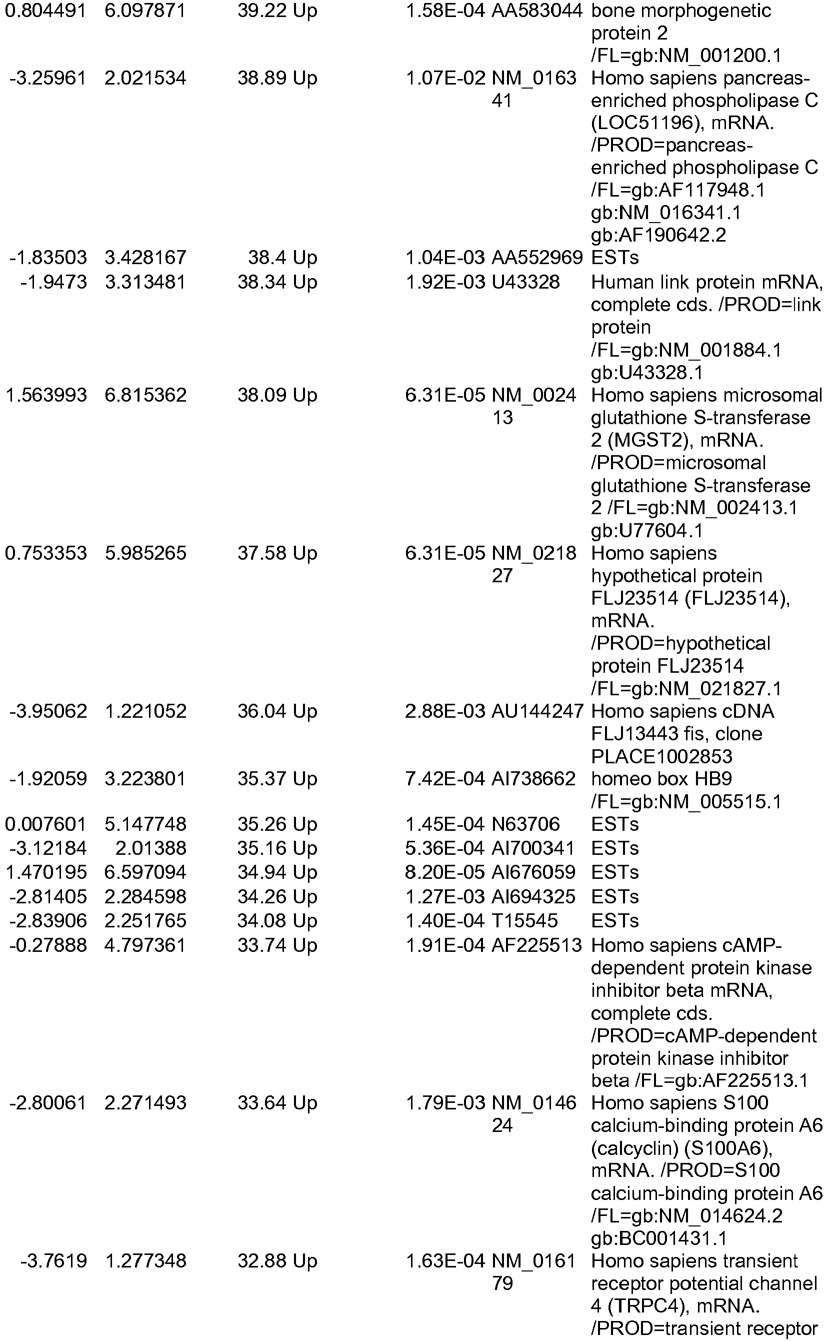

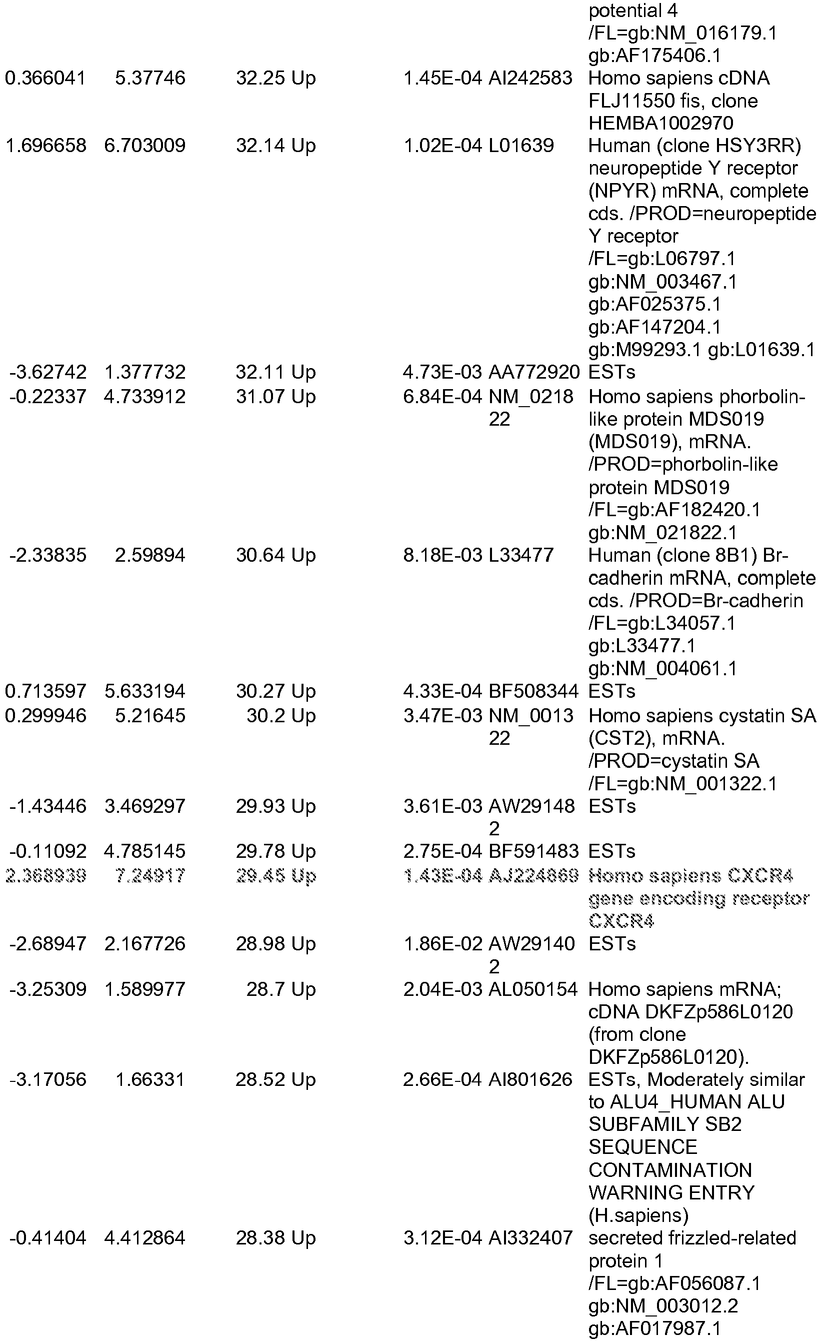

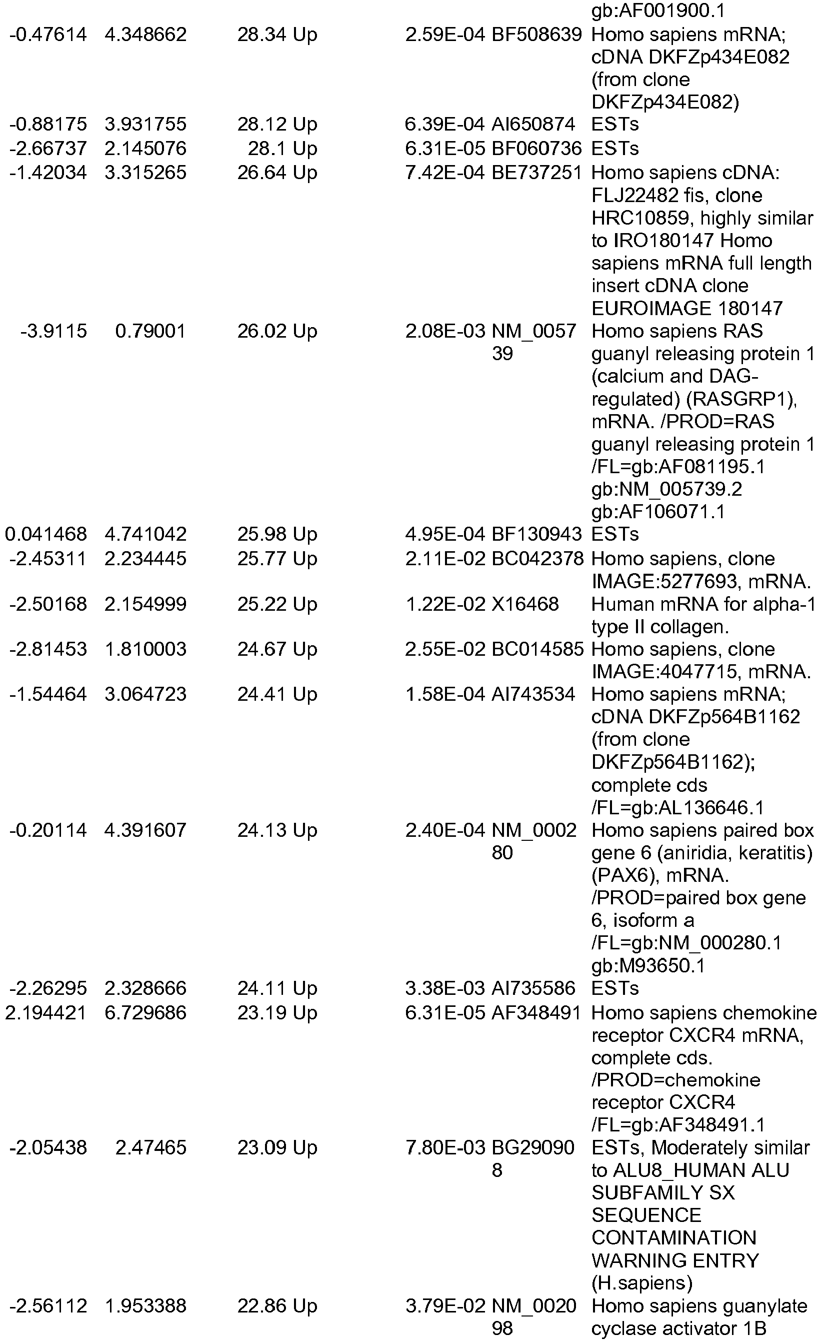

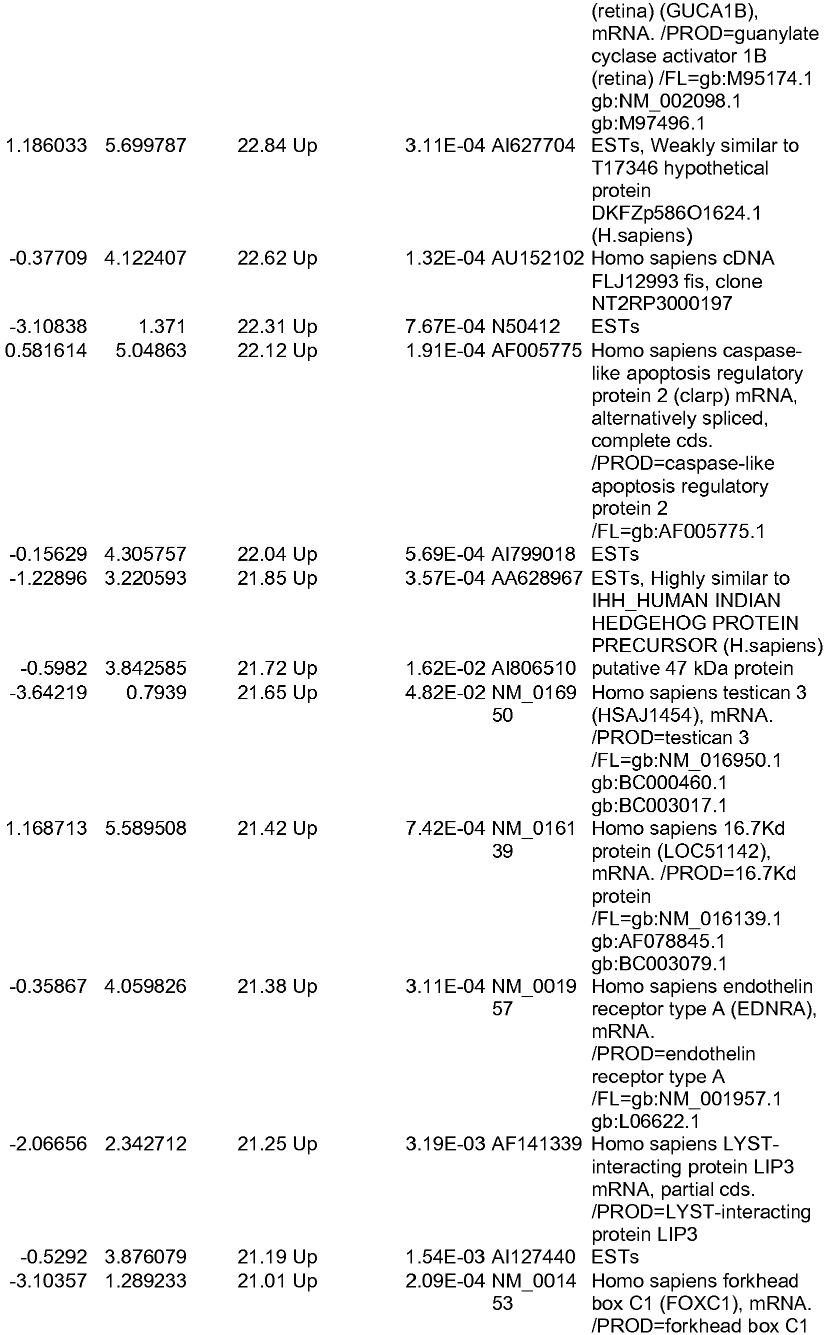

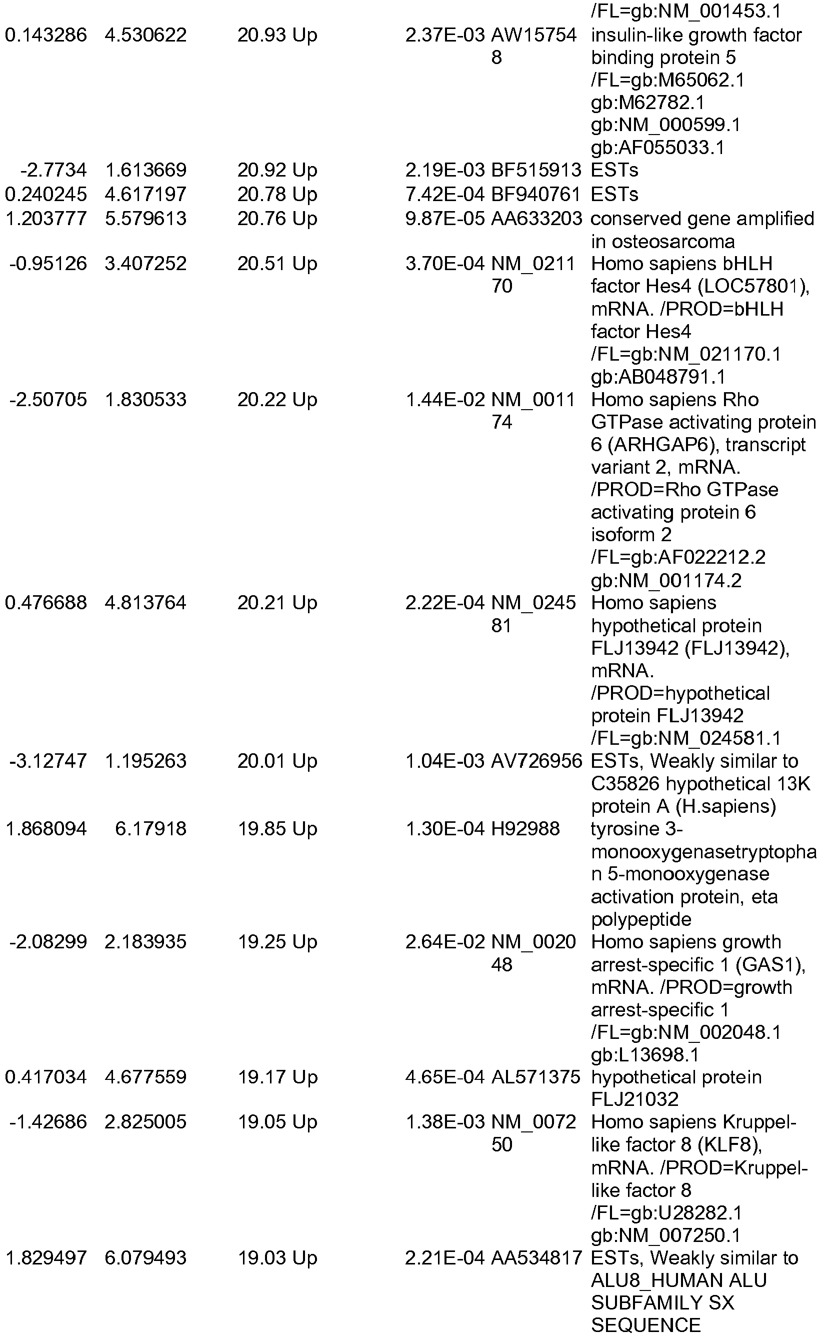

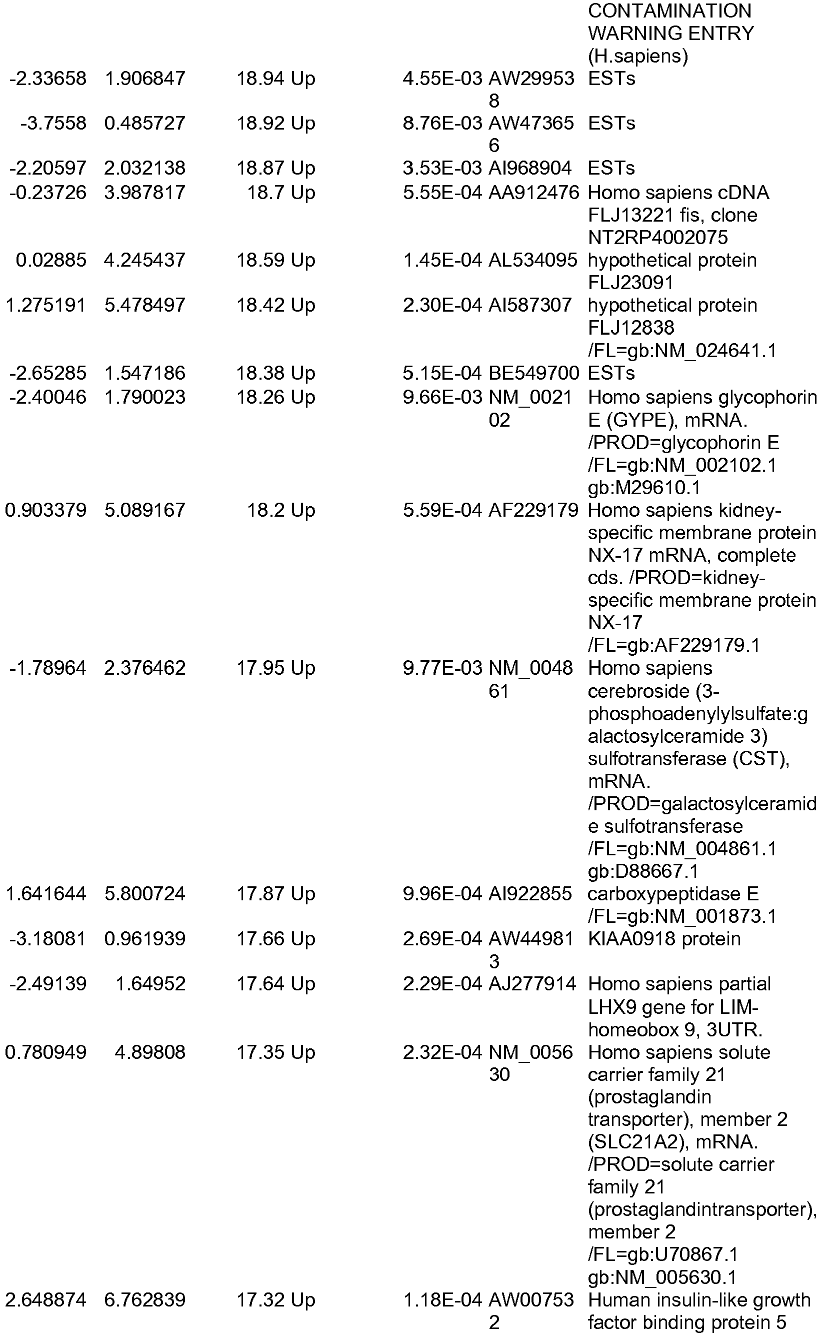

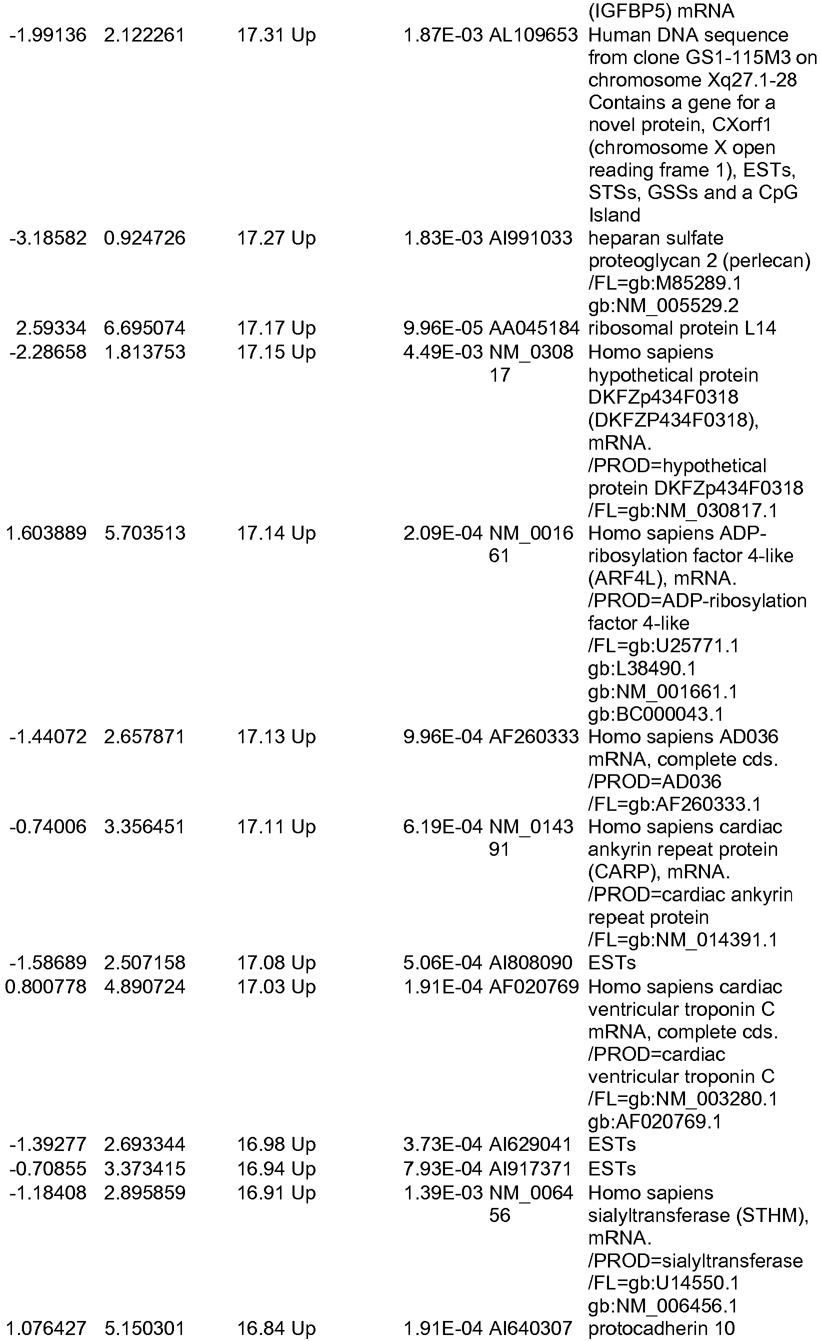

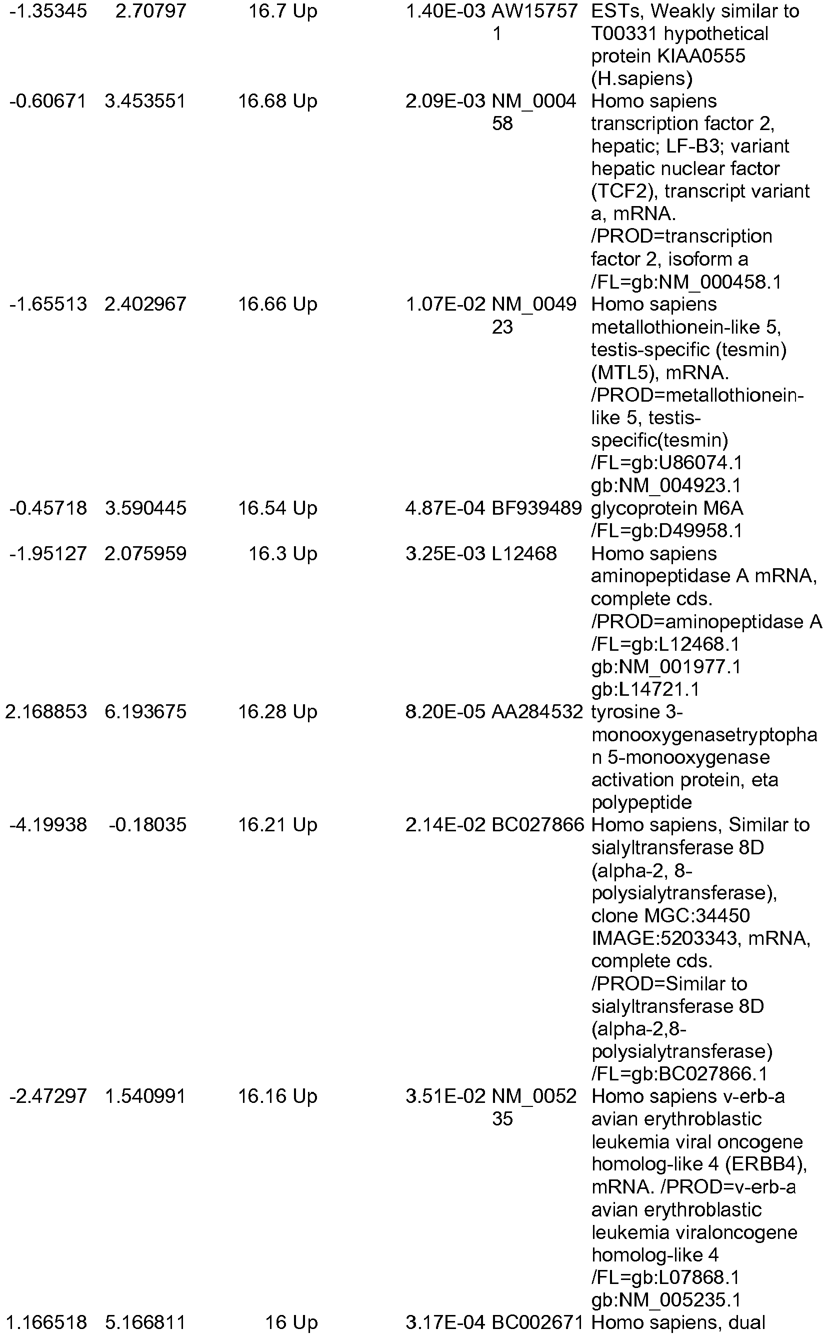

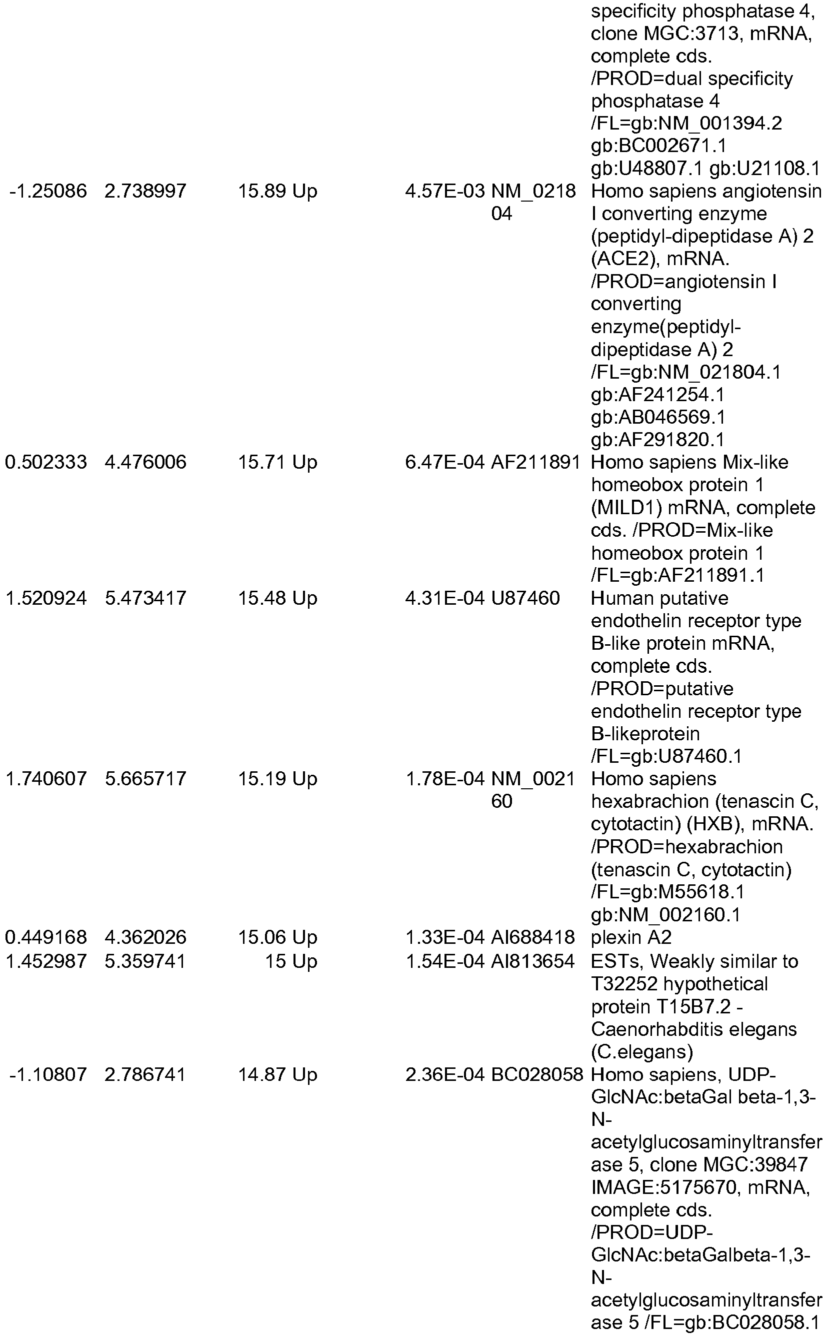

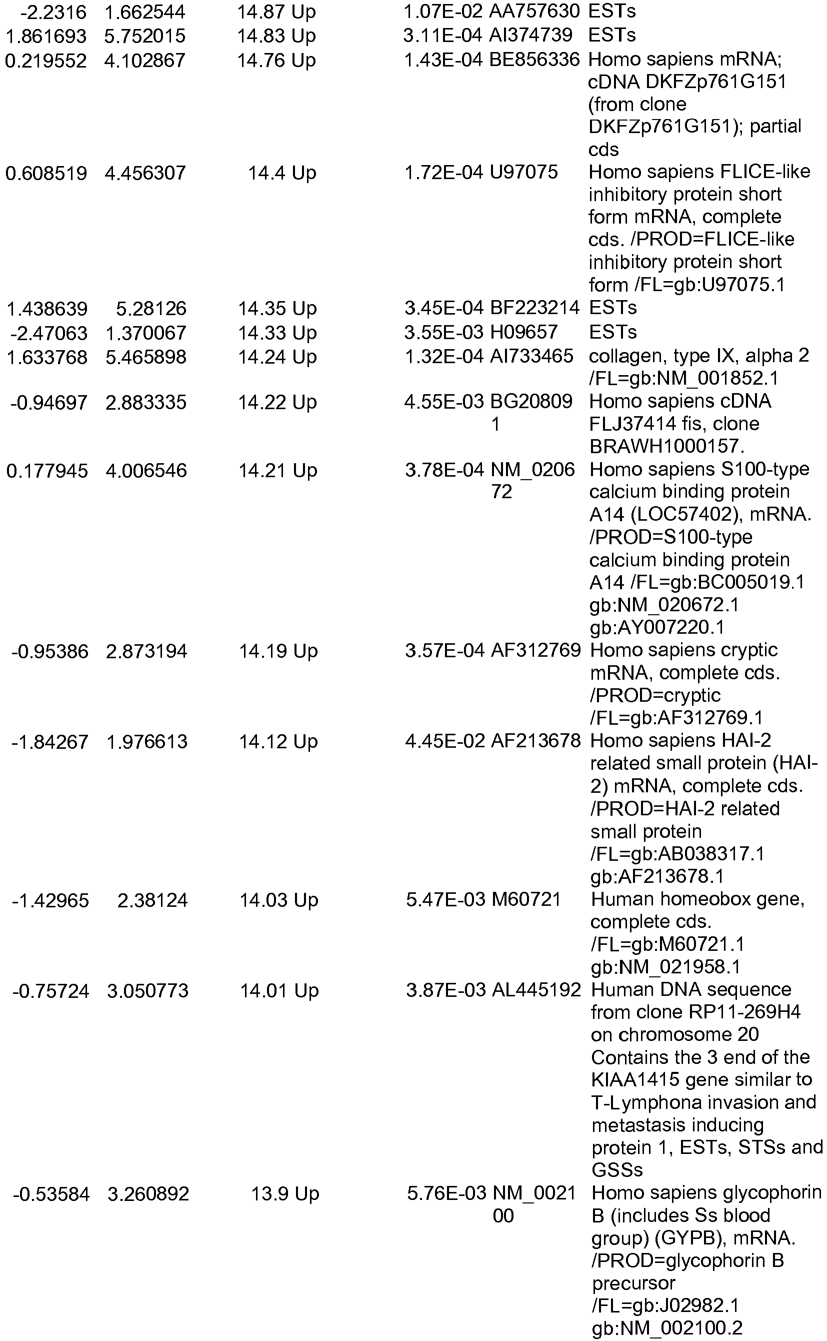

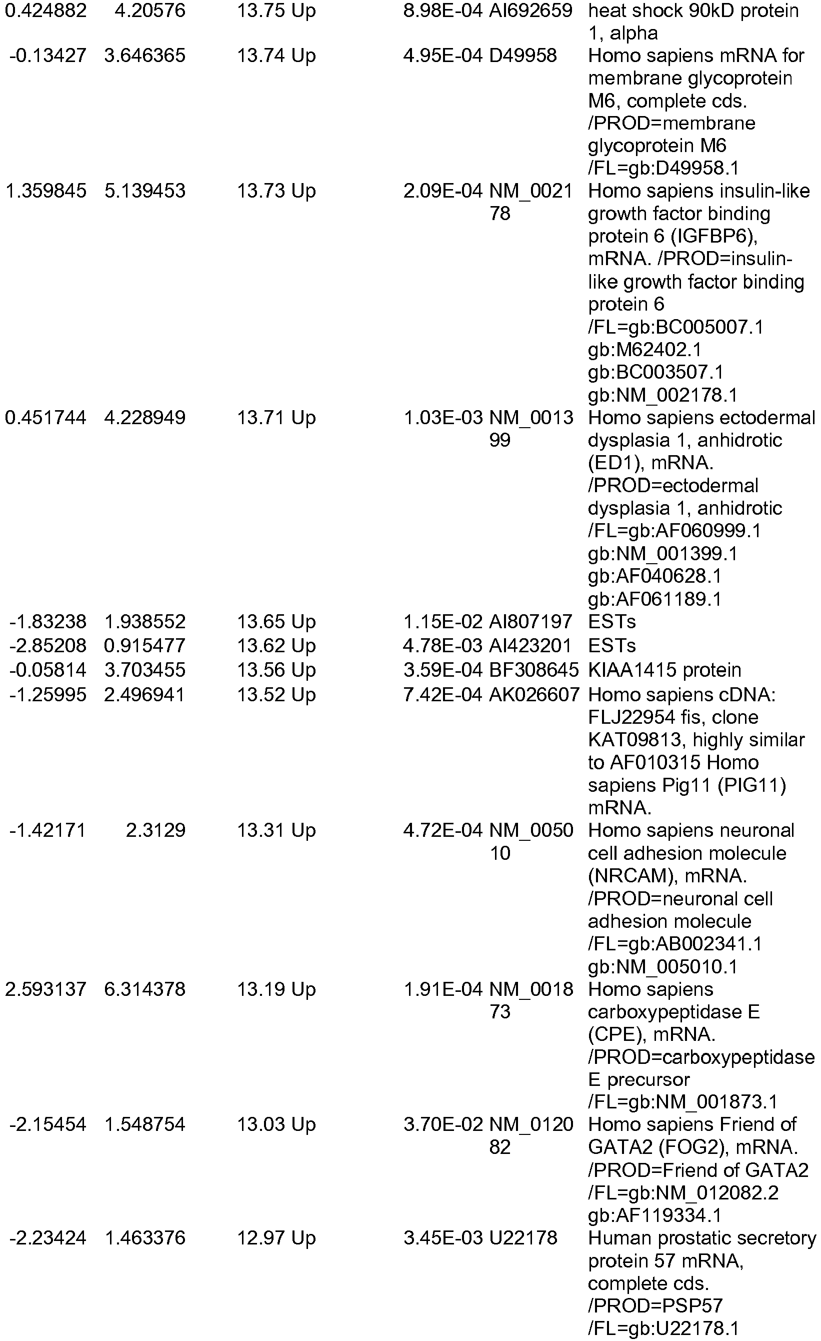

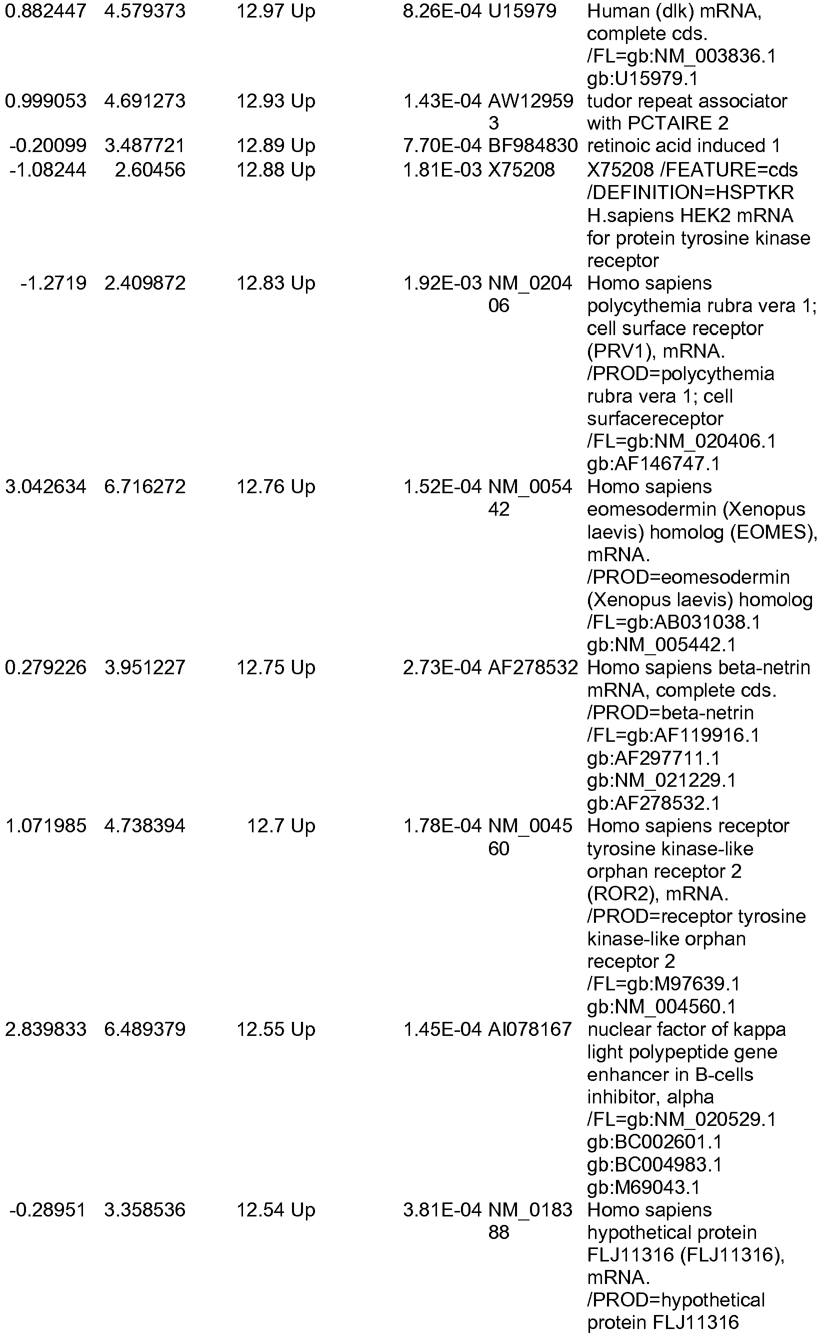

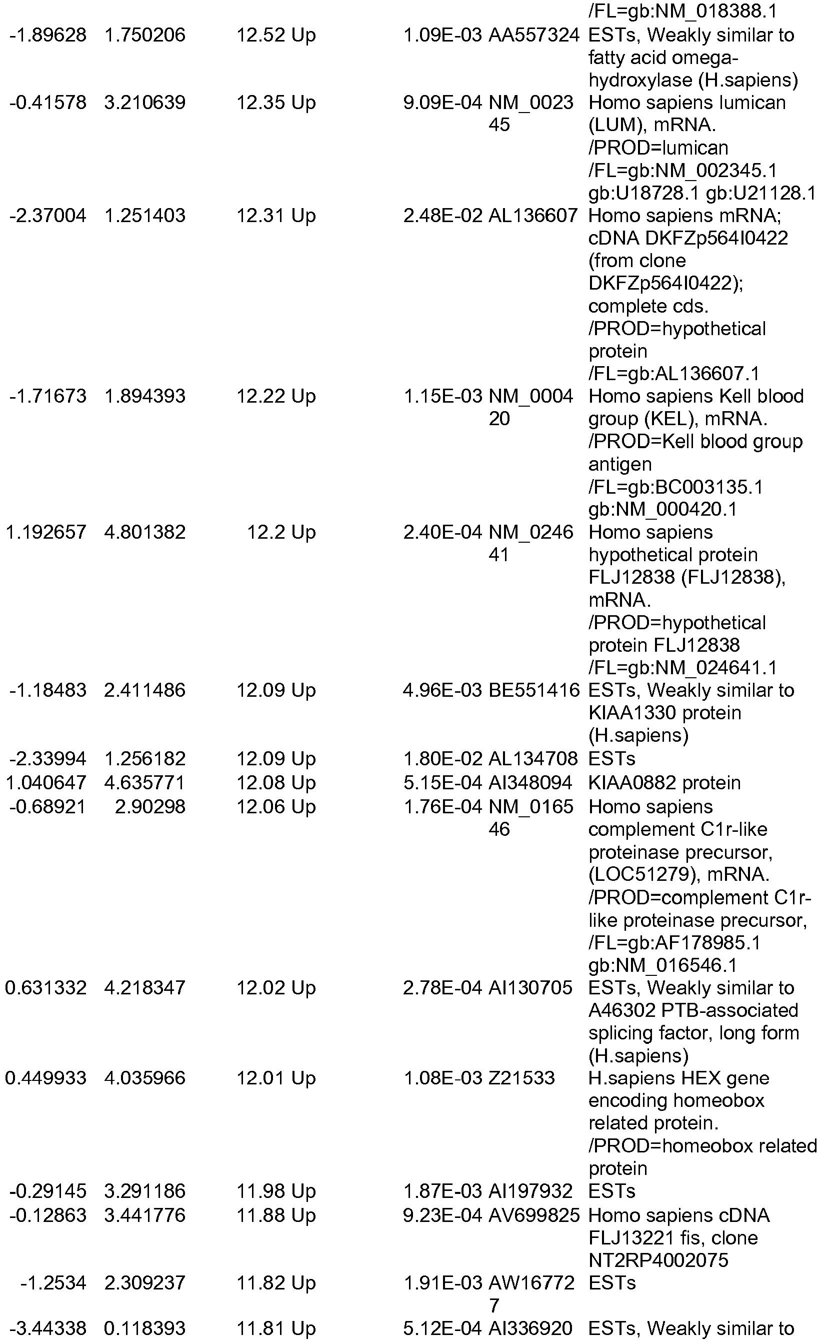

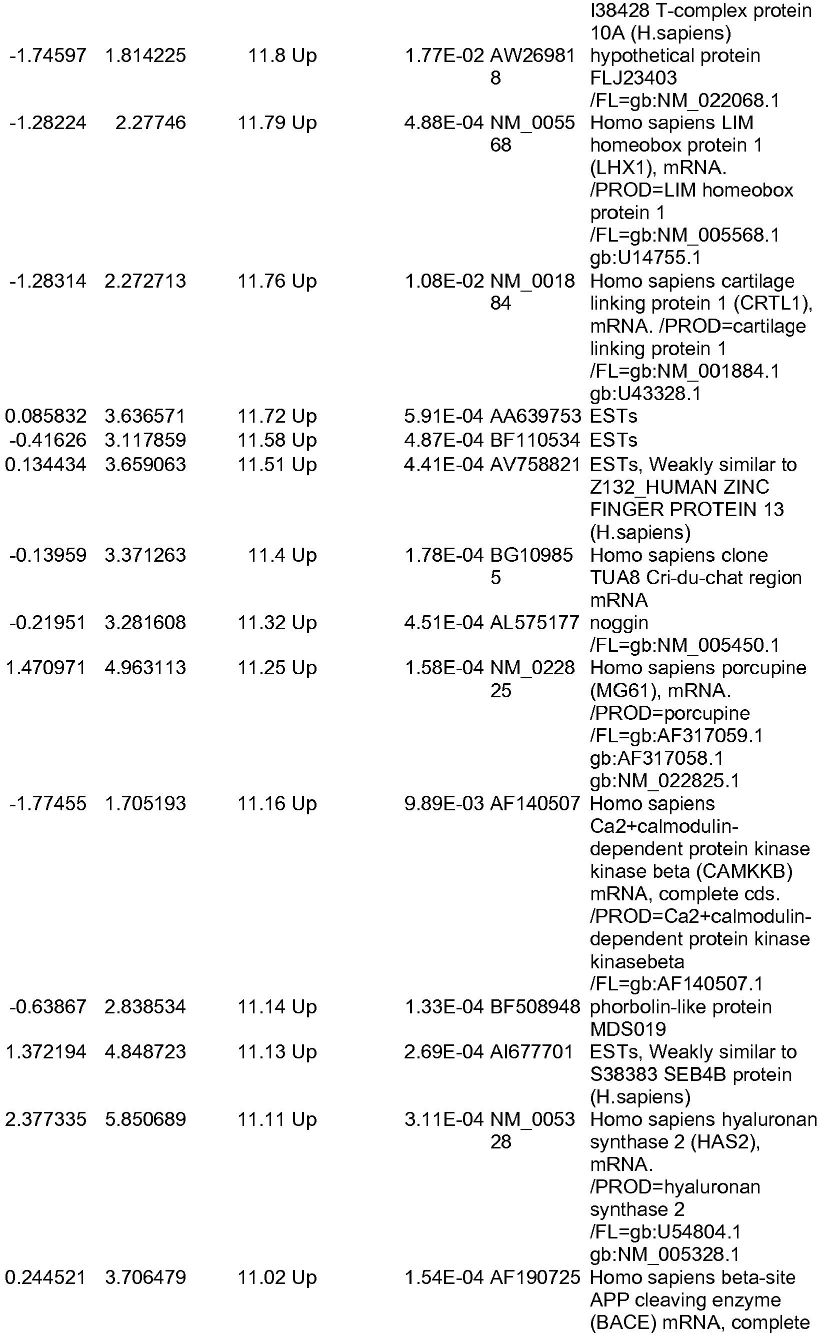

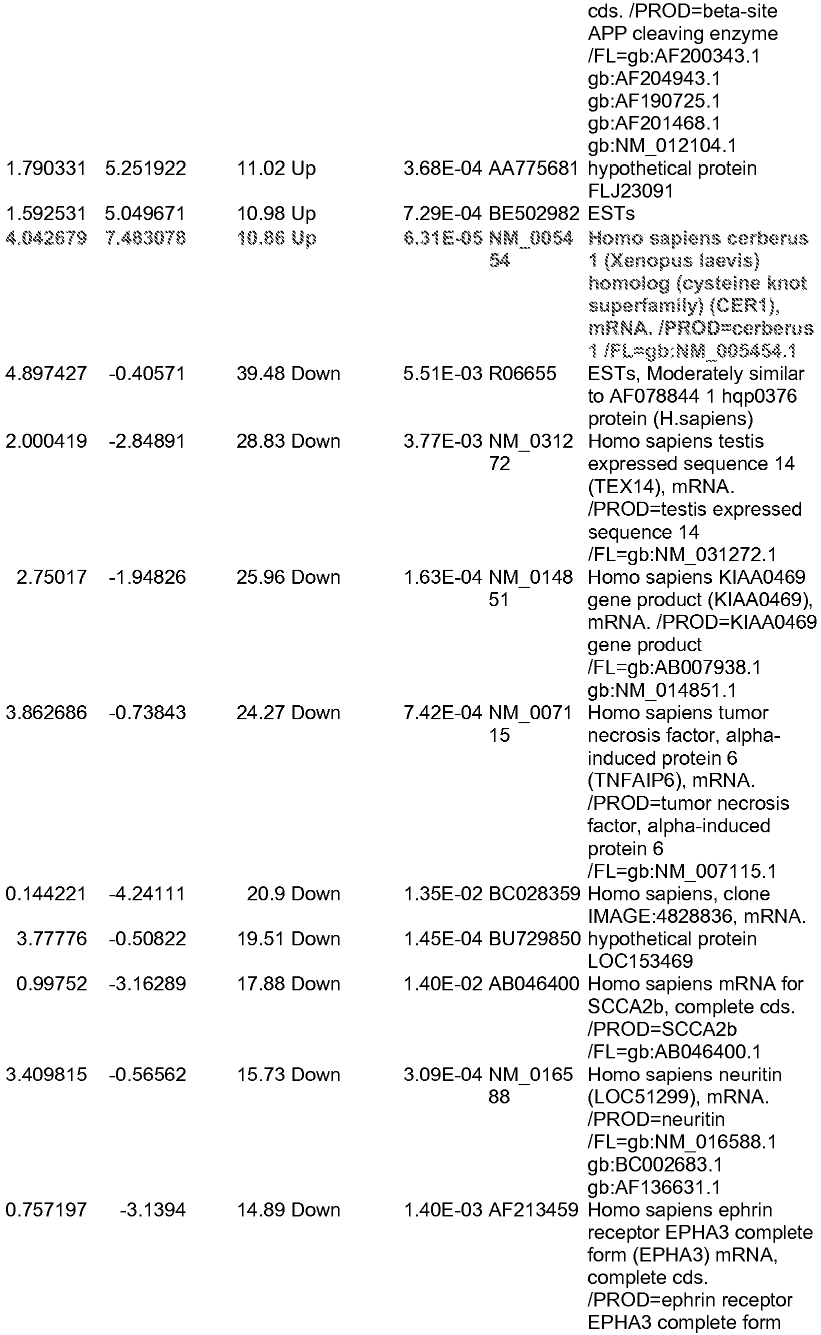

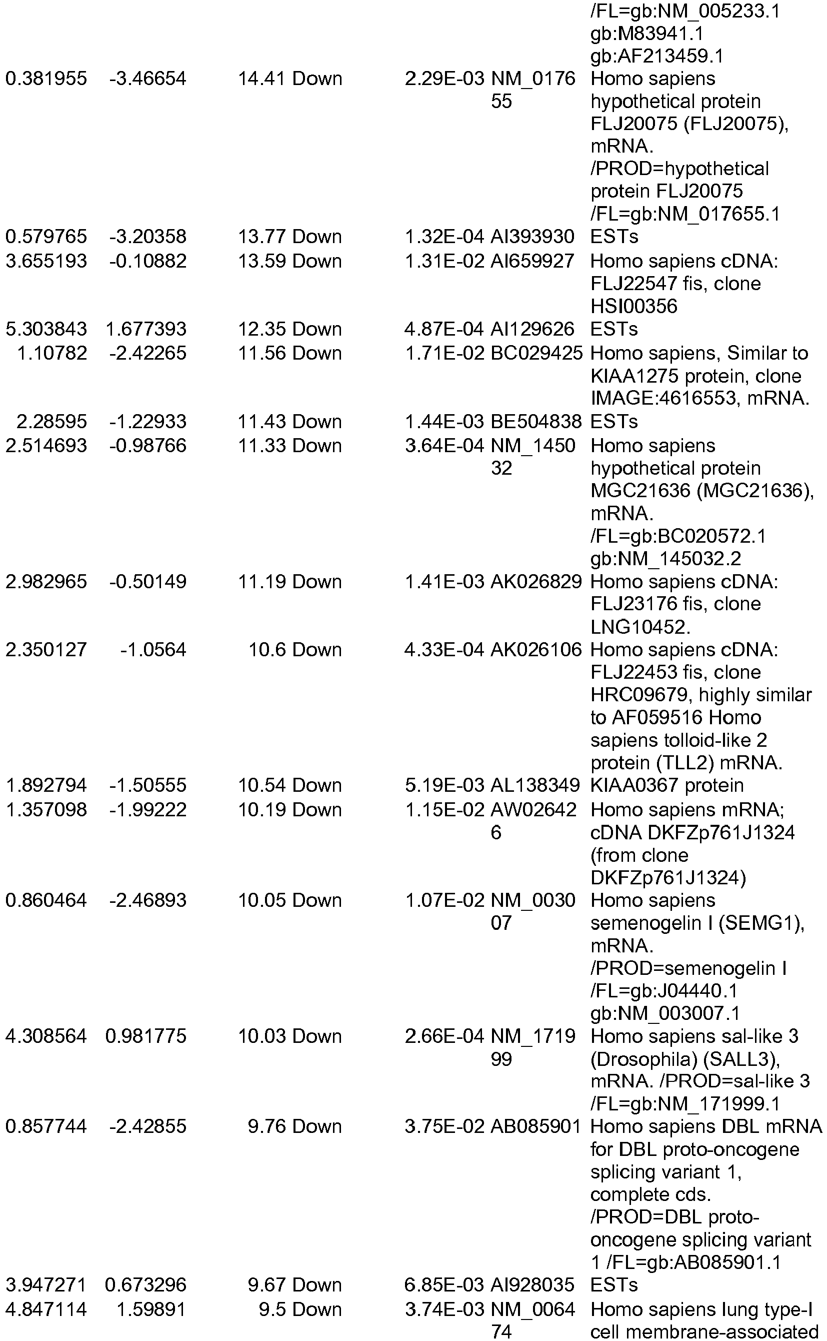

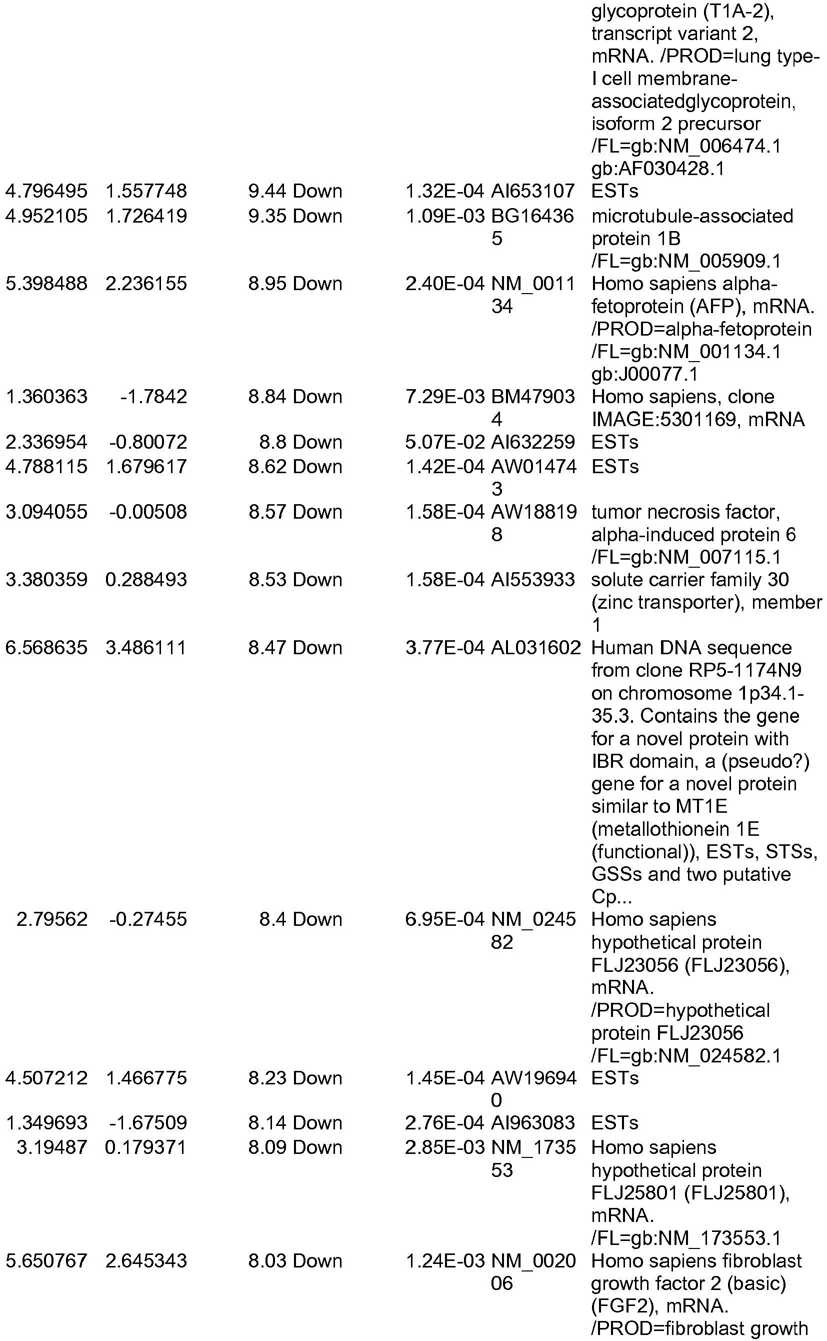

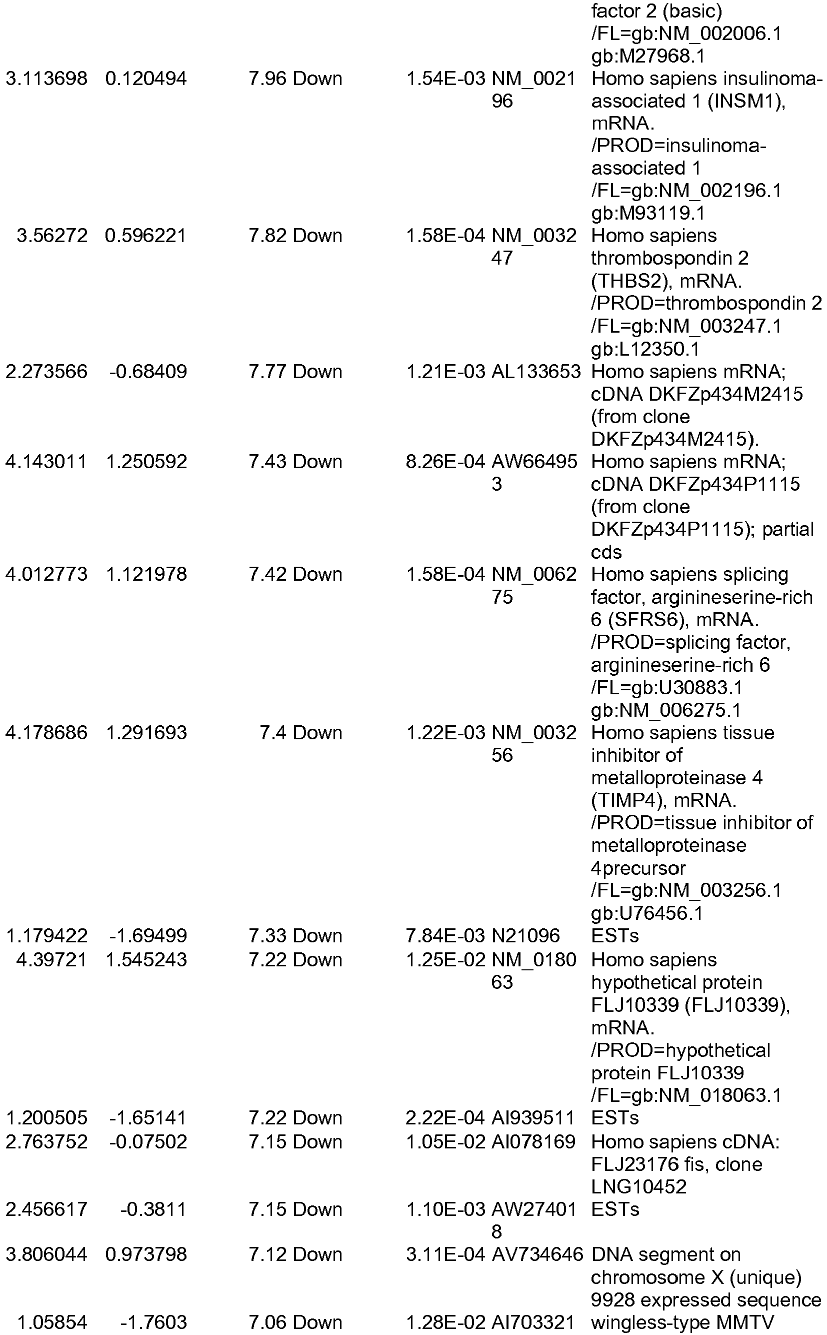

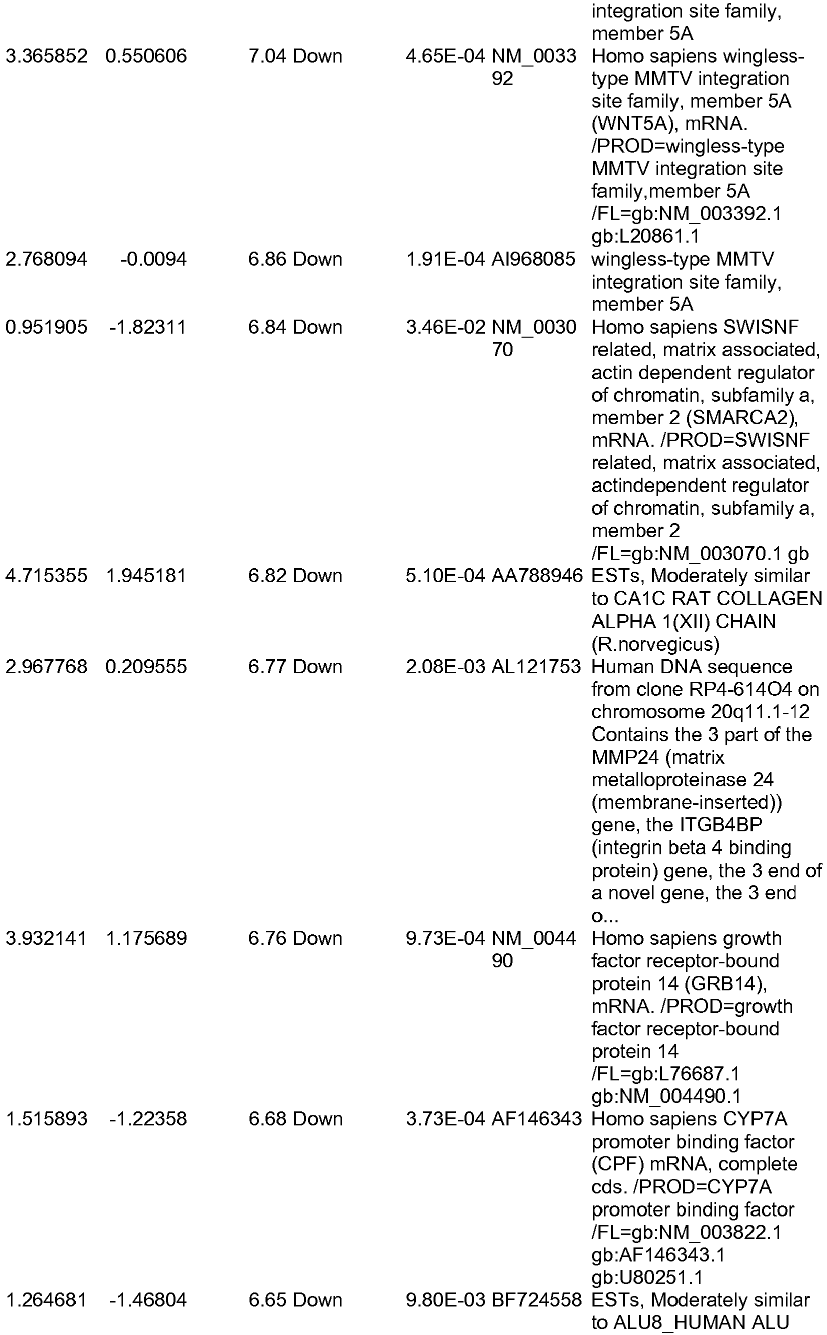

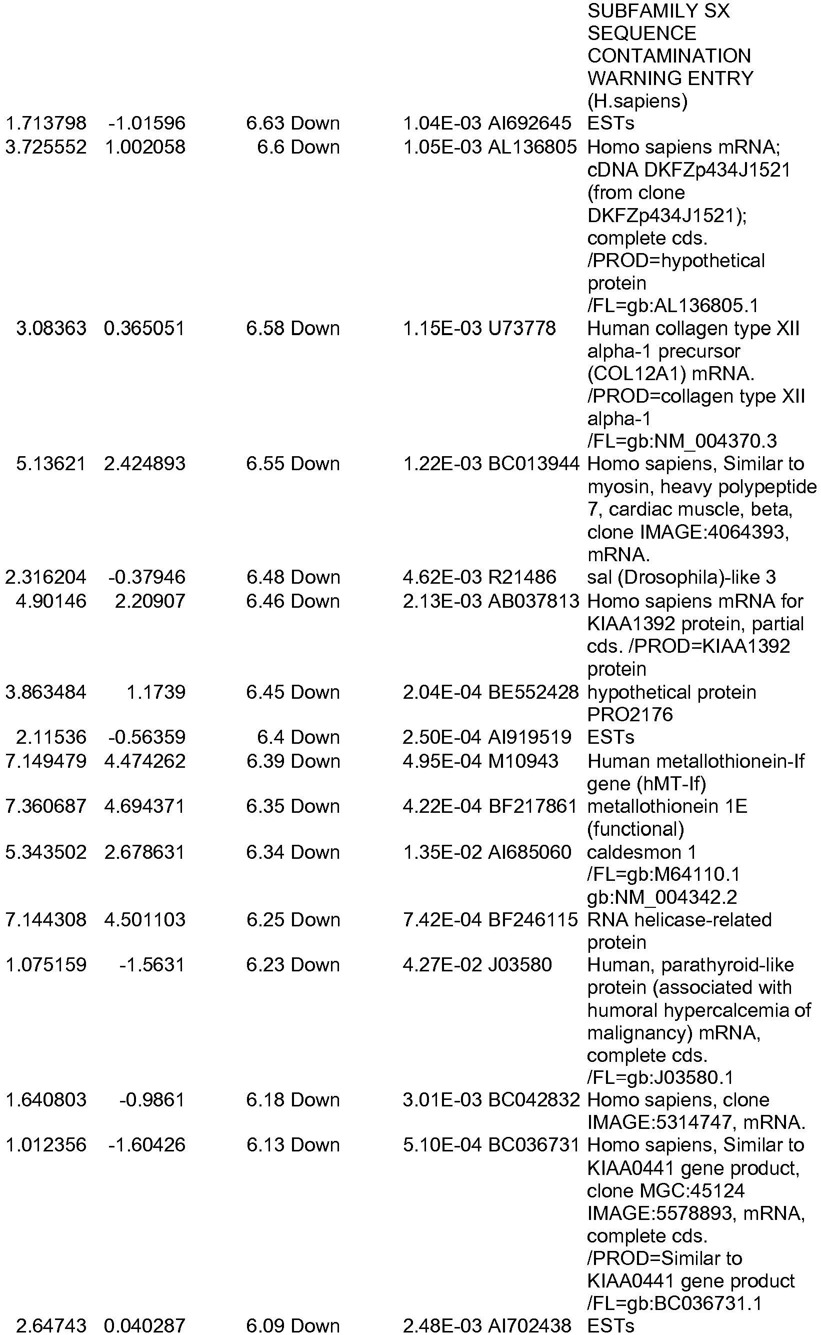

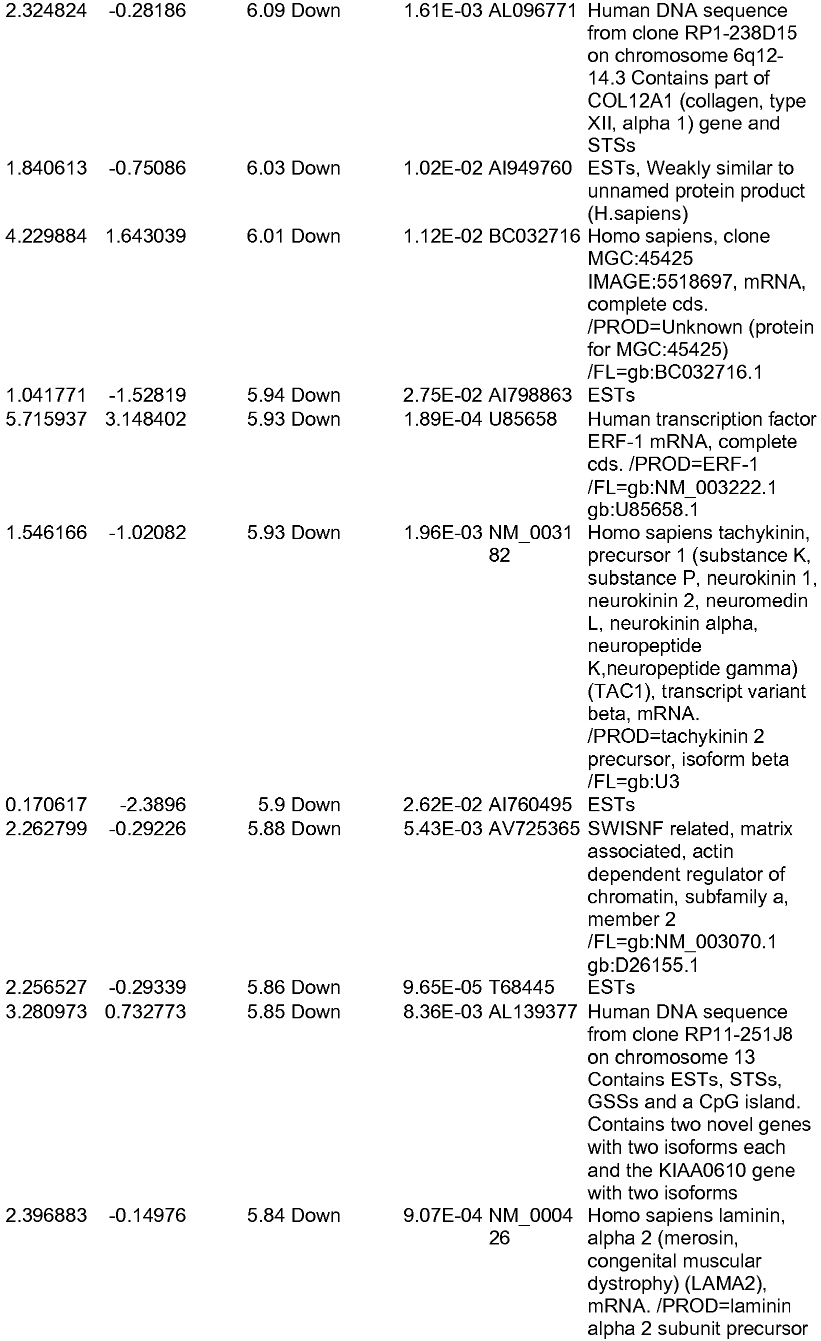

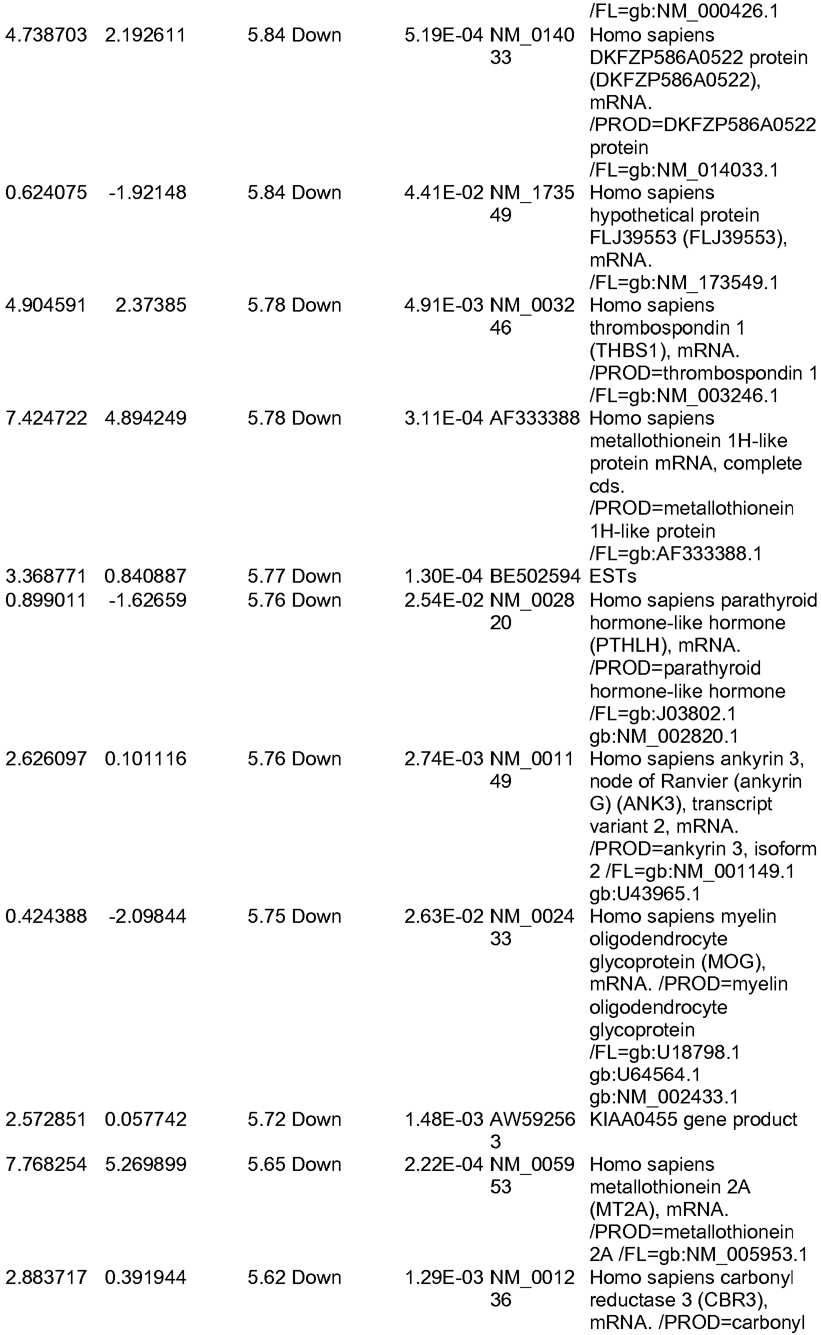

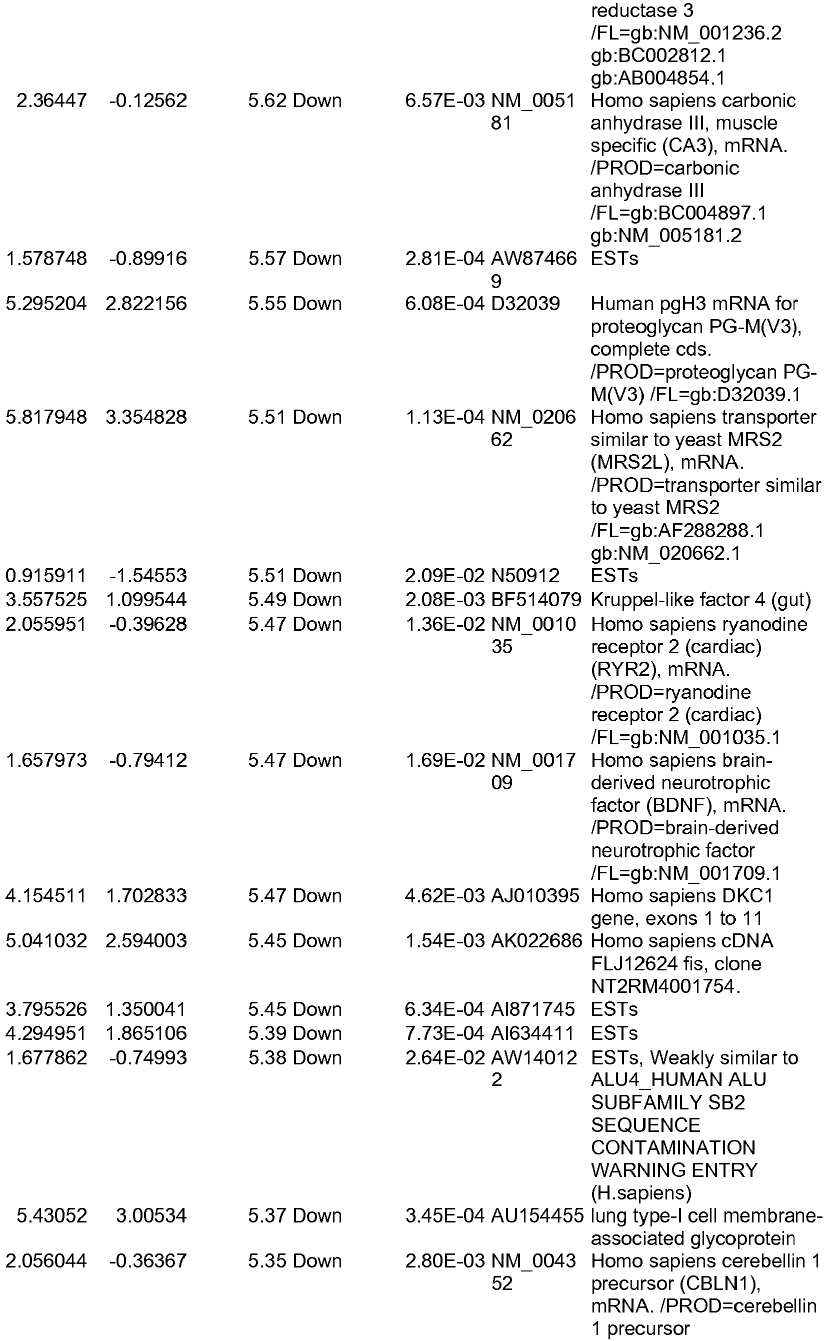

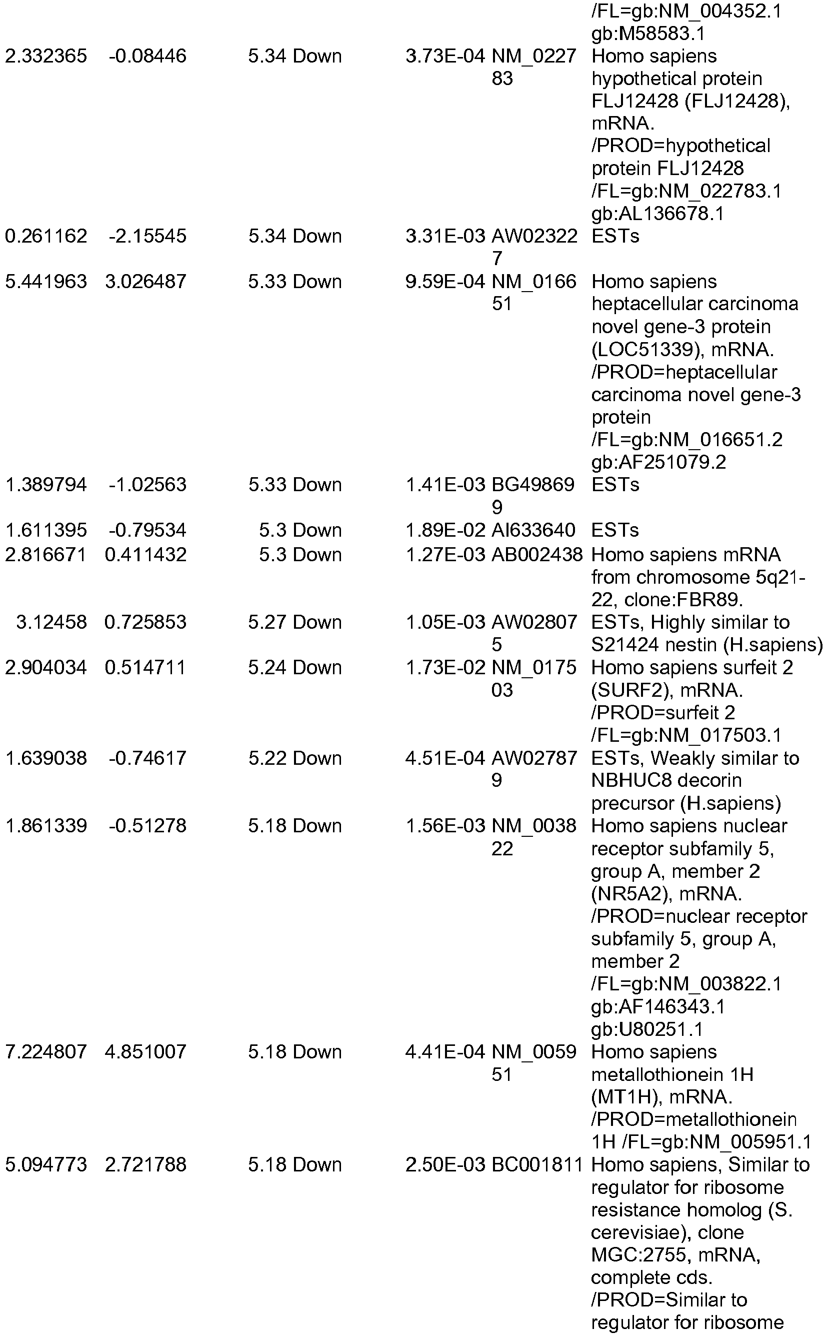

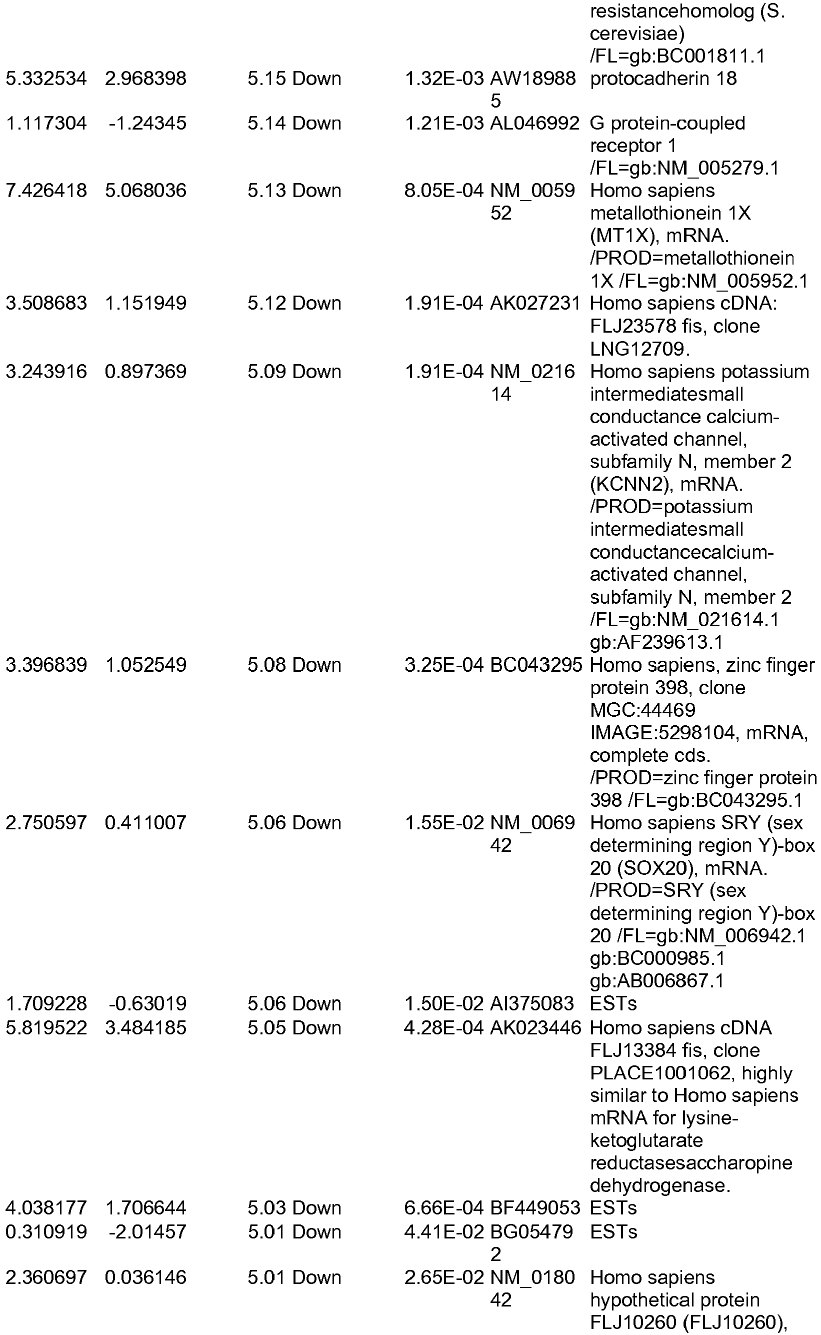

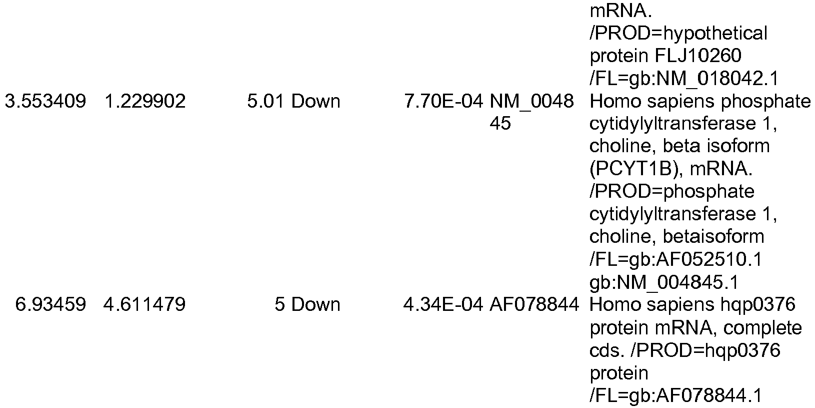

**Table VII- Correlation coefficient between differentiated ES cells, EXPRES 01, EXPRES 02 and various time points during differentiation to DE**

| | ES | EXPRES 02 | EXPRES 01 |
|---|---|---|---|
| EXPRES 02 | 0.882 | | |
| EXPRES 01 | 0.906 | 0.912 | |
| 2 hr DE | 0.914 | 0.895 | 0.922 |
| 6 hr DE | 0.904 | 0.887 | 0.915 |
| 24hr DE | 0.912 | 0.898 | 0.924 |
| 30hr DE | 0.91 | 0.902 | 0.926 |
| 48hr DE | 0.906 | 0.913 | 0.921 |
| 72hr DE | 0.896 | 0.918 | 0.91 |
| 96hr DE | 0.895 | 0.919 | 0.912 |
| 5 days DE | 0.883 | 0.925 | 0.904 |

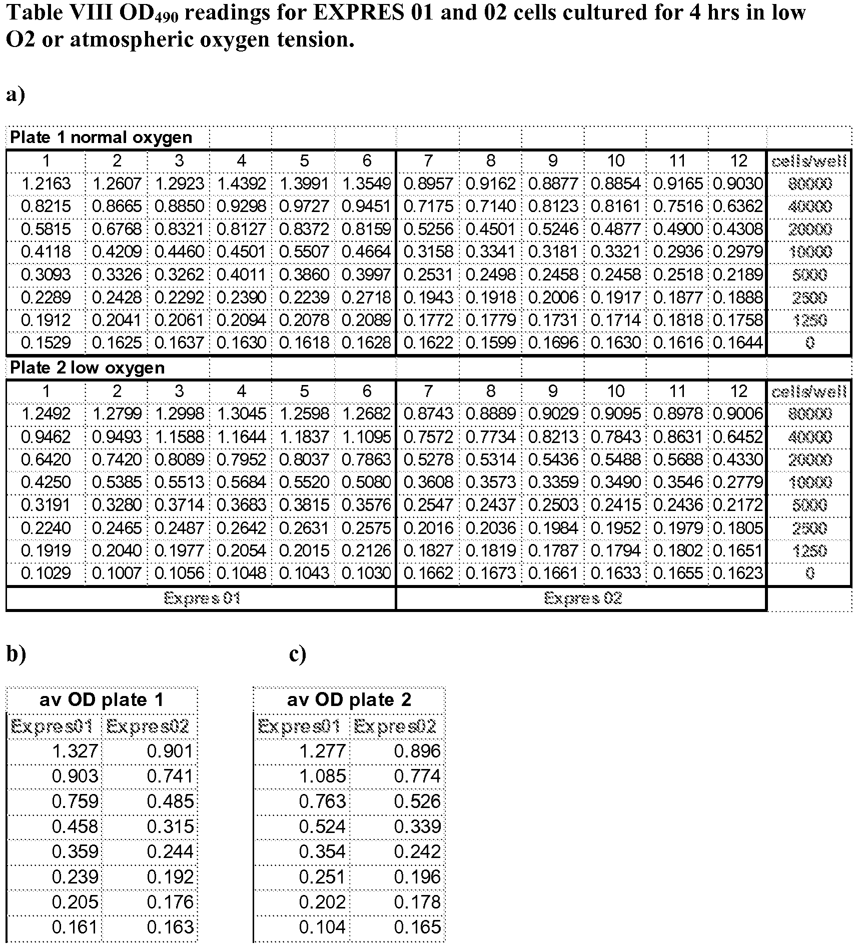

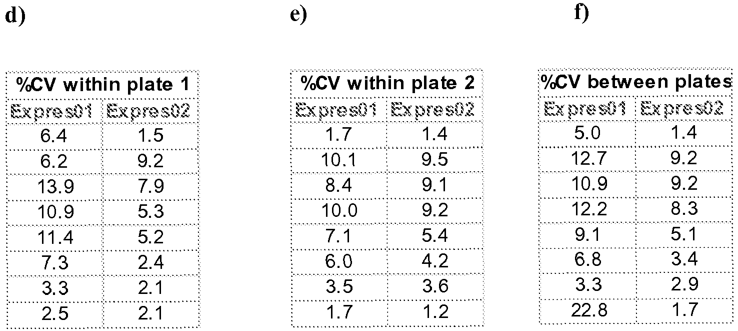

**Table IX Expression of cytokines, growth factors, and receptors as measured by protein arrays**

| **a)** | | | | |
|---|---|---|---|---|
| | **EXPRES 01 Sample #1** | **EXPRES 01 Sample #2** | **EXPRES 02 Sample #1** | **EXPRES 02 Sample #2** |
| **POS** | 54,220 | 54,220 | 54,220 | 54,220 |
| **NEG** | 0 | 0 | 0 | 0 |
| **Angiogenin** | 1,049 | 957 | 2,098 | 1,494 |
| **BDNF** | 461 | 383 | 165 | 219 |
| **BLC** | 173 | 182 | 0 | 206 |
| **BMP-4** | 0 | 0 | 0 | 29 |
| **BMP-6** | 0 | 0 | 0 | 114 |
| **CK beta 8-1** | 0 | 0 | 0 | 0 |
| **CNTF** | 751 | 870 | 745 | 654 |
| **EGF** | 38 | 88 | 118 | 137 |
| **Eotaxin** | 0 | 43 | 0 | 30 |
| **Eotaxin-2** | 0 | 0 | 36 | 44 |
| **Eotaxin-3** | 0 | 0 | 0 | 0 |
| **FGF-6** | 0 | 0 | 0 | 0 |
| **FGF-7** | 122 | 12 | 55 | 62 |
| **Flt-3 Ligand** | 74 | 39 | 33 | 6 |
| **Fractalkine** | 468 | 376 | 457 | 341 |
| **GCP-2** | 327 | 209 | 31 | 11 |
| **GDNF** | 0 | 0 | 0 | 0 |
| **GM-CSF** | 885 | 847 | 655 | 716 |
| **I-309** | 0 | 8 | 0 | 0 |
| **IFN-gamma** | 792 | 830 | 702 | 841 |
| **IGFBP-1** | 199 | 142 | 62 | 99 |
| **IGFBP-2** | 1,262 | 1,408 | 846 | 867 |
| **IGFBP-4** | 0 | 0 | 56 | 98 |
| **IGF-I** | 1,437 | 1,368 | 1,880 | 1,597 |
| **IL-10** | 111 | 125 | 95 | 48 |
| **IL-13** | 470 | 426 | 348 | 445 |
| **IL-15** | 758 | 633 | 631 | 553 |
| **IL-16** | 87 | 0 | 0 | 0 |
| **IL-1alpha** | 1,274 | 1,022 | 1,053 | 1,055 |
| **IL-1beta** | 219 | 53 | 0 | 0 |
| **IL-1ra** | 66 | 103 | 0 | 0 |
| **IL-2** | 934 | 946 | 815 | 829 |
| **IL-3** | 825 | 930 | 819 | 904 |
| **IL-4** | 212 | 230 | 213 | 304 |
| **IL-5** | 959 | 939 | 851 | 849 |
| **IL-6** | 930 | 891 | 994 | 975 |
| **IL-7** | 1,214 | 1,013 | 1,005 | 1,074 |
| **Leptin** | 0 | 0 | 0 | 0 |
| **LIGHT** | 0 | 0 | 0 | 0 |
| **MCP-1** | 1,611 | 1,632 | 6,146 | 4,338 |
| **MCP-2** | 225 | 203 | 212 | 257 |
| **MCP-3** | 463 | 445 | 364 | 248 |
| **MCP-4** | 228 | 170 | 244 | 149 |
| **M-CSF** | 267 | 342 | 307 | 282 |
| **MDC** | 218 | 299 | 140 | 252 |
| **MIG** | 898 | 863 | 789 | 976 |
| **MIP-1-delta** | 107 | 236 | 145 | 281 |
| **MIP-3-alpha** | 386 | 518 | 472 | 495 |
| **NAP-2** | 92 | 159 | 91 | 67 |
| **NT-3** | 0 | 0 | 0 | 0 |
| **PARC** | 90 | 77 | 69 | 230 |
| **PDGF-BB** | 2,623 | 1,755 | 467 | 583 |
| **RANTES** | 0 | 0 | 127 | 102 |
| **SCF** | 0 | 0 | 0 | 0 |
| **SDF-1** | 532 | 493 | 168 | 290 |
| **TARC** | 0 | 0 | 0 | 0 |
| **TGF-beta 1** | 716 | 788 | 806 | 840 |
| **TGF-beta 3** | 73 | 111 | 0 | 16 |
| **TNF-alpha** | 748 | 825 | 760 | 908 |
| **TNF-beta** | 269 | 316 | 247 | 307 |

| **b)** | | | | |
|---|---|---|---|---|
| | **EXPRES 01 Sample #1** | **EXPRES 01 Sample #2** | **EXPRES 02 Sample #1** | **EXPRES 02 Sample #2** |
| **POS** | 43,203.88 | 39,506.01 | 36,640.81 | 42,748.84 |
| **NEG** | 0.00 | 0.00 | 0.00 | 0.00 |
| **Activin A** | 1,044.38 | 797.16 | 429.05 | 408.73 |
| **ALCAM** | 926.38 | 812.41 | 1,132.59 | 1,111.92 |
| **B7-1(CD80)** | 164.38 | 193.08 | 183.69 | 149.23 |
| **BMP-5** | 62.38 | 39.17 | 65.41 | 67.20 |
| **BMP-7** | 203.88 | 165.35 | 203.92 | 235.90 |
| **Cardiotrophin-1** | 420.38 | 390.43 | 330.99 | 529.97 |
| **CD14** | 551.38 | 581.78 | 449.28 | 504.69 |
| **CXCL- 16** | 144.38 | 126.52 | 114.65 | 144.58 |
| **DR6 (TNFRSF21)** | 317.88 | 278.12 | 254.92 | 294.20 |
| **Endoglin** | 769.88 | 610.44 | 824.79 | 738.91 |
| **ErbB3** | 453.88 | 383.50 | 352.10 | 276.14 |
| **E-Selectin** | 175.88 | 145.47 | 158.63 | 204.43 |
| **Fas Ligand** | 325.38 | 272.11 | 290.98 | 421.63 |
| **ICAM-2** | 165.88 | 105.73 | 97.51 | 244.15 |
| **IGF-II** | 8,897.88 | 9,437.80 | 8,026.40 | 5,317.62 |
| **IL-1 R II** | 92.38 | 2.66 | 28.91 | 131.69 |
| **IL-10 Rbeta** | 156.88 | 65.05 | 65.41 | 242.09 |
| **IL-13 Ralpha2** | 362.88 | 140.39 | 82.12 | 312.77 |
| **IL-18 BPalpha** | 334.88 | 267.49 | 415.86 | 535.13 |
| **IL-18 Rbeta** | 161.38 | 97.87 | 152.47 | 127.04 |
| **IL-2 Ralpha** | 0.88 | 0.00 | 30.23 | 0.00 |
| **IL-2 Rbeta** | 0.00 | 2.20 | 19.24 | 0.00 |
| **IL-2 Rgamma** | 0.00 | 0.00 | 0.00 | 0.00 |
| **IL-21R** | 452.38 | 433.42 | 399.15 | 458.26 |
| **IL-5 Ralpha** | 446.88 | 302.62 | 397.83 | 297.81 |
| **IL-9** | 340.38 | 342.83 | 454.55 | 325.67 |
| **IP-10** | 364.88 | 310.01 | 295.82 | 382.42 |
| **LAP** | 1,194.38 | 1,087.42 | 3,883.43 | 4,764.05 |
| **Leptin R** | 272.38 | 200.47 | 179.29 | 286.46 |
| **LIF** | 326.38 | 89.09 | 74.64 | 180.18 |
| **L-Selectin** | 83.38 | 55.35 | 29.79 | 97.12 |
| **M-CSF R** | 60.38 | 0.00 | 21.00 | 0.00 |
| **MMP-1** | 12.88 | 36.86 | 23.63 | 62.55 |
| **MMP-13** | 158.38 | 25.30 | 54.85 | 144.58 |
| **MMP-9** | 504.38 | 450.98 | 282.19 | 257.05 |
| **MPIF-1** | 0.00 | 16.99 | 50.46 | 30.57 |
| **NGF R** | 30.38 | 43.79 | 29.35 | 6.84 |
| **PDGF AA** | 442.38 | 454.68 | 436.52 | 356.62 |
| **PDGF-AB** | 507.38 | 459.76 | 124.33 | 187.40 |
| **PDGF Ralpha** | 278.88 | 228.21 | 254.92 | 207.01 |
| **PDGF Rbeta** | 483.38 | 379.34 | 413.66 | 465.48 |
| **PECAM-1** | 199.38 | 148.25 | 157.75 | 181.21 |
| **Prolactin** | 312.88 | 218.04 | 184.13 | 182.25 |
| **SCF R** | 472.88 | 353.92 | 454.11 | 483.02 |
| **SDF-1beta** | 1,047.88 | 765.73 | 382.44 | 469.09 |
| **Siglec-5** | 0.00 | 0.00 | 0.00 | 0.00 |
| **TGF-alpha** | 113.88 | 123.29 | 50.90 | 55.33 |
| **TGF beta2** | 68.38 | 50.73 | 14.84 | 53.27 |
| **Tie-1** | 291.38 | 218.96 | 165.22 | 198.24 |
| **Tie-2** | 57.88 | 53.50 | 119.49 | 94.02 |
| **TIMP-4** | 166.88 | 143.16 | 109.38 | 180.70 |
| **VE-Cadherin** | 432.38 | 329.89 | 1,175.24 | 208.04 |
| **VEGF R2** | 510.88 | 367.33 | 437.40 | 801.34 |
| **VEGF R3** | 41.38 | 38.25 | 20.12 | 0.00 |
| **Internal Control** | 23,824.38 | 23,824.38 | 23,824.38 | 23,824.38 |

| **c)** | | | | |
|---|---|---|---|---|
| | **EXPRES 01 Sample #1** | **EXPRES 01 Sample #2** | **EXPRES 02 Sample #1** | **EXPRES 02 Sample #2** |
| **POS** | 55,531.00 | 63,702.50 | 55,954.13 | 76,525.06 |
| **NEG** | 0.00 | 0.00 | 0.00 | 0.00 |
| **Acrp30** | 140.00 | 100.45 | 119.63 | 179.61 |
| **AgRP** | 221.50 | 239.23 | 261.80 | 443.17 |
| **Angiopoietin-2** | 0.00 | 0.00 | 58.85 | 104.61 |
| **Amphiregulin** | 0.00 | 0.00 | 0.00 | 0.00 |
| **Axl** | 210.00 | 351.20 | 368.94 | 523.07 |
| **bFGF** | 64,959.00 | 75,788.69 | 57,182.11 | 65,024.38 |
| **b-NGF** | 0.00 | 0.00 | 0.00 | 0.00 |
| **BTC** | 0.00 | 0.00 | 0.00 | 0.00 |
| **CCL-28** | 455.50 | 399.01 | 258.71 | 415.13 |
| **CTACK** | 57.00 | 48.55 | 70.18 | 12.09 |
| **Dtk** | 0.00 | 0.00 | 0.00 | 0.00 |
| **EGF-R** | 83.50 | 92.28 | 30.52 | 33.82 |
| **ENA-78** | 726.00 | 842.78 | 781.52 | 939.43 |
| **Fas/TNFRSF6** | 374.00 | 460.83 | 352.97 | 450.17 |
| **FGF-4** | 232.50 | 130.77 | 110.36 | 218.17 |
| **FGF-9** | 271.00 | 520.31 | 295.28 | 460.69 |
| **GCSF** | 688.00 | 895.85 | 223.68 | 973.77 |
| **GITR-Ligand** | 0.00 | 0.00 | 0.00 | 0.00 |
| **GITR** | 242.00 | 109.19 | 140.75 | 167.00 |
| **GRO** | 1,380.00 | 1,572.29 | 929.87 | 1,270.97 |
| **GRO-alpha** | 962.00 | 1,043.39 | 920.08 | 1,080.31 |
| **HCC-4** | 162.50 | 108.03 | 194.83 | 217.46 |
| **HGF** | 0.00 | 0.00 | 0.00 | 0.00 |
| **ICAM-1** | 752.00 | 848.62 | 571.88 | 614.89 |
| **ICAM-3** | 0.00 | 0.00 | 0.00 | 0.00 |
| **IGFBP-3** | 169.00 | 439.25 | 190.20 | 372.37 |
| **IGFBP-6** | 314.50 | 285.89 | 245.31 | 368.17 |
| **IGF-I SR** | 236.00 | 314.46 | 133.54 | 237.09 |
| **IL-1 R4/ST2** | 226.00 | 354.11 | 283.43 | 373.77 |
| **IL-1 RI** | 0.00 | 32.80 | 0.00 | 92.70 |
| **IL-11** | 187.50 | 178.59 | 135.60 | 299.47 |
| **IL-12 p40** | 174.00 | 130.19 | 91.82 | 227.28 |
| **IL-12 p70** | 163.00 | 198.41 | 140.23 | 245.50 |
| **IL-17** | 120.00 | 114.44 | 72.24 | 164.89 |
| **IL-2 Rapha** | 434.50 | 488.24 | 422.50 | 553.21 |
| **IL-6 R** | 163.00 | 114.44 | 327.73 | 144.57 |
| **IL-8** | 741.00 | 782.72 | 694.47 | 1,053.68 |
| **I-TAC** | 721.50 | 763.48 | 551.28 | 754.38 |
| **Lymphotactin** | 177.00 | 68.37 | 93.88 | 135.46 |
| **MIF** | 2,703.00 | 2,577.05 | 2,491.12 | 3,008.58 |
| **MIP-1alpha** | 251.00 | 257.89 | 236.56 | 341.53 |
| **MIP-1beta** | 15.50 | 0.00 | 14.55 | 56.95 |
| **MIP-3beta** | 242.50 | 233.99 | 184.02 | 279.15 |
| **MSP-alpha** | 376.50 | 303.38 | 250.98 | 408.12 |
| **NT-4** | 37.00 | 101.03 | 108.81 | 253.21 |
| **Osteoprotegerin** | 380.50 | 390.27 | 432.29 | 696.90 |
| **Oncostatin M** | 466.50 | 394.35 | 361.21 | 504.15 |
| **PIGF** | 180.50 | 198.41 | 195.86 | 220.27 |
| **sgp130** | 18.00 | 36.30 | 203.08 | 209.05 |
| **sTNF RII** | 0.00 | 0.00 | 0.00 | 0.00 |
| **sTNF-RI** | 11.50 | 0.00 | 694.47 | 267.93 |
| **TECK** | 0.00 | 0.00 | 0.00 | 0.00 |
| **TIMP-1** | 175.00 | 123.19 | 172.17 | 223.77 |
| **TIMP-2** | 1,718.50 | 2,953.76 | 487.41 | 502.74 |
| **Thrombopoietin** | 256.50 | 231.65 | 227.80 | 256.02 |
| **TRAIL R3** | 0.00 | 0.00 | 127.36 | 94.10 |
| **TRAIL R4** | 239.00 | 273.64 | 277.25 | 370.27 |
| **uPAR** | 104.50 | 84.12 | 103.66 | 319.10 |
| **VEGF** | 311.00 | 289.97 | 305.06 | 523.77 |
| **VEGF-D** | 0.00 | 0.00 | 0.00 | 0.00 |
| **Internal Control** | 18,664.17 | 18,664.17 | 18,664.17 | 18,664.17 |

## Claims

1. A method for deriving a population of cells comprising cells expressing pluripotency markers comprising the steps of:
a. culturing human embryonic stem cells, and
b. removing the cultured human embryonic stem cells, and subsequently culturing the removed cultured human embryonic stem cells under hypoxic conditions, on a tissue culture substrate that is not pre-treated with a protein or an extracellular matrix prior to culturing the cells, in culture medium contains serum, activin-A, a Wnt ligand, and optionally IGF-1
wherein the cells expressing pluripotency markers express at least one of the pluripotency markers selected from the group consisting of ABCG2, cripto, FoxD3, Connexin43, Connexin45, Oct4, SOX-2, Nanog, hTERT, UTF-I, ZFP42, SSEA-3, SSEA-4, Tral-60, and Tral-81,
and wherein the cells expressing pluripotency markers express markers characteristic of:
pre-primitive streak cells; or
primitive streak cells; or
mesoendoderm cells.

2. The method of claim 1, wherein the human embryonic stem cells are treated with a Rho kinase inhibitor.

3. The method of claim 1, wherein the human embryonic stem cells are cultured on an extracellular matrix prior to removing the cells.

4. The method of claim 1, wherein the human embryonic stem cells are cultured on a feeder cell layer prior to removing the cells.

5. The method of claim 1, wherein the human embryonic stem cells are cultured in normoxic conditions prior to removing the cells.

6. The method of claim 1, wherein the human embryonic stem cells are cultured in hypoxic conditions prior to removing the cells.

7. The method of claim 1, wherein the hypoxic conditions are an O₂ level from 1% to 20%.

8. The method of claim 1, wherein the hypoxic conditions are an O₂ level from 2% to 10%.

9. The method of claim 1, wherein the hypoxic conditions are an O₂ level of 3%.

10. The method of claim 1, wherein the cells are cultured under hypoxic conditions, on a tissue culture substrate that is not pre-treated with a protein or an extracellular matrix, in medium containing serum at a concentration from 2% to 5%.

11. The method of claim 1, wherein the cells are cultured under hypoxic conditions, on a tissue culture substrate that is not pre-treated with a protein or an extracellular matrix, in medium containing serum at a concentration of 2%.

12. The method of claim 1, wherein the cells are cultured under hypoxic conditions, on a tissue culture substrate that is not pre-treated with a protein or an extracellular matrix, in medium containing:
IGF-1; or
activin-A at a concentration from 50ng/ml to 100ng/m; or
activin-A at a concentration of 100ng/ml; or
a Wnt ligand at a concentration from 10ng/ml to 20ng/ml; or
a Wnt ligand at a concentration of 200ng/ml; or
IGF-1 at a concentration from 25ng/ml to 50ng/ml; or
IGF-1 at a concentration of 50ng/ml.

13. The method of claim 1, wherein the Wnt ligand is Wnt-3a.

14. The method of claim 1, wherein the cells expressing pluripotency markers express markers characteristic of:
pre-primitive streak cells and wherein the markers are Nodal or FGF8; or
primitive streak cells and wherein the markers are selected from the group consisting of Barachyury, Mix-like homeobox protein, and FGF4; or
mesoendoderm cells and wherein the markers are selected from the group consisting of CD48, eomesodermin, SOX-17, DKK4, HNF-3 beta, GSC, FGF16, and GATA6.

## Patentansprüche

1. Verfahren zum Ableiten einer Zellpopulation, die Zellen umfasst, die Pluripotenzmarker exprimieren, umfassend die Schritte:
a. Züchten von humanen embryonalen Stammzellen, und
b. Entfernen der gezüchteten humanen embryonalen Stammzellen und anschließend Züchten der entfernten gezüchteten humanen embryonalen Stammzellen unter hypoxischen Bedingungen auf einem Gewebekultursubstrat, das nicht mit einem Protein oder einer extrazellulären Matrix vor dem Züchten der Zellen vorbehandelt worden ist, in einem Kulturmedium, das Serum, Activin-A, einen Wnt-Liganden und gegebenenfalls IGF-1 enthält,
wobei die Zellen, die die Pluripotenzmarker exprimieren, mindestens einen der Pluripotenzmarker exprimieren, die ausgewählt sind aus der Gruppe bestehend aus ABCG2, Cripto, FoxD3, Connexin43, Connexin45, Oct4, SOX-2, Nanog, hTERT, UTF-I, ZFP42, SSEA-3, SSEA-4, Tra1-60 und Tra1-81, und wobei die Zellen, die die Pluripotenzmarker exprimieren, Marker exprimieren, die charakteristisch sind für:
Zellen des Präprimitivstreifens (pre-primitive streak cells); oder
Zellen des Primitivstreifens (primitive streak cells); oder
Mesoendodermzellen.

2. Verfahren nach Anspruch 1, wobei die humanen embryonalen Stammzellen mit einem Rho-Kinase-Inhibitor behandelt werden.

3. Verfahren nach Anspruch 1, wobei die humanen embryonalen Stammzellen auf einer extrazellulären Matrix gezüchtet werden, bevor die Zellen entfernt werden.

4. Verfahren nach Anspruch 1, wobei die humanen embryonalen Stammzellen auf einer Feederzellschicht gezüchtet werden, bevor die Zellen entfernt werden.

5. Verfahren nach Anspruch 1, wobei die humanen embryonalen Stammzellen unter normoxischen Bedingungen gezüchtet werden, bevor die Zellen entfernt werden.

6. Verfahren nach Anspruch 1, wobei die humanen embryonalen Stammzellen unter hypoxischen Bedingungen gezüchtet werden, bevor die Zellen entfernt werden.

7. Verfahren nach Anspruch 1, wobei die hypoxischen Bedingungen ein O₂-Spiegel von 1% bis 20% sind.

8. Verfahren nach Anspruch 1, wobei die hypoxischen Bedingungen ein O₂-Spiegel von 2% bis 10% sind.

9. Verfahren nach Anspruch 1, wobei die hypoxischen Bedingungen ein O₂-Spiegel von 3% sind.

10. Verfahren nach Anspruch 1, wobei die Zellen unter hypoxischen Bedingungen auf einem Gewebekultursubstrat gezüchtet werden, das nicht mit einem Protein oder einer extrazellulären Matrix vorbehandelt wurde, in einem Medium, das Serum mit einer Konzentration von 2% bis 5% enthält.

11. Verfahren nach Anspruch 1, wobei die Zellen unter hypoxischen Bedingungen auf einem Gewebekultursubstrat gezüchtet werden, das nicht mit einem Protein oder einer extrazellulären Matrix vorbehandelt wurde, in einem Medium, das Serum mit einer Konzentration von 2% enthält.

12. Verfahren nach Anspruch 1, wobei die Zellen unter hypoxischen Bedingungen auf einem Gewebekultursubstrat gezüchtet werden, das nicht mit einem Protein oder einer extrazellulären Matrix vorbehandelt wurde, in einem Medium, das enthält:
IGF-1; oder
Activin-A mit einer Konzentration von 50 ng/ml bis 100 ng/ml; oder
Activin-A mit einer Konzentration von 100 ng/ml; oder
einen Wnt-Liganden mit einer Konzentration von 10 ng/ml bis 20 ng/ml;
oder
einen Wnt-Liganden mit einer Konzentration von 200 ng/ml; oder
IGF-1 mit einer Konzentration von 25 ng/ml bis 50 ng/ml; oder
IGF-1 mit einer Konzentration von 50 ng/ml.

13. Verfahren nach Anspruch 1, wobei der Wnt-Ligand Wnt-3a-Ligand ist.

14. Verfahren nach Anspruch 1, wobei die Zellen, die Pluripotenzmarker exprimieren, Marker exprimieren, die charakteristisch sind für:
Zellen des Präprimitivstreifens und wobei die Marker Nodal oder FGF8 sind; oder
Zellen des Primitivstreifens und wobei die Marker ausgewählt sind aus der Gruppe bestehend aus Barachyury, Mix-like Homöobox-Protein und FGF4; oder
Mesoendodermzellen und wobei die Marker ausgewählt sind aus der Gruppe bestehend aus CD48, Eomesodermin, SOX-17, DKK4, HNF-3-Beta, GSC, FGF16 und GATA6.

## Revendications

1. Méthode pour dériver une population de cellules comprenant des cellules exprimant des marqueurs de pluripotence comprenant les étapes de :
a. culture de cellules souches embryonnaires humaines, et
b. retrait des cellules souches embryonnaires humaines cultivées, et culture ultérieure des cellules souches embryonnaires humaines cultivées retirées dans des conditions hypoxiques, sur un substrat de culture tissulaire qui n'est pas prétraité avec une protéine ou une matrice extracellulaire avant culture des cellules, dans un milieu de culture contenant du sérum, de l'activine-A, un ligand Wnt, et éventuellement IGF-1,
dans laquelle les cellules exprimant des marqueurs de pluripotence expriment au moins un des marqueurs de pluripotence choisis dans le groupe constitué par ABCG2, cripto, FoxD3, connexine43, connexine45, Oct4, SOX-2, Nanog, hTERT, UTF-1, ZFP42, SSEA-3, SSEA-4, Tral-60, et Tral-81,
et dans laquelle les cellules exprimant des marqueurs de pluripotence expriment des marqueurs caractéristiques de :
cellules de ligne pré-primitive ; ou
cellules de ligne primitive ; ou
cellules mésoendodermiques.

2. Méthode selon la revendication 1, dans laquelle les cellules souches embryonnaires humaines sont traitées avec un inhibiteur de kinase Rho.

3. Méthode selon la revendication 1, dans laquelle les cellules souches embryonnaires humaines sont cultivées sur une matrice extracellulaire avant retrait des cellules.

4. Méthode selon la revendication 1, dans laquelle les cellules souches embryonnaires humaines sont cultivées sur une couche de cellules nourricières avant retrait des cellules.

5. Méthode selon la revendication 1, dans laquelle les cellules souches embryonnaires humaines sont cultivées dans des conditions normoxiques avant retrait des cellules.

6. Méthode selon la revendication 1, dans laquelle les cellules souches embryonnaires humaines sont cultivées dans des conditions hypoxiques avant retrait des cellules.

7. Méthode selon la revendication 1, dans laquelle les conditions hypoxiques sont un niveau de O₂ de 1 % à 20 %.

8. Méthode selon la revendication 1, dans laquelle les conditions hypoxiques sont un niveau de O₂ de 2 % à 10 %.

9. Méthode selon la revendication 1, dans laquelle les conditions hypoxiques sont un niveau de O₂ de 3 %.

10. Méthode selon la revendication 1, dans laquelle les cellules sont cultivées dans des conditions hypoxiques, sur un substrat de culture tissulaire qui n'est pas prétraité avec une protéine ou une matrice extracellulaire, dans un milieu contenant du sérum à une concentration de 2 % à 5 %.

11. Méthode selon la revendication 1, dans laquelle les cellules sont cultivées dans des conditions hypoxiques, sur un substrat de culture tissulaire qui n'est pas prétraité avec une protéine ou une matrice extracellulaire, dans un milieu contenant du sérum à une concentration de 2 %.

12. Méthode selon la revendication 1, dans laquelle les cellules sont cultivées dans des conditions hypoxiques, sur un substrat de culture tissulaire qui n'est pas prétraité avec une protéine ou une matrice extracellulaire, dans un milieu contenant :
IGF-1 ; ou
activine-A à une concentration de 50 ng/ml à 100 ng/ml ; ou
activine-A à une concentration de 100 ng/ml ; ou
un ligand Wnt à une concentration de 10 ng/ml à 20 ng/ml ; ou
un ligand Wnt à une concentration de 200 ng/ml ; ou
IGF-1 à une concentration de 25 ng/ml à 50 ng/ml ; ou
IGF-1 à une concentration de 50 ng/ml.

13. Méthode selon la revendication 1, dans laquelle le ligand Wnt est Wnt-3a.

14. Méthode selon la revendication 1, dans laquelle les cellules exprimant des marqueurs de pluripotence expriment des marqueurs caractéristiques de :
cellules de ligne pré-primitive et dans laquelle les marqueurs sont Nodal ou FGF8 ; ou
cellules de ligne primitive et dans laquelle les marqueurs sont choisis dans le groupe constitué par Barachyury, protéine homéobox de type Mix, et FGF4 ; ou
cellules mésoendodermiques et dans laquelle les marqueurs sont choisis dans le groupe constitué par CD48, éomésodermine, SOX-17, DKK4, HNF-3 bêta, GSC, FGF16, et GATA6.
